# EUROPEAN PATENT APPLICATION

(11) **EP 2 471 909 A1**
(43) Date of publication of application: **04.07.2012**
(21) Application number: 10016217.1
(22) Date of filing: 30.12.2010
(51) Int. Cl.: C12N 7/02, C12N 15/861

(54) **Nucleic acid molecules for generating adenoviral vectors**

(71) Applicant: SIRION BIOTECH GmbH, 82152 Planegg/Martinsried (DE)
(72) Inventor: Thirion, Christian, 80337 Munich (DE)
(74) Representative: Bohmann, Armin K.

(57) **Abstract**

The present invention is related to a nucleic acid molecule, which is also referred to as third nucleic molecule, wherein the third nucleic acid molecule comprises
(1) a nucleic acid molecule comprising the following elements:
(a) optionally, a first part of a genome of a virus;
(b) a nucleotide sequence, preferably a genomic nucleotide sequence, or a transcription unit;
(c) a regulatory nucleic acid sequence which has a regulatory activity in a prokaryote;
(d) a site-specific recombination site;
(e) a nucleotide sequence providing for a negative selection marker;
(f) a bacterial nucleotide sequence unit comprising (i) bacterial nucleotide sequences for conditional replication and (ii) a nucleotide sequence providing for a positive selection marker;
(g) optionally a first restriction site; or

(2) a nucleic acid molecule comprising a nucleotide sequence according to SEQ ID NO: 6; or
(3) a nucleic acid molecule identical or similar to the nucleic acid molecule contained in the organism deposited with the DSMZ under the Budapest treaty under accession number DSM 23754, wherein preferably the nucleic acid molecule contained in the organism is a heterologous nucleic acid molecule;

wherein the third nucleic acid molecule is either a linear or a circular molecule.

## Description

### Field of the invention

The present invention is related to a first nucleic acid molecule, a second nucleic acid molecule, a third nucleic acid, a combination of the first and the second nucleic acid molecule, a combination of the second and the third nucleic acid, a fourth nucleic acid molecule, a fifth nucleic acid molecule, methods for the generation of nucleic acid molecules coding for a virus, methods for the generation of a library of nucleic sequences, a plurality of the fourth nucleic acid molecule, a plurality of the fifth nucleic acid molecule, a plurality of individual adenoviruses and kits containing at least thereof.

### Background of the Invention

The development of recombinant viruses for gene expression since the '80s led to their widely application as gene expression vectors in vitro as well as in vivo. Cloning and expression of numerous genes, including non-coding nucleic acids such as small interfering RNAs using viral or non-viral expression libraries, is recognized as a most powerful tool in functional genomics and already led to the discovery and validation of new drug target genes. Generating virus-based expression libraries requires a cloning procedure yielding a large number of accurate clones, preferably with no need for screening positive recombinants, and ensuring stability of the viral genomes in the DNA-based constructs during amplification.

Particularly, preferred viral vectors are adenoviral vectors. The construction of adenoviral vectors can be effected by various means. The first protocols provided in the literature involved co-transfection of permissive cells, usually gene complementing cell lines such as 293 or 911 cells, with a shuttle plasmid containing the left end of the viral genome, where the E1 region typically was replaced with foreign DNA, and isolated viral DNA cut near the left end of the genome by an appropriate restriction enzyme. Homologous recombination occurs in vivo between overlapping sequences of the shuttle plasmid and the adenoviral DNA yielding a recombined virus genome that can replicate. The applicability of this technology for vector construction is limited by the inefficient transfection of large isolated viral DNA fragments and moreover vector preparations can be contaminated by wild type adenoviruses due to only partial digestion of the adenovirus DNA.

One variation of this system comprises the use of two plasmids each providing a part of the adenovirus genome individually unable to replicate which are co-transfected into the complementing production cell line to produce replicable viral DNA through homologous recombination. This method has been described in detail (Bett et al. "Packaging capacity and stability of human adenovirus type 5 vectors", J. Virol. 67:5921-5921, 1993)₃. The disadvantage of wild type virus contamination, also referred to as wt-Virus contamination, has been overcome by this variation. The use of this method to generate large numbers of recombinant adenovirus vectors is limited by the low recombination efficiency and transfection efficiency of large vector DNAs in producer cells such as 293, however. In general, adenovirus vector construction through homologous recombination between two DNA entities in eukaryotic cells supporting replication of E1-deleted adenoviruses is time consuming, and requires screening and purification of individual virus clones by plaque purification.

Site-specific recombinases as involved in the recombination processes of the viral DNA fragments, are proteins that have both endonuclease and ligase properties and exist in multiple organisms. These recombinases recognize specific sequences of bases in DNA and exchange the DNA segments flanking those segments. Thus the resulting recombination product either consists as an insertion of the first nucleic acid into the second nucleic acid. In such case the plasmids are circular plasmids containing one recombinase recognition sequence on each nucleic acid. Alternatively, there is an excision of the nucleic acid fragment in between two recombinase recognition sequences on the same nucleic acid, or an exchange of parts of nucleic acids between two nucleic acids having each of the exchanged nucleic acid in between two recognition sites present on each of the nucleic acids. Two plasmids having each one *lox*P, or other recombinase binding sites, such as, e.g., a Frt recombinase recognition site, will form a mixture of monomer, dimer, trimer, ect. product. Numerous recombination systems from various organisms have been described. (Landy A., "Mechanistic and structural complexity in the site-specific recombination pathways of Int and Flp", Curr Opin Genet Dev. 3:699-707, 1993₄; Hoess RH., et al. "P1 site-specific recombination: Nucleotide sequence of the recombining sites", Proc. Natl. Acad. Sci. USA 79:3398-3402,1982₅; Abremski et al. "Bacteriophage P1 Cre-loxP Site-specific Recombination: Site-specific DNA Topoisomerase Activity of the Cre Recombination Protein", J Biol Chem 261:391-396, 1986₆; Campbell, AM. "Chromosomal Insertion Sites for Phages and Plasmids", J Bacteriol 174:7495-7499, 1992₇; Qian XH., et al. "Reactions between Half- and Full-Flp Recombination Target Sites: A Model System for Analyzing Early Steps in Flp Protein-Mediated Site-Specific Recombination", J. Biol. Chem. 267:7794-7805, 1992₈; Araki H., et al. "Site-specific Recombinase, R, Encoded by Yeast Plasmid pSR1" J. Mol. Biol. 225:25-37, 1992₉; Maeser S and Kahnmann R., "The Gin recombinase of phage Mu can catalyse site-specific recombination in plant protoplasts", Mol. Gen. Genet. 230:170-176, 1991₁₀; Esposito D, Scocca JJ, "The integrase family of tyrosine recombinases: evolution of a conserved active site domain," Nucl Acids Res 25:3605-3614, 1997)₁₁. The best-studied members of the integrase family of recombinases are the Integrase/att system from bacteriophage lamda, (Landy A. "Mechanistic and structural complexity in the site-specific recombination pathways of Int and Flp", Current Opinions in Genetics and Devel. 3:699-707, 1993₄, the Cre/loxP system from bacteriophage P1 (Hoess RH, Abremski K. "The Cre-lox Recombination System," (1990) In Nucleic Acids and Molecular Biology, vol. 4. Eds.: Eckstein and Lilley, Berlin-Heidelberg: Springer-Verlag; pp. 90-109₁₂, and the Flp/FRT system from the Saccharomyces cerevisiae 2.mu. circle plasmid (Broach JR., et al. "Recombination within the Yeast Plasmid 2.mu. Circle is Site-Specific", Cell 29:227-234, 1982)₁₃. A system was developed for construction of adenovirus vectors by site-specific recombination mediated by Cre from bacteriophage P1 in 293 cells (Chen L, Anton M, Graham FL., "Production and characterization of human 293 cell lines expressing the site-specific recombinase Cre", Somat. Cell and Molec. Genet. 22:477-488,-1996₁₄; Hoess and Abremski, Nucleic Acids and Molecular Biology, vol.4 Eds.: Eckstein and Lilley, Berlin-Heidelberg: Springer Verlag; pp 90-109, 1990₁₂; Hardy et al., "Construction of adenovirus vectors through Cre-lox recombination ", J. Virol. 71:1842-1849, 1997)₁₅. This method provides a means to generate E1-substituted adenoviruses with insertion of foreign DNA in this region upon recombination between a shuttle plasmid containing the gene transduction unit and one loxP site, and a helper adenovirus vector deleted for its packaging signal through intramolecular recombination between two loxP sites in Cre-expressing cells. An application of this method for construction of recombinant adenoviruses through Cre-lox mediated site-specific recombination between two plasmids in 293Cre cells was disclosed in U.S. patent 6,379,943, herein incorporated by reference₁₆. Another application of this method was described for the construction of helper-dependent gutless adenovirus vectors (Parks et al., "A new helper dependent adenovirus vector system: removal of helper virus by cre-mediated excision of the viral packaging system" Proc. Natl. Acad. Sci. U.S.A. 93:13565-13570, 1996₁₇), and disclosed as a method for "High-efficiency Cre/loxP based system for construction of adenovirus vectors" in U.S. patent 6,379,943. Genetic elements incorporated into the adenoviral (AdV) AdV genome that are flanked by LoxP sites are subject to spontaneous excision during propagation of adenovirus vectors, however (Anton M. and Graham F.L., "Site-specific recombination mediated by an adenovirus vector expressing the Cre recombinase protein: a molecular switch for control of gene expression", J.Virol. 69:4600-4606, 1995₁₈). In another application Cre-mediated recombination was used to generate adenovirus vectors after two sequential recombination events and negative selection against an adenovirus deleted for its packaging signal after recombination (Hardy S., Kitamura M., Harris-Stansil T., Dai Y., and Phipps M.L. "Construction of adenovirus vectors through Cre-lox recombination" J. Virol. 71:1842-1849, 1997)₁₉.

Methods using Cre-mediated recombination between two nucleic acids for generation of infectious adenovirus genomes in E.coli or in eukaryotic cells fail to generate stable, unbiased libraries. The ability of Cre-recombinase to catalyze the reaction in both directions, results in adenovirus preparations that still can be contaminated by the non-recombined parental adenovirus. Moreover, two mechanisms limit the use of Cre/loxP site specific recombination for construction of genomic libraries. Due to the small size of the recognized sequence by Cre recombinase, cryptic loxP sites in genomes are present, inducing either recombination between compatible sites, or introducing single- or double-strand breaks, affecting the ability to grow and modify BACs, PACs, Cosmids or Fosmids containing loxP sites in E.coli strains expressing Cre, even if an inducible system is used for Cre expression (Semprini S, Troup TJ, Kotelevtseva N, King K, Davis JR, Mullins LJ, Chapman KE, Dunbar DR, Mullins JJ. Cryptic loxP sites in mammalian genomes: genome-wide distribution and relevance for the efficiency of BAC/PAC recombineering techniques. Nucleic Acids Res. 35:1402-1410, 1997)₂₀. This process also occurs in mammalian cells and organisms; here recombination events between cryptic (pseudo) loxP sites within the genomes of mice and humans leads to genome instability inducing illegitimate chromosome rearrangements (Schmidt EE, Taylor DS, Prigge JR, Barnett S, Capecchi MR. Illegitimate Cre-dependent chromosome rearrangements in transgenic mouse spermatids. Proc. Natl. Acad. Sci. U. S. A. 97:13702-13707, 2000₂₁; Sauer B. Identification of cryptic lox sites in the yeast genome by selection for Cre-mediated chromosome translocations that confer multiple drug resistance. J. Mol. Biol. 223:911-928, 1992₂₂). A Library of adenovirus vector genomes constructed by site-specific Cre-mediated homologous recombination thus can be subject to a significant degree of contamination, requiring intensive cell culture work and virologic methods to get single clones.

A method using Cre-lox mediated recombination to construct adenoviruses was further refined and described in Graham et al., U.S. patent 7,132,290, incorporated herein by reference23. The use of DNA-TP complexes was embodied in said patent to overcome limitations related to the low infectivity of adenovirus encoding nucleic acids when transfected in producer cells such as 293. It is known by those skilled in art that infectivity of adenovirus DNA is augmented 100-fold if DNA-TP complexes are used instead of plasmid derived DNA. The viral DNA is purified such that the terminal protein, which is attached to the 5' end of each strand of the duplex adenovirus, is left intact. Co-transfection of DNA-TP complexes harboring a loxP site together with a second plasmid yielding replication competent adenoviral DNA upon site-specific recombination in the presence of Cre recombinase can increase the number of viral plaques generated per µg viral DNA transfected significantly (Sharp PA, Moore C, Haverty J L, "The infectivity of adenovirus 5 DNA-protein complex," Virology 75:442-456, 1976₂₄; Chinnadurai G, Chinnadurai S, Green M, "Enhanced infectivity of adenovirus type 2 DNA and a DNA-protein Complex." J. Virol. 26:195-199, 1978)₂₅. The use of DNA-TP complexes (DNA-TPC) is at risk to be contaminated with parental infectious adenovirus DNA form which the DNA-TP complexes are derived from by restriction digestion.

The construction of recombinant adenovirus genomes through homologous recombination of two fragments in 293 cells using DNA-TPC was further used in combination with a positive selection with library efficiency (Elahi SM, Oualikene W, Naghdi L, O'Connor-McCourt M, Massie B. "Adenovirus-based libraries: efficient generation of recombinant adenoviruses by positive selection with the adenovirus protease. Gene Ther. 9:1238-1246, 2002)₂₆; a patent application for this method has been filed and the reader is referred for technical details to U.S. Pat. Appl. No. 20062109652₂₇. Here co-transfection of a plasmid harboring the left end ITR and the adenovirus protease expression cassette along with viral DNA-TPC deleted for the adenovirus protease gene yielded high amounts of recombinant viral vectors. A Library of adenovirus vector genomes constructed by site-specific or homologous recombination in 293 cells, however, can be subject to a significant degree of bias due to selection of virus mutants which have a variable growth properties (e.g in the case of cDNA expression libraries where the expression of the cDNA confers a growth advantage or disadvantage), and thus are over- or underrepresented in the library population. Propagation of such a library is critical, and moreover requires intensive cell culture work and virologic methods to get single clones.

Several methods of the prior art allow the construction of recombinant adenoviruses without any background of parental adenovirus genomes. Methods using direct ligation of DNA fragments to the adenovirus genome for construction of recombinant adenoviruses have been developed early on (Ballay A, Levrero M, Buendia MA, Tiollais P, Perricaudet M. "In vitro and in vivo synthesis of the hepatitis B virus surface antigen and of the receptor for polymerized human serum albumin from recombinant human adenoviruses" EMBO J. 30: 3861-5, 1985₂₈; Rosenfeld MA, Siegfried W, Yoshimura K, Yoneyama K, Fukayama M, Stier LE, Pääkkö PK, Gilardi P, Stratford-Perncaudet LD, Perricaudet M. "Adenovirus-mediated transfer of a recombinant alpha 1-antitrypsin gene to the lung epithelium in vivo" Science 252:431-4, 1991₂₉; Bett AJ, Hidaka W, Prevec L, Graham FL. "An efficient and flexible system for construction of adenovirus vectors with insertions or deletions in early regions 1 and 3" Proc. Natl. Acad. Sci. USA, 91:8801-8806,199430; Mizuguchi H, Kay MA. "Efficient construction of a recombinant adenovirus vector by an improved in vitro ligation method", Hum. Gene Ther. 9:2577-2583, 1998₃₁). However, ligation of large fragments is little efficient and scarcity of unique restriction sites limit the use of this method for construction of viral genome libraries.

Recombination between genetic elements in bacteria rather than in eukaryotic cells can be used to construct adenovirus vectors without the need for plaque purification. In an application commercialized as AdEasy^{®} system (He T-C, Zhou S, Yu J, Kinzler KW, and Vogelstein B. "A simplified system for generating recombinant adenoviruses" Proc. Natl. Acad. Sci. U.S. 95:2509-2514, 1998₃₂) recombination between a co-transfected supercoiled adenovirus genome and a shuttle plasmid occurs in BJ5183 bacteria. This bacterial strain has favorable properties for the maintenance of genetic stability of adenovirus genomes. According to the information given by the manual of the producer more than 20% of the colonies are correct recombination products. In another method described by Chartier et al. (Chartier C, Degryse E, Gantzer M, Dieterle A, Pavirani A, Mehtali M. "Efficient generation of recombinant adenovirus vectors by homologous recombination in Escherichia coli" J. Virol. 70 :4805-4810, 1996₃₃) the increased length of the two homology arms increases the recombination efficiency. An improvement of this method was described by Crouzet et al. (Crouzet J, Naudin L, Orsini C, Vigne E, Ferrero L, Le Roux A, Benoit P, Latta M, Torrent C, Branellec D, Denèfle P, Mayaux JF, Perricaudet M, Yeh P. "Recombinational construction in Escherichia coli of infectious adenoviral genomes". Proc. Natl. Acad. Sci USA, 94 :1414- 1419, 1997₃₄). Here the number of background colonies was reduced by introducing a negative selection marker. However, the efficiency and genetic stability of the system is not sufficient for large library generation, since DNA sequences cloned in plasmid vectors harboring direct repeats or repetitive DNA sequences suffers from genetic instability. This is especially true for plasmid vectors which replicate with high copy numbers in E*.coli.* In another successful attempt to use this method for construction of recombinant adenoviruses, homologous recombination in yeast was established (Ketner G, Spencer F, Tugendreich S, Connelly C, Hieter P. "Efficient manipulation of the human adenovirus genome as an infectious yeast artificial chromosome clone." Proc. Natl. Acad. Sci. USA, 91:6186-6190, 1994₃₅). This method, however, relies on linearization of DNA to induce the recombination between identical sequences. The scarcity of unique restriction sites in adenoviral genomes in addition to low YAC DNA yields obtained from large (typically 500ml) yeast spheroblast cultures limit this application.

The Gateway™ system as commercialized by Invitrogen Corp. uses site specific recombination for recombination in vitro between nucleic acids generating a third nucleic acid being selectable in host cells. For technical details it is referred to US Patent No. 7,282,326 (Invitrogen Corp.)36, and U.S. Pat No. 5,888,732 (Life Technologies)₃₇. This system yields the recombinant plasmid with high efficiency and accuracy with no background from the non-recombined plasmid vectors, and circumvents the unpredictable recombination events occurring during recombination in E.coli hosts. Recombinant adenovirus genomes, (commercialized as ViralPower™), can be generated with high efficacy, typically >90% correct recombined viral genomes (own observation and according to the manual) using this method.

However, the efficiency of the in vitro recombination decreases with the size of the DNA fragments (Katzen, F.Gateway® recombinational cloning: a biological operating system. Expert Opinion Drug Discovery 2:571-589, 2007₃₈), and in case of adenovirus genomes the resulting number of colonies obtained after transformation of appropriate E.*coli* host cells are decreased several fold, if compared to in vitro recombination between small DNA molecules, thus limiting the use of the Gateway system for construction of sized large-DNA libraries. Moreover, the efficiency of bacterial transformation, which here is the limiting factor for library construction, decreases with the size of the transformed DNA in a bacterial strain dependent way (Sheng Y, Mancino V, Birren B. "Transformation of Escherichia coli with large DNA molecules by electroporation", Nucleic Acids Res. 23:1990-1996, 1995₃₉).

Using BACs instead of plasmid vectors circumvents the instability of genomic sequences cloned into plasmid vectors in E.*coli*. Examples apply to large viral genomes cloned in plasmid vectors (Bzymek M, Lovett ST "Instability of repetitive DNA sequences: the role of replication in multiple mechanisms." Proc Natl Acad Sci U S A. 98:8319-8325, 2001₄₀; Fan ZC, Bird RC." An improved reverse genetics system for generation of bovine viral diarrhea virus as a BAC cDNA." J. Virol. Methods 149:309-315, 2008₄₁) and adenovirus vector genomes from other subgroups (Ruzsics Z, Wagner M, Osterlehner A, Cook J, Koszinowski U, Burgert HG." Transposon-assisted cloning and traceless mutagenesis of adenoviruses: Development of a novel vector based on species D." J. Virol. 80:8100-8113, 2006₄₂). The viral vectors are instable if propagated on plasmid vector in E.coli hosts and require propagation as stable genomes on bacterial artificial chromosome (BAC) plasmids. Although genomes can be maintained and manipulated in BACs, the selection procedure involves multiple steps and no method is available yet for construction of large libraries of such genomes.

The occurrence of genomic instability of genomic BACs due to the presence of cryptic Frt sites and Flp has not been observed to date. This system therefore is used here in this invention for genomic library construction. Targeted exchange of parts of nucleic acids between two nucleic acids can be achieved by use of Flp-mediated site-specific recombination if two non identical Frt sites are used. A method for targeted modification of a genome of a eukaryotic cell has been claimed in the PCT patent application WO 1999/025854, incorporated herein by reference43. Adaptation of this system for construction of genome libraries would require the exchange of a selectable marker and the gene transduction unit. However, similar to the gateway system, the efficiency of this reaction decreases with the size of the nucleic acid fragment to be exchanged and is not 100% reliable, thus making an extensive characterization of the obtained library necessary. The construction of a library of adenovirus vector genomes using Cre-mediated site-specific homologous recombination was only achieved in eukaryotic cells and therefore subject to a significant degree of contamination, requiring intensive cell culture work and virologic methods to get single clones. Usage of Cre-mediated site-specific recombination in E*.coli* is associated with genomic instability and cannot be used with state of the art high copy plasmid systems. Especially if the virus library is constructed in eukaryotic cells, a significant degree of library bias occurs due to selection of virus mutants which have variable growth properties, leading to a library with over- or underrepresented viruses. Stable propagation of such a library is critical, and moreover requires intensive cell culture work and virologic methods to get single clones. Moreover, the use of DNA-TPC fragments to enhance the infectivity of the viral DNA is at risk to be contaminated with parental infectious adenovirus DNA from which the DNA-TP complexes are derived' from by restriction digestion. The use of methods involving site-specific recombination mediated double-reciprocal exchange of nucleic acid sequences between two non-identical recombination sites for genomic library construction are limited by the efficiency and fidelity of the reaction, making an extensive screening and characterization of the resulting library necessary. Alternative systems using in vitro site-specific recombination are limited by the efficiency of the recombination reaction especially if large plasmids are used, and moreover suffer from decreased transformation efficiency of the resulting large plasmids into E*.coli.*

The problem underlying the present invention is to overcome the shortcomings of the methods of the prior art in the generation of adenovirus genomes, and to provide improved respective methods and means for performing such methods.

A still further problem underlying the present invention is to provide methods, and means for performing such methods, which allow the cloning of large nucleic acid sequences such as genomic nucleic acid sequences with high efficiency.

### Summary of the invention

These and other problems underlying the instant invention are solved by the subject matter of the independent claims. Preferred embodiments may be taken from the dependent claims.

The problem underlying the present invention is solved in a first aspect, which is also the first embodiment of the first aspect, by a first nucleic acid molecule comprising
(1) a nucleic acid molecule comprising, the following elements:
   (a) a site-specific recombination site;
   (b) a bacterial nucleotide sequence unit comprising (i) bacterial nucleotide sequences for conditional replication and (ii) a nucleotide sequence providing for a first selection marker;
   (c) a first part of a genome of a virus;
   (d) a transcription unit; and
   (e) optionally a first restriction site; or
(2) a nucleic acid molecule comprising a nucleotide sequence according to SEQ ID NO:1; or
(3) a nucleic acid molecule being similar or identical to the nucleic acid molecule contained in the organism deposited with the DSMZ according to the Budapest treaty under accession number DSM 23753, wherein preferably the nucleic acid molecule contained in the organism is a heterologous nucleic acid molecule;
wherein the first nucleic acid molecule is either a circular or a linear molecule.

In a second embodiment of the first aspect which is also an embodiment of the first embodiment of the first aspect, the first nucleic acid molecule comprises a packaging signal.

In a third embodiment of the first aspect which is als an embodiment of the first and the second embodiment of the first aspect, the first part of a genome of a virus comprises a terminal sequence of a genome of a or the virus, preferably of a genome of the virus.

In a fourth embodiment of the first aspect which is also an embodiment of the third embodiment of the first aspect, the terminal sequence of a genome of a or the virus comprises a terminal repeat of the genome of a or the virus, preferably a viral inverted terminal repeat.

In a fifth embodiment of the first aspect which is also an embodiment of the first, the second, the third and the fourth embodiment of the first aspect, the first restriction site is absent in the first part of a or the viral genome and the transcription unit.

In a sixth embodiment of the first aspect which is also an embodiment of the first, the second, the third, the fourth and the fifth embodiment of the first aspect, the first restriction site is selected from the group comprising AbsI, BstBI, PacI, PsrI, SgrDI, and SwaI.

In a seventh embodiment of the first aspect which is also an embodiment of the first, the second, the third, the fourth, the fifth and the sixth embodiment of the first aspect, the virus is an adenovirus.

In an eighth embodiment of the first aspect which is also an embodiment of the first, the second, the third, the fourth, the fifth, the sixth and the seventh embodiment of the first aspect, the virus is a human adenovirus type 5.

In a ninth embodiment of the first aspect which is also an embodiment of the first, the second, the third, the fourth, the fifth, the sixth, the seventh and the eighth embodiment of the first aspect, the elements (a) to (e) are arranged in a 5'-> 3' direction.

In a tenth embodiment of the first aspect which is also an embodiment of the ninth embodiment of the first aspect, the terminal repeat is a viral terminal repeat, preferably a left viral terminal repeat.

In an eleventh embodiment of the first aspect which is also an embodiment of the first, the second, the third, the fourth, the fifth, the sixth, the seventh and the eighth embodiment of the first aspect, the first nucleic acid molecule is a linear molecule, wherein elements (a) to (d), preferably upon cleavage of the circular molecule of the first nucleic acid molecule with the first restriction enzyme which recognizes and cleaves at the first restriction site, are arranged in a 5'-> 3' direction in the following sequence:
1. the first part of a genome of a virus;
2. the transcription unit;
3. the site-specific recombination site;
4. the bacterial nucleotide sequence unit comprising (i) bacterial nucleotide sequences for conditional replication and (ii) a nucleotide sequence providing for a first selection marker.
5. optionally a restriction site which is referred to as first restriction site

In a twelfth embodiment of the first aspect which is also an embodiment of the eleventh embodiment of the first aspect, the first part of a or the genome of a virus comprises a terminal sequence of a genome of a virus, preferably a terminal repeat sequence of a genome of a virus, and more preferably an inverted terminal repeat sequence of a genome of a virus.

In a thirteenth embodiment of the first aspect which is also an embodiment of the twelfth embodiment of the first aspect, the terminal sequence of a genome of a virus comprises a terminal repeat of a or the genome of a virus, preferably a first left terminal repeat of a or the genome of a or the virus and more preferably the terminal sequence is a first left inverted terminal repeat of a or the genome of a virus.

In a fourteenth embodiment of the first aspect which is also an embodiment of the first, the second, the third, the fourth, the fifth, the sixth, the seventh, the eighth, the ninth, the tenth, the eleventh, the twelfth and the thirteenth embodiment of the first aspect, the inverted terminal repeat is the inverted terminal sequence of adenovirus and preferably has any length from about 18 to 103 base pairs.

In a fifteenth embodiment of the first aspect which is also an embodiment of the second, the third, the fourth, the fifth, the sixth, the seventh, the eighth, the ninth, the tenth, the eleventh, the twelfth, the thirteenth and fourteenth embodiment of the first aspect, preferably to the extent they directly or indirectly refer to the ninth embodiment of the first aspect, the packing signal is a packing signal of an adenovirus.

In a sixteenth embodiment of the first aspect which is also an embodiment of the first, the second, the third, the fourth, the fifth, the sixth, the seventh, the eighth, the ninth, the tenth, the eleventh, the twelfth, the thirteenth, the fourteenth and the fifteenth embodiment of the first aspect, the transcription unit comprises a promoter, optionally a nucleic acid sequence to be expressed, and a termination signal, whereby preferably, the promoter and the nucleic acid to be expressed are operably linked to each other, more preferably to promoter, the nucleic acid and the termination signal are operably linked to each other.

In a seventeenth embodiment of the first aspect which is also an embodiment of the sixteenth embodiment of the first aspect, the promoter is selected from the group comprising eukaryotic promoters, viral promoters, promoters recognized by RNA Polymerase II and promoters recognized by RNA Polymerase III , wherein, preferably, the promoters recognized by RNA polymerase II are selected from the group comprising the PGK promoter and the CMV promoter, and wherein, preferably, the promoters recognized by RNA polymerase are selected from the group comprising the U6 promoter, the H1 promoter, the tRNA promoter and the adenovirus VA promoter.

In an eighteenth embodiment of the first aspect which is also an embodiment of the sixteenth and the seventeenth embodiment of the first aspect the sequence to be expressed is a sequence selected from the group comprising a nucleic acid coding for a peptide, a nucleic acid coding for a polypeptide, a nucleic acid coding for a protein, a non-coding RNA, an siRNA, a microRNA.

In a nineteenth embodiment of the first aspect which is also an embodiment of the sixteenth, the seventeenth and the eighteenth embodiment of the first aspect, the termination signal is selected from the group comprising eukaryotic, viral termination signals and termination signals for RNA Polymerase III-dependent promoters, preferably the termination signal is a polyA signal.

In a twentieth embodiment of the first aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth and the nineteenth embodiment of the first aspect, the site-specific recombination site is selected from the group comprising the recombination site for Flp recombinase.

In a twenty-first embodiment of the first aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth and the twentieth embodiment of the first aspect, the bacterial sequences for conditional replication comprise a replication origin.

In a twenty-second embodiment of the first aspect which is also an embodiment of the twenty-first embodiment of the first aspect, the bacterial nucleotide sequences for conditional replication comprise an origin of replication, whereby preferably the origin of replication is the minimal origin of phage gR6K.

In a twenty-third embodiment of the first aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first and the twenty-second embodiment of the first aspect, the sequence providing for a first selection marker is a nucleic acid sequence coding for an enzyme which is conferring resistance to a host cell harbouring such nucleic acid sequence coding for an enzyme.

In a twenty-fourth embodiment of the first aspect which is also an embodiment of the twenty-third embodiment of the first aspect, the resistance is resistance against an agent, preferably against an antibiotic, wherein more preferably such agent comprising gentamycin, kanamycin, zeocin, chloramphenicol, ampicillin, tetracycline.

In a twenty-fifth embodiment of the first aspect which is also an embodiment of the twenty-fourth embodiment of the first aspect, the resistance conferring gene is selected from the group comprising *bla, ant(3'')-Ia, aph(3')-II, aph(3')-II, cmlA,* ble, *aad*A, *aad*B, *sac*B and *tet*A*.*

The problem underlying the present invention is solved in a second aspect, which is also the first embodiment of the second aspect, by a second nucleic acid molecule comprising
(1) a nucleic acid molecule comprising the following elements:
   (a) a bacterial nucleotide sequence unit comprising (i) bacterial nucleotide sequences for single copy replication, and (ii) a nucleotide sequence providing for a second selection marker;
   (b) a site-specific recombination site;
   (c) a second part of a genome of a virus; and
   (d) optionally a restriction site which is referred to as second restriction site; or
(2) a nucleic acid molecule comprising a nucleotide sequence according to SEQ ID No:2 and/or SEQ ID NO:13; or
(3) a nucleic acid molecule being similar or identical to the nucleic acid molecule contained in the organisms deposited with the DSMZ according to the Budapest treaty under the accession numbers DSM 24298 and/or DSM 24299, wherein preferably the nucleic acid molecule contained in the organism is a heterologous nucleic acid molecule;
wherein the second nucleic acid molecule is either a circular or a linear molecule.

In a second embodiment of the second aspect which is also an embodiment of the first embodiment of the second aspect, the second part of a genome of a or the virus results in a complete genome, which is replication competent if combined with one or several other parts of a or the genome of a or the virus.

In a third embodiment of the second aspect which is also an embodiment of the first and the second embodiment of the second aspect, the site-specific recombination site is selected from the group comprising the recombination site for Flp recombinase.

In a fourth embodiment of the second aspect which is also an embodiment of the first, the second and the third embodiment of the second aspect, the virus is an adenovirus.

In a fifth embodiment of the second aspect which is also an embodiment of the first, the second, the third and the fourth embodiment of the second aspect, the virus is a human adenoviral type 5 genome.

In a sixth embodiment of the second aspect which is also an embodiment of the first, the second, the third, the fourth and the fifth embodiment of the second aspect, the second part of the genome of a or the virus comprises a terminal sequence of a genome of a virus, preferably of a genome of the virus.

In a seventh embodiment of the second aspect which is also an embodiment of the sixth embodiment of the second aspect, the sequence of a genome of a or the virus comprises a terminal repeat, preferably a viral terminal sequence and more preferably a terminal repeat of a or the virus and further more preferably an inverted terminal repeat of a or the virus, and even further more preferably a right inverted terminal repeat of a or the virus.

In an eighth embodiment of the second aspect which is also an embodiment of the first, the second, the third, the fourth, the fifth, the sixth and the seventh embodiment of the second aspect, the second restriction site is absent in the second part of the genome.

In a ninth embodiment of the second aspect which is also an embodiment of the first, the second, the third, the fourth, the fifth, the sixth, the seventh and the eighth embodiment of the second aspect, the second restriction site is selected from the group comprising the restriction sites for restriction enzymes AbsI, BstBI, PacI, PsrI, SgrDI, and SwaI, whereby the virus is preferably human adenovirus type 5.

In a tenth embodiment of the second aspect which is also an embodiment of the first, the second, the third, the fourth, the fifth, the sixth, the seventh, the eighth and the ninth embodiment of the second aspect, the bacterial nucleotide sequences for replication, preferably single copy replication, comprise a replication origin for single copy maintenance in prokaryotic host cells.

In an eleventh embodiment of the second aspect which is also an embodiment of the tenth embodiment of the second aspect, the replication origin is a single copy origin derived from the f-episomal factor or a P1 origin of replication.

In a twelfth embodiment of the second aspect which is also an embodiment of the first, the second, the third, the fourth, the fifth, the sixth, the seventh, the eighth, the ninth, the tenth and the eleventh embodiment of the second aspect, the second nucleic acid molecule is a linear molecule, wherein elements (a) to (d), preferably upon cleavage of the circular molecule of the second nucleic acid molecule with the second restriction enzyme which recognizes and cleaves at the second restriction site, are arranged in a 5'-> 3' direction in the following sequence:
1. a bacterial nucleotide sequence unit comprising (i) bacterial nucleotide sequences for single copy replication, and (ii) a nucleotide sequence providing for a second selection marker;
2. a site-specific recombination site;
3. a second part of a genome of a virus; and
4. optionally a restriction site which is referred to as second restriction site.

In a thirteenth embodiment of the second aspect which is also an embodiment of the first, the second, the third, the fourth, the fifth, the sixth, the seventh, the eighth, the ninth, the tenth, the eleventh and the twelfth embodiment of the second aspect, the nucleotide sequence providing for a second selection marker is a nucleic acid sequence coding for an enzyme which is conferring resistance to a host cell harbouring such nucleic acid sequence coding to an enzyme.

In a fourteenth embodiment of the second aspect which is also an embodiment of the thirteenth embodiment of the second aspect, the resistance is resistance against an agent selected from the group of positive selection markers comprising gentamycin, kanamycin, zeocin, chloramphenicol, ampicillin and streptomycin.

In a fifteenth embodiment of the second aspect which is also an embodiment of the thirteenth and the fourteenth embodiment of the second aspect, the gene conferring resistance is selected from the group comprising *bla, ant(3'')-Ia, aph(3')-II, aph(3')-II,* cmlA, ble, *aad*A, *and aad*B.

In a sixteenth embodiment of the second aspect which is also an embodiment of the first, the second, the third, the fourth, the fifth, the sixth, the seventh, the eighth, the ninth, the tenth, the eleventh, the twelfth, the thirteenth, the fourteenth and the fifteenth embodiment of the second aspect, the second nucleic acid molecule is a linear molecule, wherein elements (a) to (c), preferably upon cleavage of the circular molecule of the second nucleic acid molecule with the second restriction enzyme which recognizes and cleaves at the second restriction site, are arranged in a 5'-> 3' direction
(1) the bacterial nucleotide sequence unit comprising (i) bacterial nucleotide sequences for single copy replication, and (ii) a nucleotide sequence providing for a second selection marker
(2) the site-specific recombination site; and
(3) the second part of a genome of a virus.

In a seventeenth embodiment of the second aspect which is also an embodiment of the sixteenth embodiment of the second aspect, the second part of a or the genome of a or the virus comprises a terminal sequence of a genome of a or the virus, preferably of a genome of the virus.

In an eighteenth embodiment of the second aspect which is also an embodiment of the seventeenth embodiment of the second aspect, the terminal sequence of a or the virus comprises a terminal repeat of a or the virus, preferably a right terminal repeat of a or the virus.

In a nineteenth embodiment of the second aspect which is also an embodiment of the sixteenth, the seventeenth and the eighteenth embodiment of the second aspect, the second part of a or the genome of a or the virus comprises a packaging signal.

In a twentieth embodiment of the second aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth and the nineteenth embodiment of the second aspect, the second nucleic acid molecule is a BAC.

The problem underlying the present invention is solved in a third aspect, which is also the first embodiment of the third aspect, by a combination of a first nucleic acid molecule according to any of the first, the second, the third, the fourth, the fifth, the sixth, the seventh, the eighth, the ninth, the tenth, the eleventh, the twelfth, the thirteenth, the fourteenth, the fifteenth ,the sixteenth, the seventeenth, the eighteenth, the nineteenth, the twentieth, the twenty-first, the twenty-second, the twenty-third, the twenty-fourth and the twenty-fifth embodiment of the first aspect and a second nucleic acid molecule according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth and the twentieth embodiment of the second aspect.

In a second embodiment of the third aspect which is also an embodiment of the first embodiment of the third aspect, both the first nucleic acid molecule and the second nucleic acid molecule are present as circular closed nucleic acid molecules.

In a third embodiment of the third aspect which is also an embodiment of the first and the second embodiment of the third aspect, the first nucleic acid molecule and the second nucleic acid molecule are present as separate molecules.

In a fourth embodiment of the third aspect, which is also an embodiment of the first, the second and the third embodiment of the third aspect, the virus is an adenovirus.

In a fifth embodiment of the third aspect which is also an embodiment of the first, the second, the third and the fourth embodiment of the third aspect, the first restriction site and second restriction site are the same on both the first nucleic acid molecule and the second nucleic acid molecule.

In a sixth embodiment of the third aspect which is also an embodiment of the fifth embodiment of the third aspect, the first and second restriction site is selected from the group comprising the restriction sited for AbsI, BstBI, PacI, PsrI, SgrDI, and SwaI.

In a seventh embodiment of the third aspect which is also an embodiment of the first, the second, the third, the fourth, the fifth and the sixth embodiment of the third aspect, the first part of a or the genome of a or the virus and the second part of a or the genome of a or the virus form, if taken together, the complete virus genome.

In an eighth embodiment of the third aspect which is also an embodiment of the first, the second, the third, the fourth, the fifth, the sixth and the seventh embodiment of the third aspect, the first part of a or the genome of a or the virus and the second part of a or the genome of a or the virus form, if taken together, a or the virus genome which is replication competent in permissive cells.

In a ninth embodiment of the third aspect which is also an embodiment of the first, the second, the third, the fourth, the fifth, the sixth, the seventh and the eighth embodiment of the third aspect, the first selection marker and the second selection marker is an enzyme which is conferring resistance against an antibiotic, wherein such antibiotic is selected from the group comprising kanamycin, streptomycin, neomycin, puromycin, ampicillin, zeocin, gentamycin and chloramphenicol wherein the first selection marker is different from the second selection marker.

In a tenth embodiment of the third aspect which is also an embodiment of the first, the second, the third, the fourth, the fifth, the sixth, the seventh and the eighth embodiment of the third aspect, the first selection marker is an enzyme which is conferring resistance for kanamycin, and the second selection marker is an enzyme which is conferring a resistance for chloramphenicol.

In an eleventh embodiment of the third aspect which is also an embodiment of the first, the second, the third, the fourth, the fifth, the sixth, the seventh, the eighth, the ninth and the tenth embodiment of the third aspect, the packing signal is provided by either the first or the second nucleic acid molecule.

In a twelfth embodiment of the third aspect which is also an embodiment of the first, the second, the third, the fourth, the fifth, the sixth, the seventh, the eighth, the ninth, the tenth and the eleventh embodiment of the third aspect, one terminal repeat is provided by the first nucleic acid molecule and one terminal repeat is provided by the second nucleic acid molecule.

In a thirteenth embodiment of the third aspect which is also an embodiment of the first, the second, the third, the fourth, the fifth, the sixth, the seventh, the eighth, the ninth, the tenth, the eleventh and the twelfth embodiment of the third aspect, the first nucleic acid provides both the first part of a or the genome of a or the virus and preferably the terminal sequence of a or the genome of a or the virus, and the second part of a or the genome of a or the virus and preferably the terminal sequence of a or the genome of a or the virus, and the second nucleic acid molecule does provide neither the first part of a or the genome of a or the virus and preferably the terminal sequence of a or the genome of a or the virus, nor the second part of a or the genome of a or the virus and preferably the terminal sequence of a or the genome of a or the terminal repeat of a or the virus.

In a fourteenth embodiment of the third aspect which is also an embodiment of the first, the second, the third, the fourth, the fifth, the sixth, the seventh, the eighth, the ninth, the tenth, the eleventh, the twelfth and the thirteenth embodiment of the third aspect, the first nucleic acid provides the two inverted terminal repeats and the second nucleic acid molecule does not provide any inverted terminal repeat or the second nucleic acid molecule provides at least one inverted terminal repeat.

In a fifteenth embodiment of the third aspect which is also an embodiment of the first, the second, the third, the fourth, the fifth, the sixth, the seventh, the eighth, the ninth, the tenth, the eleventh, the twelfth, the thirteenth and the fourteenth embodiment of the third aspect, the first restriction site and the second restriction site are absent in the transcription unit, in the first and in the second part of a or the genome.

In a sixteenth embodiment of the third aspect which is also an embodiment of the first, the second, the third, the fourth, the fifth, the sixth, the seventh, the eighth, the ninth, the tenth, the eleventh, the twelfth, the thirteenth, the fourteenth and the fifteenth embodiment of the third aspect, the virus is an adenovirus, more preferably human adenovirus serotype 5.

The problem underlying the present invention is solved in a fourth aspect, which is also the first embodiment of the fourth aspect, by a method for the generation of a nucleic acid molecule coding for a virus comprising the following steps
a) providing a first nucleic acid molecule according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth and the twenty-fifth embodiment of the first aspect;
b) providing a second nucleic acid molecule according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth and the twentieth embodiment of the second aspect; or
c) providing a combination according to any of the first, the second, the third, the fourth, the fifth, the sixth, the seventh, the eighth, the ninth, the tenth, the eleventh, the twelfth, the thirteenth, the fourteenth, the fifteenth and the sixteenth embodiment of the third aspect;
d) allowing the first nucleic acid molecule and the second nucleic acid molecule to react so that a site-specific recombination occurs, wherein the site-specific recombination is mediated by a site-specific recombinase and the site-specific recombination forms a recombination product comprising a copy, preferably a single copy of a or the genome of a or the virus, whereby the genome is a complemented complete genome and the complemented complete genome is complemented by the site-specific recombination;
e) optionally selecting the recombination product; and
f) optionally cleaving the recombination product with the first restriction enzyme or the second restriction enzyme, or cleaving with both, the first and second restriction enzyme.

In a second embodiment of the fourth aspect which is also an embodiment of the first embodiment of the fourth aspect, the first nucleic acid molecule and the second nucleic acid molecule are reacted in a prokaryotic host cell and the recombination product is selected, and wherein preferably the prokaryotic host cell is *E. coli,* more preferably the host cell is the organism or an organism similar to such organism which has been deposited with the DSMZ according to the Budapest treaty under accession number DSM 23743 and/or DSM 23742,

In a third embodiment of the fourth aspect which is also an embodiment of the first and the second embodiment of the fourth aspect, the host cell is selected from a group of *E.coli* strains lacking the F-factor and being sensitive to the first and the second selection marker.

In a fourth embodiment of the fourth aspect which is also an embodiment of the first, the second and the third embodiment of the fourth aspect, the host cell is the *E. coli* K12-derived type, preferably DH10B.

In a fifth embodiment of the fourth aspect which is also an embodiment of the first embodiment of the fourth aspect, the first nucleic acid molecule and the second nucleic acid molecule are reacted in a eukaryotic host cell, more preferably the host cell is the organism or an organism similar to such organism which has been deposited with the DSMZ according to the Budapest treaty under accession number DSM ACC3077m or DSM ACC3077, in the presence of the site-specific recombinase and the step of selecting the recombination product is absent, wherein preferably the eukaryotic host cell is a permissive host cell.

In a sixth embodiment of the fourth aspect which is also an embodiment of the fifth embodiment of the fourth aspect,the site-specific recombinase is provided, and wherein the permissive host cell is a cell which is selected from the group comprising 293 cells, 911 1 cells, PER.C6 cells and N52.E6 cells.

In a seventh embodiment of the fourth aspect which is also an embodiment of the first, the second, the third, the fourth, the fifth and the sixth embodiment of the fourth aspect, the first selection marker is kanamycin and the second selection marker chloramphenicol.

In an eighth embodiment of the fourth aspect which is also an embodiment of the first, the second, the third, the fourth, the fifth, the sixth and the seventh embodiment of the fourth aspect, the first nucleic acid molecule and the second nucleic acid molecule are reacted in the presence of the recombinase.

In a ninth embodiment of the fourth aspect which is also an embodiment of the first, the second, the third, the fourth, the fifth, the sixth, the seventh and the eighth embodiment of the fourth aspect, the recombinase is interacting with the site-specific recombination site provided by the first nucleic acid molecule and the site-specific recombination site provided by the second nucleic acid molecule.

In a tenth embodiment of the fourth aspect which is also an embodiment of the eighth and the ninth embodiment of the fourth aspect, the recombinase is Flp recombinase.

In an eleventh embodiment of the fourth aspect which is also an embodiment of the first, the second, the third, the fourth, the fifth, the sixth, the seventh, the eighth, the ninth and the tenth embodiment of the fourth aspect, the recombinase is encoded by either the first nucleic acid molecule or the second nucleic acid molecule.

In a twelfth embodiment of the fourth aspect which is also an embodiment of the first, the second, the third, the fourth, the fifth, the sixth, the seventh, the eighth, the ninth and the tenth embodiment of the fourth aspect, the recombinase is provided or produced by the prokaryotic host cell or the eukaryotic host cell.

In a thirteenth embodiment of the fourth aspect which is also an embodiment of the first, the second, the third, the fourth, the fifth, the sixth, the seventh, the eighth, the ninth, the tenth, the eleventh and the twelfth embodiment of the fourth aspect, after the recombination, preferably the site-specific recombination, the recombinase is inactivated.

In a fourteenth embodiment of the fourth aspect which is also an embodiment of the first embodiment of the fourth aspect, the recombinase and preferably the Flp recombinase is controlled by an inducible promoter or a temperature-sensitive repressor.

In a fifteenth embodiment of the fourth aspect which is also an embodiment of the first, the second, the third, the fourth, the fifth, the sixth, the seventh, the eighth, the ninth, the tenth, the eleventh, the twelfth, the thirteenth and the fourteenth embodiment of the fourth aspect, the restriction enzymes are selected from the group comprising AbsI, BstBI, PacI, PsrI, SgrDI, and SwaI.

In a sixteenth embodiment of the fourth aspect which is also an embodiment of the first, the second, the third, the fourth, the fifth, the sixth, the seventh, the eighth, the ninth, the tenth, the eleventh, the twelfth, the thirteenth, the fourteenth and the fifteenth embodiment of the fourth aspect, the method comprises as a further step transfecting the complemented genome of a or the virus which is preferably a replicable virus genome into a permissive host cell.

In a seventeenth embodiment of the fourth aspect which is also an embodiment of the first, the second, the third, the fourth, the fifth, the sixth, the seventh, the eighth, the ninth, the tenth, the eleventh, the twelfth, the thirteenth, the fourteenth, the fifteenth and the sixteenth embodiment of the fourth aspect, the expression of the recombinase is controlled by a temperature-sensitive origin of replication.

In an eighteenth embodiment of the fourth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth and the seventeenth embodiment of the fourth aspect, the method is used in the construction of vectors for gene transfer, vaccine or therapeutic applications.

In a nineteenth embodiment of the fourth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth and the eighteenth embodiment of the fourth aspect, the method is used in the construction of a library of virus genomes.

The problem underlying the present invention is solved in a fifth aspect, which is also the first embodiment of the fifth aspect, by a third nucleic acid molecule comprising
(1) a nucleic acid molecule comprising the following elements:
   (a) optionally, a first part of a genome of a virus;
   (b) a nucleotide sequence, preferably a genomic nucleotide sequence, or atranscription unit;
   (c) a regulatory nucleic acid sequence which has a regulatory activity in a prokaryote;
   (d) a site-specific recombination site;
   (e) a nucleotide sequence providing for a negative selection marker;
   (f) a bacterial nucleotide sequence unit comprising (i) bacterial nucleotide sequences for conditional replication and (ii) a nucleotide sequence providing for a positive selection marker;
   (g) optionally a first restriction site; or
(2) a nucleic acid molecule comprising a nucleotide sequence according to SEQ ID NO:6; or
(3) a nucleic acid moelcule being similar or identical to the nucleic acid molecule contained in the organism deposited with the DSMZ according to the Budapest treaty under accession number DSM 23754, wherein preferably the nucleic acid molecule contained in the organism is a heterologous nucleic acid molecule;
wherein the third nucleic acid molecule is either a linear or a circular molecule.

In a second embodiment of the fifth aspect which is also an embodiment of the first embodiment of the fifth aspect, the third nucleic acid molecule is a linear molecule, wherein elements (a) to (f), preferably upon cleavage of the circular molecule of the third nucleic acid molecule with the first restriction enzyme which recognized and cleaves at the first restriction site, are arranged in a 5' -> 3' direction in the following sequence as follows:
1. optionally the first part of a genome of a virus;
2. the nucleotide sequence, preferably a genomic nucleotide sequence, or a transcription unit;
3. the regulatory nucleic acid sequence which has a regulatory activity in a prokaryote;
4. the site-specific recombination site;
5. the nucleotide sequence providing for a negative selection marker; and
6. the bacterial nucleotide sequence unit comprising (i) bacterial nucleotide sequences for conditional replication and (ii) a nucleotide sequence providing for a positive selection marker.

In a third embodiment of the fifth aspect which is also an embodiment of the first and the second embodiment of the fifth aspect, the third nucleic acid molecule further comprises the first part of a genome of a virus and, wherein, preferably the first part comprises one or more terminal sequences of a or the genome of a or the virus.

In a fourth embodiment of the fifth aspect which is also an embodiment of the third embodiment of the fifth aspect, the first part of a genome of a virus mediates or is needed for the replication and/or packaging of a or the virus in a permissive cell.

In a fifth embodiment of the fifth aspect which is also an embodiment of the first, the second, the third and the fourth embodiment of the fifth aspect, the transcription unit is a transcription unit for a nucleic acid sequence, whereby such nucleic acid sequence is a heterologous nucleic acid sequence.

In a sixth embodiment of the fifth aspect which is also an embodiment of the first, the second, the third, the fourth and the fifth embodiment of the fifth aspect, the third nucleic acid molecule is a bacterial plasmid or a bacterial artificial chromosome.

In a seventh embodiment of the fifth aspect which is also an embodiment of the first, the second, the third, the fourth, the fifth and the sixth embodiment of the fifth aspect, the third nucleic acid molecule further comprises a first restriction site.

In an eighth embodiment of the fifth aspect which is also an embodiment of the first, the second, the third, the fourth, the fifth, the sixth and the seventh embodiment of the fifth aspect, the third nucleic acid molecule further comprises a first part of a genome of a virus.

In a ninth embodiment of the fifth aspect which is also an embodiment of the eighth embodiment of the fifth aspect, the first part of a genome of a virus comprises a packaging signal.

In a tenth embodiment of the fifth aspect which is also an embodiment of the ninth embodiment of the fifth aspect, the packaging signal is derived from an adenovirus genome, preferably the packaging signal is the packaging signal Ψ5 from human adenovirus type 5.

In an eleventh embodiment of the fifth aspect which is also an embodiment of the eighth, the ninth and the tenth embodiment of the fifth aspect, the first part of a or the genome of a virus comprises the terminal sequence of a or the genome of a or the virus or one or several parts of the terminal sequence.

In a twelfth embodiment of the fifth aspect which is also an embodiment of the eleventh embodiment of the fifth aspect, the terminal sequence of a or the virus comprises one or several terminal repeats of a or the genome of a or the virus.

In a thirteenth embodiment of the fifth aspect which is also an embodiment of the eighth, the ninth, the tenth, the eleventh and the twelfth embodiment of the fifth aspect, the first part of a or the genome of a or the virus is a first part of the genome of an adenovirus, preferably a human adenovirus and more preferably the adenovirus is human adenovirus type 5, and most preferably the entire left end of adenovirus type 5 upstream of the TATA box of the E 1 transcription unit, or one or several parts thereof.

In a fourteenth embodiment of the fifth aspect which is also an embodiment of the eleventh, the twelfth and the thirteenth embodiment of the fifth aspect, the terminal sequence of a or the genome of a or the virus or one or several parts of the terminal sequence comprises an inverted terminal repeat, wherein preferably the inverted terminal repeat is the inverted terminal repeat of an adenovirus and more preferably the inverted terminal repeat is from the left end of the human adenovirus type 5.

In a fifteenth embodiment of the fifth aspect which is also an embodiment of the fourteenth embodiment of the fifth aspect, the inverted terminal repeat comprises any length from about 18 to 103 base pairs.

In a sixteenth embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth and the fifteenth embodiment of the fifth aspect, the first restriction site is absent from both the first part of a or the genome of a or the virus and the transcription unit.

In a seventeenth embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth and the sixteenth embodiment of the fifth aspect, the restriction site is absent form an adenoviral genome.

In an eighteenth embodiment of the fifth aspect which is also an embodiment of the sixteenth and the seventeenth embodiment of the fifth aspect, the virus is an adenovirus, preferably a human adenovirus, and more preferably human adenovirus type 5.

In a nineteenth embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth and the eighteenth embodiment of the fifth aspect, the restriction site is a restriction site for a restriction enzyme, whereby the restriction enzyme is selected from the group comprising AbsI, BstBI, PacI, PsrI, SgrDI, and SwaI.

In a twentieth embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth and the nineteenth embodiment of the fifth aspect, the third nucleic acid molecule comprises, preferably in a 5' -> 3' orientation,
1. the regulatory nucleic acid sequence which has a regulatory activity in a prokaryote;
2. the site-specific recombination site;
3. the nucleotide sequence providing for a negative selection marker;
4. the bacterial nucleotide sequence unit comprising (i) bacterial nucleotide sequences for conditional replication and (ii) a nucleotide sequence providing for a positive selection marker;
5. the first restriction site;
6. the first part of a or the genome of a or the virus; and
7. the transcription unit.

In a twenty-first embodiment of the fifth aspect which is also an embodiment of the twentieth embodiment of the fifth aspect, the first part of a or the genome of a or the virus comprises a terminal repeat, whereby preferably said terminal repeat is a left terminal repeat and more preferably the left terminal repeat is the rigth inverted terminal repeat of an adenovirus genome.

In a twenty-second embodiment of the fifth aspect which is also an embodiment of the twentieth embodiment of the fifth aspect, the first part of a or the genome of a or the virus comprises a terminal repeat, preferably an inverted terminal repeat, more preferably a right terminal repeat of a virus genome, and even more preferably the right terminal repeat of an adenovirus.

In a twenty-third embodiment of the fifth aspect which is also an embodiment of the twenty-second embodiment of the fifth aspect, the right terminal repeat of an adenovirus comprises at least 103 nucleotides of the genome of a human adenovirus type 5 virus genome.

In a twenty-fourth embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth and the nineteenth embodiment of the fifth aspect, the first part of a or the genome of a or the virus comprises a packaging signal, whereby preferably the packaging signal is an adenoviral packaging signal, and more preferably the adenoviral packaging signal is the Ψ5 of the left end of the human adenovirus type 5.

In a twenty-fifth embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third and the twenty-fourth embodiment of the fifth aspect, preferably of the twentieth, the twenty-first, the twenty-second, the twenty-third and the twenty-fourth embodiment of the fifth aspect, the transcription unit comprises a promoter, a nucleic acid to be expressed, and a termination signal.

In a twenty-sixth embodiment of the fifth aspect which is also an embodiment of the twenty-fifth embodiment of the fifth aspect, the promoter is active in eukaryotic cells, preferably the promoter is selected from the group comprising eukaryotic or prokaryotic promoters.

In a twenty-seventh embodiment of the fifth aspect which is also an embodiment of the twenty-fifth and twenty-sixth embodiment of the fifth aspect, the promoter is one which is recognized by RNA polymerase II, whereby such promoter is preferably selected from the group comprising the PGK promoter and CMV promoter.

In a twenty-eighth embodiment of the fifth aspect which is also an embodiment of the twenty-fifth and twenty-sixth embodiment of the fifth aspect, the promoter is one which is recognized by RNA polymerase III, whereby such promoter is preferably selected from the group comprising the U6 promoter, the H1 promoter, the tRNA promoter, the adenovirus VA promoter.

In a twenty-ninth embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, twenty-sixth, the twenty-seventh and the twenty-eighth embodiment of the fifth aspect, prefereably of the twentieth, the twenty-first, the twenty-second, the twenty-third, the twenty-fourth, the twenty-fifth, twenty-sixth, the twenty-seventh and the twenty-eighth embodiment of the fifth aspect, the nucleic acid to be expressed is selected from the group comprising coding nucleic acids and non-coding nucleic acid sequences.

In a thirtieth embodiment of the fifth aspect which is also an embodiment of the twenty-ninth embodiment of the fifth aspect, the nucleic acid to be expressed is a coding nucleic acid, whereby the coding nucleic acid codes for a protein, a polypeptide or a peptide.

In a thirty-first embodiment of the fifth aspect which is also an embodiment of the twenty-ninth embodiment of the fifth aspect, the nucleic acid to be expressed is a non-coding RNA, preferably the nucleic acid to be expressed is a microRNA, a small interfering RNA (siRNA) or a shRNA.

In a thirty-second embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, twenty-sixth, the twenty-seventh, the twenty-eighth, the twenty-ninth, the thirtiest and the thirty-first embodiment of the fifth aspect, preferably of any of the twentieth, the twenty-first, the twenty-second, the twenty-third, the twenty-fourth, the twenty-fifth, twenty-sixth, the twenty-seventh, the twenty-eighth, the twenty-ninth, the thirtiest and the thirty-first embodiment of the fifth aspect, the termination is signal is a termination signal of a eukaryotic or a viral gene, preferably the termination signal is selected from the group comprising a polyA signal and termination signals for RNA Pol III-transcribed genes.

In a thirty-third embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first and the thirty-second embodiment of the fifth aspect, preferably of any of the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first and the thirty-second embodiment of the fifth aspect the site-specific recombination site is the site for the Flp recombinase.

In a thirty-fourth embodiment of the fifth aspect which is also an embodiment of the thirty-third embodiment of the fifth aspect, the site for the Flp recombinase is the wild type site or a derivative thereof, whereby the derivative thereof is suitable to bind to the Flp recombinase.

In a thirty-fifth embodiment of the fifth aspect which is also an embodiment of the thirty-fourth embodiment of the fifth aspect, the derivative of the site for the Flp recombinase is a minimal recombination site having a length of 34 nucleotides and comprising the R2, the U and the R3 element of the wild type FRT site.

In a thirty-sixth embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth and the thirty-fifth embodiment of the fifth aspect, preferably of any of the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth and the thirty-fifth embodiment of the fifth aspect, the bacterial nucleotide sequences for conditional replication comprise an origin of replication, whereby preferably the origin of replication is the minimal origin of phage gR6K.

In a thirty-seventh embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth and the thirty-sixth embodiment of the fifth aspect, preferably of any of the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth and the thirty-sixth embodiment of the fifth aspect, the positive selection marker is mediating resistance against a selecting agent.

In a thirty-eighth embodiment of the fifth aspect which is also an embodiment of the thirty-seventh embodiment of the fifth aspect, the selecting agent is selected from the group comprising ampicillin, zeocin, gentamycin, chloramphenicol, kanamycin, neomycin and puromycin.

In a thirty-ninth embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth, the thirty-sixth, the thirty-seventh and the thirty-eighth embodiment of the fifth aspect, preferably of any of the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth, the thirty-sixth, the thirty-seventh and the thirty-eighth embodiment of the fifth aspect, the nucleotide sequence providing for a positive selection marker is gene selected from the group of genes comprising *bla, ant(3")-Ia, aph(3')-II, aph(3')-II, ble,* and *cmlA.*

In a fortieth embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth, the thirty-sixth, the thirty-seventh, the thirty-eighth and the thirty-ninth embodiment of the fifth aspect, preferably of any of the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth, the thirty-sixth, the thirty-seventh, the thirty-eighth and the thirty-ninth embodiment of the fifth aspect, the regulatory sequence which has a regulatory activity in a prokaryote is a sequence which directs expression of a nucleotide sequence in a prokaryote, preferably in a prokaryotic host cell.

In a forty-first embodiment of the fifth aspect which is also an embodiment of the fortieth embodiment of the fifth aspect, the regulatory sequence is a promoter, preferably according to the fortieth embodiment of the fifth aspect a prokaryotic promoter, and even more preferably according to the fortieth embodiment of the fifth aspect the E*.coli* galaktokinase promoter.

In a forty-second embodiment of the fifth aspect which is also an embodiment of the forty-first embodiment of the fifth aspect, the promoter is an inducible promoter, preferably an inducible prokaryotic promoter.

In a forty-third embodiment of the fifth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth, the thirty-sixth, the thirty-seventh, the thirty-eighth, the thirty-ninth, the fortieth, the forty-first and the forty-second embodiment of the fifth aspect, preferably of any of the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth, the thirty-sixth, the thirty-seventh, the thirty-eighth, thirty-ninth, the fortieth, the forty-first and the forty-second embodiment of the fifth aspect, the negative selection marker or the expression of the nucleotide sequence providing for a negative selection marker mediates or confers sensitivity to a selecting agent and/or a selecting condition.

In a forty-fourth embodiment of the fifth aspect which is also an embodiment of the forty-third embodiment of the fifth aspect, the nucleotide sequence providing for a negative selection marker is a gene selected from the group comprising the *galK, tetAR, pheS, thyA, lacy, ccdB* and *rpsL* gene.

In a forty-fifth embodiment of the fifth aspect which is also an embodiment of the forty-third and forty-fourth embodiment of the fifth aspect, the selecting agent is selected form the group comprising lipophilic compounds, sucrose, p-chlorophenylalanine, trimethoprim, t-o-nitrophenyl-□-D-galactopyranoside and streptomycin.

The problem underlying the present invention is solved in a sixth aspect, which is also the first embodiment of the sixth aspect, by a combination of a second nucleic acid molecule according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth and the twentieth embodiment of the second aspect and a third nucleic acid molecule according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth, the thirty-sixth, the thirty-seventh, the thirty-eighth, the thirty-ninth, the fortieth, the forty-first, the forty-second, the forty-third, the forty-fourth and the forty-fifth embodiment of the fifth aspect.

In a second embodiment of the sixth aspect which is also an embodiment of the first embodiment of the sixth aspect, both the third nucleic acid and the second nucleic acid are present as circular closed nucleic acid molecules.

In a third embodiment of the sixth aspect which is also an embodiment of the first and the second embodiment of the sixth aspect, the second and the third nucleic acid molecule are each present as a separate molecule.

In a fourth embodiment of the sixth aspect which is also an embodiment of the first, the second and the third embodiment of the sixth aspect, the third nucleic acid molecule is a plasmid, and the second nucleic acid molecule is a BAC.

In a fifth embodiment of the sixth aspect which is also an embodiment of the first, the second, the third and the fourth embodiment of the sixth aspect, the virus is an adenovirus.

In a sixth embodiment of the sixth aspect which is also an embodiment of the first, the second, the third, the fourth and the fifth embodiment of the sixth aspect, the first part of a or the genome of a or the virus and the second part of a or the genome of a or the virus form, if taken together, the complete genome of a virus.

In a seventh embodiment of the sixth aspect which is also an embodiment of the sixth embodiment of the sixth aspect, the virus is adenovirus, preferably human adenovirus, more preferably adenovirus type 5.

In an eighth embodiment of the sixth aspect which is also an embodiment of the first, the second, the third, the fourth, the fifth, the sixth and the seventh embodiment of the sixth aspect, the complete genome contains one, preferably exactly one transduction unit.

In a ninth embodiment of the sixth aspect which is also an embodiment of the first, the second, the third, the fourth, the fifth, the sixth, the seventh and the eighth embodiment of the sixth aspect, the first restriction site and the second restriction site are absent from both the first part of a or the genome of a or the virus and the second part of a or the genome of a or the virus, whereby preferably the virus is an adenovirus and more preferably a human adenovirus and even more preferably a human adenovirus type 5.

In a tenth embodiment of the sixth aspect which is also an embodiment of the first, the second, the third, the fourth, the fifth, the sixth, the seventh, the eighth and the ninth embodiment of the sixth aspect, the first restriction enzyme and the second restriction enzyme is selected from the group comprising AbsI, BstBI, PacI, PsrI, SgrDI, and SwaI.

In an eleventh embodiment of the sixth aspect which is also an embodiment of the first, the second, the third, the fourth, the fifth, the sixth, the seventh, the eighth, the ninth and the tenth embodiment of the sixth aspect, the complete genome can be released by digestion with the first and the second restriction enzyme, whereby preferably the first restriction enzyme and the second restriction enzyme are the same, more preferably the first and the second restriction enzyme is PacI.

In a twelfth embodiment of the sixth aspect which is also an embodiment of the first, the second, the third, the fourth, the fifth, the sixth, the seventh, the eighth, the ninth, the tenth and the eleventh embodiment of the sixth aspect, the virus comprising the complete genome is viable and replication competent in a permissive host cell, preferably a permissive cell line.

In a thirteenth embodiment of the sixth aspect which is also an embodiment of the first, the second, the third, the fourth, the fifth, the sixth, the seventh, the eighth, the ninth, the tenth, the eleventh and the twelfth embodiment of the sixth aspect, the complete genome comprises a deletion of parts of the genome of a or the virus, preferably a deletion of one or several genes or coding regions.

In a fourteenth embodiment of the sixth aspect which is also an embodiment of the thirteenth embodiment of the sixth aspect, the virus is an adenovirus and the deletion is a deletion of a region of the adenoviral genome, whereby the region is selected from the group comprising the E1 region, E2 region, E3 region, E4 region and combinations thereof.

In a fifteenth embodiment of the sixth aspect which is also an embodiment of the first, the second, the third, the fourth, the fifth, the sixth, the seventh, the eighth, the ninth, the tenth, the eleventh, the twelfth, the thirteenth and the fourteenth embodiment of the sixth aspect, the positive selection marker of the third nucleic acid molecule is conferring resistance against kanamycin, the negative selection marker of the third nucleic acid molecule is conferring sensitivity to streptomycin, and the second selection marker of the second nucleic acid molecule is conferring resistance for chloramphenicol.

In a sixteenth embodiment of the sixth aspect which is also an embodiment of the first, the second, the third, the fourth, the fifth, the sixth, the seventh, the eighth, the ninth, the tenth, the eleventh, the twelfth, the thirteenth, the fourteenth and the fifteenth embodiment of the sixth aspect, the positive selection marker of the third nucleic acid molecule is conferring resistance against kanamycin, the negative selection marker of the third nucleic acid molecule is conferring sensitivity to streptomycin, and the second selection marker of the second nucleic acid molecule is conferring resistance against chloramphenicol but not to kanamycin and/or streptomycin.

In a seventeenth embodiment of the sixth aspect which is also an embodiment of the first, the second, the third, the fourth, the fifth, the sixth, the seventh, the eighth, the ninth, the tenth, the eleventh, the twelfth, the thirteenth, the fourteenth and the fifteenth embodiment of the sixth aspect, the positive selection marker of the third nucleic acid molecule is conferring resistance against a first selecting agent, the negative selection marker of the third nucleic acid molecule is conferring sensitivity to a second selecting agent, and the second selection marker of the second nucleic acid molecule is conferring resistance against a third selecting agent, wherein the first selecting agent, the second selecting agent and the third selecting agent are different form each other.

In an eighteenth embodiment of the sixth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth and the seventeenth embodiment of the sixth aspect, the nucleotide sequence providing for a negative selection marker is under the control of a promoter, preferably under the control of a prokaryotic promoter.

In a nineteenth embodiment of the sixth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth and the eighteenth embodiment of the sixth aspect, the second nucleic acid molecule comprises a bacterial nucleotide sequence for single copy replication.

In a twentieth embodiment of the sixth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth and the nineteenth embodiment of the sixth aspect, the combination, upon having been introduced into a host cell, allows only the replication of the second nucleic acid molecule.

In a twenty-first embodiment of the sixth aspect which is also an embodiment of the twentieth embodiment of the sixth aspect, the bacterial nucleotide sequences for conditional replication comprises the minimal origin of phage gR6K, and the sequences for single copy replication comprises a nucleotide sequence which codes for factors which are parts of or the F-factor origin of replication.

In a twenty-second embodiment of the sixth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth and the twenty-first embodiment of the sixth aspect, the third nucleic acid molecule provides one terminal repeat and the second nucleic acid molecule provides one terminal repeat.

The problem underlying the present invention is solved in a seventh aspect, which is also the first embodiment of the seventh aspect, by a fourth nucleic acid molecule comprising:
the following elements of the second nucleic acid molecule as preferably defmed in the first, the second, the third, the fourth, the fifth, the sixth, the seventh, the eighth, the ninth, the tenth, the twelfth, the thirteenth, the fourteenth, the fifteenth, the sixteenth, the seventeenth, the eighteenth, the nineteenth and the twentieth embodiment of the second aspect, namely
   (a) the bacterial nucleotide sequence unit comprising (i) bacterial nucleotide sequences for single copy replication, and (ii) a nucleotide sequence providing for a second selection marker;
   (b) the site-specific recombination site;
   (c) the second part of a genome of a virus; and
   (d) the restriction site which is referred to as second restriction site;
and the following elements of the first nucleic acid molecule as preferably defined in the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-forth and the twenty-fifth embodiment of the first aspect, namely
   (a) the site-specific recombination site;
   (b) the bacterial nucleotide sequence unit comprising (i) bacterial nucleotide sequences for conditional replication and (ii) a nucleotide sequence providing for a first selection marker;
   (c) the first part of a genome of a virus;
   (d) the transcription unit; and
   (e) the first restriction site
wherein the fourth nucleic acid molecule is preferably a circular molecule, whereby, preferably, the fourth nucleic acid molecule is obtainable by a method according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth and the nineteenth embodiment of the fourth aspect.

The problem underlying the present invention is solved in an eighth aspect, which is also the first embodiment of the eighth aspect, by a method for the generation of a nucleic acid molecule coding for a virus comprising the following steps
a) providing a third nucleic acid molecule according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth, the thirty-sixth, the thirty-seventh, the thirty-eighth, the thirty-ninth, the fortieth, the forty-first, the forty-second, the forty-third, the forty-fourth and the forty-fifth embodiment of the fifth aspect;
b) providing a second nucleic acid molecule according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth and the twentieth embodiment of the second aspect; or
c) a combination according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first and - the twenty-second embodiment of the sixth aspect;
d) allowing the third and the second nucleic acid molecule to react so that a site-specific recombination occurs, wherein the site-specific recombination is mediated by a site-specific recombinase and the site-specific recombination forms a recombination product comprising a copy, preferably single copy of the genome of a or the virus, whereby the genome is a complemented complete genome and the complemented complete genome is complemented by the site-specific recombination;
e) optionally selecting the recombination product; and
f) optionally cleaving the recombination product with the first and second restriction enzyme.

In a second embodiment of the eighth aspect which is also an embodiment of the first embodiment of the eighth aspect, the third and the second nucleic acid molecule are reacted in a prokaryotic host cell preferably *E. coli,* more preferably the host cell is the organism or an organism similar to such organism which has been deposited with the DSMZ according to the Budapest treaty under accession number DSM 23743.

In a third embodiment of the eighth aspect which is also an embodiment of the first and the second embodiment of the eighth aspect, the virus is an adenovirus, preferably a human adenovirus, and more preferably human adenovirus type 5,

In a fourth embodiment of the eighth aspect which is also an embodiment of the first, the second and the third embodiment of the eighth aspect, the host cell is selected from a group of *E.coli* strains lacking the F-factor origin of replication and sensitive to the selection markers provided by both the third and the second nucleic acid molecule.

In a fifth embodiment of the eighth aspect which is also an embodiment of the first, the second, the third and the fourth embodiment of the eighth aspect, the host cell is an *E. coli* strain which is deficient in the expression of pi protein.

In a sixth embodiment of the eighth aspect which is also an embodiment of the first, the second, the third, the fourth and the fifth embodiment of the eighth aspect, the host cell is selected from a group comprising *E*. *coli* K12-derived cells, preferably DH10B.

In a seventh embodiment of the eighth aspect which is also an embodiment of the first embodiment of the eighth aspect, the third nucleic acid molecule and the second nucleic acid molecule are reacted in a eukaryotic host cell and the step of selecting the recombination product is absent, wherein preferably the eukaryotic host cell is a permissive host cell.

In an eighth embodiment of the eighth aspect which is also an embodiment of the seventh embodiment of the eighth aspect, the permissive host cell is a cell which is selected from the group comprising 293 cells, 911 cells, PER.C6 cells and N52.E6 cells.

In a ninth embodiment of the eighth aspect which is also an embodiment of the first, the second, the third, the fourth and the fifth embodiment of the eighth aspect, the number of recombination events is limited to one recombination event.

In a tenth embodiment of the eighth aspect which is also an embodiment of the first, the second, the third, the fourth, the fifth, the sixth, the seventh, the eighth and the ninth embodiment of the eighth aspect, the selection for the recombination product is performed by selecting the host cell(s) which harbour a recombination product providing the positive selection marker of the third nucleic acid molecule and the second selection marker of the second nucleic acid molecule, and which are not sensitive to the negative selection marker.

In an eleventh embodiment of the eighth aspect which is also an embodiment of the first, the second, the third, the fourth, the fifth, the sixth, the seventh, the eighth, the ninth and the tenth embodiment of the eighth aspect, the positive selection marker of the third nucleic acid molecule is conferring resistance against kanamycin, the negative selection marker of the third nucleic acid molecule is conferring sensitivity to streptomycin, and the second selection marker of the second nucleic acid molecule is conferring resistance against chloramphenicol, and wherein the selection for the recombination product is obtained by subjecting the prokaryotic host cell to kanamycin, streptomycin and chloramphenicol.

In an twelfth embodiment of the eighth aspect which is also an embodiment of the first, the second, the third, the fourth, the fifth, the sixth, the seventh, the eighth, the ninth and the tenth embodiment of the eighth aspect, the positive selection marker of the third nucleic acid molecule is conferring resistance against kanamycin, the negative selection marker of the third nucleic acid molecule is conferring sensitivity to streptomycin, and the second selection marker of the second nucleic acid molecule is conferring resistance against chloramphenicol but not to kanamycin and/or streptomycin, and wherein the selection for the recombination product is obtained by subjecting the prokaryotic host cell to kanamycin, streptomycin and chloramphenicol.

In an thirteenth embodiment of the eighth aspect which is also an embodiment of the first, the second, the third, the fourth, the fifth, the sixth, the seventh, the eighth, the ninth and the tenth embodiment of the eighth aspect, the positive selection marker of the third nucleic acid molecule is conferring resistance against a first selecting agent, the negative selection marker of the third nucleic acid molecule is conferring sensitivity to a second selecting agent, and the second selection marker of the second nucleic acid molecule is conferring resistance against a third selecting agent, wherein the first selecting agent, the second selecting agent and the third selecting agent are different form each other, and wherein the selection for the recombination product is obtained by subjecting the prokaryotic host cell to the first, the second and the third selecting agent.

In an fourteenth embodiment of the eighth aspect which is also an embodiment of the first, the second, the third, the fourth, the fifth, the sixth, the seventh, the eighth, the ninth, the tenth, the eleventh, the twelfth and the thirteenth embodiment of the eighth aspect, the third and the second nucleic acid molecule are reacted in the presence of a recombinase.

In an fifteenth embodiment of the eighth aspect which is also an embodiment of the first, the second, the third, the fourth, the fifth, the sixth, the seventh, the eighth, the ninth, the tenth, the eleventh, the twelfth, the thirteenth and the fourteenth embodiment of the eighth aspect, the recombinase is interacting with the site-specific recombination site provided by the third nucleic acid molecule and the site-specific recombination site provided by the second nucleic acid molecule.

In an sixteenth embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth and the fifteenth embodiment of the eighth aspect, the recombinase is Flp recombinase.

In an seventeenth embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth and the sixteenth embodiment of the eighth aspect, the recombinase is encoded by either the first nucleic acid molecule or the second nucleic acid molecule.

In an eighteenth embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth and the sixteenth embodiment of the eighth aspect, the recombinase is provided by the prokaryotic host cell or the eukaryotic hose cell.

In a nineteenth embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth and the eighteenth embodiment of the eighth aspect, after the recombination the recombinase is inactivated.

In a twentieth embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth and the nineteenth embodiment of the eighth aspect, the recombinase and preferably the Flp recombinase is controlled by a conditional or inducible promoter.

In a twenty-first embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth and the nineteenth embodiment of the eighth aspect, the expression of the recombinase is controlled by a temperature-sensitive origin of replication.

In a twenty-second embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth and the twenty-first embodiment of the eighth aspect, the restriction enzyme is selected from the group comprising AbsI, BstBI, PacI, PsrI, SgrDI, and SwaI.

In a twenty- third embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first and the twenty-second embodiment of the eighth aspect, the third and the second nucleic acid molecule are separately introduced into the host cell.

In a twenty-fourth embodiment of the eighth aspect which is also an embodiment of the twenty-third embodiment of the eighth aspect, the second nucleic acid molecule is introduced into the host cell prior to the third nucleic acid molecule.

In a twenty- fifth embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third and the twenty-fourth embodiment of the eighth aspect, the cleaving of the recombination product provides a complemented complete virus genome which is replication competent in permissive cells.

In a twenty-sixth embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth and the twenty-fifth embodiment of the eighth aspect, the method comprises as a further step transfecting the complemented complete virus genome into a complementing host cell.

In a twenty-seventh embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth and twenty-sixth embodiment of the eighth aspect, the method is used in the construction of vectors for gene transfer, vaccines or therapeutic applications.

In a twenty-eighth embodiment of the eighth aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, twenty-sixth and the twenty-seventh embodiment of the eighth aspect, the method is used in the construction of a library of virus genome.

The problem underlying the present invention is solved in a ninth aspect, which is also the first embodiment of the ninth aspect, by a fifth nucleic acid molecule comprising:
the following elements of the second nucleic acid molecule as preferably defined in the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth and the twentieth embodiment of the second aspect, namely
   (a) the bacterial nucleotide sequence unit comprising (i) bacterial nucleotide sequences for single copy replication, and (ii) a nucleotide sequence providing for a second selection marker;
   (b) the site-specific recombination site;
   (c) the second part of a genome of a virus; and
   (d) the restriction site which is referred to as second restriction site;
and the following elements of the third nucleic acid molecule as preferably defined in the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth, the thirty-sixth, the thirty-seventh, the thirty-eighth, the thirty-ninth, the fortieth, the forty-first, the forty-second, the forty-third, the forty-fourth and the forty-fifth embodiment of the fifth aspect, namely
   (a) optionally the first part of a genome of a virus;
   (b) the nucleotide sequence, preferably a genomic nucleotide sequence, or a transcription unit;
   (c) the regulatory nucleic acid sequence which has a regulatory activity in a prokaryote;
   (d) the site-specific recombination site;
   (e) the nucleotide sequence providing for a negative selection marker;
   (f) the bacterial nucleotide sequence unit comprising (i) bacterial nucleotide sequences for conditional replication and (ii) a nucleotide sequence providing for a positive selection marker; and
   (g) the first restriction site,
   wherein the fifth nucleic acid molecule is preferably a circular molecule, whereby, preferably, the fifth nucleic acid molecule is obtainable by a method according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh and the twenty-eighth embodiment of the eighth aspect.

The problem underlying the present invention is solved in a tenth aspect, which is also the first embodiment of the tenth aspect, by a method for generating a library of nucleotide sequences, wherein said library comprises a plurality of individual nucleotide sequences, wherein said library is represented by a plurality of viral genomes and each viral genome contains a single one of the individual nucleotide sequences, comprising the steps of the method as defined in any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh and the twenty-eighth embodiment of the eighth aspect, wherein the individual nucleotide sequence is part of the transcription unit of the third nucleic acid molecule.

The problem underlying the present invention is solved in an eleventh aspect, which is also the first embodiment of the eleventh aspect, by a method for generating a library of nucleotide sequences, wherein said library comprises a plurality of individual nucleotide sequences, wherein said library is represented by a plurality of viral genomes, comprising the steps of the method as defined in any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth and the nineteenth embodiment of the fourth aspect, wherein the individual nucleotide sequence is part of the transcription unit of the third nucleic acid molecule.

In a second embodiment of the tenth and the eleventh aspect which is also an embodiment of the first embodiment of the tenth aspect and of the first embodiment of the eleventh aspect, the individual nucleotide sequence is present in the complete virus genome as a single copy.

In a third embodiment of the tenth and the eleventh aspect which is also an embodiment of the first and the second embodiment of the tenth aspect and of the first and the second embodiment of the eleventh aspect, the individual nucleotide sequence is the nucleic acid to be expressed.

The problem underlying the present invention is solved in a twelfth aspect, which is also the first embodiment of the twelfth aspect, by a plurality of a fourth nucleic acid molecule according to the seventh aspect and preferably of the first and any other embodiment of the seventh aspect, wherein the plurality of the nucleic acid molecule consists of a number such individual the nucleic acid molecules, wherein the individual nucleic acid molecules differ from each other in the nucleic acid to be expressed which is part of the transcription unit.

The problem underlying the present invention is solved in a thirteenth aspect, which is also the first embodiment of the thirteenth aspect, by a plurality of a fifth nucleic acid molecule according to the ninth aspect and preferably of the first and any other embodiment of the ninth aspect, wherein the plurality of the nucleic acid molecule consists of a number such individual the nucleic acid molecules, wherein the individual nucleic acid molecules differ from each other in element (b) of the third nucleic acid molecule, preferably in the nucleotide sequence of element (b) or in the nucleic acid to be expressed which is part of the transcription unit of element (b).

In a second embodiment of the tweflth and the thirteenth aspect which is also an embodiment of the first embodiment of the tweflth aspect and of the first embodiment of the thirteenth aspect, the virus is an adenovirus.

The problem underlying the present invention is solved in a fourteenth aspect, which is also the first embodiment of the fourteenth aspect, by a plurality of individual adenoviruses, wherein the individual adenoviruses contain an individual nucleic acid as defined in any of the first and the second embodiment of the twelfth aspect and of the first and the second embodiment of the thirteenth aspect.

The problem underlying the present invention is solved in a fifteenth aspect, which is also the first embodiment of the fifteenth aspect, by a kit comprising optionally a package insert, and, in (a) suitable container(s), at least a first nucleic acid molecule as defined in any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth and the twenty-fifth embodiment of the first aspect, a second nucleic acid molecule as defined in any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth and the twentieth embodiment of the second aspect, optionally a permissive cell line providing the site-specific recombinase as defined in the twelfth embodiment of the fourth aspect, a combination of the first nucleic acid molecule and the second nucleic acid molecule as defined in any of the the first, the second, the third, the fourth, the fifth, the sixth, the seventh, the eighth, the ninth, the tenth, the eleventh, the twelfth, the thirteenth, the fourteenth, the fifteenth, the sixteenth embodiment of the third aspect, a third nucleic acid molecule as defined in any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtiest, the thirty-first, the thirty-second, the thirty-third, the thirty-fourth, the thirty-fifth, the thirty-sixth, the thirty-seventh, the thirty-eighth, the thirty-ninth, the fortieth, the forty-first, the forty-second, the forty-third, the forty-fourth and the forty-fifth embodiment of the fifth aspect, a combination of the third nucleic acid molecule and the second nucleic acid molecule as defined in any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first and the twenty-second embodiment of the sixth aspect, a fourth nucleic acid molecule as defined in the first embodiment of the seventh aspect, a fifth nucleic acid molecule as defined in the first embodiment of the ninth aspect, a plurality of a fourth nucleic acid molecule as defined in the first and the second embodiment of the twelfth aspect, a plurality of a fifth nucleic acid molecule as defined in the first and the second embodiment of the thirteenth aspect, or a plurality of individual adenoviruses as defined in the first embodiment of the fourteenth aspect.

In a second embodiment of the fifteenth aspect which is also an embodiment of the first embodiment of the fifteenth aspect, the nucleic acid molecule(s) is/are contained in a ready-to-use form.

In a third embodiment of the fifteenth aspect which is also an embodiment of the first and the second embodiment of the fifteenth aspect, the kit is for use in a method for the generation of nucleic acid molecules coding for a virus as defined in any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth and the nineteenth embodiment of the fourth aspect and the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh and the twenty-eighth embodiment of the eighth aspect.

In a fourth embodiment of the fifteenth aspect which is also an embodiment of the first, the second and the third embodiment of the fifteenth aspect, the kit is for use in a method for generating a library of nucleotide sequences as defined in any of the first, the second and the third embodiment of the eleventh aspect.

It will be understood by a person skilled in the art that the term "to provide" or "providing" as used herein in connection with the various methods preferably also means that the nucleic acid molecule which is provided, is available for the performance of any step subject to such methods and that there is no need to synthesize such nucleic acid molecule before or immediately before such step. Rather such nucleic acid molecule may be taken from any stock of such nucleic acid molecule.

The methods of the present invention allow the construction of infectious viral vector genomes irrespectively of the number of recombination events when using site-specific recombination in bacteria, and limit the number of recombination events to one when using site-specific recombination as described for the second nucleic acid system, whereby a first such system consists of a combination of the first nucleic acid molecule according to the present invention and the second nucleic acid molecule according to the present invention, and a second such system consists of a combination of the third nucleic acid molecule according to the present invention and the second nucleic acid molecule according to the present invention. It will be acknowledged by the persons skilled in the art that in accordance with the present invention, a certain percentage of the recombination events results in multiple recombinations. Preferably, such percentage is less than 5 %, preferably less than 3 % and more preferably less than 2.5 %. The occurrence of multiple recombination events is a contamination making it necessary to screen and characterize the recombinants. Such screening is performed in connection with the methods of the invention for the generation of nucleic acid molecules coding for a virus and is referred to as selecting the recombinant product.

Both systems disclosed can be used to generate recombinant virus vector genomes. The resulting recombination products in both systems contain exactly one copy of a virus genome irrespectively of the number of integration events. This invention provided a solution to eliminate multiple recombinations and avoid screening of a library containing a plurality of viral vector genomes. Such virus genome or plurality of virus genomes is a complemented and complete genome of a virus, preferably of an adenovirus. The genome of such virus is released by restriction digest with a unique restriction enzyme thereby removing all bacterial sequences connected to the virus genome. Therefore, the virus genomes generated by these methods are essentially free of any unwanted bacterial sequences.

The arrangement of the genetic elements in the first, the second, and the third nucleic acid molecules imply that the resulting recombination products contain exactly one copy of a complemented complete virus genome irrespectively of the number of integration events. A method is therefore disclosed in this invention in which a linearized form of the first or the third nucleic acid is reacted with a linear form of the second nucleic acid in a eukaryotic host cell permissive for the or an adenovirus in the presence of the site-specific recombinase. This method does not require the step of selecting for the recombination product.

The methods of the present invention substantially overcome current limitations of technologies making use of site-specific recombination as, e.g., subject to the Gateway ™ system, for the construction of adenovirus genomes or a plurality of adenovirus genomes. More specifically, recombination between one Frt site present on either the first or the third nucleic acid molecule according to the present invention, and on the second nucleic acid molecule circumvents the disadvantages associated with recombination between two non-identical recombination sites in vitro, and allows the generation of a plurality of viral genomes with high efficiency and fidelity.

The methods of the present invention also solve the problem of chimerism in yeast artificial chromosomes (YACs) and genomic DNA instability in multicopy cosmid or plasmid vectors. In connection the such methods this effect is mediated by the use of bacterial artificial chromosome (BAC) which replicate using the single-copy F-factor replicon (Kim UJ, Shizuya H, de Jong PJ, Birren B, Simon MI, "Stable propagation of cosmid sized human DNA inserts in an F factor based vector", Nucleic Acids Res., 20:1083-1085, 19921; Shizuya H, Kouros-Mehr H., "The development and applications of the bacterial artificial chromosome cloning system", Keio J. Med. 50:26-30,2001(Review)2) Moreover, the methods of the invention overcome the limitation of site-specific recombination for construction of pure libraries, through limiting the number of recombination events to one for recombination reactions occurring in E.coli subject to the above considerations on the percentage of multiple recombination events. The library can be stably maintained in single copy BACs, and thus overcomes the limitation of current viral expression libraries. In the case of adenovirus, libraries are maintained as life viruses and thus subject to a bias due to selection of virus mutants which have a growth advantage, which is due in the case of cDNA expression libraries where the expression of the cDNA confers a growth advantage or disadvantage, and thus are over- or underrepresented in the library population. Furthermore, by use of a selection in an inventive manner, those recombination products having multiple insertions are altogether eliminated. As preferably used herein, selection, in case of the method for the generation of a nucleic acid molecule coding for a virus using the third nucleic acid molecule and the second nucleic acid molecule, means that the reaction product can be selected by means of use of a combination of positive and negative selection markers provided by both nucleic acid molecules, whereby the positive selection marker provided by the third nucleic acid molecule confers resistance against a selecting agent, the negative selection marker provided by the third nucleic acid confers sensitivity to a selecting agent, and the second selection marker provided by the second nucleic acid, provides resistance against a second selecting agent.

Finally, the instant invention is related to recombination system allowing the construction of large DNA libraries and a solution to apply the Frt/Flp system for construction of recombinant vector genomes *in vivo.*

The instant inventor has surprisingly found that a system comprising site-specific recombination between two nucleic acids, each with one recognition site for the recombinase Flp, in an *E.coli* host harboring a plasmid which allows conditional recombinase expression was able to overcome the above mentioned limitations and allowed the construction of an adenovirus genome, and a plurality of adenovirus genomes with high efficiency, accuracy, preserving the genetic stability. Consequently the problem of the present invention is also solved by a two nucleic acid system for site specific recombination mediated by Flp recombinase, providing a high efficient reliable and simple method for construction of viral vectors based on site-specific recombination in *E.coli,* rather than relying on site-specific recombination *in vitro* or homologous recombination in eukaryotic cells.

It is a further objective of the invention to use the Flp-Frt-mediated site-specific recombination together with the disclosed two vector DNA systems, i.e. the system comprising the first nucleic acid molecules and the second nucleic acid molecule, and the system comprising the second nucleic acid molecule and the third nucleic acid molecule. This provides a high efficient, reliable, and simple method for construction of a plurality of recombined nucleic acids containing a nucleotide sequence. Preferably such nucleotide sequence is a genomic nucleotide sequence of a virus, preferably adenovirus, or (a) part(s) thereof. It is possible to use this aspect of the invention for stably maintaining genomes in excess to 300kb, in order to preserve a good transformation efficiency, however, the size of the first and the third nucleic acids should be less than 100kb and, if a library of nucleic acids shall be constructed, preferably less than 40kb, and even more preferably less than 1 Okb.

The invention also relates to nucleic acid molecules, vectors and methods using site-specific recombination to recombine said nucleic acid molecules in *E.coli.* Methods are disclosed for the generation of nucleic acid libraries, whereby in a preferred embodiment of the method of the present invention the method is for the generation of virus genomes and pluralities of virus genomes thereof. The nucleic acid molecules individually or as a combination, and methods disclosed in the present invention are more reliable and expand the use of site-specific recombination systems, allowing, among others, the construction of complemented and complete, respectively, viral genomes, and the generation of nonbiased stable, and libraries of viral genomes.

The invention also relates to methods for generation of nucleic acid molecules, generation of virus genomes, or a plurality of virus genomes thereof, using site-specific recombination to recombine said nucleic acid molecules in permissive eukaryotic host cells in the presence of the site-specific recombinase. Methods are disclosed for the generation of virus genomes or a plurality of virus genomes, whereby in a preferred embodiment of the method of the present invention the method is for the generation of adenovirus vectors. The methods disclosed in the present invention are considerably faster than established methods for adenovirus generation, and expand the use of site-specific recombination systems, allowing, the generation of virus genomes or a plurality of virus genomes, which are replication competent in a complementing cell line.

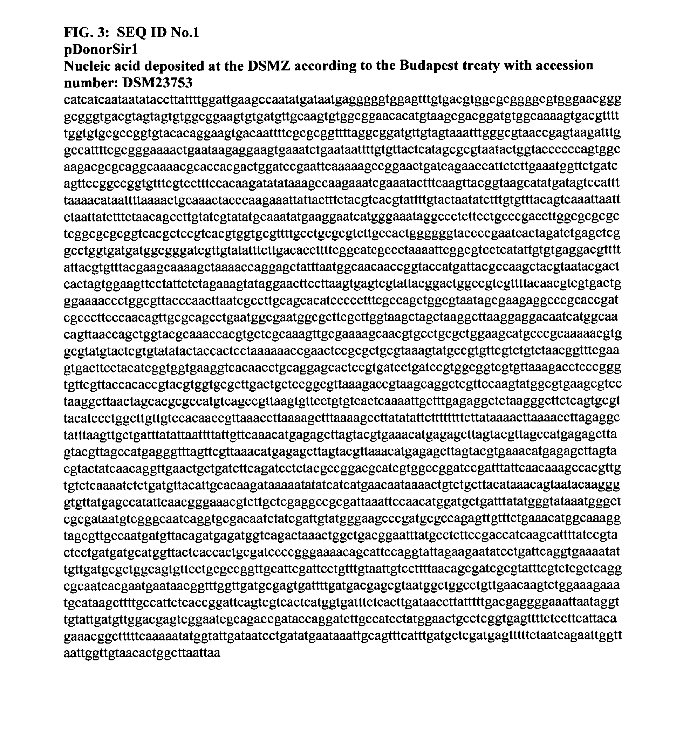

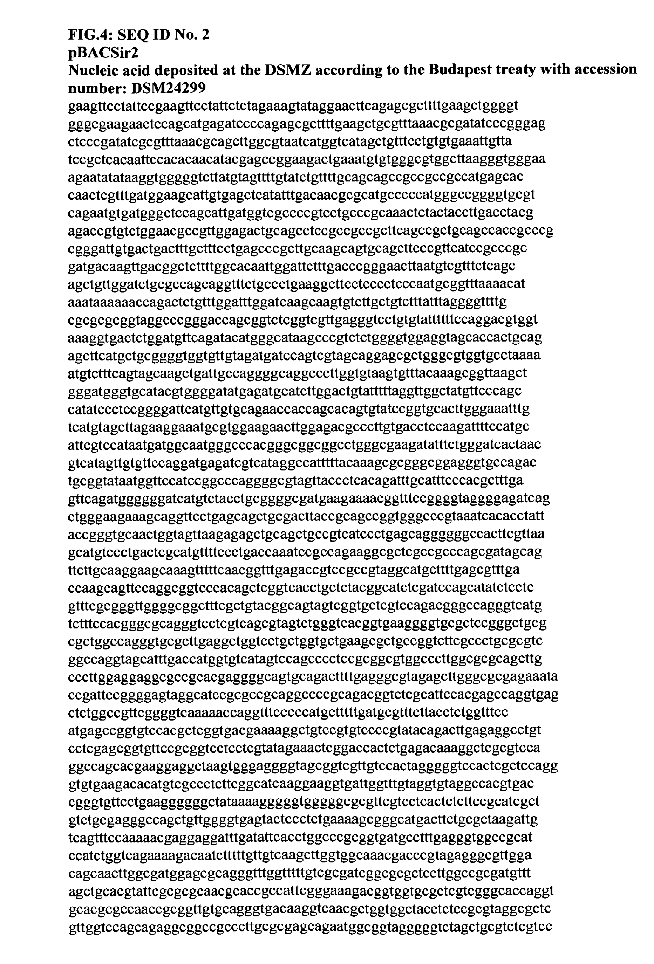

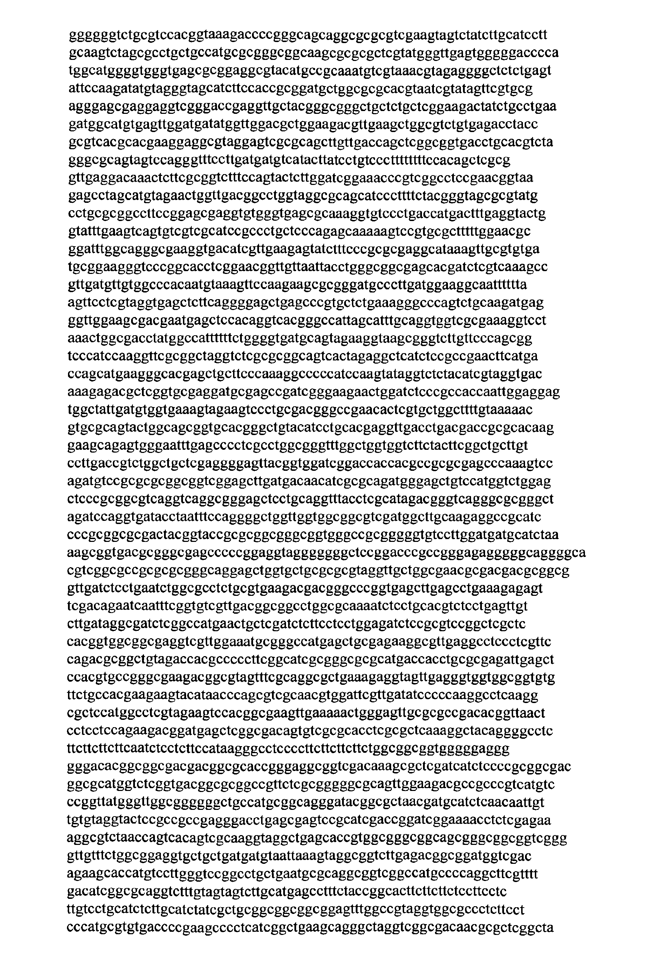

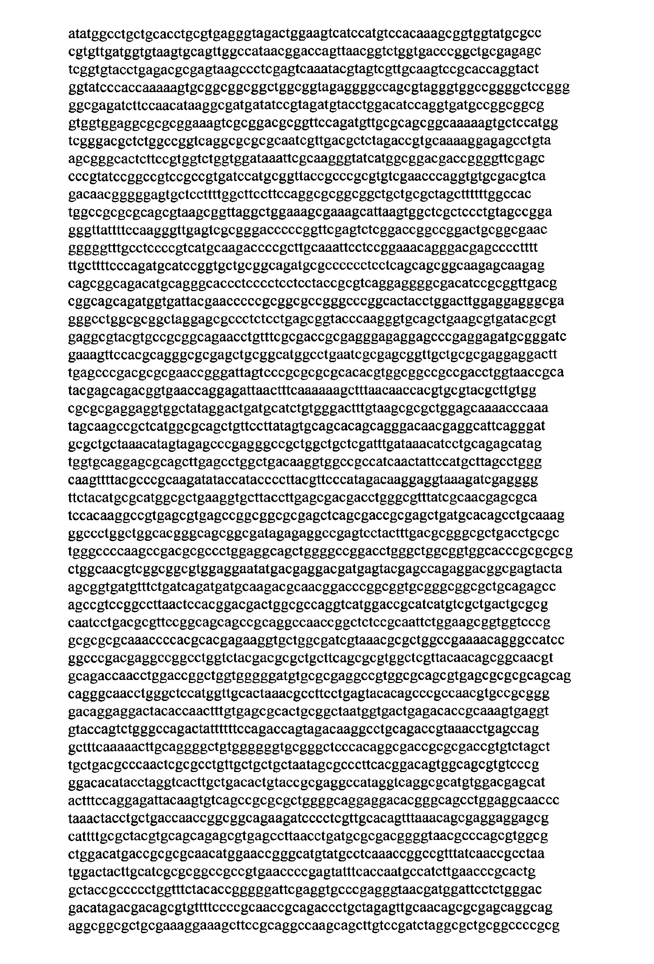

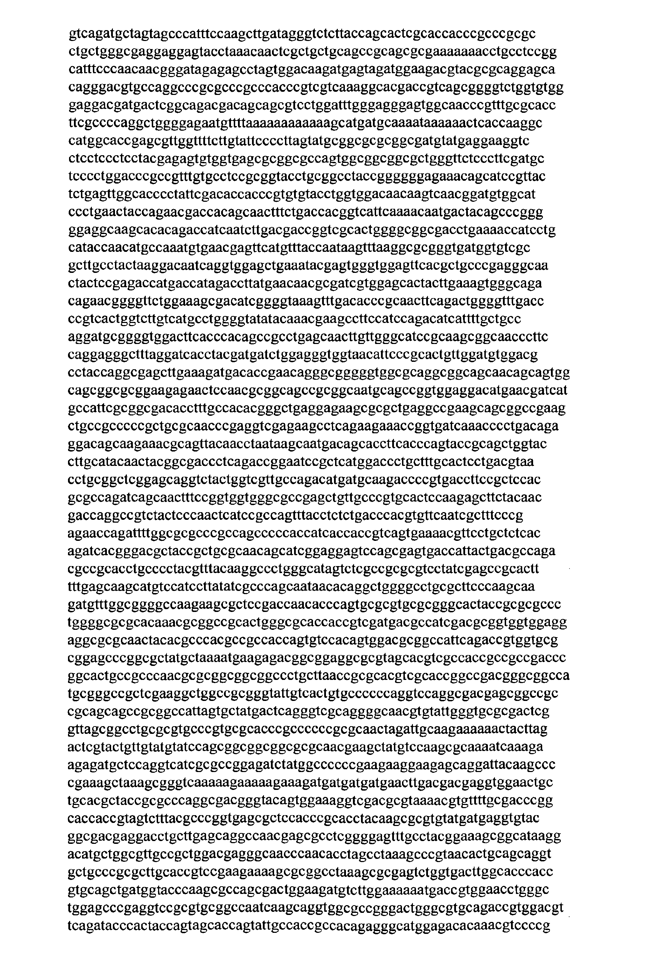

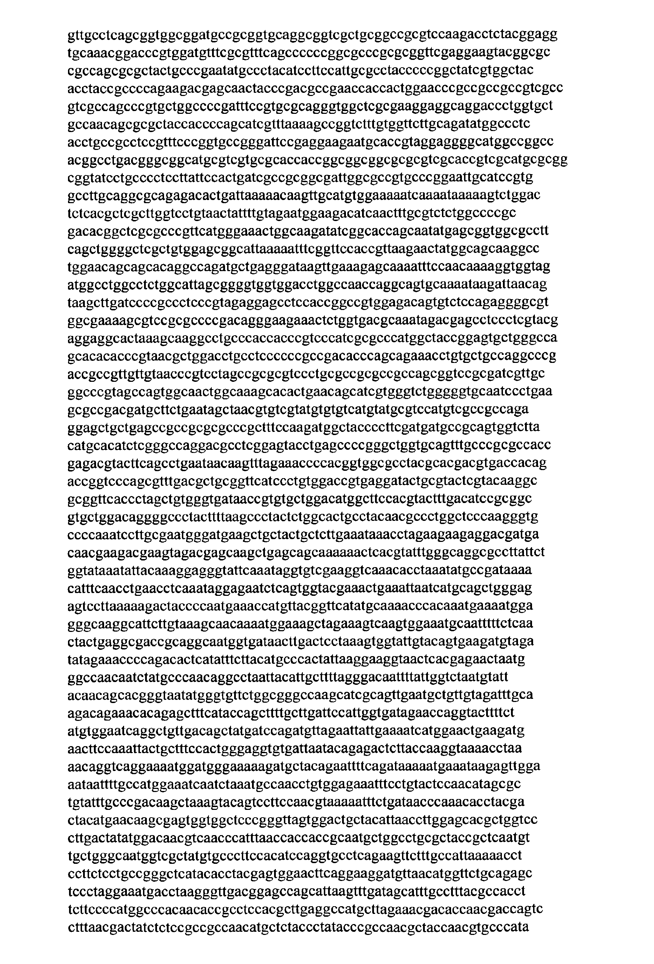

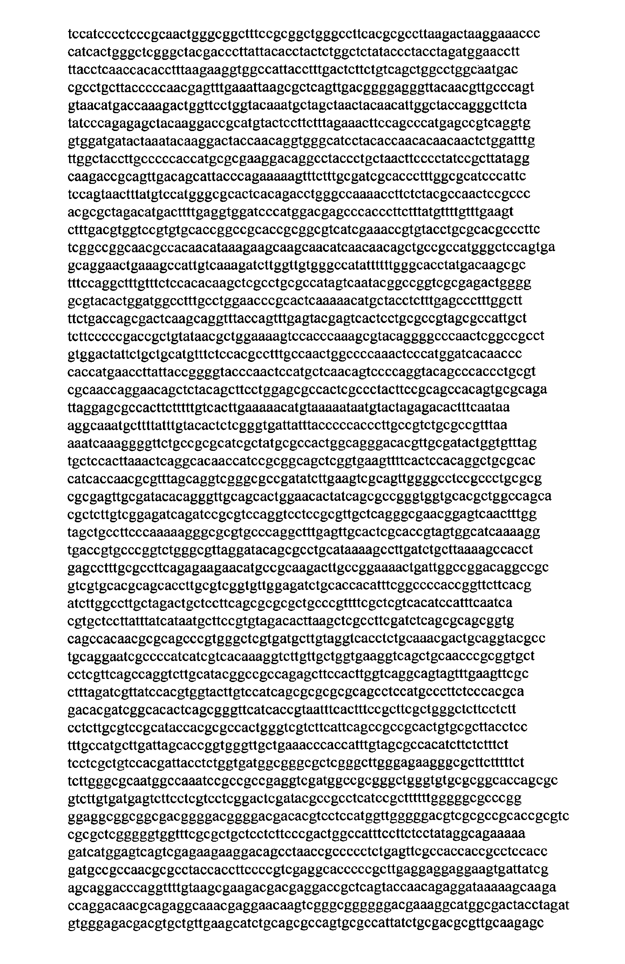

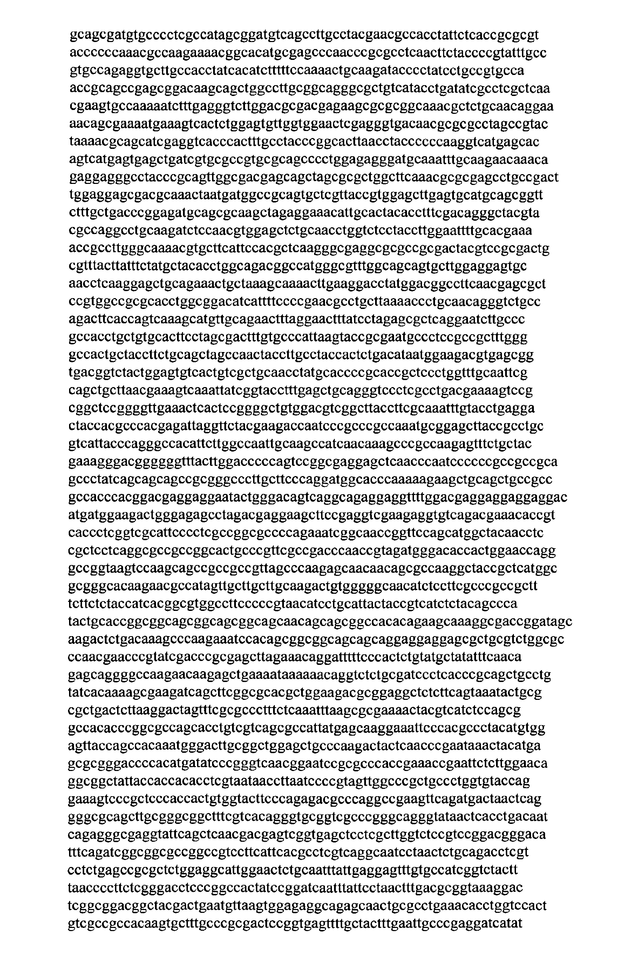

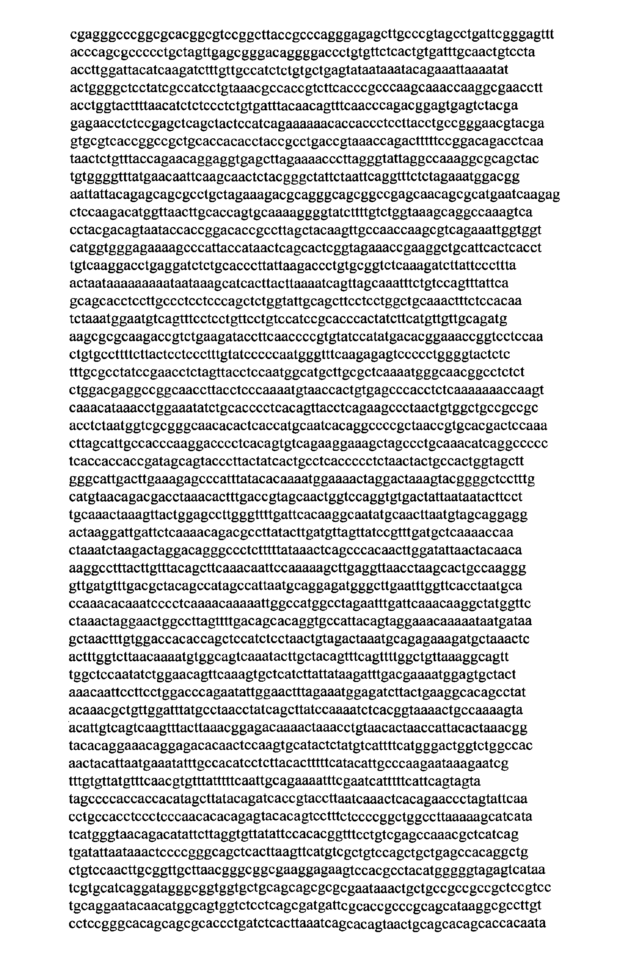

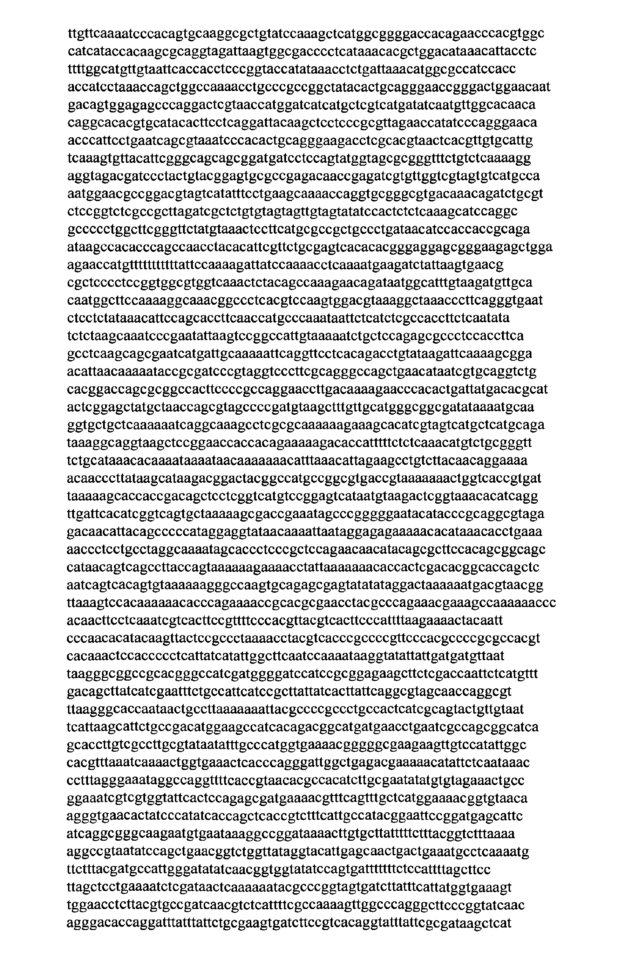

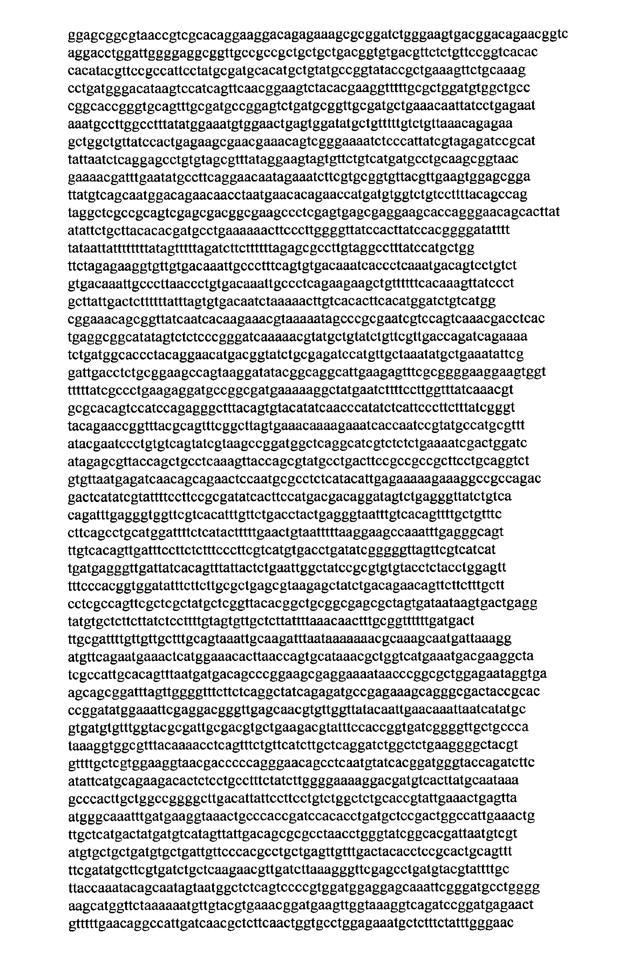

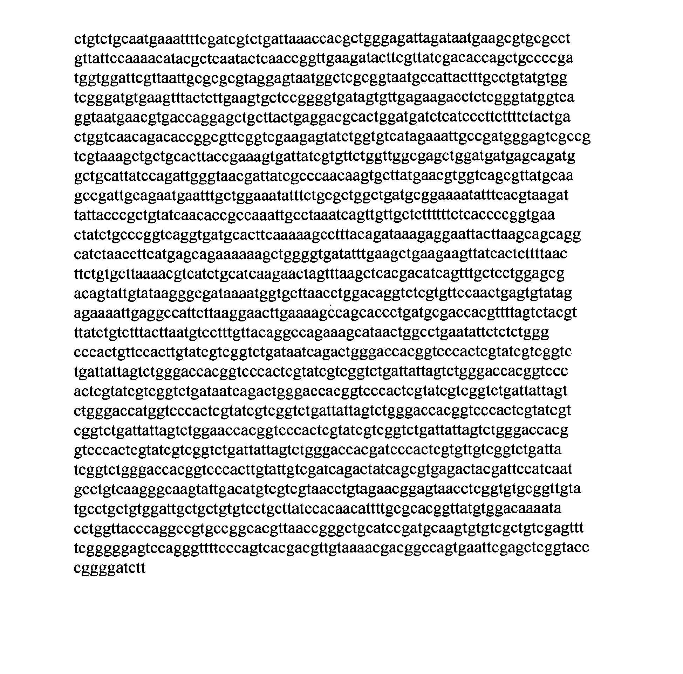

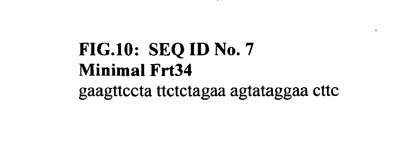

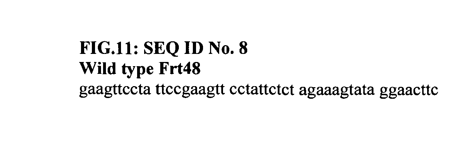

The present invention provides a first nucleic acid system in E.coli with high efficiency for site-specific recombination mediated by Flp recombinase, to join two DNAs that separately are non-replicating, and form a complemented complete genome of a virus, preferably adenovirus after recombination. One application of nucleic acid molecules of the present invention, and more specifically the first nucleic acid molecule, the second nucleic acid molecule and the third nucleic acid molecule, is the construction of adenovirus genomes deleted for E1 and optionally E3 genes, containing foreign DNA instead of the E1 gene (first-generation adenovirus vectors). In accordance with the present invention a second nucleic acid molecule which is preferably a BAC, is disclosed, containing one wild type Frt site, identical or similar to the vector pBACSir2 (Fig.4, SEQ ID No.2) comprising a bacterial nucleotide sequence unit comprising (i) bacterial nucleotide sequence for single copy replication, and (ii) a nucleotide sequence providing for a second selection marker, a site-specific recombination site, preferentially a wild type Frt site (Fig. 11 SEQ. ID 8), a second part of a genome of a virus, preferentially a second part of a genome of an adenovirus, and even more preferentially a second part of an adenovirus type 5, and optionally a second restriction site. Furthermore a plasmid identical or similar to pDonorSirlis provided (Fig.3, SEQ. ID No.1) corresponding to the first nucleic acid molecule comprising a first part of a genome of a virus, preferentially a first part of a genome of an adenovirus, and even more preferentially a second part of an adenovirus type 5, a transcription unit, a site specific recombination site, preferentially a minimal Frt site (Fig.10 SEQ. ID No.7), a bacterial nucleotide sequence unit comprising (i) bacterial nucleotide sequences for conditional replication and (ii) a nucleotide sequence providing for a first selection marker.

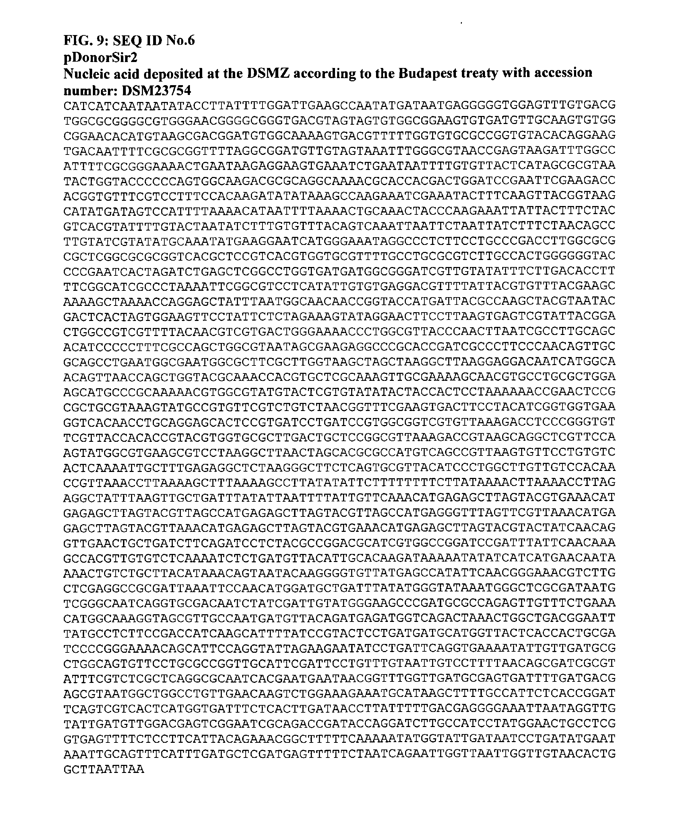

Another embodiment of the second nucleic acid system disclosed is a plasmid similar or identical to pDonorSir2 (Fig.9, SEQ ID No.6) corresponding to the third nucleic acid molecule of the present invention, containing optionally a first part of a genome of a virus, preferentially a first part of a genome of an adenovirus, and even more preferentially a second part of an adenovirus type 5 a nucleotide sequence, preferably a genomic nucleotide sequence, or a transcription unit, a regulatory nucleic acid sequence which has regulatory activity in a prokaryote, preferentially a bacterial promoter, a site-specific Frt site, preferentially a minimal Frt (Fig.10 SEQ. ID No.7), a nucleotide sequence providing for a negative selection marker, a bacterial nucleotide sequence unit comprising (i) bacterial nucleotide sequences for conditional replication and (ii) a nucleotide sequence providing for a positive selection marker, and optionally a first restriction site.

One application of this method using the two nucleic acid systems is the construction of virus genomes, or a plurality of virus genomes thereof, preferentially adenovirus genomes, or a plurality of adenovirus genomes, and more preferentially adenovirus type 5 genomes or a plurality of adenovirus type 5 genomes. One further application of the disclosed methods is the generation of viral expression libraries. The nucleic acid systems according to the present invention and the methods for the generation of a nucleic acid molecule coding for a virus genome are applicable by analogy to the manipulation and construction of equivalent non-adenovirus type 5 genomes as well. In a further embodiment such nucleic acid molecules and methods, respectively, may be applied in the mirror setting, and nucleotide sequences rescued into the right end of the adenovirus genome (e.g. E3), or applied to delete E4 genes, or to generate adenovirus genomes, or a plurality of adenovirus genomes carrying such nucleotide sequences. One application of the use of the two nucleic acid systems in the "mirror setting" is the construction of an adenovirus genome or a plurality of adenovirus genomes with mutated viral proteins.

The present invention discloses the use of a combination comprising the first nucleic acid molecule and the second nucleic acid molecule, and the use of a combination comprising the third nucleic acid molecule and the second nucleic acid molecule, respectively. The two nucleic acid molecules of each nucleic acid system are reacted in *E.coli* with high efficiency by means of site-specific recombination mediated by Flp recombinase. In the present invention the second nucleic acid molecule is a BAC and contains one, preferably a wild type Frt site, whereby the second nucleic acid molecule is identical or similar to the vector pBACSir1 or pBACSir2. Furthermore, a third nucleic acid identical or similar to pDonorSir2 is disclosed. One application of this system is the construction of a fourth, a fifth nucleic acids or a plurality of fourth or fifth nucleic acid molecules, each containing one copy of a complemented complete virus genome.

### Brief description of the examples

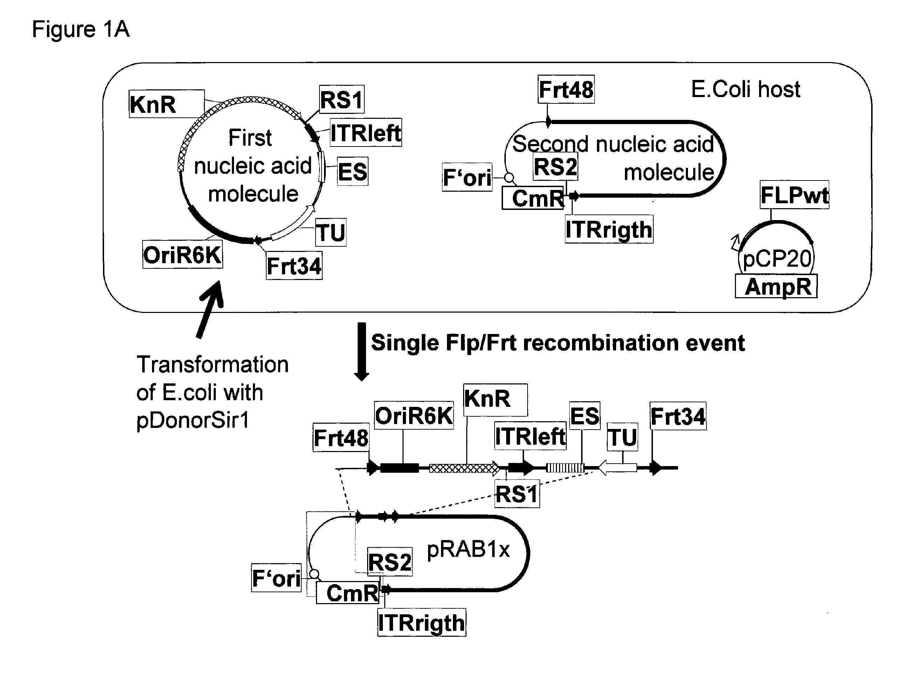

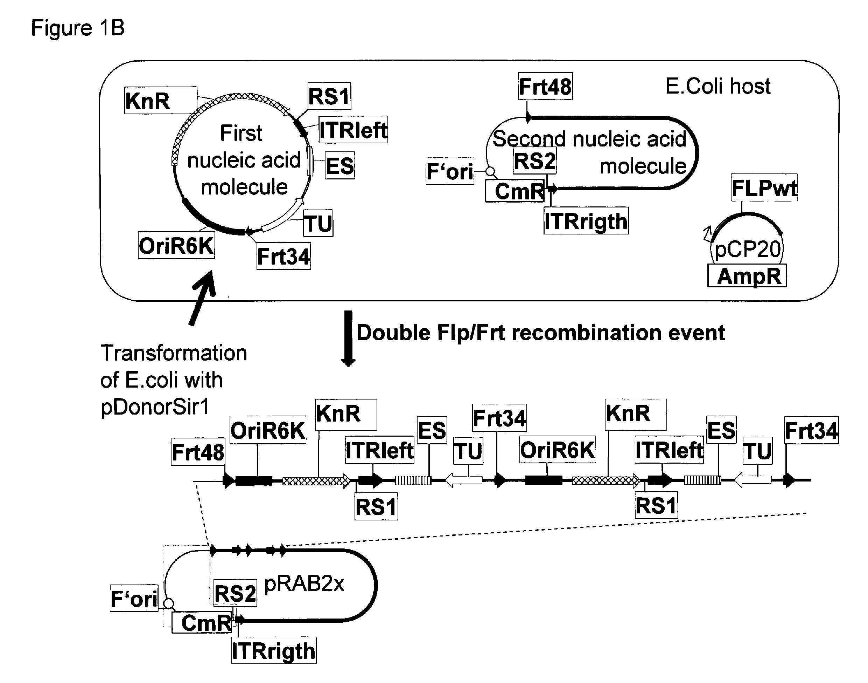

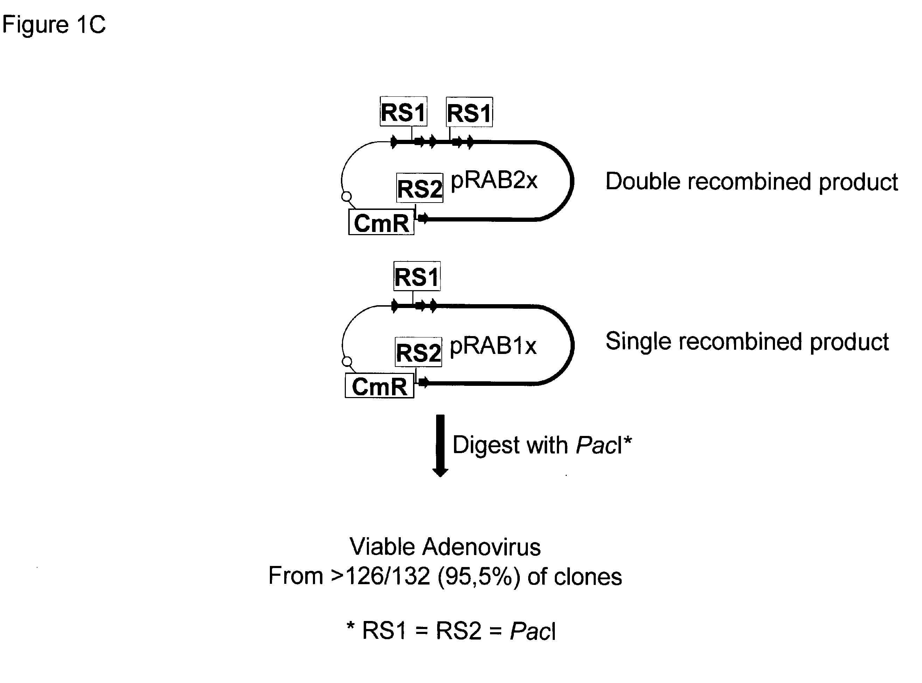

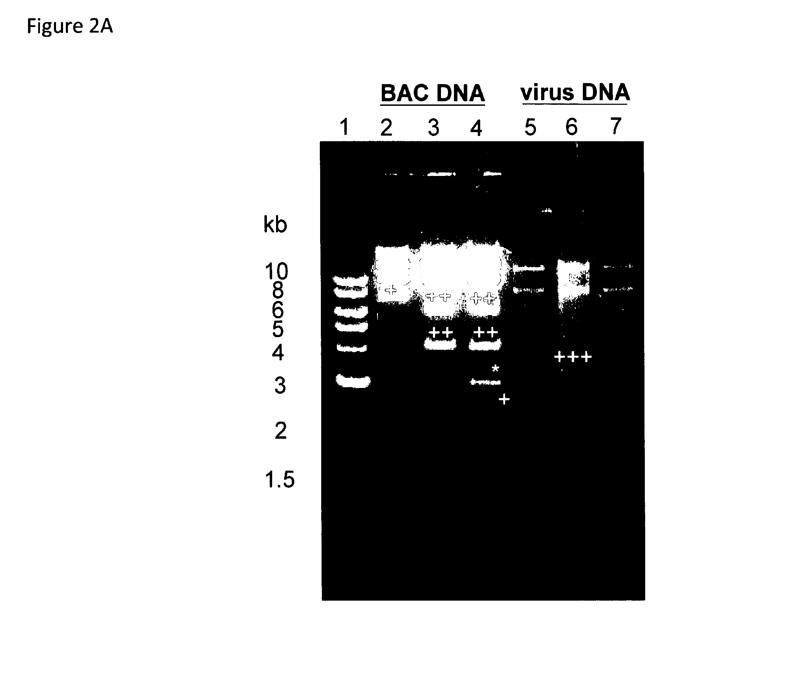

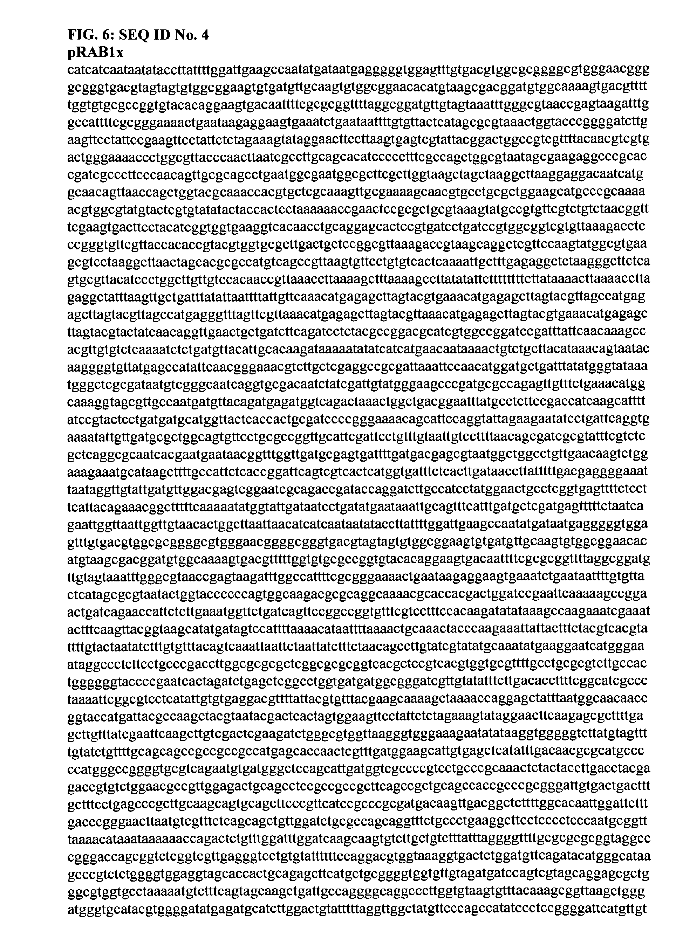

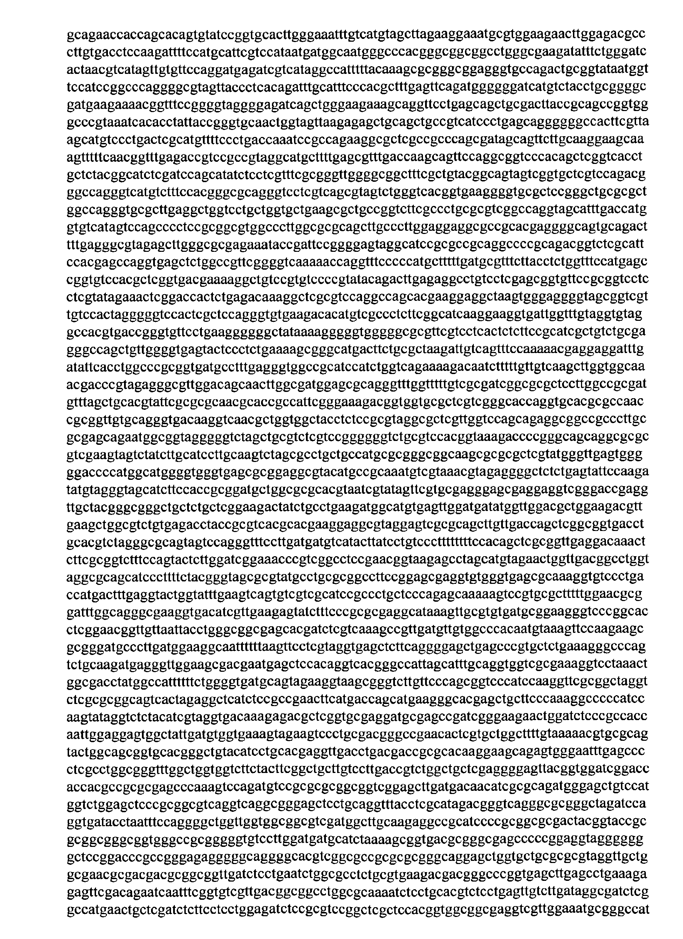

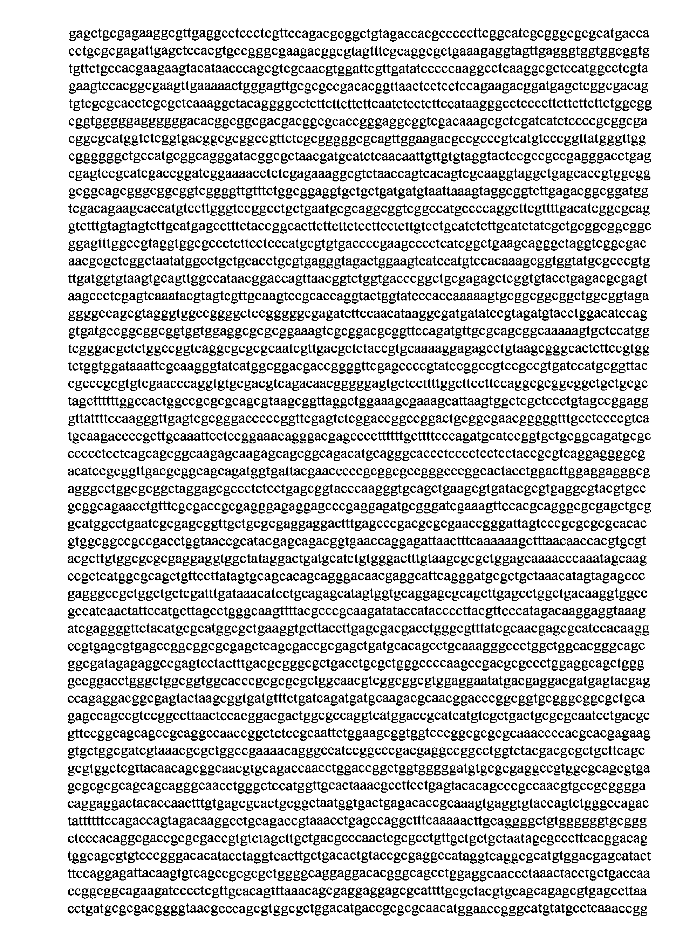

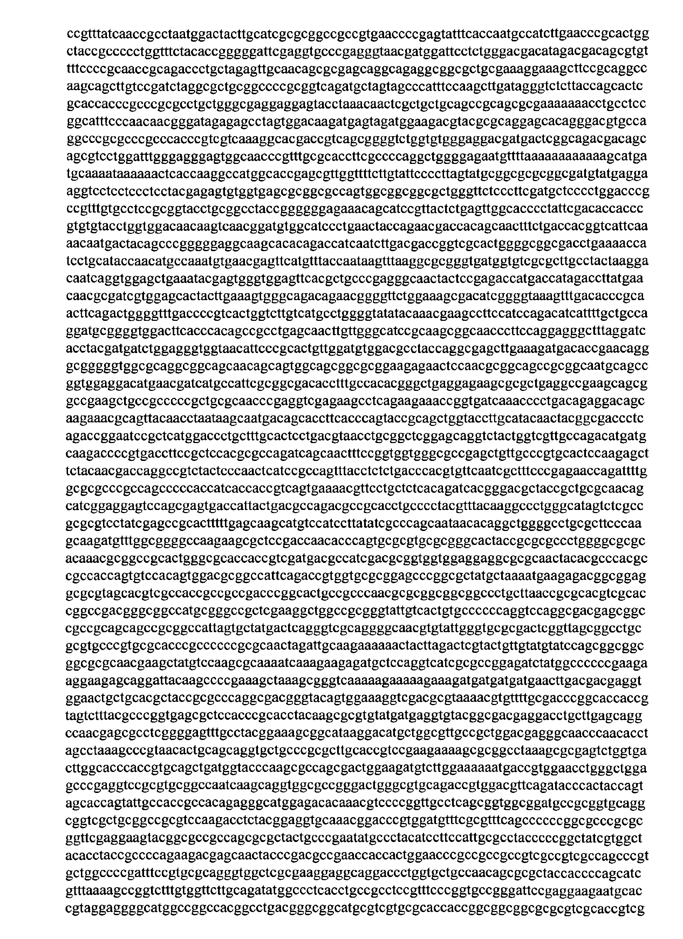

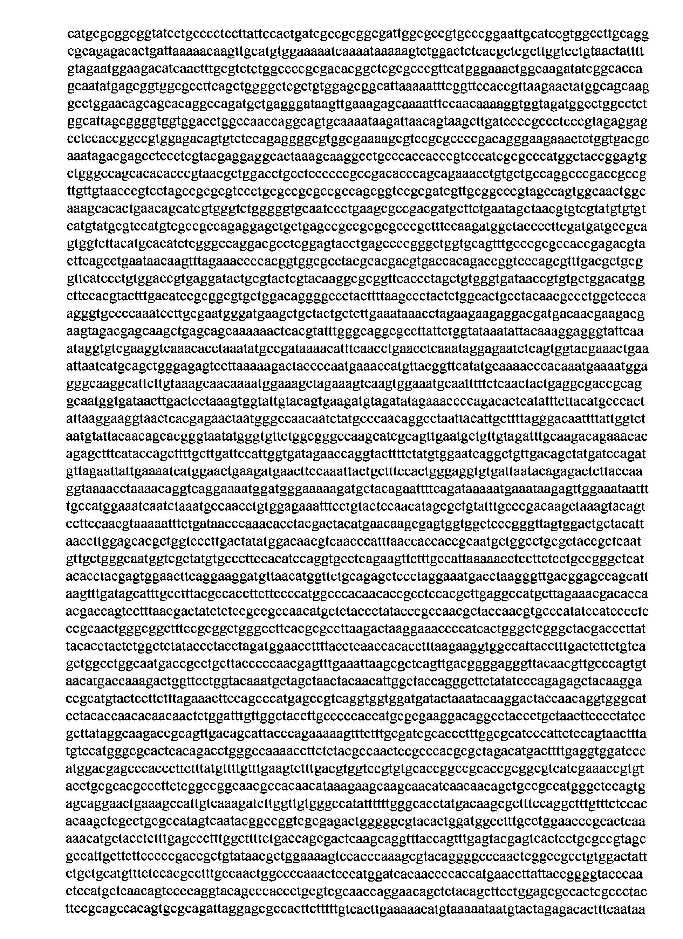

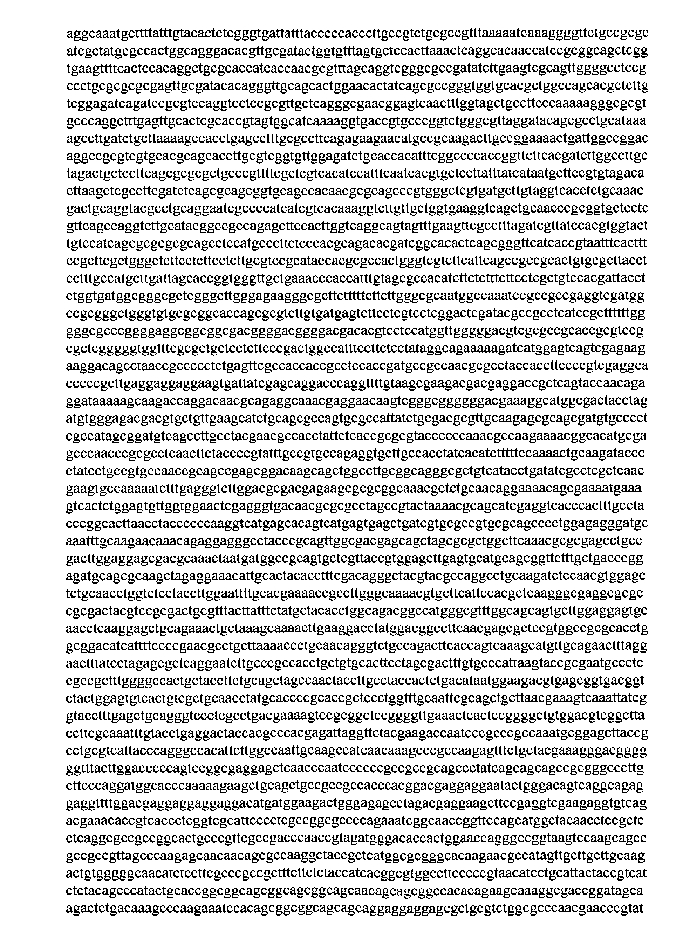

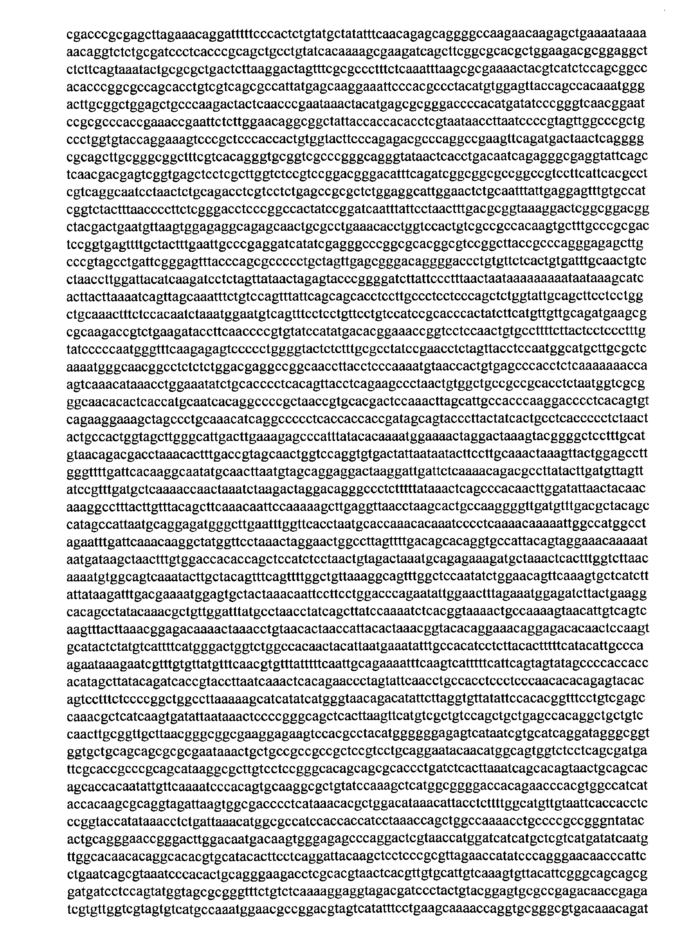

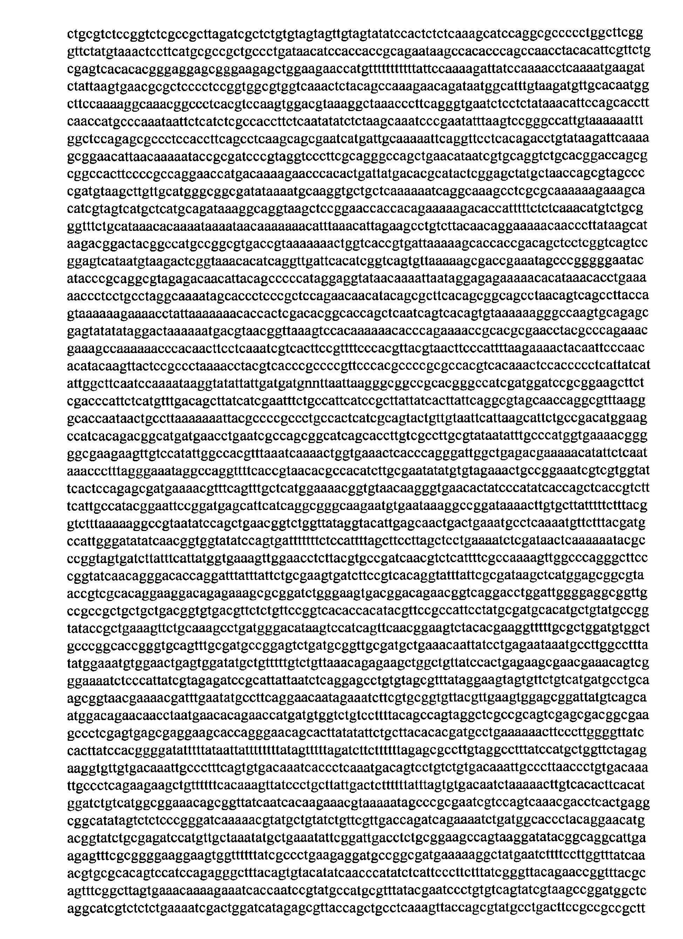

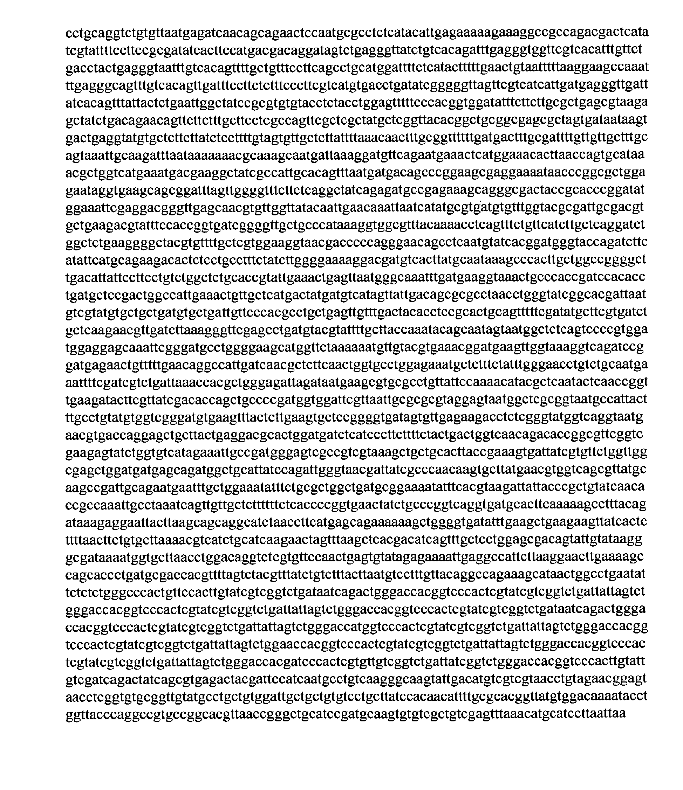

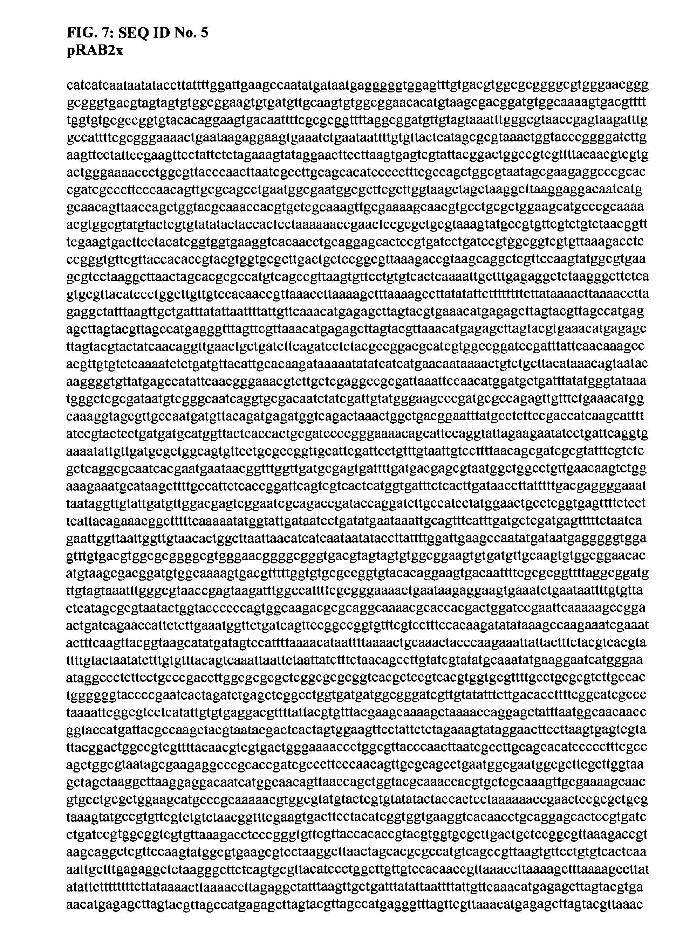

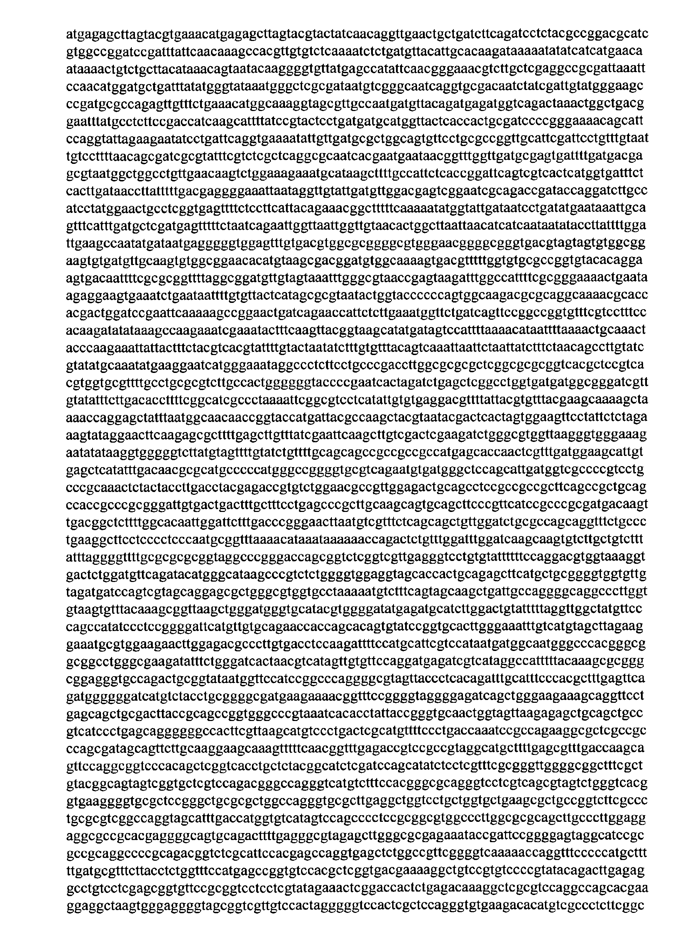

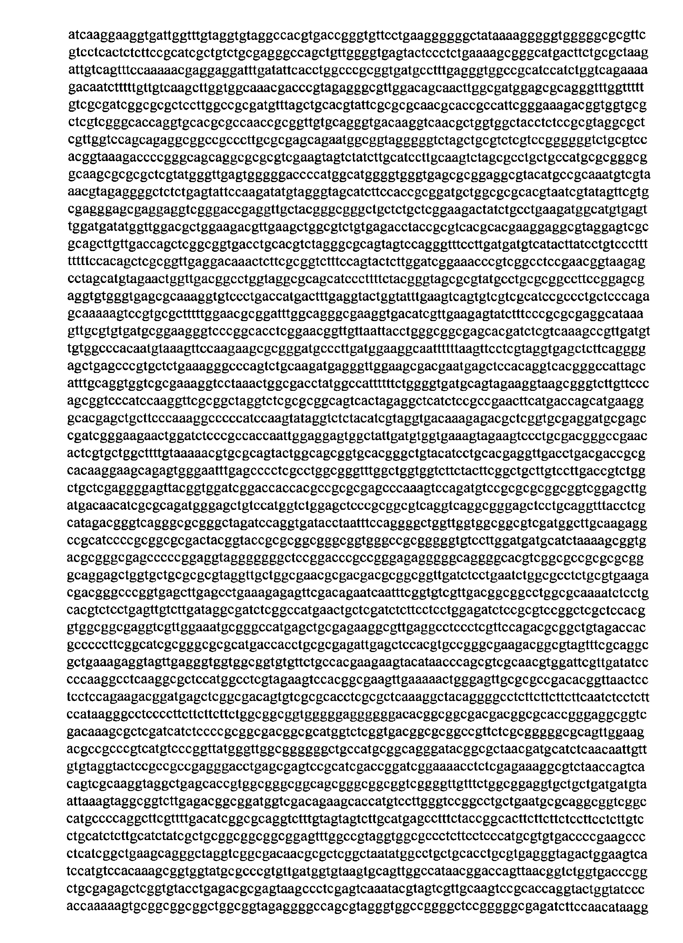

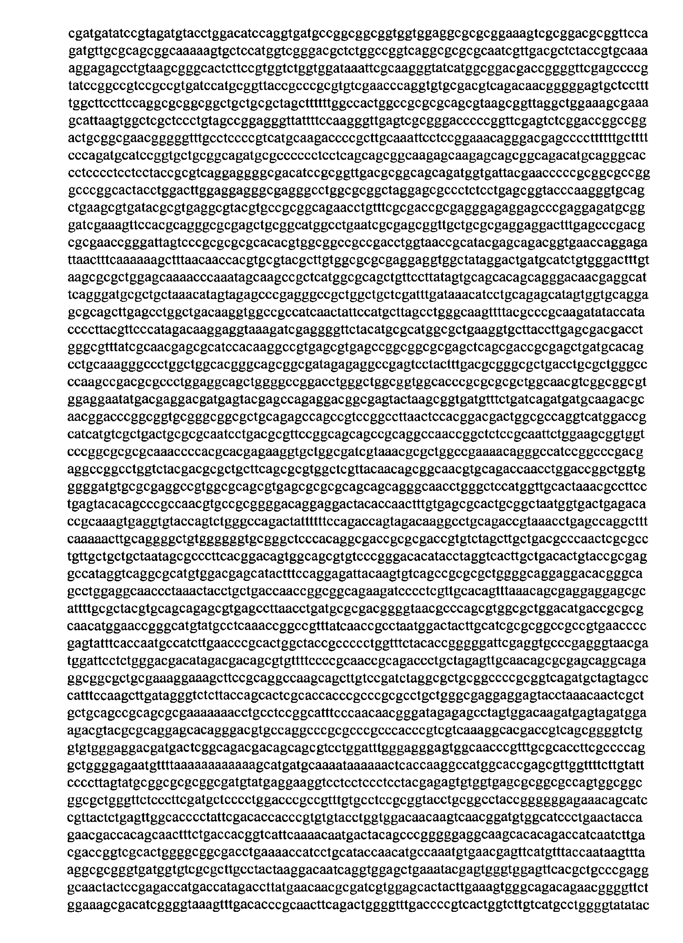

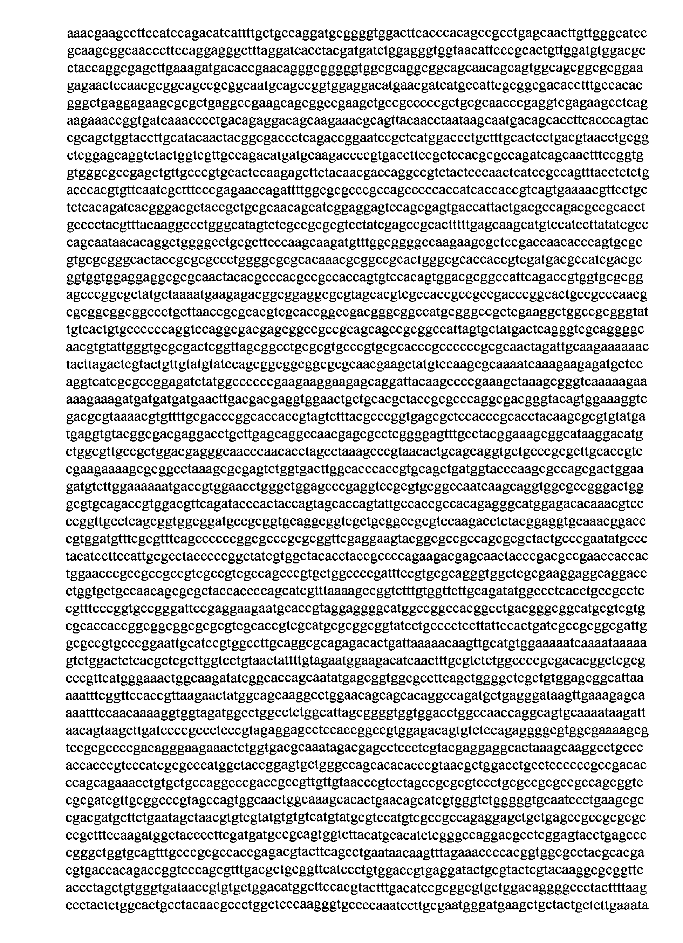

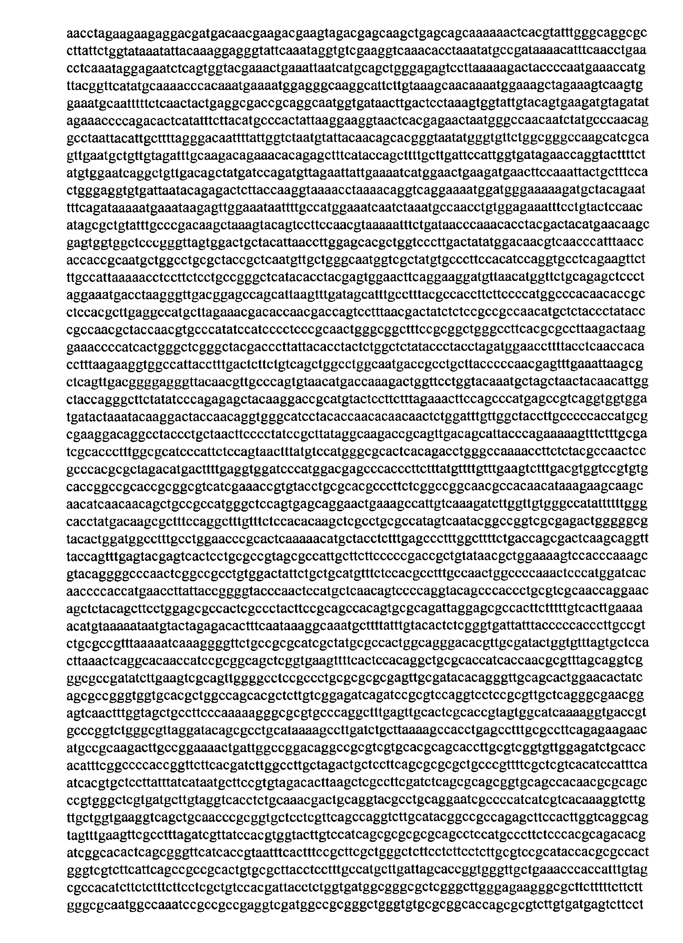

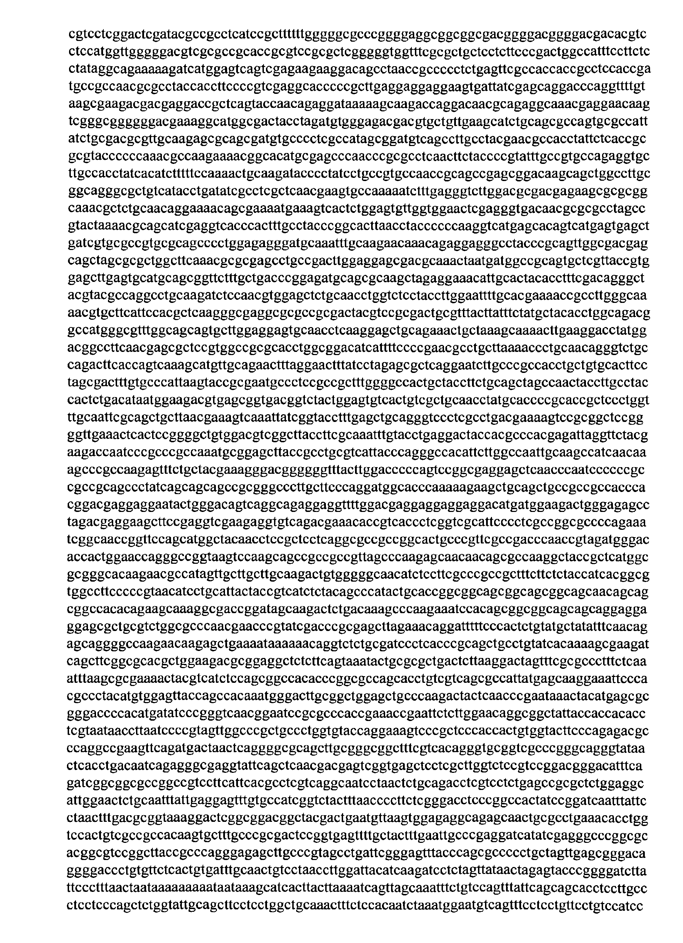

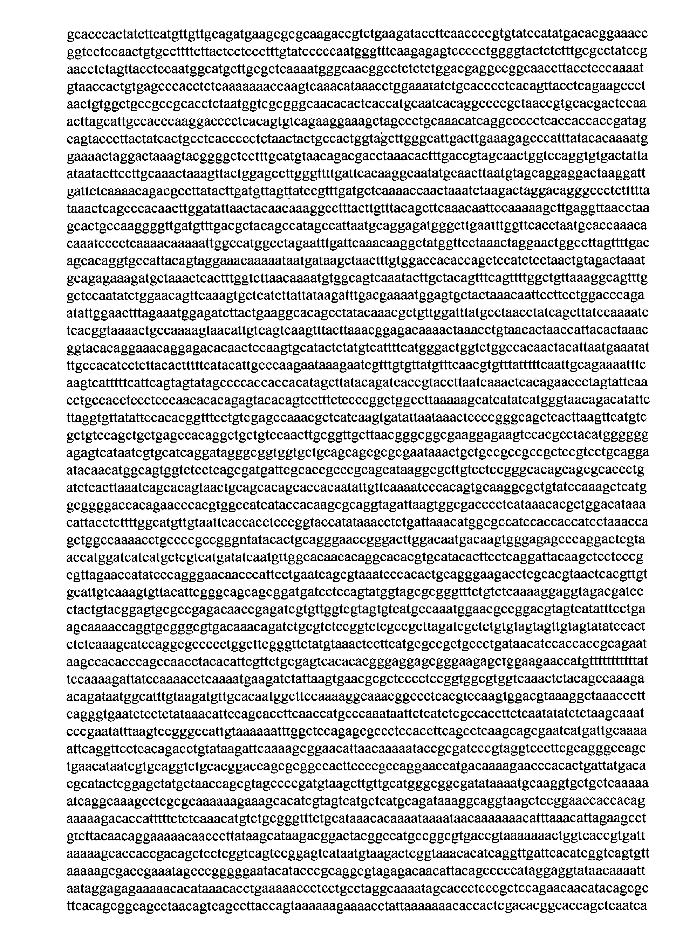

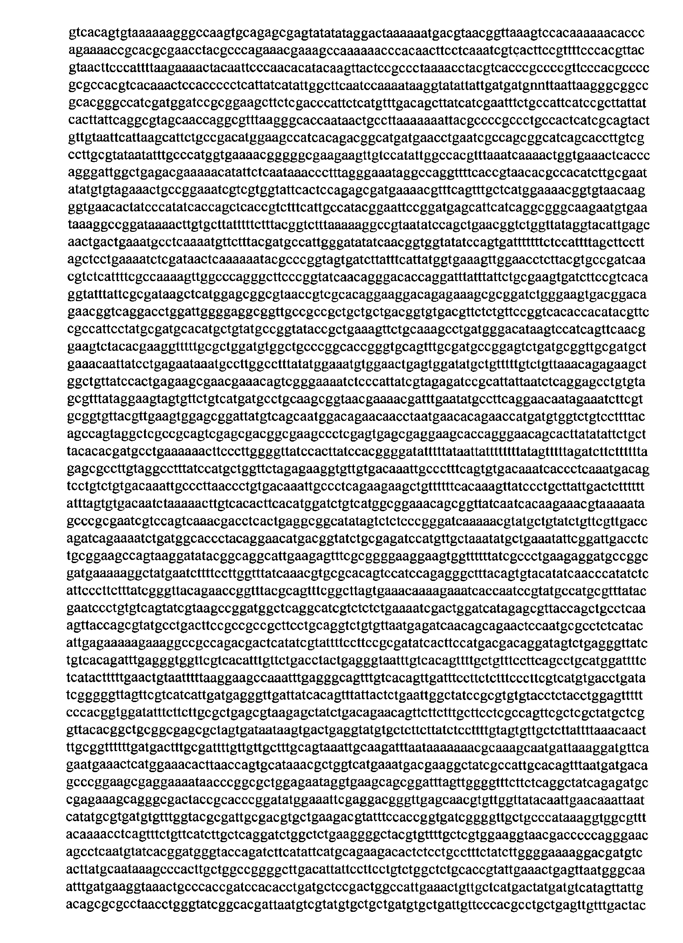

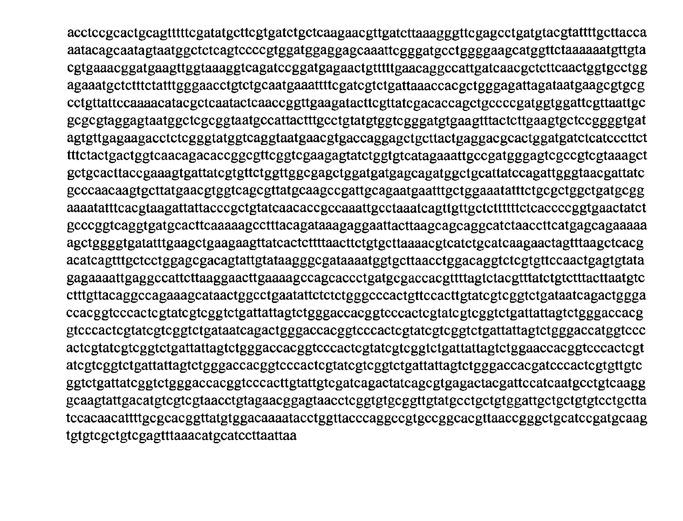

The reaction product, corresponding to a fourth nucleic acid molecule according to the method provided in this invention, results from combination followed by site-specific recombination in an *E.coli* host cell between the nucleic acid molecules pDonorSir1 and a nucleotide acid molecule identical or similar to pBACSir1, respectively. The reaction products obtained were characterized by restriction analysis using XhoI (Figure 2A). Restriction analysis of the once recombined reaction product pRAB1x (FIG. 6 SEQ. ID 4) and the twice recombined reaction product pRAB2x (Fig. 7 SEQ. ID5) is shown in Fig. 2A. The obtained reaction products pRAB1x and pRAB2x contained exactly one copy of a complemented complete human adenovirus type 5 genome, which were released from the reaction products by restriction digest with PacI. The PacI-digested DNA was transfected into 293 cells, and a recombinant adenovirus obtained. DNA from the adenoviruses isolated after productive infection of 293 cells, was analyzed by restriction digest with XhoI (Fig. 2A). A schematic representation of the Flp-mediated recombination reaction and the resulting once and twice recombined reaction products is given in figure 1.

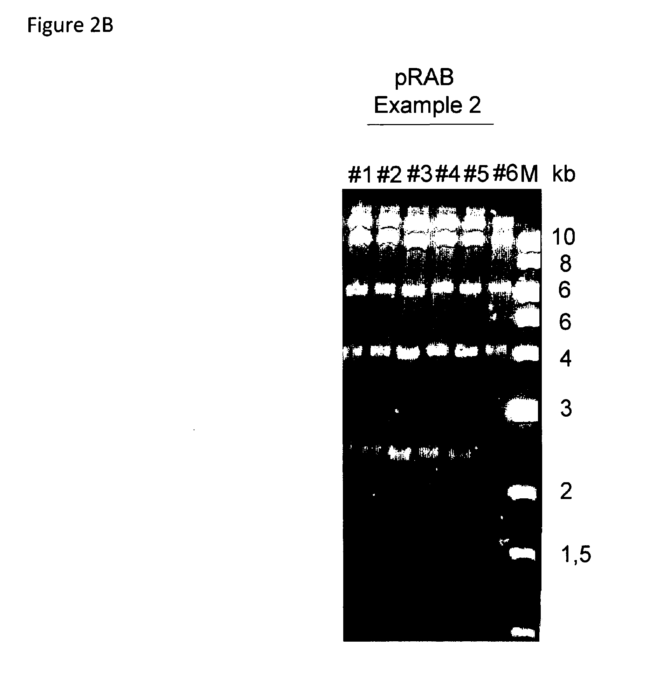

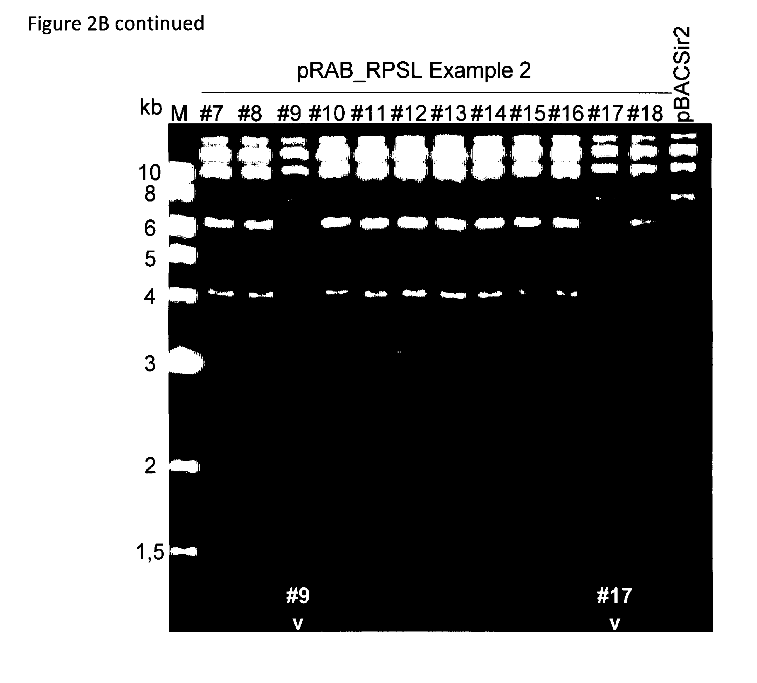

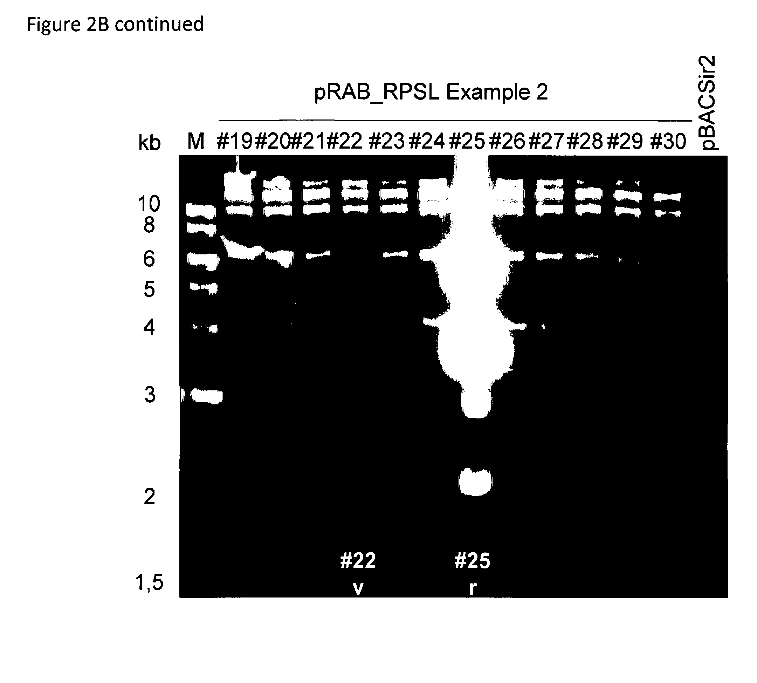

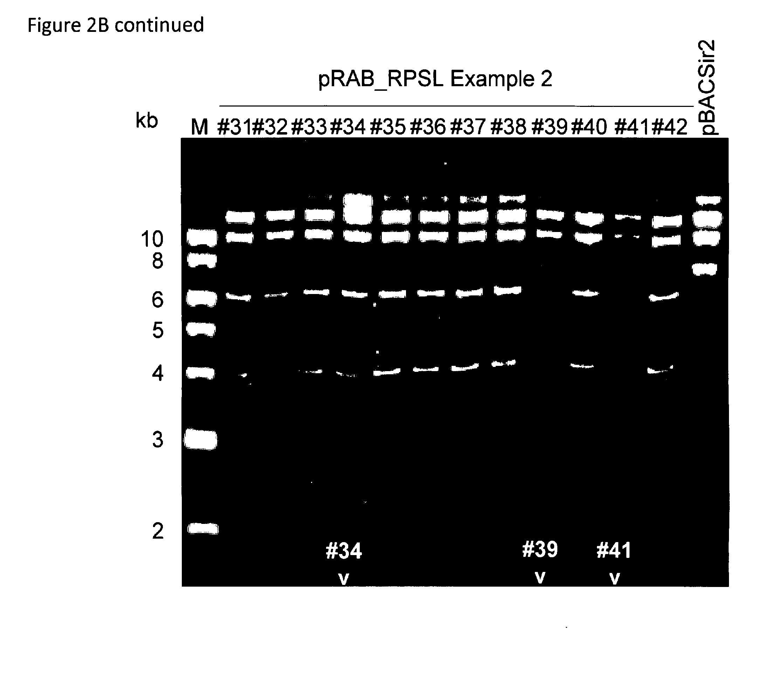

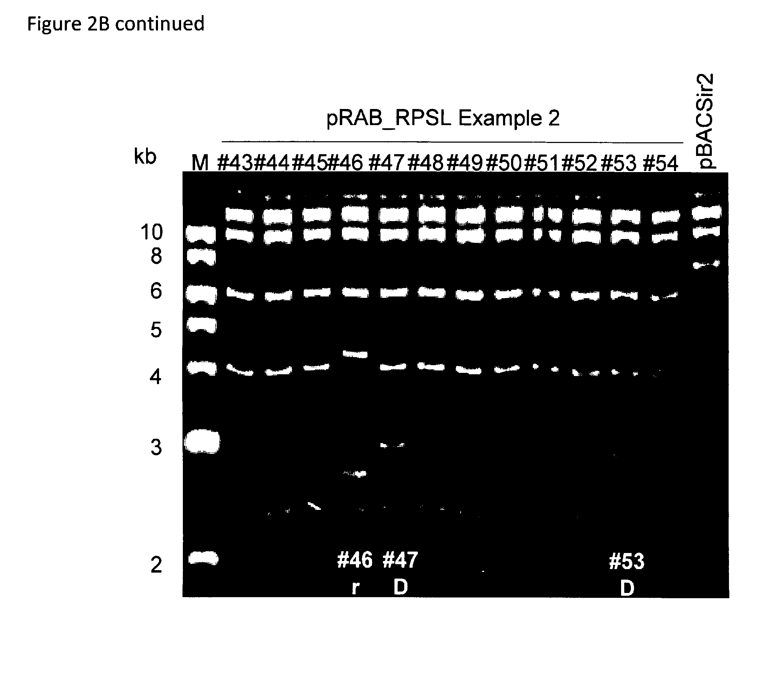

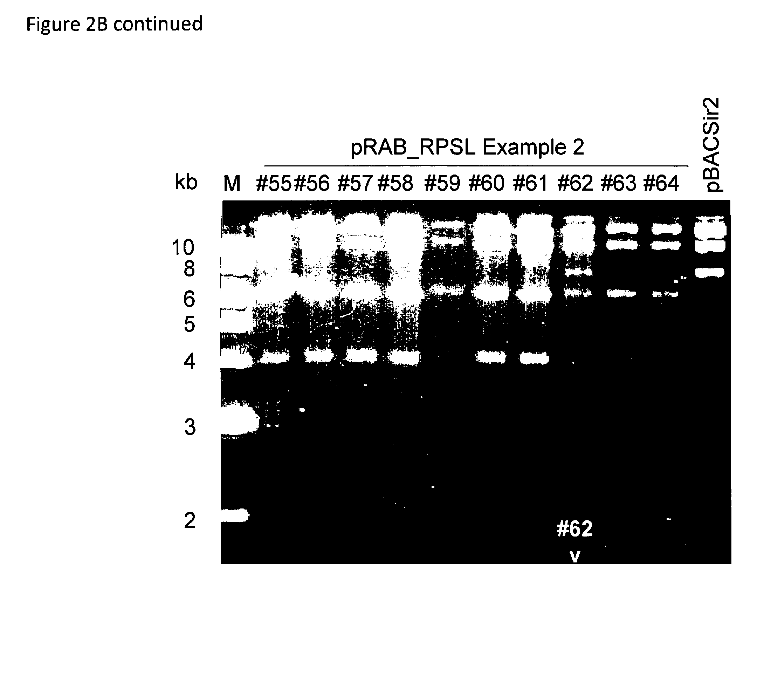

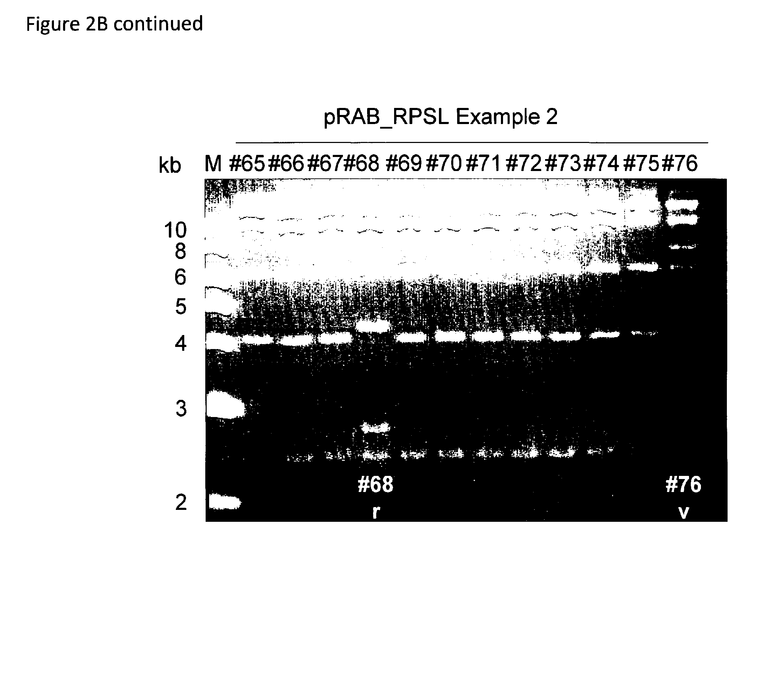

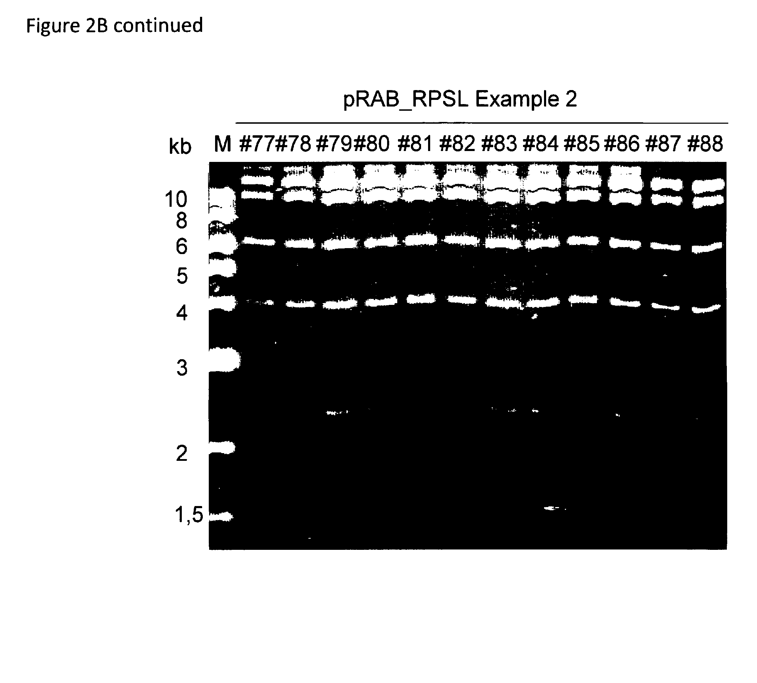

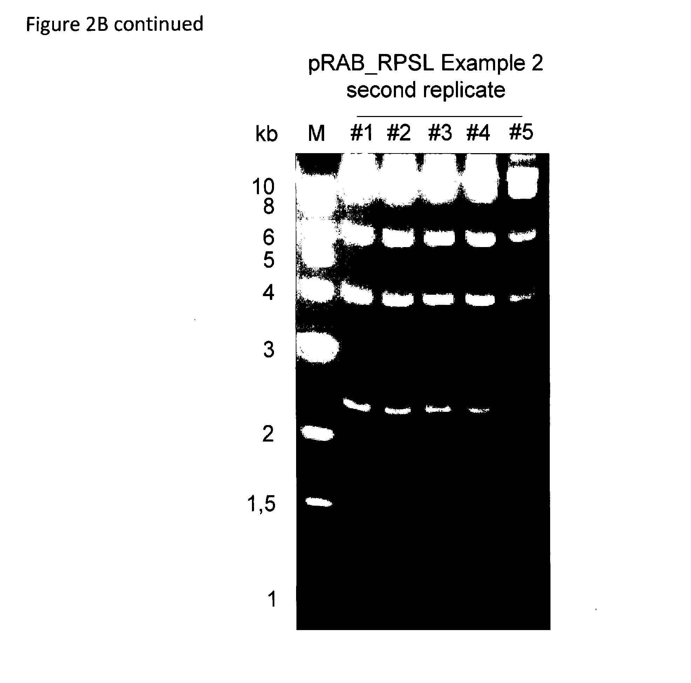

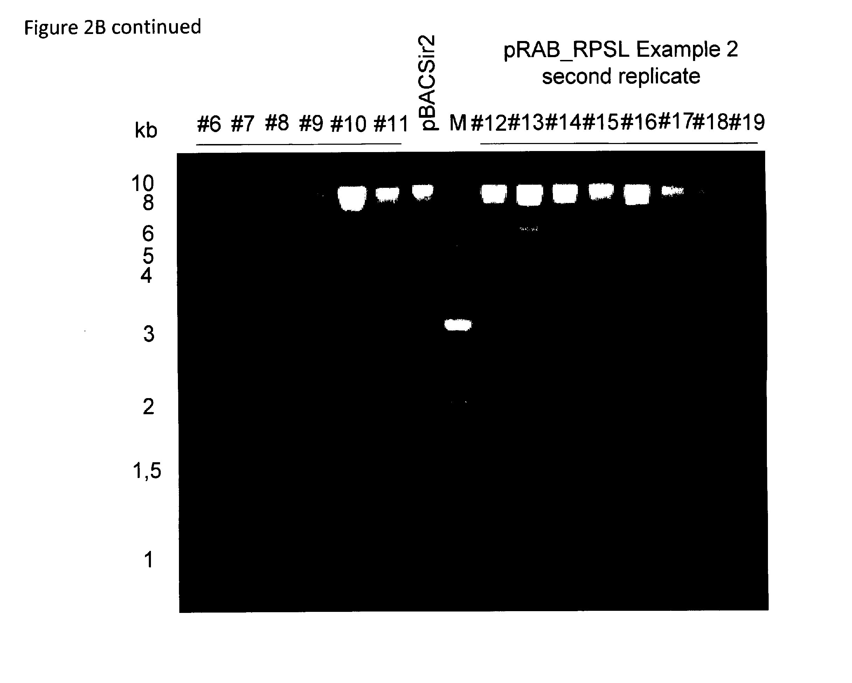

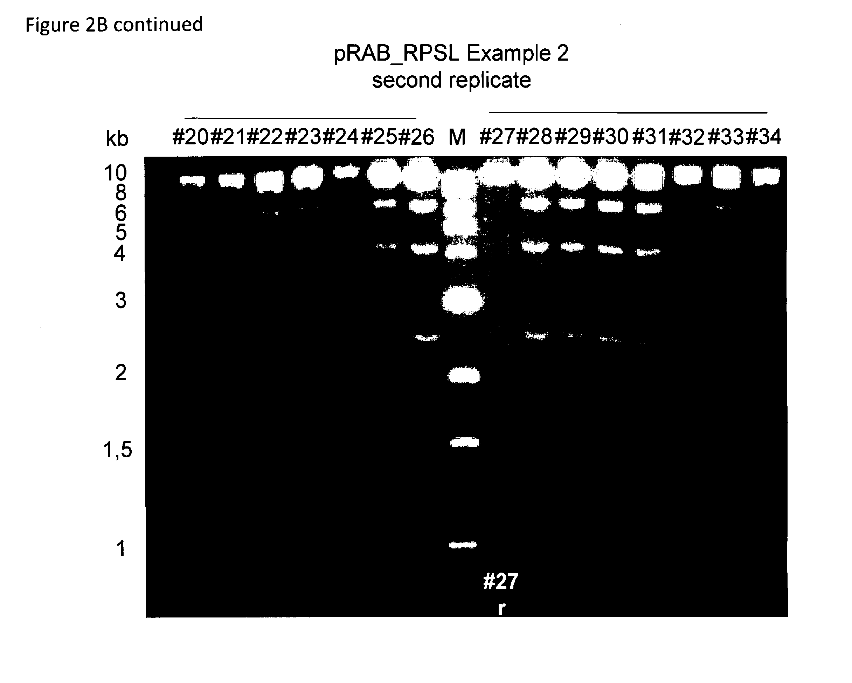

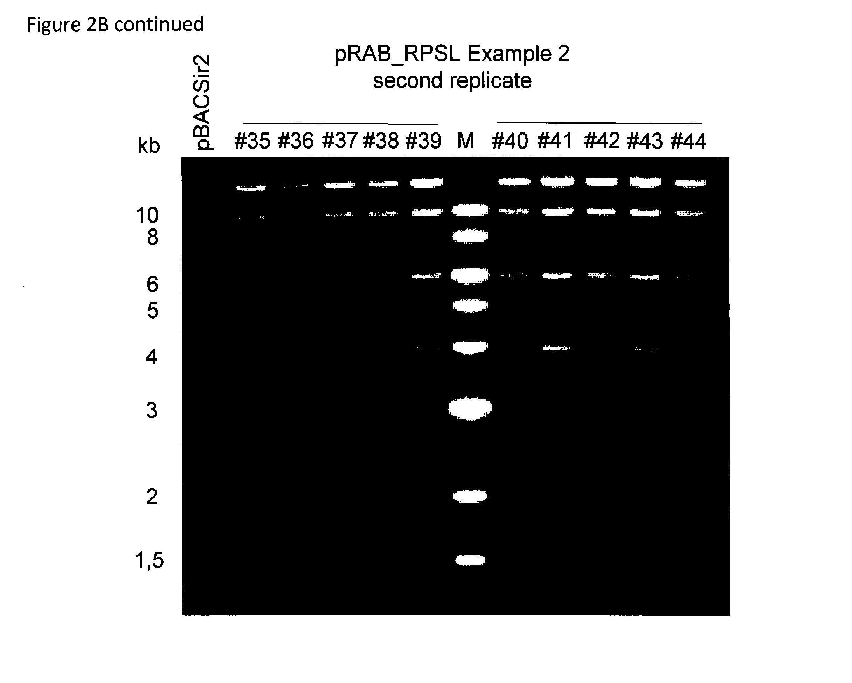

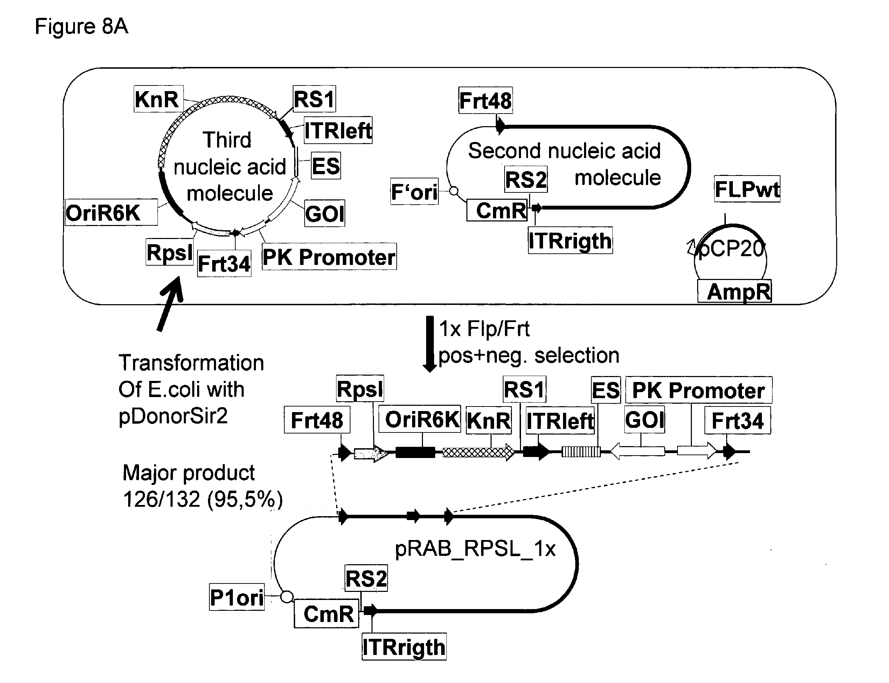

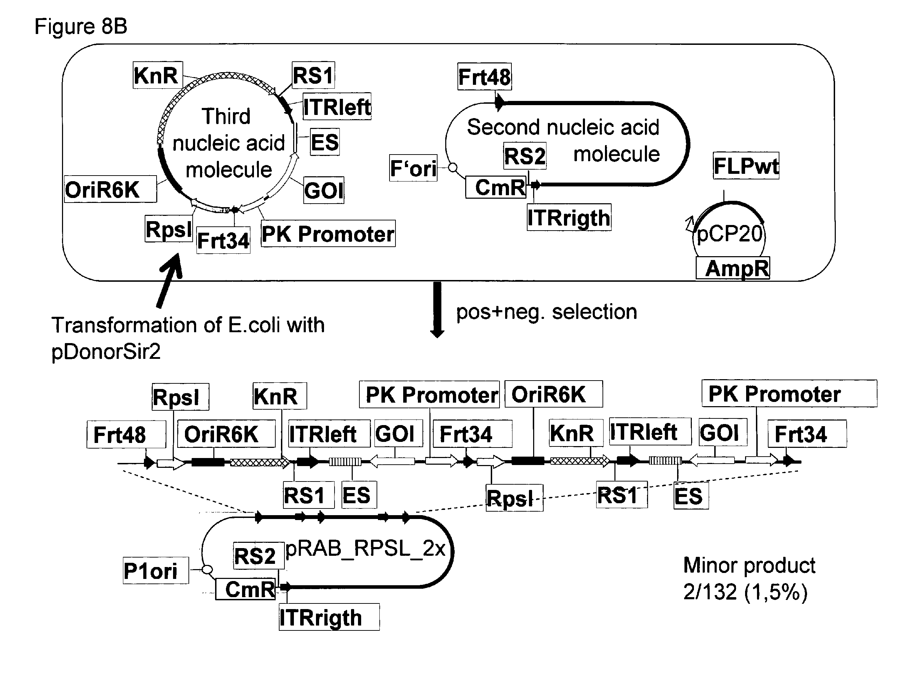

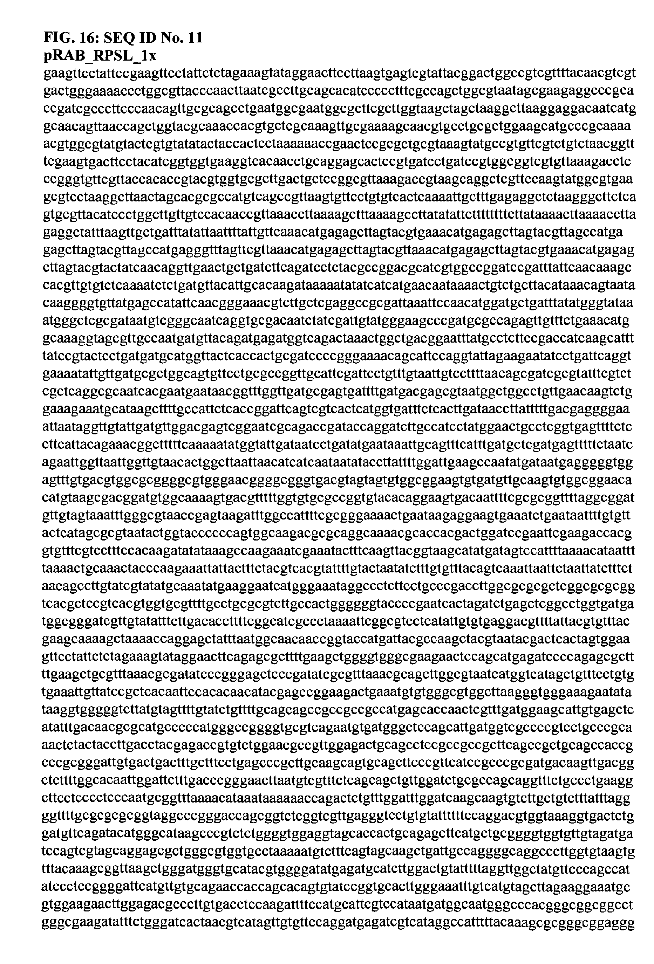

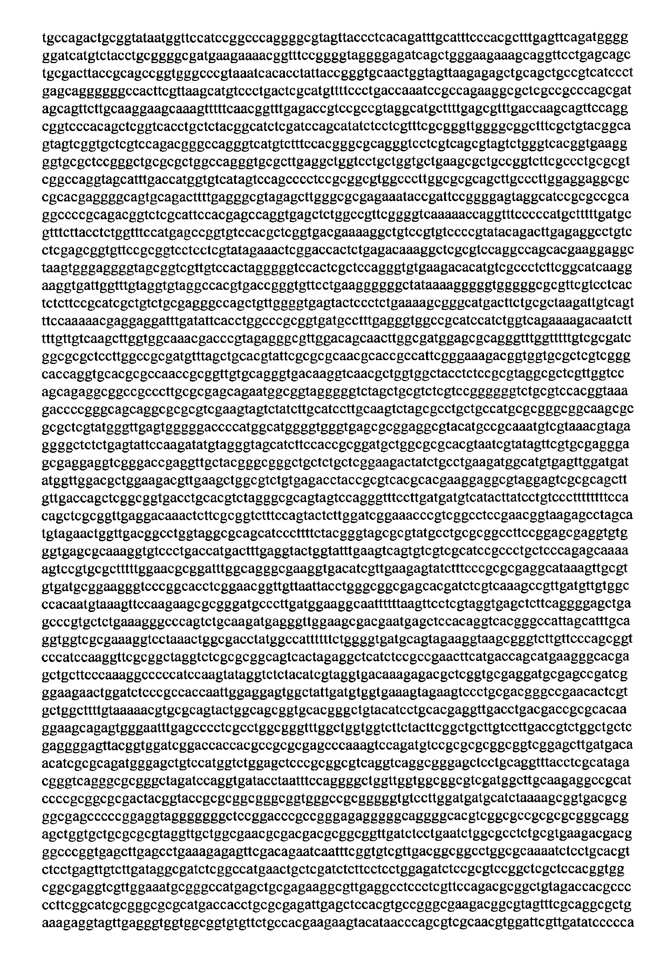

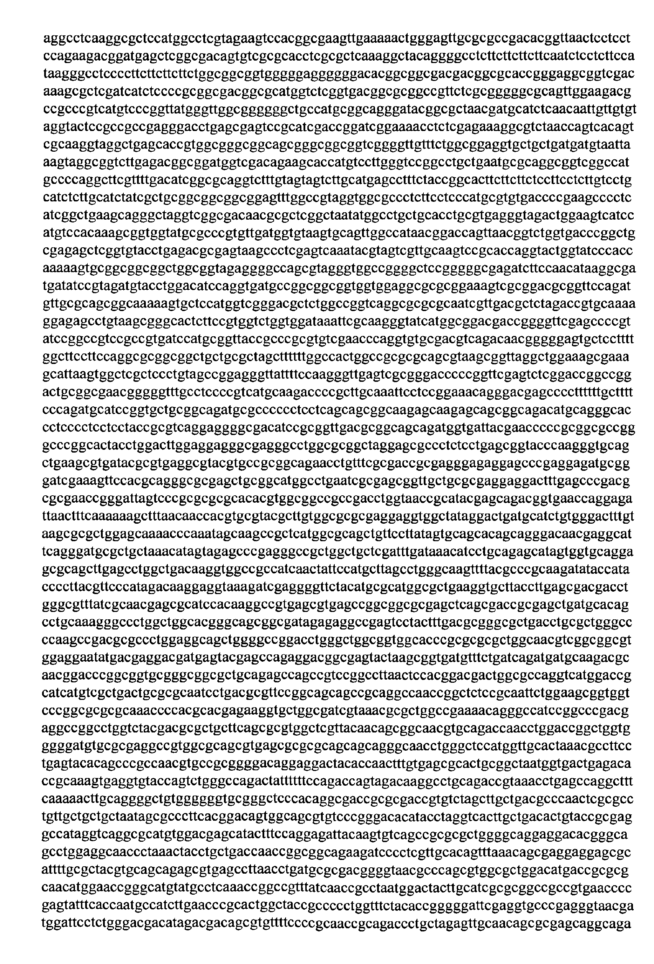

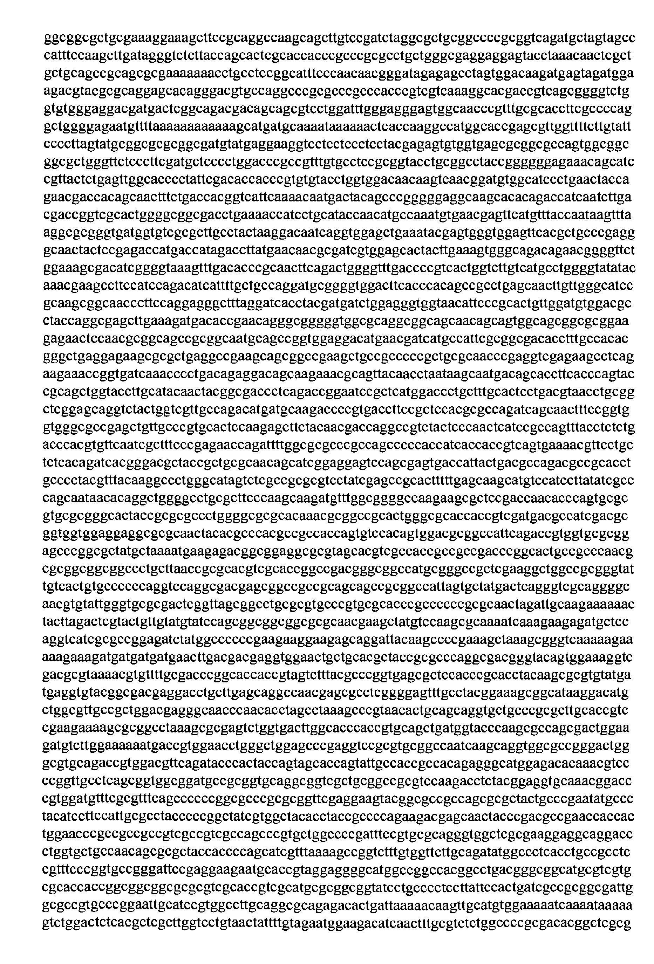

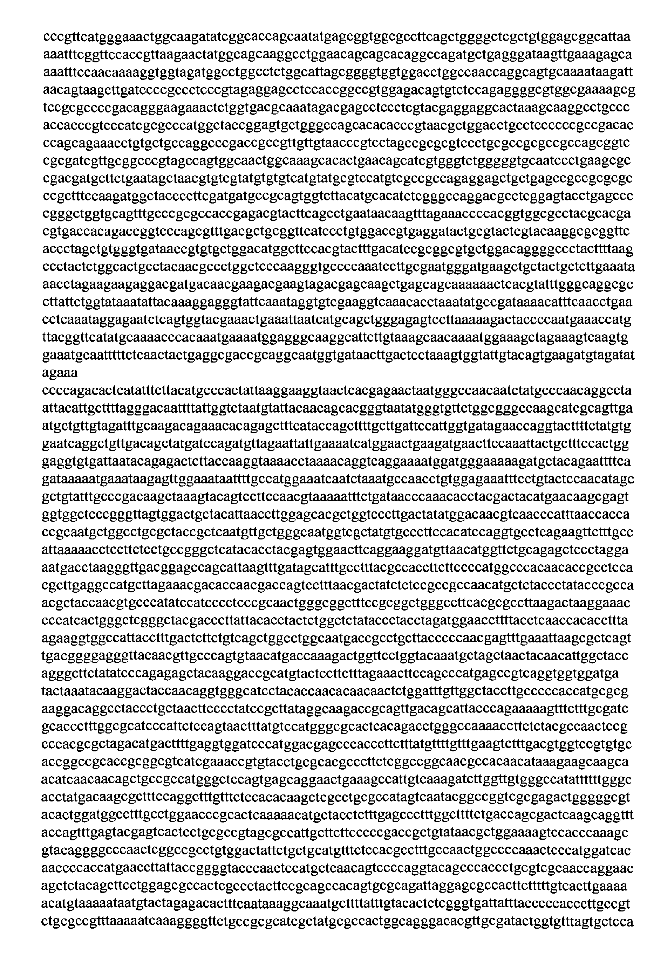

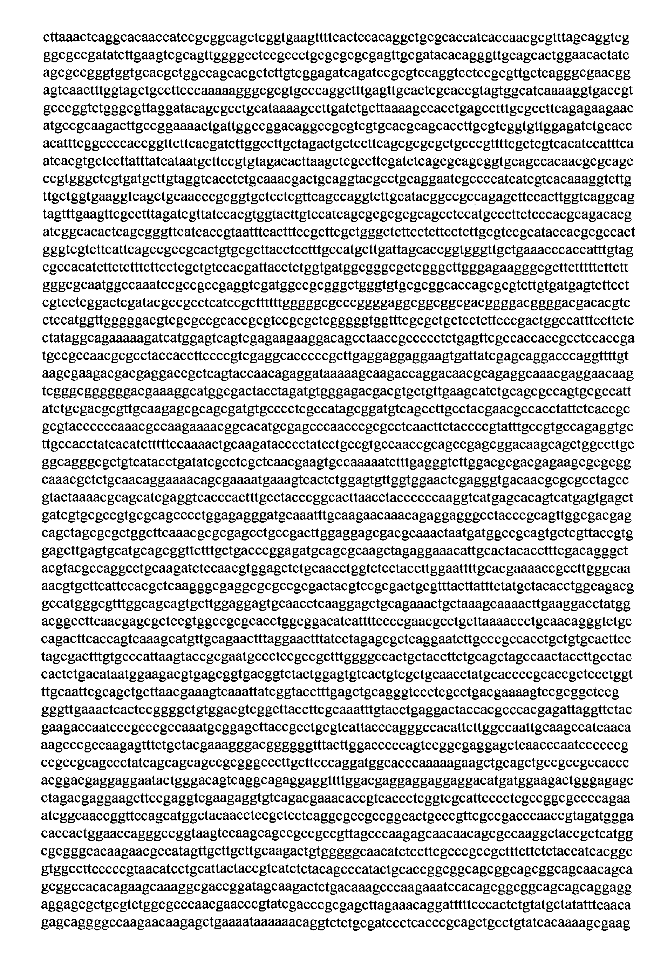

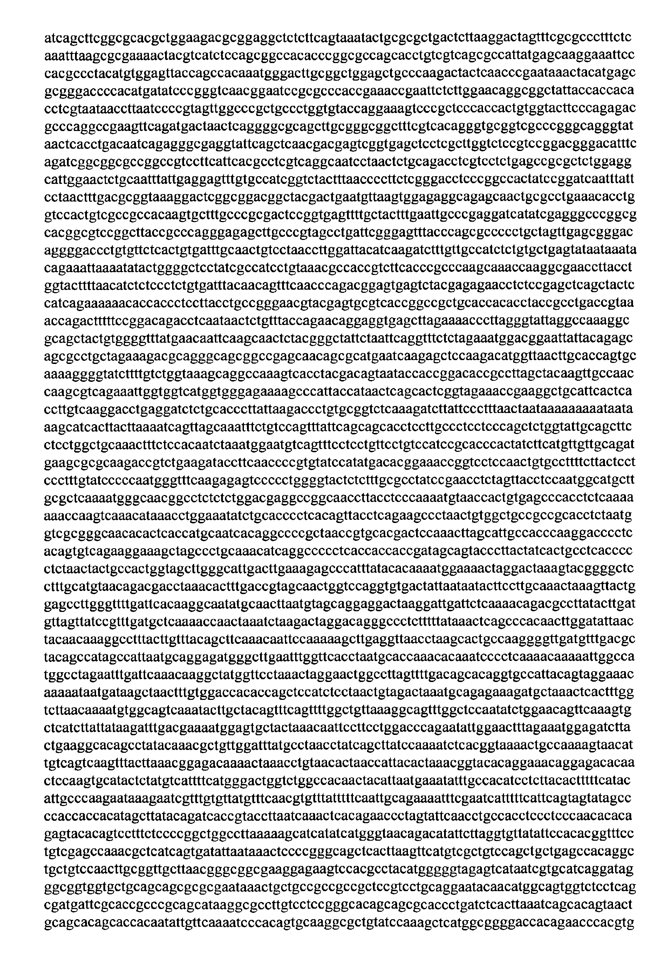

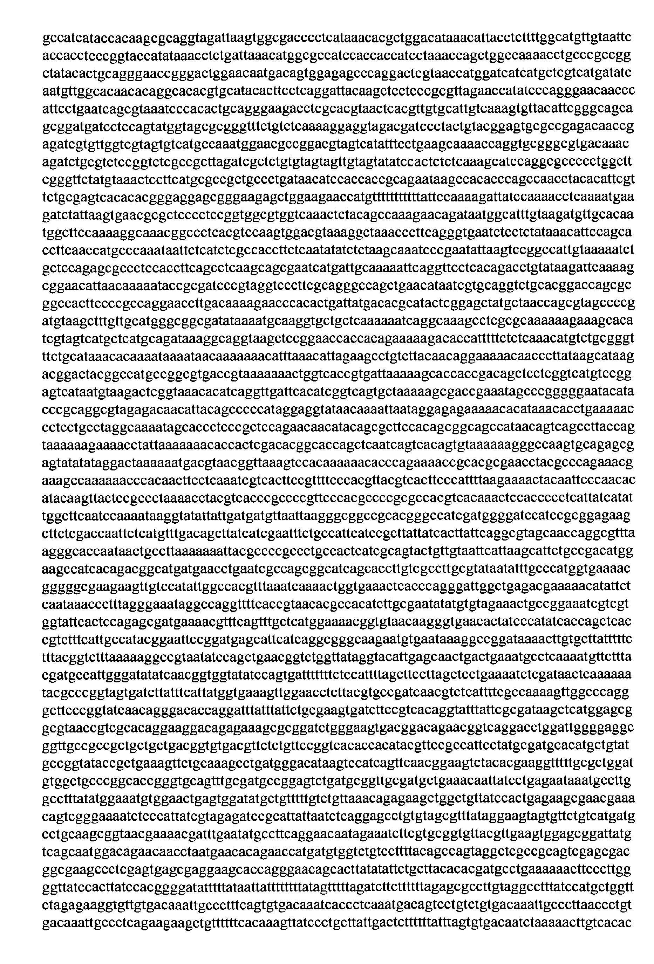

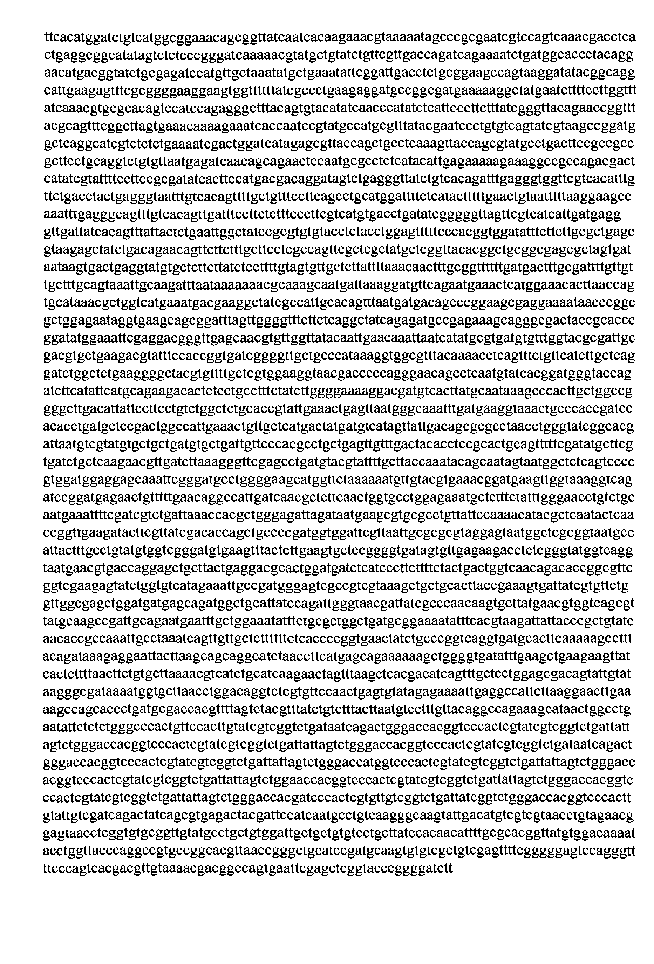

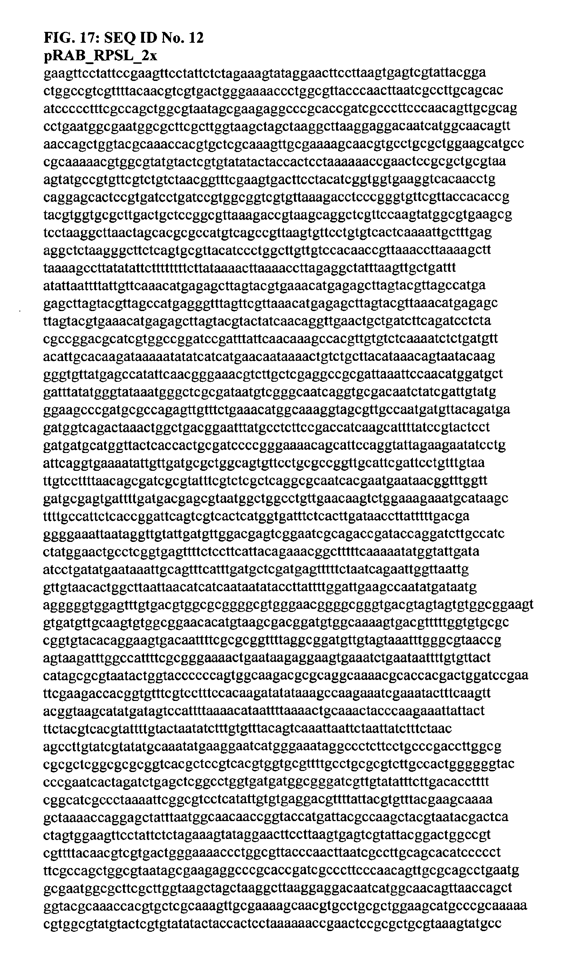

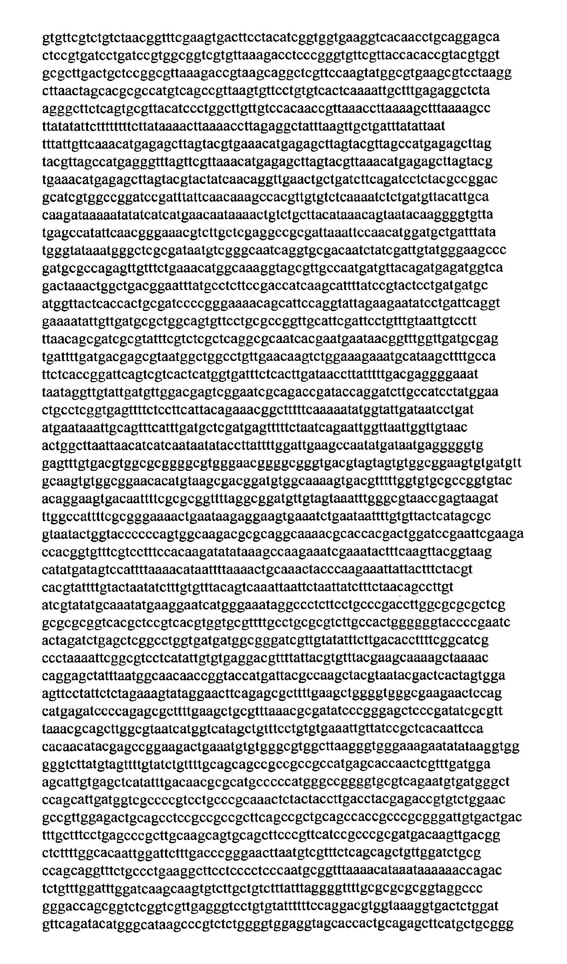

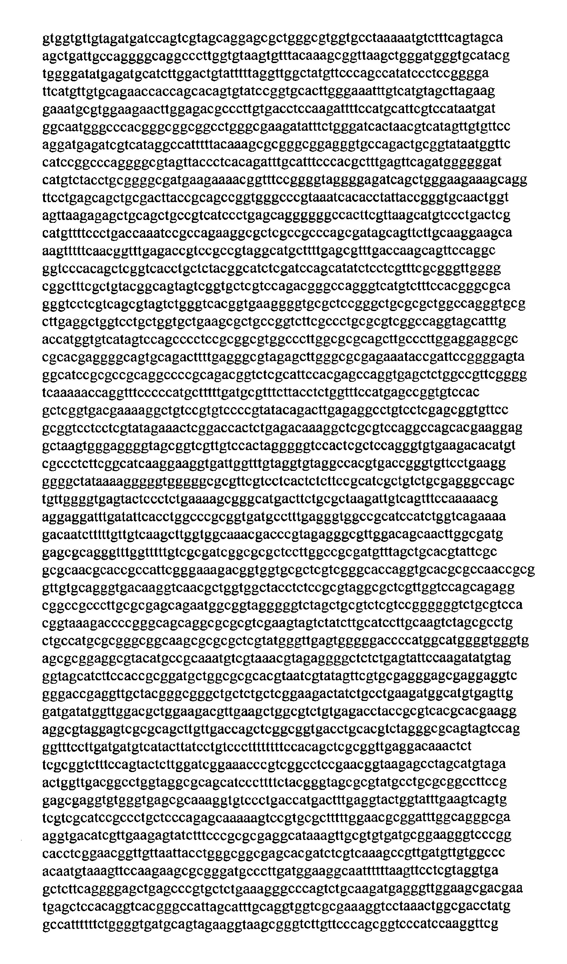

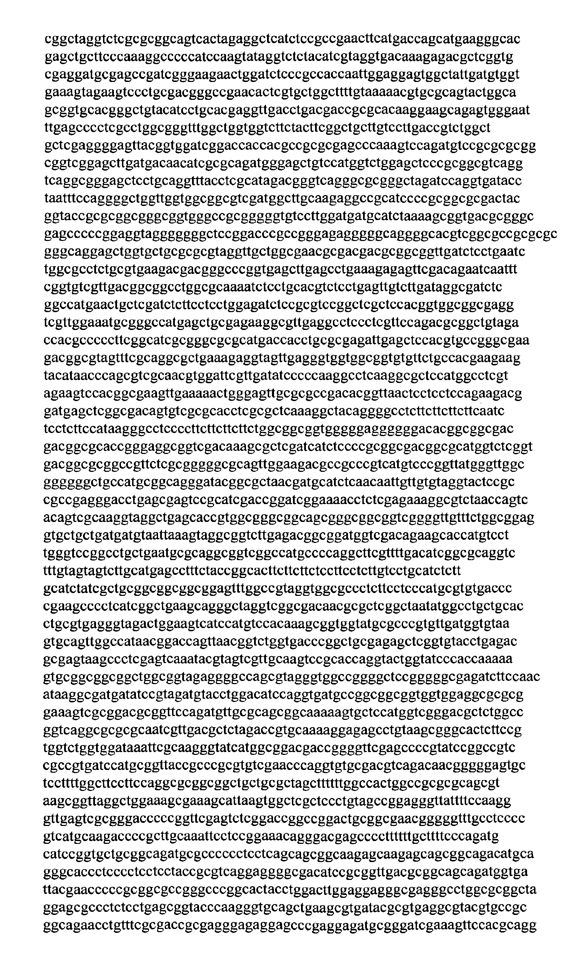

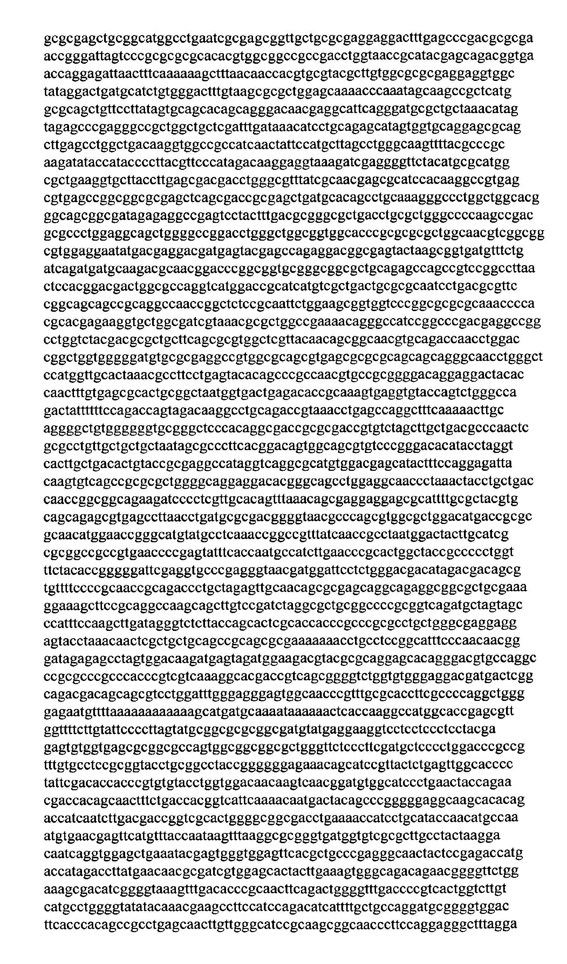

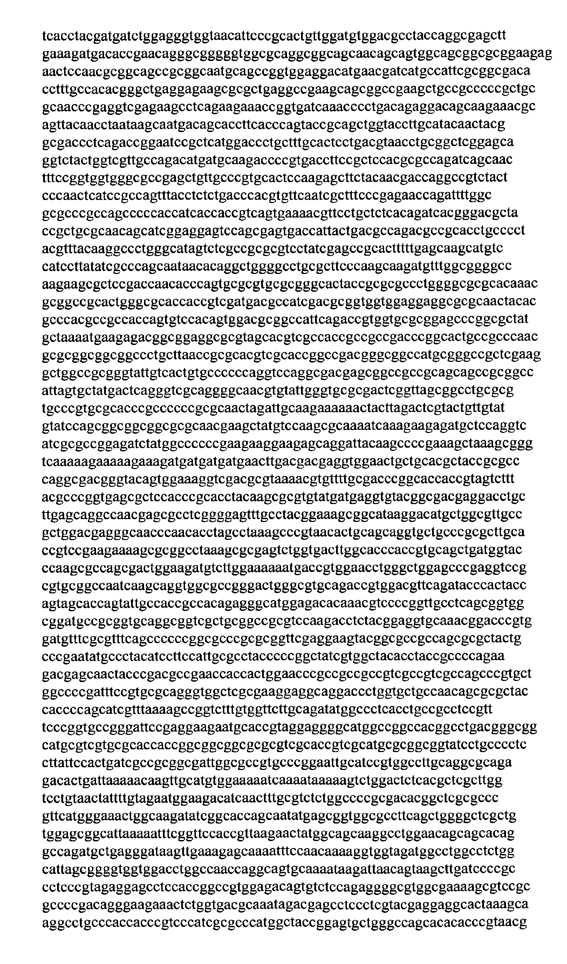

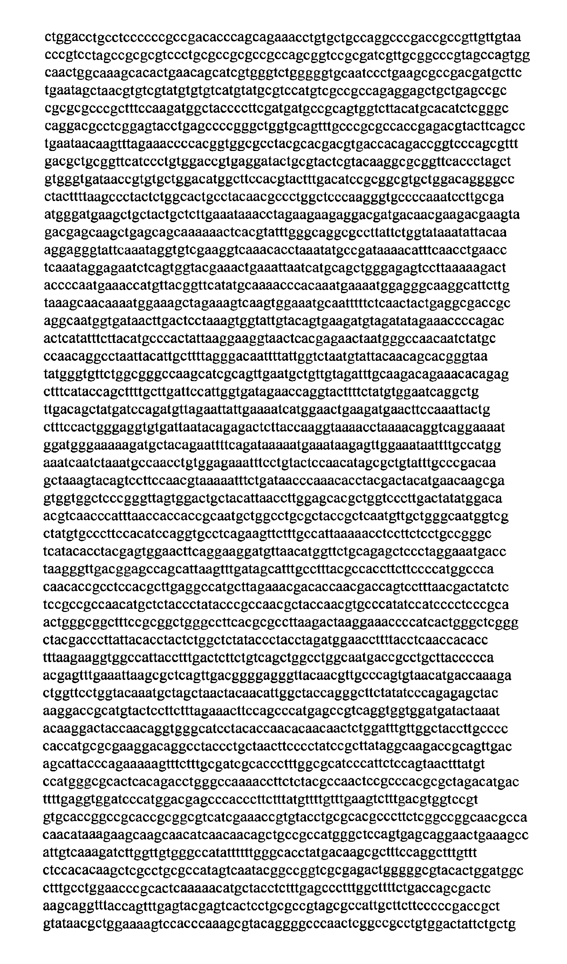

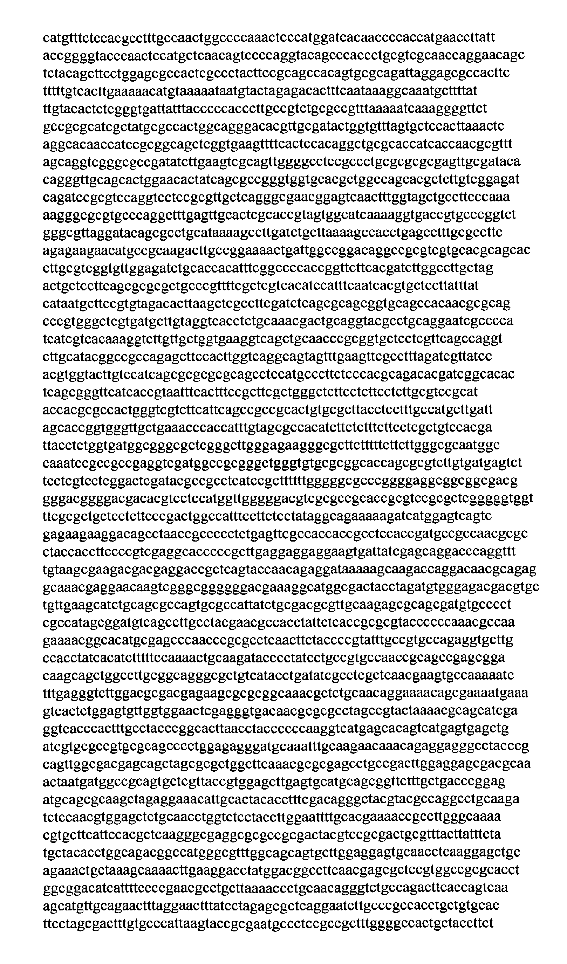

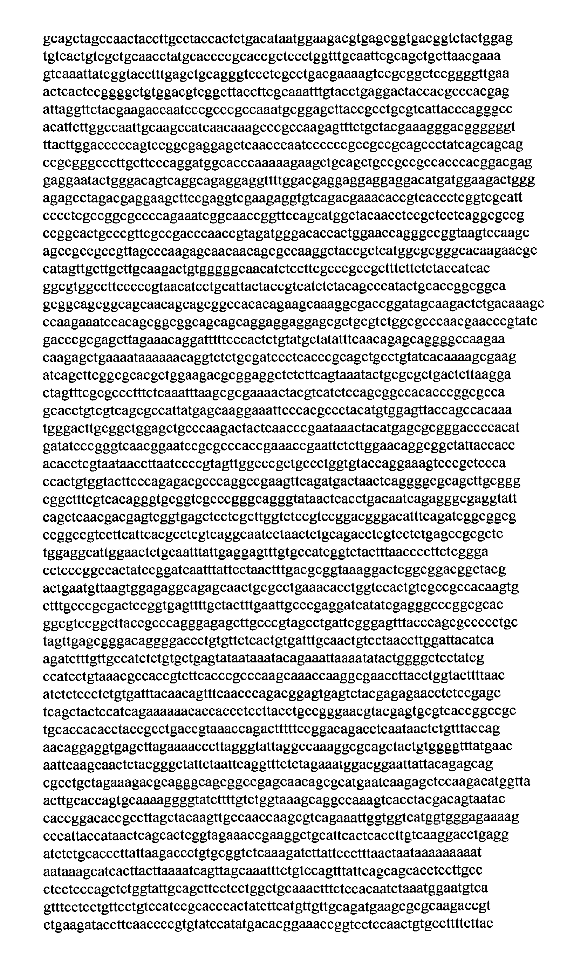

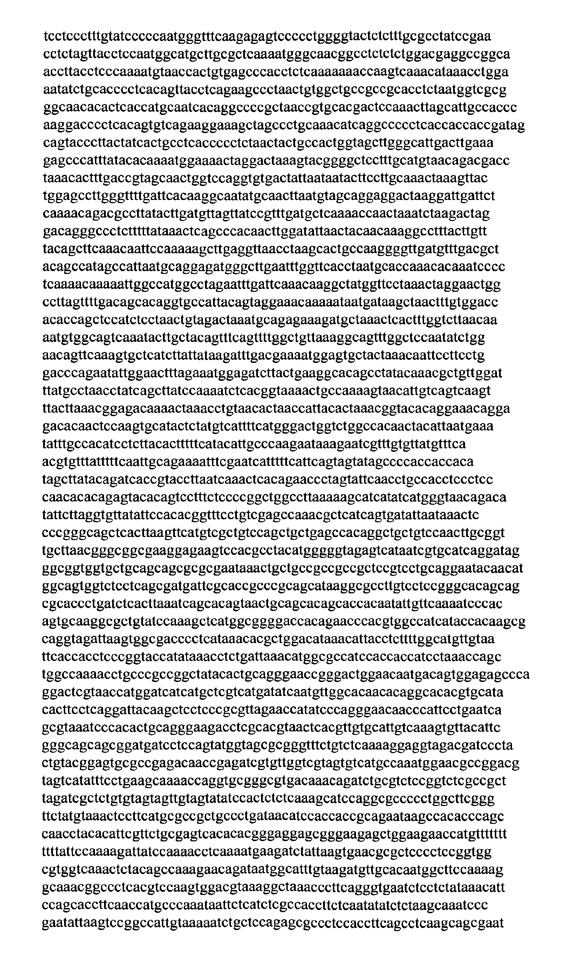

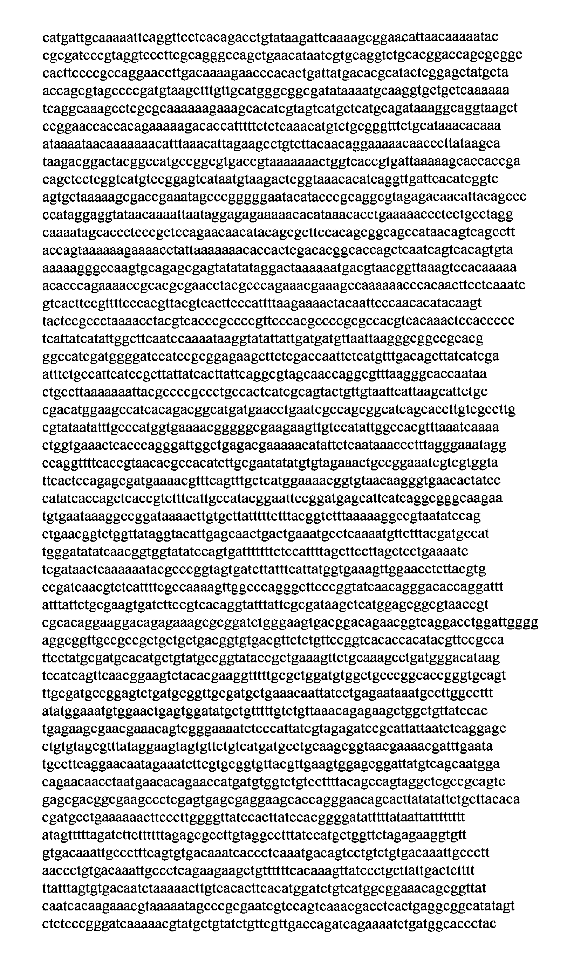

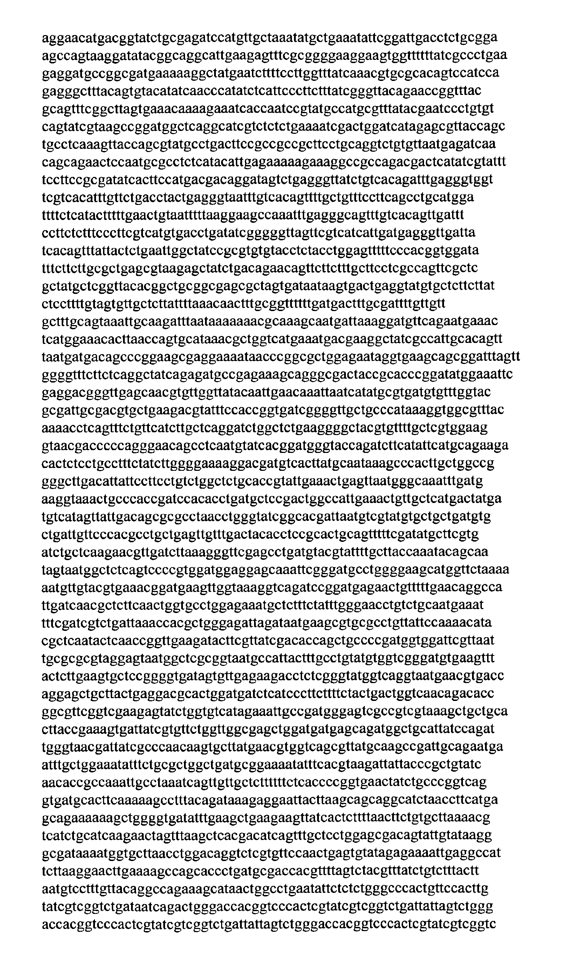

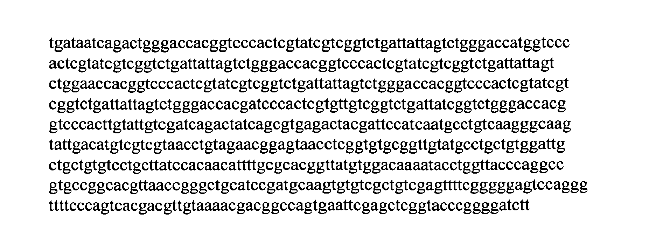

A plurality of reaction products, corresponding each to a fifth nucleic acid molecule according to the method provided in this invention, resulting from a combination and site-specific recombination in an *E.coli* host cell of pDonorSir2 and pBACSir2 were characterized by restriction analysis using XhoI (Figure 2B). Restriction analysis with *Xho*I of the once recombined reaction product pRAB_RPSL_1 (Fig. 16 SEQ. ID 11) and the twice recombined reaction product pRAB_RPSL_2x (Fig. 17 SEQ. ID 12) is shown in figure 2B. The majority (83/88) of the obtained reaction products resulted from single recombination between pDonorSir2 and pBACSir2 (Fig.2B). The restriction digest pattern of (2/88) recombination products corresponded to the double recombined product pRAB_RPSL_2x. A schematic representation of the reaction scheme and the reaction products is given in figure 8. In the single recombined product pRAB_RPSL_1x the regulatory nucleic acid sequence which has regulatory activity in a prokaryote, here the E.*coli* galaktokinase promoter, and the negative selection marker, here the RPSL gene, are functionally separated from each other, whereas, surprisingly, for the double recombined product pRAB_RPSL_2x the negative selection marker was functional, though both elements were separated by a Frt site.

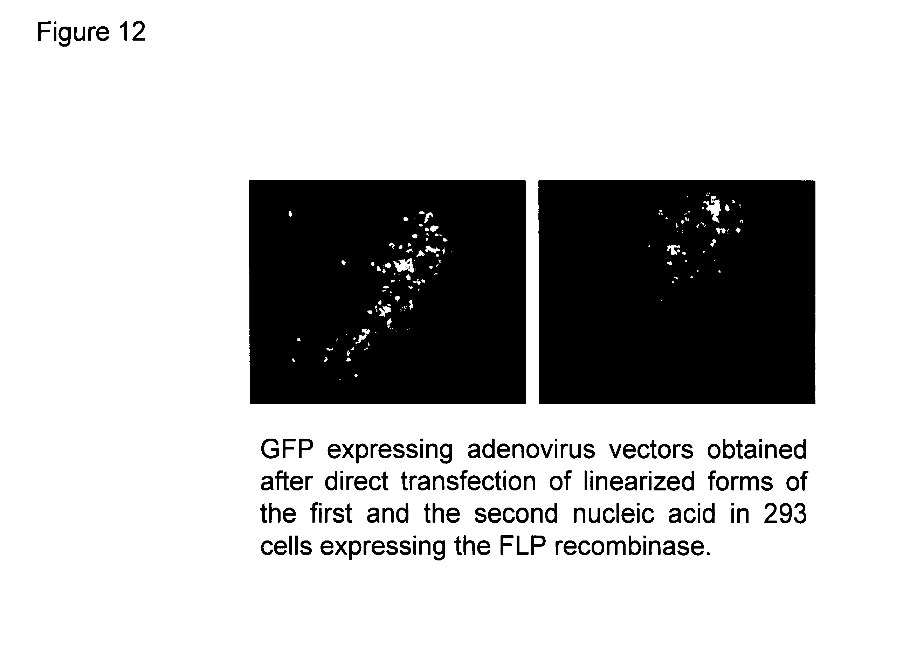

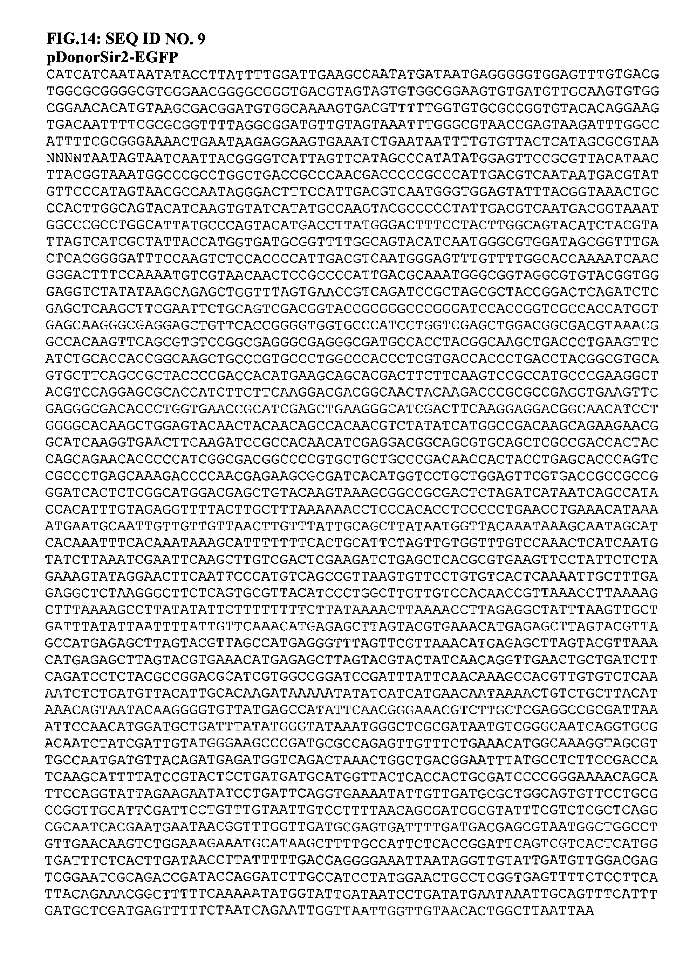

In another example the site-specific recombination mediated by Flp recombinase between the first nucleic acid molecule and the second nucleic acid molecule was done in a eukaryotic host cell providing the site-specific recombinase. The nucleic acid molecules pDonorSirl-EGFP (Fig.14 Seq. ID No.9) and pBACSir2 were digested with an enzyme recognizing the first and second restriction site, respectively, and the linearized nucleic acid molecules transfected into 293 cells stably expressing the Flp recombinase. The resulting recombination products contained one copy of a complemented complete human adenovirus type 5 genome expressing the EGFP gene being replication competent in 293 cells (Fig. 12).

### Description of the preferred embodiments

The present invention discloses a first nucleic acid with a first part of a genome of a virus which is combined with a second nucleic acid molecule comprising a second part of a genome of a virus, whereby the first and the second nucleic acid molecule are combined and reacted by site-specific recombination in *E.coli* host cells providing a site-specific recombinase. The resulting nucleic acid molecule contains exactly one copy of a complemented complete genome of a or the virus, whereby the virus genome is replication competent in permissive cells. A schematic illustration of this invention is shown in Fig.1. In a further embodiment of this invention a third nucleic acid molecule is described. The third nucleic acid is combined and reacted by site-specific recombination with a second nucleic acid molecule in *E.coli* host cells providing a site-specific recombinase. The organization of the genetic elements in the third nucleic acid molecule is inventive, and according to the method provided in this invention restricts the number of recombination events to one in >97.5% of cases. This efficiency is sufficient for the construction of a plurality or library of fifth nucleic acid molecules and solves the problem of the need for screening such plurality of nucleic acid or library for single recombined products. A diagrammatic representation of this method is shown in Fig.8.

The first nucleic acid molecule comprises a first part of a genome of a virus, preferentially a first part of a genome of an adenovirus, and even more preferentially a first part of the human adenovirus type 5. Moreover, the first nucleic acid molecule comprises a transcription unit, a site specific recombination site, preferentially a minimal Frt site (Fig.10 SEQ. ID No.7), a bacterial nucleotide sequence unit comprising (i) bacterial nucleotide sequences for conditional replication and (ii) a nucleotide sequence providing for a first selection marker. In a preferred embodiment the first nucleic acid is a bacterial plasmid containing a first part of a or the genome of a virus, whereby the first part of a genome of a or the virus is a terminal repeat, preferably an inverted terminal repeat. In a more preferred embodiment the virus is an adenovirus, and the terminal repeat is an inverted terminal repeat of an adenovirus. In a most preferred embodiment, the virus is the human adenovirus type 5 and the first part of the genome of the human adenovirus type 5 is the left inverted terminal repeat.

In one embodiment of the present invention the first part of a genome of a virus comprises a packaging signal. In a preferred embodiment the packaging signal is part of the terminal sequence, whereby in a more preferred embodiment the packaging signal is the packaging signal (ψ5) from human adenovirus type 5 extending from nt194 to nt385 of the AV5 genome (Grable M. and Hearing P. J. Virol. 66:723-731, 1992₄₉). Packaging of adenoviral vectors depend on a series of 7 'A' repeats that are used in a hierarchical order with some being more important than others. Therefore it is possible to define synthetic or minimal packaging sequences by combining parts of sequences derived from this region (Ostapchuk P, Hearing P. Curr. Top. Microbiol. Immunol. 272:165-85, 2003₅₀). The location of these cis-acting packaging elements to the left part of the adenovirus genome has been experimentally confirmed for many other types of adenoviruses. Moreover, the identification of trans-acting factors for the packaging process has identified several adenovirus proteins acting in a subtype specific way, allowing only packaging of viral DNA if the encapsidation signal ψ and the trans acting factors are derived from the same subtype or are compatible.

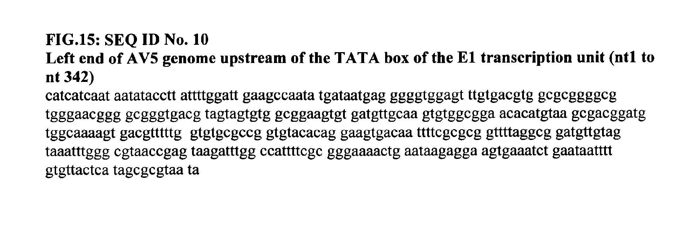

In a further embodiment of the invention the first part of a genome of a virus comprising the entire or parts of the left end of AV5 genome upstream of the TATA box of the E1 transcription unit from nt1 to nt 342 (Fig.15 SEQ ID 10) (Yamamoto M. et al., J. Virol. 77:1633-1637,2003₄₄).

In a further embodiment of this invention the first part of a genome of a virus contains an inverted terminal repeat (ITR), whereby in a preferred embodiment the inverted repeat is derived from the left end of the human adenovirus type 5 (AV5) and comprises the left inverted terminal repeat. The length of the left inverted terminal repeat sequence (left ITR) extends from nucleotide 1 to nucleotide 103 of the AV5 sequence (Hearing and Shenk, Cell 33:695-703, 1983₄₅). The size of the ITRs vary among the serologically distinct types of adenoviruses, and minimal terminal ITR sequences as short as 18bp (nt1 to nt18) supporting human Adenovirus type 4 virus replication in vivo can be defined (Liu H, Naismith JH, Hay RT. Curr. Top. Microbiol. Immunol. 272:131-64, 2003₄₆). Although the terminal 18-bp of the ITR supports basal level of DNA replication, the auxiliary region is needed for maximum efficiency in subgroup C adenoviruses, AV2 and AV5, respectively. Other virus types (e.g. adeno-associated viruses AVVs) do also rely on the presence of an ITR for virus replication. For human AAV type 2 the length of the ITR is 145 nucleotides and is an essential terminal sequence required for virus replication (Goncalves MA. Virol J. 2:43, 2005₄₇). The principle also applies to other types of viruses that contain terminal sequences other than ITRs (e.g. SV40, baculovirus, gamma herpesviruses) needed for replication and encapsidation. As an example, the alpha sequence of the cytomegalovirus genome functions as a cleavage/packaging signal for herpes simplex virus defective genomes. (Spate R and Mocarski E. J. Virol. 54:817-24, 1985₄₈).

In one embodiment the invention the first nucleic acid comprises a first restriction site, whereby this sequence is absent in the first part of the genome of a virus and in the transcription unit present in the first nucleic acid. In a preferred embodiment of this invention the restriction site is chosen from a group of restriction sites absent in the genome of an adenovirus. In a more preferred embodiment, the restriction site is selected from a group of sites absent in human adenovirus type 5 (AV5) comprising AbsI, BstBI, PacI, PsrI, SgrDI, and SwaI.

The first nucleic acid molecule comprises the following elements: a site-specific recombination site, a bacterial nucleotide sequence comprising (i) bacterial nucleotide sequences for conditional replication, and (ii) a nucleotide sequence providing for a first selection marker, a first restriction site, a first part of a genome of a virus, and a transcription unit, whereby in a preferred embodiment the virus is an adenovirus, and in more preferred embodiment, the virus is a human adenovirus type 5.

In a further embodiment the preset invention provides a first nucleic acid molecule comprising the following elements in a 5'to 3'orientation obtained after linearization of the first nucleic acid molecule optionally with the first restriction enzyme: the first part of a genome of a virus, a transcription unit, a site-specific recombination site, the bacterial nucleotide sequence unit comprising (i) bacterial nucleotide sequences for conditional replication and (ii) a nucleotide sequence providing for a first selection marker, optionally a first restriction site, By an inventive matter this is the preferred orientation of the genetic elements of the first nucleic acid molecule.

In one embodiment of this invention the first nucleic acid contains the genetic elements in a "mirror conformation", comprising in a in 5'-> 3'orientation: a site specific recombination site, a transcription unit, a first part of a genome of a virus, optionally a first restriction site, and bacterial nucleotide sequence unit comprising (i) bacterial nucleotide sequences for conditional replication, and (ii) a sequence providing for a first selection marker. By an inventive matter this is a further orientation of the genetic elements of the first nucleic acid molecule.

In a further embodiment the first part of a genome of a virus in a "mirror conformation" comprises a terminal repeat. In a preferred embodiment the first part of a genome of a virus comprises an inverted terminal repeat. In a more preferred embodiment it comprises a right terminal repeat of a virus genome, and even more preferably the right terminal repeat of an adenovirus, being the last 103 nucleotides of the genome of a human adenovirus type 5 virus genome.

A further embodiment of the invention relates to a transcription unit containing a promoter, optionally a nucleic acid sequence to be expressed, and a termination signal.

A further embodiment of the invention the first nucleic acid contains a transcription unit comprising a nucleic acid sequence to be expressed operable linked to a a promoter, and a termination signal, whereby the promoter shall be selected from the group of eukaryotic or viral promoters recognized by eukaryotic RNA Pol II such as PGK, and CMV (Juven-Gershon T, Hsu JY, Theisen JW, Kadonaga JT.. Curr. Opin. Cell Biol. 20:253-259, 2008₅₁), or from the group of eukaryotic or viral promoters recognized by RNA Pol III such as U6, H1, tRNA, and Adenovirus VA promoter.

A further embodiment of the invention relates to a transcription unit containing a promoter, a nucleic acid sequence to be expressed, and a termination signal, whereby the nucleic acid to be expressed is chosen from the group of nucleic acids encoding a protein, a peptide, a nucleic acid encoding non-coding RNA, including microRNAs, and small interfering RNAs (siRNAs), and shRNAs.

In another aspect of the invention the transcription unit contains a promoter, a sequence to be expressed, and a transcriptional termination signal, whereby the termination signal is derived from eukaryotic or viral genes such as a poly A signal, termination signals for RNA PolIII-transcribed genes, such as a stretch T nucleotides (Geiduschek EP and GP Tocchini-Valentini. Annu. Rev. Biochem. 57:873-914, 1988₅₂).

In a further embodiment of this invention the first nucleic acid comprises a site-specific recombination site for Flp recombinase. The Frt site used in the first nucleic acid molecule is based on the wild type Frt site from µ plasmid of *S. cerevisiae* (Broach JR, Guarascio VR, Jayaram M. Cell 29:227-234, 1982₅₃). In one embodiment of the invention the Frt site used is not restricted to forms derived from the wild type 48 Frt site (Fig. 11 SEQ ID. 8). It may be chosen from a group of other Frt sited including mutated Frt sites known in the art. (Schlake T. and Bode J. Biochemistry 33:12746-12751, 1994₅₄; WO/1999/025854₄₃). In a preferred embodiment of this invention the Frt site used in the first nucleic acid molecule is a minimal recombination site of 34nt length (Fig.10 SEQ. ID 7) containing the R2, the U and the R3 element of the wt FRT site (Cherepanov PP and Wackernagel W. Gene 158:9-14, 1995₅₅).

In one embodiment of the invention the first nucleic acid molecule with a bacterial sequence unit comprises (i) bacterial sequences for conditional replication and (ii) a sequence providing for a first selection marker, whereby the bacterial sequences for replication contain an origin of replication (ori).

In another embodiment of the invention the first nucleic acid molecule with a bacterial sequence unit comprises (i) bacterial sequences for conditional replication and (ii) a sequence providing for a first selection marker, whereby the bacterial sequences for replication contain an origin of replication for conditional replication in special *E. coli* strains or in normal *E. coli* strains under specific conditions where the bacterial cell provides all functions necessary. In a preferred embodiment, bacterial sequences in the first nucleic acid molecule contain the minimal ori of phage gR6K as conditional replicon which can be maintained only in the presence of pi protein expression (Shafferman A, Kolter R, Stalker D, Helinski DR. J. Mol. Biol. 161:57-76,1982₅₆).

One embodiment of this invention provides a first nucleic acid molecule with a sequence providing for a first selection marker, whereby the selection marker is a nucleic acid that confers resistance to a cell harboring such nucleic acid against a selecting agent. In a preferred embodiment of the invention, the first selection marker encodes a gene, and in a more preferred embodiment the first selection marker preferably mediates resistance against an antibiotic including ampicillin, zeocin, gentamycin, chloramphenicol, tetracycline, and kanamycin among others known in the art. In a most preferred embodiment of this invention the first selection marker mediates resistance against kanamycin.

The first selection marker can be selected from a group of genes mediating resistance to antibiotics, including *bla, ant(3'')-Ia, aph(3)-II, aph(3)-II, ble,* and *cml*A, *aad*A, *aad*B, *sac*B, *and tet*A genes among other genes known in the art. In a preferred embodiment a gene encoding a protein mediating resistance to kanamycin is the first selection marker.

One embodiment of the invention is a second nucleic acid molecule comprising the following elements: a bacterial nucleotide sequence unit comprising (i) bacterial nucleotide sequence for single copy replication, and (ii) a nucleotide sequence providing for a second selection marker, a site-specific recombination site, a second part of a genome of a virus, and optionally a restriction site which is referred to as second restriction site..

One embodiment of the invention is a second nucleic acid molecule containing a second part of a genome of a virus, whereby the second part of a genome of a virus combined with the first part of a genome of a virus form a complemented complete virus genome able to replicate in a complementing cell line.

In a preferred embodiment, the second nucleic acid contains the second part of an adenovirus genome, and in a more preferred embodiment the second nucleic acid contains the second part of a human adenovirus type 5 (AV5) genome. In a more preferred embodiment the second part of a virus genome is the AV5 genome deleted for the left ITR, the E1 region and the E3 region of AV5, and optionally for the encapsidation signal ψ5, whereby the first nucleic acid complements this virus genome for the left ITR and optionally the encapsidation signal. Moreover, the deletions of the second part of the AV5 genome are not limited to E1 and E3, since additional sequences from the E2 or the E4 region may be deleted as well, provided, that a permissive cell line can complement for the deleted sequences in cis or trans.

One embodiment of the invention is a second nucleic acid containing a site specific recombination site. The site-specific recombination site is selected from the group comprising the recombination site for Flp recombinase. In a preferred embodiment of the invention the Frt site used is the wild type Frt48 site from µ plasmid of *S*. *cerevisiae* (Broach JR, Guarascio VR, Jayaram M. Cell 29:227-234, 1982₅₃) without being restricted to it. Other Frt sites can be used, including mutated Frt sites known in the art (Schlake T. and Bode J. Biochemistry 33:12746-12751, 1994₅₄; WO/1999/025854₄₃ and references cited therein).

In a further embodiment of this invention, the second part of the genome of a virus comprises a terminal repeat, preferably a viral terminal repeat, and more preferably an inverted terminal repeat. Preferably, the inverted terminal repeat is the right inverted terminal repeat from an adenovirus genome, and in a most preferred embodiment the adenovirus genome is derived from human adenovirus type 5, and the inverted terminal repeat is the right inverted terminal repeat of human adenovirus type 5.

One embodiment of the invention provides a second nucleic acid molecule comprising a second restriction site, whereby the restriction site is absent in the second part of the genome of a virus. The restriction site is used for linearization of the nucleic acids contained according to the methods disclosed in this patent. Moreover, the second restriction site is absent in the first part of the genome of a virus provided by the first nucleic acid molecule, and in the sequence part of the first nucleic acid ranging from the first restriction site to the recombination site and encompassing the first part of the virus genome. In a more preferred embodiment of this invention the restriction site is chosen from a group of restriction sites absent in the genome of an adenovirus. In an even more preferred embodiment, the restriction site is selected from a group of sites absent in human adenovirus type 5 (AV5) comprising AbsI, BstBI, PacI, PsrI, SgrDI, and SwaI, among other restriction sites known by persons skilled in the art, including other types of sites recognized by homing endonucleases and synthetic binding sites for zinc finger nucleases (Marcaida MJ et al. Homing endonucleases: from basics to therapeutic applications. Cell Mol Life Sci. 67(5):727-48, 2010₇₆; Klug A. The discovery of zinc fingers and their applications in gene regulation and genome manipulation. Annu Rev Biochem. 79:213-31, 2010₇₇)

One embodiment of the invention provides a second nucleic acid containing a bacterial nucleotide sequence unit comprising (i) bacterial nucleotide sequences for single copy replication and (ii) a nucleotide sequence providing for a second selection marker. In a preferred embodiment the bacterial nucleotide sequences for replication (ori) contain all elements necessary low copy, preferably singly copy maintenance in *E. coli.* In a more preferred embodiment the ori in the second nucleic acid is based on the f-episomal factor (F-factor) (Scott JR. Regulation of plasmid replication. Microbiol. Rev. 48:1-23, 1984₆₅), and contains all elements which are necessary for replication and maintenance in E. *coli.* One further embodiment of this invention is a second nucleic acid molecule comprising the following elements in a 5'-> 3' direction upon linearization of the second nucleic acid molecule with a restriction enzyme, preferably a restriction enzyme recognizing and cutting at the second restriction site: a bacterial nucleotide sequence unit comprising (i) bacterial nucleotide sequence for single copy replication, and (ii) a nucleotide sequence providing for a second selection marker, a site-specific recombination site, a second part of a genome of a virus, and optionally a second restriction site.

In a further embodiment of this invention the second nucleic acid molecule contains a sequence providing for a second selection marker coding for a resistance mediating gene, and more preferably for an resistance mediating gene encoding for an enzyme. The selection marker used in the second nucleic acid is different from the selection marker present in the first nucleic acid molecule. In a preferred embodiment the second selection marker confers resistance against antibiotics, including ampicillin, zeocin, gentamycin, chloramphenicol, and kanamycin among others known in the art. In a most preferred embodiment the second selection marker mediates resistance against chloramphenicol.

The second selection marker in the second nucleic acid molecule can be selected from a group of genes mediating resistance to antibiotics, including *bla, ant(3")-Ia, aph(3)-II, aph(3)-II, ble, aad*A*, aad*B*,* and *cmlA* genes among other genes known in the art. In a more preferred embodiment the second selection marker is a gene encoding a protein mediating resistance to chloramphenicol.

In a further embodiment of the invention, referred to as "the mirror conformation" the second nucleic acid molecules provides the following elements in a 5'-> 3' direction: optionally a second restriction site, a second part of a or the genome of a virus, a recombination site, and a bacterial nucleotide sequence unit comprising (i) bacterial nucleotide sequences for single copy replication and (ii) a nucleotide sequence providing for a second selection marker, whereby the second part of the genome of the virus provides a left terminal repeat, and in a preferred embodiment the left inverted terminal repeat (ITR) of a virus genome. In a more preferred embodiment, the virus is an adenovirus, and in an even more preferred embodiment, the adenovirus is the human adenovirus type 5.

In a further embodiment of the invention the second nucleic acid molecules in a"mirror conformation" provides the second part of a or the genome of the virus providing a packaging signal, whereby in a preferred embodiment the virus is an adenovirus, and in an more preferred embodiment, the adenovirus is the human adenovirus type 5.

In one embodiment of the present invention the second nucleic acid replicates as a single copy vector in *E. coli,* whereby the ori used is based on F-factor or a P1 replicon. In a preferred embodiment the second nucleic acid is a bacterial artificial chromosome (BAC) without being limited to a BAC. However, the system requires low copy, preferably single copy maintenance of the second nucleic acid in E*.coli* in order to retain full functionality. In a more preferred embodiment, the BAC vector identical or similar to pBACSir1 or pBACSir2 encodes a first generation E1 and E3 deleted Ad vector genome deleted for the left ITR and the encapsidation signal, and contains the *parS* the *parA, parB and parC* genes as elements of the origin of replication which are necessary for single copy maintenance (Ruzsics Z, Wagner M, Osterlehner A, Cook J, Koszinowski U, Burgert HG. Transposon assisted cloning and traceless mutagenesis of adenoviruses: Development of a novel vector based on species D. J. Virol. 80:8100-8113, 2006₄₂).

One embodiment of the invention is the combination of a circular closed form of the first nucleic acid molecule with a circular closed form of the second nucleic acid molecule, whereby in a preferred embodiment the first nucleic acid used is a plasmid and the second nucleic acid is a BAC vector.

A further embodiment of the invention is a combination of the first and the second nucleic acid molecule, whereby both nucleic acid molecules are present as separate molecules. The term "separate molecules" means that each molecule is dissociable in physical distinct compartments. In a preferred embodiment of this invention the first nucleic acid molecule is a plasmid and the second nucleic acid molecule is a BAC.

One embodiment of the invention is the combination of a first nucleic acid molecule with the second nucleic acid molecule, whereby the first part of a genome of a virus provided by the first nucleic acid, and the second part of a genome of a virus provided by the second nucleic acid, if taken together form a complete virus genome. The term "complete virus genome" describes a nucleic acid encoding a viral genomic sequence which upon transfection into a eukaryotic cell lines gives rise to viable and replication competent virus. Such a cell line is termed a permissive cell line. In a preferred embodiment of the invention the virus genome is an adenovirus genome, and in a more preferred embodiment of the invention the virus genome is the human adenovirus type 5 genome.

One embodiment of the invention is the combination of a first nucleic acid molecule with the second nucleic acid molecule, whereby the first restriction site provided by the first nucleic acid molecule and the second restriction site provided by the second nucleic acid molecule are chosen from a group comprising restriction sites that are absent in the first part and the second part of the genome of a virus, and the transcription unit. In a preferred embodiment the restriction sites are selected from the group that does not cut in the human adenovirus type 5 genome: AbsI, BstBI, PacI, PsrI, SgrDI, and SwaI. In a further preferred embodiment the first and the second restriction site are identical, an in an even more preferred embodiment the first and second restriction site is PacI.

One embodiment of the invention the complete virus genome is an adenovirus genome, whereby in a more preferred embodiment the adenovirus is AV5. In an even more preferred embodiment, the complete virus genome is a first generation - E1 and E3-deleted AV5 without being limited to this type of adenovirus genome, since additional sequences from the E2 region may be additionally deleted (Schaack J. Front. Biosci. 10:1146-1155, 2005₆₆) or multiple regions changed (Gorziglia MI, Lapcevich C, Roy S, Kang Q, Kadan M, Wu V, Pechan P, Kaleko M. J Virol. 73:6048-6055, 1999₆₇), or even the complete virus genome except for the left and right ITR and the packaging signal deleted in gutless adenovirus vectors (Kochanek S, Schiedner G, Volpers C. Curr. Opin. Mol. Ther 3:454-463, 2001₆₈).

One embodiment of the invention is the combination of a first nucleic acid molecule with the second nucleic acid molecule, whereby the first selection marker provided by the first nucleic acid molecule and the second selection marker provided by the second nucleic acid molecule, is a gene preferably encoding for an enzyme conferring resistance agianst an antibiotic. The gene may be chosen from a group conferring resistance against kanamycin, neomycin, puromycin, ampicillin, zeocin, gentamycin, and chloramphenociol among others known in the art. In a preferred embodiment the first selection marker is a gene confers resistance against kanamycin, and the selection marker is a gene conferring resistance against chloramphenicol but not kanamycin. It is know in the art, that several genes mediate resistance against more than one selection agent, especially if the selection agent is an antibiotic (Tenorio C, Zarazaga M, Martinez C, Torres C. J. Clin. Microbiol. 39:824-825, 2001₆₉), limiting the possible combinations of selection markers for the first and second nucleic acid.

One embodiment of the invention is the combination of a first nucleic acid molecule with the second nucleic acid molecule, whereby the bacterial sequences for replication provided by the first nucleic acid molecule allow for conditional replication in special E *coli* strains or in normal *E. coli* strains under specific conditions where the bacterial cell provides all functions necessary. Moreover, the combination of the sequences for replication of the first and the second nucleic acid allow only for replication of the second nucleic acid in a host cell. It is known in the art, that the combination of sequences for bacterial replication is restricted to the presence of factors provided by the host cell or the nucleic acid itself (Scott JR. Regulation of plasmid replication. Microbiol. Rev. 48:1-23, 1984₆₅). In a preferred embodiment, bacterial sequences in the first nucleic acid molecule contain the minimal ori of phage gR6K as conditional replicon which can be maintained only in the presence of pi protein expression (Shafferman A, Kolter R, Stalker D, Helinski DR. Plasmid R6K DNA replication. III. Regulatory properties of the pi initiation protein. J. Mol. Biol. 161:57-76, 1982₅₇), and the sequences for replication of the second nucleic acid are based on the F-factor and allow for single copy maintenance in E*.coli* cells.

One embodiment of the invention is the combination of a first nucleic acid molecule providing the first part of a or the genome of a virus and a second nucleic acid molecule providing the second part of a or the genome of a virus, whereby the packaging signal may be provided by either the first or the second part of the genome of a virus. In a preferred embodiment the virus is an adenovirus, and in a more preferred embodiment the virus is a human adenovirus and in an even more preferred embodiment the virus is human adenovirus type 5 (AV5) and the packaging signal is derived from AV5 (ψ5) and provided by the first nucleic acid molecule.

One embodiment of the invention is the combination of a first nucleic acid molecule with the second nucleic acid molecule, whereby a first terminal repeat sequence is part of the first part of the genome of a or the virus provided by the first nucleic acid molecule, and a second terminal repeat sequence is part of the second part of a genome of a virus provided by the second nucleic acid molecule.

In a further embodiment of this invention either the first or the second nucleic acid molecule can provide all terminal repeat sequences. In a preferred embodiment the terminal repeat sequences are the inverted terminal repeat sequences derived from an adenovirus, and in an even more preferred embodiment the inverted terminal repeats are derived from AV5.

One embodiment of the invention discloses a method for the generation of nucleic acid molecules coding for a virus, comprising a combination of a first nucleic acid molecule with a second nucleic acid molecule, whereby both nucleic acids are reacted through their site-specific recombination sites forming a recombination product, whereby the recombination product is selected and contains only one copy of a complete virus genome, and whereby the recombination product is cleaved with the first and second restriction enzyme.

One embodiment of the invention discloses a method for the generation of nucleic acid molecules coding for a virus where the first and the second nucleic acid molecules are combined and reacted through their site-specific recombination sites in a prokaryotic host cell. The host cell is preferably a bacteria cell and can accept nucleic acids by either being electroporated or made chemically competent according to standard methods. In a preferred embodiment the bacterial host cell harbors the second nucleic acid molecule and accepts the first nucleic acid molecule by means of electroporation. In a most preferred embodiment the bacteria is E*.coli.*

In a further embodiment of this invention the bacterial host cell is selected from a group of E*. coli* cells lacking the F-factor and being sensitive to the first and second selecting agent. In a preferred embodiment the E*.coli* strain is K12-derived and does not provide or express the pi protein. The pi protein sustains the replication of the first nucleic acid molecule, but not of the second nucleic acid molecule. In a more preferred embodiment the E.*coli* strain is sensitive to kanamycin and chloramphenicol, and selected from a group comprising DH5alpha, DH10B, among others known in the art.

One embodiment of the invention discloses a method for the generation of nucleic acid molecules coding for a virus, whereby a first nucleic acid molecule with a first selection marker and a second nucleic acid molecule with a second selection marker are combined and reacted through their recombination sites in the presence of a site-specific recombinase, forming a recombination product in a prokaryotic host cell. The method is such, that the reaction product is selected in the host cell by conferring resistance against both selection markers. The use of a conditional origin of replication in the first nucleic acid ensures that the method selects exclusively for reacted products. In a preferred embodiment, the first selection marker is kanamycin, and the second selection marker is chloramphenicol.

One embodiment of the invention discloses a method for the generation of nucleic acid molecules coding for a virus where a first nucleic acid molecule and a second nucleic acid molecule are combined and reacted in the presence of a site-specific recombinase which catalyses without the need of a source of energy like ATP the recombination between the first site-specific recombination sites provided by the first nucleic acid molecule and the second site-specific recombination site provided by second nucleic acid molecule. In a preferred embodiment the site-specific recombinase is Flp, whereby it mediates the recombination between the Frt site-specific recombination site present on the first nucleic acid and the Frt site-specific recombination site present on the second nucleic acid. Flp catalyzes the site-specific recombination between Frt sites, whereby the recognized site-specific recombination sites are large enough to be statistically absent in the human and bacterial genome. According to the invention, a minimal wild type Frt34 site is used in the first nucleic acid and reacted with a wild type Frt48 site present in the second nucleic acid. However, other site-specific recombinases known in the art may be used, provided they function with equally high selectivity and efficiency.

One embodiment of the invention discloses a method for the generation of nucleic acid molecules coding for a virus whereby a first nucleic acid molecule and a second nucleic acid molecule are combined and reacted in the presence of a site-specific recombinase, and whereby the recombinase is inactivated. It is generally acknowledged that a prolonged presence of a site-specific recombinase in *E.coli* interferes with genome stability. In the case of Cre, cryptic loxP sites are recognized in the mammalian genome causing genetic instability, and limiting the use of Cre-containing E.coli for receiving BAC and PAC vectors (Semprini S et al. Cryptic loxP sites in mammalian genomes: genome-wide distribution and relevance for the efficiency of BAC/PAC recombineering techniques. Nucleic Acids Res. 35:1402-1410, 1997). Preferably, transient expression of the site-specific recombination is desired when nucleic acid molecules need to be recombined and further propagated in E*.coli,* and even more preferably, expression of the site specific recombinase is fully eliminated after the recombination has occurred and during the growth of the bacteria.

In a preferred embodiment of the method for the generation of nucleic acid molecules coding for a virus the Flp expression is controlled by a temperature sensitive repressor from lambda phage. The Flp expression is induced by shifting the culture temperature to 43oC. This procedure allows elimination (curing) of the plasmid at the same time. Other systems for conditional and/or inducible expression of a site-specific recombinase may be used instead, for example, without being limited to it, use of an arabinose-inducible *AraC-P_{BAD}* promoter to induce expression (Lee EC., et al. A highly efficient Escherichia coli-based chromosome engineering system adapted for recombinogenic targeting and subcloning of BAC DNA. Genomics 73:56-65, 2001₇₀).

In a further embodiment of the method for the generation of nucleic acid molecules coding for a virus conditional expression for a site-specific recombinase in bacterial cells is used, whereby the replication of a plasmid harboring an expression unit for the Flp site-specific recombinase is controlled by a temperature-sensitive origin of replication. In a preferred embodiment E.*coli* host cell harboring the second nucleic acid molecule and a bacterial plasmid (pCP20) providing a Flp expression unit, can be maintained and propagated at 30oC in the presence of ampicillin. The Flp expression is induced by shifting the culture temperature to 43oC. This procedure allows elimination (curing) the pCP20 in the same time. (Cherepanov PP and Wackernagel W. Gene disruption in Escherichia coli: TcR and KmR cassettes with the option of Flp-catalyzed excision of the antibiotic-resistance determinant. Gene 158:9-14, 1995₅₅).

In the method disclosed for the generation of nucleic acid molecules coding for a virus, the nucleic acid coding for the complete virus can be released by restriction digest with the first and second restriction enzyme. In a preferred embodiment of this invention the restriction site is chosen from a group of restriction sites absent in the genome of an adenovirus, and in an even more preferred embodiment, the restriction site is selected from a group of sites absent in human adenovirus type 5 (AV5) comprising AbsI, BstBI, PacI, PsrI, SgrDI, and SwaI.

One embodiment of the invention discloses a method for the generation of nucleic acid molecules coding for a virus, comprising a combination of a linear form of a first nucleic acid molecule with a linear form of the second nucleic acid molecule, whereby both nucleic acids are reacted through their site-specific recombination sites forming a recombination product in a permissive cell, whereby the recombination product is not selected, and whereby it contains only one copy of a complete virus genome, and whereby the site-specific recombinase is provided by the permissive cell, and whereby the site-specific recombinase is either expressed in a constitutive, a conditional or in an induced way. Conditional or induced expression of the site-specific recombinase can be achieved with the tetracyclin-regulated expression system among other systems known in the art (Gossen M, Bujard H. Tight control of gene expression in mammalian cells by tetracycline-responsive promoters. Proc Natl Acad Sci USA. 89:5547-51, 1992₇₈). In a preferred embodiment of the invention the permissive cell expresses the site-specific recombinase stably, whereby the permissive cell is selected from a group comprising 293, 911, Per.C6 and N52.E6 cells. In an even more preferred embodiment the permissive cell is 293, and the site-specific recombinase Flp is constitutively expressed.

One embodiment of the invention provides a method for the generation of nucleic acid molecules coding for a virus where a first nucleic acid molecule and a second nucleic acid molecule are combined and reacted in the presence of a site-specific recombinase, whereby according to the invention the resulting nucleic acid molecule contains one copy of a complete virus genome, which can be released by restriction digest with the first and second restriction enzyme, and generates a viable replication-competent virus when transfected into a permissive cell line, whereby the permissive cell is selected from a group comprising 293, 911, Per.C6 and N52.E6 cells. In an even more preferred embodiment the permissive cell is 293.

One embodiment of the invention provides a method for the generation of nucleic acid molecules coding for a virus whereby the virus can be used as gene transfer vector, as vaccine or used for therapeutic applications.

One embodiment of the invention provides a method for the generation of nucleic acid molecules coding for a virus whereby the method can be used to generate large numbers of viruses or a library of viruses expressing nucleic acids.

The present invention discloses a third nucleic acid, whereby the third nucleic acid molecule comprises the following elements: optionally a first part of a or the genome of a or the virus, a nucleotide sequence, preferably a genomic nucleotide sequence or a transcription unit, a regulatory nucleic acid sequence which has regulatory activity in a prokaryote, a site-specific recombination site, a nucleotide sequence providing for a negative selection marker, a bacterial nucleotide sequence unit comprising (i) bacterial nucleotide sequences for conditional replication and (ii) a nucleotide sequence providing for a positive selection marker, and optionally a first restriction site.

In a further embodiment of the invention the third nucleic acid molecule comprises the following elements in a 5'to 3'orientation preferably upon cleavage with the first restriction enzyme: optionally the first part of a or the genome of a or the virus, the nucleotide sequence, preferably a genomic nucleotide sequence, or a transcription unit, the regulatory nucleic acid sequence which has regulatory activity in a prokaryote, a site-specific recombination site, a nucleotide sequence providing for a negative selection marker, a bacterial nucleotide sequence unit comprising (i) bacterial nucleotide sequences for conditional replication and (ii) a nucleotide sequence providing for a positive selection marker

In one embodiment of the invention the first part of a or the genome of a or the virus provided by the third nucleic acid contains parts of the terminal sequence. In a preferred embodiment of this invention the terminal sequences comprise a terminal repeat. Moreover, the first part of a or the genome of a or the virus must be present in order to be a complete virus genome able to replicable in a permissive cell line. In a more preferred embodiment of the invention, the first part of a or the genome of a or the virus is derived from an adenovirus genome, and in an even more preferred embodiment, the first part of the human adenovirus genome is derived from a human adenovirus type 5 (AV5) genome, comprising the entire or parts of the left end of AV5 genome upstream of the TATA box of the E1 transcription unit (ntl to nt 342) (Fig. 15 SEQ.ID. No.10) (Yamamoto M. et al., J. Virol. 77:1633-1637, 2003₄₄). In a further embodiment of this invention the third nucleic acid molecule provides a first part of a or the genome of a or the virus comprising a packaging signal as part of the viral genome. In a more preferred embodiment the packaging signal is derived from an adenovirus genome, and in an even more preferred embodiment, the first part of the adenovirus genome contains the packaging signal ψ5 derived from the left end of the human adenovirus type 5 (AV5).

In one embodiment of this invention the first part of a or the genome of a or the virus provided by the third nucleic acid molecule comprises the terminal sequence of a or the genome of a or the virus. In a more preferred embodiment the terminal sequence is an inverted terminal repeat (ITR), and even more preferred the inverted repeat is derived from an adenovirus genome, and in a most more preferred embodiment, the first part of the adenovirus genome contains an inverted terminal repeat derived from the left end of the human adenovirus type 5 (AV5).

In one embodiment the invention the third nucleic acid comprises a first restriction site, whereby the first restriction site is absent in the first part of a or the genome of a or the virus and in the transcription unit present in the third nucleic acid. In a preferred embodiment of this invention the restriction site is chosen from a group of restriction sites absent in the genome of an adenovirus, and in a more preferred embodiment, the restriction site is selected from a group of sites absent in human adenovirus type 5 (AV5) comprising AbsI, BstBI, PacI, PsrI, SgrDI, and SwaI.

In a further embodiment the third nucleic acid molecule provides in a mirror confirmation, preferably upon cleavage with the first restriction enzyme in 5'-> 3'orientation: a regulatory nucleic acid sequence which has activity in a prokaryote, a site specific recombination site, a nucleotide sequence providing for a negative selection marker, a bacterial nucleotide sequence comprising (i) bacterial nucleotide sequences for conditional replication and (ii) a nucleotide sequence providing for a positive selection marker, a first restriction site, a first part of a or the genome of a or the virus, and a transcription unit, whereby the first part of a or the genome of a or the virus comprises a terminal repeat. In a preferred embodiment the first part of a or the genome of a or the virus provided by the third nucleic acid in the mirror confirmation comprises an inverted terminal repeat. In a more preferred embodiment the inverted terminal repeat is a right terminal repeat of a virus genome, and even more preferably the right terminal repeat of an adenovirus, whereby in a most preferred embodiment the right terminal repeat of an adenovirus is the right ITR from human adenovirus type 5 encompassing the last 18-103 nucleotides of AV5

In one embodiment of the invention the third nucleic acid molecule comprises a gene transcription unit, whereby the transcription unit contains a promoter, a nucleic acid sequence to be expressed, and a termination signal. The promoter is selected from the group of eukaryotic or viral promoters recognized by eukaryotic RNA Pol II such as PGK, and CMV (Juven-Gershon T, Hsu JY, Theisen JW, Kadonaga JT. Curr. Opin. Cell Biol. 20:253-259, 2008₅₁), or from the group of eukaryotic or viral promoters recognized by RNA Pol III such as U6, H1, tRNA, and Adenovirus VA promoter. The nucleic acid to be expressed is chosen from the group of nucleic acids encoding a protein, a peptide, a nucleic acid encoding non-coding RNA, including microRNAs, and small interfering RNAs (siRNAs), and shRNAs. The termination is signal is derived from eukaryotic or viral genes such as a poly A signal, termination signals for PolIII-transcribed genes, such as a stretch T nucleotides (Geiduschek EP and GP Tocchini- Valentini. Transcription by RNA polymerase III. Annu. Rev. Biochem. 57:873-914,1988₅₂).

In a further embodiment of this invention the third nucleic acid comprises a site-specific recombination site recognized by the Flp recombinase. The Frt site used in the third nucleic acid molecule is based on the wild type Frt site from µ plasmid of *S. cerevisiae* (Broach JR, Guarascio VR, Jayaram M. Cell 29:227-234, 1982₅₃). In a further embodiment of the invention the Frt site used is not restricted to forms derived from the wild type 48 Fit site. It may be chosen from a group of other Frt sited including mutated Frt sites known in the art. (Schlake T. and Bode J. Biochemistry 33:12746-12751, 1994₅₄; WO/1999/025854₄₃ and references cited therein). In a preferred embodiment of this invention the Frt site used in the third nucleic acid molecule is a minimal recombination site of 34nt length (Frt34 site, SEQ.ID. 7) containing the R2, the U and the R3 element of the wt FRT48 site (SEQ.ID.NO. 8).

In one embodiment of the invention the third nucleic acid molecule with a bacterial sequence unit comprises (i) bacterial sequences for conditional replication and (ii) a sequence providing for a positive selection marker, whereby the bacterial sequences for conditional replication contain an origin of replication (ori) for replication in special *E. coli* strains or in normal *E. coli* strains under specific conditions where the bacterial cell provides all functions necessary. Replication of plasmid vectors in gram negative bacteria is controlled by host enzymes and determinants that are provided by the plasmid. Replication of plasmids only occur if all the factors necessary for replication are present in cis or in tarns in the bacterial host (Kües U and Stahl U. Microbial reviews 53:491-516₅₆). In a preferred embodiment, bacterial sequences in the third nucleic acid molecule contain the minimal ori of phage gR6K as conditional replicon which can be maintained only in the presence of pi protein expression (Shafferman A, Kolter R, Stalker D, Helinski DR. Plasmid R6K DNA replication. III. Regulatory properties of the pi initiation protein. J. Mol. Biol. 161:57-76, 1982₅₇).

One embodiment of this invention provides a third nucleic acid molecule with a sequence providing for a positive selection marker, whereby the selection marker is a nucleic acid coding for an enzyme, and the enzyme mediates resistance against a selecting agent, whereby the positive selection marker can be selected from a group of genes mediating resistance against antibiotics, including *bla, ant(3")-Ia, aph(3')-II, aph(3')-II, ble,* and *cmlA,* genes among other genes known in the art. In a preferred embodiment a gene encoding a protein mediating resistance to kanamycin is used as the positive selection marker.

The third nucleic acid molecule disclosed in this invention provides a negative selection marker, whereby the selection marker is a nucleic acid coding for an enzyme mediating sensitivity to a selecting agent and conditions, whereby the expression of the negative selection marker in a prokaryotic host cell is controlled by a nucleotide sequence which has regulatory activity in a prokaryote. In a preferred embodiment the regulatory nucleotide sequence is a promoter, whereby the promoter is preferentially selected from the group of prokaryotic promoters. (Busby S, Ebright RH. Cell 79:743-746, 1994₅₈; Mao Fd, Dam P, Chou J, Olman V, Xu Y. Nucleic Acids Res. 37:D459-463, 2009₅₉). In an even more preferred embodiment the promoter is the E.*coli* galactokinase promoter.

In a further embodiment of the invention, the regulatory nucleotide sequence can be chosen from the group of inducible prokaryotic promoters, whereby the activity of the promoter can be regulated by various means including derepression of operons, induction of genes by ions and molecules, regulation of promoter activity by temperature, among other methods and systems known in the art (Ullmann A. Escherichia coli Lactose Operon. In: Encyclopedia of Life Sciences. doi:10.1038/npg.els.0000849, 2001₆₀; Zhang X, Pan Z, Fang Q, Zheng J, Hu M, Jiao X. J Microbiol Methods. 79:199-204, 2009₆₁. Bukrinsky et al. An auto-inducible Escherichia coli lysis system controlled by magnesium Gene 70:415-417, 1988₆₂).

In a further embodiment of the invention the negative selection marker provided by the third nucleic acid molecule is chosen from a class of genes coding for an enzyme, whereby the enzyme confers sensitivity to a selecting agent or condition including: sensitivity to streptomycin, lipophilic compounds (fusaric and quinaric acid), sucrose, p-chlorophenylalanine, trimethoprim, *t*-*o*-nitrophenyl-β-D-galactopyranoside among others known in the art. In a preferred embodiment of this invention the enzyme coded by the negative selection marker mediates sensitivity to streptomycin. Accordingly, the nucleic acid encoding the negative selection marker can be selected from a group of genes including *galK, tetAR, pheS, thyA, lacy, ccdB, and rpsL* among other genes known in the art. In a preferred embodiment the *rpsL* gene encoding a protein dominantly mediating sensitivity to streptomycin is used as the negative selection marker (Reyrat JM, Pelicic V, Gicquel B, Rappuoli R. Infect Immun. 6:4011-4017, 1998₆₃; Dean D. A plasmid cloning vector for the direct selection of strains carrying recombinant plasmids. Gene 15:99-102, 1981₆₄).

### Description of combinations between the second and the third nucleic acid

A further embodiment of the invention is a combination of the third and the second nucleic acid molecule, whereby both nucleic acids are present as circular closed molecules. In a preferred embodiment the third nucleic acid molecule used is a plasmid and the second nucleic acid is a BAC vector.

A further embodiment of the invention is a combination of the third and the second nucleic acid molecule, whereby both nucleic acid molecules are present as separate molecules. The term "separate molecules" means that each molecule is dissociable in physical distinct compartments. In a preferred embodiment of this invention the third nucleic acid molecule is a plasmid and the second nucleic acid molecule is a BAC.

In a preferred embodiment the nucleic acid provided by third nucleic acid provides a first part of the genome of a virus, and a second nucleic acid molecule provides a second part of the genome of a virus. The resulting nucleic acid after combination of the third with the second nucleic acid molecule contains one copy of a complete virus genome. In a preferred embodiment of the invention the virus genome is an adenovirus genome, and in a more preferred embodiment of the invention the virus genome is the human adenovirus type 5 genome.

A further embodiment of the invention is a combination of a third nucleic acid with a second nucleic acid, whereby the nucleic acid provided by third nucleic acid is the first part of a or the genome of a or the virus and contains a gene transduction unit, and is combined with a second nucleic acid molecule providing a second part of a or the genome of the virus. The resulting nucleic acid molecule contains one copy of the complete virus genome containing exactly one gene transduction unit. In a preferred embodiment of the invention the virus genome is an adenovirus genome, and in a more preferred embodiment of the invention the virus genome is the human adenovirus type 5 genome.

According to the invention the resulting complete virus genome can be released by restriction digest with the first and second restriction enzyme. In a preferred embodiment of the invention the first and the second restriction sites are identical on both nucleic acid molecules, and in a more preferred embodiment of the invention the restriction site recognized by *Pac*I enzyme is used.

In one embodiment of the invention a third nucleic acid molecule providing the first part of a or the genome of a virus with a first restriction site and a transcription unit is combined with a second nucleic acid molecule providing the second part of a or the genome of a virus with a second restriction site, whereby the restriction sites are chosen from a group comprising restriction sites that are absent in the first part and the second part of a or the genome of a or the virus, and the transcription unit. In a preferred embodiment the restriction sites are selected from the group that does not cut in the human adenovirus type 5genome: *AbsI, BstBI, PacI, PsrI, SgrDI,* and *Swa*I. In an even more preferred embodiment of the invention, the first and second restriction site is *Pac*I

One embodiment of the invention is the combination of a third nucleic acid molecule providing the first part of a or the genome of a virus with a second nucleic acid molecule providing a second part of a or the genome of a e virus, whereby both nucleic acids can be recombined through their Frt sites to form a molecule which contains one copy of a complete complemented virus genome. The resulting complete complemented virus genome can be released by restriction digest with the first and second restriction enzyme, accordingly, and is viable and replication competent if transfected into a permissive cell line. In a preferred embodiment of the invention the virus is an adenovirus, and in a more preferred embodiment the adenovirus is the human adenovirus type 5 (AV5). In an even more preferred embodiment, the resulting complete virus genome is a first generation - E1 and E3 deleted AV5, whereby the composition of the adenovirus genome is not limited to E1 and E3 deleted genomes, since additional sequences from the E2 an E4 region may be additionally deleted (Schaack J. Front. Biosci. 10:1146-1155, 2005₆₆) or multiple regions changed (Gorziglia MI, Lapcevich C, Roy S, Kang Q, Kadan M, Wu V, Pechan P, Kaleko M. J Virol. 73:6048-6055, 1999₆₇), or even the complete virus genome except for the left and right ITR and the packaging signal deleted in gutless adenovirus vectors (Kochanek S, Schiedner G, Volpers C. Curr. Opin. Mol. Ther 3:454-463, 2001₆₈). The cell line used for reconstitution and propagation of the virus, also termed a "permissive cell line", is able to complement for all the deleted or changed regions in cis or trans. In case of the first generation AV5 virus genome, a cell line complementing for E1 may be used, such as 293, 911, Per.C6, N52.E6 among others known in the art. Other cell lines providing additional components of the viral genome in trans may be used as well if required Moreover, transient or conditional expression of said deleted components may also be used to allow virus reconstitution and replication.

In an embodiment of the invention the third nucleic acid molecule provides a positive selection marker and a negative selection marker. The second nucleic acid molecule provides a second selection marker. Upon combination of the third with the second nucleic acid molecule the resulting nucleic acid molecule comprises the positive and the negative selection marker from the third nucleic acid and the second selection marker form the second nucleic acid molecule. In a preferred embodiment of the invention, the positive selection marker of the third nucleic acid confers resistance against kanamycin and the negative selection marker confers sensitivity to streptomycin, and the selection marker provided by the second nucleic acid molecule confers resistance against chloramphenicol.

A third nucleic acid molecule is combined with a second nucleic acid molecule, whereby the second selection marker provided by the second nucleic acid molecule is a resistance mediating gene coding for an enzyme conferring resistance against a selecting agent distinct from the positive selecting agent and negative selection agent provided by the third nucleic acid molecule. In a preferred embodiment of the invention, the positive selection marker is a gene conferring resistance against kanamycin, and the negative selection marker is a gene conferring sensitivity to streptomycin, and the second resistance marker provided by the second nucleic acid confers resistance against chloramphenicol but not kanamycin or streptomycin. It is know in the art, that several genes mediate resistance to more than one selection agent, especially if the selection agent is an antibiotic (Tenorio C, Zarazaga M, Martinez C, Torres C. J. Clin. Microbiol. 39:824-825, 200169), limiting the possible combinations of selection markers in the third and second nucleic acid.

A further embodiment of this invention is the combination of a third nucleic with a second nucleic acid, and the third nucleic acid molecule provides a positive selection marker and a negative selection marker, whereby the activity of the negative selection marker is controlled by a nucleic acid sequence provided by the third nucleic acid, which has regulatory activity in a prokaryote. In a preferred embodiment the nucleic acid sequence controlling the activity of the negative selection marker is a promoter, and in a more preferred embodiment a prokaryotic promoter. In an even more preferred embodiment the promoter is the *E.coli* galactokinase promoter.

One embodiment of the invention is the combination of a third nucleic acid molecule comprising a bacterial nucleotide sequences for conditional replication, with a second nucleic acid molecule comprising a further nucleotide bacterial sequences for single copy replication. Thereby the bacterial sequences for replication of the third nucleic acid molecule allow for conditional replication in special *E. coli* strains or in normal *E. coli* strains under specific conditions, whereby the bacterial cell provides all functions necessary. In a preferred embodiment, the bacterial nucleotide sequence unit in the third nucleic acid molecule contains the minimal ori of phage gR6K as conditional replicon, which can be maintained only in the presence of pi protein expression, and the sequences for replication of the second nucleic acid are based on the F-factor and allow for single copy maintenance in *E.coli* cells[65].

One embodiment of the invention is the combination of a third nucleic acid molecule providing the first part of a or the genome of a virus and a second nucleic acid molecule providing the second part of a or the genome of a virus, whereby the packaging signal may be provided by either the first or the second part of a or the genome of a virus. In a preferred embodiment the virus is an adenovirus, and in a more preferred embodiment the virus is AV5, and the packaging signal is the packaging signal of AV5 and provided by the third nucleic acid molecule.

One embodiment of the invention is the combination of a third nucleic acid molecule providing the first part of a or the genome of a virus and a second nucleic acid molecule providing the second part of a or the genome of a virus, whereby at least one terminal repeat sequence is provided by the third and one terminal repeat sequence is provided by the second nucleic acid. In a further embodiment of this invention one nucleic acid can provide all terminal sequences, however in this case the resulting nucleic acid will then contain a complete viral genome containing the bacterial nucleotide sequence unit of one of the nucleic acid molecules. In a preferred embodiment of this invention the terminal repeat sequence is the inverted terminal sequence (ITR) of a or the genome of a virus. In an even more preferred embodiment the ITR is from derived from an adenovirus, whereby the third nucleic acid molecule provides the left ITR, and the second nucleic acid molecule provides the right ITR. In a most preferred embodiment the ITR is the ITR from the human adenovirus type 5.

According to the invention the third and second nucleic acids can be combined and reacted in a host cell through their Frt recombination sites by action of a site-specific recombinase. The resulting nucleic acid molecule contains exactly one copy of a complemented complete virus genome, which can be released by restriction digest with the first and second restriction enzyme, whereby the restriction site are being absent in the transcription unit provided by the third nucleic acid, the first part of a or the genome of a virus, and the second part of a or the genome of a virus. In a more preferred embodiment of this invention the restriction site is chosen from a group of restriction sites absent in the genome of an adenovirus, and in an even more preferred embodiment the restriction site is selected from a group of sites absent in human adenovirus type 5 (AV5) comprising *Abs*I, *BstB*I, *Pac*I, *Psr*I, *SgrD*I, and *Swa*I.

A preferred embodiment of the invention is the combination of a third nucleic acid molecule with a second nucleic acid, whereby the virus is an adenovirus, and in a more preferred embodiment the virus is the human adenovirus type 5.

According to the method provided in this invention the recombination product of a first with a second nucleic acid molecule is a fourth, preferable circular, nucleic acid molecule comprising preferably the following elements: a bacterial nucleotide sequence unit comprising (i) bacterial sequences for single copy replication, and (ii) a nucleotide sequence providing for a second selection marker, the site-specific recombination site, the second part of a genome of a virus, optionally a second restriction site, a further site-specific recombination site, a bacterial nucleotide sequence unit comprising (i) bacterial nucleotide sequences for conditional replication, and (ii) a nucleotide sequence providing for first selection marker, the first part of a genome of a virus, preferably a transcription unit, and the first restriction site.

In this invention a method for the generation of a fourth nucleic acid molecule coding for a virus is disclosed, whereby a first nucleic acid molecule and a second nucleic acid molecule are provided and combined and allowed to react so that site-specific recombination occurs and a site-specific recombination product forms. Preferably the site-specific recombination occurs in a host cell, whereby more preferably the host cell is *E.coli.* The recombination product may be optionally selected, and contains a copy, preferably a single copy of a or the genome of a or the virus, whereby the genome of a or the virus is a complemented and complete virus genome, and upon optional cleavage of with the first and second restriction enzyme the resulting nucleic acid can be transfected into a permissive cell line and a or the virus generated and propagated in this permissive cell line. In a most preferred embodiment the virus is an adenovirus, and in even more preferred embodiment the virus is the human adenovirus type 5.

In one embodiment of the invention a method for the generation of a nucleic acid molecule coding for a virus is provided, whereby a first nucleic acid molecule and a second nucleic are combined and allowed to react in a host cell so that site-specific recombination occurs and a site-specific recombination product forms. The host cell allows selection of the reaction product, whereby the host cell genome is deficient for parts of or the F-factor which allows single copy replication of the second nucleic acid, and whereby the host cell is deficient for expression of factors that allow conditional replication of the first nucleic acid. This allows selection against any non reacted first nucleic acid molecule in the host cell. In a preferred embodiment the host cell is a prokaryotic host cell, and more preferably E*.coli.* In an even more preferred embodiment the host cell is selected from a group comprising K12-derived E*.coli* host cells including DH10B among others known in the art.

In a further embodiment of the invention a method for the generation of a nucleic acid molecule coding for a virus is provided, whereby a first nucleic acid molecule and a second nucleic acid molecule are provided. The first and second nucleic acid molecules are combined and allowed to react in a host cell so that site-specific recombination occurs and a site-specific recombination product forms, whereby the reaction product does not need to be selected and contains one complete complemented genome of a or the virus. According to the method provided the host cell is an eukaryotic host cell, and the first and second nucleic acid molecules preferably are linear nucleic acid molecules, preferably upon cleavage with the first and second restriction enzymes, and whereby the eukaryotic host cell preferably is a permissive host cell, and even more preferably the host cell is selected from a group allowing replication of the human adenovirus type 5 comprising 293, 911, Per.C6, N52.E6 cells among others known in the art. If the adenovirus is other than the human adenovirus type 5, a permissive host cell is defined as such, that it will allow replication of this virus. It is know in the art that linear nucleic acid molecules which contain one complete adenovirus genome are replicable in a permissive host cell. The efficiency of virus replication is optimal if the ends of the adenovirus genome are exactly ending with the ITRs of the adenovirus, and even more efficient if the terminal protein is attached to the left end of the adenovirus genome, however, this is not a prerequisite for adenovirus replication in a permissive cell, since nucleic acid molecules containing a complete adenovirus genome with sequences extending the ITRs will also be replicated16,24,25.

In this invention a method for the generation of a fifth nucleic acid molecule coding for a virus is provided, whereby a third nucleic acid molecule and a second nucleic acid molecule are combined and allowed to react so that site-specific recombination occurs and a site-specific recombination product forms, and whereby a reaction product is generated wherein the number of recombination events is limited to one. Preferably the site-specific recombination occurs in a host cell, whereby more preferably the host cell is E*.coli,* and whereby the selection of the recombination product is performed by selecting the host cell(s) which harbor the recombination product providing the positive selection marker of the third nucleic acid molecule, the negative selection marker of the third nucleic acid molecule, and the second selection marker of the second nucleic acid molecule, and whereby the host cell is not sensitive to the negative selection marker. According to this method, a host cell is used that is not sensitive to the negative selecting agent, whereby preferably the negative selecting agent is streptomycin. In a preferred embodiment the host cell is E*.coli,* expresses a mutant form of the *rpsL* gene conferring resistance to streptomycin. The host cells can thus be selected from a group of E*.coli* cells expressing the mutant form of the *rpsL* gene, and preferably the host cells are selected from group comprising DH10B among others know in the art. In a preferred embodiment, the selecting agents used to select host cells harboring the reaction product are kanamycin for the positive selection marker, chloramphenicol for the second selection agent, and streptomycin as the negative selecting agent. The negative selection marker encodes the wild type form of *rps*L, whereby the resistance to streptomycin conferred by the host cells expressing the mutant *rpsL* is recessive if both the wild-type and mutant alleles of *rpsL* are expressed in the same host cell strain, resulting in sensitivity to streptomycin 74,75.

According to this method the third nucleic acid molecule providing a site-specific recombination site is combined with a second nucleic acid molecule providing a site-specific recombination site, and both nucleic acid molecules are allowed to react by site-specific recombination in the host cell, whereby the site-specific recombinase is provided by the prokaryotic host cell. Thereby the site-specific recombinase is provided preferably by the host cell, either as part of the genome a host cell, or as extrachromosomal element. In a preferred embodiment the host cell provides the site-specific recombinase as an extrachromosomal plasmid, whereby in a more preferred embodiment the site-specific recombinase is Flp and the plasmid is pCP20. According to this method the expression of a site-specific recombinase is controlled during the reaction, whereby the control of the expression can be achieved by various ways including the use of a inducible expression system such as the arabinose-inducible *AraC-PBAD* promoter to induce expression (Lee EC., et al. Genomics 73:56-65, 200169) without being limited to this. In a preferred embodiment the expression of the site-specific recombinase and the replication of this plasmid is controlled by temperature, whereby in an even more preferred embodiment expression Flp is controlled by a temperature sensitive repressor from lambda phage, and replication of the plasmid controlled by a temperature-sensitive origin of replication, and whereby the temperature-sensitive FLP expression plasmid pCP20 is used 55,72.

According to this method the selected reaction product resulting from a combination of a third nucleic acid molecule and a second nucleic acid molecule and the subsequent site-specific recombination reaction in a host cell comprises a complete virus genome, whereby the selected nucleic acid molecule harbors a first restriction site, and a second restriction site, preferably being absent in an adenovirus genome, and more preferably being selected from a group of restriction sites comprising *Abs*I, *BstB*I, *Pac*I, *Psr*I, *SgrD*I, and *Swa*I, being absent in the genome of a human adenovirus type 5. According to this method the third and the second nucleic acid molecules can be introduced separately into the prokaryotic host cell, whereby in a preferred embodiment the host cell harbors the second nucleic acid and is made competent for transformation with a third nucleic acid molecule using state-of-the- art techniques. According to the method provided, the selected recombination product comprises a complete complemented virus genome, which can be released from the reaction product upon restriction digest, preferably upon restriction digest with one or more restriction enzymes binding and cleaving the nucleic acid at the first and second restriction site, respectively. This method comprises a further transfection step, whereby the released virus genome is introduced into a permissive eukaryotic host cell using standard methods, preferably using the transfection reagent polyethylenenimine (PEI) or the calcium phosphate transfection method, among other methods known in the art. Transfection of the complete complemented virus genome into the eukaryotic permissive host cell yields a replication competent adenovirus vector, whereby the vector is used for gene transfer, vaccine or any therapeutic applications.

In this invention a method is provided for the generation of a library of nucleic acid molecule coding for a virus genome, whereby a plurality of third nucleic acid molecules and a second nucleic acid molecule is provided, whereby the plurality of third nucleic acid molecules and a plurality of second nucleic acid molecules are combined and allowed to react so that site-specific recombination occurs and a plurality of nucleic acid molecules is formed, whereby the plurality in its totality forms a library, and whereby the library consists of a plurality of fifth nucleic acid molecules, comprising the following elements of the second nucleic acid molecule: the bacterial nucleotide sequence unit comprising (i) bacterial nucleotide sequences for single copy replication, and (ii) a nucleotide sequence unit providing for a second selection marker, the site-specific recombination site, the second part of a genome of a virus, and the restriction site which is referred to as second restriction site, and the following elements from a third nucleic acid molecule comprising optionally the first part of a genome of a virus the nucleotide sequence, preferably a genomic nucleotide sequence, or a transcription unit, the regulatory nucleic acid sequence which has regulatory activity in a prokaryote, the site-specific recombination site, the nucleotide sequence providing for a negative selection marker, the bacterial nucleotide sequence unit comprising (i) bacterial nucleotide sequences for conditional replication and (ii) a nucleotide sequence providing for a positive selection marker, and the first restriction site.

One embodiment of the invention discloses a method for the generation of a plurality of or a library of fifth nucleic acid molecules each coding for a complete complemented virus genome, whereby a plurality of third nucleic acid molecules and the second nucleic acid molecule are combined and reacted according to the method provided in a prokaryotic host cell, and whereby the host cell is preferably a bacteria cell and can accept nucleic acids by either being electroporated or made chemically competent according to standard methods. In a preferred embodiment the bacterial host cell harbors the second nucleic acid molecule and accepts a third nucleic acid molecule by electroporation. In a more preferred embodiment the bacteria is *E.coli.*

According to the method provided the library of nucleic acid molecules does not need to be screened for multiple recombined products. The method is such, that host cell harboring the recombination product confers resistance to the second selection marker and the positive selection marker, and is not sensitive to the negative selection agent without expression of the negative selection marker. Moreover, according to the method provided, conditional replication of the third nucleic acid molecule ensures that the host cells will only replicate the reaction product avoiding any unwanted contaminating nucleic acid molecule. In a preferred embodiment, the positive selection marker confers resistance to kanamycin, the negative selection maker confers sensitivity to streptomycin, and the second selection marker provided by the second nucleic acid confers resistance to chloramphenicol

According to the method provided in this invention, a method for the generation of a library of nucleotide sequences comprising a plurality of individual nucleotide sequences is provided, whereby the library is represented by a plurality of virus genomes, each containing a single one of the individual nucleotide sequences, whereby the nucleotide sequence is part of a transcription unit. In a preferred embodiment of the invention the nucleic sequence is a nucleic acid to be expressed, and the nucleotide sequence is present in the complete virus genome as a single copy. In a more preferred embodiment the virus is an adenovirus and the method provides a mean to construct a plurality of individual adenoviruses. In an even more preferred embodiment the adenovirus is the human adenovirus type 5. The resulting adenovirus virus library can be used for identification of gene functions, in screening applications, for the construction of an expression or genomic library, and for gene transfer.

According to the method provided, a plurality of complete complemented adenovirus genomes each containing a nucleotide sequence, whereby each complete complemented ' adenovirus genome can be released by restriction digest with the first and second restriction enzyme, generating a viable replication-competent adenovirus upon transfection into a permissive host cell. The permissive cell line used for reconstitution and propagation of the adenovirus is able to complement for all the deleted regions in cis or trans. In case of the first generation AV5 virus genome the permissive cell line complementing for the E1 function may be used, whereby the cell line may be chosen from a group comprising 293, 911, Per.C6, N52.E6, among others known in the art. Other cell lines providing additional components of the viral genome in trans may be used as well if required. Moreover, transient expression of said deleted components may also be used to allow virus reconstitution and replication.

In one embodiment of the invention a kit is provided, comprising optionally a package insert, and, in (a) suitable container(s), at least a first nucleic acid molecule, a second nucleic acid molecule, optionally a permissive cell line providing the site-specific recombinase, a combination of the first nucleic acid molecule and the second nucleic acid, a third nucleic acid molecule, a combination of the third nucleic acid molecule and the second nucleic acid molecule, a fourth nucleic acid molecule, a fifth nucleic acid, a plurality of a fourth nucleic acid molecule, a plurality of a fifth nucleic acid molecule, or a plurality of individual adenoviruses.

In a further embodiment of the invention, the kit comprises a first nucleic acid molecule, parts of or a second nucleic acid, preferably a linear form of the second nucleic acid, and a permissive cell line providing the site-specific recombinase. In a preferred embodiment the part of the second nucleic acid comprises at least the site-specific recombination site and the second part of a genome of a virus. According to the method provided in this invention a nucleic acid or library of said nucleic acid can be constructed comprising a nucleotide sequence or library of nucleotide sequences, each in a complete complemented virus genome, whereby the nucleic acid molecules are ready to be used and can be directly introduced into said permissive cell line in order to generate an adenovirus, or plurality of individual adenoviruses, whereby in a preferred embodiment the cell line is 293 and the recombinase is the wild type Flp recombinase, and the adenovirus the human adenovirus type 5.

### The invention will now be described by reference to the following figures and examples which are merely illustrative and are not to be considered as a limitation of the scope of the invention.

- FIG. 1: is a diagrammatic representation showing a method for constructing a first generation adenovirus genome, whereby a first nucleic acid identical or similar to pDonorSir1, and a second nucleic acid molecule identical or similar to pBACSir1 are combined and reacted through their recombination sites forming a fourth nucleic acid (pRAB) as recombination product which can be selected and contains exactly one copy of a complete complemented virus genome. Bacteria harboring the fourth nucleic acid containing the first and second selection marker and can be selected with the first and second selecting agent. The composition of a fourth nucleic acid molecule resulting from a single recombination event (pRAB1x) is given in figure 1A, the composition of a fourth nucleic acid molecule resulting from a double recombination event (pRAB2x) is given in figure 1B.
- FIG. 2A: illustrates the composition of DNA from recombinant adenovirus BACs analyzed by restriction digest with a restriction enzyme, and the composition of the DNA from two reconstituted complemented first generation adenovirus viruses generated from these BACs using the disclosed method of example 1 und example 2
- FIG. 2B: illustrates the composition of DNA from recombinant adenovirus BACs analyzed by restriction digest with a restriction enzyme obtained after site-specific recombination in E*.coli* using the disclosed method of example 3
- FIG. 3: shows the nucleotide sequence of SEQ.ID. No. 1.
- FIG. 4: shows the nucleotide sequence of SEQ.ID. No. 2.
- FIG. 5: shows the nucleotide sequence of SEQ.ID. No. 3
- FIG. 6: shows the nucleotide sequence of SEQ.ID. No. 4.
- FIG. 7: shows the nucleotide sequence of SEQ.ID. No. 5.
- FIG. 8: is a diagrammatic representation showing a method suited for constructing a BAC library of genomic fragments, whereby two nucleic acids are combined and reacted through their recombination sites forming a recombination product which can be selected whereby the number of recombination events is limited to one according to the disclosed method of example 3. A third nucleic acid identical or similar to pDonorSir2, and a second nucleic identical or similar to pBACSir2 acid are combined and reacted through their recombination sites forming a fifth nucleic acid as recombination product which can be selected and contains exactly one copy of a complete complemented virus genome (Fig.8A). Bacteria harboring the fifth nucleic acid containing the positive, the negative selection marker from the third nucleic acid, and the second selection marker, can be selected. The schematic composition of a fifth nucleic acid molecule resulting from a single recombination event (pRAB_RPSL1x) is given in figure 8A, the schematic composition of a fifth nucleic acid molecule resulting from a double recombination event ((pRAB_RPSL2x)) is given in figure 8B.

- FIG. 9: shows the nucleotide sequence of SEQ.ID. No. 6
- FIG. 10: shows the nucleotide sequence of SEQ.ID. No. 7
- FIG. 11: shows the nucleotide sequence of SEQ.ID. No. 8
- FIG. 12: shows GFP expressing adenovirus vectors obtained after direct transfection of linearized forms of the first and the second nucleic acid in 293 cells expressing the site-specific recombinase Flp using the disclosed method of example 5
- FIG. 13: illustrates the selective inhibition of growth of bacteria in medium containing the negative selecting agent at different concentrations harboring a double recombined BAC (pRAB_RPSL2x) according to the disclosed method of example 3.
- FIG.14: shows the nucleotide sequence of SEQ.ID. No. 9
- FIG.15: shows the nucleotide sequence of SEQ.ID. No. 10
- FIG.16: shows the nucleotide sequence of SEQ.ID. No. 11
- FIG.17: shows the nucleotide sequence of SEQ.ID. No. 12
- FIG.18: shows the nucleotide sequence of SEQ.ID. No. 13

### Brief Description of the Figures

FIG. 1 Diagrammatic representation showing a method for constructing a first generation adenovirus genome, whereby two nucleic acids are combined and reacted through their recombination sites forming a recombination product corresponding to a fourth nucleic acid molecule according to a disclosed method of example 1 and 2. The fourth nucleic acid generated can be selected and contains exactly one copy of a complete complemented virus. genome. The reaction product can be cleaved optionally with a first and a second restriction enzyme in order to release a complete virus genome that can be replicated in a permissive cell. According to example 1 a first nucleic acid vector identical or similar to pDonorSir1 (Seq. ID. No. 1) contains a minimal Frt34 recombination site (SEQ.ID. No. 7) derived from the wild type Frt site, a bacterial nucleotide sequence comprising (i) bacterial nucleotide sequences for conditional replication (OriR6K) and a nucleotide sequence providing for a first selection marker conferring a host cell resistance against kanamycin (KnR), a first restriction site (RS1), a first part of a genome of a virus containing the left ITR of an adenovirus genome (ITRleft) and the packaging signal ES, and a transcription unit (TU). The second nucleic acid is a BAC vector identical or similar to pBACSir1 (Seq. ID NO. 13) or pBACSir2 (Seq. ID No.2), comprising a wild type Frt48 recombination (SEQ.ID. No. 8) site, a second part of the genome of an adenovirus (Ad) comprising the right ITR of art adenovirus genome (ITRrigth), a second restriction site (RS2), and a bacterial nucleotide sequence unit comprising (i) bacterial nucleotide sequences for single copy replication (F'ori), and (ii) a nucleotide sequence providing for a second selection marker conferring a host cell resistance against chloramphenicol (CmR). Both nucleic acid molecules are reacted through their recombination sites in a bacterial host cell in the presence of Flp recombinase. The resulting fourth nucleic acid molecules are recombinant adenovirus BACs (pRABs) either consisting of single recombined products, or multiple recombined products. In figure 1A the schematic composition of a single recombination product (pRAB1x) (Seq.ID. No. 4) is given. In figure 1B the schematic composition of a double (pRAB2x) recombination product (Seq.ID. No. 5) is given. Upon digestion of the DNA of a fourth nucleic acid with the first and the second restriction enzyme, a complete adenovirus genome is released from the pRABs containing the left and the right ITR, the packaging signal, and the transcription unit (Fig. 1C). Viable first generation adenovirus vectors are obtained in 97% of the cases if the DNA of a fourth nucleic acid obtained according to the disclosed method in example 1 and digested with the first and second restriction enzyme according to the disclosed method in example 2 is transfected into permissive 293 cells.

FIG. 2A illustrates the composition of two reconstituted first generation adenovirus viruses obtained using the method disclosed in example 1. The DNA of two types of reaction products are pRAB1x and pRAB2x, resulting from single and double insertion of pDonorSir1 into pBACSir2, whereby pDonorSir1 is identical to the deposited organism at the DSMZ according to the Budapest treaty with the accession number DSM 23753, and whereby pBAcSir1 is identical to the deposited organism at the DSMZ according to the Budapest treaty with the accession number DSM 24298. The respective reaction products were isolated from a growing culture of DH10B bacteria according to standard protocols, and characterized by restriction digest with *Xho*I (FIG.2A, lanes 3-4). In lane 1 a nucleotide length marker was loaded, providing reference fragments with defined length between 1 and 10 kb. In lane 2 the restriction pattern of the recombinant adenovirus BAC vector pRABref (Seq. ID. No. 3) is given as a reference. The in silico generated *Xho*I restriction pattern of pRABref is as follows: 14.5kb, 10.274kb, 7.403kb, 2.466kb, 1.445kb, and 0.595kb. Analysis of the single recombination product RAB1x (lane 3) yields a characteristic additional pair of bands
of 6.266kb and 4.187kb length, respectively. The *in silico* generated pattern for digestion with *Xho*I of the single recombined reaction product RAB1x (Seq. ID. No. 4) is as follows: 14.5kb, 10.274kb, 6.266kb, 4.187kb, 2.466kb, 1.445kb, and 0.595kb. In case of the double recombined product, a third additional band of 3.05kb appears in the (lane4). The in silico generated pattern for digestion with *Xho*I of the double recombined reaction product RAB2x (Seq. ID. No. 5) is as follows: 14.5kb, 10.274kb, 6.266kb, 4.187kb, 3.05kb, 2.466kb, 1.445kb, and 0.595kb. In a further experiment the RAB1x and RAB2x DNA was isolated and cut with *PacI* restriction enzymes (corresponding to RS1 and RS2 in figure 1, respectively), and transfected into permissive 293 cells. Figure 2A shows the restriction pattern of virus DNA isolated from 293 cells transfected with PacI-restricted RAB1x (lane 6) and RAB2x (lane7). The *in silico* generated pattern for digestion with *XhoI* of the viral DNA obtained from both reconstituted recombinant adenoviruses, RAB1x and RAB2x respectively, is as follows: 14.5kb, 8.499kb, 3.365kb, 2.466kb, 1.445kb, and 0.595kb. The restriction fragment pattern is identical for both viruses since the identical complete complemented adenovirus genome is liberated from the pRABs upon digestion with PacI. The restriction pattern of RAB1x or RAB2x was compared to an empty adenovirus, being essentially the same as RAB1x and RAB2x, but lacking the transcription unit (Fig.2, lane 5). The *in silico* restriction fragment pattern upon digestion with *XhoI* of the adenovirus DNA isolated form RAB1x or RAB2x, respectively, is as follows: 14.5kb, 8.499kb, 3.365kb, 2.466kb, 1.445kb, and 0.595kb.

FIG. 2B shows the restriction fragment analysis of the recombination products between pDonorSir2 and pBACSir2 obtained according the method disclosed in example 3, whereby pDonorSir2 is identical to the deposited organism at the DSMZ according to the Budapest treaty with the accession number DSM 23754, and whereby pBACSir2 is identical to the deposited organism at the DSMZ with the accession number according to the Budapest treaty DSM 24299. Selection of recombination products took place onto agar plates which contained kanamycin (25µg/ml) chloramphenicol (25µg/ml), and streptomycin sulphate (50µg/ml) as selecting agents. Under these conditions E*.coli* contained recombined *recombinant adenovirus BACs* (pRABs), and contained pRAB_RPSL_1x (Seq. ID. No. 11) as reaction product in 83 out of 88 analyzed reaction products. In only 2 out of 88 cases a double recombined reaction product pRAB_RPSL_2x (Seq. ID. No. 12) was obtained. Single colonies were picked from the selection plate, subcultured in liquid media containing chloramphenicol (25µg/ml) and BAC DNA from subsequent subcultures of the colonies containing pRABs was isolated and the integrity of the reaction products analyzed by restriction fragment analysis upon digestion with *Xho*I (figure 2B). All the 6 analysed recombinants analyzed, contained pRAB_RPSL_1x. To test the reliability of the method, the experiment was repeated and a further 82 clones were picked from the selection plates and characterized as above. We could find only 2 clones which contained multiple insertion products corresponding to pRAB_RPSL_2x marked with D in Fig. 2B; clones number #47 and #53, respectively. Further 7 BAC DNA preparations were contaminated by the parental vector pBACSir2 (marked with V, clone number #9,#17,#22,#39,#41,#62,#67), and 3 were recombination products resulted from rearrangements (marked with r, clone number #25,#46,#68. In a further experiment a total of 44BACs corresponding to a fifth nucleic acid were analyzed, whereby only 1 recombination product corresponded to pRAB_RPSL_2x and 43 of the 44 clones corresponded to pRAB_RPSL_1x. Altogether 126/132 BACs - corresponded to the single recombination product pRAB_RPSL_1x (95,5% of the recombination products analyzed) and multiple recombination was observed in 3/132 corresponding to 2,3% of the clones.

FIG. 3 shows the nucleotide sequence of SEQ.ID. No. 1.

FIG. 4 shows the nucleotide sequence of SEQ.ID. No. 2.

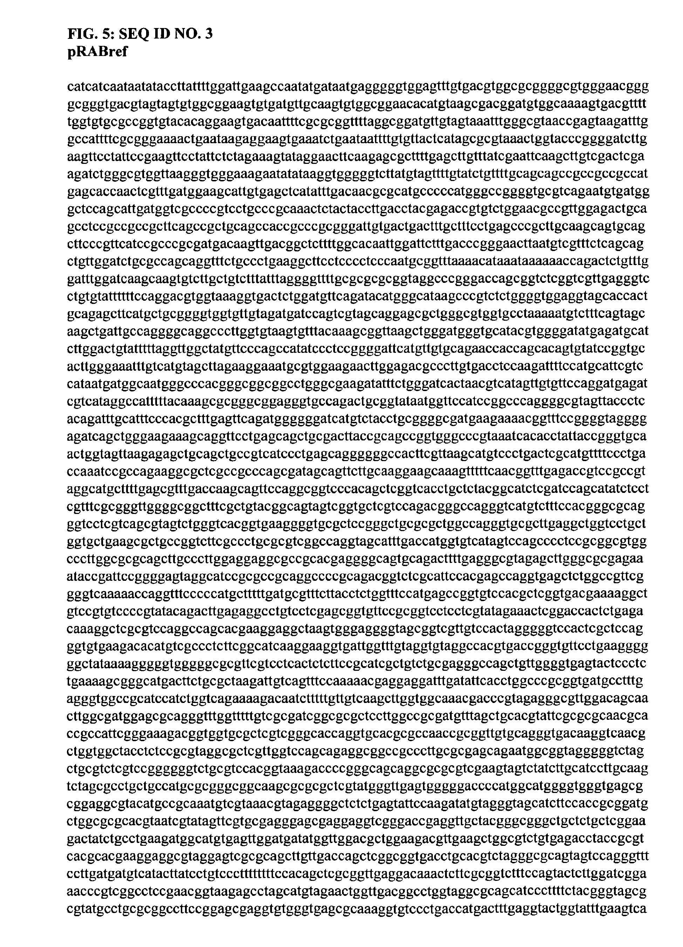

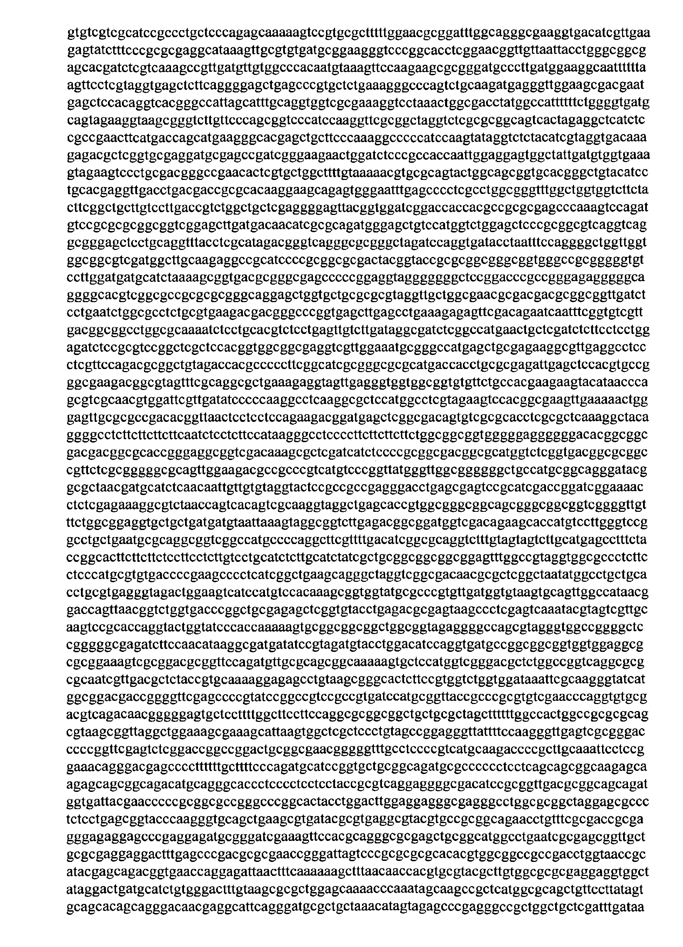

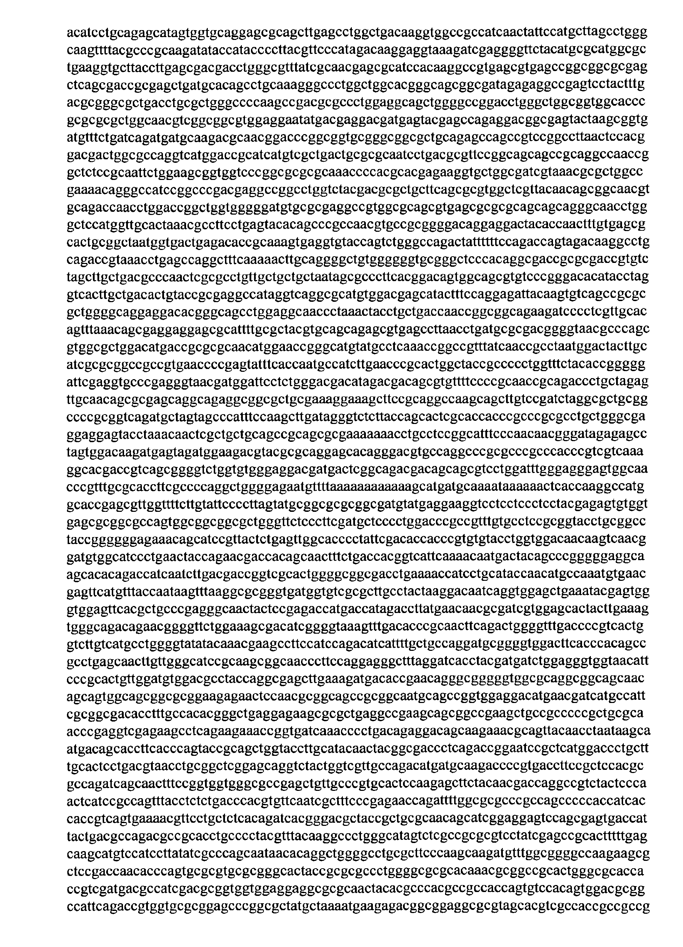

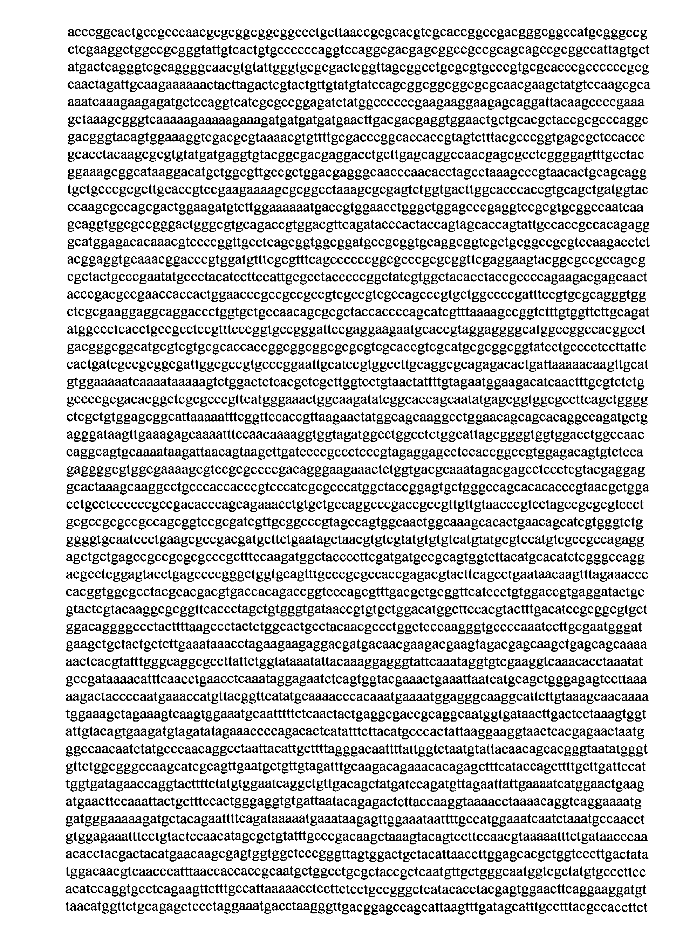

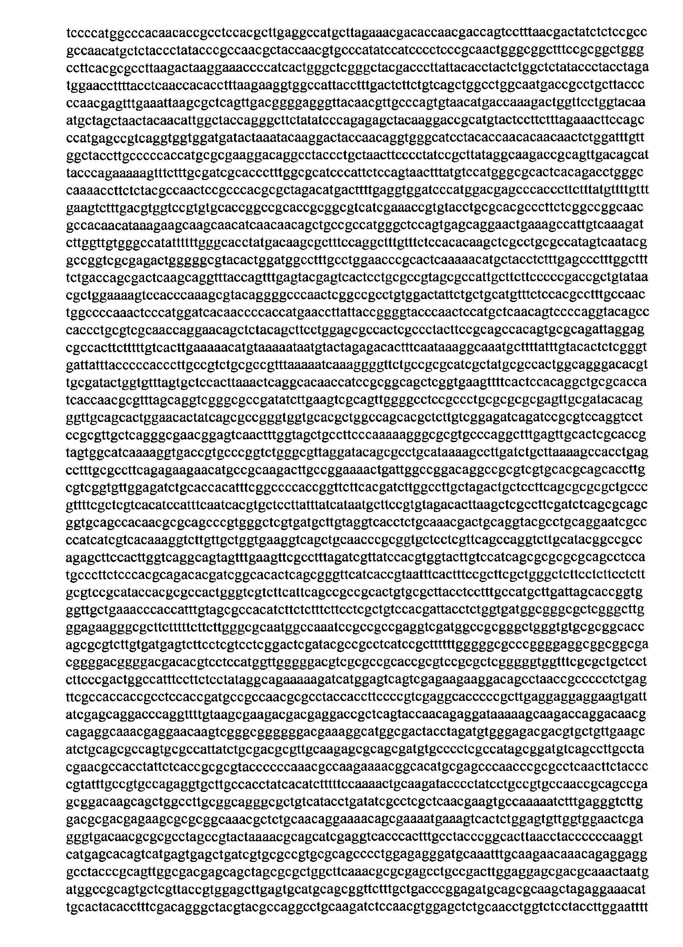

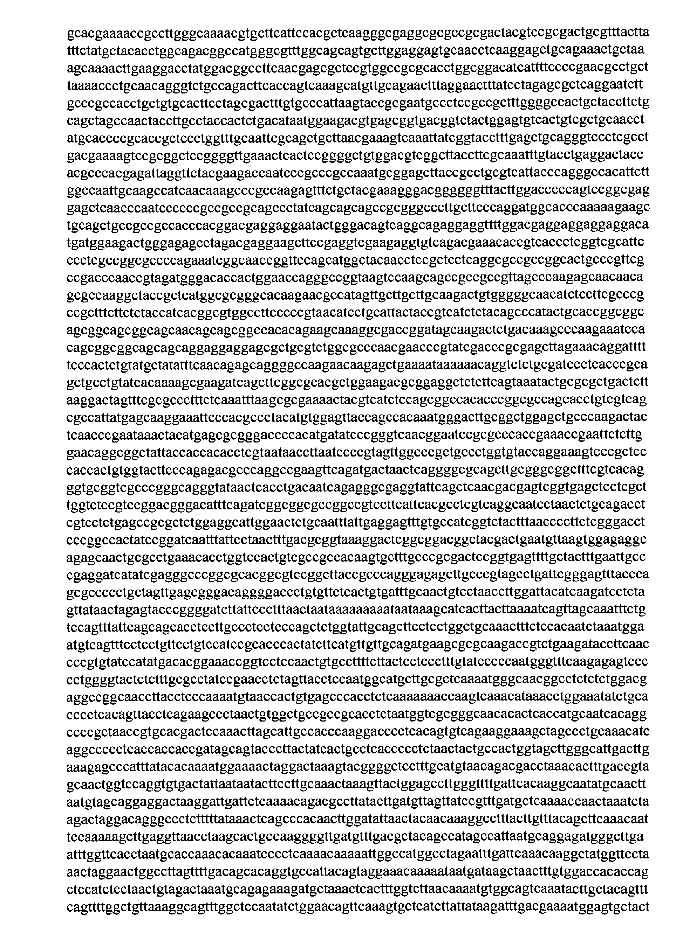

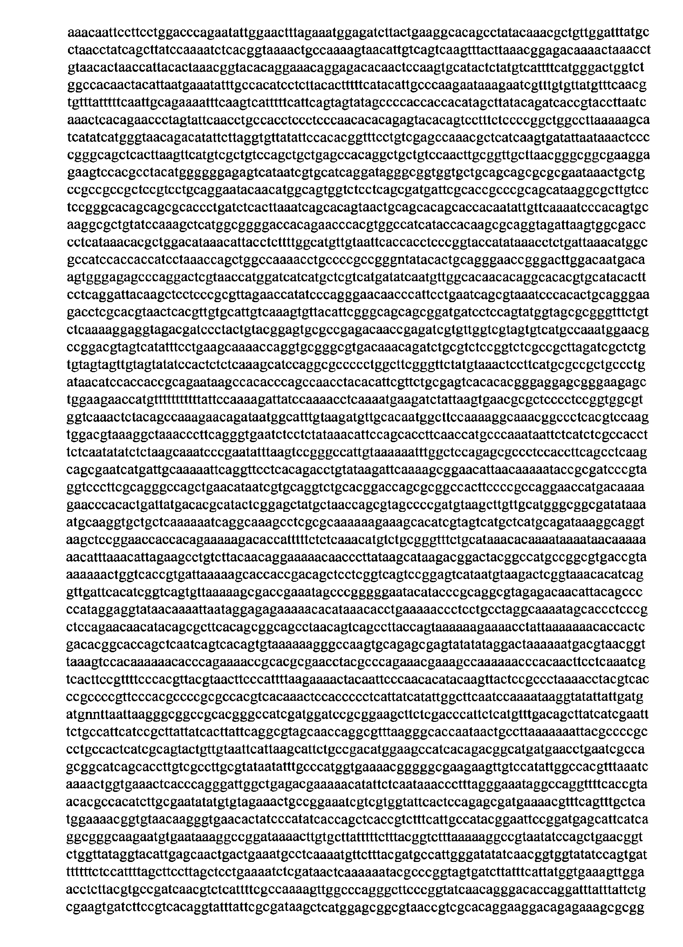

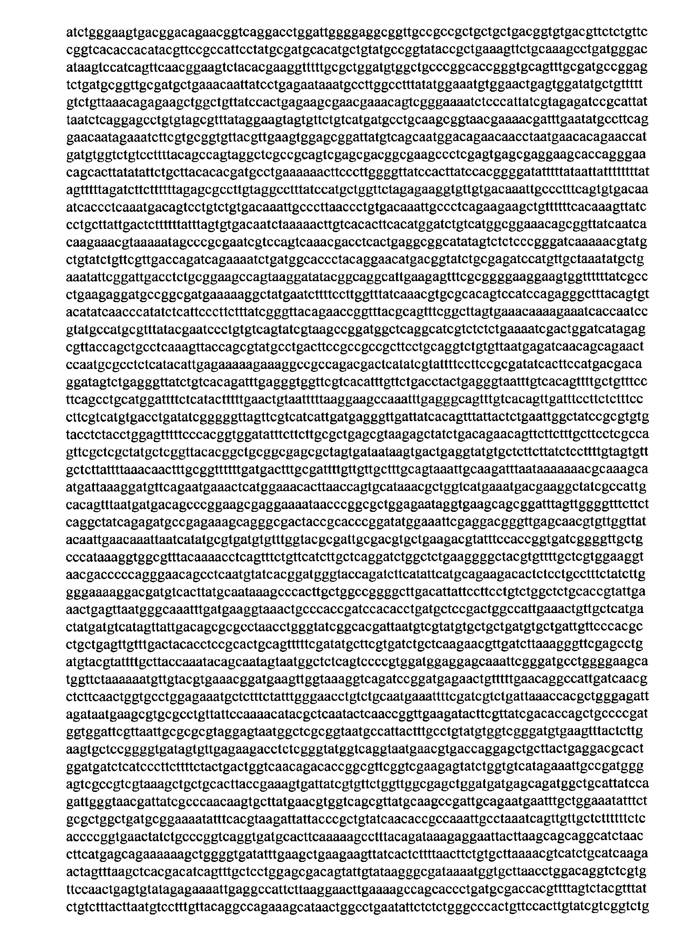

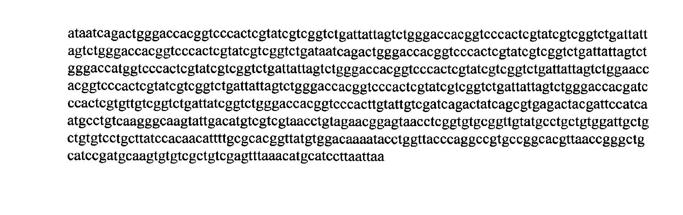

FIG. 5 shows the nucleotide sequence of SEQ.ID. No. 3.

FIG. 6 shows the nucleotide sequence of SEQ.ID. No. 4.

FIG. 7 shows the nucleotide sequence of SEQ.ID. No. 5.

FIG. 8 Diagrammatic representation showing the method disclosed in example 3 for constructing complemented complete adenovirus vector genomes, or a plurality or library of those. The recombination between a third nucleic acid molecule and a second nucleic acid molecule are combined and reacted through their Frt recombination sites forming a recombination product which can be selected, and whereby the number of recombination events is limited to one. According to example 3 a third nucleic acid molecule identical or similar to pDonorSir2 containing a prokaryotic promoter (PK promoter), a minimal Frt34 recombination site derived from the wild type Frt site, a negative selection marker (Rps1), a bacterial nucleotide sequence comprising (i) bacterial nucleotide sequences for conditional replication (OriR6K) and a nucleotide sequence providing for a positive selection marker conferring a host cell resistance to kanamycin (KnR), a first restriction site (RS 1), a first part of a genome of a virus containing the left ITR of an adenovirus genome (ITRleft) and the packaging signal ES, a transcription unit or gene of interest (GOI). The second nucleic acid, which is identical or similar to pBACSir2 (SEQ. ID. No. 2) comprises a wild type Frt48 recombination site (SEQ.ID. No. 8), a second part of the genome of an adenovirus comprising the right ITR of an adenovirus genome (ITRrigth), a second restriction site (RS2), and a bacterial nucleotide sequence unit comprising (i) bacterial nucleotide sequences for single copy replication (F'ori), and (ii) a nucleotide sequence providing for a second selection marker conferring a host cell resistance to chloramphenicol (CmR). Both nucleic acid molecules are reacted through their recombination sites in a bacterial host cell in the presence of Flp recombinase. Using the method disclosed, the resulting recombinant BACs predominantly (>95%) consist of single recombined products. In figure 8A the schematic composition of a single recombination product pRAB_RPSL_1x is given. In figure 8B the double recombination is depicted schematically. In the recombination product pRAB_RPSL_2x the prokaryotic promoter (PK Promoter) is in proximity to the open reading frame of the negative selection marker (Rpsl). This product is observed in less than 2,5% of the recombination products according to the disclosed method in example 3.

FIG. 9 shows the nucleotide sequence of SEQ.ID. No. 6.

FIG. 10 shows the nucleotide sequence of SEQ.ID. No. 7.

FIG. 11 shows the nucleotide sequence of SEQ.ID. No. 8.

FIG. 12 shows the reconstitution of complemented infectious adenovirus viruses in 293 Flp cells expressing Flp recombinase according to a disclosed method in example 5, whereby 293 Flp cells are identical to the deposited organism at the DSMZ with the accession number according to the Budapest treaty DSM ACC3077. 293Flp cells were transfected with a first nucleic acid molecule corresponding to pSirDonor1-EGFP (SEQ. ID No.9), and a second nucleic acid molecule corresponding to pBACSir2, whereby both nucleic acids were treated with *PacI* prior to transfection. After 3 days cultivation at 37°C under standard cell culture conditions comet-shaped fluorescent conglomerates of cells showing cytopathic effect (CPE) characteristic for productive adenovirus production in 293 cells were microscopically detected.

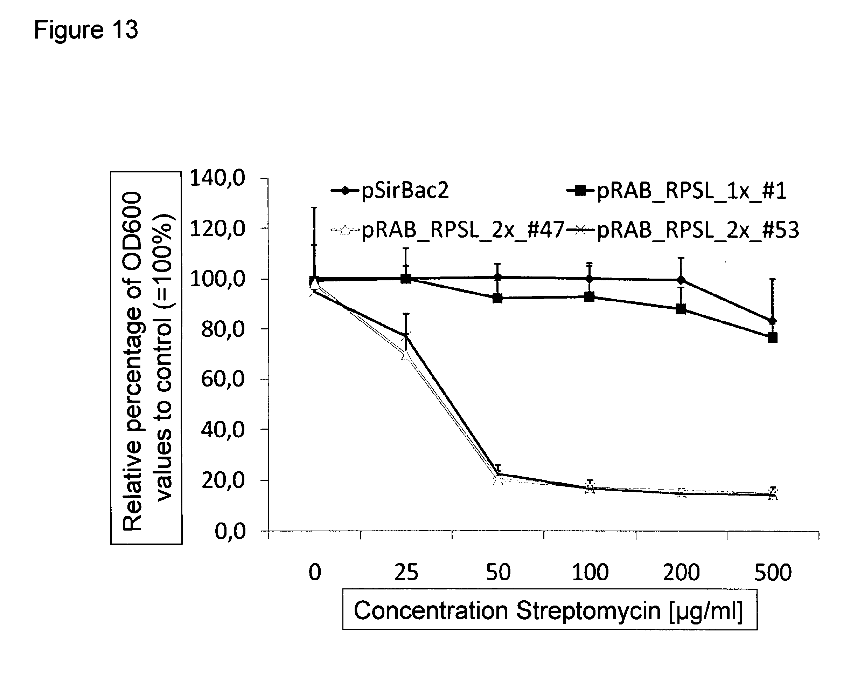

FIG. 13 shows the selective inhibition of the growth by streptomycin of E*.coli* DH10B cells carrying pRAB_RPSL_2x obtained from double recombination of pDonorSir2 with pBACSir2. Growth curves were generated from E.coli DH10B cells transformed with the empty vector pBACSir2, the single recombined pRAB_RPSL_1x-#1, or two double recombined adenovirus BAC vectors pRAB_RPSL_2x_#47, and pRAB_RPSL_2x_#53, respectively. Growth of bacterial cultures was done at different concentrations of streptomycin starting from a diluted saturated overnight culture as starting material, and monitoring of the OD600 over time. After the average of the OD600 of the replicate culture containing the control BAC vector pBACSir2 reached 0.8, usually 8 hours post inoculation, the OD600 values were measured and the optical density calculated and referenced to the average OD600 of the control culture which was set to 100%. The results of 5 independent experiments were plotted for each growth conditions and standard deviations of the relative optical densities within the plotted 5 experiments included as error bars. The E. coli clones carrying the control BAC vector pBACSir2 or the single recombined BAC vector pRAB_RPSL_1x grew well even in the presence of very high concentration (200µg/ml) of streptomycin. In contrast the growth of the clones carrying a double insertion of pDonorSir2 (pRAB_RPSL_2x #47) and pRAB_RPSL_2x_#53) was blocked by 50µg/ml streptomycin, some inhibition was detectable already in the presence of 25µg/ml streptomycin.

FIG.14 shows the nucleotide sequence of SEQ.ID. No. 9

FIG.15 shows the nucleotide sequence of SEQ.ID. No. 10

FIG.16 shows the nucleotide sequence of SEQ.ID. No. 11

FIG.17 shows the nucleotide sequence of SEQ.ID. No. 12

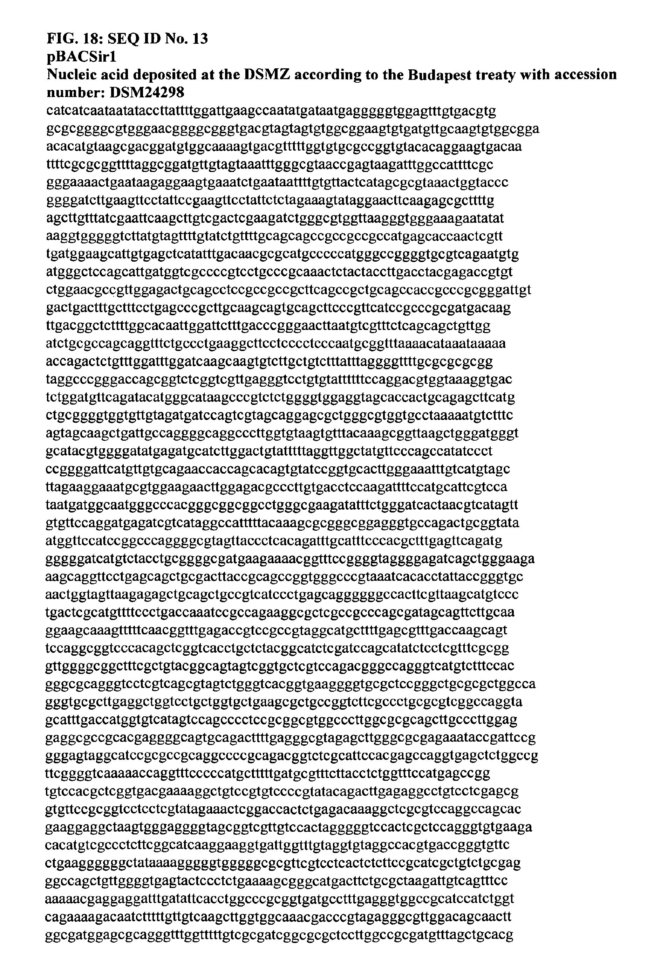

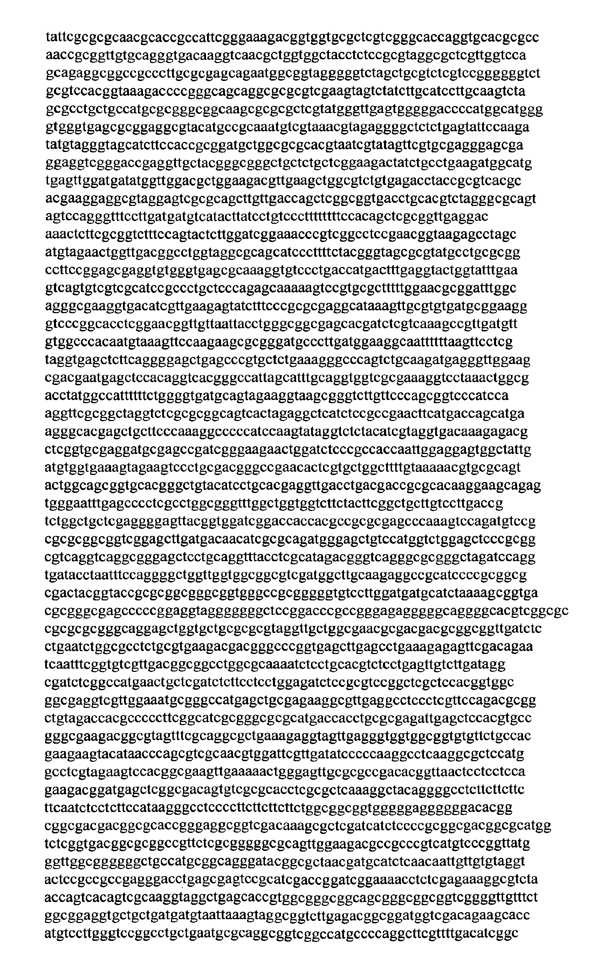

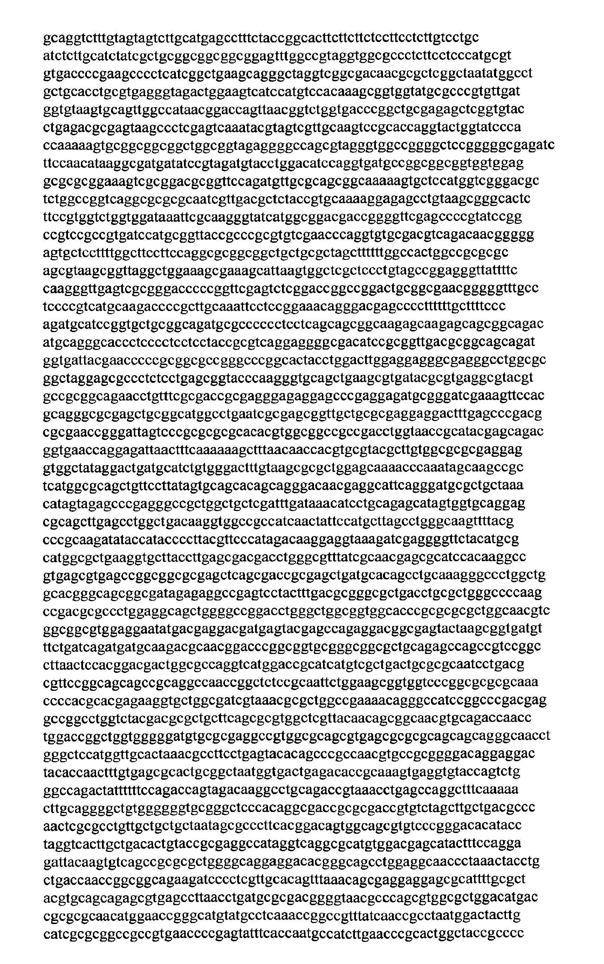

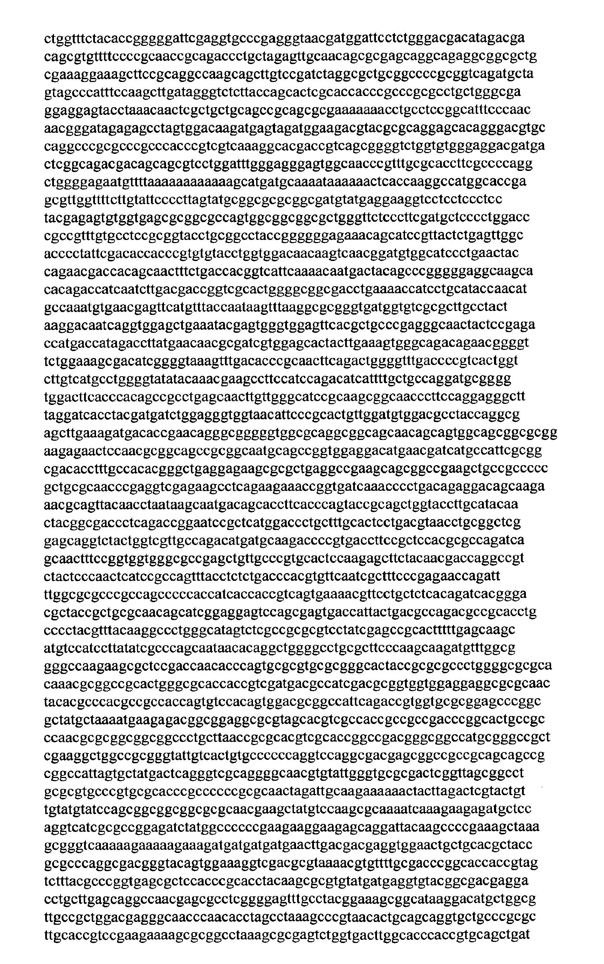

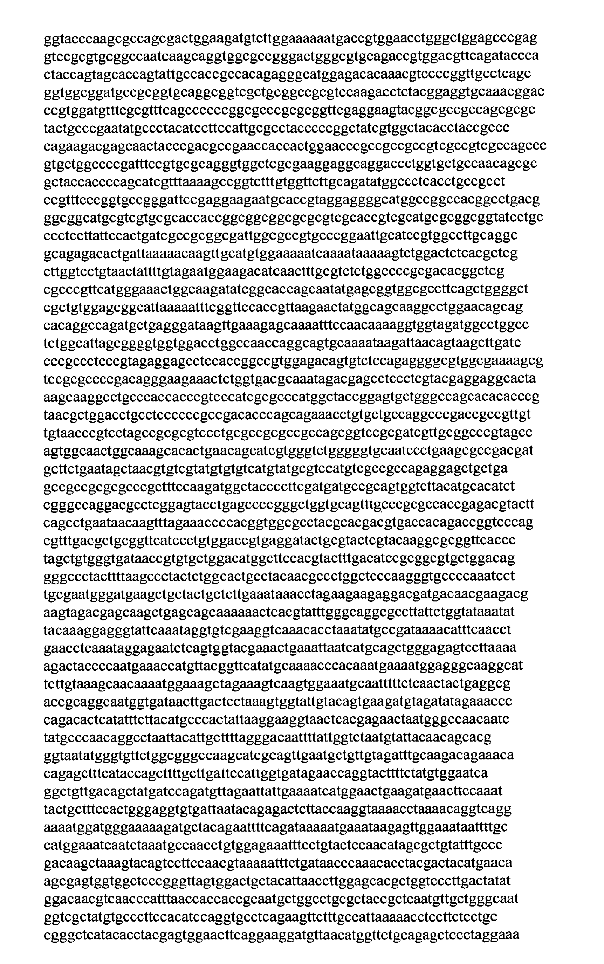

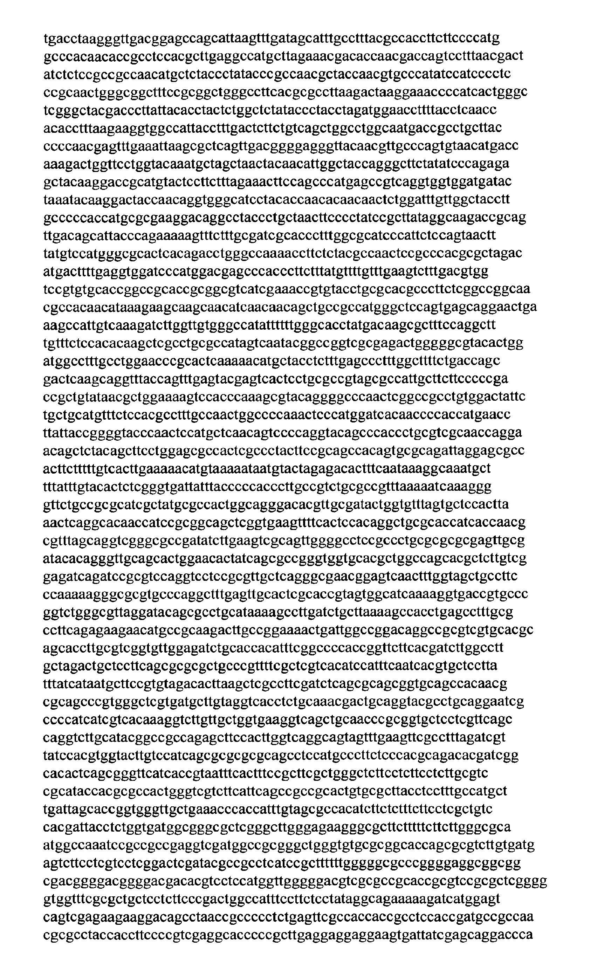

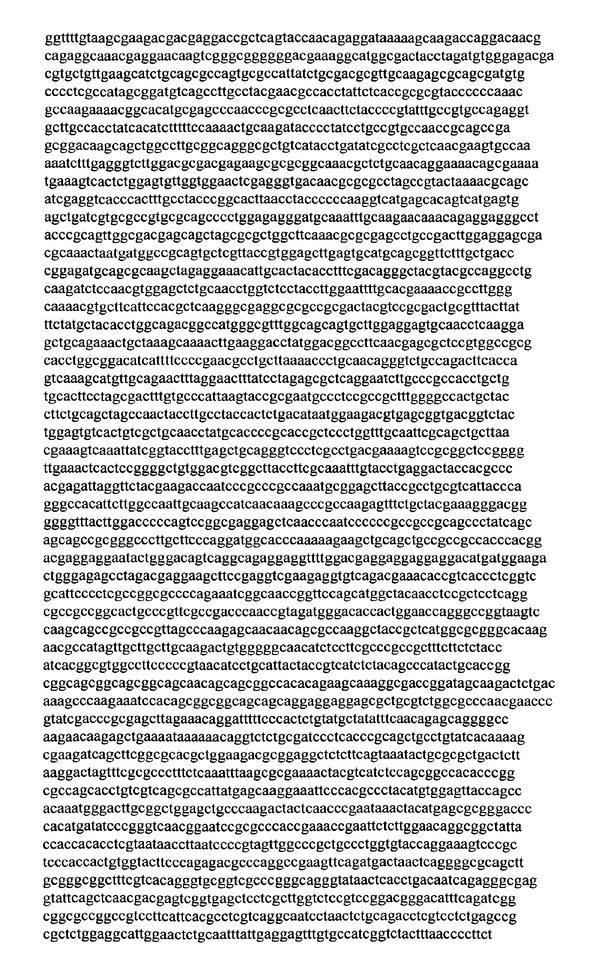

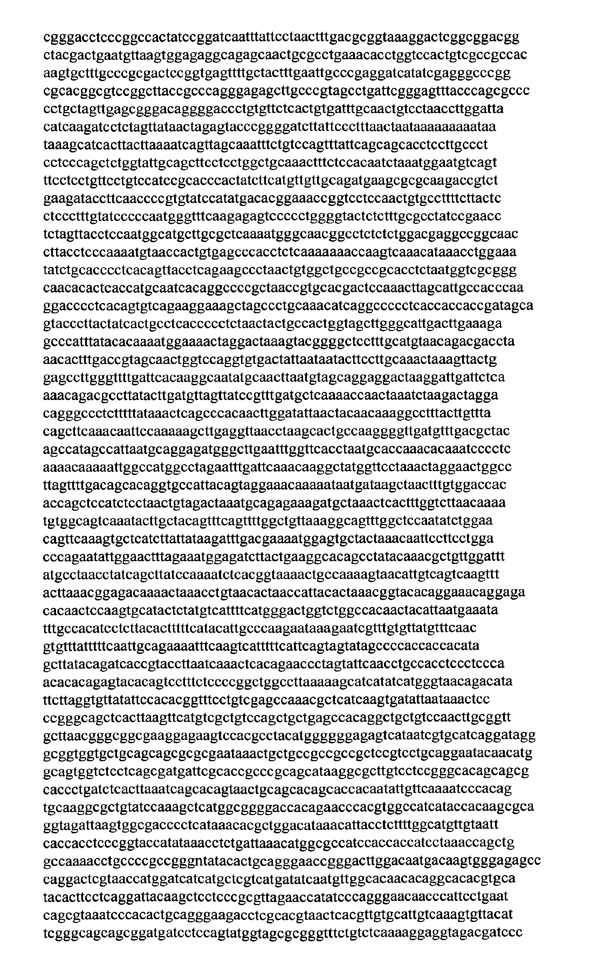

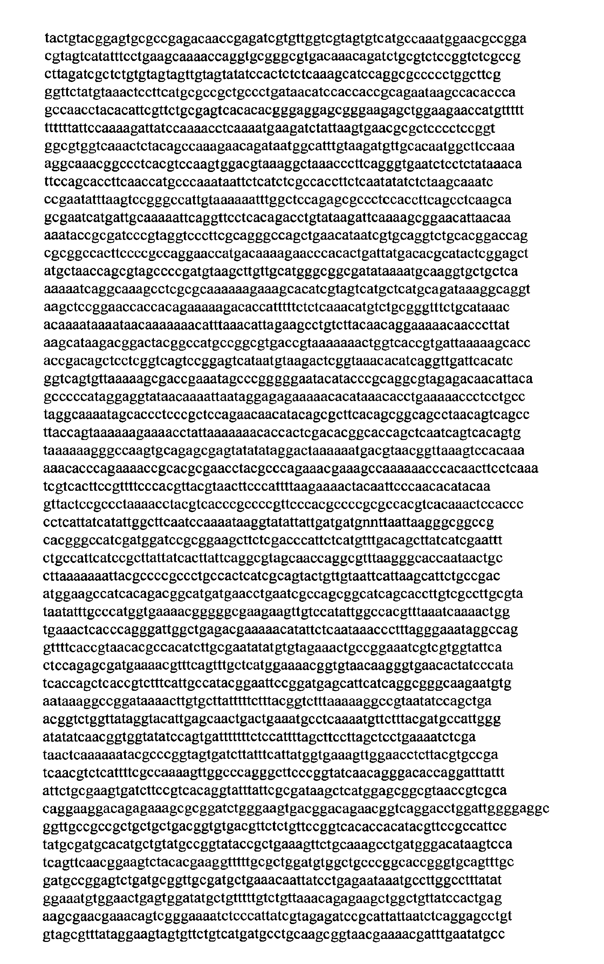

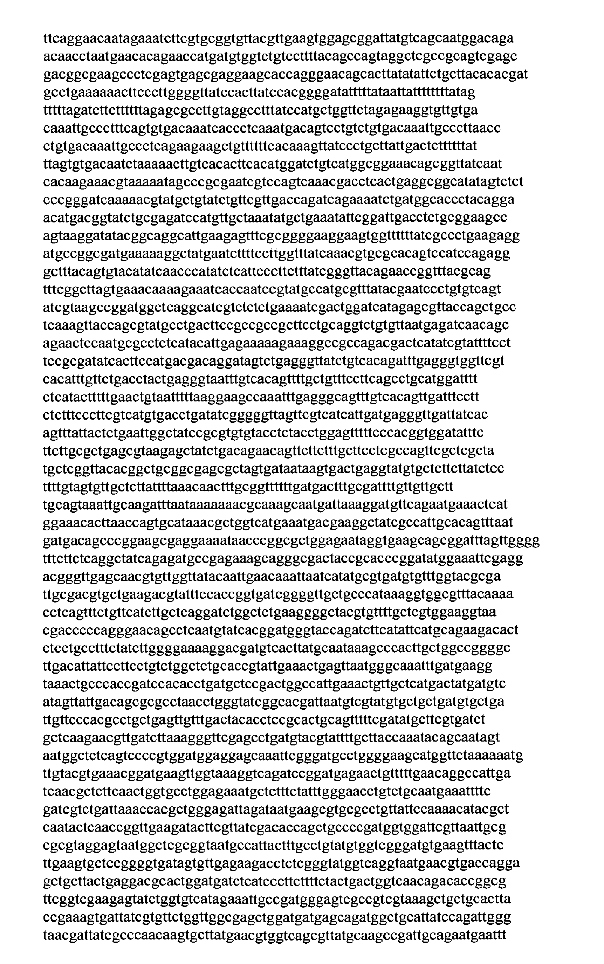

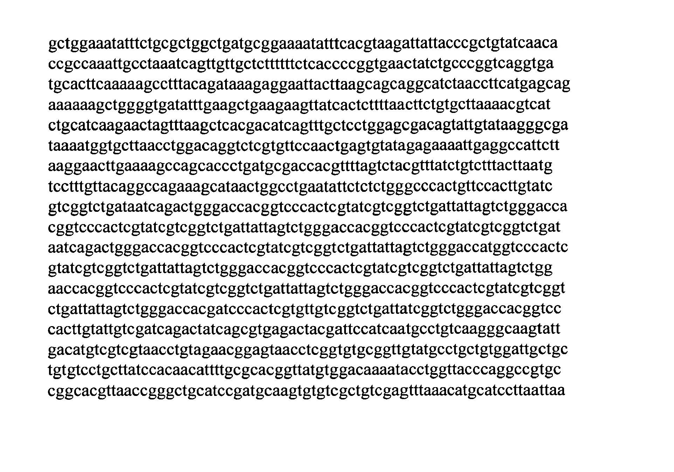

FIG.18 shows the nucleotide sequence of SEQ.ID. No. 13

### Examples

### Example 1: Construction of recombinant Adenovirus BACs using site-specific recombination in E.coli expressing Flp recombinase.

For construction of a recombinant adenovirus genome, a first nucleic acid pDonorSir1 and a second nucleic acid molecule pBACSir1 were combined and reacted in DH10B *E.coli* cells harbouring pBACSirland the plasmid pCP20 for conditional expression of FLP recombinase, whereby pDonorSir1 is identical to the deposited organism at the DSMZ with the accession number according to the Budapest treaty DSM 23753, and whereby pBACSir1 is identical to the deposited organism at the DSMZ according to the Budapest treaty with the accession number DSM24298, and whereby E*.coli* cells harbouring pBACSir1 and pCP20 are identical to the deposited organism at the DSMZ according to the Budapest treaty with the accession number DSM 23742. The plasmid pDonorSir1 was introduced into the DH10B *E.coli* cells by means of electroporation using a standard protocol. The nucleic acid molecule pBACSir1 is a derivative of the pKSO BAC vector (Messerle et al. Proc. Natl. Acad. Sci. U. S. A. 94:14759-14763, 199771) and contain the right part of the human adenovirus type 5 (AV5) genome deleted for the E1 region and the E3 region. The nucleic acid molecule pBACSir1 was maintained in *E.coli* DH10B (or equivalent *E.coli* K12-derived strains lacking the F- factor) harbouring a conditional expression system for expression of Flp. Here, in example 1, the DH10B cells harboured the adenovirus BAC pBACSir1, and the Flp recombinase was provided by the plasmid pCP20, which replication is controlled by a temperature-sensitive origin of replication (Bubeck A. et al., J. Virol. 78:8026-8035, 200472). DH10B cells harbouring pBACSir1 and the pCP20 were maintained at 30°C in the presence of ampicillin (50µg/ml) and chloramphenicol (25µg/ml). Next, these DH10B cells were electro-transformed with pDonorSir1 and cultured for 60 minutes at 42°C in the absence of any antibiotics. The expressed Flp induced site-specific recombination between FRT sites present on pDonorSir1 and pBACSir2, respectively. At the same time the elimination of Flp expression also started, since pCP20 cannot replicate in *E. coli* at elevated temperature. The transformed culture was plated onto agar plates which contained kanamycin (25µg/ml) and chloramphenicol (25µg/ml) as selecting agents. Under these conditions *E.coli* containing recombined *recombinant adenovirus BACs* (pRABs) were selected in which at least one pDonorSir1 plasmid had recombined with pBACSir1. DNA from growing cultures of DH10B cells containing pRABs was isolated and the integrity of the reaction products analyzed by restriction digestion *with Xho*I (Fig. 2A (lanes2-4). All the recombination products analyzed contained pRABs, either being single (pRAB1x) or multiply recombined products (pRAB2x).

### Example 2: Reconstitution of recombinant adenoviruses generated by site-specific recombination in E.coli expressing Flp recombinase

The two predominant types of BAC vectors obtained from site-specific recombination according to the disclosed method in example 1 were pRAB1x and pRAB2x, respectively. The pRABs generated by the Flp-recombination in DH10B cells contained one, and only one continuous sequences of a complete complemented adenovirus genome, which was replication competent in 293 cells. The DNA of pRABs was purified from saturated E.coli over night cultures (100ml) in LB medium using a kit for plasmid preparation. Here, the Nucleobond PC-100 kit from Macherey and Nagel, Germany was used according to the manufacturer's recommendations. The identity of the pRBAs obtained was verified by means of restriction analysis of the pRAB DNAs (Fig. 2A, lanes 2-4). For virus reconstitution purified pRAB DNA was treated with 10U *PacI* per µg DNA for 2h according to the manufacture's recommendations. Subsequently the PacI-digested pRAB1x and pRAB2x DNAs were purified using phenol-chloroform according to standard protocols prior to transfection into 293 cells. In brief, 10µg pRAB DNA was digested in a volume of 100µl for 1,5 h at 37°C in a water bath. Subsequently 50 µl phenol/chloroform (1:1mixture) was added to the reaction tube (Eppendorff cup size 1,5ml, Eppendorf AG, Hamburg, Germany) and vortexed for 20 sec. here, the Vortexer MS-3 basic was used (IKAIKA® Werke GmbH & Co. KG, Staufen, Germany). The tube was centrifuged in a table top centrifuge at maximum speed (20000 x g) for 5 min at room temperature and 80 µl of the aqueous upper phase was transfered into a fresh tube and 10 µl 3 M NaAc (pH 4,5) and 200 µl EtOH was added. All reagents and chemicals were purchased from Sigma-Aldrich, St Louis, USA. The tube was mixed with the finger tips until the precipitated DNA became visible. Moreover, the tube was incubated for 5 min at room temperature and the DNA was pelleted in a table top centrifuge at maximum speed for 15 min at room temperature. The supernatant was quantitatively removed and the pellet immediately dissolved in 20 µl sterile deionized water. Transfection of 293 cells was done using lipofection. Here, the Superfect transfection reagent (Qiagen, Hilden, Germany) was used according to the manufacturer's recommendation. The resulting adenoviruses were replication competent in 293 cells and could be propagated according to standard protocols (Green M and Loewenstein P, Human Adenoviruses: Propagation, Purification, Quantification, and Storage in Current Protocols in Microbiology79). The identity of the recombinant adenovirus vectors obtained according to the disclosed method in this example was verified by restriction digest of adenovirus vector DNA with *XhoI* and analysis of DNA fragments using agarose gel electrophoresis (Fig. 2A). For preparation of genomic adenovirus vector DNA, 293 cells (2,5x107 cells) were infected with a MOI of 3 with the recombinant adenovirus vectors obtained after transfection of the PacI-digested pRABs into the 293 cells. After the cytopathic effect (CPE) was complete the infected cells were washed once in PBS, scraped from the plates and resuspent in PBS. Cells (∼4x106 cells/ml) were lysed by adding an equal volume of TST buffer (2% TritonX-100, 400mM NaCl, 20mM Tris-HCl pH8.0) to the cell suspension followed by incubation on ice for 30 minutes. Cell debris were removed by centrifugation at 20,000g for 10 minutes at 4°C and the supernatant was treated with 50µg/ml proteinase K (Roche) in the presence of 0.5% SDS for 60 minutes at 56°C. After extraction of the nucleic acids by phenol/chloroform and ethanol precipitation the extract was treated with RNase A (Sigma). RNA-free viral DNA was again phenol/chloroform extracted and precipitated with ethanol. The *Xho*I restriction pattern of reconstituted virus derived from pRAB1x and pRAB2x corresponded to the *in silico* generated pattern, confirming the integrity of the adenovirus genome in the recombinant adenovirus viruses obtained (Fig.2A lanes 5-7).

### Example 3: Generation of recombinant RABs with controlled recombination through negative selection

To avoid multiple insertions and improve the construction of an adenovirus expression library, we constructed pDonorSir2 which is a derivative of pDonorSir1, whereby pDonorSir2 is identical to the deposited organism at the DSMZ according to the Budapest treaty with the accession number DSM 23754. pDonorSir2 differs from pDonorSir1 at its FRT locus, next to this pDonorSir2 contains a strong E*.coli* galaktokinase promoter (Warming, S., N. Costantino, Court DL, N. A. Jenkins, and N. G. Copeland. Simple and highly efficient BAC recombineering using galK selection. Nucleic Acids Res 2005, 33:e3673) upstream to the FRT site and downstream of the FRT site a *rpsL* open reading frame, which mediated Streptomycin sensitivity if expressed (Reyrat JM, Pelicic V, Gicquel B, Rappuoli R. Counterselectable markers: untapped tools for bacterial genetics and pathogenesis. Infect. Immun. 1998, 66:4011-401774). The use of pDonorSir2 is exemplified as follows: DH10B cells harbouring pBACSir2 and pCP20 were maintained at 30°C in the presence of ampicillin and chloramphenicol, whereby pBACSir2 is identical to the deposited organism at the DSMZ with the accession number according to the Budapest treaty DSM 24299, and whereby E*.coli* cells harbouring pBACSir2 and pCP20 are identical to the deposited organism at the DSMZ according to the Budapest treaty with the accession number DSM 23743. Next, the DH10B cells were electro-transformed with pDonorSir2 and cultured for 150 minutes at 42°C in the absence of any antibiotics. The expressed Flp induced site-specific recombination between FRT sites present on pDonorSir2 and pBACSir2, respectively. At the same time the elimination of Flp expression also started, since pCP20 cannot replicate in *E. coli* at elevated temperature. The transformed culture was plated onto agar plates which contained kanamycin (25µg/ml) chloramphenicol (25µg/ml) and streptomycin sulphate (50µg/ml) as selecting agents. Under these conditions E.*coli* containing recombined *recombinant adenovirus BACs* (pRAB_RPSL) were selected, in which the pDonorSir2 plasmid had recombined with pBACSir2. Single colonies were picked from the selection plate, and cultured in 10ml liquid LB media containing chloramphenicol (25µg/ml) over night at 37°C in a shaking incubator. All chemicals and media used were purchased from Sigma-Aldrich, St Louis, USA. pRAB_RPSL DNA from these cultures was subsequently isolated according to the manufacture's recommendations using a DNA-plasmid isolation kit, and the integrity of the reaction products analyzed by restriction digestion with *XhoI* (fig. 2B). Here, the Nucleobond PC-100 kit from Macherey and Nagel, Germany was used for isolation of pRAB_RPSL-DNA according to the manufacturer's recommendations. The *XhoI* restriction pattern of all 6 pRABs analysed corresponded to single recombined products (pRAB_RPSL_1x). To test the reliability of the applied counter selection we picked further 82 clones from the selection plates ant tested as above. Only 2 clones contained multiple insertion products (marked by 'D' in figure 2B), further 7 clones were contaminated by pBACSir2 (marked by 'V' in figure 2B), and 3 contained other unidentified rearrangements (marked by 'r' in figure 2B). Altogether the great majority (83/88) of the colonies contained only pRAB_RPSL_1x (Fig. 2B). In a further experiment a total of 44clones were analyzed were analyzed, whereby only 1 recombination product corresponded to pRAB_RPSL_2x, and 43 of the 44 clones corresponded to pRAB_RPSL_1x. Altogether 126/132 BACs corresponded to the single recombination product pRAB_RPSL_1x (95.45% of the recombination products analyzed) and multiple recombination was observed in 3/132 corresponding to 2.3% of the clones.

### Example 4: Determination of the average library efficiency for generation of recombinant adenovirus BAC libraries

To test the efficiency of our E. coli recombination system and avoid the contamination of pRAB_RPSL DNA preparations according to example 3 with non-recombined pBACSir2 vector, the experiment described in Example 3 was repeated two more times with the following modifications:
i) To test the primary cloning efficiency we took 50µl of a 10ml post-transformation culture and serial 10-fold dilutions were plated on a triple selection agarose plate containing kanamycin (25µg/ml), chloramphenicol (25µg/ml), and streptomycin sulphate (50µg/ml) as selecting agents (Experiment 2). All chemicals and media used were purchased from Sigma-Aldrich, St Louis, USA. After 60 minutes the rest of the culture was incubated for another 90 minutes giving finally 150 minutes total post-transformation culture time as above (Experiment 3). Two plates were inoculated by 200µl out of 1ml final volume of each dilution of the 50µl post-transfection culture (1:10¹, 1:10², and 1:10³) from each experiment.
ii) after the colonies containing the pRABs appeared on selection plates we made replica plates on a second round of triple selection plates containing kanamycin (25µg/ml), chloramphenicol (25µg/ml), and streptomycin sulphate (50µg/ml) as selecting agents. This procedure minimized the contamination by both the vector and the multiple insertion products, whereby the generation of replica plates is applied regularly in maintaining E. coli libraries.

The colony counts on replica plates were 52 and 89 in experiment 2, and 204 and 129 in experiment 3 with the dilution 1:10². Taking in account that 50ng DNA of pDonorSir2 was used, and the volume of the post-transformation culture was 50µl, and one fifth of each dilution was plated, the average cloning efficiency was 1,85x10⁶ colony for 1µg input.

### Example 5: Generation of replication competent adenovirus in 293 cells expressing FLP recombinase

For construction of HEK 293 Flp cells expressing Flp recombinase 2,5 x 10⁵ HEK 293 cells were transfected using lipofection with 10 µg of the plasmid pFlp-Puro linearized with *Pvu*I, whereby 293 Flp cells are identical to the deposited organism at the DSMZ according to the Budapest treaty with the accession number DSM ACC3077. Here, the Superfect transfection reagent (Qiagen, Hilden, Germany) was used according to the manufacturer's recommendation.. The transfected cells were incubated for 48 h at 37°C under standard cell culture conditions (95% humidity, 5% CO2). The cell culture medium used was DMEM containing 10% FCS, 2 mM Glutamin, and 1% penicillin/streptavidin (P/S)). For selection puromycin was added to a final concentration of 1 µg/µl to the medium, and cells were cultivated under selective conditions for 12 days to obtain 293 cells stably expressing FLP recombinase. All chemicals and media used were purchased from Sigma-Aldrich, St Louis, USA. The stable cell pool was expanded and a master cell bank established. For reconstitution of recombinant adenovirus 2 x 10⁵ 293 FlpP cells per well were plated onto a 6 well plate and 5 hours after plating cells were co-transfected with 0,8 µg pDonorSir2-EGFP (seq. ID No. 9, figure 14) and 2,5 µg pBACSir2, both linearized with *PacI,* using Lipofection. Here, the Superfect transfection reagent (Qiagen, Hilden, Germany) was used according to the manufacturer's recommendation. Following a 3 days cultivation at 37°C under standard cell culture conditions, cells were harvested by scraping and collected by subsequent centrifugation for 5 min at 200 x g. Cell pellets were resuspent in 400 µl cell culture medium (DMEM, 10% FCS, 2 mM Glutamin, 1% P/S) and subjected to three successive freeze/thaw cycles. Cell debris was separated from soluble material by centrifugation at 4.400 x g for 15 min. In order to demonstrate a successful rescue of adenovirus vectors expressing the EGFP gene, 2 x 10⁵ HEK-293 cells/well were plated onto a 6 well plate and infected 12 h later with 200 µl of the freeze/thaw lysate followed by 3 days incubation at 37°C under standard cell culture conditions. At this time point comet-shaped fluorescent conglomerates of cells showing cytopathic effect (CPE) characteristic for productive adenovirus replication were microscopically detectable (see Fig.12). The method thus allowed for the generation of first generation recombinant replication competent adenovirus vectors by co-transfection of a third nucleic acid molecule with a second nucleic acid molecule into 293 Flp cells.

### Biological material

The invention uses and/or relates to biological material deposited under the Budapest Treaty. More specifically, the following depositions have been made with "Deutsche Sammlung von Mikroorganismen und Zellkulturen GmbH (DSMZ)", also referred to herein as DSMZ:
DSM 23753; DSM 24298; DSM 24299; DSM 23743 ; DSM 23742; DSM ACC3077m; DSM ACC3077; and DSM 23754.

The following is a list of the various references recited herein the disclosure of which is incorporated herein in its entirety by reference.
1 Kim UJ, Shizuya H, de Jong PJ, Birren B, Simon MI. Stable propagation of cosmid sized human DNA inserts in an F factor based vector. Nucleic Acids Res. 20:1083-1085, 1992.
2 Shizuya H, Kouros-Mehr H. The development and applications of the bacterial artificial chromosome cloning system. Keio J. Med. 50:26-30, 2001 (Review).
3 Bett et al. "Packaging capacity and stability of human adenovirus type 5 vectors", J. Virol. 67:5921-5921, 1993
4 Landy, A. Mechanistic and structural complexity in the site-specific recombination pathways of Int and Flp. Curr Opin Genet Dev. 3:699-707, 1993.
5 Hoess RH, Ziese M, Sternberg N. P1 site-specific recombination: Nucleotide sequence of the recombining sites", Proc. Natl. Acad. Sci. USA 79:3398-3402, 1982.
6 Abremski K, Wierzbicki A, Frommer B, Hoess RH. Bacteriophage P1 Cre-loxP Sitespecific Recombination: Site-specific DNA Topoisomerase Activity of the Cre Recombination Protein. J. Biol. Chem. 261:391-396, 1986.
7 Campbell, AM. Chromosomal Insertion Sites for Phages and Plasmids. J. Bacteriol. 174:7495-7499, 1992.
8 Qian XH, Inman RB, Cox MM. Reactions between Half- and Full-Flp Recombination Target Sites: A Model System for Analyzing Early Steps in Flp Protein-Mediated Site-Specific Recombination. J. Biol. Chem. 267:7794-7805, 1992
9 Araki H, Nakanishi N, Evans BR, Matsuzaki H, Jayaram M, Oshima Y. Site-specific Recombinase, R, Encoded by Yeast Plasmid pSR1" J. Mol. Biol. 225:25-37, 1992.
10 Maeser S, Kahnmann R. The Gin recombinase of phage Mu can catalyse site-specific recombination in plant protoplasts. Mol. Gen. Genet. 230:170-176, 1991.
11 Esposito D, Scocca JJ. The integrase family of tyrosine recombinases: evolution of a conserved active site domain," Nucl. Acids Res. 25:3605-3614, 1997.
12 Hoess RH, Abremski K. The Cre-lox Recombination System. In Nucleic Acids and Molecular Biology, vol. 4. Eds.: Eckstein and Lilley, Berlin-Heidelberg: Springer-Verlag; pp. 90-109, 1990.
13 Broach JR, Guarascio VR, Jayaram M. Recombination within the Yeast Plasmid 2.mu. Circle is Site-Specific. Cell 29:227-234, 1982.
14 Chen L, Anton M, Graham FL. Production and characterization of human 293 cell lines expressing the site-specific recombinase Cre. Somat. Cell and Molec. Genet. 22:477-488, 1996.
15 Hardy S, Kitamura M, Harris-Stansil T, Dai Y, Phipps ML. Construction of adenovirus vectors through Cre-lox recombination. J. Virol. 71:1842-1849, 1997.
16 U.S. patent document No. 6,379,943
17 Parks RJ, Chen L, Anton M, Sankar U, Rudnicki MA, Graham FL. A new helper-dependent adenovirus vector system: removal of helper virus by cre-mediated excision of the viral packaging system. Proc. Natl. Acad. Sci. U.S.A. 93:13565-13570, 1996.
18 Anton M. and Graham FL. Site-specific recombination mediated by an adenovirus vector expressing the Cre recombinase protein: a molecular switch for control of gene expression. J.Virol. 69:4600-4606, 1995.
19 Hardy S, Kitamura M, Harris-Stansil T, Dai Y, and Phipps ML. Construction of adenovirus vectors through Cre-lox recombination. J. Virol. 71:1842-1849, 1997.
20 Semprini S, Troup TJ, Kotelevtseva N, King K, Davis JR, Mullins LJ, Chapman KE, Dunbar DR, Mullins JJ. Cryptic loxP sites in mammalian genomes: genome-wide distribution and relevance for the efficiency of BAC/PAC recombineering techniques. Nucleic Acids Res. 35:1402-1410, 1997.
21 Schmidt EE, Taylor DS, Prigge JR, Barnett S, Capecchi MR. Illegitimate Cre-dependent chromosome rearrangements in transgenic mouse spermatids. Proc. Natl. Acad. Sci. U. S. A. 97:13702-13707, 2000.
22 Sauer B. Identification of cryptic lox sites in the yeast genome by selection for Cremediated chromosome translocations that confer multiple drug resistance. J. Mol. Biol. 223:911-928, 1992.
23 U.S. patent document No. 7,132,290
24 Sharp PA, Moore C, Haverty J L. The infectivity of adenovirus 5 DNA-protein complex. Virology 75:442-456, 1976.
25 Chinnadurai G, Chinnadurai S, Green M. Enhanced infectivity of adenovirus type 2 DNA and a DNA-protein Complex. J. Virol. 26:195-199, 1978
26 Elahi SM, Oualikene W, Naghdi L, O'Connor-McCourt M, Massie B. Adenovirus-based libraries: efficient generation of recombinant adenoviruses by positive selection with the adenovirus protease. Gene Ther. 9:1238-1246, 2002.
27U.S. patent application No. 2006210965
28 Ballay A, Levrero M, Buendia MA, Tiollais P, Perricaudet M. In vitro and in vivo synthesis of the hepatitis B virus surface antigen and of the receptor for polymerized human serum albumin from recombinant human adenoviruses. EMBO J. 30: 3861-5, 1985.
29 Rosenfeld MA, Siegfried W, Yoshimura K, Yoneyama K, Fukayama M, Stier LE, Pääkkö PK, Gilardi P, Stratford-Perricaudet LD, Perricaudet M. Adenovirus-mediated transfer of a recombinant alpha 1-antitrypsin gene to the lung epithelium in vivo. Science 252:431-4, 1991.
30 Bett AJ, Hidaka W, Prevec L, Graham FL. An efficient and flexible system for construction of adenovirus vectors with insertions or deletions in early regions 1 and 3. Proc. Natl. Acad. Sci. U.S.A. 91:8801-8806, 1994
31 Mizuguchi H, Kay MA. Efficient construction of a recombinant adenovirus vector by an improved in vitro ligation method. Hum. Gene Ther. 9:2577-2583, 1998.
32 He T-C, Zhou S, Yu J, Kinzler KW, and Vogelstein B. A simplified system for generating recombinant adenoviruses. Proc. Natl. Acad. Sci. U.S.A. 95:2509-2514, 1998.
33 Chartier C, Degryse E, Gantzer M, Dieterle A, Pavirani A, Mehtali M. Efficient generation of recombinant adenovirus vectors by homologous recombination in Escherichia coli. J. Virol. 70 :4805-4810, 1996.
34 Crouzet J, Naudin L, Orsini C, Vigne E, Ferrero L, Le Roux A, Benoit P, Latta M, Torrent C, Branellec D, Denèfle P, Mayaux JF, Perricaudet M, Yeh P. Recombinational construction in Escherichia coli of infectious adenoviral genomes. Proc. Natl. Acad. Sci. U.S.A. 94:1414-1419, 1997.
35 Ketner G, Spencer F, Tugendreich S, Connelly C, Hieter P. Efficient manipulation of the human adenovirus genome as an infectious yeast artificial chromosome clone. Proc. Natl. Acad. Sci. U.S.A. 91:6186-6190, 1994.
36 U.S. patent document No. 7,282,326
37 U.S. patent document No. 5,888,732
38 Katzen, F.Gateway® recombinational cloning: a biological operating system. Expert Opinion Drug Discovery 2:571-589, 2007.
39 Sheng Y, Mancino V, Birren B. Transformation of Escherichia coli with large DNA molecules by electroporation. Nucleic Acids Res. 23:1990-1996, 1995.
40 Bzymek M, Lovett ST. Instability of repetitive DNA sequences: the role of replication in multiple mechanisms. Proc. Natl. Acad. Sci. U. S. A. 98:8319-8325, 2001.
41 Fan ZC, Bird RC. An improved reverse genetics system for generation of bovine viral diarrhea virus as a BAC cDNA. J. Virol. Methods 149:309-315, 2008.
42 Ruzsics Z, Wagner M, Osterlehner A, Cook J, Koszinowski U, Burgert HG. Transposon assisted cloning and traceless mutagenesis of adenoviruses: Development of a novel vector based on species D. J. Virol. 80:8100-8113, 2006.
43 PCT patent application WO/1999/025854
44 Yamamoto M, Davydova J, Takayama K, Alemany R, Curiel DT. Transcription initiation activity of adenovirus left-end sequence in adenovirus vectors with el deleted. J. Virol. 77:1633-1637, 2003.
45 Hearing P. and Shenk T. The adenovirus type 5 E1A transcriptional control region contains a duplicated enhancer element. Cell 33:695-703.
46 Liu H, Naismith JH, Hay RT. Adenovirus DNA replication. Curr. Top. Microbiol. Immunol. 272:131-64, 2003 (Review).
47 Goncalves MA. Adeno-associated virus: from defective virus to effective vector. Virol J. 6;2:43, 2005 (Review).
48 Spate R and Mocarski E. The alpha sequence of the cytomegalovirus genome functions as a cleavage/packaging signal for herpes simplex virus defective genomes. J. Virol. 54:817-24, 1985
49 Garble M. and Hearing P. cis and trans requirements for the selective packaging of adenovirus type 5 DNA. J. Virol. 66:723-731, 1992.
50 Ostapchuk P, Hearing P. Regulation of adenovirus packaging. Curr. Top. Microbiol. Immunol. 272:165-85, 2003.
51 Juven-Gershon T, Hsu JY, Theisen JW, Kadonaga JT. The RNA polymerase II core promoter - the gateway to transcription. Curr. Opin. Cell Biol. 20:253-259, 2008.
52 Geiduschek EP and Tocchini-Valentini GP. Transcription by RNA polymerase III. Annu. Rev. Biochem. 57:873-914, 1988.
53 Broach JR, Guarascio VR, Jayaram M. Recombination within the yeast plasmid 2mu circle is site-specific. Cell 29:227-234, 1982.
54 Schlake T. and Bode J. Use of mutated Flp recognition target (FRT) sites for the exchange of expression cassettes at defined chromosomal loci Biochemistry 33:12746-12751, 1994
55 Cherepanov PP and Wackernagel W. Gene disruption in Escherichia coli: TcR and KmR cassettes with the option of Flp-catalyzed excision of the antibiotic-resistance determinant.Gene 158:9-14, 1995
56 Kües U, Stahl U. replication of plasmids in gram-negative bacteria. Microbiological. Rev. 53:491-516, 1989.
57 Shafferman A, Kolter R, Stalker D, Helinski DR. Plasmid R6K DNA replication. III. Regulatory properties of the pi initiation protein. J. Mol. Biol. 161:57-76, 1982.
58 Busby S, Ebright RH. Promoter structure, promoter recognition, anated by d transcription activation in prokaryotes. Cell. 79:743-46, 1994. Review.
59 Mao F, Dam P, Chou J, Olman V, Xu Y. DOOR: a database for prokaryotic operons. Nucleic Acids Res. 37:D459-63, 2009.
60 Ullmann A. Escherichia coli Lactose Operon. In: Encyclopedia of ife Sciences. doi:10.1038/npg.els.0000849, 2001.
61 Zhang X, Pan Z, Fang Q, Zheng J, Hu M, Jiao X. J Microbiol Methods. An auto-inducible Escherichia coli lysis system controlled by magnesium. 79:199-204, 2009.
62 Bukrinsky et al. Multicopy expression vector based on temperature-regulated lac repressor: expression of human immunodeficiency virus env gene in Escherichia coli. Gene 70, 415-417, 1988.
63 Reyrat JM, Pelicic V, Gicquel B, Rappuoli R. Counterselectable markers: untapped tools for bacterial genetics and pathogenesis. Infect Immun. 6:4011-4017, 1998.
64 Dean D. A plasmid cloning vector for the direct selection of strains carrying recombinant plasmids. Gene 15:99-102, 1981.
65 Scott JR. Regulation of plasmid replication. Microbiol. Rev. 48:1-23, 1984
66 Schaack J. Adenovirus vectors deleted for genes essential for viral DNA replication. Front. Biosci. 10:1146-1155,2005
67 Gorziglia MI, Lapcevich C, Roy S, Kang Q, Kadan M, Wu V, Pechan P, Kaleko M.Generation of an adenovirus vector lacking E1, e2a, E3, and all of E4 except open reading frame 3. J Virol. 73:6048-6055, 1999
68 Kochanek S, Schiedner G, Volpers C. High-capacity 'gutless' adenoviral vectors. Curr. Opin. Mol. Ther 3:454-463, 2001
69 Tenorio C, Zarazaga M, et al. Bifunctional Enzyme 6'-N-Aminoglycoside Acetyltransferase-2"-O- Aminoglycoside Phosphotransferase in Lactobacillus and Pediococcus Isolates of Animal Origin. J. Clin .Microbiol. 39:824-825, 2001
70 Lee EC., et al. A highly efficient Escherichia coli-based chromosome engineering system adapted for recombinogenic targeting and subcloning of BAC DNA. Genomics 73:56-65, 2001
71 Messerle M, Crnkovic I, Hammerschmidt W, Ziegler H, Koszinowski UH. Cloning and mutagenesis of a herpesvirus genome as an infectious bacterial artificial chromosome. Proc. Natl. Acad. Sci. U. S. A. 94:14759-14763, 1997.
72 Bubeck A, Wagner M, Ruzsics Z, Lötzerich M, Iglesias M, Singh IR, Koszinowski UH. Comprehensive mutational analysis of a herpesvirus gene in the viral genome context reveals a region essential for virus replication. J. Virol. 78:8026-8035, 2004.
73 Warming S, Costantino N, Court DL, Jenkins NA, Copeland NG. Simple and highly efficient BAC recombineering using galK selection. Nucleic Acids Res 2005, 33:e36
74 Reyrat JM, Pelicic V, Gicquel B, Rappuoli R. Counterselectable markers: untapped tools for bacterial genetics and pathogenesis. Infect. Immun. 1998, 66:4011-4017
75 Lederberg J. Streptomycin resistance: a genetically recessive mutation. J. Bacteriol. 1951, 61:549-550
76 Marcaida MJ et al. Homing endonucleases: from basics to therapeutic applications. Cell Mol Life Sci. 67(5):727-48, 2010
77 Klug A. The discovery of zinc fingers and their applications in gene regulation and genome manipulation. Annu Rev Biochem. 79:213-31, 2010
78 Gossen M, Bujard H. Tight control of gene expression in mammalian cells by tetracycline-responsive promoters. Proc Natl Acad Sci U S A. 89:5547-51, 1992
79 Green M, Loewenstein PM. Human Adenoviruses: Propagation, Purification, Quantification, and Storage. Current Protocols in Microbiology 2006, UNIT 14C.1, DOI: 10.1002/9780471729259.mc14c01s00

The features of the present invention disclosed in the specification, the claims and/or the drawings may both separately and in any combination thereof be material for realizing the invention in various forms thereof.

## Claims

1. A nucleic acid molecule, which is also referred to as third nucleic molecule, wherein the third nucleic acid molecule comprises
(1) a nucleic acid molecule comprising the following elements:
(a) optionally, a first part of a genome of a virus;
(b) a nucleotide sequence, preferably a genomic nucleotide sequence, or a transcription unit;
(c) a regulatory nucleic acid sequence which has a regulatory activity in a prokaryote;
(d) a site-specific recombination site;
(e) a nucleotide sequence providing for a negative selection marker;
(f) a bacterial nucleotide sequence unit comprising (i) bacterial nucleotide sequences for conditional replication and (ii) a nucleotide sequence providing for a positive selection marker;
(g) optionally a first restriction site; or
(2) a nucleic acid molecule comprising a nucleotide sequence according to SEQ ID NO: 6; or
(3) a nucleic acid molecule identical or similar to the nucleic acid molecule contained in the organism deposited with the DSMZ under the Budapest treaty under accession number DSM 23754, wherein preferably the nucleic acid molecule contained in the organism is a heterologous nucleic acid molecule;
wherein the third nucleic acid molecule is either a linear or a circular molecule.

2. The third nucleic acid molecule according to claim 1, wherein the third nucleic acid molecule is a linear molecule, wherein elements (a) to (f), preferably upon cleavage of the circular molecule of the third nucleic acid molecule with the first restriction enzyme which recognized and cleaves at the first restriction site, are arranged in a 5' -> 3' direction in the following sequence as follows:
1. optionally the first part of a genome of a virus;
2. the nucleotide sequence, preferably a genomic nucleotide sequence, or a transcription unit;
3. the regulatory nucleic acid sequence which has a regulatory activity in a prokaryote;
4. the site-specific recombination site;
5. the nucleotide sequence providing for a negative selection marker; and
6. the bacterial nucleotide sequence unit comprising (i) bacterial nucleotide sequences for conditional replication and (ii) a nucleotide sequence providing for a positive selection marker.

3. The third nucleic acid molecule according to any of claims 1 to 2, wherein the third nucleic acid molecule further comprises a first part of a genome of a virus.

4. The third nucleic acid molecule according to claim 3, wherein the first part of a or the genome of a virus comprises the terminal sequence of a or the genome of a or the virus or one or several parts of the terminal sequence.

5. The third nucleic acid molecule according to any of claim 3 to 4, wherein the first part of a or the genome of a or the virus is a first part of the genome of an adenovirus, preferably a human adenovirus and more preferably the adenovirus is human adenovirus type 5, and most preferably the entire left end of adenovirus type 5 upstream of the TATA box of the E 1 transcription unit, or one or several parts thereof.

6. The third nucleic acid molecule according to any of claims 1 to 5, preferably claim 5, wherein the bacterial nucleotide sequences for conditional replication comprise an origin of replication, whereby preferably the origin of replication is the minimal origin of phage gR6K.

7. The third nucleic acid molecule according to any of claims 1 to 6, preferably any of claims 5 to 6, wherein the regulatory sequence which has a regulatory activity in a prokaryote is a sequence which directs expression of a nucleotide sequence in a prokaryote, preferably in a prokaryotic host cell.

8. The third nucleic acid molecule according to any of claims 1 to 7, preferably any of claims 6 to 7, wherein the negative selection marker or the expression of the nucleotide sequence providing for a negative selection marker mediates or confers sensitivity to a selecting agent and/or a selecting condition.

9. The third nucleic acid molecule according to claim 9, wherein the nucleotide sequence providing for a negative selection marker is a gene selected from the group comprising the *galK, tetAR, pheS, thyA, lacy, ccdB* and *rpsL* gene.

10. A combination of a third nucleic acid molecule as defined in any of claims 1 to 9 and a nucleic acid molecule which is also referred to as second nucleic acid molecule,
wherein the second nucleic acid molecule comprises
(1) a nucleic acid molecule comprising the following elements:
(a) a bacterial nucleotide sequence unit comprising (i) bacterial nucleotide sequences for single copy replication, and (ii) a nucleotide sequence providing for a second selection marker;
(b) a site-specific recombination site;
(c) a second part of a genome of a virus; and
(d) optionally a restriction site which is referred to as second restriction site; or
(2) a nucleic acid molecule comprising a nucleotide sequence according to SEQ ID NO: 2 and/or SEQ ID NO: 13; or
(3) a nucleic acid molecule identical or similar to the nucleic acid molecule contained in the organism deposited with the DSMZ under the Budapest treaty under accession number DSM 24298 and/or DSM 24299, wherein preferably the nucleic acid molecule contained in the organism is a heterologous nucleic acid molecule;
wherein the third nucleic acid molecule is either a linear or a circular molecule.

11. A method for the generation of a nucleic acid molecule coding for a virus comprising the following steps
a) providing a third nucleic acid molecule as defined in any one of claims 1 to 9;
b) providing a second nucleic acid molecule as defined in claims 10; or
c) a combination according to claim 10;
d) allowing the third and the second nucleic acid molecule to react so that a site-specific recombination occurs, wherein the site-specific recombination is mediated by a site-specific recombinase and the site-specific recombination forms a recombination product comprising a copy, preferably single copy of the genome of a or the virus, whereby the genome is a complemented complete genome and the complemented complete genome is complemented by the site-specific recombination;
e) optionally selecting the recombination product; and
f) optionally cleaving the recombination product with the first and second restriction enzyme.

12. The method according to claim 11, wherein the third and the second nucleic acid molecule are reacted in a prokaryotic host cell preferably *E. coli,* being similar or identical to the deposited organisms at the DSMZ with the accession numbers according to the Budapest treaty DSM 23743.

13. A method for generating a library of nucleotide sequences, wherein said library comprises a plurality of individual nucleotide sequences, wherein said library is represented by a plurality of viral genomes and each viral genome contains a single one of the individual nucleotide sequences, comprising the steps of the method as defined in any of claims 11 to 12, wherein the individual nucleotide sequence is part of the transcription unit of the third nucleic acid molecule.

14. A kit comprising optionally a package insert, and, in (a) suitable container(s), at least a third nucleic acid molecule as defined in any one of claims 1 to 9 and/or a combination of the third nucleic acid molecule and the second nucleic acid molecule as defined in claim 10.

15. The kit according to claim 14, wherein the nucleic acid molecule(s) is/are contained in a ready- to-use form.


SEQUENCE LISTING

<110>  SIRION BIOTECH GmbH

<120>  Nucleic acid molecules and use thereof

<130>  S 10056 EP

<160>  13

<170>  PatentIn version 3.3

<210>  1
<211>  3050
<212>  DNA
<213>  Artificial

<220>
<223>  synthetic

<400>  1
catcatcaat aatatacctt attttggatt gaagccaata tgataatgag ggggtggagt      60

ttgtgacgtg gcgcggggcg tgggaacggg gcgggtgacg tagtagtgtg gcggaagtgt     120

gatgttgcaa gtgtggcgga acacatgtaa gcgacggatg tggcaaaagt gacgtttttg     180

gtgtgcgccg gtgtacacag gaagtgacaa ttttcgcgcg gttttaggcg gatgttgtag     240

taaatttggg cgtaaccgag taagatttgg ccattttcgc gggaaaactg aataagagga     300

agtgaaatct gaataatttt gtgttactca tagcgcgtaa tactggtacc ccccagtggc     360

aagacgcgca ggcaaaacgc accacgactg gatccgaatt caaaaagccg gaactgatca     420

gaaccattct cttgaaatgg ttctgatcag ttccggccgg tgtttcgtcc tttccacaag     480

atatataaag ccaagaaatc gaaatacttt caagttacgg taagcatatg atagtccatt     540

ttaaaacata attttaaaac tgcaaactac ccaagaaatt attactttct acgtcacgta     600

ttttgtacta atatctttgt gtttacagtc aaattaattc taattatctt tctaacagcc     660

ttgtatcgta tatgcaaata tgaaggaatc atgggaaata ggccctcttc ctgcccgacc     720

ttggcgcgcg ctcggcgcgc ggtcacgctc cgtcacgtgg tgcgttttgc ctgcgcgtct     780

tgccactggg gggtaccccg aatcactaga tctgagctcg gcctggtgat gatggcggga     840

tcgttgtata tttcttgaca ccttttcggc atcgccctaa aattcggcgt cctcatattg     900

tgtgaggacg ttttattacg tgtttacgaa gcaaaagcta aaaccaggag ctatttaatg     960

gcaacaaccg gtaccatgat tacgccaagc tacgtaatac gactcactag tggaagttcc    1020

tattctctag aaagtatagg aacttcctta agtgagtcgt attacggact ggccgtcgtt    1080

ttacaacgtc gtgactggga aaaccctggc gttacccaac ttaatcgcct tgcagcacat    1140

ccccctttcg ccagctggcg taatagcgaa gaggcccgca ccgatcgccc ttcccaacag    1200

ttgcgcagcc tgaatggcga atggcgcttc gcttggtaag ctagctaagg cttaaggagg    1260

acaatcatgg caacagttaa ccagctggta cgcaaaccac gtgctcgcaa agttgcgaaa 1320

agcaacgtgc ctgcgctgga agcatgcccg caaaaacgtg gcgtatgtac tcgtgtatat 1380

actaccactc ctaaaaaacc gaactccgcg ctgcgtaaag tatgccgtgt tcgtctgtct 1440

aacggtttcg aagtgacttc ctacatcggt ggtgaaggtc acaacctgca ggagcactcc 1500

gtgatcctga tccgtggcgg tcgtgttaaa gacctcccgg gtgttcgtta ccacaccgta 1560

cgtggtgcgc ttgactgctc cggcgttaaa gaccgtaagc aggctcgttc caagtatggc 1620

gtgaagcgtc ctaaggctta actagcacgc gccatgtcag ccgttaagtg ttcctgtgtc 1680

actcaaaatt gctttgagag gctctaaggg cttctcagtg cgttacatcc ctggcttgtt 1740

gtccacaacc gttaaacctt aaaagcttta aaagccttat atattctttt ttttcttata 1800

aaacttaaaa ccttagaggc tatttaagtt gctgatttat attaatttta ttgttcaaac 1860

atgagagctt agtacgtgaa acatgagagc ttagtacgtt agccatgaga gcttagtacg 1920

ttagccatga gggtttagtt cgttaaacat gagagcttag tacgttaaac atgagagctt 1980

agtacgtgaa acatgagagc ttagtacgta ctatcaacag gttgaactgc tgatcttcag 2040

atcctctacg ccggacgcat cgtggccgga tccgatttat tcaacaaagc cacgttgtgt 2100

ctcaaaatct ctgatgttac attgcacaag ataaaaatat atcatcatga acaataaaac 2160

tgtctgctta cataaacagt aatacaaggg gtgttatgag ccatattcaa cgggaaacgt 2220

cttgctcgag gccgcgatta aattccaaca tggatgctga tttatatggg tataaatggg 2280

ctcgcgataa tgtcgggcaa tcaggtgcga caatctatcg attgtatggg aagcccgatg 2340

cgccagagtt gtttctgaaa catggcaaag gtagcgttgc caatgatgtt acagatgaga 2400

tggtcagact aaactggctg acggaattta tgcctcttcc gaccatcaag cattttatcc 2460

gtactcctga tgatgcatgg ttactcacca ctgcgatccc cgggaaaaca gcattccagg 2520

tattagaaga atatcctgat tcaggtgaaa atattgttga tgcgctggca gtgttcctgc 2580

gccggttgca ttcgattcct gtttgtaatt gtccttttaa cagcgatcgc gtatttcgtc 2640

tcgctcaggc gcaatcacga atgaataacg gtttggttga tgcgagtgat tttgatgacg 2700

agcgtaatgg ctggcctgtt gaacaagtct ggaaagaaat gcataagctt ttgccattct 2760

caccggattc agtcgtcact catggtgatt tctcacttga taaccttatt tttgacgagg 2820

ggaaattaat aggttgtatt gatgttggac gagtcggaat cgcagaccga taccaggatc 2880

ttgccatcct atggaactgc ctcggtgagt tttctccttc attacagaaa cggctttttc 2940

aaaaatatgg tattgataat cctgatatga ataaattgca gtttcatttg atgctcgatg 3000

agtttttcta atcagaattg gttaattggt tgtaacactg gcttaattaa 3050

<210> 2
<211> 37320

<212>  DNA
<213>  Artificial

<220>
<223>  synthetic

<400>  2
gaagttccta ttccgaagtt cctattctct agaaagtata ggaacttcag agcgcttttg      60

aagctggggt gggcgaagaa ctccagcatg agatccccag agcgcttttg aagctgcgtt     120

taaacgcgat atcccgggag ctcccgatat cgcgtttaaa cgcagcttgg cgtaatcatg     180

gtcatagctg tttcctgtgt gaaattgtta tccgctcaca attccacaca acatacgagc     240

cggaagactg aaatgtgtgg gcgtggctta agggtgggaa agaatatata aggtgggggt     300

cttatgtagt tttgtatctg ttttgcagca gccgccgccg ccatgagcac caactcgttt     360

gatggaagca ttgtgagctc atatttgaca acgcgcatgc ccccatgggc cggggtgcgt     420

cagaatgtga tgggctccag cattgatggt cgccccgtcc tgcccgcaaa ctctactacc     480

ttgacctacg agaccgtgtc tggaacgccg ttggagactg cagcctccgc cgccgcttca     540

gccgctgcag ccaccgcccg cgggattgtg actgactttg ctttcctgag cccgcttgca     600

agcagtgcag cttcccgttc atccgcccgc gatgacaagt tgacggctct tttggcacaa     660

ttggattctt tgacccggga acttaatgtc gtttctcagc agctgttgga tctgcgccag     720

caggtttctg ccctgaaggc ttcctcccct cccaatgcgg tttaaaacat aaataaaaaa     780

ccagactctg tttggatttg gatcaagcaa gtgtcttgct gtctttattt aggggttttg     840

cgcgcgcggt aggcccggga ccagcggtct cggtcgttga gggtcctgtg tattttttcc     900

aggacgtggt aaaggtgact ctggatgttc agatacatgg gcataagccc gtctctgggg     960

tggaggtagc accactgcag agcttcatgc tgcggggtgg tgttgtagat gatccagtcg    1020

tagcaggagc gctgggcgtg gtgcctaaaa atgtctttca gtagcaagct gattgccagg    1080

ggcaggccct tggtgtaagt gtttacaaag cggttaagct gggatgggtg catacgtggg    1140

gatatgagat gcatcttgga ctgtattttt aggttggcta tgttcccagc catatccctc    1200

cggggattca tgttgtgcag aaccaccagc acagtgtatc cggtgcactt gggaaatttg    1260

tcatgtagct tagaaggaaa tgcgtggaag aacttggaga cgcccttgtg acctccaaga    1320

ttttccatgc attcgtccat aatgatggca atgggcccac gggcggcggc ctgggcgaag    1380

atatttctgg gatcactaac gtcatagttg tgttccagga tgagatcgtc ataggccatt    1440

tttacaaagc gcgggcggag ggtgccagac tgcggtataa tggttccatc cggcccaggg    1500

gcgtagttac cctcacagat ttgcatttcc cacgctttga gttcagatgg ggggatcatg    1560

tctacctgcg gggcgatgaa gaaaacggtt tccggggtag gggagatcag ctgggaagaa    1620

agcaggttcc tgagcagctg cgacttaccg cagccggtgg gcccgtaaat cacacctatt    1680

accgggtgca actggtagtt aagagagctg cagctgccgt catccctgag caggggggcc 1740
acttcgttaa gcatgtccct gactcgcatg ttttccctga ccaaatccgc cagaaggcgc 1800
tcgccgccca gcgatagcag ttcttgcaag gaagcaaagt ttttcaacgg tttgagaccg 1860
tccgccgtag gcatgctttt gagcgtttga ccaagcagtt ccaggcggtc ccacagctcg 1920
gtcacctgct ctacggcatc tcgatccagc atatctcctc gtttcgcggg ttggggcggc 1980
tttcgctgta cggcagtagt cggtgctcgt ccagacgggc cagggtcatg tctttccacg 2040
ggcgcagggt cctcgtcagc gtagtctggg tcacggtgaa ggggtgcgct ccgggctgcg 2100
cgctggccag ggtgcgcttg aggctggtcc tgctggtgct gaagcgctgc cggtcttcgc 2160
cctgcgcgtc ggccaggtag catttgacca tggtgtcata gtccagcccc tccgcggcgt 2220
ggcccttggc gcgcagcttg cccttggagg aggcgccgca cgaggggcag tgcagacttt 2280
tgagggcgta gagcttgggc gcgagaaata ccgattccgg ggagtaggca tccgcgccgc 2340
aggccccgca gacggtctcg cattccacga gccaggtgag ctctggccgt tcggggtcaa 2400
aaaccaggtt tcccccatgc tttttgatgc gtttcttacc tctggtttcc atgagccggt 2460
gtccacgctc ggtgacgaaa aggctgtccg tgtccccgta tacagacttg agaggcctgt 2520
cctcgagcgg tgttccgcgg tcctcctcgt atagaaactc ggaccactct gagacaaagg 2580
ctcgcgtcca ggccagcacg aaggaggcta agtgggaggg gtagcggtcg ttgtccacta 2640
gggggtccac tcgctccagg gtgtgaagac acatgtcgcc ctcttcggca tcaaggaagg 2700
tgattggttt gtaggtgtag gccacgtgac cgggtgttcc tgaagggggg ctataaaagg 2760
gggtgggggc gcgttcgtcc tcactctctt ccgcatcgct gtctgcgagg gccagctgtt 2820
ggggtgagta ctccctctga aaagcgggca tgacttctgc gctaagattg tcagtttcca 2880
aaaacgagga ggatttgata ttcacctggc ccgcggtgat gcctttgagg gtggccgcat 2940
ccatctggtc agaaaagaca atctttttgt tgtcaagctt ggtggcaaac gacccgtaga 3000
gggcgttgga cagcaacttg gcgatggagc gcagggtttg gtttttgtcg cgatcggcgc 3060
gctccttggc cgcgatgttt agctgcacgt attcgcgcgc aacgcaccgc cattcgggaa 3120
agacggtggt gcgctcgtcg ggcaccaggt gcacgcgcca accgcggttg tgcagggtga 3180
caaggtcaac gctggtggct acctctccgc gtaggcgctc gttggtccag cagaggcggc 3240
cgcccttgcg cgagcagaat ggcggtaggg ggtctagctg cgtctcgtcc ggggggtctg 3300
cgtccacggt aaagaccccg ggcagcaggc gcgcgtcgaa gtagtctatc ttgcatcctt 3360
gcaagtctag cgcctgctgc catgcgcggg cggcaagcgc gcgctcgtat gggttgagtg 3420
ggggacccca tggcatgggg tgggtgagcg cggaggcgta catgccgcaa atgtcgtaaa 3480
cgtagagggg ctctctgagt attccaagat atgtagggta gcatcttcca ccgcggatgc 3540
tggcgcgcac gtaatcgtat agttcgtgcg agggagcgag gaggtcggga ccgaggttgc 3600

tacgggcggg ctgctctgct cggaagacta tctgcctgaa gatggcatgt gagttggatg 3660

atatggttgg acgctggaag acgttgaagc tggcgtctgt gagacctacc gcgtcacgca 3720

cgaaggaggc gtaggagtcg cgcagcttgt tgaccagctc ggcggtgacc tgcacgtcta 3780

gggcgcagta gtccagggtt tccttgatga tgtcatactt atcctgtccc ttttttttcc 3840

acagctcgcg gttgaggaca aactcttcgc ggtctttcca gtactcttgg atcggaaacc 3900

cgtcggcctc cgaacggtaa gagcctagca tgtagaactg gttgacggcc tggtaggcgc 3960

agcatccctt ttctacgggt agcgcgtatg cctgcgcggc cttccggagc gaggtgtggg 4020

tgagcgcaaa ggtgtccctg accatgactt tgaggtactg gtatttgaag tcagtgtcgt 4080

cgcatccgcc ctgctcccag agcaaaaagt ccgtgcgctt tttggaacgc ggatttggca 4140

gggcgaaggt gacatcgttg aagagtatct ttcccgcgcg aggcataaag ttgcgtgtga 4200

tgcggaaggg tcccggcacc tcggaacggt tgttaattac ctgggcggcg agcacgatct 4260

cgtcaaagcc gttgatgttg tggcccacaa tgtaaagttc caagaagcgc gggatgccct 4320

tgatggaagg caatttttta agttcctcgt aggtgagctc ttcaggggag ctgagcccgt 4380

gctctgaaag ggcccagtct gcaagatgag ggttggaagc gacgaatgag ctccacaggt 4440

cacgggccat tagcatttgc aggtggtcgc gaaaggtcct aaactggcga cctatggcca 4500

ttttttctgg ggtgatgcag tagaaggtaa gcgggtcttg ttcccagcgg tcccatccaa 4560

ggttcgcggc taggtctcgc gcggcagtca ctagaggctc atctccgccg aacttcatga 4620

ccagcatgaa gggcacgagc tgcttcccaa aggcccccat ccaagtatag gtctctacat 4680

cgtaggtgac aaagagacgc tcggtgcgag gatgcgagcc gatcgggaag aactggatct 4740

cccgccacca attggaggag tggctattga tgtggtgaaa gtagaagtcc ctgcgacggg 4800

ccgaacactc gtgctggctt ttgtaaaaac gtgcgcagta ctggcagcgg tgcacgggct 4860

gtacatcctg cacgaggttg acctgacgac cgcgcacaag gaagcagagt gggaatttga 4920

gcccctcgcc tggcgggttt ggctggtggt cttctacttc ggctgcttgt ccttgaccgt 4980

ctggctgctc gaggggagtt acggtggatc ggaccaccac gccgcgcgag cccaaagtcc 5040

agatgtccgc gcgcggcggt cggagcttga tgacaacatc gcgcagatgg gagctgtcca 5100

tggtctggag ctcccgcggc gtcaggtcag gcgggagctc ctgcaggttt acctcgcata 5160

gacgggtcag ggcgcgggct agatccaggt gatacctaat ttccaggggc tggttggtgg 5220

cggcgtcgat ggcttgcaag aggccgcatc cccgcggcgc gactacggta ccgcgcggcg 5280

ggcggtgggc cgcgggggtg tccttggatg atgcatctaa aagcggtgac gcgggcgagc 5340

ccccggaggt agggggggct ccggacccgc cgggagaggg ggcaggggca cgtcggcgcc 5400

gcgcgcgggc aggagctggt gctgcgcgcg taggttgctg gcgaacgcga cgacgcggcg 5460

gttgatctcc tgaatctggc gcctctgcgt gaagacgacg ggcccggtga gcttgagcct 5520

gaaagagagt tcgacagaat caatttcggt gtcgttgacg gcggcctggc gcaaaatctc 5580

ctgcacgtct cctgagttgt cttgataggc gatctcggcc atgaactgct cgatctcttc 5640

ctcctggaga tctccgcgtc cggctcgctc cacggtggcg gcgaggtcgt tggaaatgcg 5700

ggccatgagc tgcgagaagg cgttgaggcc tccctcgttc cagacgcggc tgtagaccac 5760

gcccccttcg gcatcgcggg cgcgcatgac cacctgcgcg agattgagct ccacgtgccg 5820

ggcgaagacg gcgtagtttc gcaggcgctg aaagaggtag ttgagggtgg tggcggtgtg 5880

ttctgccacg aagaagtaca taacccagcg tcgcaacgtg gattcgttga tatcccccaa 5940

ggcctcaagg cgctccatgg cctcgtagaa gtccacggcg aagttgaaaa actgggagtt 6000

gcgcgccgac acggttaact cctcctccag aagacggatg agctcggcga cagtgtcgcg 6060

cacctcgcgc tcaaaggcta caggggcctc ttcttcttct tcaatctcct cttccataag 6120

ggcctcccct tcttcttctt ctggcggcgg tgggggaggg gggacacggc ggcgacgacg 6180

gcgcaccggg aggcggtcga caaagcgctc gatcatctcc ccgcggcgac ggcgcatggt 6240

ctcggtgacg gcgcggccgt tctcgcgggg gcgcagttgg aagacgccgc ccgtcatgtc 6300

ccggttatgg gttggcgggg ggctgccatg cggcagggat acggcgctaa cgatgcatct 6360

caacaattgt tgtgtaggta ctccgccgcc gagggacctg agcgagtccg catcgaccgg 6420

atcggaaaac ctctcgagaa aggcgtctaa ccagtcacag tcgcaaggta ggctgagcac 6480

cgtggcgggc ggcagcgggc ggcggtcggg gttgtttctg gcggaggtgc tgctgatgat 6540

gtaattaaag taggcggtct tgagacggcg gatggtcgac agaagcacca tgtccttggg 6600

tccggcctgc tgaatgcgca ggcggtcggc catgccccag gcttcgtttt gacatcggcg 6660

caggtctttg tagtagtctt gcatgagcct ttctaccggc acttcttctt ctccttcctc 6720

ttgtcctgca tctcttgcat ctatcgctgc ggcggcggcg gagtttggcc gtaggtggcg 6780

ccctcttcct cccatgcgtg tgaccccgaa gcccctcatc ggctgaagca gggctaggtc 6840

ggcgacaacg cgctcggcta atatggcctg ctgcacctgc gtgagggtag actggaagtc 6900

atccatgtcc acaaagcggt ggtatgcgcc cgtgttgatg gtgtaagtgc agttggccat 6960

aacggaccag ttaacggtct ggtgacccgg ctgcgagagc tcggtgtacc tgagacgcga 7020

gtaagccctc gagtcaaata cgtagtcgtt gcaagtccgc accaggtact ggtatcccac 7080

caaaaagtgc ggcggcggct ggcggtagag gggccagcgt agggtggccg gggctccggg 7140

ggcgagatct tccaacataa ggcgatgata tccgtagatg tacctggaca tccaggtgat 7200

gccggcggcg gtggtggagg cgcgcggaaa gtcgcggacg cggttccaga tgttgcgcag 7260

cggcaaaaag tgctccatgg tcgggacgct ctggccggtc aggcgcgcgc aatcgttgac 7320

gctctagacc gtgcaaaagg agagcctgta agcgggcact cttccgtggt ctggtggata 7380

aattcgcaag ggtatcatgg cggacgaccg gggttcgagc cccgtatccg gccgtccgcc 7440

gtgatccatg cggttaccgc ccgcgtgtcg aacccaggtg tgcgacgtca gacaacgggg 7500

gagtgctcct tttggcttcc ttccaggcgc ggcggctgct gcgctagctt ttttggccac 7560

tggccgcgcg cagcgtaagc ggttaggctg gaaagcgaaa gcattaagtg gctcgctccc 7620

tgtagccgga gggttatttt ccaagggttg agtcgcggga cccccggttc gagtctcgga 7680

ccggccggac tgcggcgaac gggggtttgc ctccccgtca tgcaagaccc cgcttgcaaa 7740

ttcctccgga aacagggacg agcccctttt ttgcttttcc cagatgcatc cggtgctgcg 7800

gcagatgcgc ccccctcctc agcagcggca agagcaagag cagcggcaga catgcagggc 7860

accctcccct cctcctaccg cgtcaggagg ggcgacatcc gcggttgacg cggcagcaga 7920

tggtgattac gaacccccgc ggcgccgggc ccggcactac ctggacttgg aggagggcga 7980

gggcctggcg cggctaggag cgccctctcc tgagcggtac ccaagggtgc agctgaagcg 8040

tgatacgcgt gaggcgtacg tgccgcggca gaacctgttt cgcgaccgcg agggagagga 8100

gcccgaggag atgcgggatc gaaagttcca cgcagggcgc gagctgcggc atggcctgaa 8160

tcgcgagcgg ttgctgcgcg aggaggactt tgagcccgac gcgcgaaccg ggattagtcc 8220

cgcgcgcgca cacgtggcgg ccgccgacct ggtaaccgca tacgagcaga cggtgaacca 8280

ggagattaac tttcaaaaaa gctttaacaa ccacgtgcgt acgcttgtgg cgcgcgagga 8340

ggtggctata ggactgatgc atctgtggga ctttgtaagc gcgctggagc aaaacccaaa 8400

tagcaagccg ctcatggcgc agctgttcct tatagtgcag cacagcaggg acaacgaggc 8460

attcagggat gcgctgctaa acatagtaga gcccgagggc cgctggctgc tcgatttgat 8520

aaacatcctg cagagcatag tggtgcagga gcgcagcttg agcctggctg acaaggtggc 8580

cgccatcaac tattccatgc ttagcctggg caagttttac gcccgcaaga tataccatac 8640

cccttacgtt cccatagaca aggaggtaaa gatcgagggg ttctacatgc gcatggcgct 8700

gaaggtgctt accttgagcg acgacctggg cgtttatcgc aacgagcgca tccacaaggc 8760

cgtgagcgtg agccggcggc gcgagctcag cgaccgcgag ctgatgcaca gcctgcaaag 8820

ggccctggct ggcacgggca gcggcgatag agaggccgag tcctactttg acgcgggcgc 8880

tgacctgcgc tgggccccaa gccgacgcgc cctggaggca gctggggccg gacctgggct 8940

ggcggtggca cccgcgcgcg ctggcaacgt cggcggcgtg gaggaatatg acgaggacga 9000

tgagtacgag ccagaggacg gcgagtacta agcggtgatg tttctgatca gatgatgcaa 9060

gacgcaacgg acccggcggt gcgggcggcg ctgcagagcc agccgtccgg ccttaactcc 9120

acggacgact ggcgccaggt catggaccgc atcatgtcgc tgactgcgcg caatcctgac 9180

gcgttccggc agcagccgca ggccaaccgg ctctccgcaa ttctggaagc ggtggtcccg 9240

gcgcgcgcaa accccacgca cgagaaggtg ctggcgatcg taaacgcgct ggccgaaaac 9300

agggccatcc ggcccgacga ggccggcctg gtctacgacg cgctgcttca gcgcgtggct 9360

cgttacaaca gcggcaacgt gcagaccaac ctggaccggc tggtggggga tgtgcgcgag 9420

gccgtggcgc agcgtgagcg cgcgcagcag cagggcaacc tgggctccat ggttgcacta 9480

aacgccttcc tgagtacaca gcccgccaac gtgccgcggg gacaggagga ctacaccaac 9540

tttgtgagcg cactgcggct aatggtgact gagacaccgc aaagtgaggt gtaccagtct 9600

gggccagact attttttcca gaccagtaga caaggcctgc agaccgtaaa cctgagccag 9660

gctttcaaaa acttgcaggg gctgtggggg gtgcgggctc ccacaggcga ccgcgcgacc 9720

gtgtctagct tgctgacgcc caactcgcgc ctgttgctgc tgctaatagc gcccttcacg 9780

gacagtggca gcgtgtcccg ggacacatac ctaggtcact tgctgacact gtaccgcgag 9840

gccataggtc aggcgcatgt ggacgagcat actttccagg agattacaag tgtcagccgc 9900

gcgctggggc aggaggacac gggcagcctg gaggcaaccc taaactacct gctgaccaac 9960

cggcggcaga agatcccctc gttgcacagt ttaaacagcg aggaggagcg cattttgcgc 10020

tacgtgcagc agagcgtgag ccttaacctg atgcgcgacg gggtaacgcc cagcgtggcg 10080

ctggacatga ccgcgcgcaa catggaaccg ggcatgtatg cctcaaaccg gccgtttatc 10140

aaccgcctaa tggactactt gcatcgcgcg gccgccgtga accccgagta tttcaccaat 10200

gccatcttga acccgcactg gctaccgccc cctggtttct acaccggggg attcgaggtg 10260

cccgagggta acgatggatt cctctgggac gacatagacg acagcgtgtt ttccccgcaa 10320

ccgcagaccc tgctagagtt gcaacagcgc gagcaggcag aggcggcgct gcgaaaggaa 10380

agcttccgca ggccaagcag cttgtccgat ctaggcgctg cggccccgcg gtcagatgct 10440

agtagcccat ttccaagctt gatagggtct cttaccagca ctcgcaccac ccgcccgcgc 10500

ctgctgggcg aggaggagta cctaaacaac tcgctgctgc agccgcagcg cgaaaaaaac 10560

ctgcctccgg catttcccaa caacgggata gagagcctag tggacaagat gagtagatgg 10620

aagacgtacg cgcaggagca cagggacgtg ccaggcccgc gcccgcccac ccgtcgtcaa 10680

aggcacgacc gtcagcgggg tctggtgtgg gaggacgatg actcggcaga cgacagcagc 10740

gtcctggatt tgggagggag tggcaacccg tttgcgcacc ttcgccccag gctggggaga 10800

atgttttaaa aaaaaaaaag catgatgcaa aataaaaaac tcaccaaggc catggcaccg 10860

agcgttggtt ttcttgtatt ccccttagta tgcggcgcgc ggcgatgtat gaggaaggtc 10920

ctcctccctc ctacgagagt gtggtgagcg cggcgccagt ggcggcggcg ctgggttctc 10980

ccttcgatgc tcccctggac ccgccgtttg tgcctccgcg gtacctgcgg cctaccgggg 11040

ggagaaacag catccgttac tctgagttgg cacccctatt cgacaccacc cgtgtgtacc 11100

tggtggacaa caagtcaacg gatgtggcat ccctgaacta ccagaacgac cacagcaact 11160

ttctgaccac ggtcattcaa aacaatgact acagcccggg ggaggcaagc acacagacca 11220
tcaatcttga cgaccggtcg cactggggcg gcgacctgaa aaccatcctg cataccaaca 11280
tgccaaatgt gaacgagttc atgtttacca ataagtttaa ggcgcgggtg atggtgtcgc 11340
gcttgcctac taaggacaat caggtggagc tgaaatacga gtgggtggag ttcacgctgc 11400
ccgagggcaa ctactccgag accatgacca tagaccttat gaacaacgcg atcgtggagc 11460
actacttgaa agtgggcaga cagaacgggg ttctggaaag cgacatcggg gtaaagtttg 11520
acacccgcaa cttcagactg gggtttgacc ccgtcactgg tcttgtcatg cctggggtat 11580
atacaaacga agccttccat ccagacatca ttttgctgcc aggatgcggg gtggacttca 11640
cccacagccg cctgagcaac ttgttgggca tccgcaagcg gcaacccttc caggagggct 11700
ttaggatcac ctacgatgat ctggagggtg gtaacattcc cgcactgttg gatgtggacg 11760
cctaccaggc gagcttgaaa gatgacaccg aacagggcgg gggtggcgca ggcggcagca 11820
acagcagtgg cagcggcgcg gaagagaact ccaacgcggc agccgcggca atgcagccgg 11880
tggaggacat gaacgatcat gccattcgcg gcgacacctt tgccacacgg gctgaggaga 11940
agcgcgctga ggccgaagca gcggccgaag ctgccgcccc cgctgcgcaa cccgaggtcg 12000
agaagcctca gaagaaaccg gtgatcaaac ccctgacaga ggacagcaag aaacgcagtt 12060
acaacctaat aagcaatgac agcaccttca cccagtaccg cagctggtac cttgcataca 12120
actacggcga ccctcagacc ggaatccgct catggaccct gctttgcact cctgacgtaa 12180
cctgcggctc ggagcaggtc tactggtcgt tgccagacat gatgcaagac cccgtgacct 12240
tccgctccac gcgccagatc agcaactttc cggtggtggg cgccgagctg ttgcccgtgc 12300
actccaagag cttctacaac gaccaggccg tctactccca actcatccgc cagtttacct 12360
ctctgaccca cgtgttcaat cgctttcccg agaaccagat tttggcgcgc ccgccagccc 12420
ccaccatcac caccgtcagt gaaaacgttc ctgctctcac agatcacggg acgctaccgc 12480
tgcgcaacag catcggagga gtccagcgag tgaccattac tgacgccaga cgccgcacct 12540
gcccctacgt ttacaaggcc ctgggcatag tctcgccgcg cgtcctatcg agccgcactt 12600
tttgagcaag catgtccatc cttatatcgc ccagcaataa cacaggctgg ggcctgcgct 12660
tcccaagcaa gatgtttggc ggggccaaga agcgctccga ccaacaccca gtgcgcgtgc 12720
gcgggcacta ccgcgcgccc tggggcgcgc acaaacgcgg ccgcactggg cgcaccaccg 12780
tcgatgacgc catcgacgcg gtggtggagg aggcgcgcaa ctacacgccc acgccgccac 12840
cagtgtccac agtggacgcg gccattcaga ccgtggtgcg cggagcccgg cgctatgcta 12900
aaatgaagag acggcggagg cgcgtagcac gtcgccaccg ccgccgaccc ggcactgccg 12960
cccaacgcgc ggcggcggcc ctgcttaacc gcgcacgtcg caccggccga cgggcggcca 13020

tgcgggccgc tcgaaggctg gccgcgggta ttgtcactgt gccccccagg tccaggcgac 13080

gagcggccgc cgcagcagcc gcggccatta gtgctatgac tcagggtcgc aggggcaacg 13140

tgtattgggt gcgcgactcg gttagcggcc tgcgcgtgcc cgtgcgcacc cgccccccgc 13200

gcaactagat tgcaagaaaa aactacttag actcgtactg ttgtatgtat ccagcggcgg 13260

cggcgcgcaa cgaagctatg tccaagcgca aaatcaaaga agagatgctc caggtcatcg 13320

cgccggagat ctatggcccc ccgaagaagg aagagcagga ttacaagccc cgaaagctaa 13380

agcgggtcaa aaagaaaaag aaagatgatg atgatgaact tgacgacgag gtggaactgc 13440

tgcacgctac cgcgcccagg cgacgggtac agtggaaagg tcgacgcgta aaacgtgttt 13500

tgcgacccgg caccaccgta gtctttacgc ccggtgagcg ctccacccgc acctacaagc 13560

gcgtgtatga tgaggtgtac ggcgacgagg acctgcttga gcaggccaac gagcgcctcg 13620

gggagtttgc ctacggaaag cggcataagg acatgctggc gttgccgctg gacgagggca 13680

acccaacacc tagcctaaag cccgtaacac tgcagcaggt gctgcccgcg cttgcaccgt 13740

ccgaagaaaa gcgcggccta aagcgcgagt ctggtgactt ggcacccacc gtgcagctga 13800

tggtacccaa gcgccagcga ctggaagatg tcttggaaaa aatgaccgtg gaacctgggc 13860

tggagcccga ggtccgcgtg cggccaatca agcaggtggc gccgggactg ggcgtgcaga 13920

ccgtggacgt tcagataccc actaccagta gcaccagtat tgccaccgcc acagagggca 13980

tggagacaca aacgtccccg gttgcctcag cggtggcgga tgccgcggtg caggcggtcg 14040

ctgcggccgc gtccaagacc tctacggagg tgcaaacgga cccgtggatg tttcgcgttt 14100

cagccccccg gcgcccgcgc ggttcgagga agtacggcgc cgccagcgcg ctactgcccg 14160

aatatgccct acatccttcc attgcgccta cccccggcta tcgtggctac acctaccgcc 14220

ccagaagacg agcaactacc cgacgccgaa ccaccactgg aacccgccgc cgccgtcgcc 14280

gtcgccagcc cgtgctggcc ccgatttccg tgcgcagggt ggctcgcgaa ggaggcagga 14340

ccctggtgct gccaacagcg cgctaccacc ccagcatcgt ttaaaagccg gtctttgtgg 14400

ttcttgcaga tatggccctc acctgccgcc tccgtttccc ggtgccggga ttccgaggaa 14460

gaatgcaccg taggaggggc atggccggcc acggcctgac gggcggcatg cgtcgtgcgc 14520

accaccggcg gcggcgcgcg tcgcaccgtc gcatgcgcgg cggtatcctg cccctcctta 14580

ttccactgat cgccgcggcg attggcgccg tgcccggaat tgcatccgtg gccttgcagg 14640

cgcagagaca ctgattaaaa acaagttgca tgtggaaaaa tcaaaataaa aagtctggac 14700

tctcacgctc gcttggtcct gtaactattt tgtagaatgg aagacatcaa ctttgcgtct 14760

ctggccccgc gacacggctc gcgcccgttc atgggaaact ggcaagatat cggcaccagc 14820

aatatgagcg gtggcgcctt cagctggggc tcgctgtgga gcggcattaa aaatttcggt 14880

tccaccgtta agaactatgg cagcaaggcc tggaacagca gcacaggcca gatgctgagg 14940

gataagttga aagagcaaaa tttccaacaa aaggtggtag atggcctggc ctctggcatt 15000

agcggggtgg tggacctggc caaccaggca gtgcaaaata agattaacag taagcttgat 15060

ccccgccctc ccgtagagga gcctccaccg gccgtggaga cagtgtctcc agaggggcgt 15120

ggcgaaaagc gtccgcgccc cgacagggaa gaaactctgg tgacgcaaat agacgagcct 15180

ccctcgtacg aggaggcact aaagcaaggc ctgcccacca cccgtcccat cgcgcccatg 15240

gctaccggag tgctgggcca gcacacaccc gtaacgctgg acctgcctcc ccccgccgac 15300

acccagcaga aacctgtgct gccaggcccg accgccgttg ttgtaacccg tcctagccgc 15360

gcgtccctgc gccgcgccgc cagcggtccg cgatcgttgc ggcccgtagc cagtggcaac 15420

tggcaaagca cactgaacag catcgtgggt ctgggggtgc aatccctgaa gcgccgacga 15480

tgcttctgaa tagctaacgt gtcgtatgtg tgtcatgtat gcgtccatgt cgccgccaga 15540

ggagctgctg agccgccgcg cgcccgcttt ccaagatggc taccccttcg atgatgccgc 15600

agtggtctta catgcacatc tcgggccagg acgcctcgga gtacctgagc cccgggctgg 15660

tgcagtttgc ccgcgccacc gagacgtact tcagcctgaa taacaagttt agaaacccca 15720

cggtggcgcc tacgcacgac gtgaccacag accggtccca gcgtttgacg ctgcggttca 15780

tccctgtgga ccgtgaggat actgcgtact cgtacaaggc gcggttcacc ctagctgtgg 15840

gtgataaccg tgtgctggac atggcttcca cgtactttga catccgcggc gtgctggaca 15900

ggggccctac ttttaagccc tactctggca ctgcctacaa cgccctggct cccaagggtg 15960

ccccaaatcc ttgcgaatgg gatgaagctg ctactgctct tgaaataaac ctagaagaag 16020

aggacgatga caacgaagac gaagtagacg agcaagctga gcagcaaaaa actcacgtat 16080

ttgggcaggc gccttattct ggtataaata ttacaaagga gggtattcaa ataggtgtcg 16140

aaggtcaaac acctaaatat gccgataaaa catttcaacc tgaacctcaa ataggagaat 16200

ctcagtggta cgaaactgaa attaatcatg cagctgggag agtccttaaa aagactaccc 16260

caatgaaacc atgttacggt tcatatgcaa aacccacaaa tgaaaatgga gggcaaggca 16320

ttcttgtaaa gcaacaaaat ggaaagctag aaagtcaagt ggaaatgcaa tttttctcaa 16380

ctactgaggc gaccgcaggc aatggtgata acttgactcc taaagtggta ttgtacagtg 16440

aagatgtaga tatagaaacc ccagacactc atatttctta catgcccact attaaggaag 16500

gtaactcacg agaactaatg ggccaacaat ctatgcccaa caggcctaat tacattgctt 16560

ttagggacaa ttttattggt ctaatgtatt acaacagcac gggtaatatg ggtgttctgg 16620

cgggccaagc atcgcagttg aatgctgttg tagatttgca agacagaaac acagagcttt 16680

cataccagct tttgcttgat tccattggtg atagaaccag gtacttttct atgtggaatc 16740

aggctgttga cagctatgat ccagatgtta gaattattga aaatcatgga actgaagatg 16800

aacttccaaa ttactgcttt ccactgggag gtgtgattaa tacagagact cttaccaagg 16860

taaaacctaa aacaggtcag gaaaatggat gggaaaaaga tgctacagaa ttttcagata 16920

aaaatgaaat aagagttgga aataattttg ccatggaaat caatctaaat gccaacctgt 16980

ggagaaattt cctgtactcc aacatagcgc tgtatttgcc cgacaagcta aagtacagtc 17040

cttccaacgt aaaaatttct gataacccaa acacctacga ctacatgaac aagcgagtgg 17100

tggctcccgg gttagtggac tgctacatta accttggagc acgctggtcc cttgactata 17160

tggacaacgt caacccattt aaccaccacc gcaatgctgg cctgcgctac cgctcaatgt 17220

tgctgggcaa tggtcgctat gtgcccttcc acatccaggt gcctcagaag ttctttgcca 17280

ttaaaaacct ccttctcctg ccgggctcat acacctacga gtggaacttc aggaaggatg 17340

ttaacatggt tctgcagagc tccctaggaa atgacctaag ggttgacgga gccagcatta 17400

agtttgatag catttgcctt tacgccacct tcttccccat ggcccacaac accgcctcca 17460

cgcttgaggc catgcttaga aacgacacca acgaccagtc ctttaacgac tatctctccg 17520

ccgccaacat gctctaccct atacccgcca acgctaccaa cgtgcccata tccatcccct 17580

cccgcaactg ggcggctttc cgcggctggg ccttcacgcg ccttaagact aaggaaaccc 17640

catcactggg ctcgggctac gacccttatt acacctactc tggctctata ccctacctag 17700

atggaacctt ttacctcaac cacaccttta agaaggtggc cattaccttt gactcttctg 17760

tcagctggcc tggcaatgac cgcctgctta cccccaacga gtttgaaatt aagcgctcag 17820

ttgacgggga gggttacaac gttgcccagt gtaacatgac caaagactgg ttcctggtac 17880

aaatgctagc taactacaac attggctacc agggcttcta tatcccagag agctacaagg 17940

accgcatgta ctccttcttt agaaacttcc agcccatgag ccgtcaggtg gtggatgata 18000

ctaaatacaa ggactaccaa caggtgggca tcctacacca acacaacaac tctggatttg 18060

ttggctacct tgcccccacc atgcgcgaag gacaggccta ccctgctaac ttcccctatc 18120

cgcttatagg caagaccgca gttgacagca ttacccagaa aaagtttctt tgcgatcgca 18180

ccctttggcg catcccattc tccagtaact ttatgtccat gggcgcactc acagacctgg 18240

gccaaaacct tctctacgcc aactccgccc acgcgctaga catgactttt gaggtggatc 18300

ccatggacga gcccaccctt ctttatgttt tgtttgaagt ctttgacgtg gtccgtgtgc 18360

accggccgca ccgcggcgtc atcgaaaccg tgtacctgcg cacgcccttc tcggccggca 18420

acgccacaac ataaagaagc aagcaacatc aacaacagct gccgccatgg gctccagtga 18480

gcaggaactg aaagccattg tcaaagatct tggttgtggg ccatattttt tgggcaccta 18540

tgacaagcgc tttccaggct ttgtttctcc acacaagctc gcctgcgcca tagtcaatac 18600

ggccggtcgc gagactgggg gcgtacactg gatggccttt gcctggaacc cgcactcaaa 18660

aacatgctac ctctttgagc cctttggctt ttctgaccag cgactcaagc aggtttacca 18720

gtttgagtac gagtcactcc tgcgccgtag cgccattgct tcttcccccg accgctgtat 18780

aacgctggaa aagtccaccc aaagcgtaca ggggcccaac tcggccgcct gtggactatt 18840

ctgctgcatg tttctccacg cctttgccaa ctggccccaa actcccatgg atcacaaccc 18900

caccatgaac cttattaccg gggtacccaa ctccatgctc aacagtcccc aggtacagcc 18960

caccctgcgt cgcaaccagg aacagctcta cagcttcctg gagcgccact cgccctactt 19020

ccgcagccac agtgcgcaga ttaggagcgc cacttctttt tgtcacttga aaaacatgta 19080

aaaataatgt actagagaca ctttcaataa aggcaaatgc ttttatttgt acactctcgg 19140

gtgattattt acccccaccc ttgccgtctg cgccgtttaa aaatcaaagg ggttctgccg 19200

cgcatcgcta tgcgccactg gcagggacac gttgcgatac tggtgtttag tgctccactt 19260

aaactcaggc acaaccatcc gcggcagctc ggtgaagttt tcactccaca ggctgcgcac 19320

catcaccaac gcgtttagca ggtcgggcgc cgatatcttg aagtcgcagt tggggcctcc 19380

gccctgcgcg cgcgagttgc gatacacagg gttgcagcac tggaacacta tcagcgccgg 19440

gtggtgcacg ctggccagca cgctcttgtc ggagatcaga tccgcgtcca ggtcctccgc 19500

gttgctcagg gcgaacggag tcaactttgg tagctgcctt cccaaaaagg gcgcgtgccc 19560

aggctttgag ttgcactcgc accgtagtgg catcaaaagg tgaccgtgcc cggtctgggc 19620

gttaggatac agcgcctgca taaaagcctt gatctgctta aaagccacct gagcctttgc 19680

gccttcagag aagaacatgc cgcaagactt gccggaaaac tgattggccg gacaggccgc 19740

gtcgtgcacg cagcaccttg cgtcggtgtt ggagatctgc accacatttc ggccccaccg 19800

gttcttcacg atcttggcct tgctagactg ctccttcagc gcgcgctgcc cgttttcgct 19860

cgtcacatcc atttcaatca cgtgctcctt atttatcata atgcttccgt gtagacactt 19920

aagctcgcct tcgatctcag cgcagcggtg cagccacaac gcgcagcccg tgggctcgtg 19980

atgcttgtag gtcacctctg caaacgactg caggtacgcc tgcaggaatc gccccatcat 20040

cgtcacaaag gtcttgttgc tggtgaaggt cagctgcaac ccgcggtgct cctcgttcag 20100

ccaggtcttg catacggccg ccagagcttc cacttggtca ggcagtagtt tgaagttcgc 20160

ctttagatcg ttatccacgt ggtacttgtc catcagcgcg cgcgcagcct ccatgccctt 20220

ctcccacgca gacacgatcg gcacactcag cgggttcatc accgtaattt cactttccgc 20280

ttcgctgggc tcttcctctt cctcttgcgt ccgcatacca cgcgccactg ggtcgtcttc 20340

attcagccgc cgcactgtgc gcttacctcc tttgccatgc ttgattagca ccggtgggtt 20400

gctgaaaccc accatttgta gcgccacatc ttctctttct tcctcgctgt ccacgattac 20460

ctctggtgat ggcgggcgct cgggcttggg agaagggcgc ttctttttct tcttgggcgc 20520

aatggccaaa tccgccgccg aggtcgatgg ccgcgggctg ggtgtgcgcg gcaccagcgc 20580

gtcttgtgat gagtcttcct cgtcctcgga ctcgatacgc cgcctcatcc gcttttttgg 20640

gggcgcccgg ggaggcggcg gcgacgggga cggggacgac acgtcctcca tggttggggg 20700

acgtcgcgcc gcaccgcgtc cgcgctcggg ggtggtttcg cgctgctcct cttcccgact 20760

ggccatttcc ttctcctata ggcagaaaaa gatcatggag tcagtcgaga agaaggacag 20820

cctaaccgcc ccctctgagt tcgccaccac cgcctccacc gatgccgcca acgcgcctac 20880

caccttcccc gtcgaggcac ccccgcttga ggaggaggaa gtgattatcg agcaggaccc 20940

aggttttgta agcgaagacg acgaggaccg ctcagtacca acagaggata aaaagcaaga 21000

ccaggacaac gcagaggcaa acgaggaaca agtcgggcgg ggggacgaaa ggcatggcga 21060

ctacctagat gtgggagacg acgtgctgtt gaagcatctg cagcgccagt gcgccattat 21120

ctgcgacgcg ttgcaagagc gcagcgatgt gcccctcgcc atagcggatg tcagccttgc 21180

ctacgaacgc cacctattct caccgcgcgt accccccaaa cgccaagaaa acggcacatg 21240

cgagcccaac ccgcgcctca acttctaccc cgtatttgcc gtgccagagg tgcttgccac 21300

ctatcacatc tttttccaaa actgcaagat acccctatcc tgccgtgcca accgcagccg 21360

agcggacaag cagctggcct tgcggcaggg cgctgtcata cctgatatcg cctcgctcaa 21420

cgaagtgcca aaaatctttg agggtcttgg acgcgacgag aagcgcgcgg caaacgctct 21480

gcaacaggaa aacagcgaaa atgaaagtca ctctggagtg ttggtggaac tcgagggtga 21540

caacgcgcgc ctagccgtac taaaacgcag catcgaggtc acccactttg cctacccggc 21600

acttaaccta ccccccaagg tcatgagcac agtcatgagt gagctgatcg tgcgccgtgc 21660

gcagcccctg gagagggatg caaatttgca agaacaaaca gaggagggcc tacccgcagt 21720

tggcgacgag cagctagcgc gctggcttca aacgcgcgag cctgccgact tggaggagcg 21780

acgcaaacta atgatggccg cagtgctcgt taccgtggag cttgagtgca tgcagcggtt 21840

ctttgctgac ccggagatgc agcgcaagct agaggaaaca ttgcactaca cctttcgaca 21900

gggctacgta cgccaggcct gcaagatctc caacgtggag ctctgcaacc tggtctccta 21960

ccttggaatt ttgcacgaaa accgccttgg gcaaaacgtg cttcattcca cgctcaaggg 22020

cgaggcgcgc cgcgactacg tccgcgactg cgtttactta tttctatgct acacctggca 22080

gacggccatg ggcgtttggc agcagtgctt ggaggagtgc aacctcaagg agctgcagaa 22140

actgctaaag caaaacttga aggacctatg gacggccttc aacgagcgct ccgtggccgc 22200

gcacctggcg gacatcattt tccccgaacg cctgcttaaa accctgcaac agggtctgcc 22260

agacttcacc agtcaaagca tgttgcagaa ctttaggaac tttatcctag agcgctcagg 22320

aatcttgccc gccacctgct gtgcacttcc tagcgacttt gtgcccatta agtaccgcga 22380

atgccctccg ccgctttggg gccactgcta ccttctgcag ctagccaact accttgccta 22440

ccactctgac ataatggaag acgtgagcgg tgacggtcta ctggagtgtc actgtcgctg 22500

caacctatgc accccgcacc gctccctggt ttgcaattcg cagctgctta acgaaagtca 22560
aattatcggt acctttgagc tgcagggtcc ctcgcctgac gaaaagtccg cggctccggg 22620
gttgaaactc actccggggc tgtggacgtc ggcttacctt cgcaaatttg tacctgagga 22680
ctaccacgcc cacgagatta ggttctacga agaccaatcc cgcccgccaa atgcggagct 22740
taccgcctgc gtcattaccc agggccacat tcttggccaa ttgcaagcca tcaacaaagc 22800
ccgccaagag tttctgctac gaaagggacg gggggtttac ttggaccccc agtccggcga 22860
ggagctcaac ccaatccccc cgccgccgca gccctatcag cagcagccgc gggcccttgc 22920
ttcccaggat ggcacccaaa aagaagctgc agctgccgcc gccacccacg gacgaggagg 22980
aatactggga cagtcaggca gaggaggttt tggacgagga ggaggaggac atgatggaag 23040
actgggagag cctagacgag gaagcttccg aggtcgaaga ggtgtcagac gaaacaccgt 23100
caccctcggt cgcattcccc tcgccggcgc cccagaaatc ggcaaccggt tccagcatgg 23160
ctacaacctc cgctcctcag gcgccgccgg cactgcccgt tcgccgaccc aaccgtagat 23220
gggacaccac tggaaccagg gccggtaagt ccaagcagcc gccgccgtta gcccaagagc 23280
aacaacagcg ccaaggctac cgctcatggc gcgggcacaa gaacgccata gttgcttgct 23340
tgcaagactg tgggggcaac atctccttcg cccgccgctt tcttctctac catcacggcg 23400
tggccttccc ccgtaacatc ctgcattact accgtcatct ctacagccca tactgcaccg 23460
gcggcagcgg cagcggcagc aacagcagcg gccacacaga agcaaaggcg accggatagc 23520
aagactctga caaagcccaa gaaatccaca gcggcggcag cagcaggagg aggagcgctg 23580
cgtctggcgc ccaacgaacc cgtatcgacc cgcgagctta gaaacaggat ttttcccact 23640
ctgtatgcta tatttcaaca gagcaggggc caagaacaag agctgaaaat aaaaaacagg 23700
tctctgcgat ccctcacccg cagctgcctg tatcacaaaa gcgaagatca gcttcggcgc 23760
acgctggaag acgcggaggc tctcttcagt aaatactgcg cgctgactct taaggactag 23820
tttcgcgccc tttctcaaat ttaagcgcga aaactacgtc atctccagcg gccacacccg 23880
gcgccagcac ctgtcgtcag cgccattatg agcaaggaaa ttcccacgcc ctacatgtgg 23940
agttaccagc cacaaatggg acttgcggct ggagctgccc aagactactc aacccgaata 24000
aactacatga gcgcgggacc ccacatgata tcccgggtca acggaatccg cgcccaccga 24060
aaccgaattc tcttggaaca ggcggctatt accaccacac ctcgtaataa ccttaatccc 24120
cgtagttggc ccgctgccct ggtgtaccag gaaagtcccg ctcccaccac tgtggtactt 24180
cccagagacg cccaggccga agttcagatg actaactcag gggcgcagct tgcgggcggc 24240
tttcgtcaca gggtgcggtc gcccgggcag ggtataactc acctgacaat cagagggcga 24300
ggtattcagc tcaacgacga gtcggtgagc tcctcgcttg gtctccgtcc ggacgggaca 24360

tttcagatcg gcggcgccgg ccgtccttca ttcacgcctc gtcaggcaat cctaactctg 24420

cagacctcgt cctctgagcc gcgctctgga ggcattggaa ctctgcaatt tattgaggag 24480

tttgtgccat cggtctactt taaccccttc tcgggacctc ccggccacta tccggatcaa 24540

tttattccta actttgacgc ggtaaaggac tcggcggacg gctacgactg aatgttaagt 24600

ggagaggcag agcaactgcg cctgaaacac ctggtccact gtcgccgcca caagtgcttt 24660

gcccgcgact ccggtgagtt ttgctacttt gaattgcccg aggatcatat cgagggcccg 24720

gcgcacggcg tccggcttac cgcccaggga gagcttgccc gtagcctgat tcgggagttt 24780

acccagcgcc ccctgctagt tgagcgggac aggggaccct gtgttctcac tgtgatttgc 24840

aactgtccta accttggatt acatcaagat ctttgttgcc atctctgtgc tgagtataat 24900

aaatacagaa attaaaatat actggggctc ctatcgccat cctgtaaacg ccaccgtctt 24960

cacccgccca agcaaaccaa ggcgaacctt acctggtact tttaacatct ctccctctgt 25020

gatttacaac agtttcaacc cagacggagt gagtctacga gagaacctct ccgagctcag 25080

ctactccatc agaaaaaaca ccaccctcct tacctgccgg gaacgtacga gtgcgtcacc 25140

ggccgctgca ccacacctac cgcctgaccg taaaccagac tttttccgga cagacctcaa 25200

taactctgtt taccagaaca ggaggtgagc ttagaaaacc cttagggtat taggccaaag 25260

gcgcagctac tgtggggttt atgaacaatt caagcaactc tacgggctat tctaattcag 25320

gtttctctag aaatggacgg aattattaca gagcagcgcc tgctagaaag acgcagggca 25380

gcggccgagc aacagcgcat gaatcaagag ctccaagaca tggttaactt gcaccagtgc 25440

aaaaggggta tcttttgtct ggtaaagcag gccaaagtca cctacgacag taataccacc 25500

ggacaccgcc ttagctacaa gttgccaacc aagcgtcaga aattggtggt catggtggga 25560

gaaaagccca ttaccataac tcagcactcg gtagaaaccg aaggctgcat tcactcacct 25620

tgtcaaggac ctgaggatct ctgcaccctt attaagaccc tgtgcggtct caaagatctt 25680

attcccttta actaataaaa aaaaataata aagcatcact tacttaaaat cagttagcaa 25740

atttctgtcc agtttattca gcagcacctc cttgccctcc tcccagctct ggtattgcag 25800

cttcctcctg gctgcaaact ttctccacaa tctaaatgga atgtcagttt cctcctgttc 25860

ctgtccatcc gcacccacta tcttcatgtt gttgcagatg aagcgcgcaa gaccgtctga 25920

agataccttc aaccccgtgt atccatatga cacggaaacc ggtcctccaa ctgtgccttt 25980

tcttactcct ccctttgtat cccccaatgg gtttcaagag agtccccctg gggtactctc 26040

tttgcgccta tccgaacctc tagttacctc caatggcatg cttgcgctca aaatgggcaa 26100

cggcctctct ctggacgagg ccggcaacct tacctcccaa aatgtaacca ctgtgagccc 26160

acctctcaaa aaaaccaagt caaacataaa cctggaaata tctgcacccc tcacagttac 26220

ctcagaagcc ctaactgtgg ctgccgccgc acctctaatg gtcgcgggca acacactcac 26280

catgcaatca caggccccgc taaccgtgca cgactccaaa cttagcattg ccacccaagg 26340

acccctcaca gtgtcagaag gaaagctagc cctgcaaaca tcaggccccc tcaccaccac 26400

cgatagcagt acccttacta tcactgcctc accccctcta actactgcca ctggtagctt 26460

gggcattgac ttgaaagagc ccatttatac acaaaatgga aaactaggac taaagtacgg 26520

ggctcctttg catgtaacag acgacctaaa cactttgacc gtagcaactg gtccaggtgt 26580

gactattaat aatacttcct tgcaaactaa agttactgga gccttgggtt ttgattcaca 26640

aggcaatatg caacttaatg tagcaggagg actaaggatt gattctcaaa acagacgcct 26700

tatacttgat gttagttatc cgtttgatgc tcaaaaccaa ctaaatctaa gactaggaca 26760

gggccctctt tttataaact cagcccacaa cttggatatt aactacaaca aaggccttta 26820

cttgtttaca gcttcaaaca attccaaaaa gcttgaggtt aacctaagca ctgccaaggg 26880

gttgatgttt gacgctacag ccatagccat taatgcagga gatgggcttg aatttggttc 26940

acctaatgca ccaaacacaa atcccctcaa aacaaaaatt ggccatggcc tagaatttga 27000

ttcaaacaag gctatggttc ctaaactagg aactggcctt agttttgaca gcacaggtgc 27060

cattacagta ggaaacaaaa ataatgataa gctaactttg tggaccacac cagctccatc 27120

tcctaactgt agactaaatg cagagaaaga tgctaaactc actttggtct taacaaaatg 27180

tggcagtcaa atacttgcta cagtttcagt tttggctgtt aaaggcagtt tggctccaat 27240

atctggaaca gttcaaagtg ctcatcttat tataagattt gacgaaaatg gagtgctact 27300

aaacaattcc ttcctggacc cagaatattg gaactttaga aatggagatc ttactgaagg 27360

cacagcctat acaaacgctg ttggatttat gcctaaccta tcagcttatc caaaatctca 27420

cggtaaaact gccaaaagta acattgtcag tcaagtttac ttaaacggag acaaaactaa 27480

acctgtaaca ctaaccatta cactaaacgg tacacaggaa acaggagaca caactccaag 27540

tgcatactct atgtcatttt catgggactg gtctggccac aactacatta atgaaatatt 27600

tgccacatcc tcttacactt tttcatacat tgcccaagaa taaagaatcg tttgtgttat 27660

gtttcaacgt gtttattttt caattgcaga aaatttcgaa tcatttttca ttcagtagta 27720

tagccccacc accacatagc ttatacagat caccgtacct taatcaaact cacagaaccc 27780

tagtattcaa cctgccacct ccctcccaac acacagagta cacagtcctt tctccccggc 27840

tggccttaaa aagcatcata tcatgggtaa cagacatatt cttaggtgtt atattccaca 27900

cggtttcctg tcgagccaaa cgctcatcag tgatattaat aaactccccg ggcagctcac 27960

ttaagttcat gtcgctgtcc agctgctgag ccacaggctg ctgtccaact tgcggttgct 28020

taacgggcgg cgaaggagaa gtccacgcct acatgggggt agagtcataa tcgtgcatca 28080

ggatagggcg gtggtgctgc agcagcgcgc gaataaactg ctgccgccgc cgctccgtcc 28140

tgcaggaata caacatggca gtggtctcct cagcgatgat tcgcaccgcc cgcagcataa 28200
ggcgccttgt cctccgggca cagcagcgca ccctgatctc acttaaatca gcacagtaac 28260
tgcagcacag caccacaata ttgttcaaaa tcccacagtg caaggcgctg tatccaaagc 28320
tcatggcggg gaccacagaa cccacgtggc catcatacca caagcgcagg tagattaagt 28380
ggcgacccct cataaacacg ctggacataa acattacctc ttttggcatg ttgtaattca 28440
ccacctcccg gtaccatata aacctctgat taaacatggc gccatccacc accatcctaa 28500
accagctggc caaaacctgc ccgccggcta tacactgcag ggaaccggga ctggaacaat 28560
gacagtggag agcccaggac tcgtaaccat ggatcatcat gctcgtcatg atatcaatgt 28620
tggcacaaca caggcacacg tgcatacact tcctcaggat tacaagctcc tcccgcgtta 28680
gaaccatatc ccagggaaca acccattcct gaatcagcgt aaatcccaca ctgcagggaa 28740
gacctcgcac gtaactcacg ttgtgcattg tcaaagtgtt acattcgggc agcagcggat 28800
gatcctccag tatggtagcg cgggtttctg tctcaaaagg aggtagacga tccctactgt 28860
acggagtgcg ccgagacaac cgagatcgtg ttggtcgtag tgtcatgcca aatggaacgc 28920
cggacgtagt catatttcct gaagcaaaac caggtgcggg cgtgacaaac agatctgcgt 28980
ctccggtctc gccgcttaga tcgctctgtg tagtagttgt agtatatcca ctctctcaaa 29040
gcatccaggc gccccctggc ttcgggttct atgtaaactc cttcatgcgc cgctgccctg 29100
ataacatcca ccaccgcaga ataagccaca cccagccaac ctacacattc gttctgcgag 29160
tcacacacgg gaggagcggg aagagctgga agaaccatgt tttttttttt attccaaaag 29220
attatccaaa acctcaaaat gaagatctat taagtgaacg cgctcccctc cggtggcgtg 29280
gtcaaactct acagccaaag aacagataat ggcatttgta agatgttgca caatggcttc 29340
caaaaggcaa acggccctca cgtccaagtg gacgtaaagg ctaaaccctt cagggtgaat 29400
ctcctctata aacattccag caccttcaac catgcccaaa taattctcat ctcgccacct 29460
tctcaatata tctctaagca aatcccgaat attaagtccg gccattgtaa aaatctgctc 29520
cagagcgccc tccaccttca gcctcaagca gcgaatcatg attgcaaaaa ttcaggttcc 29580
tcacagacct gtataagatt caaaagcgga acattaacaa aaataccgcg atcccgtagg 29640
tcccttcgca gggccagctg aacataatcg tgcaggtctg cacggaccag cgcggccact 29700
tccccgccag gaaccttgac aaaagaaccc acactgatta tgacacgcat actcggagct 29760
atgctaacca gcgtagcccc gatgtaagct ttgttgcatg ggcggcgata taaaatgcaa 29820
ggtgctgctc aaaaaatcag gcaaagcctc gcgcaaaaaa gaaagcacat cgtagtcatg 29880
ctcatgcaga taaaggcagg taagctccgg aaccaccaca gaaaaagaca ccatttttct 29940
ctcaaacatg tctgcgggtt tctgcataaa cacaaaataa aataacaaaa aaacatttaa 30000
acattagaag cctgtcttac aacaggaaaa acaaccctta taagcataag acggactacg 30060

gccatgccgg cgtgaccgta aaaaaactgg tcaccgtgat taaaaagcac caccgacagc 30120

tcctcggtca tgtccggagt cataatgtaa gactcggtaa acacatcagg ttgattcaca 30180

tcggtcagtg ctaaaaagcg accgaaatag cccgggggaa tacatacccg caggcgtaga 30240

gacaacatta cagcccccat aggaggtata acaaaattaa taggagagaa aaacacataa 30300

acacctgaaa aaccctcctg cctaggcaaa atagcaccct cccgctccag aacaacatac 30360

agcgcttcca cagcggcagc cataacagtc agccttacca gtaaaaaaga aaacctatta 30420

aaaaaacacc actcgacacg gcaccagctc aatcagtcac agtgtaaaaa agggccaagt 30480

gcagagcgag tatatatagg actaaaaaat gacgtaacgg ttaaagtcca caaaaaacac 30540

ccagaaaacc gcacgcgaac ctacgcccag aaacgaaagc caaaaaaccc acaacttcct 30600

caaatcgtca cttccgtttt cccacgttac gtcacttccc attttaagaa aactacaatt 30660

cccaacacat acaagttact ccgccctaaa acctacgtca cccgccccgt tcccacgccc 30720

cgcgccacgt cacaaactcc accccctcat tatcatattg gcttcaatcc aaaataaggt 30780

atattattga tgatgttaat taagggcggc cgcacgggcc atcgatgggg atccatccgc 30840

ggagaagctt ctcgaccaat tctcatgttt gacagcttat catcgaattt ctgccattca 30900

tccgcttatt atcacttatt caggcgtagc aaccaggcgt ttaagggcac caataactgc 30960

cttaaaaaaa ttacgccccg ccctgccact catcgcagta ctgttgtaat tcattaagca 31020

ttctgccgac atggaagcca tcacagacgg catgatgaac ctgaatcgcc agcggcatca 31080

gcaccttgtc gccttgcgta taatatttgc ccatggtgaa aacgggggcg aagaagttgt 31140

ccatattggc cacgtttaaa tcaaaactgg tgaaactcac ccagggattg gctgagacga 31200

aaaacatatt ctcaataaac cctttaggga aataggccag gttttcaccg taacacgcca 31260

catcttgcga atatatgtgt agaaactgcc ggaaatcgtc gtggtattca ctccagagcg 31320

atgaaaacgt ttcagtttgc tcatggaaaa cggtgtaaca agggtgaaca ctatcccata 31380

tcaccagctc accgtctttc attgccatac ggaattccgg atgagcattc atcaggcggg 31440

caagaatgtg aataaaggcc ggataaaact tgtgcttatt tttctttacg gtctttaaaa 31500

aggccgtaat atccagctga acggtctggt tataggtaca ttgagcaact gactgaaatg 31560

cctcaaaatg ttctttacga tgccattggg atatatcaac ggtggtatat ccagtgattt 31620

ttttctccat tttagcttcc ttagctcctg aaaatctcga taactcaaaa aatacgcccg 31680

gtagtgatct tatttcatta tggtgaaagt tggaacctct tacgtgccga tcaacgtctc 31740

attttcgcca aaagttggcc cagggcttcc cggtatcaac agggacacca ggatttattt 31800

attctgcgaa gtgatcttcc gtcacaggta tttattcgcg ataagctcat ggagcggcgt 31860

aaccgtcgca caggaaggac agagaaagcg cggatctggg aagtgacgga cagaacggtc 31920

aggacctgga ttggggaggc ggttgccgcc gctgctgctg acggtgtgac gttctctgtt 31980

ccggtcacac cacatacgtt ccgccattcc tatgcgatgc acatgctgta tgccggtata 32040

ccgctgaaag ttctgcaaag cctgatggga cataagtcca tcagttcaac ggaagtctac 32100

acgaaggttt ttgcgctgga tgtggctgcc cggcaccggg tgcagtttgc gatgccggag 32160

tctgatgcgg ttgcgatgct gaaacaatta tcctgagaat aaatgccttg gcctttatat 32220

ggaaatgtgg aactgagtgg atatgctgtt tttgtctgtt aaacagagaa gctggctgtt 32280

atccactgag aagcgaacga aacagtcggg aaaatctccc attatcgtag agatccgcat 32340

tattaatctc aggagcctgt gtagcgttta taggaagtag tgttctgtca tgatgcctgc 32400

aagcggtaac gaaaacgatt tgaatatgcc ttcaggaaca atagaaatct tcgtgcggtg 32460

ttacgttgaa gtggagcgga ttatgtcagc aatggacaga acaacctaat gaacacagaa 32520

ccatgatgtg gtctgtcctt ttacagccag taggctcgcc gcagtcgagc gacggcgaag 32580

ccctcgagtg agcgaggaag caccagggaa cagcacttat atattctgct tacacacgat 32640

gcctgaaaaa acttcccttg gggttatcca cttatccacg gggatatttt tataattatt 32700

ttttttatag tttttagatc ttctttttta gagcgccttg taggccttta tccatgctgg 32760

ttctagagaa ggtgttgtga caaattgccc tttcagtgtg acaaatcacc ctcaaatgac 32820

agtcctgtct gtgacaaatt gcccttaacc ctgtgacaaa ttgccctcag aagaagctgt 32880

tttttcacaa agttatccct gcttattgac tcttttttat ttagtgtgac aatctaaaaa 32940

cttgtcacac ttcacatgga tctgtcatgg cggaaacagc ggttatcaat cacaagaaac 33000

gtaaaaatag cccgcgaatc gtccagtcaa acgacctcac tgaggcggca tatagtctct 33060

cccgggatca aaaacgtatg ctgtatctgt tcgttgacca gatcagaaaa tctgatggca 33120

ccctacagga acatgacggt atctgcgaga tccatgttgc taaatatgct gaaatattcg 33180

gattgacctc tgcggaagcc agtaaggata tacggcaggc attgaagagt ttcgcgggga 33240

aggaagtggt tttttatcgc cctgaagagg atgccggcga tgaaaaaggc tatgaatctt 33300

ttccttggtt tatcaaacgt gcgcacagtc catccagagg gctttacagt gtacatatca 33360

acccatatct cattcccttc tttatcgggt tacagaaccg gtttacgcag tttcggctta 33420

gtgaaacaaa agaaatcacc aatccgtatg ccatgcgttt atacgaatcc ctgtgtcagt 33480

atcgtaagcc ggatggctca ggcatcgtct ctctgaaaat cgactggatc atagagcgtt 33540

accagctgcc tcaaagttac cagcgtatgc ctgacttccg ccgccgcttc ctgcaggtct 33600

gtgttaatga gatcaacagc agaactccaa tgcgcctctc atacattgag aaaaagaaag 33660

gccgccagac gactcatatc gtattttcct tccgcgatat cacttccatg acgacaggat 33720

agtctgaggg ttatctgtca cagatttgag ggtggttcgt cacatttgtt ctgacctact 33780

gagggtaatt tgtcacagtt ttgctgtttc cttcagcctg catggatttt ctcatacttt 33840

ttgaactgta atttttaagg aagccaaatt tgagggcagt ttgtcacagt tgatttcctt 33900
ctctttccct tcgtcatgtg acctgatatc gggggttagt tcgtcatcat tgatgagggt 33960
tgattatcac agtttattac tctgaattgg ctatccgcgt gtgtacctct acctggagtt 34020
tttcccacgg tggatatttc ttcttgcgct gagcgtaaga gctatctgac agaacagttc 34080
ttctttgctt cctcgccagt tcgctcgcta tgctcggtta cacggctgcg gcgagcgcta 34140
gtgataataa gtgactgagg tatgtgctct tcttatctcc ttttgtagtg ttgctcttat 34200
tttaaacaac tttgcggttt tttgatgact ttgcgatttt gttgttgctt tgcagtaaat 34260
tgcaagattt aataaaaaaa cgcaaagcaa tgattaaagg atgttcagaa tgaaactcat 34320
ggaaacactt aaccagtgca taaacgctgg tcatgaaatg acgaaggcta tcgccattgc 34380
acagtttaat gatgacagcc cggaagcgag gaaaataacc cggcgctgga gaataggtga 34440
agcagcggat ttagttgggg tttcttctca ggctatcaga gatgccgaga aagcagggcg 34500
actaccgcac ccggatatgg aaattcgagg acgggttgag caacgtgttg gttatacaat 34560
tgaacaaatt aatcatatgc gtgatgtgtt tggtacgcga ttgcgacgtg ctgaagacgt 34620
atttccaccg gtgatcgggg ttgctgccca taaaggtggc gtttacaaaa cctcagtttc 34680
tgttcatctt gctcaggatc tggctctgaa ggggctacgt gttttgctcg tggaaggtaa 34740
cgacccccag ggaacagcct caatgtatca cggatgggta ccagatcttc atattcatgc 34800
agaagacact ctcctgcctt tctatcttgg ggaaaaggac gatgtcactt atgcaataaa 34860
gcccacttgc tggccggggc ttgacattat tccttcctgt ctggctctgc accgtattga 34920
aactgagtta atgggcaaat ttgatgaagg taaactgccc accgatccac acctgatgct 34980
ccgactggcc attgaaactg ttgctcatga ctatgatgtc atagttattg acagcgcgcc 35040
taacctgggt atcggcacga ttaatgtcgt atgtgctgct gatgtgctga ttgttcccac 35100
gcctgctgag ttgtttgact acacctccgc actgcagttt ttcgatatgc ttcgtgatct 35160
gctcaagaac gttgatctta aagggttcga gcctgatgta cgtattttgc ttaccaaata 35220
cagcaatagt aatggctctc agtccccgtg gatggaggag caaattcggg atgcctgggg 35280
aagcatggtt ctaaaaaatg ttgtacgtga aacggatgaa gttggtaaag gtcagatccg 35340
gatgagaact gtttttgaac aggccattga tcaacgctct tcaactggtg cctggagaaa 35400
tgctctttct atttgggaac ctgtctgcaa tgaaattttc gatcgtctga ttaaaccacg 35460
ctgggagatt agataatgaa gcgtgcgcct gttattccaa aacatacgct caatactcaa 35520
ccggttgaag atacttcgtt atcgacacca gctgccccga tggtggattc gttaattgcg 35580
cgcgtaggag taatggctcg cggtaatgcc attactttgc ctgtatgtgg tcgggatgtg 35640
aagtttactc ttgaagtgct ccggggtgat agtgttgaga agacctctcg ggtatggtca 35700

ggtaatgaac gtgaccagga gctgcttact gaggacgcac tggatgatct catcccttct 35760

tttctactga ctggtcaaca gacaccggcg ttcggtcgaa gagtatctgg tgtcatagaa 35820

attgccgatg ggagtcgccg tcgtaaagct gctgcactta ccgaaagtga ttatcgtgtt 35880

ctggttggcg agctggatga tgagcagatg gctgcattat ccagattggg taacgattat 35940

cgcccaacaa gtgcttatga acgtggtcag cgttatgcaa gccgattgca gaatgaattt 36000

gctggaaata tttctgcgct ggctgatgcg gaaaatattt cacgtaagat tattacccgc 36060

tgtatcaaca ccgccaaatt gcctaaatca gttgttgctc ttttttctca ccccggtgaa 36120

ctatctgccc ggtcaggtga tgcacttcaa aaagccttta cagataaaga ggaattactt 36180

aagcagcagg catctaacct tcatgagcag aaaaaagctg gggtgatatt tgaagctgaa 36240

gaagttatca ctcttttaac ttctgtgctt aaaacgtcat ctgcatcaag aactagttta 36300

agctcacgac atcagtttgc tcctggagcg acagtattgt ataagggcga taaaatggtg 36360

cttaacctgg acaggtctcg tgttccaact gagtgtatag agaaaattga ggccattctt 36420

aaggaacttg aaaagccagc accctgatgc gaccacgttt tagtctacgt ttatctgtct 36480

ttacttaatg tcctttgtta caggccagaa agcataactg gcctgaatat tctctctggg 36540

cccactgttc cacttgtatc gtcggtctga taatcagact gggaccacgg tcccactcgt 36600

atcgtcggtc tgattattag tctgggacca cggtcccact cgtatcgtcg gtctgattat 36660

tagtctggga ccacggtccc actcgtatcg tcggtctgat aatcagactg ggaccacggt 36720

cccactcgta tcgtcggtct gattattagt ctgggaccat ggtcccactc gtatcgtcgg 36780

tctgattatt agtctgggac cacggtccca ctcgtatcgt cggtctgatt attagtctgg 36840

aaccacggtc ccactcgtat cgtcggtctg attattagtc tgggaccacg gtcccactcg 36900

tatcgtcggt ctgattatta gtctgggacc acgatcccac tcgtgttgtc ggtctgatta 36960

tcggtctggg accacggtcc cacttgtatt gtcgatcaga ctatcagcgt gagactacga 37020

ttccatcaat gcctgtcaag ggcaagtatt gacatgtcgt cgtaacctgt agaacggagt 37080

aacctcggtg tgcggttgta tgcctgctgt ggattgctgc tgtgtcctgc ttatccacaa 37140

cattttgcgc acggttatgt ggacaaaata cctggttacc caggccgtgc cggcacgtta 37200

accgggctgc atccgatgca agtgtgtcgc tgtcgagttt tcgggggagt ccaggttttt 37260

cccagtcacg acgttgtaaa acgacggcca gtgaattcga gctcggtacc cggggatctt 37320

<210> 3
<211> 36680
<212> DNA
<213> Artificial

<220>
<223> synthetic

<220>
<221>  misc_feature
<222>  (27952)..(27952)
<223>  n is a, c, g, or t

<220>
<221>  misc_feature
<222>  (30222)..(30223)
<223>  n is a, c, g, or t

<400>  3
catcatcaat aatatacctt attttggatt gaagccaata tgataatgag ggggtggagt      60

ttgtgacgtg gcgcggggcg tgggaacggg gcgggtgacg tagtagtgtg gcggaagtgt     120

gatgttgcaa gtgtggcgga acacatgtaa gcgacggatg tggcaaaagt gacgtttttg     180

gtgtgcgccg gtgtacacag gaagtgacaa ttttcgcgcg gttttaggcg gatgttgtag     240

taaatttggg cgtaaccgag taagatttgg ccattttcgc gggaaaactg aataagagga     300

agtgaaatct gaataatttt gtgttactca tagcgcgtaa actggtaccc ggggatcttg     360

aagttcctat tccgaagttc ctattctcta gaaagtatag gaacttcaag agcgcttttg     420

agcttgttta tcgaattcaa gcttgtcgac tcgaagatct gggcgtggtt aagggtggga     480

aagaatatat aaggtggggg tcttatgtag ttttgtatct gttttgcagc agccgccgcc     540

gccatgagca ccaactcgtt tgatggaagc attgtgagct catatttgac aacgcgcatg     600

cccccatggg ccggggtgcg tcagaatgtg atgggctcca gcattgatgg tcgccccgtc     660

ctgcccgcaa actctactac cttgacctac gagaccgtgt ctggaacgcc gttggagact     720

gcagcctccg ccgccgcttc agccgctgca gccaccgccc gcgggattgt gactgacttt     780

gctttcctga gcccgcttgc aagcagtgca gcttcccgtt catccgcccg cgatgacaag     840

ttgacggctc ttttggcaca attggattct ttgacccggg aacttaatgt cgtttctcag     900

cagctgttgg atctgcgcca gcaggtttct gccctgaagg cttcctcccc tcccaatgcg     960

gtttaaaaca taaataaaaa accagactct gtttggattt ggatcaagca agtgtcttgc    1020

tgtctttatt taggggtttt gcgcgcgcgg taggcccggg accagcggtc tcggtcgttg    1080

agggtcctgt gtattttttc caggacgtgg taaaggtgac tctggatgtt cagatacatg    1140

ggcataagcc cgtctctggg gtggaggtag caccactgca gagcttcatg ctgcggggtg    1200

gtgttgtaga tgatccagtc gtagcaggag cgctgggcgt ggtgcctaaa aatgtctttc    1260

agtagcaagc tgattgccag gggcaggccc ttggtgtaag tgtttacaaa gcggttaagc    1320

tgggatgggt gcatacgtgg ggatatgaga tgcatcttgg actgtatttt taggttggct    1380

atgttcccag ccatatccct ccggggattc atgttgtgca gaaccaccag cacagtgtat    1440

ccggtgcact tgggaaattt gtcatgtagc ttagaaggaa atgcgtggaa gaacttggag    1500

acgcccttgt gacctccaag attttccatg cattcgtcca taatgatggc aatgggccca    1560

cgggcggcgg cctgggcgaa gatatttctg ggatcactaa cgtcatagtt gtgttccagg 1620

atgagatcgt cataggccat ttttacaaag cgcgggcgga gggtgccaga ctgcggtata 1680

atggttccat ccggcccagg ggcgtagtta ccctcacaga tttgcatttc ccacgctttg 1740

agttcagatg gggggatcat gtctacctgc ggggcgatga agaaaacggt ttccggggta 1800

ggggagatca gctgggaaga aagcaggttc ctgagcagct gcgacttacc gcagccggtg 1860

ggcccgtaaa tcacacctat taccgggtgc aactggtagt taagagagct gcagctgccg 1920

tcatccctga gcaggggggc cacttcgtta agcatgtccc tgactcgcat gttttccctg 1980

accaaatccg ccagaaggcg ctcgccgccc agcgatagca gttcttgcaa ggaagcaaag 2040

tttttcaacg gtttgagacc gtccgccgta ggcatgcttt tgagcgtttg accaagcagt 2100

tccaggcggt cccacagctc ggtcacctgc tctacggcat ctcgatccag catatctcct 2160

cgtttcgcgg gttggggcgg ctttcgctgt acggcagtag tcggtgctcg tccagacggg 2220

ccagggtcat gtctttccac gggcgcaggg tcctcgtcag cgtagtctgg gtcacggtga 2280

aggggtgcgc tccgggctgc gcgctggcca gggtgcgctt gaggctggtc ctgctggtgc 2340

tgaagcgctg ccggtcttcg ccctgcgcgt cggccaggta gcatttgacc atggtgtcat 2400

agtccagccc ctccgcggcg tggcccttgg cgcgcagctt gcccttggag gaggcgccgc 2460

acgaggggca gtgcagactt ttgagggcgt agagcttggg cgcgagaaat accgattccg 2520

gggagtaggc atccgcgccg caggccccgc agacggtctc gcattccacg agccaggtga 2580

gctctggccg ttcggggtca aaaaccaggt ttcccccatg ctttttgatg cgtttcttac 2640

ctctggtttc catgagccgg tgtccacgct cggtgacgaa aaggctgtcc gtgtccccgt 2700

atacagactt gagaggcctg tcctcgagcg gtgttccgcg gtcctcctcg tatagaaact 2760

cggaccactc tgagacaaag gctcgcgtcc aggccagcac gaaggaggct aagtgggagg 2820

ggtagcggtc gttgtccact agggggtcca ctcgctccag ggtgtgaaga cacatgtcgc 2880

cctcttcggc atcaaggaag gtgattggtt tgtaggtgta ggccacgtga ccgggtgttc 2940

ctgaaggggg gctataaaag ggggtggggg cgcgttcgtc ctcactctct tccgcatcgc 3000

tgtctgcgag ggccagctgt tggggtgagt actccctctg aaaagcgggc atgacttctg 3060

cgctaagatt gtcagtttcc aaaaacgagg aggatttgat attcacctgg cccgcggtga 3120

tgcctttgag ggtggccgca tccatctggt cagaaaagac aatctttttg ttgtcaagct 3180

tggtggcaaa cgacccgtag agggcgttgg acagcaactt ggcgatggag cgcagggttt 3240

ggtttttgtc gcgatcggcg cgctccttgg ccgcgatgtt tagctgcacg tattcgcgcg 3300

caacgcaccg ccattcggga aagacggtgg tgcgctcgtc gggcaccagg tgcacgcgcc 3360

aaccgcggtt gtgcagggtg acaaggtcaa cgctggtggc tacctctccg cgtaggcgct 3420

cgttggtcca gcagaggcgg ccgcccttgc gcgagcagaa tggcggtagg gggtctagct 3480

gcgtctcgtc cggggggtct gcgtccacgg taaagacccc gggcagcagg cgcgcgtcga 3540

agtagtctat cttgcatcct tgcaagtcta gcgcctgctg ccatgcgcgg gcggcaagcg 3600

cgcgctcgta tgggttgagt gggggacccc atggcatggg gtgggtgagc gcggaggcgt 3660

acatgccgca aatgtcgtaa acgtagaggg gctctctgag tattccaaga tatgtagggt 3720

agcatcttcc accgcggatg ctggcgcgca cgtaatcgta tagttcgtgc gagggagcga 3780

ggaggtcggg accgaggttg ctacgggcgg gctgctctgc tcggaagact atctgcctga 3840

agatggcatg tgagttggat gatatggttg gacgctggaa gacgttgaag ctggcgtctg 3900

tgagacctac cgcgtcacgc acgaaggagg cgtaggagtc gcgcagcttg ttgaccagct 3960

cggcggtgac ctgcacgtct agggcgcagt agtccagggt ttccttgatg atgtcatact 4020

tatcctgtcc cttttttttc cacagctcgc ggttgaggac aaactcttcg cggtctttcc 4080

agtactcttg gatcggaaac ccgtcggcct ccgaacggta agagcctagc atgtagaact 4140

ggttgacggc ctggtaggcg cagcatccct tttctacggg tagcgcgtat gcctgcgcgg 4200

ccttccggag cgaggtgtgg gtgagcgcaa aggtgtccct gaccatgact ttgaggtact 4260

ggtatttgaa gtcagtgtcg tcgcatccgc cctgctccca gagcaaaaag tccgtgcgct 4320

ttttggaacg cggatttggc agggcgaagg tgacatcgtt gaagagtatc tttcccgcgc 4380

gaggcataaa gttgcgtgtg atgcggaagg gtcccggcac ctcggaacgg ttgttaatta 4440

cctgggcggc gagcacgatc tcgtcaaagc cgttgatgtt gtggcccaca atgtaaagtt 4500

ccaagaagcg cgggatgccc ttgatggaag gcaatttttt aagttcctcg taggtgagct 4560

cttcagggga gctgagcccg tgctctgaaa gggcccagtc tgcaagatga gggttggaag 4620

cgacgaatga gctccacagg tcacgggcca ttagcatttg caggtggtcg cgaaaggtcc 4680

taaactggcg acctatggcc attttttctg gggtgatgca gtagaaggta agcgggtctt 4740

gttcccagcg gtcccatcca aggttcgcgg ctaggtctcg cgcggcagtc actagaggct 4800

catctccgcc gaacttcatg accagcatga agggcacgag ctgcttccca aaggccccca 4860

tccaagtata ggtctctaca tcgtaggtga caaagagacg ctcggtgcga ggatgcgagc 4920

cgatcgggaa gaactggatc tcccgccacc aattggagga gtggctattg atgtggtgaa 4980

agtagaagtc cctgcgacgg gccgaacact cgtgctggct tttgtaaaaa cgtgcgcagt 5040

actggcagcg gtgcacgggc tgtacatcct gcacgaggtt gacctgacga ccgcgcacaa 5100

ggaagcagag tgggaatttg agcccctcgc ctggcgggtt tggctggtgg tcttctactt 5160

cggctgcttg tccttgaccg tctggctgct cgaggggagt tacggtggat cggaccacca 5220

cgccgcgcga gcccaaagtc cagatgtccg cgcgcggcgg tcggagcttg atgacaacat 5280

cgcgcagatg ggagctgtcc atggtctgga gctcccgcgg cgtcaggtca ggcgggagct 5340

cctgcaggtt tacctcgcat agacgggtca gggcgcgggc tagatccagg tgatacctaa 5400

tttccagggg ctggttggtg gcggcgtcga tggcttgcaa gaggccgcat ccccgcggcg 5460

cgactacggt accgcgcggc gggcggtggg ccgcgggggt gtccttggat gatgcatcta 5520

aaagcggtga cgcgggcgag cccccggagg tagggggggc tccggacccg ccgggagagg 5580

gggcaggggc acgtcggcgc cgcgcgcggg caggagctgg tgctgcgcgc gtaggttgct 5640

ggcgaacgcg acgacgcggc ggttgatctc ctgaatctgg cgcctctgcg tgaagacgac 5700

gggcccggtg agcttgagcc tgaaagagag ttcgacagaa tcaatttcgg tgtcgttgac 5760

ggcggcctgg cgcaaaatct cctgcacgtc tcctgagttg tcttgatagg cgatctcggc 5820

catgaactgc tcgatctctt cctcctggag atctccgcgt ccggctcgct ccacggtggc 5880

ggcgaggtcg ttggaaatgc gggccatgag ctgcgagaag gcgttgaggc ctccctcgtt 5940

ccagacgcgg ctgtagacca cgcccccttc ggcatcgcgg gcgcgcatga ccacctgcgc 6000

gagattgagc tccacgtgcc gggcgaagac ggcgtagttt cgcaggcgct gaaagaggta 6060

gttgagggtg gtggcggtgt gttctgccac gaagaagtac ataacccagc gtcgcaacgt 6120

ggattcgttg atatccccca aggcctcaag gcgctccatg gcctcgtaga agtccacggc 6180

gaagttgaaa aactgggagt tgcgcgccga cacggttaac tcctcctcca gaagacggat 6240

gagctcggcg acagtgtcgc gcacctcgcg ctcaaaggct acaggggcct cttcttcttc 6300

ttcaatctcc tcttccataa gggcctcccc ttcttcttct tctggcggcg gtgggggagg 6360

ggggacacgg cggcgacgac ggcgcaccgg gaggcggtcg acaaagcgct cgatcatctc 6420

cccgcggcga cggcgcatgg tctcggtgac ggcgcggccg ttctcgcggg ggcgcagttg 6480

gaagacgccg cccgtcatgt cccggttatg ggttggcggg gggctgccat gcggcaggga 6540

tacggcgcta acgatgcatc tcaacaattg ttgtgtaggt actccgccgc cgagggacct 6600

gagcgagtcc gcatcgaccg gatcggaaaa cctctcgaga aaggcgtcta accagtcaca 6660

gtcgcaaggt aggctgagca ccgtggcggg cggcagcggg cggcggtcgg ggttgtttct 6720

ggcggaggtg ctgctgatga tgtaattaaa gtaggcggtc ttgagacggc ggatggtcga 6780

cagaagcacc atgtccttgg gtccggcctg ctgaatgcgc aggcggtcgg ccatgcccca 6840

ggcttcgttt tgacatcggc gcaggtcttt gtagtagtct tgcatgagcc tttctaccgg 6900

cacttcttct tctccttcct cttgtcctgc atctcttgca tctatcgctg cggcggcggc 6960

ggagtttggc cgtaggtggc gccctcttcc tcccatgcgt gtgaccccga agcccctcat 7020

cggctgaagc agggctaggt cggcgacaac gcgctcggct aatatggcct gctgcacctg 7080

cgtgagggta gactggaagt catccatgtc cacaaagcgg tggtatgcgc ccgtgttgat 7140

ggtgtaagtg cagttggcca taacggacca gttaacggtc tggtgacccg gctgcgagag 7200

ctcggtgtac ctgagacgcg agtaagccct cgagtcaaat acgtagtcgt tgcaagtccg 7260

caccaggtac tggtatccca ccaaaaagtg cggcggcggc tggcggtaga ggggccagcg 7320

tagggtggcc ggggctccgg gggcgagatc ttccaacata aggcgatgat atccgtagat 7380

gtacctggac atccaggtga tgccggcggc ggtggtggag gcgcgcggaa agtcgcggac 7440

gcggttccag atgttgcgca gcggcaaaaa gtgctccatg gtcgggacgc tctggccggt 7500

caggcgcgcg caatcgttga cgctctaccg tgcaaaagga gagcctgtaa gcgggcactc 7560

ttccgtggtc tggtggataa attcgcaagg gtatcatggc ggacgaccgg ggttcgagcc 7620

ccgtatccgg ccgtccgccg tgatccatgc ggttaccgcc cgcgtgtcga acccaggtgt 7680

gcgacgtcag acaacggggg agtgctcctt ttggcttcct tccaggcgcg gcggctgctg 7740

cgctagcttt tttggccact ggccgcgcgc agcgtaagcg gttaggctgg aaagcgaaag 7800

cattaagtgg ctcgctccct gtagccggag ggttattttc caagggttga gtcgcgggac 7860

ccccggttcg agtctcggac cggccggact gcggcgaacg ggggtttgcc tccccgtcat 7920

gcaagacccc gcttgcaaat tcctccggaa acagggacga gccccttttt tgcttttccc 7980

agatgcatcc ggtgctgcgg cagatgcgcc cccctcctca gcagcggcaa gagcaagagc 8040

agcggcagac atgcagggca ccctcccctc ctcctaccgc gtcaggaggg gcgacatccg 8100

cggttgacgc ggcagcagat ggtgattacg aacccccgcg gcgccgggcc cggcactacc 8160

tggacttgga ggagggcgag ggcctggcgc ggctaggagc gccctctcct gagcggtacc 8220

caagggtgca gctgaagcgt gatacgcgtg aggcgtacgt gccgcggcag aacctgtttc 8280

gcgaccgcga gggagaggag cccgaggaga tgcgggatcg aaagttccac gcagggcgcg 8340

agctgcggca tggcctgaat cgcgagcggt tgctgcgcga ggaggacttt gagcccgacg 8400

cgcgaaccgg gattagtccc gcgcgcgcac acgtggcggc cgccgacctg gtaaccgcat 8460

acgagcagac ggtgaaccag gagattaact ttcaaaaaag ctttaacaac cacgtgcgta 8520

cgcttgtggc gcgcgaggag gtggctatag gactgatgca tctgtgggac tttgtaagcg 8580

cgctggagca aaacccaaat agcaagccgc tcatggcgca gctgttcctt atagtgcagc 8640

acagcaggga caacgaggca ttcagggatg cgctgctaaa catagtagag cccgagggcc 8700

gctggctgct cgatttgata aacatcctgc agagcatagt ggtgcaggag cgcagcttga 8760

gcctggctga caaggtggcc gccatcaact attccatgct tagcctgggc aagttttacg 8820

cccgcaagat ataccatacc ccttacgttc ccatagacaa ggaggtaaag atcgaggggt 8880

tctacatgcg catggcgctg aaggtgctta ccttgagcga cgacctgggc gtttatcgca 8940

acgagcgcat ccacaaggcc gtgagcgtga gccggcggcg cgagctcagc gaccgcgagc 9000

tgatgcacag cctgcaaagg gccctggctg gcacgggcag cggcgataga gaggccgagt 9060

cctactttga cgcgggcgct gacctgcgct gggccccaag ccgacgcgcc ctggaggcag 9120

ctggggccgg acctgggctg gcggtggcac ccgcgcgcgc tggcaacgtc ggcggcgtgg 9180
aggaatatga cgaggacgat gagtacgagc cagaggacgg cgagtactaa gcggtgatgt 9240
ttctgatcag atgatgcaag acgcaacgga cccggcggtg cgggcggcgc tgcagagcca 9300
gccgtccggc cttaactcca cggacgactg gcgccaggtc atggaccgca tcatgtcgct 9360
gactgcgcgc aatcctgacg cgttccggca gcagccgcag gccaaccggc tctccgcaat 9420
tctggaagcg gtggtcccgg cgcgcgcaaa ccccacgcac gagaaggtgc tggcgatcgt 9480
aaacgcgctg gccgaaaaca gggccatccg gcccgacgag gccggcctgg tctacgacgc 9540
gctgcttcag cgcgtggctc gttacaacag cggcaacgtg cagaccaacc tggaccggct 9600
ggtgggggat gtgcgcgagg ccgtggcgca gcgtgagcgc gcgcagcagc agggcaacct 9660
gggctccatg gttgcactaa acgccttcct gagtacacag cccgccaacg tgccgcgggg 9720
acaggaggac tacaccaact ttgtgagcgc actgcggcta atggtgactg agacaccgca 9780
aagtgaggtg taccagtctg ggccagacta ttttttccag accagtagac aaggcctgca 9840
gaccgtaaac ctgagccagg ctttcaaaaa cttgcagggg ctgtgggggg tgcgggctcc 9900
cacaggcgac cgcgcgaccg tgtctagctt gctgacgccc aactcgcgcc tgttgctgct 9960
gctaatagcg cccttcacgg acagtggcag cgtgtcccgg gacacatacc taggtcactt 10020
gctgacactg taccgcgagg ccataggtca ggcgcatgtg gacgagcata ctttccagga 10080
gattacaagt gtcagccgcg cgctggggca ggaggacacg ggcagcctgg aggcaaccct 10140
aaactacctg ctgaccaacc ggcggcagaa gatcccctcg ttgcacagtt taaacagcga 10200
ggaggagcgc attttgcgct acgtgcagca gagcgtgagc cttaacctga tgcgcgacgg 10260
ggtaacgccc agcgtggcgc tggacatgac cgcgcgcaac atggaaccgg gcatgtatgc 10320
ctcaaaccgg ccgtttatca accgcctaat ggactacttg catcgcgcgg ccgccgtgaa 10380
ccccgagtat ttcaccaatg ccatcttgaa cccgcactgg ctaccgcccc ctggtttcta 10440
caccggggga ttcgaggtgc ccgagggtaa cgatggattc ctctgggacg acatagacga 10500
cagcgtgttt tccccgcaac cgcagaccct gctagagttg caacagcgcg agcaggcaga 10560
ggcggcgctg cgaaaggaaa gcttccgcag gccaagcagc ttgtccgatc taggcgctgc 10620
ggccccgcgg tcagatgcta gtagcccatt tccaagcttg atagggtctc ttaccagcac 10680
tcgcaccacc cgcccgcgcc tgctgggcga ggaggagtac ctaaacaact cgctgctgca 10740
gccgcagcgc gaaaaaaacc tgcctccggc atttcccaac aacgggatag agagcctagt 10800
ggacaagatg agtagatgga agacgtacgc gcaggagcac agggacgtgc caggcccgcg 10860
cccgcccacc cgtcgtcaaa ggcacgaccg tcagcggggt ctggtgtggg aggacgatga 10920
ctcggcagac gacagcagcg tcctggattt gggagggagt ggcaacccgt ttgcgcacct 10980

tcgccccagg ctggggagaa tgttttaaaa aaaaaaaagc atgatgcaaa ataaaaaact 11040

caccaaggcc atggcaccga gcgttggttt tcttgtattc cccttagtat gcggcgcgcg 11100

gcgatgtatg aggaaggtcc tcctccctcc tacgagagtg tggtgagcgc ggcgccagtg 11160

gcggcggcgc tgggttctcc cttcgatgct cccctggacc cgccgtttgt gcctccgcgg 11220

tacctgcggc ctaccggggg gagaaacagc atccgttact ctgagttggc acccctattc 11280

gacaccaccc gtgtgtacct ggtggacaac aagtcaacgg atgtggcatc cctgaactac 11340

cagaacgacc acagcaactt tctgaccacg gtcattcaaa acaatgacta cagcccgggg 11400

gaggcaagca cacagaccat caatcttgac gaccggtcgc actggggcgg cgacctgaaa 11460

accatcctgc ataccaacat gccaaatgtg aacgagttca tgtttaccaa taagtttaag 11520

gcgcgggtga tggtgtcgcg cttgcctact aaggacaatc aggtggagct gaaatacgag 11580

tgggtggagt tcacgctgcc cgagggcaac tactccgaga ccatgaccat agaccttatg 11640

aacaacgcga tcgtggagca ctacttgaaa gtgggcagac agaacggggt tctggaaagc 11700

gacatcgggg taaagtttga cacccgcaac ttcagactgg ggtttgaccc cgtcactggt 11760

cttgtcatgc ctggggtata tacaaacgaa gccttccatc cagacatcat tttgctgcca 11820

ggatgcgggg tggacttcac ccacagccgc ctgagcaact tgttgggcat ccgcaagcgg 11880

caacccttcc aggagggctt taggatcacc tacgatgatc tggagggtgg taacattccc 11940

gcactgttgg atgtggacgc ctaccaggcg agcttgaaag atgacaccga acagggcggg 12000

ggtggcgcag gcggcagcaa cagcagtggc agcggcgcgg aagagaactc caacgcggca 12060

gccgcggcaa tgcagccggt ggaggacatg aacgatcatg ccattcgcgg cgacaccttt 12120

gccacacggg ctgaggagaa gcgcgctgag gccgaagcag cggccgaagc tgccgccccc 12180

gctgcgcaac ccgaggtcga gaagcctcag aagaaaccgg tgatcaaacc cctgacagag 12240

gacagcaaga aacgcagtta caacctaata agcaatgaca gcaccttcac ccagtaccgc 12300

agctggtacc ttgcatacaa ctacggcgac cctcagaccg gaatccgctc atggaccctg 12360

ctttgcactc ctgacgtaac ctgcggctcg gagcaggtct actggtcgtt gccagacatg 12420

atgcaagacc ccgtgacctt ccgctccacg cgccagatca gcaactttcc ggtggtgggc 12480

gccgagctgt tgcccgtgca ctccaagagc ttctacaacg accaggccgt ctactcccaa 12540

ctcatccgcc agtttacctc tctgacccac gtgttcaatc gctttcccga gaaccagatt 12600

ttggcgcgcc cgccagcccc caccatcacc accgtcagtg aaaacgttcc tgctctcaca 12660

gatcacggga cgctaccgct gcgcaacagc atcggaggag tccagcgagt gaccattact 12720

gacgccagac gccgcacctg cccctacgtt tacaaggccc tgggcatagt ctcgccgcgc 12780

gtcctatcga gccgcacttt ttgagcaagc atgtccatcc ttatatcgcc cagcaataac 12840

acaggctggg gcctgcgctt cccaagcaag atgtttggcg gggccaagaa gcgctccgac 12900

caacacccag tgcgcgtgcg cgggcactac cgcgcgccct ggggcgcgca caaacgcggc 12960
cgcactgggc gcaccaccgt cgatgacgcc atcgacgcgg tggtggagga ggcgcgcaac 13020
tacacgccca cgccgccacc agtgtccaca gtggacgcgg ccattcagac cgtggtgcgc 13080
ggagcccggc gctatgctaa aatgaagaga cggcggaggc gcgtagcacg tcgccaccgc 13140
cgccgacccg gcactgccgc ccaacgcgcg gcggcggccc tgcttaaccg cgcacgtcgc 13200
accggccgac gggcggccat gcgggccgct cgaaggctgg ccgcgggtat tgtcactgtg 13260
ccccccaggt ccaggcgacg agcggccgcc gcagcagccg cggccattag tgctatgact 13320
cagggtcgca ggggcaacgt gtattgggtg cgcgactcgg ttagcggcct gcgcgtgccc 13380
gtgcgcaccc gccccccgcg caactagatt gcaagaaaaa actacttaga ctcgtactgt 13440
tgtatgtatc cagcggcggc ggcgcgcaac gaagctatgt ccaagcgcaa aatcaaagaa 13500
gagatgctcc aggtcatcgc gccggagatc tatggccccc cgaagaagga agagcaggat 13560
tacaagcccc gaaagctaaa gcgggtcaaa aagaaaaaga aagatgatga tgatgaactt 13620
gacgacgagg tggaactgct gcacgctacc gcgcccaggc gacgggtaca gtggaaaggt 13680
cgacgcgtaa aacgtgtttt gcgacccggc accaccgtag tctttacgcc cggtgagcgc 13740
tccacccgca cctacaagcg cgtgtatgat gaggtgtacg gcgacgagga cctgcttgag 13800
caggccaacg agcgcctcgg ggagtttgcc tacggaaagc ggcataagga catgctggcg 13860
ttgccgctgg acgagggcaa cccaacacct agcctaaagc ccgtaacact gcagcaggtg 13920
ctgcccgcgc ttgcaccgtc cgaagaaaag cgcggcctaa agcgcgagtc tggtgacttg 13980
gcacccaccg tgcagctgat ggtacccaag cgccagcgac tggaagatgt cttggaaaaa 14040
atgaccgtgg aacctgggct ggagcccgag gtccgcgtgc ggccaatcaa gcaggtggcg 14100
ccgggactgg gcgtgcagac cgtggacgtt cagataccca ctaccagtag caccagtatt 14160
gccaccgcca cagagggcat ggagacacaa acgtccccgg ttgcctcagc ggtggcggat 14220
gccgcggtgc aggcggtcgc tgcggccgcg tccaagacct ctacggaggt gcaaacggac 14280
ccgtggatgt ttcgcgtttc agccccccgg cgcccgcgcg gttcgaggaa gtacggcgcc 14340
gccagcgcgc tactgcccga atatgcccta catccttcca ttgcgcctac ccccggctat 14400
cgtggctaca cctaccgccc cagaagacga gcaactaccc gacgccgaac caccactgga 14460
acccgccgcc gccgtcgccg tcgccagccc gtgctggccc cgatttccgt gcgcagggtg 14520
gctcgcgaag gaggcaggac cctggtgctg ccaacagcgc gctaccaccc cagcatcgtt 14580
taaaagccgg tctttgtggt tcttgcagat atggccctca cctgccgcct ccgtttcccg 14640
gtgccgggat tccgaggaag aatgcaccgt aggaggggca tggccggcca cggcctgacg 14700
ggcggcatgc gtcgtgcgca ccaccggcgg cggcgcgcgt cgcaccgtcg catgcgcggc 14760

ggtatcctgc ccctccttat tccactgatc gccgcggcga ttggcgccgt gcccggaatt 14820

gcatccgtgg ccttgcaggc gcagagacac tgattaaaaa caagttgcat gtggaaaaat 14880

caaaataaaa agtctggact ctcacgctcg cttggtcctg taactatttt gtagaatgga 14940

agacatcaac tttgcgtctc tggccccgcg acacggctcg cgcccgttca tgggaaactg 15000

gcaagatatc ggcaccagca atatgagcgg tggcgccttc agctggggct cgctgtggag 15060

cggcattaaa aatttcggtt ccaccgttaa gaactatggc agcaaggcct ggaacagcag 15120

cacaggccag atgctgaggg ataagttgaa agagcaaaat ttccaacaaa aggtggtaga 15180

tggcctggcc tctggcatta gcggggtggt ggacctggcc aaccaggcag tgcaaaataa 15240

gattaacagt aagcttgatc cccgccctcc cgtagaggag cctccaccgg ccgtggagac 15300

agtgtctcca gaggggcgtg gcgaaaagcg tccgcgcccc gacagggaag aaactctggt 15360

gacgcaaata gacgagcctc cctcgtacga ggaggcacta aagcaaggcc tgcccaccac 15420

ccgtcccatc gcgcccatgg ctaccggagt gctgggccag cacacacccg taacgctgga 15480

cctgcctccc cccgccgaca cccagcagaa acctgtgctg ccaggcccga ccgccgttgt 15540

tgtaacccgt cctagccgcg cgtccctgcg ccgcgccgcc agcggtccgc gatcgttgcg 15600

gcccgtagcc agtggcaact ggcaaagcac actgaacagc atcgtgggtc tgggggtgca 15660

atccctgaag cgccgacgat gcttctgaat agctaacgtg tcgtatgtgt gtcatgtatg 15720

cgtccatgtc gccgccagag gagctgctga gccgccgcgc gcccgctttc caagatggct 15780

accccttcga tgatgccgca gtggtcttac atgcacatct cgggccagga cgcctcggag 15840

tacctgagcc ccgggctggt gcagtttgcc cgcgccaccg agacgtactt cagcctgaat 15900

aacaagttta gaaccccacg gtggcgccta cgcacgacgt gaccacagac cggtcccagc 15960

gtttgacgct gcggttcatc cctgtggacc gtgaggatac tgcgtactcg tacaaggcgc 16020

ggttcaccct agctgtgggt gataaccgtg tgctggacat ggcttccacg tactttgaca 16080

tccgcggcgt gctggacagg ggccctactt ttaagcccta ctctggcact gcctacaacg 16140

ccctggctcc caagggtgcc ccaaatcctt gcgaatggga tgaagctgct actgctcttg 16200

aaataaacct agaagaagag gacgatgaca acgaagacga agtagacgag caagctgagc 16260

agcaaaaaac tcacgtattt gggcaggcgc cttattctgg tataaatatt acaaaggagg 16320

gtattcaaat aggtgtcgaa ggtcaaacac ctaaatatgc cgataaaaca tttcaacctg 16380

aacctcaaat aggagaatct cagtggtacg aaactgaaat taatcatgca gctgggagag 16440

tccttaaaaa gactacccca atgaaaccat gttacggttc atatgcaaaa cccacaaatg 16500

aaaatggagg gcaaggcatt cttgtaaagc aacaaaatgg aaagctagaa agtcaagtgg 16560

aaatgcaatt tttctcaact actgaggcga ccgcaggcaa tggtgataac ttgactccta 16620

aagtggtatt gtacagtgaa gatgtagata tagaaacccc agacactcat atttcttaca 16680

atgcccacta ttaaggaagg taactcacga gaactaatgg gccaacaatc tatgcccaac 16740
aggcctaatt acattgcttt tagggacaat tttattggtc taatgtatta caacagcacg 16800
ggtaatatgg gtgttctggc gggccaagca tcgcagttga atgctgttgt agatttgcaa 16860
gacagaaaca cagagctttc ataccagctt ttgcttgatt ccattggtga tagaaccagg 16920
tacttttcta tgtggaatca ggctgttgac agctatgatc cagatgttag aattattgaa 16980
aatcatggaa ctgaagatga acttccaaat tactgctttc cactgggagg tgtgattaat 17040
acagagactc ttaccaaggt aaaacctaaa acaggtcagg aaaatggatg ggaaaaagat 17100
gctacagaat tttcagataa aaatgaaata agagttggaa ataattttgc catggaaatc 17160
aatctaaatg ccaacctgtg gagaaatttc ctgtactcca acatagcgct gtatttgccc 17220
gacaagctaa agtacagtcc ttccaacgta aaaatttctg ataacccaaa cacctacgac 17280
tacatgaaca agcgagtggt ggctcccggg ttagtggact gctacattaa ccttggagca 17340
cgctggtccc ttgactatat ggacaacgtc aacccattta accaccaccg caatgctggc 17400
ctgcgctacc gctcaatgtt gctgggcaat ggtcgctatg tgcccttcca catccaggtg 17460
cctcagaagt tctttgccat taaaaacctc cttctcctgc cgggctcata cacctacgag 17520
tggaacttca ggaaggatgt taacatggtt ctgcagagct ccctaggaaa tgacctaagg 17580
gttgacggag ccagcattaa gtttgatagc atttgccttt acgccacctt cttccccatg 17640
gcccacaaca ccgcctccac gcttgaggcc atgcttagaa acgacaccaa cgaccagtcc 17700
tttaacgact atctctccgc cgccaacatg ctctacccta tacccgccaa cgctaccaac 17760
gtgcccatat ccatcccctc ccgcaactgg gcggctttcc gcggctgggc cttcacgcgc 17820
cttaagacta aggaaacccc atcactgggc tcgggctacg acccttatta cacctactct 17880
ggctctatac cctacctaga tggaaccttt tacctcaacc acacctttaa gaaggtggcc 17940
attacctttg actcttctgt cagctggcct ggcaatgacc gcctgcttac ccccaacgag 18000
tttgaaatta agcgctcagt tgacggggag ggttacaacg ttgcccagtg taacatgacc 18060
aaagactggt tcctggtaca aatgctagct aactacaaca ttggctacca gggcttctat 18120
atcccagaga gctacaagga ccgcatgtac tccttcttta gaaacttcca gcccatgagc 18180
cgtcaggtgg tggatgatac taaatacaag gactaccaac aggtgggcat cctacaccaa 18240
cacaacaact ctggatttgt tggctacctt gcccccacca tgcgcgaagg acaggcctac 18300
cctgctaact tcccctatcc gcttataggc aagaccgcag ttgacagcat tacccagaaa 18360
aagtttcttt gcgatcgcac cctttggcgc atcccattct ccagtaactt tatgtccatg 18420
ggcgcactca cagacctggg ccaaaacctt ctctacgcca actccgccca cgcgctagac 18480
atgacttttg aggtggatcc catggacgag cccacccttc tttatgtttt gtttgaagtc 18540

tttgacgtgg tccgtgtgca ccggccgcac cgcggcgtca tcgaaaccgt gtacctgcgc 18600

acgcccttct cggccggcaa cgccacaaca taaagaagca agcaacatca acaacagctg 18660

ccgccatggg ctccagtgag caggaactga aagccattgt caaagatctt ggttgtgggc 18720

catatttttt gggcacctat gacaagcgct ttccaggctt tgtttctcca cacaagctcg 18780

cctgcgccat agtcaatacg gccggtcgcg agactggggg cgtacactgg atggcctttg 18840

cctggaaccc gcactcaaaa acatgctacc tctttgagcc ctttggcttt tctgaccagc 18900

gactcaagca ggtttaccag tttgagtacg agtcactcct gcgccgtagc gccattgctt 18960

cttcccccga ccgctgtata acgctggaaa agtccaccca aagcgtacag gggcccaact 19020

cggccgcctg tggactattc tgctgcatgt ttctccacgc ctttgccaac tggccccaaa 19080

ctcccatgga tcacaacccc accatgaacc ttattaccgg ggtacccaac tccatgctca 19140

acagtcccca ggtacagccc accctgcgtc gcaaccagga acagctctac agcttcctgg 19200

agcgccactc gccctacttc cgcagccaca gtgcgcagat taggagcgcc acttcttttt 19260

gtcacttgaa aaacatgtaa aaataatgta ctagagacac tttcaataaa ggcaaatgct 19320

tttatttgta cactctcggg tgattattta cccccaccct tgccgtctgc gccgtttaaa 19380

aatcaaaggg gttctgccgc gcatcgctat gcgccactgg cagggacacg ttgcgatact 19440

ggtgtttagt gctccactta aactcaggca caaccatccg cggcagctcg gtgaagtttt 19500

cactccacag gctgcgcacc atcaccaacg cgtttagcag gtcgggcgcc gatatcttga 19560

agtcgcagtt ggggcctccg ccctgcgcgc gcgagttgcg atacacaggg ttgcagcact 19620

ggaacactat cagcgccggg tggtgcacgc tggccagcac gctcttgtcg gagatcagat 19680

ccgcgtccag gtcctccgcg ttgctcaggg cgaacggagt caactttggt agctgccttc 19740

ccaaaaaggg cgcgtgccca ggctttgagt tgcactcgca ccgtagtggc atcaaaaggt 19800

gaccgtgccc ggtctgggcg ttaggataca gcgcctgcat aaaagccttg atctgcttaa 19860

aagccacctg agcctttgcg ccttcagaga agaacatgcc gcaagacttg ccggaaaact 19920

gattggccgg acaggccgcg tcgtgcacgc agcaccttgc gtcggtgttg gagatctgca 19980

ccacatttcg gccccaccgg ttcttcacga tcttggcctt gctagactgc tccttcagcg 20040

cgcgctgccc gttttcgctc gtcacatcca tttcaatcac gtgctcctta tttatcataa 20100

tgcttccgtg tagacactta agctcgcctt cgatctcagc gcagcggtgc agccacaacg 20160

cgcagcccgt gggctcgtga tgcttgtagg tcacctctgc aaacgactgc aggtacgcct 20220

gcaggaatcg ccccatcatc gtcacaaagg tcttgttgct ggtgaaggtc agctgcaacc 20280

cgcggtgctc ctcgttcagc caggtcttgc atacggccgc cagagcttcc acttggtcag 20340

gcagtagttt gaagttcgcc tttagatcgt tatccacgtg gtacttgtcc atcagcgcgc 20400

gcgcagcctc catgcccttc tcccacgcag acacgatcgg cacactcagc gggttcatca 20460

ccgtaatttc actttccgct tcgctgggct cttcctcttc ctcttgcgtc cgcataccac 20520

gcgccactgg gtcgtcttca ttcagccgcc gcactgtgcg cttacctcct ttgccatgct 20580

tgattagcac cggtgggttg ctgaaaccca ccatttgtag cgccacatct tctctttctt 20640

cctcgctgtc cacgattacc tctggtgatg gcgggcgctc gggcttggga gaagggcgct 20700

tctttttctt cttgggcgca atggccaaat ccgccgccga ggtcgatggc cgcgggctgg 20760

gtgtgcgcgg caccagcgcg tcttgtgatg agtcttcctc gtcctcggac tcgatacgcc 20820

gcctcatccg ctttttgggg ggcgcccggg gaggcggcgg cgacggggac ggggacgaca 20880

cgtcctccat ggttggggga cgtcgcgccg caccgcgtcc gcgctcgggg gtggtttcgc 20940

gctgctcctc ttcccgactg gccatttcct tctcctatag gcagaaaaag atcatggagt 21000

cagtcgagaa gaaggacagc ctaaccgccc cctctgagtt cgccaccacc gcctccaccg 21060

atgccgccaa cgcgcctacc accttccccg tcgaggcacc cccgcttgag gaggaggaag 21120

tgattatcga gcaggaccca ggttttgtaa gcgaagacga cgaggaccgc tcagtaccaa 21180

cagaggataa aaagcaagac caggacaacg cagaggcaaa cgaggaacaa gtcgggcggg 21240

gggacgaaag gcatggcgac tacctagatg tgggagacga cgtgctgttg aagcatctgc 21300

agcgccagtg cgccattatc tgcgacgcgt tgcaagagcg cagcgatgtg cccctcgcca 21360

tagcggatgt cagccttgcc tacgaacgcc acctattctc accgcgcgta ccccccaaac 21420

gccaagaaaa cggcacatgc gagcccaacc cgcgcctcaa cttctacccc gtatttgccg 21480

tgccagaggt gcttgccacc tatcacatct ttttccaaaa ctgcaagata cccctatcct 21540

gccgtgccaa ccgcagccga gcggacaagc agctggcctt gcggcagggc gctgtcatac 21600

ctgatatcgc ctcgctcaac gaagtgccaa aaatctttga gggtcttgga cgcgacgaga 21660

agcgcgcggc aaacgctctg caacaggaaa acagcgaaaa tgaaagtcac tctggagtgt 21720

tggtggaact cgagggtgac aacgcgcgcc tagccgtact aaaacgcagc atcgaggtca 21780

cccactttgc ctacccggca cttaacctac cccccaaggt catgagcaca gtcatgagtg 21840

agctgatcgt gcgccgtgcg cagcccctgg agagggatgc aaatttgcaa gaacaaacag 21900

aggagggcct acccgcagtt ggcgacgagc agctagcgcg ctggcttcaa acgcgcgagc 21960

ctgccgactt ggaggagcga cgcaaactaa tgatggccgc agtgctcgtt accgtggagc 22020

ttgagtgcat gcagcggttc tttgctgacc cggagatgca gcgcaagcta gaggaaacat 22080

tgcactacac ctttcgacag ggctacgtac gccaggcctg caagatctcc aacgtggagc 22140

tctgcaacct ggtctcctac cttggaattt tgcacgaaaa ccgccttggg caaaacgtgc 22200

ttcattccac gctcaagggc gaggcgcgcc gcgactacgt ccgcgactgc gtttacttat 22260

ttctatgcta cacctggcag acggccatgg gcgtttggca gcagtgcttg gaggagtgca 22320

acctcaagga gctgcagaaa ctgctaaagc aaaacttgaa ggacctatgg acggccttca 22380
acgagcgctc cgtggccgcg cacctggcgg acatcatttt ccccgaacgc ctgcttaaaa 22440
ccctgcaaca gggtctgcca gacttcacca gtcaaagcat gttgcagaac tttaggaact 22500
ttatcctaga gcgctcagga atcttgcccg ccacctgctg tgcacttcct agcgactttg 22560
tgcccattaa gtaccgcgaa tgccctccgc cgctttgggg ccactgctac cttctgcagc 22620
tagccaacta ccttgcctac cactctgaca taatggaaga cgtgagcggt gacggtctac 22680
tggagtgtca ctgtcgctgc aacctatgca ccccgcaccg ctccctggtt tgcaattcgc 22740
agctgcttaa cgaaagtcaa attatcggta cctttgagct gcagggtccc tcgcctgacg 22800
aaaagtccgc ggctccgggg ttgaaactca ctccggggct gtggacgtcg gcttaccttc 22860
gcaaatttgt acctgaggac taccacgccc acgagattag gttctacgaa gaccaatccc 22920
gcccgccaaa tgcggagctt accgcctgcg tcattaccca gggccacatt cttggccaat 22980
tgcaagccat caacaaagcc cgccaagagt ttctgctacg aaagggacgg ggggtttact 23040
tggaccccca gtccggcgag gagctcaacc caatcccccc gccgccgcag ccctatcagc 23100
agcagccgcg ggcccttgct tcccaggatg gcacccaaaa agaagctgca gctgccgccg 23160
ccacccacgg acgaggagga atactgggac agtcaggcag aggaggtttt ggacgaggag 23220
gaggaggaca tgatggaaga ctgggagagc ctagacgagg aagcttccga ggtcgaagag 23280
gtgtcagacg aaacaccgtc accctcggtc gcattcccct cgccggcgcc ccagaaatcg 23340
gcaaccggtt ccagcatggc tacaacctcc gctcctcagg cgccgccggc actgcccgtt 23400
cgccgaccca accgtagatg ggacaccact ggaaccaggg ccggtaagtc caagcagccg 23460
ccgccgttag cccaagagca acaacagcgc caaggctacc gctcatggcg cgggcacaag 23520
aacgccatag ttgcttgctt gcaagactgt gggggcaaca tctccttcgc ccgccgcttt 23580
cttctctacc atcacggcgt ggccttcccc cgtaacatcc tgcattacta ccgtcatctc 23640
tacagcccat actgcaccgg cggcagcggc agcggcagca acagcagcgg ccacacagaa 23700
gcaaaggcga ccggatagca agactctgac aaagcccaag aaatccacag cggcggcagc 23760
agcaggagga ggagcgctgc gtctggcgcc caacgaaccc gtatcgaccc gcgagcttag 23820
aaacaggatt tttcccactc tgtatgctat atttcaacag agcaggggcc aagaacaaga 23880
gctgaaaata aaaaacaggt ctctgcgatc cctcacccgc agctgcctgt atcacaaaag 23940
cgaagatcag cttcggcgca cgctggaaga cgcggaggct ctcttcagta aatactgcgc 24000
gctgactctt aaggactagt ttcgcgccct ttctcaaatt taagcgcgaa aactacgtca 24060
tctccagcgg ccacacccgg cgccagcacc tgtcgtcagc gccattatga gcaaggaaat 24120
tcccacgccc tacatgtgga gttaccagcc acaaatggga cttgcggctg gagctgccca 24180
agactactca acccgaataa actacatgag cgcgggaccc cacatgatat cccgggtcaa 24240

cggaatccgc gcccaccgaa accgaattct cttggaacag gcggctatta ccaccacacc 24300
tcgtaataac cttaatcccc gtagttggcc cgctgccctg gtgtaccagg aaagtcccgc 24360
tcccaccact gtggtacttc ccagagacgc ccaggccgaa gttcagatga ctaactcagg 24420
ggcgcagctt gcgggcggct ttcgtcacag ggtgcggtcg cccgggcagg gtataactca 24480
cctgacaatc agagggcgag gtattcagct caacgacgag tcggtgagct cctcgcttgg 24540
tctccgtccg gacgggacat ttcagatcgg cggcgccggc cgtccttcat tcacgcctcg 24600
tcaggcaatc ctaactctgc agacctcgtc ctctgagccg cgctctggag gcattggaac 24660
tctgcaattt attgaggagt ttgtgccatc ggtctacttt aaccccttct cgggacctcc 24720
cggccactat ccggatcaat ttattcctaa ctttgacgcg gtaaaggact cggcggacgg 24780
ctacgactga atgttaagtg gagaggcaga gcaactgcgc ctgaaacacc tggtccactg 24840
tcgccgccac aagtgctttg cccgcgactc cggtgagttt tgctactttg aattgcccga 24900
ggatcatatc gagggcccgg cgcacggcgt ccggcttacc gcccagggag agcttgcccg 24960
tagcctgatt cgggagttta cccagcgccc cctgctagtt gagcggacag gggaccctg 25020
tgttctcact gtgatttgca actgtcctaa ccttggatta catcaagatc ctctagttat 25080
aactagagta cccggggatc ttattccctt taactaataa aaaaaaataa taaagcatca 25140
cttacttaaa atcagttagc aaatttctgt ccagtttatt cagcagcacc tccttgccct 25200
cctcccagct ctggtattgc agcttcctcc tggctgcaaa ctttctccac aatctaaatg 25260
gaatgtcagt ttcctcctgt tcctgtccat ccgcacccac tatcttcatg ttgttgcaga 25320
tgaagcgcgc aagaccgtct gaagatacct tcaaccccgt gtatccatat gacacggaaa 25380
ccggtcctcc aactgtgcct tttcttactc ctccctttgt atcccccaat gggtttcaag 25440
agagtccccc tggggtactc tctttgcgcc tatccgaacc tctagttacc tccaatggca 25500
tgcttgcgct caaaatgggc aacggcctct ctctggacga ggccggcaac cttacctccc 25560
aaaatgtaac cactgtgagc ccacctctca aaaaaaccaa gtcaaacata aacctggaaa 25620
tatctgcacc cctcacagtt acctcagaag ccctaactgt ggctgccgcc gcacctctaa 25680
tggtcgcggg caacacactc accatgcaat cacaggcccc gctaaccgtg cacgactcca 25740
aacttagcat tgccacccaa ggacccctca cagtgtcaga aggaaagcta gccctgcaaa 25800
catcaggccc cctcaccacc accgatagca gtacccttac tatcactgcc tcaccccctc 25860
taactactgc cactggtagc ttgggcattg acttgaaaga gcccatttat acacaaaatg 25920
gaaaactagg actaaagtac ggggctcctt tgcatgtaac agacgaccta aacactttga 25980
ccgtagcaac tggtccaggt gtgactatta ataatacttc cttgcaaact aaagttactg 26040
gagccttggg ttttgattca caaggcaata tgcaacttaa tgtagcagga ggactaagga 26100

ttgattctca aaacagacgc cttatacttg atgttagtta tccgtttgat gctcaaaacc 26160
aactaaatct aagactagga cagggccctc tttttataaa ctcagcccac aacttggata 26220
ttaactacaa caaaggcctt tacttgttta cagcttcaaa caattccaaa aagcttgagg 26280
ttaacctaag cactgccaag gggttgatgt ttgacgctac agccatagcc attaatgcag 26340
gagatgggct tgaatttggt tcacctaatg caccaaacac aaatcccctc aaaacaaaaa 26400
ttggccatgg cctagaattt gattcaaaca aggctatggt tcctaaacta ggaactggcc 26460
ttagttttga cagcacaggt gccattacag taggaaacaa aaataatgat aagctaactt 26520
tgtggaccac accagctcca tctcctaact gtagactaaa tgcagagaaa gatgctaaac 26580
tcactttggt cttaacaaaa tgtggcagtc aaatacttgc tacagtttca gttttggctg 26640
ttaaaggcag tttggctcca atatctggaa cagttcaaag tgctcatctt attataagat 26700
ttgacgaaaa tggagtgcta ctaaacaatt ccttcctgga cccagaatat tggaacttta 26760
gaaatggaga tcttactgaa ggcacagcct atacaaacgc tgttggattt atgcctaacc 26820
tatcagctta tccaaaatct cacggtaaaa ctgccaaaag taacattgtc agtcaagttt 26880
acttaaacgg agacaaaact aaacctgtaa cactaaccat tacactaaac ggtacacagg 26940
aaacaggaga cacaactcca agtgcatact ctatgtcatt ttcatgggac tggtctggcc 27000
acaactacat taatgaaata tttgccacat cctcttacac tttttcatac attgcccaag 27060
aataaagaat cgtttgtgtt atgtttcaac gtgtttattt ttcaattgca gaaaatttca 27120
agtcattttt cattcagtag tatagcccca ccaccacata gcttatacag atcaccgtac 27180
cttaatcaaa ctcacagaac cctagtattc aacctgccac ctccctccca acacacagag 27240
tacacagtcc tttctccccg gctggcctta aaaagcatca tatcatgggt aacagacata 27300
ttcttaggtg ttatattcca cacggtttcc tgtcgagcca aacgctcatc aagtgatatt 27360
aataaactcc ccgggcagct cacttaagtt catgtcgctg tccagctgct gagccacagg 27420
ctgctgtcca acttgcggtt gcttaacggg cggcgaagga gaagtccacg cctacatggg 27480
gggagagtca taatcgtgca tcaggatagg gcggtggtgc tgcagcagcg cgcgaataaa 27540
ctgctgccgc cgccgctccg tcctgcagga atacaacatg gcagtggtct cctcagcgat 27600
gattcgcacc gcccgcagca taaggcgctt gtcctccggg cacagcagcg caccctgatc 27660
tcacttaaat cagcacagta actgcagcac agcaccacaa tattgttcaa aatcccacag 27720
tgcaaggcgc tgtatccaaa gctcatggcg gggaccacag aacccacgtg gccatcatac 27780
cacaagcgca ggtagattaa gtggcgaccc ctcataaaca cgctggacat aaacattacc 27840
tcttttggca tgttgtaatt caccacctcc cggtaccata taaacctctg attaaacatg 27900
gcgccatcca ccaccatcct aaaccagctg gccaaaacct gccccgccgg gntatacact 27960
gcagggaacc gggacttgga caatgacaag tgggagagcc caggactcgt aaccatggat 28020

catcatgctc gtcatgatat caatgttggc acaacacagg cacacgtgca tacacttcct 28080

caggattaca agctcctccc gcgttagaac catatcccag ggaacaaccc attcctgaat 28140

cagcgtaaat cccacactgc agggaagacc tcgcacgtaa ctcacgttgt gcattgtcaa 28200

agtgttacat tcgggcagca gcggatgatc ctccagtatg gtagcgcggg tttctgtctc 28260

aaaaggaggt agacgatccc tactgtacgg agtgcgccga gacaaccgag atcgtgttgg 28320

tcgtagtgtc atgccaaatg gaacgccgga cgtagtcata tttcctgaag caaaaccagg 28380

tgcgggcgtg acaaacagat ctgcgtctcc ggtctcgccg cttagatcgc tctgtgtagt 28440

agttgtagta tatccactct ctcaaagcat ccaggcgccc cctggcttcg ggttctatgt 28500

aaactccttc atgcgccgct gccctgataa catccaccac cgcagaataa gccacaccca 28560

gccaacctac acattcgttc tgcgagtcac acacgggagg agcgggaaga gctggaagaa 28620

ccatgttttt ttttttattc caaaagatta tccaaaacct caaaatgaag atctattaag 28680

tgaacgcgct cccctccggt ggcgtggtca aactctacag ccaaagaaca gataatggca 28740

tttgtaagat gttgcacaat ggcttccaaa aggcaaacgg ccctcacgtc caagtggacg 28800

taaaggctaa acccttcagg gtgaatctcc tctataaaca ttccagcacc ttcaaccatg 28860

cccaaataat tctcatctcg ccaccttctc aatatatctc taagcaaatc ccgaatattt 28920

aagtccgggc cattgtaaaa aatttggctc cagagcgccc tccaccttca gcctcaagca 28980

gcgaatcatg attgcaaaaa ttcaggttcc tcacagacct gtataagatt caaaagcgga 29040

acattaacaa aaataccgcg atcccgtagg tcccttcgca gggccagctg aacataatcg 29100

tgcaggtctg cacggaccag cgcggccact tccccgccag gaaccatgac aaaagaaccc 29160

acactgatta tgacacgcat actcggagct atgctaacca gcgtagcccc gatgtaagct 29220

tgttgcatgg gcggcgatat aaaatgcaag gtgctgctca aaaaatcagg caaagcctcg 29280

cgcaaaaaag aaagcacatc gtagtcatgc tcatgcagat aaaggcaggt aagctccgga 29340

accaccacag aaaaagacac catttttctc tcaaacatgt ctgcgggttt ctgcataaac 29400

acaaaataaa ataacaaaaa aacatttaaa cattagaagc ctgtcttaca acaggaaaaa 29460

caacccttat aagcataaga cggactacgg ccatgccggc gtgaccgtaa aaaaactggt 29520

caccgtgatt aaaaagcacc accgacagct cctcggtcag tccggagtca taatgtaaga 29580

ctcggtaaac acatcaggtt gattcacatc ggtcagtgtt aaaaagcgac cgaaatagcc 29640

cgggggaata catacccgca ggcgtagaga caacattaca gcccccatag gaggtataac 29700

aaaattaata ggagagaaaa acacataaac acctgaaaaa ccctcctgcc taggcaaaat 29760

agcaccctcc cgctccagaa caacatacag cgcttcacag cggcagccta acagtcagcc 29820

ttaccagtaa aaaagaaaac ctattaaaaa aacaccactc gacacggcac cagctcaatc 29880

agtcacagtg taaaaaaggg ccaagtgcag agcgagtata tataggacta aaaaatgacg 29940

taacggttaa agtccacaaa aaacacccag aaaaccgcac gcgaacctac gcccagaaac 30000

gaaagccaaa aaacccacaa cttcctcaaa tcgtcacttc cgttttccca cgttacgtaa 30060

cttcccattt taagaaaact acaattccca acacatacaa gttactccgc cctaaaacct 30120

acgtcacccg ccccgttccc acgccccgcg ccacgtcaca aactccaccc cctcattatc 30180

atattggctt caatccaaaa taaggtatat tattgatgat gnnttaatta agggcggccg 30240

cacgggccat cgatggatcc gcggaagctt ctcgacccat tctcatgttt gacagcttat 30300

catcgaattt ctgccattca tccgcttatt atcacttatt caggcgtagc aaccaggcgt 30360

ttaagggcac caataactgc cttaaaaaaa ttacgccccg ccctgccact catcgcagta 30420

ctgttgtaat tcattaagca ttctgccgac atggaagcca tcacagacgg catgatgaac 30480

ctgaatcgcc agcggcatca gcaccttgtc gccttgcgta taatatttgc ccatggtgaa 30540

aacgggggcg aagaagttgt ccatattggc cacgtttaaa tcaaaactgg tgaaactcac 30600

ccagggattg gctgagacga aaaacatatt ctcaataaac cctttaggga aataggccag 30660

gttttcaccg taacacgcca catcttgcga atatatgtgt agaaactgcc ggaaatcgtc 30720

gtggtattca ctccagagcg atgaaaacgt ttcagtttgc tcatggaaaa cggtgtaaca 30780

agggtgaaca ctatcccata tcaccagctc accgtctttc attgccatac ggaattccgg 30840

atgagcattc atcaggcggg caagaatgtg aataaaggcc ggataaaact tgtgcttatt 30900

tttctttacg gtctttaaaa aggccgtaat atccagctga acggtctggt tataggtaca 30960

ttgagcaact gactgaaatg cctcaaaatg ttctttacga tgccattggg atatatcaac 31020

ggtggtatat ccagtgattt ttttctccat tttagcttcc ttagctcctg aaaatctcga 31080

taactcaaaa aatacgcccg gtagtgatct tatttcatta tggtgaaagt tggaacctct 31140

tacgtgccga tcaacgtctc attttcgcca aaagttggcc cagggcttcc cggtatcaac 31200

agggacacca ggatttattt attctgcgaa gtgatcttcc gtcacaggta tttattcgcg 31260

ataagctcat ggagcggcgt aaccgtcgca caggaaggac agagaaagcg cggatctggg 31320

aagtgacgga cagaacggtc aggacctgga ttggggaggc ggttgccgcc gctgctgctg 31380

acggtgtgac gttctctgtt ccggtcacac cacatacgtt ccgccattcc tatgcgatgc 31440

acatgctgta tgccggtata ccgctgaaag ttctgcaaag cctgatggga cataagtcca 31500

tcagttcaac ggaagtctac acgaaggttt ttgcgctgga tgtggctgcc cggcaccggg 31560

tgcagtttgc gatgccggag tctgatgcgg ttgcgatgct gaaacaatta tcctgagaat 31620

aaatgccttg gcctttatat ggaaatgtgg aactgagtgg atatgctgtt tttgtctgtt 31680

aaacagagaa gctggctgtt atccactgag aagcgaacga aacagtcggg aaaatctccc 31740

attatcgtag agatccgcat tattaatctc aggagcctgt gtagcgttta taggaagtag 31800

tgttctgtca tgatgcctgc aagcggtaac gaaaacgatt tgaatatgcc ttcaggaaca 31860
atagaaatct tcgtgcggtg ttacgttgaa gtggagcgga ttatgtcagc aatggacaga 31920
acaacctaat gaacacagaa ccatgatgtg gtctgtcctt ttacagccag taggctcgcc 31980
gcagtcgagc gacggcgaag ccctcgagtg agcgaggaag caccagggaa cagcacttat 32040
atattctgct tacacacgat gcctgaaaaa acttcccttg gggttatcca cttatccacg 32100
gggatatttt tataattatt ttttttatag tttttagatc ttctttttta gagcgccttg 32160
taggccttta tccatgctgg ttctagagaa ggtgttgtga caaattgccc tttcagtgtg 32220
acaaatcacc ctcaaatgac agtcctgtct gtgacaaatt gcccttaacc ctgtgacaaa 32280
ttgccctcag aagaagctgt tttttcacaa agttatccct gcttattgac tcttttttat 32340
ttagtgtgac aatctaaaaa cttgtcacac ttcacatgga tctgtcatgg cggaaacagc 32400
ggttatcaat cacaagaaac gtaaaaatag cccgcgaatc gtccagtcaa acgacctcac 32460
tgaggcggca tatagtctct cccgggatca aaaacgtatg ctgtatctgt tcgttgacca 32520
gatcagaaaa tctgatggca ccctacagga acatgacggt atctgcgaga tccatgttgc 32580
taaatatgct gaaatattcg gattgacctc tgcggaagcc agtaaggata tacggcaggc 32640
attgaagagt ttcgcgggga aggaagtggt tttttatcgc cctgaagagg atgccggcga 32700
tgaaaaaggc tatgaatctt ttccttggtt tatcaaacgt gcgcacagtc catccagagg 32760
gctttacagt gtacatatca acccatatct cattcccttc tttatcgggt tacagaaccg 32820
gtttacgcag tttcggctta gtgaaacaaa agaaatcacc aatccgtatg ccatgcgttt 32880
atacgaatcc ctgtgtcagt atcgtaagcc ggatggctca ggcatcgtct ctctgaaaat 32940
cgactggatc atagagcgtt accagctgcc tcaaagttac cagcgtatgc ctgacttccg 33000
ccgccgcttc ctgcaggtct gtgttaatga gatcaacagc agaactccaa tgcgcctctc 33060
atacattgag aaaaagaaag gccgccagac gactcatatc gtattttcct tccgcgatat 33120
cacttccatg acgacaggat agtctgaggg ttatctgtca cagatttgag ggtggttcgt 33180
cacatttgtt ctgacctact gagggtaatt tgtcacagtt ttgctgtttc cttcagcctg 33240
catggatttt ctcatacttt ttgaactgta atttttaagg aagccaaatt tgagggcagt 33300
ttgtcacagt tgatttcctt ctctttccct tcgtcatgtg acctgatatc gggggttagt 33360
tcgtcatcat tgatgagggt tgattatcac agtttattac tctgaattgg ctatccgcgt 33420
gtgtacctct acctggagtt tttcccacgg tggatatttc ttcttgcgct gagcgtaaga 33480
gctatctgac agaacagttc ttctttgctt cctcgccagt tcgctcgcta tgctcggtta 33540
cacggctgcg gcgagcgcta gtgataataa gtgactgagg tatgtgctct tcttatctcc 33600
ttttgtagtg ttgctcttat tttaaacaac tttgcggttt tttgatgact ttgcgatttt 33660

gttgttgctt tgcagtaaat tgcaagattt aataaaaaaa cgcaaagcaa tgattaaagg 33720
atgttcagaa tgaaactcat ggaaacactt aaccagtgca taaacgctgg tcatgaaatg 33780
acgaaggcta tcgccattgc acagtttaat gatgacagcc cggaagcgag gaaaataacc 33840
cggcgctgga gaataggtga agcagcggat ttagttgggg tttcttctca ggctatcaga 33900
gatgccgaga aagcagggcg actaccgcac ccggatatgg aaattcgagg acgggttgag 33960
caacgtgttg gttatacaat tgaacaaatt aatcatatgc gtgatgtgtt tggtacgcga 34020
ttgcgacgtg ctgaagacgt atttccaccg gtgatcgggg ttgctgccca taaaggtggc 34080
gtttacaaaa cctcagtttc tgttcatctt gctcaggatc tggctctgaa ggggctacgt 34140
gttttgctcg tggaaggtaa cgacccccag ggaacagcct caatgtatca cggatgggta 34200
ccagatcttc atattcatgc agaagacact ctcctgcctt tctatcttgg ggaaaaggac 34260
gatgtcactt atgcaataaa gcccacttgc tggccggggc ttgacattat tccttcctgt 34320
ctggctctgc accgtattga aactgagtta atgggcaaat ttgatgaagg taaactgccc 34380
accgatccac acctgatgct ccgactggcc attgaaactg ttgctcatga ctatgatgtc 34440
atagttattg acagcgcgcc taacctgggt atcggcacga ttaatgtcgt atgtgctgct 34500
gatgtgctga ttgttcccac gcctgctgag ttgtttgact acacctccgc actgcagttt 34560
ttcgatatgc ttcgtgatct gctcaagaac gttgatctta aagggttcga gcctgatgta 34620
cgtattttgc ttaccaaata cagcaatagt aatggctctc agtccccgtg gatggaggag 34680
caaattcggg atgcctgggg aagcatggtt ctaaaaaatg ttgtacgtga aacggatgaa 34740
gttggtaaag gtcagatccg gatgagaact gtttttgaac aggccattga tcaacgctct 34800
tcaactggtg cctggagaaa tgctctttct atttgggaac ctgtctgcaa tgaaattttc 34860
gatcgtctga ttaaaccacg ctgggagatt agataatgaa gcgtgcgcct gttattccaa 34920
aacatacgct caatactcaa ccggttgaag atacttcgtt atcgacacca gctgccccga 34980
tggtggattc gttaattgcg cgcgtaggag taatggctcg cggtaatgcc attactttgc 35040
ctgtatgtgg tcgggatgtg aagtttactc ttgaagtgct ccggggtgat agtgttgaga 35100
agacctctcg ggtatggtca ggtaatgaac gtgaccagga gctgcttact gaggacgcac 35160
tggatgatct catcccttct tttctactga ctggtcaaca gacaccggcg ttcggtcgaa 35220
gagtatctgg tgtcatagaa attgccgatg ggagtcgccg tcgtaaagct gctgcactta 35280
ccgaaagtga ttatcgtgtt ctggttggcg agctggatga tgagcagatg gctgcattat 35340
ccagattggg taacgattat cgcccaacaa gtgcttatga acgtggtcag cgttatgcaa 35400
gccgattgca gaatgaattt gctggaaata tttctgcgct ggctgatgcg gaaaatattt 35460
cacgtaagat tattacccgc tgtatcaaca ccgccaaatt gcctaaatca gttgttgctc 35520
ttttttctca ccccggtgaa ctatctgccc ggtcaggtga tgcacttcaa aaagccttta 35580

cagataaaga ggaattactt aagcagcagg catctaacct tcatgagcag aaaaaagctg 35640

gggtgatatt tgaagctgaa gaagttatca ctcttttaac ttctgtgctt aaaacgtcat 35700

ctgcatcaag aactagttta agctcacgac atcagtttgc tcctggagcg acagtattgt 35760

ataagggcga taaaatggtg cttaacctgg acaggtctcg tgttccaact gagtgtatag 35820

agaaaattga ggccattctt aaggaacttg aaaagccagc accctgatgc gaccacgttt 35880

tagtctacgt ttatctgtct ttacttaatg tcctttgtta caggccagaa agcataactg 35940

gcctgaatat tctctctggg cccactgttc cacttgtatc gtcggtctga taatcagact 36000

gggaccacgg tcccactcgt atcgtcggtc tgattattag tctgggacca cggtcccact 36060

cgtatcgtcg gtctgattat tagtctggga ccacggtccc actcgtatcg tcggtctgat 36120

aatcagactg ggaccacggt cccactcgta tcgtcggtct gattattagt ctgggaccat 36180

ggtcccactc gtatcgtcgg tctgattatt agtctgggac cacggtccca ctcgtatcgt 36240

cggtctgatt attagtctgg aaccacggtc ccactcgtat cgtcggtctg attattagtc 36300

tgggaccacg gtcccactcg tatcgtcggt ctgattatta gtctgggacc acgatcccac 36360

tcgtgttgtc ggtctgatta tcggtctggg accacggtcc cacttgtatt gtcgatcaga 36420

ctatcagcgt gagactacga ttccatcaat gcctgtcaag ggcaagtatt gacatgtcgt 36480

cgtaacctgt agaacggagt aacctcggtg tgcggttgta tgcctgctgt ggattgctgc 36540

tgtgtcctgc ttatccacaa cattttgcgc acggttatgt ggacaaaata cctggttacc 36600

caggccgtgc cggcacgtta accgggctgc atccgatgca agtgtgtcgc tgtcgagttt 36660

aaacatgcat ccttaattaa 36680

<210> 4
<211> 39730
<212> DNA
<213> Artificial

<220>
<223> synthetic


<220>
<221> misc_feature
<222> (31002)..(31002)
<223> n is a, c, g, or t

<220>
<221> misc_feature
<222> (33272)..(33273)
<223> n is a, c, g, or t

<400> 4
   catcatcaat aatatacctt attttggatt gaagccaata tgataatgag ggggtggagt 60
   ttgtgacgtg gcgcggggcg tgggaacggg gcgggtgacg tagtagtgtg gcggaagtgt 120
   gatgttgcaa gtgtggcgga acacatgtaa gcgacggatg tggcaaaagt gacgtttttg 180
   gtgtgcgccg gtgtacacag gaagtgacaa ttttcgcgcg gttttaggcg gatgttgtag 240
   taaatttggg cgtaaccgag taagatttgg ccattttcgc gggaaaactg aataagagga 300
   agtgaaatct gaataatttt gtgttactca tagcgcgtaa actggtaccc ggggatcttg 360
   aagttcctat tccgaagttc ctattctcta gaaagtatag gaacttcctt aagtgagtcg 420
   tattacggac tggccgtcgt tttacaacgt cgtgactggg aaaaccctgg cgttacccaa 480
   cttaatcgcc ttgcagcaca tccccctttc gccagctggc gtaatagcga agaggcccgc 540
   accgatcgcc cttcccaaca gttgcgcagc ctgaatggcg aatggcgctt cgcttggtaa 600
   gctagctaag gcttaaggag gacaatcatg gcaacagtta accagctggt acgcaaacca 660
   cgtgctcgca aagttgcgaa aagcaacgtg cctgcgctgg aagcatgccc gcaaaaacgt 720
   ggcgtatgta ctcgtgtata tactaccact cctaaaaaac cgaactccgc gctgcgtaaa 780
   gtatgccgtg ttcgtctgtc taacggtttc gaagtgactt cctacatcgg tggtgaaggt 840
   cacaacctgc aggagcactc cgtgatcctg atccgtggcg gtcgtgttaa agacctcccg 900
   ggtgttcgtt accacaccgt acgtggtgcg cttgactgct ccggcgttaa agaccgtaag 960
   caggctcgtt ccaagtatgg cgtgaagcgt cctaaggctt aactagcacg cgccatgtca 1020
   gccgttaagt gttcctgtgt cactcaaaat tgctttgaga ggctctaagg gcttctcagt 1080
   gcgttacatc cctggcttgt tgtccacaac cgttaaacct taaaagcttt aaaagcctta 1140
   tatattcttt tttttcttat aaaacttaaa accttagagg ctatttaagt tgctgattta 1200
   tattaatttt attgttcaaa catgagagct tagtacgtga aacatgagag cttagtacgt 1260
   tagccatgag agcttagtac gttagccatg agggtttagt tcgttaaaca tgagagctta 1320
   gtacgttaaa catgagagct tagtacgtga aacatgagag cttagtacgt actatcaaca 1380
   ggttgaactg ctgatcttca gatcctctac gccggacgca tcgtggccgg atccgattta 1440
   ttcaacaaag ccacgttgtg tctcaaaatc tctgatgtta cattgcacaa gataaaaata 1500
   tatcatcatg aacaataaaa ctgtctgctt acataaacag taatacaagg ggtgttatga 1560
   gccatattca acgggaaacg tcttgctcga ggccgcgatt aaattccaac atggatgctg 1620
   atttatatgg gtataaatgg gctcgcgata atgtcgggca atcaggtgcg acaatctatc 1680
   gattgtatgg gaagcccgat gcgccagagt tgtttctgaa acatggcaaa ggtagcgttg 1740
   ccaatgatgt tacagatgag atggtcagac taaactggct gacggaattt atgcctcttc 1800
   cgaccatcaa gcattttatc cgtactcctg atgatgcatg gttactcacc actgcgatcc 1860
   ccgggaaaac agcattccag gtattagaag aatatcctga ttcaggtgaa aatattgttg 1920
   atgcgctggc agtgttcctg cgccggttgc attcgattcc tgtttgtaat tgtcctttta 1980
   acagcgatcg cgtatttcgt ctcgctcagg cgcaatcacg aatgaataac ggtttggttg 2040
   atgcgagtga ttttgatgac gagcgtaatg gctggcctgt tgaacaagtc tggaaagaaa 2100
   tgcataagct tttgccattc tcaccggatt cagtcgtcac tcatggtgat ttctcacttg 2160
   ataaccttat ttttgacgag gggaaattaa taggttgtat tgatgttgga cgagtcggaa 2220
   tcgcagaccg ataccaggat cttgccatcc tatggaactg cctcggtgag ttttctcctt 2280
   cattacagaa acggcttttt caaaaatatg gtattgataa tcctgatatg aataaattgc 2340
   agtttcattt gatgctcgat gagtttttct aatcagaatt ggttaattgg ttgtaacact 2400
   ggcttaatta acatcatcaa taatatacct tattttggat tgaagccaat atgataatga 2460
   gggggtggag tttgtgacgt ggcgcggggc gtgggaacgg ggcgggtgac gtagtagtgt 2520
   ggcggaagtg tgatgttgca agtgtggcgg aacacatgta agcgacggat gtggcaaaag 2580
   tgacgttttt ggtgtgcgcc ggtgtacaca ggaagtgaca attttcgcgc ggttttaggc 2640
   ggatgttgta gtaaatttgg gcgtaaccga gtaagatttg gccattttcg cgggaaaact 2700
   gaataagagg aagtgaaatc tgaataattt tgtgttactc atagcgcgta atactggtac 2760
   cccccagtgg caagacgcgc aggcaaaacg caccacgact ggatccgaat tcaaaaagcc 2820
   ggaactgatc agaaccattc tcttgaaatg gttctgatca gttccggccg gtgtttcgtc 2880
   ctttccacaa gatatataaa gccaagaaat cgaaatactt tcaagttacg gtaagcatat 2940
   gatagtccat tttaaaacat aattttaaaa ctgcaaacta cccaagaaat tattactttc 3000
   tacgtcacgt attttgtact aatatctttg tgtttacagt caaattaatt ctaattatct 3060
   ttctaacagc cttgtatcgt atatgcaaat atgaaggaat catgggaaat aggccctctt 3120
   cctgcccgac cttggcgcgc gctcggcgcg cggtcacgct ccgtcacgtg gtgcgttttg 3180
   cctgcgcgtc ttgccactgg ggggtacccc gaatcactag atctgagctc ggcctggtga 3240
   tgatggcggg atcgttgtat atttcttgac accttttcgg catcgcccta aaattcggcg 3300
   tcctcatatt gtgtgaggac gttttattac gtgtttacga agcaaaagct aaaaccagga 3360
   gctatttaat ggcaacaacc ggtaccatga ttacgccaag ctacgtaata cgactcacta 3420
   gtggaagttc ctattctcta gaaagtatag gaacttcaag agcgcttttg agcttgttta 3480
   tcgaattcaa gcttgtcgac tcgaagatct gggcgtggtt aagggtggga aagaatatat 3540
   aaggtggggg tcttatgtag ttttgtatct gttttgcagc agccgccgcc gccatgagca 3600
   ccaactcgtt tgatggaagc attgtgagct catatttgac aacgcgcatg cccccatggg 3660
   ccggggtgcg tcagaatgtg atgggctcca gcattgatgg tcgccccgtc ctgcccgcaa 3720
   actctactac cttgacctac gagaccgtgt ctggaacgcc gttggagact gcagcctccg 3780
   ccgccgcttc agccgctgca gccaccgccc gcgggattgt gactgacttt gctttcctga 3840
   gcccgcttgc aagcagtgca gcttcccgtt catccgcccg cgatgacaag ttgacggctc 3900
   ttttggcaca attggattct ttgacccggg aacttaatgt cgtttctcag cagctgttgg 3960
   atctgcgcca gcaggtttct gccctgaagg cttcctcccc tcccaatgcg gtttaaaaca 4020
   taaataaaaa accagactct gtttggattt ggatcaagca agtgtcttgc tgtctttatt 4080
   taggggtttt gcgcgcgcgg taggcccggg accagcggtc tcggtcgttg agggtcctgt 4140
   gtattttttc caggacgtgg taaaggtgac tctggatgtt cagatacatg ggcataagcc 4200
   cgtctctggg gtggaggtag caccactgca gagcttcatg ctgcggggtg gtgttgtaga 4260
   tgatccagtc gtagcaggag cgctgggcgt ggtgcctaaa aatgtctttc agtagcaagc 4320
   tgattgccag gggcaggccc ttggtgtaag tgtttacaaa gcggttaagc tgggatgggt 4380
   gcatacgtgg ggatatgaga tgcatcttgg actgtatttt taggttggct atgttcccag 4440
   ccatatccct ccggggattc atgttgtgca gaaccaccag cacagtgtat ccggtgcact 4500
   tgggaaattt gtcatgtagc ttagaaggaa atgcgtggaa gaacttggag acgcccttgt 4560
   gacctccaag attttccatg cattcgtcca taatgatggc aatgggccca cgggcggcgg 4620
   cctgggcgaa gatatttctg ggatcactaa cgtcatagtt gtgttccagg atgagatcgt 4680
   cataggccat ttttacaaag cgcgggcgga gggtgccaga ctgcggtata atggttccat 4740
   ccggcccagg ggcgtagtta ccctcacaga tttgcatttc ccacgctttg agttcagatg 4800
   gggggatcat gtctacctgc ggggcgatga agaaaacggt ttccggggta ggggagatca 4860
   gctgggaaga aagcaggttc ctgagcagct gcgacttacc gcagccggtg ggcccgtaaa 4920
   tcacacctat taccgggtgc aactggtagt taagagagct gcagctgccg tcatccctga 4980
   gcaggggggc cacttcgtta agcatgtccc tgactcgcat gttttccctg accaaatccg 5040
   ccagaaggcg ctcgccgccc agcgatagca gttcttgcaa ggaagcaaag tttttcaacg 5100
   gtttgagacc gtccgccgta ggcatgcttt tgagcgtttg accaagcagt tccaggcggt 5160
   cccacagctc ggtcacctgc tctacggcat ctcgatccag catatctcct cgtttcgcgg 5220
   gttggggcgg ctttcgctgt acggcagtag tcggtgctcg tccagacggg ccagggtcat 5280
   gtctttccac gggcgcaggg tcctcgtcag cgtagtctgg gtcacggtga aggggtgcgc 5340
   tccgggctgc gcgctggcca gggtgcgctt gaggctggtc ctgctggtgc tgaagcgctg 5400
   ccggtcttcg ccctgcgcgt cggccaggta gcatttgacc atggtgtcat agtccagccc 5460
   ctccgcggcg tggcccttgg cgcgcagctt gcccttggag gaggcgccgc acgaggggca 5520
   gtgcagactt ttgagggcgt agagcttggg cgcgagaaat accgattccg gggagtaggc 5580
   atccgcgccg caggccccgc agacggtctc gcattccacg agccaggtga gctctggccg 5640
   ttcggggtca aaaaccaggt ttcccccatg ctttttgatg cgtttcttac ctctggtttc 5700
   catgagccgg tgtccacgct cggtgacgaa aaggctgtcc gtgtccccgt atacagactt 5760
   gagaggcctg tcctcgagcg gtgttccgcg gtcctcctcg tatagaaact cggaccactc 5820
   tgagacaaag gctcgcgtcc aggccagcac gaaggaggct aagtgggagg ggtagcggtc 5880
   gttgtccact agggggtcca ctcgctccag ggtgtgaaga cacatgtcgc cctcttcggc 5940
   atcaaggaag gtgattggtt tgtaggtgta ggccacgtga ccgggtgttc ctgaaggggg 6000
   gctataaaag ggggtggggg cgcgttcgtc ctcactctct tccgcatcgc tgtctgcgag 6060
   ggccagctgt tggggtgagt actccctctg aaaagcgggc atgacttctg cgctaagatt 6120
   gtcagtttcc aaaaacgagg aggatttgat attcacctgg cccgcggtga tgcctttgag 6180
   ggtggccgca tccatctggt cagaaaagac aatctttttg ttgtcaagct tggtggcaaa 6240
   cgacccgtag agggcgttgg acagcaactt ggcgatggag cgcagggttt ggtttttgtc 6300
   gcgatcggcg cgctccttgg ccgcgatgtt tagctgcacg tattcgcgcg caacgcaccg 6360
   ccattcggga aagacggtgg tgcgctcgtc gggcaccagg tgcacgcgcc aaccgcggtt 6420
   gtgcagggtg acaaggtcaa cgctggtggc tacctctccg cgtaggcgct cgttggtcca 6480
   gcagaggcgg ccgcccttgc gcgagcagaa tggcggtagg gggtctagct gcgtctcgtc 6540
   cggggggtct gcgtccacgg taaagacccc gggcagcagg cgcgcgtcga agtagtctat 6600
   cttgcatcct tgcaagtcta gcgcctgctg ccatgcgcgg gcggcaagcg cgcgctcgta 6660
   tgggttgagt gggggacccc atggcatggg gtgggtgagc gcggaggcgt acatgccgca 6720
   aatgtcgtaa acgtagaggg gctctctgag tattccaaga tatgtagggt agcatcttcc 6780
   accgcggatg ctggcgcgca cgtaatcgta tagttcgtgc gagggagcga ggaggtcggg 6840
   accgaggttg ctacgggcgg gctgctctgc tcggaagact atctgcctga agatggcatg 6900
   tgagttggat gatatggttg gacgctggaa gacgttgaag ctggcgtctg tgagacctac 6960
   cgcgtcacgc acgaaggagg cgtaggagtc gcgcagcttg ttgaccagct cggcggtgac 7020
   ctgcacgtct agggcgcagt agtccagggt ttccttgatg atgtcatact tatcctgtcc 7080
   cttttttttc cacagctcgc ggttgaggac aaactcttcg cggtctttcc agtactcttg 7140
   gatcggaaac ccgtcggcct ccgaacggta agagcctagc atgtagaact ggttgacggc 7200
   ctggtaggcg cagcatccct tttctacggg tagcgcgtat gcctgcgcgg ccttccggag 7260
   cgaggtgtgg gtgagcgcaa aggtgtccct gaccatgact ttgaggtact ggtatttgaa 7320
   gtcagtgtcg tcgcatccgc cctgctccca gagcaaaaag tccgtgcgct ttttggaacg 7380
   cggatttggc agggcgaagg tgacatcgtt gaagagtatc tttcccgcgc gaggcataaa 7440
   gttgcgtgtg atgcggaagg gtcccggcac ctcggaacgg ttgttaatta cctgggcggc 7500
   gagcacgatc tcgtcaaagc cgttgatgtt gtggcccaca atgtaaagtt ccaagaagcg 7560
   cgggatgccc ttgatggaag gcaatttttt aagttcctcg taggtgagct cttcagggga 7620
   gctgagcccg tgctctgaaa gggcccagtc tgcaagatga gggttggaag cgacgaatga 7680
   gctccacagg tcacgggcca ttagcatttg caggtggtcg cgaaaggtcc taaactggcg 7740
   acctatggcc attttttctg gggtgatgca gtagaaggta agcgggtctt gttcccagcg 7800
   gtcccatcca aggttcgcgg ctaggtctcg cgcggcagtc actagaggct catctccgcc 7860
   gaacttcatg accagcatga agggcacgag ctgcttccca aaggccccca tccaagtata 7920
   ggtctctaca tcgtaggtga caaagagacg ctcggtgcga ggatgcgagc cgatcgggaa 7980
   gaactggatc tcccgccacc aattggagga gtggctattg atgtggtgaa agtagaagtc 8040
   cctgcgacgg gccgaacact cgtgctggct tttgtaaaaa cgtgcgcagt actggcagcg 8100
   gtgcacgggc tgtacatcct gcacgaggtt gacctgacga ccgcgcacaa ggaagcagag 8160
   tgggaatttg agcccctcgc ctggcgggtt tggctggtgg tcttctactt cggctgcttg 8220
   tccttgaccg tctggctgct cgaggggagt tacggtggat cggaccacca cgccgcgcga 8280
   gcccaaagtc cagatgtccg cgcgcggcgg tcggagcttg atgacaacat cgcgcagatg 8340
   ggagctgtcc atggtctgga gctcccgcgg cgtcaggtca ggcgggagct cctgcaggtt 8400
   tacctcgcat agacgggtca gggcgcgggc tagatccagg tgatacctaa tttccagggg 8460
   ctggttggtg gcggcgtcga tggcttgcaa gaggccgcat ccccgcggcg cgactacggt 8520
   accgcgcggc gggcggtggg ccgcgggggt gtccttggat gatgcatcta aaagcggtga 8580
   cgcgggcgag cccccggagg tagggggggc tccggacccg ccgggagagg gggcaggggc 8640
   acgtcggcgc cgcgcgcggg caggagctgg tgctgcgcgc gtaggttgct ggcgaacgcg 8700
   acgacgcggc ggttgatctc ctgaatctgg cgcctctgcg tgaagacgac gggcccggtg 8760
   agcttgagcc tgaaagagag ttcgacagaa tcaatttcgg tgtcgttgac ggcggcctgg 8820
   cgcaaaatct cctgcacgtc tcctgagttg tcttgatagg cgatctcggc catgaactgc 8880
   tcgatctctt cctcctggag atctccgcgt ccggctcgct ccacggtggc ggcgaggtcg 8940
   ttggaaatgc gggccatgag ctgcgagaag gcgttgaggc ctccctcgtt ccagacgcgg 9000
   ctgtagacca cgcccccttc ggcatcgcgg gcgcgcatga ccacctgcgc gagattgagc 9060
   tccacgtgcc gggcgaagac ggcgtagttt cgcaggcgct gaaagaggta gttgagggtg 9120
   gtggcggtgt gttctgccac gaagaagtac ataacccagc gtcgcaacgt ggattcgttg 9180
   atatccccca aggcctcaag gcgctccatg gcctcgtaga agtccacggc gaagttgaaa 9240
   aactgggagt tgcgcgccga cacggttaac tcctcctcca gaagacggat gagctcggcg 9300
   acagtgtcgc gcacctcgcg ctcaaaggct acaggggcct cttcttcttc ttcaatctcc 9360
   tcttccataa gggcctcccc ttcttcttct tctggcggcg gtgggggagg ggggacacgg 9420
   cggcgacgac ggcgcaccgg gaggcggtcg acaaagcgct cgatcatctc cccgcggcga 9480
   cggcgcatgg tctcggtgac ggcgcggccg ttctcgcggg ggcgcagttg gaagacgccg 9540
   cccgtcatgt cccggttatg ggttggcggg gggctgccat gcggcaggga tacggcgcta 9600
   acgatgcatc tcaacaattg ttgtgtaggt actccgccgc cgagggacct gagcgagtcc 9660
   gcatcgaccg gatcggaaaa cctctcgaga aaggcgtcta accagtcaca gtcgcaaggt 9720
   aggctgagca ccgtggcggg cggcagcggg cggcggtcgg ggttgtttct ggcggaggtg 9780
   ctgctgatga tgtaattaaa gtaggcggtc ttgagacggc ggatggtcga cagaagcacc 9840
   atgtccttgg gtccggcctg ctgaatgcgc aggcggtcgg ccatgcccca ggcttcgttt 9900
   tgacatcggc gcaggtcttt gtagtagtct tgcatgagcc tttctaccgg cacttcttct 9960
   tctccttcct cttgtcctgc atctcttgca tctatcgctg cggcggcggc ggagtttggc 10020
   cgtaggtggc gccctcttcc tcccatgcgt gtgaccccga agcccctcat cggctgaagc 10080
   agggctaggt cggcgacaac gcgctcggct aatatggcct gctgcacctg cgtgagggta 10140
   gactggaagt catccatgtc cacaaagcgg tggtatgcgc ccgtgttgat ggtgtaagtg 10200
   cagttggcca taacggacca gttaacggtc tggtgacccg gctgcgagag ctcggtgtac 10260
   ctgagacgcg agtaagccct cgagtcaaat acgtagtcgt tgcaagtccg caccaggtac 10320
   tggtatccca ccaaaaagtg cggcggcggc tggcggtaga ggggccagcg tagggtggcc 10380
   ggggctccgg gggcgagatc ttccaacata aggcgatgat atccgtagat gtacctggac 10440
   atccaggtga tgccggcggc ggtggtggag gcgcgcggaa agtcgcggac gcggttccag 10500
   atgttgcgca gcggcaaaaa gtgctccatg gtcgggacgc tctggccggt caggcgcgcg 10560
   caatcgttga cgctctaccg tgcaaaagga gagcctgtaa gcgggcactc ttccgtggtc 10620
   tggtggataa attcgcaagg gtatcatggc ggacgaccgg ggttcgagcc ccgtatccgg 10680
   ccgtccgccg tgatccatgc ggttaccgcc cgcgtgtcga acccaggtgt gcgacgtcag 10740
   acaacggggg agtgctcctt ttggcttcct tccaggcgcg gcggctgctg cgctagcttt 10800
   tttggccact ggccgcgcgc agcgtaagcg gttaggctgg aaagcgaaag cattaagtgg 10860
   ctcgctccct gtagccggag ggttattttc caagggttga gtcgcgggac ccccggttcg 10920
   agtctcggac cggccggact gcggcgaacg ggggtttgcc tccccgtcat gcaagacccc 10980
   gcttgcaaat tcctccggaa acagggacga gccccttttt tgcttttccc agatgcatcc 11040
   ggtgctgcgg cagatgcgcc cccctcctca gcagcggcaa gagcaagagc agcggcagac 11100
   atgcagggca ccctcccctc ctcctaccgc gtcaggaggg gcgacatccg cggttgacgc 11160
   ggcagcagat ggtgattacg aacccccgcg gcgccgggcc cggcactacc tggacttgga 11220
   ggagggcgag ggcctggcgc ggctaggagc gccctctcct gagcggtacc caagggtgca 11280
   gctgaagcgt gatacgcgtg aggcgtacgt gccgcggcag aacctgtttc gcgaccgcga 11340
   gggagaggag cccgaggaga tgcgggatcg aaagttccac gcagggcgcg agctgcggca 11400
   tggcctgaat cgcgagcggt tgctgcgcga ggaggacttt gagcccgacg cgcgaaccgg 11460
   gattagtccc gcgcgcgcac acgtggcggc cgccgacctg gtaaccgcat acgagcagac 11520
   ggtgaaccag gagattaact ttcaaaaaag ctttaacaac cacgtgcgta cgcttgtggc 11580
   gcgcgaggag gtggctatag gactgatgca tctgtgggac tttgtaagcg cgctggagca 11640
   aaacccaaat agcaagccgc tcatggcgca gctgttcctt atagtgcagc acagcaggga 11700
   caacgaggca ttcagggatg cgctgctaaa catagtagag cccgagggcc gctggctgct 11760
   cgatttgata aacatcctgc agagcatagt ggtgcaggag cgcagcttga gcctggctga 11820
   caaggtggcc gccatcaact attccatgct tagcctgggc aagttttacg cccgcaagat 11880
   ataccatacc ccttacgttc ccatagacaa ggaggtaaag atcgaggggt tctacatgcg 11940
   catggcgctg aaggtgctta ccttgagcga cgacctgggc gtttatcgca acgagcgcat 12000
   ccacaaggcc gtgagcgtga gccggcggcg cgagctcagc gaccgcgagc tgatgcacag 12060
   cctgcaaagg gccctggctg gcacgggcag cggcgataga gaggccgagt cctactttga 12120
   cgcgggcgct gacctgcgct gggccccaag ccgacgcgcc ctggaggcag ctggggccgg 12180
   acctgggctg gcggtggcac ccgcgcgcgc tggcaacgtc ggcggcgtgg aggaatatga 12240
   cgaggacgat gagtacgagc cagaggacgg cgagtactaa gcggtgatgt ttctgatcag 12300
   atgatgcaag acgcaacgga cccggcggtg cgggcggcgc tgcagagcca gccgtccggc 12360
   cttaactcca cggacgactg gcgccaggtc atggaccgca tcatgtcgct gactgcgcgc 12420
   aatcctgacg cgttccggca gcagccgcag gccaaccggc tctccgcaat tctggaagcg 12480
   gtggtcccgg cgcgcgcaaa ccccacgcac gagaaggtgc tggcgatcgt aaacgcgctg 12540
   gccgaaaaca gggccatccg gcccgacgag gccggcctgg tctacgacgc gctgcttcag 12600
   cgcgtggctc gttacaacag cggcaacgtg cagaccaacc tggaccggct ggtgggggat 12660
   gtgcgcgagg ccgtggcgca gcgtgagcgc gcgcagcagc agggcaacct gggctccatg 12720
   gttgcactaa acgccttcct gagtacacag cccgccaacg tgccgcgggg acaggaggac 12780
   tacaccaact ttgtgagcgc actgcggcta atggtgactg agacaccgca aagtgaggtg 12840
   taccagtctg ggccagacta tttttttcag accagtagac aaggcctgca gaccgtaaac 12900
   ctgagccagg ctttcaaaaa cttgcagggg ctgtgggggg tgcgggctcc cacaggcgac 12960
   cgcgcgaccg tgtctagctt gctgacgccc aactcgcgcc tgttgctgct gctaatagcg 13020
   cccttcacgg acagtggcag cgtgtcccgg gacacatacc taggtcactt gctgacactg 13080
   taccgcgagg ccataggtca ggcgcatgtg gacgagcata ctttccagga gattacaagt 13140
   gtcagccgcg cgctggggca ggaggacacg ggcagcctgg aggcaaccct aaactacctg 13200
   ctgaccaacc ggcggcagaa gatcccctcg ttgcacagtt taaacagcga ggaggagcgc 13260
   attttgcgct acgtgcagca gagcgtgagc cttaacctga tgcgcgacgg ggtaacgccc 13320
   agcgtggcgc tggacatgac cgcgcgcaac atggaaccgg gcatgtatgc ctcaaaccgg 13380
   ccgtttatca accgcctaat ggactacttg catcgcgcgg ccgccgtgaa ccccgagtat 13440
   ttcaccaatg ccatcttgaa cccgcactgg ctaccgcccc ctggtttcta caccggggga 13500
   ttcgaggtgc ccgagggtaa cgatggattc ctctgggacg acatagacga cagcgtgttt 13560
   tccccgcaac cgcagaccct gctagagttg caacagcgcg agcaggcaga ggcggcgctg 13620
   cgaaaggaaa gcttccgcag gccaagcagc ttgtccgatc taggcgctgc ggccccgcgg 13680
   tcagatgcta gtagcccatt tccaagcttg atagggtctc ttaccagcac tcgcaccacc 13740
   cgcccgcgcc tgctgggcga ggaggagtac ctaaacaact cgctgctgca gccgcagcgc 13800
   gaaaaaaacc tgcctccggc atttcccaac aacgggatag agagcctagt ggacaagatg 13860
   agtagatgga agacgtacgc gcaggagcac agggacgtgc caggcccgcg cccgcccacc 13920
   cgtcgtcaaa ggcacgaccg tcagcggggt ctggtgtggg aggacgatga ctcggcagac 13980
   gacagcagcg tcctggattt gggagggagt ggcaacccgt ttgcgcacct tcgccccagg 14040
   ctggggagaa tgttttaaaa aaaaaaaagc atgatgcaaa ataaaaaact caccaaggcc 14100
   atggcaccga gcgttggttt tcttgtattc cccttagtat gcggcgcgcg gcgatgtatg 14160
   aggaaggtcc tcctccctcc tacgagagtg tggtgagcgc ggcgccagtg gcggcggcgc 14220
   tgggttctcc cttcgatgct cccctggacc cgccgtttgt gcctccgcgg tacctgcggc 14280
   ctaccggggg gagaaacagc atccgttact ctgagttggc acccctattc gacaccaccc 14340
   gtgtgtacct ggtggacaac aagtcaacgg atgtggcatc cctgaactac cagaacgacc 14400
   acagcaactt tctgaccacg gtcattcaaa acaatgacta cagcccgggg gaggcaagca 14460
   cacagaccat caatcttgac gaccggtcgc actggggcgg cgacctgaaa accatcctgc 14520
   ataccaacat gccaaatgtg aacgagttca tgtttaccaa taagtttaag gcgcgggtga 14580
   tggtgtcgcg cttgcctact aaggacaatc aggtggagct gaaatacgag tgggtggagt 14640
   tcacgctgcc cgagggcaac tactccgaga ccatgaccat agaccttatg aacaacgcga 14700
   tcgtggagca ctacttgaaa gtgggcagac agaacggggt tctggaaagc gacatcgggg 14760
   taaagtttga cacccgcaac ttcagactgg ggtttgaccc cgtcactggt cttgtcatgc 14820
   ctggggtata tacaaacgaa gccttccatc cagacatcat tttgctgcca ggatgcgggg 14880
   tggacttcac ccacagccgc ctgagcaact tgttgggcat ccgcaagcgg caacccttcc 14940
   aggagggctt taggatcacc tacgatgatc tggagggtgg taacattccc gcactgttgg 15000
   atgtggacgc ctaccaggcg agcttgaaag atgacaccga acagggcggg ggtggcgcag 15060
   gcggcagcaa cagcagtggc agcggcgcgg aagagaactc caacgcggca gccgcggcaa 15120
   tgcagccggt ggaggacatg aacgatcatg ccattcgcgg cgacaccttt gccacacggg 15180
   ctgaggagaa gcgcgctgag gccgaagcag cggccgaagc tgccgccccc gctgcgcaac 15240
   ccgaggtcga gaagcctcag aagaaaccgg tgatcaaacc cctgacagag gacagcaaga 15300
   aacgcagtta caacctaata agcaatgaca gcaccttcac ccagtaccgc agctggtacc 15360
   ttgcatacaa ctacggcgac cctcagaccg gaatccgctc atggaccctg ctttgcactc 15420
   ctgacgtaac ctgcggctcg gagcaggtct actggtcgtt gccagacatg atgcaagacc 15480
   ccgtgacctt ccgctccacg cgccagatca gcaactttcc ggtggtgggc gccgagctgt 15540
   tgcccgtgca ctccaagagc ttctacaacg accaggccgt ctactcccaa ctcatccgcc 15600
   agtttacctc tctgacccac gtgttcaatc gctttcccga gaaccagatt ttggcgcgcc 15660
   cgccagcccc caccatcacc accgtcagtg aaaacgttcc tgctctcaca gatcacggga 15720
   cgctaccgct gcgcaacagc atcggaggag tccagcgagt gaccattact gacgccagac 15780
   gccgcacctg cccctacgtt tacaaggccc tgggcatagt ctcgccgcgc gtcctatcga 15840
   gccgcacttt ttgagcaagc atgtccatcc ttatatcgcc cagcaataac acaggctggg 15900
   gcctgcgctt cccaagcaag atgtttggcg gggccaagaa gcgctccgac caacacccag 15960
   tgcgcgtgcg cgggcactac cgcgcgccct ggggcgcgca caaacgcggc cgcactgggc 16020
   gcaccaccgt cgatgacgcc atcgacgcgg tggtggagga ggcgcgcaac tacacgccca 16080
   cgccgccacc agtgtccaca gtggacgcgg ccattcagac cgtggtgcgc ggagcccggc 16140
   gctatgctaa aatgaagaga cggcggaggc gcgtagcacg tcgccaccgc cgccgacccg 16200
   gcactgccgc ccaacgcgcg gcggcggccc tgcttaaccg cgcacgtcgc accggccgac 16260
   gggcggccat gcgggccgct cgaaggctgg ccgcgggtat tgtcactgtg ccccccaggt 16320
   ccaggcgacg agcggccgcc gcagcagccg cggccattag tgctatgact cagggtcgca 16380
   ggggcaacgt gtattgggtg cgcgactcgg ttagcggcct gcgcgtgccc gtgcgcaccc 16440
   gccccccgcg caactagatt gcaagaaaaa actacttaga ctcgtactgt tgtatgtatc 16500
   cagcggcggc ggcgcgcaac gaagctatgt ccaagcgcaa aatcaaagaa gagatgctcc 16560
   aggtcatcgc gccggagatc tatggccccc cgaagaagga agagcaggat tacaagcccc 16620
   gaaagctaaa gcgggtcaaa aagaaaaaga aagatgatga tgatgaactt gacgacgagg 16680
   tggaactgct gcacgctacc gcgcccaggc gacgggtaca gtggaaaggt cgacgcgtaa 16740
   aacgtgtttt gcgacccggc accaccgtag tctttacgcc cggtgagcgc tccacccgca 16800
   cctacaagcg cgtgtatgat gaggtgtacg gcgacgagga cctgcttgag caggccaacg 16860
   agcgcctcgg ggagtttgcc tacggaaagc ggcataagga catgctggcg ttgccgctgg 16920
   acgagggcaa cccaacacct agcctaaagc ccgtaacact gcagcaggtg ctgcccgcgc 16980
   ttgcaccgtc cgaagaaaag cgcggcctaa agcgcgagtc tggtgacttg gcacccaccg 17040
   tgcagctgat ggtacccaag cgccagcgac tggaagatgt cttggaaaaa atgaccgtgg 17100
   aacctgggct ggagcccgag gtccgcgtgc ggccaatcaa gcaggtggcg ccgggactgg 17160
   gcgtgcagac cgtggacgtt cagataccca ctaccagtag caccagtatt gccaccgcca 17220
   cagagggcat ggagacacaa acgtccccgg ttgcctcagc ggtggcggat gccgcggtgc 17280
   aggcggtcgc tgcggccgcg tccaagacct ctacggaggt gcaaacggac ccgtggatgt 17340
   ttcgcgtttc agccccccgg cgcccgcgcg gttcgaggaa gtacggcgcc gccagcgcgc 17400
   tactgcccga atatgcccta catccttcca ttgcgcctac ccccggctat cgtggctaca 17460
   cctaccgccc cagaagacga gcaactaccc gacgccgaac caccactgga acccgccgcc 17520
   gccgtcgccg tcgccagccc gtgctggccc cgatttccgt gcgcagggtg gctcgcgaag 17580
   gaggcaggac cctggtgctg ccaacagcgc gctaccaccc cagcatcgtt taaaagccgg 17640
   tctttgtggt tcttgcagat atggccctca cctgccgcct ccgtttcccg gtgccgggat 17700
   tccgaggaag aatgcaccgt aggaggggca tggccggcca cggcctgacg ggcggcatgc 17760
   gtcgtgcgca ccaccggcgg cggcgcgcgt cgcaccgtcg catgcgcggc ggtatcctgc 17820
   ccctccttat tccactgatc gccgcggcga ttggcgccgt gcccggaatt gcatccgtgg 17880
   ccttgcaggc gcagagacac tgattaaaaa caagttgcat gtggaaaaat caaaataaaa 17940
   agtctggact ctcacgctcg cttggtcctg taactatttt gtagaatgga agacatcaac 18000
   tttgcgtctc tggccccgcg acacggctcg cgcccgttca tgggaaactg gcaagatatc 18060
   ggcaccagca atatgagcgg tggcgccttc agctggggct cgctgtggag cggcattaaa 18120
   aatttcggtt ccaccgttaa gaactatggc agcaaggcct ggaacagcag cacaggccag 18180
   atgctgaggg ataagttgaa agagcaaaat ttccaacaaa aggtggtaga tggcctggcc 18240
   tctggcatta gcggggtggt ggacctggcc aaccaggcag tgcaaaataa gattaacagt 18300
   aagcttgatc cccgccctcc cgtagaggag cctccaccgg ccgtggagac agtgtctcca 18360
   gaggggcgtg gcgaaaagcg tccgcgcccc gacagggaag aaactctggt gacgcaaata 18420
   gacgagcctc cctcgtacga ggaggcacta aagcaaggcc tgcccaccac ccgtcccatc 18480
   gcgcccatgg ctaccggagt gctgggccag cacacacccg taacgctgga cctgcctccc 18540
   cccgccgaca cccagcagaa acctgtgctg ccaggcccga ccgccgttgt tgtaacccgt 18600
   cctagccgcg cgtccctgcg ccgcgccgcc agcggtccgc gatcgttgcg gcccgtagcc 18660
   agtggcaact ggcaaagcac actgaacagc atcgtgggtc tgggggtgca atccctgaag 18720
   cgccgacgat gcttctgaat agctaacgtg tcgtatgtgt gtcatgtatg cgtccatgtc 18780
   gccgccagag gagctgctga gccgccgcgc gcccgctttc caagatggct accccttcga 18840
   tgatgccgca gtggtcttac atgcacatct cgggccagga cgcctcggag tacctgagcc 18900
   ccgggctggt gcagtttgcc cgcgccaccg agacgtactt cagcctgaat aacaagttta 18960
   gaaaccccac ggtggcgcct acgcacgacg tgaccacaga ccggtcccag cgtttgacgc 19020
   tgcggttcat ccctgtggac cgtgaggata ctgcgtactc gtacaaggcg cggttcaccc 19080
   tagctgtggg tgataaccgt gtgctggaca tggcttccac gtactttgac atccgcggcg 19140
   tgctggacag gggccctact tttaagccct actctggcac tgcctacaac gccctggctc 19200
   ccaagggtgc cccaaatcct tgcgaatggg atgaagctgc tactgctctt gaaataaacc 19260
   tagaagaaga ggacgatgac aacgaagacg aagtagacga gcaagctgag cagcaaaaaa 19320
   ctcacgtatt tgggcaggcg ccttattctg gtataaatat tacaaaggag ggtattcaaa 19380
   taggtgtcga aggtcaaaca cctaaatatg ccgataaaac atttcaacct gaacctcaaa 19440
   taggagaatc tcagtggtac gaaactgaaa ttaatcatgc agctgggaga gtccttaaaa 19500
   agactacccc aatgaaacca tgttacggtt catatgcaaa acccacaaat gaaaatggag 19560
   ggcaaggcat tcttgtaaag caacaaaatg gaaagctaga aagtcaagtg gaaatgcaat 19620
   ttttctcaac tactgaggcg accgcaggca atggtgataa cttgactcct aaagtggtat 19680
   tgtacagtga agatgtagat atagaaaccc cagacactca tatttcttac atgcccacta 19740
   ttaaggaagg taactcacga gaactaatgg gccaacaatc tatgcccaac aggcctaatt 19800
   acattgcttt tagggacaat tttattggtc taatgtatta caacagcacg ggtaatatgg 19860
   gtgttctggc gggccaagca tcgcagttga atgctgttgt agatttgcaa gacagaaaca 19920
   cagagctttc ataccagctt ttgcttgatt ccattggtga tagaaccagg tacttttcta 19980
   tgtggaatca ggctgttgac agctatgatc cagatgttag aattattgaa aatcatggaa 20040
   ctgaagatga acttccaaat tactgctttc cactgggagg tgtgattaat acagagactc 20100
   ttaccaaggt aaaacctaaa acaggtcagg aaaatggatg ggaaaaagat gctacagaat 20160
   tttcagataa aaatgaaata agagttggaa ataattttgc catggaaatc aatctaaatg 20220
   ccaacctgtg gagaaatttc ctgtactcca acatagcgct gtatttgccc gacaagctaa 20280
   agtacagtcc ttccaacgta aaaatttctg ataacccaaa cacctacgac tacatgaaca 20340
   agcgagtggt ggctcccggg ttagtggact gctacattaa ccttggagca cgctggtccc 20400
   ttgactatat ggacaacgtc aacccattta accaccaccg caatgctggc ctgcgctacc 20460
   gctcaatgtt gctgggcaat ggtcgctatg tgcccttcca catccaggtg cctcagaagt 20520
   tctttgccat taaaaacctc cttctcctgc cgggctcata cacctacgag tggaacttca 20580
   ggaaggatgt taacatggtt ctgcagagct ccctaggaaa tgacctaagg gttgacggag 20640
   ccagcattaa gtttgatagc atttgccttt acgccacctt cttccccatg gcccacaaca 20700
   ccgcctccac gcttgaggcc atgcttagaa acgacaccaa cgaccagtcc tttaacgact 20760
   atctctccgc cgccaacatg ctctacccta tacccgccaa cgctaccaac gtgcccatat 20820
   ccatcccctc ccgcaactgg gcggctttcc gcggctgggc cttcacgcgc cttaagacta 20880
   aggaaacccc atcactgggc tcgggctacg acccttatta cacctactct ggctctatac 20940
   cctacctaga tggaaccttt tacctcaacc acacctttaa gaaggtggcc attacctttg 21000
   actcttctgt cagctggcct ggcaatgacc gcctgcttac ccccaacgag tttgaaatta 21060
   agcgctcagt tgacggggag ggttacaacg ttgcccagtg taacatgacc aaagactggt 21120
   tcctggtaca aatgctagct aactacaaca ttggctacca gggcttctat atcccagaga 21180
   gctacaagga ccgcatgtac tccttcttta gaaacttcca gcccatgagc cgtcaggtgg 21240
   tggatgatac taaatacaag gactaccaac aggtgggcat cctacaccaa cacaacaact 21300
   ctggatttgt tggctacctt gcccccacca tgcgcgaagg acaggcctac cctgctaact 21360
   tcccctatcc gcttataggc aagaccgcag ttgacagcat tacccagaaa aagtttcttt 21420
   gcgatcgcac cctttggcgc atcccattct ccagtaactt tatgtccatg ggcgcactca 21480
   cagacctggg ccaaaacctt ctctacgcca actccgccca cgcgctagac atgacttttg 21540
   aggtggatcc catggacgag cccacccttc tttatgtttt gtttgaagtc tttgacgtgg 21600
   tccgtgtgca ccggccgcac cgcggcgtca tcgaaaccgt gtacctgcgc acgcccttct 21660
   cggccggcaa cgccacaaca taaagaagca agcaacatca acaacagctg ccgccatggg 21720
   ctccagtgag caggaactga aagccattgt caaagatctt ggttgtgggc catatttttt 21780
   gggcacctat gacaagcgct ttccaggctt tgtttctcca cacaagctcg cctgcgccat 21840
   agtcaatacg gccggtcgcg agactggggg cgtacactgg atggcctttg cctggaaccc 21900
   gcactcaaaa acatgctacc tctttgagcc ctttggcttt tctgaccagc gactcaagca 21960
   ggtttaccag tttgagtacg agtcactcct gcgccgtagc gccattgctt cttcccccga 22020
   ccgctgtata acgctggaaa agtccaccca aagcgtacag gggcccaact cggccgcctg 22080
   tggactattc tgctgcatgt ttctccacgc ctttgccaac tggccccaaa ctcccatgga 22140
   tcacaacccc accatgaacc ttattaccgg ggtacccaac tccatgctca acagtcccca 22200
   ggtacagccc accctgcgtc gcaaccagga acagctctac agcttcctgg agcgccactc 22260
   gccctacttc cgcagccaca gtgcgcagat taggagcgcc acttcttttt gtcacttgaa 22320
   aaacatgtaa aaataatgta ctagagacac tttcaataaa ggcaaatgct tttatttgta 22380
   cactctcggg tgattattta cccccaccct tgccgtctgc gccgtttaaa aatcaaaggg 22440
   gttctgccgc gcatcgctat gcgccactgg cagggacacg ttgcgatact ggtgtttagt 22500
   gctccactta aactcaggca caaccatccg cggcagctcg gtgaagtttt cactccacag 22560
   gctgcgcacc atcaccaacg cgtttagcag gtcgggcgcc gatatcttga agtcgcagtt 22620
   ggggcctccg ccctgcgcgc gcgagttgcg atacacaggg ttgcagcact ggaacactat 22680
   cagcgccggg tggtgcacgc tggccagcac gctcttgtcg gagatcagat ccgcgtccag 22740
   gtcctccgcg ttgctcaggg cgaacggagt caactttggt agctgccttc ccaaaaaggg 22800
   cgcgtgccca ggctttgagt tgcactcgca ccgtagtggc atcaaaaggt gaccgtgccc 22860
   ggtctgggcg ttaggataca gcgcctgcat aaaagccttg atctgcttaa aagccacctg 22920
   agcctttgcg ccttcagaga agaacatgcc gcaagacttg ccggaaaact gattggccgg 22980
   acaggccgcg tcgtgcacgc agcaccttgc gtcggtgttg gagatctgca ccacatttcg 23040
   gccccaccgg ttcttcacga tcttggcctt gctagactgc tccttcagcg cgcgctgccc 23100
   gttttcgctc gtcacatcca tttcaatcac gtgctcctta tttatcataa tgcttccgtg 23160
   tagacactta agctcgcctt cgatctcagc gcagcggtgc agccacaacg cgcagcccgt 23220
   gggctcgtga tgcttgtagg tcacctctgc aaacgactgc aggtacgcct gcaggaatcg 23280
   ccccatcatc gtcacaaagg tcttgttgct ggtgaaggtc agctgcaacc cgcggtgctc 23340
   ctcgttcagc caggtcttgc atacggccgc cagagcttcc acttggtcag gcagtagttt 23400
   gaagttcgcc tttagatcgt tatccacgtg gtacttgtcc atcagcgcgc gcgcagcctc 23460
   catgcccttc tcccacgcag acacgatcgg cacactcagc gggttcatca ccgtaatttc 23520
   actttccgct tcgctgggct cttcctcttc ctcttgcgtc cgcataccac gcgccactgg 23580
   gtcgtcttca ttcagccgcc gcactgtgcg cttacctcct ttgccatgct tgattagcac 23640
   cggtgggttg ctgaaaccca ccatttgtag cgccacatct tctctttctt cctcgctgtc 23700
   cacgattacc tctggtgatg gcgggcgctc gggcttggga gaagggcgct tctttttctt 23760
   cttgggcgca atggccaaat ccgccgccga ggtcgatggc cgcgggctgg gtgtgcgcgg 23820
   caccagcgcg tcttgtgatg agtcttcctc gtcctcggac tcgatacgcc gcctcatccg 23880
   cttttttggg ggcgcccggg gaggcggcgg cgacggggac ggggacgaca cgtcctccat 23940
   ggttggggga cgtcgcgccg caccgcgtcc gcgctcgggg gtggtttcgc gctgctcctc 24000
   ttcccgactg gccatttcct tctcctatag gcagaaaaag atcatggagt cagtcgagaa 24060
   gaaggacagc ctaaccgccc cctctgagtt cgccaccacc gcctccaccg atgccgccaa 24120
   cgcgcctacc accttccccg tcgaggcacc cccgcttgag gaggaggaag tgattatcga 24180
   gcaggaccca ggttttgtaa gcgaagacga cgaggaccgc tcagtaccaa cagaggataa 24240
   aaagcaagac caggacaacg cagaggcaaa cgaggaacaa gtcgggcggg gggacgaaag 24300
   gcatggcgac tacctagatg tgggagacga cgtgctgttg aagcatctgc agcgccagtg 24360
   cgccattatc tgcgacgcgt tgcaagagcg cagcgatgtg cccctcgcca tagcggatgt 24420
   cagccttgcc tacgaacgcc acctattctc accgcgcgta ccccccaaac gccaagaaaa 24480
   cggcacatgc gagcccaacc cgcgcctcaa cttctacccc gtatttgccg tgccagaggt 24540
   gcttgccacc tatcacatct ttttccaaaa ctgcaagata cccctatcct gccgtgccaa 24600
   ccgcagccga gcggacaagc agctggcctt gcggcagggc gctgtcatac ctgatatcgc 24660
   ctcgctcaac gaagtgccaa aaatctttga gggtcttgga cgcgacgaga agcgcgcggc 24720
   aaacgctctg caacaggaaa acagcgaaaa tgaaagtcac tctggagtgt tggtggaact 24780
   cgagggtgac aacgcgcgcc tagccgtact aaaacgcagc atcgaggtca cccactttgc 24840
   ctacccggca cttaacctac cccccaaggt catgagcaca gtcatgagtg agctgatcgt 24900
   gcgccgtgcg cagcccctgg agagggatgc aaatttgcaa gaacaaacag aggagggcct 24960
   acccgcagtt ggcgacgagc agctagcgcg ctggcttcaa acgcgcgagc ctgccgactt 25020
   ggaggagcga cgcaaactaa tgatggccgc agtgctcgtt accgtggagc ttgagtgcat 25080
   gcagcggttc tttgctgacc cggagatgca gcgcaagcta gaggaaacat tgcactacac 25140
   ctttcgacag ggctacgtac gccaggcctg caagatctcc aacgtggagc tctgcaacct 25200
   ggtctcctac cttggaattt tgcacgaaaa ccgccttggg caaaacgtgc ttcattccac 25260
   gctcaagggc gaggcgcgcc gcgactacgt ccgcgactgc gtttacttat ttctatgcta 25320
   cacctggcag acggccatgg gcgtttggca gcagtgcttg gaggagtgca acctcaagga 25380
   gctgcagaaa ctgctaaagc aaaacttgaa ggacctatgg acggccttca acgagcgctc 25440
   cgtggccgcg cacctggcgg acatcatttt ccccgaacgc ctgcttaaaa ccctgcaaca 25500
   gggtctgcca gacttcacca gtcaaagcat gttgcagaac tttaggaact ttatcctaga 25560
   gcgctcagga atcttgcccg ccacctgctg tgcacttcct agcgactttg tgcccattaa 25620
   gtaccgcgaa tgccctccgc cgctttgggg ccactgctac cttctgcagc tagccaacta 25680
   ccttgcctac cactctgaca taatggaaga cgtgagcggt gacggtctac tggagtgtca 25740
   ctgtcgctgc aacctatgca ccccgcaccg ctccctggtt tgcaattcgc agctgcttaa 25800
   cgaaagtcaa attatcggta cctttgagct gcagggtccc tcgcctgacg aaaagtccgc 25860
   ggctccgggg ttgaaactca ctccggggct gtggacgtcg gcttaccttc gcaaatttgt 25920
   acctgaggac taccacgccc acgagattag gttctacgaa gaccaatccc gcccgccaaa 25980
   tgcggagctt accgcctgcg tcattaccca gggccacatt cttggccaat tgcaagccat 26040
   caacaaagcc cgccaagagt ttctgctacg aaagggacgg ggggtttact tggaccccca 26100
   gtccggcgag gagctcaacc caatcccccc gccgccgcag ccctatcagc agcagccgcg 26160
   ggcccttgct tcccaggatg gcacccaaaa agaagctgca gctgccgccg ccacccacgg 26220
   acgaggagga atactgggac agtcaggcag aggaggtttt ggacgaggag gaggaggaca 26280
   tgatggaaga ctgggagagc ctagacgagg aagcttccga ggtcgaagag gtgtcagacg 26340
   aaacaccgtc accctcggtc gcattcccct cgccggcgcc ccagaaatcg gcaaccggtt 26400
   ccagcatggc tacaacctcc gctcctcagg cgccgccggc actgcccgtt cgccgaccca 26460
   accgtagatg ggacaccact ggaaccaggg ccggtaagtc caagcagccg ccgccgttag 26520
   cccaagagca acaacagcgc caaggctacc gctcatggcg cgggcacaag aacgccatag 26580
   ttgcttgctt gcaagactgt gggggcaaca tctccttcgc ccgccgcttt cttctctacc 26640
   atcacggcgt ggccttcccc cgtaacatcc tgcattacta ccgtcatctc tacagcccat 26700
   actgcaccgg cggcagcggc agcggcagca acagcagcgg ccacacagaa gcaaaggcga 26760
   ccggatagca agactctgac aaagcccaag aaatccacag cggcggcagc agcaggagga 26820
   ggagcgctgc gtctggcgcc caacgaaccc gtatcgaccc gcgagcttag aaacaggatt 26880
   tttcccactc tgtatgctat atttcaacag agcaggggcc aagaacaaga gctgaaaata 26940
   aaaaacaggt ctctgcgatc cctcacccgc agctgcctgt atcacaaaag cgaagatcag 27000
   cttcggcgca cgctggaaga cgcggaggct ctcttcagta aatactgcgc gctgactctt 27060
   aaggactagt ttcgcgccct ttctcaaatt taagcgcgaa aactacgtca tctccagcgg 27120
   ccacacccgg cgccagcacc tgtcgtcagc gccattatga gcaaggaaat tcccacgccc 27180
   tacatgtgga gttaccagcc acaaatggga cttgcggctg gagctgccca agactactca 27240
   acccgaataa actacatgag cgcgggaccc cacatgatat cccgggtcaa cggaatccgc 27300
   gcccaccgaa accgaattct cttggaacag gcggctatta ccaccacacc tcgtaataac 27360
   cttaatcccc gtagttggcc cgctgccctg gtgtaccagg aaagtcccgc tcccaccact 27420
   gtggtacttc ccagagacgc ccaggccgaa gttcagatga ctaactcagg ggcgcagctt 27480
   gcgggcggct ttcgtcacag ggtgcggtcg cccgggcagg gtataactca cctgacaatc 27540
   agagggcgag gtattcagct caacgacgag tcggtgagct cctcgcttgg tctccgtccg 27600
   gacgggacat ttcagatcgg cggcgccggc cgtccttcat tcacgcctcg tcaggcaatc 27660
   ctaactctgc agacctcgtc ctctgagccg cgctctggag gcattggaac tctgcaattt 27720
   attgaggagt ttgtgccatc ggtctacttt aaccccttct cgggacctcc cggccactat 27780
   ccggatcaat ttattcctaa ctttgacgcg gtaaaggact cggcggacgg ctacgactga 27840
   atgttaagtg gagaggcaga gcaactgcgc ctgaaacacc tggtccactg tcgccgccac 27900
   aagtgctttg cccgcgactc cggtgagttt tgctactttg aattgcccga ggatcatatc 27960
   gagggcccgg cgcacggcgt ccggcttacc gcccagggag agcttgcccg tagcctgatt 28020
   cgggagttta cccagcgccc cctgctagtt gagcggacaa ggggaccctg tgttctcact 28080
   gtgatttgca actgtcctaa ccttggatta catcaagatc ctctagttat aactagagta 28140
   cccggggatc ttattccctt taactaataa aaaaaaataa taaagcatca cttacttaaa 28200
   atcagttagc aaatttctgt ccagtttatt cagcagcacc tccttgccct cctcccagct 28260
   ctggtattgc agcttcctcc tggctgcaaa ctttctccac aatctaaatg gaatgtcagt 28320
   ttcctcctgt tcctgtccat ccgcacccac tatcttcatg ttgttgcaga tgaagcgcgc 28380
   aagaccgtct gaagatacct tcaaccccgt gtatccatat gacacggaaa ccggtcctcc 28440
   aactgtgcct tttcttactc ctccctttgt atcccccaat gggtttcaag agagtccccc 28500
   tggggtactc tctttgcgcc tatccgaacc tctagttacc tccaatggca tgcttgcgct 28560
   caaaatgggc aacggcctct ctctggacga ggccggcaac cttacctccc aaaatgtaac 28620
   cactgtgagc ccacctctca aaaaaaccaa gtcaaacata aacctggaaa tatctgcacc 28680
   cctcacagtt acctcagaag ccctaactgt ggctgccgcc gcacctctaa tggtcgcggg 28740
   caacacactc accatgcaat cacaggcccc gctaaccgtg cacgactcca aacttagcat 28800
   tgccacccaa ggacccctca cagtgtcaga aggaaagcta gccctgcaaa catcaggccc 28860
   cctcaccacc accgatagca gtacccttac tatcactgcc tcaccccctc taactactgc 28920
   cactggtagc ttgggcattg acttgaaaga gcccatttat acacaaaatg gaaaactagg 28980
   actaaagtac ggggctcctt tgcatgtaac agacgaccta aacactttga ccgtagcaac 29040
   tggtccaggt gtgactatta ataatacttc cttgcaaact aaagttactg gagccttggg 29100
   ttttgattca caaggcaata tgcaacttaa tgtagcagga ggactaagga ttgattctca 29160
   aaacagacgc cttatacttg atgttagtta tccgtttgat gctcaaaacc aactaaatct 29220
   aagactagga cagggccctc tttttataaa ctcagcccac aacttggata ttaactacaa 29280
   caaaggcctt tacttgttta cagcttcaaa caattccaaa aagcttgagg ttaacctaag 29340
   cactgccaag gggttgatgt ttgacgctac agccatagcc attaatgcag gagatgggct 29400
   tgaatttggt tcacctaatg caccaaacac aaatcccctc aaaacaaaaa ttggccatgg 29460
   cctagaattt gattcaaaca aggctatggt tcctaaacta ggaactggcc ttagttttga 29520
   cagcacaggt gccattacag taggaaacaa aaataatgat aagctaactt tgtggaccac 29580
   accagctcca tctcctaact gtagactaaa tgcagagaaa gatgctaaac tcactttggt 29640
   cttaacaaaa tgtggcagtc aaatacttgc tacagtttca gttttggctg ttaaaggcag 29700
   tttggctcca atatctggaa cagttcaaag tgctcatctt attataagat ttgacgaaaa 29760
   tggagtgcta ctaaacaatt ccttcctgga cccagaatat tggaacttta gaaatggaga 29820
   tcttactgaa ggcacagcct atacaaacgc tgttggattt atgcctaacc tatcagctta 29880
   tccaaaatct cacggtaaaa ctgccaaaag taacattgtc agtcaagttt acttaaacgg 29940
   agacaaaact aaacctgtaa cactaaccat tacactaaac ggtacacagg aaacaggaga 30000
   cacaactcca agtgcatact ctatgtcatt ttcatgggac tggtctggcc acaactacat 30060
   taatgaaata tttgccacat cctcttacac tttttcatac attgcccaag aataaagaat 30120
   cgtttgtgtt atgtttcaac gtgtttattt ttcaattgca gaaaatttca agtcattttt 30180
   cattcagtag tatagcccca ccaccacata gcttatacag atcaccgtac cttaatcaaa 30240
   ctcacagaac cctagtattc aacctgccac ctccctccca acacacagag tacacagtcc 30300
   tttctccccg gctggcctta aaaagcatca tatcatgggt aacagacata ttcttaggtg 30360
   ttatattcca cacggtttcc tgtcgagcca aacgctcatc aagtgatatt aataaactcc 30420
   ccgggcagct cacttaagtt catgtcgctg tccagctgct gagccacagg ctgctgtcca 30480
   acttgcggtt gcttaacggg cggcgaagga gaagtccacg cctacatggg gggagagtca 30540
   taatcgtgca tcaggatagg gcggtggtgc tgcagcagcg cgcgaataaa ctgctgccgc 30600
   cgccgctccg tcctgcagga atacaacatg gcagtggtct cctcagcgat gattcgcacc 30660
   gcccgcagca taaggcgctt gtcctccggg cacagcagcg caccctgatc tcacttaaat 30720
   cagcacagta actgcagcac agcaccacaa tattgttcaa aatcccacag tgcaaggcgc 30780
   tgtatccaaa gctcatggcg gggaccacag aacccacgtg gccatcatac cacaagcgca 30840
   ggtagattaa gtggcgaccc ctcataaaca cgctggacat aaacattacc tcttttggca 30900
   tgttgtaatt caccacctcc cggtaccata taaacctctg attaaacatg gcgccatcca 30960
   ccaccatcct aaaccagctg gccaaaacct gccccgccgg gntatacact gcagggaacc 31020
   gggacttgga caatgacaag tgggagagcc caggactcgt aaccatggat catcatgctc 31080
   gtcatgatat caatgttggc acaacacagg cacacgtgca tacacttcct caggattaca 31140
   agctcctccc gcgttagaac catatcccag ggaacaaccc attcctgaat cagcgtaaat 31200
   cccacactgc agggaagacc tcgcacgtaa ctcacgttgt gcattgtcaa agtgttacat 31260
   tcgggcagca gcggatgatc ctccagtatg gtagcgcggg tttctgtctc aaaaggaggt 31320
   agacgatccc tactgtacgg agtgcgccga gacaaccgag atcgtgttgg tcgtagtgtc 31380
   atgccaaatg gaacgccgga cgtagtcata tttcctgaag caaaaccagg tgcgggcgtg 31440
   acaaacagat ctgcgtctcc ggtctcgccg cttagatcgc tctgtgtagt agttgtagta 31500
   tatccactct ctcaaagcat ccaggcgccc cctggcttcg ggttctatgt aaactccttc 31560
   atgcgccgct gccctgataa catccaccac cgcagaataa gccacaccca gccaacctac 31620
   acattcgttc tgcgagtcac acacgggagg agcgggaaga gctggaagaa ccatgttttt 31680
   ttttttattc caaaagatta tccaaaacct caaaatgaag atctattaag tgaacgcgct 31740
   cccctccggt ggcgtggtca aactctacag ccaaagaaca gataatggca tttgtaagat 31800
   gttgcacaat ggcttccaaa aggcaaacgg ccctcacgtc caagtggacg taaaggctaa 31860
   acccttcagg gtgaatctcc tctataaaca ttccagcacc ttcaaccatg cccaaataat 31920
   tctcatctcg ccaccttctc aatatatctc taagcaaatc ccgaatattt aagtccgggc 31980
   cattgtaaaa aatttggctc cagagcgccc tccaccttca gcctcaagca gcgaatcatg 32040
   attgcaaaaa ttcaggttcc tcacagacct gtataagatt caaaagcgga acattaacaa 32100
   aaataccgcg atcccgtagg tcccttcgca gggccagctg aacataatcg tgcaggtctg 32160
   cacggaccag cgcggccact tccccgccag gaaccatgac aaaagaaccc acactgatta 32220
   tgacacgcat actcggagct atgctaacca gcgtagcccc gatgtaagct tgttgcatgg 32280
   gcggcgatat aaaatgcaag gtgctgctca aaaaatcagg caaagcctcg cgcaaaaaag 32340
   aaagcacatc gtagtcatgc tcatgcagat aaaggcaggt aagctccgga accaccacag 32400
   aaaaagacac catttttctc tcaaacatgt ctgcgggttt ctgcataaac acaaaataaa 32460
   ataacaaaaa aacatttaaa cattagaagc ctgtcttaca acaggaaaaa caacccttat 32520
   aagcataaga cggactacgg ccatgccggc gtgaccgtaa aaaaactggt caccgtgatt 32580
   aaaaagcacc accgacagct cctcggtcag tccggagtca taatgtaaga ctcggtaaac 32640
   acatcaggtt gattcacatc ggtcagtgtt aaaaagcgac cgaaatagcc cgggggaata 32700
   catacccgca ggcgtagaga caacattaca gcccccatag gaggtataac aaaattaata 32760
   ggagagaaaa acacataaac acctgaaaaa ccctcctgcc taggcaaaat agcaccctcc 32820
   cgctccagaa caacatacag cgcttcacag cggcagccta acagtcagcc ttaccagtaa 32880
   aaaagaaaac ctattaaaaa aacaccactc gacacggcac cagctcaatc agtcacagtg 32940
   taaaaaaggg ccaagtgcag agcgagtata tataggacta aaaaatgacg taacggttaa 33000
   agtccacaaa aaacacccag aaaaccgcac gcgaacctac gcccagaaac gaaagccaaa 33060
   aaacccacaa cttcctcaaa tcgtcacttc cgttttccca cgttacgtaa cttcccattt 33120
   taagaaaact acaattccca acacatacaa gttactccgc cctaaaacct acgtcacccg 33180
   ccccgttccc acgccccgcg ccacgtcaca aactccaccc cctcattatc atattggctt 33240
   caatccaaaa taaggtatat tattgatgat gnnttaatta agggcggccg cacgggccat 33300
   cgatggatcc gcggaagctt ctcgacccat tctcatgttt gacagcttat catcgaattt 33360
   ctgccattca tccgcttatt atcacttatt caggcgtagc aaccaggcgt ttaagggcac 33420
   caataactgc cttaaaaaaa ttacgccccg ccctgccact catcgcagta ctgttgtaat 33480
   tcattaagca ttctgccgac atggaagcca tcacagacgg catgatgaac ctgaatcgcc 33540
   agcggcatca gcaccttgtc gccttgcgta taatatttgc ccatggtgaa aacgggggcg 33600
   aagaagttgt ccatattggc cacgtttaaa tcaaaactgg tgaaactcac ccagggattg 33660
   gctgagacga aaaacatatt ctcaataaac cctttaggga aataggccag gttttcaccg 33720
   taacacgcca catcttgcga atatatgtgt agaaactgcc ggaaatcgtc gtggtattca 33780
   ctccagagcg atgaaaacgt ttcagtttgc tcatggaaaa cggtgtaaca agggtgaaca 33840
   ctatcccata tcaccagctc accgtctttc attgccatac ggaattccgg atgagcattc 33900
   atcaggcggg caagaatgtg aataaaggcc ggataaaact tgtgcttatt tttctttacg 33960
   gtctttaaaa aggccgtaat atccagctga acggtctggt tataggtaca ttgagcaact 34020
   gactgaaatg cctcaaaatg ttctttacga tgccattggg atatatcaac ggtggtatat 34080
   ccagtgattt ttttctccat tttagcttcc ttagctcctg aaaatctcga taactcaaaa 34140
   aatacgcccg gtagtgatct tatttcatta tggtgaaagt tggaacctct tacgtgccga 34200
   tcaacgtctc attttcgcca aaagttggcc cagggcttcc cggtatcaac agggacacca 34260
   ggatttattt attctgcgaa gtgatcttcc gtcacaggta tttattcgcg ataagctcat 34320
   ggagcggcgt aaccgtcgca caggaaggac agagaaagcg cggatctggg aagtgacgga 34380
   cagaacggtc aggacctgga ttggggaggc ggttgccgcc gctgctgctg acggtgtgac 34440
   gttctctgtt ccggtcacac cacatacgtt ccgccattcc tatgcgatgc acatgctgta 34500
   tgccggtata ccgctgaaag ttctgcaaag cctgatggga cataagtcca tcagttcaac 34560
   ggaagtctac acgaaggttt ttgcgctgga tgtggctgcc cggcaccggg tgcagtttgc 34620
   gatgccggag tctgatgcgg ttgcgatgct gaaacaatta tcctgagaat aaatgccttg 34680
   gcctttatat ggaaatgtgg aactgagtgg atatgctgtt tttgtctgtt aaacagagaa 34740
   gctggctgtt atccactgag aagcgaacga aacagtcggg aaaatctccc attatcgtag 34800
   agatccgcat tattaatctc aggagcctgt gtagcgttta taggaagtag tgttctgtca 34860
   tgatgcctgc aagcggtaac gaaaacgatt tgaatatgcc ttcaggaaca atagaaatct 34920
   tcgtgcggtg ttacgttgaa gtggagcgga ttatgtcagc aatggacaga acaacctaat 34980
   gaacacagaa ccatgatgtg gtctgtcctt ttacagccag taggctcgcc gcagtcgagc 35040
   gacggcgaag ccctcgagtg agcgaggaag caccagggaa cagcacttat atattctgct 35100
   tacacacgat gcctgaaaaa acttcccttg gggttatcca cttatccacg gggatatttt 35160
   tataattatt ttttttatag tttttagatc ttctttttta gagcgccttg taggccttta 35220
   tccatgctgg ttctagagaa ggtgttgtga caaattgccc tttcagtgtg acaaatcacc 35280
   ctcaaatgac agtcctgtct gtgacaaatt gcccttaacc ctgtgacaaa ttgccctcag 35340
   aagaagctgt tttttcacaa agttatccct gcttattgac tcttttttat ttagtgtgac 35400
   aatctaaaaa cttgtcacac ttcacatgga tctgtcatgg cggaaacagc ggttatcaat 35460
   cacaagaaac gtaaaaatag cccgcgaatc gtccagtcaa acgacctcac tgaggcggca 35520
   tatagtctct cccgggatca aaaacgtatg ctgtatctgt tcgttgacca gatcagaaaa 35580
   tctgatggca ccctacagga acatgacggt atctgcgaga tccatgttgc taaatatgct 35640
   gaaatattcg gattgacctc tgcggaagcc agtaaggata tacggcaggc attgaagagt 35700
   ttcgcgggga aggaagtggt tttttatcgc cctgaagagg atgccggcga tgaaaaaggc 35760
   tatgaatctt ttccttggtt tatcaaacgt gcgcacagtc catccagagg gctttacagt 35820
   gtacatatca acccatatct cattcccttc tttatcgggt tacagaaccg gtttacgcag 35880
   tttcggctta gtgaaacaaa agaaatcacc aatccgtatg ccatgcgttt atacgaatcc 35940
   ctgtgtcagt atcgtaagcc ggatggctca ggcatcgtct ctctgaaaat cgactggatc 36000
   atagagcgtt accagctgcc tcaaagttac cagcgtatgc ctgacttccg ccgccgcttc 36060
   ctgcaggtct gtgttaatga gatcaacagc agaactccaa tgcgcctctc atacattgag 36120
   aaaaagaaag gccgccagac gactcatatc gtattttcct tccgcgatat cacttccatg 36180
   acgacaggat agtctgaggg ttatctgtca cagatttgag ggtggttcgt cacatttgtt 36240
   ctgacctact gagggtaatt tgtcacagtt ttgctgtttc cttcagcctg catggatttt 36300
   ctcatacttt ttgaactgta atttttaagg aagccaaatt tgagggcagt ttgtcacagt 36360
   tgatttcctt ctctttccct tcgtcatgtg acctgatatc gggggttagt tcgtcatcat 36420
   tgatgagggt tgattatcac agtttattac tctgaattgg ctatccgcgt gtgtacctct 36480
   acctggagtt tttcccacgg tggatatttc ttcttgcgct gagcgtaaga gctatctgac 36540
   agaacagttc ttctttgctt cctcgccagt tcgctcgcta tgctcggtta cacggctgcg 36600
   gcgagcgcta gtgataataa gtgactgagg tatgtgctct tcttatctcc ttttgtagtg 36660
   ttgctcttat tttaaacaac tttgcggttt tttgatgact ttgcgatttt gttgttgctt 36720
   tgcagtaaat tgcaagattt aataaaaaaa cgcaaagcaa tgattaaagg atgttcagaa 36780
   tgaaactcat ggaaacactt aaccagtgca taaacgctgg tcatgaaatg acgaaggcta 36840
   tcgccattgc acagtttaat gatgacagcc cggaagcgag gaaaataacc cggcgctgga 36900
   gaataggtga agcagcggat ttagttgggg tttcttctca ggctatcaga gatgccgaga 36960
   aagcagggcg actaccgcac ccggatatgg aaattcgagg acgggttgag caacgtgttg 37020
   gttatacaat tgaacaaatt aatcatatgc gtgatgtgtt tggtacgcga ttgcgacgtg 37080
   ctgaagacgt atttccaccg gtgatcgggg ttgctgccca taaaggtggc gtttacaaaa 37140
   cctcagtttc tgttcatctt gctcaggatc tggctctgaa ggggctacgt gttttgctcg 37200
   tggaaggtaa cgacccccag ggaacagcct caatgtatca cggatgggta ccagatcttc 37260
   atattcatgc agaagacact ctcctgcctt tctatcttgg ggaaaaggac gatgtcactt 37320
   atgcaataaa gcccacttgc tggccggggc ttgacattat tccttcctgt ctggctctgc 37380
   accgtattga aactgagtta atgggcaaat ttgatgaagg taaactgccc accgatccac 37440
   acctgatgct ccgactggcc attgaaactg ttgctcatga ctatgatgtc atagttattg 37500
   acagcgcgcc taacctgggt atcggcacga ttaatgtcgt atgtgctgct gatgtgctga 37560
   ttgttcccac gcctgctgag ttgtttgact acacctccgc actgcagttt ttcgatatgc 37620
   ttcgtgatct gctcaagaac gttgatctta aagggttcga gcctgatgta cgtattttgc 37680
   ttaccaaata cagcaatagt aatggctctc agtccccgtg gatggaggag caaattcggg 37740
   atgcctgggg aagcatggtt ctaaaaaatg ttgtacgtga aacggatgaa gttggtaaag 37800
   gtcagatccg gatgagaact gtttttgaac aggccattga tcaacgctct tcaactggtg 37860
   cctggagaaa tgctctttct atttgggaac ctgtctgcaa tgaaattttc gatcgtctga 37920
   ttaaaccacg ctgggagatt agataatgaa gcgtgcgcct gttattccaa aacatacgct 37980
   caatactcaa ccggttgaag atacttcgtt atcgacacca gctgccccga tggtggattc 38040
   gttaattgcg cgcgtaggag taatggctcg cggtaatgcc attactttgc ctgtatgtgg 38100
   tcgggatgtg aagtttactc ttgaagtgct ccggggtgat agtgttgaga agacctctcg 38160
   ggtatggtca ggtaatgaac gtgaccagga gctgcttact gaggacgcac tggatgatct 38220
   catcccttct tttctactga ctggtcaaca gacaccggcg ttcggtcgaa gagtatctgg 38280
   tgtcatagaa attgccgatg ggagtcgccg tcgtaaagct gctgcactta ccgaaagtga 38340
   ttatcgtgtt ctggttggcg agctggatga tgagcagatg gctgcattat ccagattggg 38400
   taacgattat cgcccaacaa gtgcttatga acgtggtcag cgttatgcaa gccgattgca 38460
   gaatgaattt gctggaaata tttctgcgct ggctgatgcg gaaaatattt cacgtaagat 38520
   tattacccgc tgtatcaaca ccgccaaatt gcctaaatca gttgttgctc ttttttctca 38580
   ccccggtgaa ctatctgccc ggtcaggtga tgcacttcaa aaagccttta cagataaaga 38640
   ggaattactt aagcagcagg catctaacct tcatgagcag aaaaaagctg gggtgatatt 38700
   tgaagctgaa gaagttatca ctcttttaac ttctgtgctt aaaacgtcat ctgcatcaag 38760
   aactagttta agctcacgac atcagtttgc tcctggagcg acagtattgt ataagggcga 38820
   taaaatggtg cttaacctgg acaggtctcg tgttccaact gagtgtatag agaaaattga 38880
   ggccattctt aaggaacttg aaaagccagc accctgatgc gaccacgttt tagtctacgt 38940
   ttatctgtct ttacttaatg tcctttgtta caggccagaa agcataactg gcctgaatat 39000
   tctctctggg cccactgttc cacttgtatc gtcggtctga taatcagact gggaccacgg 39060
   tcccactcgt atcgtcggtc tgattattag tctgggacca cggtcccact cgtatcgtcg 39120
   gtctgattat tagtctggga ccacggtccc actcgtatcg tcggtctgat aatcagactg 39180
   ggaccacggt cccactcgta tcgtcggtct gattattagt ctgggaccat ggtcccactc 39240
   gtatcgtcgg tctgattatt agtctgggac cacggtccca ctcgtatcgt cggtctgatt 39300
   attagtctgg aaccacggtc ccactcgtat cgtcggtctg attattagtc tgggaccacg 39360
   gtcccactcg tatcgtcggt ctgattatta gtctgggacc acgatcccac tcgtgttgtc 39420
   ggtctgatta tcggtctggg accacggtcc cacttgtatt gtcgatcaga ctatcagcgt 39480
   gagactacga ttccatcaat gcctgtcaag ggcaagtatt gacatgtcgt cgtaacctgt 39540
   agaacggagt aacctcggtg tgcggttgta tgcctgctgt ggattgctgc tgtgtcctgc 39600
   ttatccacaa cattttgcgc acggttatgt ggacaaaata cctggttacc caggccgtgc 39660
   cggcacgtta accgggctgc atccgatgca agtgtgtcgc tgtcgagttt aaacatgcat 39720
   ccttaattaa 39730

<210> 5
<211> 42780
<212> DNA
<213> Artificial

<220>
<223> synthetic


<220>
<221> misc_feature
<222> (34052)..(34052)
<223> n is a, c, g, or t

<220>
<221> misc_feature
<222> (36322)..(36323)
<223> n is a, c, g, or t

<400> 5
catcatcaat aatatacctt attttggatt gaagccaata tgataatgag ggggtggagt 60

ttgtgacgtg gcgcggggcg tgggaacggg gcgggtgacg tagtagtgtg gcggaagtgt 120

gatgttgcaa gtgtggcgga acacatgtaa gcgacggatg tggcaaaagt gacgtttttg 180

gtgtgcgccg gtgtacacag gaagtgacaa ttttcgcgcg gttttaggcg gatgttgtag 240

taaatttggg cgtaaccgag taagatttgg ccattttcgc gggaaaactg aataagagga 300

agtgaaatct gaataatttt gtgttactca tagcgcgtaa actggtaccc ggggatcttg 360

aagttcctat tccgaagttc ctattctcta gaaagtatag gaacttcctt aagtgagtcg 420

tattacggac tggccgtcgt tttacaacgt cgtgactggg aaaaccctgg cgttacccaa 480

cttaatcgcc ttgcagcaca tccccctttc gccagctggc gtaatagcga agaggcccgc 540

accgatcgcc cttcccaaca gttgcgcagc ctgaatggcg aatggcgctt cgcttggtaa 600

gctagctaag gcttaaggag gacaatcatg gcaacagtta accagctggt acgcaaacca 660

cgtgctcgca aagttgcgaa aagcaacgtg cctgcgctgg aagcatgccc gcaaaaacgt 720

ggcgtatgta ctcgtgtata tactaccact cctaaaaaac cgaactccgc gctgcgtaaa 780

gtatgccgtg ttcgtctgtc taacggtttc gaagtgactt cctacatcgg tggtgaaggt 840

cacaacctgc aggagcactc cgtgatcctg atccgtggcg gtcgtgttaa agacctcccg 900

ggtgttcgtt accacaccgt acgtggtgcg cttgactgct ccggcgttaa agaccgtaag 960

caggctcgtt ccaagtatgg cgtgaagcgt cctaaggctt aactagcacg cgccatgtca 1020

gccgttaagt gttcctgtgt cactcaaaat tgctttgaga ggctctaagg gcttctcagt 1080

gcgttacatc cctggcttgt tgtccacaac cgttaaacct taaaagcttt aaaagcctta 1140

tatattcttt tttttcttat aaaacttaaa accttagagg ctatttaagt tgctgattta 1200

tattaatttt attgttcaaa catgagagct tagtacgtga aacatgagag cttagtacgt 1260

tagccatgag agcttagtac gttagccatg agggtttagt tcgttaaaca tgagagctta 1320

gtacgttaaa catgagagct tagtacgtga aacatgagag cttagtacgt actatcaaca 1380

ggttgaactg ctgatcttca gatcctctac gccggacgca tcgtggccgg atccgattta 1440

ttcaacaaag ccacgttgtg tctcaaaatc tctgatgtta cattgcacaa gataaaaata 1500

tatcatcatg aacaataaaa ctgtctgctt acataaacag taatacaagg ggtgttatga 1560

gccatattca acgggaaacg tcttgctcga ggccgcgatt aaattccaac atggatgctg 1620

atttatatgg gtataaatgg gctcgcgata atgtcgggca atcaggtgcg acaatctatc 1680

gattgtatgg gaagcccgat gcgccagagt tgtttctgaa acatggcaaa ggtagcgttg 1740

ccaatgatgt tacagatgag atggtcagac taaactggct gacggaattt atgcctcttc 1800

cgaccatcaa gcattttatc cgtactcctg atgatgcatg gttactcacc actgcgatcc 1860

ccgggaaaac agcattccag gtattagaag aatatcctga ttcaggtgaa aatattgttg 1920

atgcgctggc agtgttcctg cgccggttgc attcgattcc tgtttgtaat tgtcctttta 1980

acagcgatcg cgtatttcgt ctcgctcagg cgcaatcacg aatgaataac ggtttggttg 2040

atgcgagtga ttttgatgac gagcgtaatg gctggcctgt tgaacaagtc tggaaagaaa 2100

tgcataagct tttgccattc tcaccggatt cagtcgtcac tcatggtgat ttctcacttg 2160

ataaccttat ttttgacgag gggaaattaa taggttgtat tgatgttgga cgagtcggaa 2220

tcgcagaccg ataccaggat cttgccatcc tatggaactg cctcggtgag ttttctcctt 2280

cattacagaa acggcttttt caaaaatatg gtattgataa tcctgatatg aataaattgc 2340

agtttcattt gatgctcgat gagtttttct aatcagaatt ggttaattgg ttgtaacact 2400

ggcttaatta acatcatcaa taatatacct tattttggat tgaagccaat atgataatga 2460

gggggtggag tttgtgacgt ggcgcggggc gtgggaacgg ggcgggtgac gtagtagtgt 2520

ggcggaagtg tgatgttgca agtgtggcgg aacacatgta agcgacggat gtggcaaaag 2580

tgacgttttt ggtgtgcgcc ggtgtacaca ggaagtgaca attttcgcgc ggttttaggc 2640

ggatgttgta gtaaatttgg gcgtaaccga gtaagatttg gccattttcg cgggaaaact 2700

gaataagagg aagtgaaatc tgaataattt tgtgttactc atagcgcgta atactggtac 2760

cccccagtgg caagacgcgc aggcaaaacg caccacgact ggatccgaat tcaaaaagcc 2820

ggaactgatc agaaccattc tcttgaaatg gttctgatca gttccggccg gtgtttcgtc 2880

ctttccacaa gatatataaa gccaagaaat cgaaatactt tcaagttacg gtaagcatat 2940

gatagtccat tttaaaacat aattttaaaa ctgcaaacta cccaagaaat tattactttc 3000

tacgtcacgt attttgtact aatatctttg tgtttacagt caaattaatt ctaattatct 3060

ttctaacagc cttgtatcgt atatgcaaat atgaaggaat catgggaaat aggccctctt 3120

cctgcccgac cttggcgcgc gctcggcgcg cggtcacgct ccgtcacgtg gtgcgttttg 3180

cctgcgcgtc ttgccactgg ggggtacccc gaatcactag atctgagctc ggcctggtga 3240

tgatggcggg atcgttgtat atttcttgac accttttcgg catcgcccta aaattcggcg 3300

tcctcatatt gtgtgaggac gttttattac gtgtttacga agcaaaagct aaaaccagga 3360

gctatttaat ggcaacaacc ggtaccatga ttacgccaag ctacgtaata cgactcacta 3420

gtggaagttc ctattctcta gaaagtatag gaacttcctt aagtgagtcg tattacggac 3480

tggccgtcgt tttacaacgt cgtgactggg aaaaccctgg cgttacccaa cttaatcgcc 3540

ttgcagcaca tccccctttc gccagctggc gtaatagcga agaggcccgc accgatcgcc 3600

cttcccaaca gttgcgcagc ctgaatggcg aatggcgctt cgcttggtaa gctagctaag 3660

gcttaaggag gacaatcatg gcaacagtta accagctggt acgcaaacca cgtgctcgca 3720

aagttgcgaa aagcaacgtg cctgcgctgg aagcatgccc gcaaaaacgt ggcgtatgta 3780

ctcgtgtata tactaccact cctaaaaaac cgaactccgc gctgcgtaaa gtatgccgtg 3840

ttcgtctgtc taacggtttc gaagtgactt cctacatcgg tggtgaaggt cacaacctgc 3900

aggagcactc cgtgatcctg atccgtggcg gtcgtgttaa agacctcccg ggtgttcgtt 3960

accacaccgt acgtggtgcg cttgactgct ccggcgttaa agaccgtaag caggctcgtt 4020

ccaagtatgg cgtgaagcgt cctaaggctt aactagcacg cgccatgtca gccgttaagt 4080

gttcctgtgt cactcaaaat tgctttgaga ggctctaagg gcttctcagt gcgttacatc 4140

cctggcttgt tgtccacaac cgttaaacct taaaagcttt aaaagcctta tatattcttt 4200

tttttcttat aaaacttaaa accttagagg ctatttaagt tgctgattta tattaatttt 4260

attgttcaaa catgagagct tagtacgtga aacatgagag cttagtacgt tagccatgag 4320

agcttagtac gttagccatg agggtttagt tcgttaaaca tgagagctta gtacgttaaa 4380

catgagagct tagtacgtga aacatgagag cttagtacgt actatcaaca ggttgaactg 4440

ctgatcttca gatcctctac gccggacgca tcgtggccgg atccgattta ttcaacaaag 4500

ccacgttgtg tctcaaaatc tctgatgtta cattgcacaa gataaaaata tatcatcatg 4560

aacaataaaa ctgtctgctt acataaacag taatacaagg ggtgttatga gccatattca 4620

acgggaaacg tcttgctcga ggccgcgatt aaattccaac atggatgctg atttatatgg 4680

gtataaatgg gctcgcgata atgtcgggca atcaggtgcg acaatctatc gattgtatgg 4740

gaagcccgat gcgccagagt tgtttctgaa acatggcaaa ggtagcgttg ccaatgatgt 4800

tacagatgag atggtcagac taaactggct gacggaattt atgcctcttc cgaccatcaa 4860

gcattttatc cgtactcctg atgatgcatg gttactcacc actgcgatcc ccgggaaaac 4920

agcattccag gtattagaag aatatcctga ttcaggtgaa aatattgttg atgcgctggc 4980

agtgttcctg cgccggttgc attcgattcc tgtttgtaat tgtcctttta acagcgatcg 5040

cgtatttcgt ctcgctcagg cgcaatcacg aatgaataac ggtttggttg atgcgagtga 5100

ttttgatgac gagcgtaatg gctggcctgt tgaacaagtc tggaaagaaa tgcataagct 5160

tttgccattc tcaccggatt cagtcgtcac tcatggtgat ttctcacttg ataaccttat 5220

ttttgacgag gggaaattaa taggttgtat tgatgttgga cgagtcggaa tcgcagaccg 5280

ataccaggat cttgccatcc tatggaactg cctcggtgag ttttctcctt cattacagaa 5340

acggcttttt caaaaatatg gtattgataa tcctgatatg aataaattgc agtttcattt 5400

gatgctcgat gagtttttct aatcagaatt ggttaattgg ttgtaacact ggcttaatta 5460

acatcatcaa taatatacct tattttggat tgaagccaat atgataatga gggggtggag 5520

tttgtgacgt ggcgcggggc gtgggaacgg ggcgggtgac gtagtagtgt ggcggaagtg 5580

tgatgttgca agtgtggcgg aacacatgta agcgacggat gtggcaaaag tgacgttttt 5640

ggtgtgcgcc ggtgtacaca ggaagtgaca attttcgcgc ggttttaggc ggatgttgta 5700

gtaaatttgg gcgtaaccga gtaagatttg gccattttcg cgggaaaact gaataagagg 5760

aagtgaaatc tgaataattt tgtgttactc atagcgcgta atactggtac cccccagtgg 5820

caagacgcgc aggcaaaacg caccacgact ggatccgaat tcaaaaagcc ggaactgatc 5880

agaaccattc tcttgaaatg gttctgatca gttccggccg gtgtttcgtc ctttccacaa 5940

gatatataaa gccaagaaat cgaaatactt tcaagttacg gtaagcatat gatagtccat 6000

tttaaaacat aattttaaaa ctgcaaacta cccaagaaat tattactttc tacgtcacgt 6060

attttgtact aatatctttg tgtttacagt caaattaatt ctaattatct ttctaacagc 6120

cttgtatcgt atatgcaaat atgaaggaat catgggaaat aggccctctt cctgcccgac 6180

cttggcgcgc gctcggcgcg cggtcacgct ccgtcacgtg gtgcgttttg cctgcgcgtc 6240

ttgccactgg ggggtacccc gaatcactag atctgagctc ggcctggtga tgatggcggg 6300

atcgttgtat atttcttgac accttttcgg catcgcccta aaattcggcg tcctcatatt 6360

gtgtgaggac gttttattac gtgtttacga agcaaaagct aaaaccagga gctatttaat 6420

ggcaacaacc ggtaccatga ttacgccaag ctacgtaata cgactcacta gtggaagttc 6480

ctattctcta gaaagtatag gaacttcaag agcgcttttg agcttgttta tcgaattcaa 6540

gcttgtcgac tcgaagatct gggcgtggtt aagggtggga aagaatatat aaggtggggg 6600

tcttatgtag ttttgtatct gttttgcagc agccgccgcc gccatgagca ccaactcgtt 6660

tgatggaagc attgtgagct catatttgac aacgcgcatg cccccatggg ccggggtgcg 6720

tcagaatgtg atgggctcca gcattgatgg tcgccccgtc ctgcccgcaa actctactac 6780

cttgacctac gagaccgtgt ctggaacgcc gttggagact gcagcctccg ccgccgcttc 6840

agccgctgca gccaccgccc gcgggattgt gactgacttt gctttcctga gcccgcttgc 6900

aagcagtgca gcttcccgtt catccgcccg cgatgacaag ttgacggctc ttttggcaca 6960

attggattct ttgacccggg aacttaatgt cgtttctcag cagctgttgg atctgcgcca 7020

gcaggtttct gccctgaagg cttcctcccc tcccaatgcg gtttaaaaca taaataaaaa 7080

accagactct gtttggattt ggatcaagca agtgtcttgc tgtctttatt taggggtttt 7140

gcgcgcgcgg taggcccggg accagcggtc tcggtcgttg agggtcctgt gtattttttc 7200

caggacgtgg taaaggtgac tctggatgtt cagatacatg ggcataagcc cgtctctggg 7260

gtggaggtag caccactgca gagcttcatg ctgcggggtg gtgttgtaga tgatccagtc 7320

gtagcaggag cgctgggcgt ggtgcctaaa aatgtctttc agtagcaagc tgattgccag 7380

gggcaggccc ttggtgtaag tgtttacaaa gcggttaagc tgggatgggt gcatacgtgg 7440

ggatatgaga tgcatcttgg actgtatttt taggttggct atgttcccag ccatatccct 7500

ccggggattc atgttgtgca gaaccaccag cacagtgtat ccggtgcact tgggaaattt 7560

gtcatgtagc ttagaaggaa atgcgtggaa gaacttggag acgcccttgt gacctccaag 7620

attttccatg cattcgtcca taatgatggc aatgggccca cgggcggcgg cctgggcgaa 7680

gatatttctg ggatcactaa cgtcatagtt gtgttccagg atgagatcgt cataggccat 7740

ttttacaaag cgcgggcgga gggtgccaga ctgcggtata atggttccat ccggcccagg 7800

ggcgtagtta ccctcacaga tttgcatttc ccacgctttg agttcagatg gggggatcat 7860

gtctacctgc ggggcgatga agaaaacggt ttccggggta ggggagatca gctgggaaga 7920

aagcaggttc ctgagcagct gcgacttacc gcagccggtg ggcccgtaaa tcacacctat 7980

taccgggtgc aactggtagt taagagagct gcagctgccg tcatccctga gcaggggggc 8040

cacttcgtta agcatgtccc tgactcgcat gttttccctg accaaatccg ccagaaggcg 8100

ctcgccgccc agcgatagca gttcttgcaa ggaagcaaag tttttcaacg gtttgagacc 8160

gtccgccgta ggcatgcttt tgagcgtttg accaagcagt tccaggcggt cccacagctc 8220

ggtcacctgc tctacggcat ctcgatccag catatctcct cgtttcgcgg gttggggcgg 8280

ctttcgctgt acggcagtag tcggtgctcg tccagacggg ccagggtcat gtctttccac 8340

gggcgcaggg tcctcgtcag cgtagtctgg gtcacggtga aggggtgcgc tccgggctgc 8400

gcgctggcca gggtgcgctt gaggctggtc ctgctggtgc tgaagcgctg ccggtcttcg 8460

ccctgcgcgt cggccaggta gcatttgacc atggtgtcat agtccagccc ctccgcggcg 8520

tggcccttgg cgcgcagctt gcccttggag gaggcgccgc acgaggggca gtgcagactt 8580

ttgagggcgt agagcttggg cgcgagaaat accgattccg gggagtaggc atccgcgccg 8640

caggccccgc agacggtctc gcattccacg agccaggtga gctctggccg ttcggggtca 8700

aaaaccaggt ttcccccatg ctttttgatg cgtttcttac ctctggtttc catgagccgg 8760

tgtccacgct cggtgacgaa aaggctgtcc gtgtccccgt atacagactt gagaggcctg 8820

tcctcgagcg gtgttccgcg gtcctcctcg tatagaaact cggaccactc tgagacaaag 8880

gctcgcgtcc aggccagcac gaaggaggct aagtgggagg ggtagcggtc gttgtccact 8940

agggggtcca ctcgctccag ggtgtgaaga cacatgtcgc cctcttcggc atcaaggaag 9000

gtgattggtt tgtaggtgta ggccacgtga ccgggtgttc ctgaaggggg gctataaaag 9060

ggggtggggg cgcgttcgtc ctcactctct tccgcatcgc tgtctgcgag ggccagctgt 9120

tggggtgagt actccctctg aaaagcgggc atgacttctg cgctaagatt gtcagtttcc 9180

aaaaacgagg aggatttgat attcacctgg cccgcggtga tgcctttgag ggtggccgca 9240

tccatctggt cagaaaagac aatctttttg ttgtcaagct tggtggcaaa cgacccgtag 9300

agggcgttgg acagcaactt ggcgatggag cgcagggttt ggtttttgtc gcgatcggcg 9360

cgctccttgg ccgcgatgtt tagctgcacg tattcgcgcg caacgcaccg ccattcggga 9420

aagacggtgg tgcgctcgtc gggcaccagg tgcacgcgcc aaccgcggtt gtgcagggtg 9480

acaaggtcaa cgctggtggc tacctctccg cgtaggcgct cgttggtcca gcagaggcgg 9540

ccgcccttgc gcgagcagaa tggcggtagg gggtctagct gcgtctcgtc cggggggtct 9600

gcgtccacgg taaagacccc gggcagcagg cgcgcgtcga agtagtctat cttgcatcct 9660

tgcaagtcta gcgcctgctg ccatgcgcgg gcggcaagcg cgcgctcgta tgggttgagt 9720

gggggacccc atggcatggg gtgggtgagc gcggaggcgt acatgccgca aatgtcgtaa 9780

acgtagaggg gctctctgag tattccaaga tatgtagggt agcatcttcc accgcggatg 9840

ctggcgcgca cgtaatcgta tagttcgtgc gagggagcga ggaggtcggg accgaggttg 9900

ctacgggcgg gctgctctgc tcggaagact atctgcctga agatggcatg tgagttggat 9960

gatatggttg gacgctggaa gacgttgaag ctggcgtctg tgagacctac cgcgtcacgc 10020

acgaaggagg cgtaggagtc gcgcagcttg ttgaccagct cggcggtgac ctgcacgtct 10080

agggcgcagt agtccagggt ttccttgatg atgtcatact tatcctgtcc cttttttttc 10140

cacagctcgc ggttgaggac aaactcttcg cggtctttcc agtactcttg gatcggaaac 10200

ccgtcggcct ccgaacggta agagcctagc atgtagaact ggttgacggc ctggtaggcg 10260

cagcatccct tttctacggg tagcgcgtat gcctgcgcgg ccttccggag cgaggtgtgg 10320

gtgagcgcaa aggtgtccct gaccatgact ttgaggtact ggtatttgaa gtcagtgtcg 10380

tcgcatccgc cctgctccca gagcaaaaag tccgtgcgct ttttggaacg cggatttggc 10440

agggcgaagg tgacatcgtt gaagagtatc tttcccgcgc gaggcataaa gttgcgtgtg 10500

atgcggaagg gtcccggcac ctcggaacgg ttgttaatta cctgggcggc gagcacgatc 10560

tcgtcaaagc cgttgatgtt gtggcccaca atgtaaagtt ccaagaagcg cgggatgccc 10620

ttgatggaag gcaatttttt aagttcctcg taggtgagct cttcagggga gctgagcccg 10680

tgctctgaaa gggcccagtc tgcaagatga gggttggaag cgacgaatga gctccacagg 10740

tcacgggcca ttagcatttg caggtggtcg cgaaaggtcc taaactggcg acctatggcc 10800
attttttctg gggtgatgca gtagaaggta agcgggtctt gttcccagcg gtcccatcca 10860
aggttcgcgg ctaggtctcg cgcggcagtc actagaggct catctccgcc gaacttcatg 10920
accagcatga agggcacgag ctgcttccca aaggccccca tccaagtata ggtctctaca 10980
tcgtaggtga caaagagacg ctcggtgcga ggatgcgagc cgatcgggaa gaactggatc 11040
tcccgccacc aattggagga gtggctattg atgtggtgaa agtagaagtc cctgcgacgg 11100
gccgaacact cgtgctggct tttgtaaaaa cgtgcgcagt actggcagcg gtgcacgggc 11160
tgtacatcct gcacgaggtt gacctgacga ccgcgcacaa ggaagcagag tgggaatttg 11220
agcccctcgc ctggcgggtt tggctggtgg tcttctactt cggctgcttg tccttgaccg 11280
tctggctgct cgaggggagt tacggtggat cggaccacca cgccgcgcga gcccaaagtc 11340
cagatgtccg cgcgcggcgg tcggagcttg atgacaacat cgcgcagatg ggagctgtcc 11400
atggtctgga gctcccgcgg cgtcaggtca ggcgggagct cctgcaggtt tacctcgcat 11460
agacgggtca gggcgcgggc tagatccagg tgatacctaa tttccagggg ctggttggtg 11520
gcggcgtcga tggcttgcaa gaggccgcat ccccgcggcg cgactacggt accgcgcggc 11580
gggcggtggg ccgcgggggt gtccttggat gatgcatcta aaagcggtga cgcgggcgag 11640
cccccggagg tagggggggc tccggacccg ccgggagagg gggcaggggc acgtcggcgc 11700
cgcgcgcggg caggagctgg tgctgcgcgc gtaggttgct ggcgaacgcg acgacgcggc 11760
ggttgatctc ctgaatctgg cgcctctgcg tgaagacgac gggcccggtg agcttgagcc 11820
tgaaagagag ttcgacagaa tcaatttcgg tgtcgttgac ggcggcctgg cgcaaaatct 11880
cctgcacgtc tcctgagttg tcttgatagg cgatctcggc catgaactgc tcgatctctt 11940
cctcctggag atctccgcgt ccggctcgct ccacggtggc ggcgaggtcg ttggaaatgc 12000
gggccatgag ctgcgagaag gcgttgaggc ctccctcgtt ccagacgcgg ctgtagacca 12060
cgcccccttc ggcatcgcgg gcgcgcatga ccacctgcgc gagattgagc tccacgtgcc 12120
gggcgaagac ggcgtagttt cgcaggcgct gaaagaggta gttgagggtg gtggcggtgt 12180
gttctgccac gaagaagtac ataacccagc gtcgcaacgt ggattcgttg atatccccca 12240
aggcctcaag gcgctccatg gcctcgtaga agtccacggc gaagttgaaa aactgggagt 12300
tgcgcgccga cacggttaac tcctcctcca gaagacggat gagctcggcg acagtgtcgc 12360
gcacctcgcg ctcaaaggct acaggggcct cttcttcttc ttcaatctcc tcttccataa 12420
gggcctcccc ttcttcttct tctggcggcg gtgggggagg ggggacacgg cggcgacgac 12480
ggcgcaccgg gaggcggtcg acaaagcgct cgatcatctc cccgcggcga cggcgcatgg 12540
tctcggtgac ggcgcggccg ttctcgcggg ggcgcagttg gaagacgccg cccgtcatgt 12600

cccggttatg ggttggcggg gggctgccat gcggcaggga tacggcgcta acgatgcatc 12660

tcaacaattg ttgtgtaggt actccgccgc cgagggacct gagcgagtcc gcatcgaccg 12720

gatcggaaaa cctctcgaga aaggcgtcta accagtcaca gtcgcaaggt aggctgagca 12780

ccgtggcggg cggcagcggg cggcggtcgg ggttgtttct ggcggaggtg ctgctgatga 12840

tgtaattaaa gtaggcggtc ttgagacggc ggatggtcga cagaagcacc atgtccttgg 12900

gtccggcctg ctgaatgcgc aggcggtcgg ccatgcccca ggcttcgttt tgacatcggc 12960

gcaggtcttt gtagtagtct tgcatgagcc tttctaccgg cacttcttct tctccttcct 13020

cttgtcctgc atctcttgca tctatcgctg cggcggcggc ggagtttggc cgtaggtggc 13080

gccctcttcc tcccatgcgt gtgaccccga agcccctcat cggctgaagc agggctaggt 13140

cggcgacaac gcgctcggct aatatggcct gctgcacctg cgtgagggta gactggaagt 13200

catccatgtc cacaaagcgg tggtatgcgc ccgtgttgat ggtgtaagtg cagttggcca 13260

taacggacca gttaacggtc tggtgacccg gctgcgagag ctcggtgtac ctgagacgcg 13320

agtaagccct cgagtcaaat acgtagtcgt tgcaagtccg caccaggtac tggtatccca 13380

ccaaaaagtg cggcggcggc tggcggtaga ggggccagcg tagggtggcc ggggctccgg 13440

gggcgagatc ttccaacata aggcgatgat atccgtagat gtacctggac atccaggtga 13500

tgccggcggc ggtggtggag gcgcgcggaa agtcgcggac gcggttccag atgttgcgca 13560

gcggcaaaaa gtgctccatg gtcgggacgc tctggccggt caggcgcgcg caatcgttga 13620

cgctctaccg tgcaaaagga gagcctgtaa gcgggcactc ttccgtggtc tggtggataa 13680

attcgcaagg gtatcatggc ggacgaccgg ggttcgagcc ccgtatccgg ccgtccgccg 13740

tgatccatgc ggttaccgcc cgcgtgtcga acccaggtgt gcgacgtcag acaacggggg 13800

agtgctcctt ttggcttcct tccaggcgcg gcggctgctg cgctagcttt tttggccact 13860

ggccgcgcgc agcgtaagcg gttaggctgg aaagcgaaag cattaagtgg ctcgctccct 13920

gtagccggag ggttattttc caagggttga gtcgcgggac ccccggttcg agtctcggac 13980

cggccggact gcggcgaacg ggggtttgcc tccccgtcat gcaagacccc gcttgcaaat 14040

tcctccggaa acagggacga gccccttttt tgcttttccc agatgcatcc ggtgctgcgg 14100

cagatgcgcc cccctcctca gcagcggcaa gagcaagagc agcggcagac atgcagggca 14160

ccctcccctc ctcctaccgc gtcaggaggg gcgacatccg cggttgacgc ggcagcagat 14220

ggtgattacg aacccccgcg gcgccgggcc cggcactacc tggacttgga ggagggcgag 14280

ggcctggcgc ggctaggagc gccctctcct gagcggtacc caagggtgca gctgaagcgt 14340

gatacgcgtg aggcgtacgt gccgcggcag aacctgtttc gcgaccgcga gggagaggag 14400

cccgaggaga tgcgggatcg aaagttccac gcagggcgcg agctgcggca tggcctgaat 14460

cgcgagcggt tgctgcgcga ggaggacttt gagcccgacg cgcgaaccgg gattagtccc 14520

gcgcgcgcac acgtggcggc cgccgacctg gtaaccgcat acgagcagac ggtgaaccag 14580
gagattaact ttcaaaaaag ctttaacaac cacgtgcgta cgcttgtggc gcgcgaggag 14640
gtggctatag gactgatgca tctgtgggac tttgtaagcg cgctggagca aaacccaaat 14700
agcaagccgc tcatggcgca gctgttcctt atagtgcagc acagcaggga caacgaggca 14760
ttcagggatg cgctgctaaa catagtagag cccgagggcc gctggctgct cgatttgata 14820
aacatcctgc agagcatagt ggtgcaggag cgcagcttga gcctggctga caaggtggcc 14880
gccatcaact attccatgct tagcctgggc aagttttacg cccgcaagat ataccatacc 14940
ccttacgttc ccatagacaa ggaggtaaag atcgaggggt tctacatgcg catggcgctg 15000
aaggtgctta ccttgagcga cgacctgggc gtttatcgca acgagcgcat ccacaaggcc 15060
gtgagcgtga gccggcggcg cgagctcagc gaccgcgagc tgatgcacag cctgcaaagg 15120
gccctggctg gcacgggcag cggcgataga gaggccgagt cctactttga cgcgggcgct 15180
gacctgcgct gggccccaag ccgacgcgcc ctggaggcag ctggggccgg acctgggctg 15240
gcggtggcac ccgcgcgcgc tggcaacgtc ggcggcgtgg aggaatatga cgaggacgat 15300
gagtacgagc cagaggacgg cgagtactaa gcggtgatgt ttctgatcag atgatgcaag 15360
acgcaacgga cccggcggtg cgggcggcgc tgcagagcca gccgtccggc cttaactcca 15420
cggacgactg gcgccaggtc atggaccgca tcatgtcgct gactgcgcgc aatcctgacg 15480
cgttccggca gcagccgcag gccaaccggc tctccgcaat tctggaagcg gtggtcccgg 15540
cgcgcgcaaa ccccacgcac gagaaggtgc tggcgatcgt aaacgcgctg gccgaaaaca 15600
gggccatccg gcccgacgag gccggcctgg tctacgacgc gctgcttcag cgcgtggctc 15660
gttacaacag cggcaacgtg cagaccaacc tggaccggct ggtgggggat gtgcgcgagg 15720
ccgtggcgca gcgtgagcgc gcgcagcagc agggcaacct gggctccatg gttgcactaa 15780
acgccttcct gagtacacag cccgccaacg tgccgcgggg acaggaggac tacaccaact 15840
ttgtgagcgc actgcggcta atggtgactg agacaccgca aagtgaggtg taccagtctg 15900
ggccagacta ttttttccag accagtagac aaggcctgca gaccgtaaac ctgagccagg 15960
ctttcaaaaa cttgcagggg ctgtgggggg tgcgggctcc cacaggcgac cgcgcgaccg 16020
tgtctagctt gctgacgccc aactcgcgcc tgttgctgct gctaatagcg cccttcacgg 16080
acagtggcag cgtgtcccgg gacacatacc taggtcactt gctgacactg taccgcgagg 16140
ccataggtca ggcgcatgtg gacgagcata ctttccagga gattacaagt gtcagccgcg 16200
cgctggggca ggaggacacg ggcagcctgg aggcaaccct aaactacctg ctgaccaacc 16260
ggcggcagaa gatcccctcg ttgcacagtt taaacagcga ggaggagcgc attttgcgct 16320
acgtgcagca gagcgtgagc cttaacctga tgcgcgacgg ggtaacgccc agcgtggcgc 16380

tggacatgac cgcgcgcaac atggaaccgg gcatgtatgc ctcaaaccgg ccgtttatca 16440

accgcctaat ggactacttg catcgcgcgg ccgccgtgaa ccccgagtat ttcaccaatg 16500

ccatcttgaa cccgcactgg ctaccgcccc ctggtttcta caccggggga ttcgaggtgc 16560

ccgagggtaa cgatggattc ctctgggacg acatagacga cagcgtgttt tccccgcaac 16620

cgcagaccct gctagagttg caacagcgcg agcaggcaga ggcggcgctg cgaaaggaaa 16680

gcttccgcag gccaagcagc ttgtccgatc taggcgctgc ggccccgcgg tcagatgcta 16740

gtagcccatt tccaagcttg atagggtctc ttaccagcac tcgcaccacc cgcccgcgcc 16800

tgctgggcga ggaggagtac ctaaacaact cgctgctgca gccgcagcgc gaaaaaaacc 16860

tgcctccggc atttcccaac aacgggatag agagcctagt ggacaagatg agtagatgga 16920

agacgtacgc gcaggagcac agggacgtgc caggcccgcg cccgcccacc cgtcgtcaaa 16980

ggcacgaccg tcagcggggt ctggtgtggg aggacgatga ctcggcagac gacagcagcg 17040

tcctggattt gggagggagt ggcaacccgt ttgcgcacct tcgccccagg ctggggagaa 17100

tgttttaaaa aaaaaaaagc atgatgcaaa ataaaaaact caccaaggcc atggcaccga 17160

gcgttggttt tcttgtattc cccttagtat gcggcgcgcg gcgatgtatg aggaaggtcc 17220

tcctccctcc tacgagagtg tggtgagcgc ggcgccagtg gcggcggcgc tgggttctcc 17280

cttcgatgct cccctggacc cgccgtttgt gcctccgcgg tacctgcggc ctaccggggg 17340

gagaaacagc atccgttact ctgagttggc acccctattc gacaccaccc gtgtgtacct 17400

ggtggacaac aagtcaacgg atgtggcatc cctgaactac cagaacgacc acagcaactt 17460

tctgaccacg gtcattcaaa acaatgacta cagcccgggg gaggcaagca cacagaccat 17520

caatcttgac gaccggtcgc actggggcgg cgacctgaaa accatcctgc ataccaacat 17580

gccaaatgtg aacgagttca tgtttaccaa taagtttaag gcgcgggtga tggtgtcgcg 17640

cttgcctact aaggacaatc aggtggagct gaaatacgag tgggtggagt tcacgctgcc 17700

cgagggcaac tactccgaga ccatgaccat agaccttatg aacaacgcga tcgtggagca 17760

ctacttgaaa gtgggcagac agaacggggt tctggaaagc gacatcgggg taaagtttga 17820

cacccgcaac ttcagactgg ggtttgaccc cgtcactggt cttgtcatgc ctggggtata 17880

tacaaacgaa gccttccatc cagacatcat tttgctgcca ggatgcgggg tggacttcac 17940

ccacagccgc ctgagcaact tgttgggcat ccgcaagcgg caacccttcc aggagggctt 18000

taggatcacc tacgatgatc tggagggtgg taacattccc gcactgttgg atgtggacgc 18060

ctaccaggcg agcttgaaag atgacaccga acagggcggg ggtggcgcag gcggcagcaa 18120

cagcagtggc agcggcgcgg aagagaactc caacgcggca gccgcggcaa tgcagccggt 18180

ggaggacatg aacgatcatg ccattcgcgg cgacaccttt gccacacggg ctgaggagaa 18240

gcgcgctgag gccgaagcag cggccgaagc tgccgccccc gctgcgcaac ccgaggtcga 18300

gaagcctcag aagaaaccgg tgatcaaacc cctgacagag gacagcaaga aacgcagtta 18360
caacctaata agcaatgaca gcaccttcac ccagtaccgc agctggtacc ttgcatacaa 18420
ctacggcgac cctcagaccg gaatccgctc atggaccctg ctttgcactc ctgacgtaac 18480
ctgcggctcg gagcaggtct actggtcgtt gccagacatg atgcaagacc ccgtgacctt 18540
ccgctccacg cgccagatca gcaactttcc ggtggtgggc gccgagctgt tgcccgtgca 18600
ctccaagagc ttctacaacg accaggccgt ctactcccaa ctcatccgcc agtttacctc 18660
tctgacccac gtgttcaatc gctttcccga gaaccagatt ttggcgcgcc cgccagcccc 18720
caccatcacc accgtcagtg aaaacgttcc tgctctcaca gatcacggga cgctaccgct 18780
gcgcaacagc atcggaggag tccagcgagt gaccattact gacgccagac gccgcacctg 18840
cccctacgtt tacaaggccc tgggcatagt ctcgccgcgc gtcctatcga gccgcacttt 18900
ttgagcaagc atgtccatcc ttatatcgcc cagcaataac acaggctggg gcctgcgctt 18960
cccaagcaag atgtttggcg gggccaagaa gcgctccgac caacacccag tgcgcgtgcg 19020
cgggcactac cgcgcgccct ggggcgcgca caaacgcggc cgcactgggc gcaccaccgt 19080
cgatgacgcc atcgacgcgg tggtggagga ggcgcgcaac tacacgccca cgccgccacc 19140
agtgtccaca gtggacgcgg ccattcagac cgtggtgcgc ggagcccggc gctatgctaa 19200
aatgaagaga cggcggaggc gcgtagcacg tcgccaccgc cgccgacccg gcactgccgc 19260
ccaacgcgcg gcggcggccc tgcttaaccg cgcacgtcgc accggccgac gggcggccat 19320
gcgggccgct cgaaggctgg ccgcgggtat tgtcactgtg ccccccaggt ccaggcgacg 19380
agcggccgcc gcagcagccg cggccattag tgctatgact cagggtcgca ggggcaacgt 19440
gtattgggtg cgcgactcgg ttagcggcct gcgcgtgccc gtgcgcaccc gccccccgcg 19500
caactagatt gcaagaaaaa actacttaga ctcgtactgt tgtatgtatc cagcggcggc 19560
ggcgcgcaac gaagctatgt ccaagcgcaa aatcaaagaa gagatgctcc aggtcatcgc 19620
gccggagatc tatggccccc cgaagaagga agagcaggat tacaagcccc gaaagctaaa 19680
gcgggtcaaa aagaaaaaga aagatgatga tgatgaactt gacgacgagg tggaactgct 19740
gcacgctacc gcgcccaggc gacgggtaca gtggaaaggt cgacgcgtaa aacgtgtttt 19800
gcgacccggc accaccgtag tctttacgcc cggtgagcgc tccacccgca cctacaagcg 19860
cgtgtatgat gaggtgtacg gcgacgagga cctgcttgag caggccaacg agcgcctcgg 19920
ggagtttgcc tacggaaagc ggcataagga catgctggcg ttgccgctgg acgagggcaa 19980
cccaacacct agcctaaagc ccgtaacact gcagcaggtg ctgcccgcgc ttgcaccgtc 20040
cgaagaaaag cgcggcctaa agcgcgagtc tggtgacttg gcacccaccg tgcagctgat 20100
ggtacccaag cgccagcgac tggaagatgt cttggaaaaa atgaccgtgg aacctgggct 20160

ggagcccgag gtccgcgtgc ggccaatcaa gcaggtggcg ccgggactgg gcgtgcagac 20220
cgtggacgtt cagataccca ctaccagtag caccagtatt gccaccgcca cagagggcat 20280
ggagacacaa acgtccccgg ttgcctcagc ggtggcggat gccgcggtgc aggcggtcgc 20340
tgcggccgcg tccaagacct ctacggaggt gcaaacggac ccgtggatgt ttcgcgtttc 20400
agccccccgg cgcccgcgcg gttcgaggaa gtacggcgcc gccagcgcgc tactgcccga 20460
atatgcccta catccttcca ttgcgcctac ccccggctat cgtggctaca cctaccgccc 20520
cagaagacga gcaactaccc gacgccgaac caccactgga acccgccgcc gccgtcgccg 20580
tcgccagccc gtgctggccc cgatttccgt gcgcagggtg gctcgcgaag gaggcaggac 20640
cctggtgctg ccaacagcgc gctaccaccc cagcatcgtt taaaagccgg tctttgtggt 20700
tcttgcagat atggccctca cctgccgcct ccgtttcccg gtgccgggat tccgaggaag 20760
aatgcaccgt aggaggggca tggccggcca cggcctgacg ggcggcatgc gtcgtgcgca 20820
ccaccggcgg cggcgcgcgt cgcaccgtcg catgcgcggc ggtatcctgc ccctccttat 20880
tccactgatc gccgcggcga ttggcgccgt gcccggaatt gcatccgtgg ccttgcaggc 20940
gcagagacac tgattaaaaa caagttgcat gtggaaaaat caaaataaaa agtctggact 21000
ctcacgctcg cttggtcctg taactatttt gtagaatgga agacatcaac tttgcgtctc 21060
tggccccgcg acacggctcg cgcccgttca tgggaaactg gcaagatatc ggcaccagca 21120
atatgagcgg tggcgccttc agctggggct cgctgtggag cggcattaaa aatttcggtt 21180
ccaccgttaa gaactatggc agcaaggcct ggaacagcag cacaggccag atgctgaggg 21240
ataagttgaa agagcaaaat ttccaacaaa aggtggtaga tggcctggcc tctggcatta 21300
gcggggtggt ggacctggcc aaccaggcag tgcaaaataa gattaacagt aagcttgatc 21360
cccgccctcc cgtagaggag cctccaccgg ccgtggagac agtgtctcca gaggggcgtg 21420
gcgaaaagcg tccgcgcccc gacagggaag aaactctggt gacgcaaata gacgagcctc 21480
cctcgtacga ggaggcacta aagcaaggcc tgcccaccac ccgtcccatc gcgcccatgg 21540
ctaccggagt gctgggccag cacacacccg taacgctgga cctgcctccc cccgccgaca 21600
cccagcagaa acctgtgctg ccaggcccga ccgccgttgt tgtaacccgt cctagccgcg 21660
cgtccctgcg ccgcgccgcc agcggtccgc gatcgttgcg gcccgtagcc agtggcaact 21720
ggcaaagcac actgaacagc atcgtgggtc tgggggtgca atccctgaag cgccgacgat 21780
gcttctgaat agctaacgtg tcgtatgtgt gtcatgtatg cgtccatgtc gccgccagag 21840
gagctgctga gccgccgcgc gcccgctttc caagatggct accccttcga tgatgccgca 21900
gtggtcttac atgcacatct cgggccagga cgcctcggag tacctgagcc ccgggctggt 21960
gcagtttgcc cgcgccaccg agacgtactt cagcctgaat aacaagttta gaaaccccac 22020
ggtggcgcct acgcacgacg tgaccacaga ccggtcccag cgtttgacgc tgcggttcat 22080

ccctgtggac cgtgaggata ctgcgtactc gtacaaggcg cggttcaccc tagctgtggg 22140

tgataaccgt gtgctggaca tggcttccac gtactttgac atccgcggcg tgctggacag 22200

gggccctact tttaagccct actctggcac tgcctacaac gccctggctc ccaagggtgc 22260

cccaaatcct tgcgaatggg atgaagctgc tactgctctt gaaataaacc tagaagaaga 22320

ggacgatgac aacgaagacg aagtagacga gcaagctgag cagcaaaaaa ctcacgtatt 22380

tgggcaggcg ccttattctg gtataaatat tacaaaggag ggtattcaaa taggtgtcga 22440

aggtcaaaca cctaaatatg ccgataaaac atttcaacct gaacctcaaa taggagaatc 22500

tcagtggtac gaaactgaaa ttaatcatgc agctgggaga gtccttaaaa agactacccc 22560

aatgaaacca tgttacggtt catatgcaaa acccacaaat gaaaatggag ggcaaggcat 22620

tcttgtaaag caacaaaatg gaaagctaga aagtcaagtg gaaatgcaat ttttctcaac 22680

tactgaggcg accgcaggca atggtgataa cttgactcct aaagtggtat tgtacagtga 22740

agatgtagat atagaaaccc cagacactca tatttcttac atgcccacta ttaaggaagg 22800

taactcacga gaactaatgg gccaacaatc tatgcccaac aggcctaatt acattgcttt 22860

tagggacaat tttattggtc taatgtatta caacagcacg ggtaatatgg gtgttctggc 22920

gggccaagca tcgcagttga atgctgttgt agatttgcaa gacagaaaca cagagctttc 22980

ataccagctt ttgcttgatt ccattggtga tagaaccagg tacttttcta tgtggaatca 23040

ggctgttgac agctatgatc cagatgttag aattattgaa aatcatggaa ctgaagatga 23100

acttccaaat tactgctttc cactgggagg tgtgattaat acagagactc ttaccaaggt 23160

aaaacctaaa acaggtcagg aaaatggatg ggaaaaagat gctacagaat tttcagataa 23220

aaatgaaata agagttggaa ataattttgc catggaaatc aatctaaatg ccaacctgtg 23280

gagaaatttc ctgtactcca acatagcgct gtatttgccc gacaagctaa agtacagtcc 23340

ttccaacgta aaaatttctg ataacccaaa cacctacgac tacatgaaca agcgagtggt 23400

ggctcccggg ttagtggact gctacattaa ccttggagca cgctggtccc ttgactatat 23460

ggacaacgtc aacccattta accaccaccg caatgctggc ctgcgctacc gctcaatgtt 23520

gctgggcaat ggtcgctatg tgcccttcca catccaggtg cctcagaagt tctttgccat 23580

taaaaacctc cttctcctgc cgggctcata cacctacgag tggaacttca ggaaggatgt 23640

taacatggtt ctgcagagct ccctaggaaa tgacctaagg gttgacggag ccagcattaa 23700

gtttgatagc atttgccttt acgccacctt cttccccatg gcccacaaca ccgcctccac 23760

gcttgaggcc atgcttagaa acgacaccaa cgaccagtcc tttaacgact atctctccgc 23820

cgccaacatg ctctacccta tacccgccaa cgctaccaac gtgcccatat ccatcccctc 23880

ccgcaactgg gcggctttcc gcggctgggc cttcacgcgc cttaagacta aggaaacccc 23940

atcactgggc tcgggctacg acccttatta cacctactct ggctctatac cctacctaga 24000

tggaaccttt tacctcaacc acacctttaa gaaggtggcc attacctttg actcttctgt 24060

cagctggcct ggcaatgacc gcctgcttac ccccaacgag tttgaaatta agcgctcagt 24120

tgacggggag ggttacaacg ttgcccagtg taacatgacc aaagactggt tcctggtaca 24180

aatgctagct aactacaaca ttggctacca gggcttctat atcccagaga gctacaagga 24240

ccgcatgtac tccttcttta gaaacttcca gcccatgagc cgtcaggtgg tggatgatac 24300

taaatacaag gactaccaac aggtgggcat cctacaccaa cacaacaact ctggatttgt 24360

tggctacctt gcccccacca tgcgcgaagg acaggcctac cctgctaact tcccctatcc 24420

gcttataggc aagaccgcag ttgacagcat tacccagaaa aagtttcttt gcgatcgcac 24480

cctttggcgc atcccattct ccagtaactt tatgtccatg ggcgcactca cagacctggg 24540

ccaaaacctt ctctacgcca actccgccca cgcgctagac atgacttttg aggtggatcc 24600

catggacgag cccacccttc tttatgtttt gtttgaagtc tttgacgtgg tccgtgtgca 24660

ccggccgcac cgcggcgtca tcgaaaccgt gtacctgcgc acgcccttct cggccggcaa 24720

cgccacaaca taaagaagca agcaacatca acaacagctg ccgccatggg ctccagtgag 24780

caggaactga aagccattgt caaagatctt ggttgtgggc catatttttt gggcacctat 24840

gacaagcgct ttccaggctt tgtttctcca cacaagctcg cctgcgccat agtcaatacg 24900

gccggtcgcg agactggggg cgtacactgg atggcctttg cctggaaccc gcactcaaaa 24960

acatgctacc tctttgagcc ctttggcttt tctgaccagc gactcaagca ggtttaccag 25020

tttgagtacg agtcactcct gcgccgtagc gccattgctt cttcccccga ccgctgtata 25080

acgctggaaa agtccaccca aagcgtacag gggcccaact cggccgcctg tggactattc 25140

tgctgcatgt ttctccacgc ctttgccaac tggccccaaa ctcccatgga tcacaacccc 25200

accatgaacc ttattaccgg ggtacccaac tccatgctca acagtcccca ggtacagccc 25260

accctgcgtc gcaaccagga acagctctac agcttcctgg agcgccactc gccctacttc 25320

cgcagccaca gtgcgcagat taggagcgcc acttcttttt gtcacttgaa aaacatgtaa 25380

aaataatgta ctagagacac tttcaataaa ggcaaatgct tttatttgta cactctcggg 25440

tgattattta cccccaccct tgccgtctgc gccgtttaaa aatcaaaggg gttctgccgc 25500

gcatcgctat gcgccactgg cagggacacg ttgcgatact ggtgtttagt gctccactta 25560

aactcaggca caaccatccg cggcagctcg gtgaagtttt cactccacag gctgcgcacc 25620

atcaccaacg cgtttagcag gtcgggcgcc gatatcttga agtcgcagtt ggggcctccg 25680

ccctgcgcgc gcgagttgcg atacacaggg ttgcagcact ggaacactat cagcgccggg 25740

tggtgcacgc tggccagcac gctcttgtcg gagatcagat ccgcgtccag gtcctccgcg 25800

ttgctcaggg cgaacggagt caactttggt agctgccttc ccaaaaaggg cgcgtgccca 25860

ggctttgagt tgcactcgca ccgtagtggc atcaaaaggt gaccgtgccc ggtctgggcg 25920
ttaggataca gcgcctgcat aaaagccttg atctgcttaa aagccacctg agcctttgcg 25980
ccttcagaga agaacatgcc gcaagacttg ccggaaaact gattggccgg acaggccgcg 26040
tcgtgcacgc agcaccttgc gtcggtgttg gagatctgca ccacatttcg gccccaccgg 26100
ttcttcacga tcttggcctt gctagactgc tccttcagcg cgcgctgccc gttttcgctc 26160
gtcacatcca tttcaatcac gtgctcctta tttatcataa tgcttccgtg tagacactta 26220
agctcgcctt cgatctcagc gcagcggtgc agccacaacg cgcagcccgt gggctcgtga 26280
tgcttgtagg tcacctctgc aaacgactgc aggtacgcct gcaggaatcg ccccatcatc 26340
gtcacaaagg tcttgttgct ggtgaaggtc agctgcaacc cgcggtgctc ctcgttcagc 26400
caggtcttgc atacggccgc cagagcttcc acttggtcag gcagtagttt gaagttcgcc 26460
tttagatcgt tatccacgtg gtacttgtcc atcagcgcgc gcgcagcctc catgcccttc 26520
tcccacgcag acacgatcgg cacactcagc gggttcatca ccgtaatttc actttccgct 26580
tcgctgggct cttcctcttc ctcttgcgtc cgcataccac gcgccactgg gtcgtcttca 26640
ttcagccgcc gcactgtgcg cttacctcct ttgccatgct tgattagcac cggtgggttg 26700
ctgaaaccca ccatttgtag cgccacatct tctctttctt cctcgctgtc cacgattacc 26760
tctggtgatg gcgggcgctc gggcttggga gaagggcgct tctttttctt cttgggcgca 26820
atggccaaat ccgccgccga ggtcgatggc cgcgggctgg gtgtgcgcgg caccagcgcg 26880
tcttgtgatg agtcttcctc gtcctcggac tcgatacgcc gcctcatccg ctttttgggg 26940
ggcgcccggg gaggcggcgg cgacggggac ggggacgaca cgtcctccat ggttggggga 27000
cgtcgcgccg caccgcgtcc gcgctcgggg gtggtttcgc gctgctcctc ttcccgactg 27060
gccatttcct tctcctatag gcagaaaaag atcatggagt cagtcgagaa gaaggacagc 27120
ctaaccgccc cctctgagtt cgccaccacc gcctccaccg atgccgccaa cgcgcctacc 27180
accttccccg tcgaggcacc cccgcttgag gaggaggaag tgattatcga gcaggaccca 27240
ggttttgtaa gcgaagacga cgaggaccgc tcagtaccaa cagaggataa aaagcaagac 27300
caggacaacg cagaggcaaa cgaggaacaa gtcgggcggg gggacgaaag gcatggcgac 27360
tacctagatg tgggagacga cgtgctgttg aagcatctgc agcgccagtg cgccattatc 27420
tgcgacgcgt tgcaagagcg cagcgatgtg cccctcgcca tagcggatgt cagccttgcc 27480
tacgaacgcc acctattctc accgcgcgta ccccccaaac gccaagaaaa cggcacatgc 27540
gagcccaacc cgcgcctcaa cttctacccc gtatttgccg tgccagaggt gcttgccacc 27600
tatcacatct ttttccaaaa ctgcaagata cccctatcct gccgtgccaa ccgcagccga 27660
gcggacaagc agctggcctt gcggcagggc gctgtcatac ctgatatcgc ctcgctcaac 27720

gaagtgccaa aaatctttga gggtcttgga cgcgacgaga agcgcgcggc aaacgctctg 27780
caacaggaaa acagcgaaaa tgaaagtcac tctggagtgt tggtggaact cgagggtgac 27840
aacgcgcgcc tagccgtact aaaacgcagc atcgaggtca cccactttgc ctacccggca 27900
cttaacctac cccccaaggt catgagcaca gtcatgagtg agctgatcgt gcgccgtgcg 27960
cagcccctgg agagggatgc aaatttgcaa gaacaaacag aggagggcct acccgcagtt 28020
ggcgacgagc agctagcgcg ctggcttcaa acgcgcgagc ctgccgactt ggaggagcga 28080
cgcaaactaa tgatggccgc agtgctcgtt accgtggagc ttgagtgcat gcagcggttc 28140
tttgctgacc cggagatgca gcgcaagcta gaggaaacat tgcactacac ctttcgacag 28200
ggctacgtac gccaggcctg caagatctcc aacgtggagc tctgcaacct ggtctcctac 28260
cttggaattt tgcacgaaaa ccgccttggg caaaacgtgc ttcattccac gctcaagggc 28320
gaggcgcgcc gcgactacgt ccgcgactgc gtttacttat ttctatgcta cacctggcag 28380
acggccatgg gcgtttggca gcagtgcttg gaggagtgca acctcaagga gctgcagaaa 28440
ctgctaaagc aaaacttgaa ggacctatgg acggccttca acgagcgctc cgtggccgcg 28500
cacctggcgg acatcatttt ccccgaacgc ctgcttaaaa ccctgcaaca gggtctgcca 28560
gacttcacca gtcaaagcat gttgcagaac tttaggaact ttatcctaga gcgctcagga 28620
atcttgcccg ccacctgctg tgcacttcct agcgactttg tgcccattaa gtaccgcgaa 28680
tgccctccgc cgctttgggg ccactgctac cttctgcagc tagccaacta ccttgcctac 28740
cactctgaca taatggaaga cgtgagcggt gacggtctac tggagtgtca ctgtcgctgc 28800
aacctatgca ccccgcaccg ctccctggtt tgcaattcgc agctgcttaa cgaaagtcaa 28860
attatcggta cctttgagct gcagggtccc tcgcctgacg aaaagtccgc ggctccgggg 28920
ttgaaactca ctccggggct gtggacgtcg gcttaccttc gcaaatttgt acctgaggac 28980
taccacgccc acgagattag gttctacgaa gaccaatccc gcccgccaaa tgcggagctt 29040
accgcctgcg tcattaccca gggccacatt cttggccaat tgcaagccat caacaaagcc 29100
cgccaagagt ttctgctacg aaagggacgg ggggtttact tggaccccca gtccggcgag 29160
gagctcaacc caatcccccc gccgccgcag ccctatcagc agcagccgcg ggcccttgct 29220
tcccaggatg gcacccaaaa agaagctgca gctgccgccg ccacccacgg acgaggagga 29280
atactgggac agtcaggcag aggaggtttt ggacgaggag gaggaggaca tgatggaaga 29340
ctgggagagc ctagacgagg aagcttccga ggtcgaagag gtgtcagacg aaacaccgtc 29400
accctcggtc gcattcccct cgccggcgcc ccagaaatcg gcaaccggtt ccagcatggc 29460
tacaacctcc gctcctcagg cgccgccggc actgcccgtt cgccgaccca accgtagatg 29520
ggacaccact ggaaccaggg ccggtaagtc caagcagccg ccgccgttag cccaagagca 29580
acaacagcgc caaggctacc gctcatggcg cgggcacaag aacgccatag ttgcttgctt 29640

gcaagactgt gggggcaaca tctccttcgc ccgccgcttt cttctctacc atcacggcgt 29700

ggccttcccc cgtaacatcc tgcattacta ccgtcatctc tacagcccat actgcaccgg 29760

cggcagcggc agcggcagca acagcagcgg ccacacagaa gcaaaggcga ccggatagca 29820

agactctgac aaagcccaag aaatccacag cggcggcagc agcaggagga ggagcgctgc 29880

gtctggcgcc caacgaaccc gtatcgaccc gcgagcttag aaacaggatt tttcccactc 29940

tgtatgctat atttcaacag agcaggggcc aagaacaaga gctgaaaata aaaaacaggt 30000

ctctgcgatc cctcacccgc agctgcctgt atcacaaaag cgaagatcag cttcggcgca 30060

cgctggaaga cgcggaggct ctcttcagta aatactgcgc gctgactctt aaggactagt 30120

ttcgcgccct ttctcaaatt taagcgcgaa aactacgtca tctccagcgg ccacacccgg 30180

cgccagcacc tgtcgtcagc gccattatga gcaaggaaat tcccacgccc tacatgtgga 30240

gttaccagcc acaaatggga cttgcggctg gagctgccca agactactca acccgaataa 30300

actacatgag cgcgggaccc cacatgatat cccgggtcaa cggaatccgc gcccaccgaa 30360

accgaattct cttggaacag gcggctatta ccaccacacc tcgtaataac cttaatcccc 30420

gtagttggcc cgctgccctg gtgtaccagg aaagtcccgc tcccaccact gtggtacttc 30480

ccagagacgc ccaggccgaa gttcagatga ctaactcagg ggcgcagctt gcgggcggct 30540

ttcgtcacag ggtgcggtcg cccgggcagg gtataactca cctgacaatc agagggcgag 30600

gtattcagct caacgacgag tcggtgagct cctcgcttgg tctccgtccg gacgggacat 30660

ttcagatcgg cggcgccggc cgtccttcat tcacgcctcg tcaggcaatc ctaactctgc 30720

agacctcgtc ctctgagccg cgctctggag gcattggaac tctgcaattt attgaggagt 30780

ttgtgccatc ggtctacttt aaccccttct cgggacctcc cggccactat ccggatcaat 30840

ttattcctaa ctttgacgcg gtaaaggact cggcggacgg ctacgactga atgttaagtg 30900

gagaggcaga gcaactgcgc ctgaaacacc tggtccactg tcgccgccac aagtgctttg 30960

cccgcgactc cggtgagttt tgctactttg aattgcccga ggatcatatc gagggcccgg 31020

cgcacggcgt ccggcttacc gcccagggag agcttgcccg tagcctgatt cgggagttta 31080

cccagcgccc cctgctagtt gagcgggaca ggggaccctg tgttctcact gtgatttgca 31140

actgtcctaa ccttggatta catcaagatc ctctagttat aactagagta cccggggatc 31200

ttattccctt taactaataa aaaaaaataa taaagcatca cttacttaaa atcagttagc 31260

aaatttctgt ccagtttatt cagcagcacc tccttgccct cctcccagct ctggtattgc 31320

agcttcctcc tggctgcaaa ctttctccac aatctaaatg gaatgtcagt ttcctcctgt 31380

tcctgtccat ccgcacccac tatcttcatg ttgttgcaga tgaagcgcgc aagaccgtct 31440

gaagatacct tcaaccccgt gtatccatat gacacggaaa ccggtcctcc aactgtgcct 31500

tttcttactc ctccctttgt atcccccaat gggtttcaag agagtccccc tggggtactc 31560

tctttgcgcc tatccgaacc tctagttacc tccaatggca tgcttgcgct caaaatgggc 31620

aacggcctct ctctggacga ggccggcaac cttacctccc aaaatgtaac cactgtgagc 31680

ccacctctca aaaaaaccaa gtcaaacata aacctggaaa tatctgcacc cctcacagtt 31740

acctcagaag ccctaactgt ggctgccgcc gcacctctaa tggtcgcggg caacacactc 31800

accatgcaat cacaggcccc gctaaccgtg cacgactcca aacttagcat tgccacccaa 31860

ggacccctca cagtgtcaga aggaaagcta gccctgcaaa catcaggccc cctcaccacc 31920

accgatagca gtacccttac tatcactgcc tcaccccctc taactactgc cactggtagc 31980

ttgggcattg acttgaaaga gcccatttat acacaaaatg gaaaactagg actaaagtac 32040

ggggctcctt tgcatgtaac agacgaccta aacactttga ccgtagcaac tggtccaggt 32100

gtgactatta ataatacttc cttgcaaact aaagttactg gagccttggg ttttgattca 32160

caaggcaata tgcaacttaa tgtagcagga ggactaagga ttgattctca aaacagacgc 32220

cttatacttg atgttagtta tccgtttgat gctcaaaacc aactaaatct aagactagga 32280

cagggccctc tttttataaa ctcagcccac aacttggata ttaactacaa caaaggcctt 32340

tacttgttta cagcttcaaa caattccaaa aagcttgagg ttaacctaag cactgccaag 32400

gggttgatgt ttgacgctac agccatagcc attaatgcag gagatgggct tgaatttggt 32460

tcacctaatg caccaaacac aaatcccctc aaaacaaaaa ttggccatgg cctagaattt 32520

gattcaaaca aggctatggt tcctaaacta ggaactggcc ttagttttga cagcacaggt 32580

gccattacag taggaaacaa aaataatgat aagctaactt tgtggaccac accagctcca 32640

tctcctaact gtagactaaa tgcagagaaa gatgctaaac tcactttggt cttaacaaaa 32700

tgtggcagtc aaatacttgc tacagtttca gttttggctg ttaaaggcag tttggctcca 32760

atatctggaa cagttcaaag tgctcatctt attataagat ttgacgaaaa tggagtgcta 32820

ctaaacaatt ccttcctgga cccagaatat tggaacttta gaaatggaga tcttactgaa 32880

ggcacagcct atacaaacgc tgttggattt atgcctaacc tatcagctta tccaaaatct 32940

cacggtaaaa ctgccaaaag taacattgtc agtcaagttt acttaaacgg agacaaaact 33000

aaacctgtaa cactaaccat tacactaaac ggtacacagg aaacaggaga cacaactcca 33060

agtgcatact ctatgtcatt ttcatgggac tggtctggcc acaactacat taatgaaata 33120

tttgccacat cctcttacac tttttcatac attgcccaag aataaagaat cgtttgtgtt 33180

atgtttcaac gtgtttattt ttcaattgca gaaaatttca agtcattttt cattcagtag 33240

tatagcccca ccaccacata gcttatacag atcaccgtac cttaatcaaa ctcacagaac 33300

cctagtattc aacctgccac ctccctccca acacacagag tacacagtcc tttctccccg 33360

gctggcctta aaaagcatca tatcatgggt aacagacata ttcttaggtg ttatattcca 33420

cacggtttcc tgtcgagcca aacgctcatc aagtgatatt aataaactcc ccgggcagct 33480
cacttaagtt catgtcgctg tccagctgct gagccacagg ctgctgtcca acttgcggtt 33540
gcttaacggg cggcgaagga gaagtccacg cctacatggg gggagagtca taatcgtgca 33600
tcaggatagg gcggtggtgc tgcagcagcg cgcgaataaa ctgctgccgc cgccgctccg 33660
tcctgcagga atacaacatg gcagtggtct cctcagcgat gattcgcacc gcccgcagca 33720
taaggcgctt gtcctccggg cacagcagcg caccctgatc tcacttaaat cagcacagta 33780
actgcagcac agcaccacaa tattgttcaa aatcccacag tgcaaggcgc tgtatccaaa 33840
gctcatggcg gggaccacag aacccacgtg gccatcatac cacaagcgca ggtagattaa 33900
gtggcgaccc ctcataaaca cgctggacat aaacattacc tcttttggca tgttgtaatt 33960
caccacctcc cggtaccata taaacctctg attaaacatg gcgccatcca ccaccatcct 34020
aaaccagctg gccaaaacct gccccgccgg gntatacact gcagggaacc gggacttgga 34080
caatgacaag tgggagagcc caggactcgt aaccatggat catcatgctc gtcatgatat 34140
caatgttggc acaacacagg cacacgtgca tacacttcct caggattaca agctcctccc 34200
gcgttagaac catatcccag ggaacaaccc attcctgaat cagcgtaaat cccacactgc 34260
agggaagacc tcgcacgtaa ctcacgttgt gcattgtcaa agtgttacat tcgggcagca 34320
gcggatgatc ctccagtatg gtagcgcggg tttctgtctc aaaaggaggt agacgatccc 34380
tactgtacgg agtgcgccga gacaaccgag atcgtgttgg tcgtagtgtc atgccaaatg 34440
gaacgccgga cgtagtcata tttcctgaag caaaaccagg tgcgggcgtg acaaacagat 34500
ctgcgtctcc ggtctcgccg cttagatcgc tctgtgtagt agttgtagta tatccactct 34560
ctcaaagcat ccaggcgccc cctggcttcg ggttctatgt aaactccttc atgcgccgct 34620
gccctgataa catccaccac cgcagaataa gccacaccca gccaacctac acattcgttc 34680
tgcgagtcac acacgggagg agcgggaaga gctggaagaa ccatgttttt ttttttattc 34740
caaaagatta tccaaaacct caaaatgaag atctattaag tgaacgcgct cccctccggt 34800
ggcgtggtca aactctacag ccaaagaaca gataatggca tttgtaagat gttgcacaat 34860
ggcttccaaa aggcaaacgg ccctcacgtc caagtggacg taaaggctaa acccttcagg 34920
gtgaatctcc tctataaaca ttccagcacc ttcaaccatg cccaaataat tctcatctcg 34980
ccaccttctc aatatatctc taagcaaatc ccgaatattt aagtccgggc cattgtaaaa 35040
aatttggctc cagagcgccc tccaccttca gcctcaagca gcgaatcatg attgcaaaaa 35100
ttcaggttcc tcacagacct gtataagatt caaaagcgga acattaacaa aaataccgcg 35160
atcccgtagg tcccttcgca gggccagctg aacataatcg tgcaggtctg cacggaccag 35220
cgcggccact tccccgccag gaaccatgac aaaagaaccc acactgatta tgacacgcat 35280

actcggagct atgctaacca gcgtagcccc gatgtaagct tgttgcatgg gcggcgatat 35340
aaaatgcaag gtgctgctca aaaaatcagg caaagcctcg cgcaaaaaag aaagcacatc 35400
gtagtcatgc tcatgcagat aaaggcaggt aagctccgga accaccacag aaaaagacac 35460
catttttctc tcaaacatgt ctgcgggttt ctgcataaac acaaaataaa ataacaaaaa 35520
aacatttaaa cattagaagc ctgtcttaca acaggaaaaa caacccttat aagcataaga 35580
cggactacgg ccatgccggc gtgaccgtaa aaaaactggt caccgtgatt aaaaagcacc 35640
accgacagct cctcggtcag tccggagtca taatgtaaga ctcggtaaac acatcaggtt 35700
gattcacatc ggtcagtgtt aaaaagcgac cgaaatagcc cgggggaata catacccgca 35760
ggcgtagaga caacattaca gcccccatag gaggtataac aaaattaata ggagagaaaa 35820
acacataaac acctgaaaaa ccctcctgcc taggcaaaat agcaccctcc cgctccagaa 35880
caacatacag cgcttcacag cggcagccta acagtcagcc ttaccagtaa aaaagaaaac 35940
ctattaaaaa aacaccactc gacacggcac cagctcaatc agtcacagtg taaaaaaggg 36000
ccaagtgcag agcgagtata tataggacta aaaaatgacg taacggttaa agtccacaaa 36060
aaacacccag aaaaccgcac gcgaacctac gcccagaaac gaaagccaaa aaacccacaa 36120
cttcctcaaa tcgtcacttc cgttttccca cgttacgtaa cttcccattt taagaaaact 36180
acaattccca acacatacaa gttactccgc cctaaaacct acgtcacccg ccccgttccc 36240
acgccccgcg ccacgtcaca aactccaccc cctcattatc atattggctt caatccaaaa 36300
taaggtatat tattgatgat gnnttaatta agggcggccg cacgggccat cgatggatcc 36360
gcggaagctt ctcgacccat tctcatgttt gacagcttat catcgaattt ctgccattca 36420
tccgcttatt atcacttatt caggcgtagc aaccaggcgt ttaagggcac caataactgc 36480
cttaaaaaaa ttacgccccg ccctgccact catcgcagta ctgttgtaat tcattaagca 36540
ttctgccgac atggaagcca tcacagacgg catgatgaac ctgaatcgcc agcggcatca 36600
gcaccttgtc gccttgcgta taatatttgc ccatggtgaa aacgggggcg aagaagttgt 36660
ccatattggc cacgtttaaa tcaaaactgg tgaaactcac ccagggattg gctgagacga 36720
aaaacatatt ctcaataaac cctttaggga aataggccag gttttcaccg taacacgcca 36780
catcttgcga atatatgtgt agaaactgcc ggaaatcgtc gtggtattca ctccagagcg 36840
atgaaaacgt ttcagtttgc tcatggaaaa cggtgtaaca agggtgaaca ctatcccata 36900
tcaccagctc accgtctttc attgccatac ggaattccgg atgagcattc atcaggcggg 36960
caagaatgtg aataaaggcc ggataaaact tgtgcttatt tttctttacg gtctttaaaa 37020
aggccgtaat atccagctga acggtctggt tataggtaca ttgagcaact gactgaaatg 37080
cctcaaaatg ttctttacga tgccattggg atatatcaac ggtggtatat ccagtgattt 37140
ttttctccat tttagcttcc ttagctcctg aaaatctcga taactcaaaa aatacgcccg 37200

gtagtgatct tatttcatta tggtgaaagt tggaacctct tacgtgccga tcaacgtctc 37260

attttcgcca aaagttggcc cagggcttcc cggtatcaac agggacacca ggatttattt 37320

attctgcgaa gtgatcttcc gtcacaggta tttattcgcg ataagctcat ggagcggcgt 37380

aaccgtcgca caggaaggac agagaaagcg cggatctggg aagtgacgga cagaacggtc 37440

aggacctgga ttggggaggc ggttgccgcc gctgctgctg acggtgtgac gttctctgtt 37500

ccggtcacac cacatacgtt ccgccattcc tatgcgatgc acatgctgta tgccggtata 37560

ccgctgaaag ttctgcaaag cctgatggga cataagtcca tcagttcaac ggaagtctac 37620

acgaaggttt ttgcgctgga tgtggctgcc cggcaccggg tgcagtttgc gatgccggag 37680

tctgatgcgg ttgcgatgct gaaacaatta tcctgagaat aaatgccttg gcctttatat 37740

ggaaatgtgg aactgagtgg atatgctgtt tttgtctgtt aaacagagaa gctggctgtt 37800

atccactgag aagcgaacga aacagtcggg aaaatctccc attatcgtag agatccgcat 37860

tattaatctc aggagcctgt gtagcgttta taggaagtag tgttctgtca tgatgcctgc 37920

aagcggtaac gaaaacgatt tgaatatgcc ttcaggaaca atagaaatct tcgtgcggtg 37980

ttacgttgaa gtggagcgga ttatgtcagc aatggacaga acaacctaat gaacacagaa 38040

ccatgatgtg gtctgtcctt ttacagccag taggctcgcc gcagtcgagc gacggcgaag 38100

ccctcgagtg agcgaggaag caccagggaa cagcacttat atattctgct tacacacgat 38160

gcctgaaaaa acttcccttg gggttatcca cttatccacg gggatatttt tataattatt 38220

ttttttatag tttttagatc ttctttttta gagcgccttg taggccttta tccatgctgg 38280

ttctagagaa ggtgttgtga caaattgccc tttcagtgtg acaaatcacc ctcaaatgac 38340

agtcctgtct gtgacaaatt gcccttaacc ctgtgacaaa ttgccctcag aagaagctgt 38400

tttttcacaa agttatccct gcttattgac tcttttttat ttagtgtgac aatctaaaaa 38460

cttgtcacac ttcacatgga tctgtcatgg cggaaacagc ggttatcaat cacaagaaac 38520

gtaaaaatag cccgcgaatc gtccagtcaa acgacctcac tgaggcggca tatagtctct 38580

cccgggatca aaaacgtatg ctgtatctgt tcgttgacca gatcagaaaa tctgatggca 38640

ccctacagga acatgacggt atctgcgaga tccatgttgc taaatatgct gaaatattcg 38700

gattgacctc tgcggaagcc agtaaggata tacggcaggc attgaagagt ttcgcgggga 38760

aggaagtggt tttttatcgc cctgaagagg atgccggcga tgaaaaaggc tatgaatctt 38820

ttccttggtt tatcaaacgt gcgcacagtc catccagagg gctttacagt gtacatatca 38880

acccatatct cattcccttc tttatcgggt tacagaaccg gtttacgcag tttcggctta 38940

gtgaaacaaa agaaatcacc aatccgtatg ccatgcgttt atacgaatcc ctgtgtcagt 39000

atcgtaagcc ggatggctca ggcatcgtct ctctgaaaat cgactggatc atagagcgtt 39060

accagctgcc tcaaagttac cagcgtatgc ctgacttccg ccgccgcttc ctgcaggtct 39120

gtgttaatga gatcaacagc agaactccaa tgcgcctctc atacattgag aaaaagaaag 39180

gccgccagac gactcatatc gtattttcct tccgcgatat cacttccatg acgacaggat 39240

agtctgaggg ttatctgtca cagatttgag ggtggttcgt cacatttgtt ctgacctact 39300

gagggtaatt tgtcacagtt ttgctgtttc cttcagcctg catggatttt ctcatacttt 39360

ttgaactgta atttttaagg aagccaaatt tgagggcagt ttgtcacagt tgatttcctt 39420

ctctttccct tcgtcatgtg acctgatatc gggggttagt tcgtcatcat tgatgagggt 39480

tgattatcac agtttattac tctgaattgg ctatccgcgt gtgtacctct acctggagtt 39540

tttcccacgg tggatatttc ttcttgcgct gagcgtaaga gctatctgac agaacagttc 39600

ttctttgctt cctcgccagt tcgctcgcta tgctcggtta cacggctgcg gcgagcgcta 39660

gtgataataa gtgactgagg tatgtgctct tcttatctcc ttttgtagtg ttgctcttat 39720

tttaaacaac tttgcggttt tttgatgact ttgcgatttt gttgttgctt tgcagtaaat 39780

tgcaagattt aataaaaaaa cgcaaagcaa tgattaaagg atgttcagaa tgaaactcat 39840

ggaaacactt aaccagtgca taaacgctgg tcatgaaatg acgaaggcta tcgccattgc 39900

acagtttaat gatgacagcc cggaagcgag gaaaataacc cggcgctgga gaataggtga 39960

agcagcggat ttagttgggg tttcttctca ggctatcaga gatgccgaga aagcagggcg 40020

actaccgcac ccggatatgg aaattcgagg acgggttgag caacgtgttg gttatacaat 40080

tgaacaaatt aatcatatgc gtgatgtgtt tggtacgcga ttgcgacgtg ctgaagacgt 40140

atttccaccg gtgatcgggg ttgctgccca taaaggtggc gtttacaaaa cctcagtttc 40200

tgttcatctt gctcaggatc tggctctgaa ggggctacgt gttttgctcg tggaaggtaa 40260

cgacccccag ggaacagcct caatgtatca cggatgggta ccagatcttc atattcatgc 40320

agaagacact ctcctgcctt tctatcttgg ggaaaaggac gatgtcactt atgcaataaa 40380

gcccacttgc tggccggggc ttgacattat tccttcctgt ctggctctgc accgtattga 40440

aactgagtta atgggcaaat ttgatgaagg taaactgccc accgatccac acctgatgct 40500

ccgactggcc attgaaactg ttgctcatga ctatgatgtc atagttattg acagcgcgcc 40560

taacctgggt atcggcacga ttaatgtcgt atgtgctgct gatgtgctga ttgttcccac 40620

gcctgctgag ttgtttgact acacctccgc actgcagttt ttcgatatgc ttcgtgatct 40680

gctcaagaac gttgatctta aagggttcga gcctgatgta cgtattttgc ttaccaaata 40740

cagcaatagt aatggctctc agtccccgtg gatggaggag caaattcggg atgcctgggg 40800

aagcatggtt ctaaaaaatg ttgtacgtga aacggatgaa gttggtaaag gtcagatccg 40860

gatgagaact gtttttgaac aggccattga tcaacgctct tcaactggtg cctggagaaa 40920

tgctctttct atttgggaac ctgtctgcaa tgaaattttc gatcgtctga ttaaaccacg 40980

ctgggagatt agataatgaa gcgtgcgcct gttattccaa aacatacgct caatactcaa 41040

ccggttgaag atacttcgtt atcgacacca gctgccccga tggtggattc gttaattgcg 41100

cgcgtaggag taatggctcg cggtaatgcc attactttgc ctgtatgtgg tcgggatgtg 41160

aagtttactc ttgaagtgct ccggggtgat agtgttgaga agacctctcg ggtatggtca 41220

ggtaatgaac gtgaccagga gctgcttact gaggacgcac tggatgatct catcccttct 41280

tttctactga ctggtcaaca gacaccggcg ttcggtcgaa gagtatctgg tgtcatagaa 41340

attgccgatg ggagtcgccg tcgtaaagct gctgcactta ccgaaagtga ttatcgtgtt 41400

ctggttggcg agctggatga tgagcagatg gctgcattat ccagattggg taacgattat 41460

cgcccaacaa gtgcttatga acgtggtcag cgttatgcaa gccgattgca gaatgaattt 41520

gctggaaata tttctgcgct ggctgatgcg gaaaatattt cacgtaagat tattacccgc 41580

tgtatcaaca ccgccaaatt gcctaaatca gttgttgctc ttttttctca ccccggtgaa 41640

ctatctgccc ggtcaggtga tgcacttcaa aaagccttta cagataaaga ggaattactt 41700

aagcagcagg catctaacct tcatgagcag aaaaaagctg ggtgatattt tgaagctgaa 41760

gaagttatca ctcttttaac ttctgtgctt aaaacgtcat ctgcatcaag aactagttta 41820

agctcacgac atcagtttgc tcctggagcg acagtattgt ataagggcga taaaatggtg 41880

cttaacctgg acaggtctcg tgttccaact gagtgtatag agaaaattga ggccattctt 41940

aaggaacttg aaaagccagc accctgatgc gaccacgttt tagtctacgt ttatctgtct 42000

ttacttaatg tcctttgtta caggccagaa agcataactg gcctgaatat tctctctggg 42060

cccactgttc cacttgtatc gtcggtctga taatcagact gggaccacgg tcccactcgt 42120

atcgtcggtc tgattattag tctgggacca cggtcccact cgtatcgtcg gtctgattat 42180

tagtctggga ccacggtccc actcgtatcg tcggtctgat aatcagactg ggaccacggt 42240

cccactcgta tcgtcggtct gattattagt ctgggaccat ggtcccactc gtatcgtcgg 42300

tctgattatt agtctgggac cacggtccca ctcgtatcgt cggtctgatt attagtctgg 42360

aaccacggtc ccactcgtat cgtcggtctg attattagtc tgggaccacg gtcccactcg 42420

tatcgtcggt ctgattatta gtctgggacc acgatcccac tcgtgttgtc ggtctgatta 42480

tcggtctggg accacggtcc cacttgtatt gtcgatcaga ctatcagcgt gagactacga 42540

ttccatcaat gcctgtcaag ggcaagtatt gacatgtcgt cgtaacctgt agaacggagt 42600

aacctcggtg tgcggttgta tgcctgctgt ggattgctgc tgtgtcctgc ttatccacaa 42660

cattttgcgc acggttatgt ggacaaaata cctggttacc caggccgtgc cggcacgtta 42720

accgggctgc atccgatgca agtgtgtcgc tgtcgagttt aaacatgcat ccttaattaa 42780

<210> 6

<211>  3002
<212>  DNA
<213>  Artificial

<220>
<223>  synthetic

<400>  6
catcatcaat aatatacctt attttggatt gaagccaata tgataatgag ggggtggagt      60

ttgtgacgtg gcgcggggcg tgggaacggg gcgggtgacg tagtagtgtg gcggaagtgt     120

gatgttgcaa gtgtggcgga acacatgtaa gcgacggatg tggcaaaagt gacgtttttg     180

gtgtgcgccg gtgtacacag gaagtgacaa ttttcgcgcg gttttaggcg gatgttgtag     240

taaatttggg cgtaaccgag taagatttgg ccattttcgc gggaaaactg aataagagga     300

agtgaaatct gaataatttt gtgttactca tagcgcgtaa tactggtacc ccccagtggc     360

aagacgcgca ggcaaaacgc accacgactg gatccgaatt cgaagaccac ggtgtttcgt     420

cctttccaca agatatataa agccaagaaa tcgaaatact ttcaagttac ggtaagcata     480

tgatagtcca ttttaaaaca taattttaaa actgcaaact acccaagaaa ttattacttt     540

ctacgtcacg tattttgtac taatatcttt gtgtttacag tcaaattaat tctaattatc     600

tttctaacag ccttgtatcg tatatgcaaa tatgaaggaa tcatgggaaa taggccctct     660

tcctgcccga ccttggcgcg cgctcggcgc gcggtcacgc tccgtcacgt ggtgcgtttt     720

gcctgcgcgt cttgccactg gggggtaccc cgaatcacta gatctgagct cggcctggtg     780

atgatggcgg gatcgttgta tatttcttga caccttttcg gcatcgccct aaaattcggc     840

gtcctcatat tgtgtgagga cgttttatta cgtgtttacg aagcaaaagc taaaaccagg     900

agctatttaa tggcaacaac cggtaccatg attacgccaa gctacgtaat acgactcact     960

agtggaagtt cctattctct agaaagtata ggaacttcct taagtgagtc gtattacgga    1020

ctggccgtcg ttttacaacg tcgtgactgg gaaaaccctg gcgttaccca acttaatcgc    1080

cttgcagcac atcccccttt cgccagctgg cgtaatagcg aagaggcccg caccgatcgc    1140

ccttcccaac agttgcgcag cctgaatggc gaatggcgct tcgcttggta agctagctaa    1200

ggcttaagga ggacaatcat ggcaacagtt aaccagctgg tacgcaaacc acgtgctcgc    1260

aaagttgcga aaagcaacgt gcctgcgctg gaagcatgcc cgcaaaaacg tggcgtatgt    1320

actcgtgtat atactaccac tcctaaaaaa ccgaactccg cgctgcgtaa agtatgccgt    1380

gttcgtctgt ctaacggttt cgaagtgact tcctacatcg gtggtgaagg tcacaacctg    1440

caggagcact ccgtgatcct gatccgtggc ggtcgtgtta aagacctccc gggtgttcgt    1500

taccacaccg tacgtggtgc gcttgactgc tccggcgtta aagaccgtaa gcaggctcgt    1560

tccaagtatg gcgtgaagcg tcctaaggct taactagcac gcgccatgtc agccgttaag    1620

tgttcctgtg tcactcaaaa ttgctttgag aggctctaag ggcttctcag tgcgttacat    1680

ccctggcttg ttgtccacaa ccgttaaacc ttaaaagctt taaaagcctt atatattctt 1740

tttttcttta taaaacttaa aaccttagag gctatttaag ttgctgattt atattaattt 1800

tattgttcaa acatgagagc ttagtacgtg aaacatgaga gcttagtacg ttagccatga 1860

gagcttagta cgttagccat gagggtttag ttcgttaaac atgagagctt agtacgttaa 1920

acatgagagc ttagtacgtg aaacatgaga gcttagtacg tactatcaac aggttgaact 1980

gctgatcttc agatcctcta cgccggacgc atcgtggccg gatccgattt attcaacaaa 2040

gccacgttgt gtctcaaaat ctctgatgtt acattgcaca agataaaaat atatcatcat 2100

gaacaataaa actgtctgct tacataaaca gtaatacaag gggtgttatg agccatattc 2160

aacgggaaac gtcttgctcg aggccgcgat taaattccaa catggatgct gatttatatg 2220

ggtataaatg ggctcgcgat aatgtcgggc aatcaggtgc gacaatctat cgattgtatg 2280

ggaagcccga tgcgccagag ttgtttctga aacatggcaa aggtagcgtt gccaatgatg 2340

ttacagatga gatggtcaga ctaaactggc tgacggaatt tatgcctctt ccgaccatca 2400

agcattttat ccgtactcct gatgatgcat ggttactcac cactgcgatc cccgggaaaa 2460

cagcattcca ggtattagaa gaatatcctg attcaggtga aaatattgtt gatgcgctgg 2520

cagtgttcct gcgccggttg cattcgattc ctgtttgtaa ttgtcctttt aacagcgatc 2580

gcgtatttcg tctcgctcag gcgcaatcac gaatgaataa cggtttggtt gatgcgagtg 2640

attttgatga cgagcgtaat ggctggcctg ttgaacaagt ctggaaagaa atgcataagc 2700

ttttgccatt ctcaccggat tcagtcgtca ctcatggtga tttctcactt gataacctta 2760

tttttgacga ggggaaatta ataggttgta ttgatgttgg acgagtcgga atcgcagacc 2820

gataccagga tcttgccatc ctatggaact gcctcggtga gttttctcct tcattacaga 2880

aacggctttt tcaaaaatat ggtattgata atcctgatat gaataaattg cagtttcatt 2940

tgatgctcga tgagtttttc taatcagaat tggttaattg gttgtaacac tggcttaatt 3000

aa 3002

<210> 7
<211> 34
<212> DNA
<213> Artificial

<220>
<223> synthetic

<400> 7
gaagttccta ttctctagaa agtataggaa cttc 34

<210> 8
<211> 48
<212> DNA

<213>  Artificial

<220>
<223>  synthetic

<400>  8
gaagttccta ttccgaagtt cctattctct agaaagtata ggaacttc                48


<210>  9
<211>  3461
<212>  DNA
<213>  Artificial

<220>
<223>  synthetic


<220>
<221>  misc_feature
<222>  (341)..(344)
<223>  n is a, c, g, or t

<400>  9
catcatcaat aatatacctt attttggatt gaagccaata tgataatgag ggggtggagt      60

ttgtgacgtg gcgcggggcg tgggaacggg gcgggtgacg tagtagtgtg gcggaagtgt     120

gatgttgcaa gtgtggcgga acacatgtaa gcgacggatg tggcaaaagt gacgtttttg     180

gtgtgcgccg gtgtacacag gaagtgacaa ttttcgcgcg gttttaggcg gatgttgtag     240

taaatttggg cgtaaccgag taagatttgg ccattttcgc gggaaaactg aataagagga     300

agtgaaatct gaataatttt gtgttactca tagcgcgtaa nnnntaatag taatcaatta     360

cggggtcatt agttcatagc ccatatatgg agttccgcgt tacataactt acggtaaatg     420

gcccgcctgg ctgaccgccc aacgaccccc gcccattgac gtcaataatg acgtatgttc     480

ccatagtaac gccaataggg actttccatt gacgtcaatg ggtggagtat ttacggtaaa     540

ctgcccactt ggcagtacat caagtgtatc atatgccaag tacgccccct attgacgtca     600

atgacggtaa atggcccgcc tggcattatg cccagtacat gaccttatgg gactttccta     660

cttggcagta catctacgta ttagtcatcg ctattaccat ggtgatgcgg ttttggcagt     720

acatcaatgg gcgtggatag cggtttgact cacggggatt tccaagtctc caccccattg     780

acgtcaatgg gagtttgttt tggcaccaaa atcaacggga ctttccaaaa tgtcgtaaca     840

actccgcccc attgacgcaa atgggcggta ggcgtgtacg gtgggaggtc tatataagca     900

gagctggttt agtgaaccgt cagatccgct agcgctaccg gactcagatc tcgagctcaa     960

gcttcgaatt ctgcagtcga cggtaccgcg ggcccgggat ccaccggtcg ccaccatggt    1020

gagcaagggc gaggagctgt tcaccggggt ggtgcccatc ctggtcgagc tggacggcga    1080

cgtaaacggc cacaagttca gcgtgtccgg cgagggcgag ggcgatgcca cctacggcaa    1140

gctgaccctg aagttcatct gcaccaccgg caagctgccc gtgccctggc ccaccctcgt    1200

gaccaccctg acctacggcg tgcagtgctt cagccgctac cccgaccaca tgaagcagca 1260
cgacttcttc aagtccgcca tgcccgaagg ctacgtccag gagcgcacca tcttcttcaa 1320
ggacgacggc aactacaaga cccgcgccga ggtgaagttc gagggcgaca ccctggtgaa 1380
ccgcatcgag ctgaagggca tcgacttcaa ggaggacggc aacatcctgg ggcacaagct 1440
ggagtacaac tacaacagcc acaacgtcta tatcatggcc gacaagcaga agaacggcat 1500
caaggtgaac ttcaagatcc gccacaacat cgaggacggc agcgtgcagc tcgccgacca 1560
ctaccagcag aacaccccca tcggcgacgg ccccgtgctg ctgcccgaca accactacct 1620
gagcacccag tccgccctga gcaaagaccc caacgagaag cgcgatcaca tggtcctgct 1680
ggagttcgtg accgccgccg ggatcactct cggcatggac gagctgtaca agtaaagcgg 1740
ccgcgactct agatcataat cagccatacc acatttgtag aggttttact tgctttaaaa 1800
aacctcccac acctccccct gaacctgaaa cataaaatga atgcaattgt tgttgttaac 1860
ttgtttattg cagcttataa tggttacaaa taaagcaata gcatcacaaa tttcacaaat 1920
aaagcatttt tttcactgca ttctagttgt ggtttgtcca aactcatcaa tgtatcttaa 1980
atcgaattca agcttgtcga ctcgaagatc tgagctcacg cgtgaagttc ctattctcta 2040
gaaagtatag gaacttcaat tcccatgtca gccgttaagt gttcctgtgt cactcaaaat 2100
tgctttgaga ggctctaagg gcttctcagt gcgttacatc cctggcttgt tgtccacaac 2160
cgttaaacct taaaagcttt aaaagcctta tatattcttt tttttcttat aaaacttaaa 2220
accttagagg ctatttaagt tgctgattta tattaatttt attgttcaaa catgagagct 2280
tagtacgtga aacatgagag cttagtacgt tagccatgag agcttagtac gttagccatg 2340
agggtttagt tcgttaaaca tgagagctta gtacgttaaa catgagagct tagtacgtga 2400
aacatgagag cttagtacgt actatcaaca ggttgaactg ctgatcttca gatcctctac 2460
gccggacgca tcgtggccgg atccgattta ttcaacaaag ccacgttgtg tctcaaaatc 2520
tctgatgtta cattgcacaa gataaaaata tatcatcatg aacaataaaa ctgtctgctt 2580
acataaacag taatacaagg ggtgttatga gccatattca acgggaaacg tcttgctcga 2640
ggccgcgatt aaattccaac atggatgctg atttatatgg gtataaatgg gctcgcgata 2700
atgtcgggca atcaggtgcg acaatctatc gattgtatgg gaagcccgat gcgccagagt 2760
tgtttctgaa acatggcaaa ggtagcgttg ccaatgatgt tacagatgag atggtcagac 2820
taaactggct gacggaattt atgcctcttc cgaccatcaa gcattttatc cgtactcctg 2880
atgatgcatg gttactcacc actgcgatcc ccgggaaaac agcattccag gtattagaag 2940
aatatcctga ttcaggtgaa aatattgttg atgcgctggc agtgttcctg cgccggttgc 3000
attcgattcc tgtttgtaat tgtcctttta acagcgatcg cgtatttcgt ctcgctcagg 3060

cgcaatcacg aatgaataac ggtttggttg atgcgagtga ttttgatgac gagcgtaatg 3120

gctggcctgt tgaacaagtc tggaaagaaa tgcataagct tttgccattc tcaccggatt 3180

cagtcgtcac tcatggtgat ttctcacttg ataaccttat ttttgacgag gggaaattaa 3240

taggttgtat tgatgttgga cgagtcggaa tcgcagaccg ataccaggat cttgccatcc 3300

tatggaactg cctcggtgag ttttctcctt cattacagaa acggcttttt caaaaatatg 3360

gtattgataa tcctgatatg aataaattgc agtttcattt gatgctcgat gagtttttct 3420

aatcagaatt ggttaattgg ttgtaacact ggcttaatta a 3461

<210> 10
<211> 342
<212> DNA
<213> Artificial

<220>
<223> synthetic

<400> 10
catcatcaat aatatacctt attttggatt gaagccaata tgataatgag ggggtggagt 60

ttgtgacgtg gcgcggggcg tgggaacggg gcgggtgacg tagtagtgtg gcggaagtgt 120

gatgttgcaa gtgtggcgga acacatgtaa gcgacggatg tggcaaaagt gacgtttttg 180

gtgtgcgccg gtgtacacag gaagtgacaa ttttcgcgcg gttttaggcg gatgttgtag 240

taaatttggg cgtaaccgag taagatttgg ccattttcgc gggaaaactg aataagagga 300

agtgaaatct gaataatttt gtgttactca tagcgcgtaa ta 342

<210> 11
<211> 40322
<212> DNA
<213> Artificial

<220>
<223> synthetic

<400> 11
gaagttccta ttccgaagtt cctattctct agaaagtata ggaacttcct taagtgagtc 60

gtattacgga ctggccgtcg ttttacaacg tcgtgactgg gaaaaccctg gcgttaccca 120

acttaatcgc cttgcagcac atcccccttt cgccagctgg cgtaatagcg aagaggcccg 180

caccgatcgc ccttcccaac agttgcgcag cctgaatggc gaatggcgct tcgcttggta 240

agctagctaa ggcttaagga ggacaatcat ggcaacagtt aaccagctgg tacgcaaacc 300

acgtgctcgc aaagttgcga aaagcaacgt gcctgcgctg gaagcatgcc cgcaaaaacg 360

tggcgtatgt actcgtgtat atactaccac tcctaaaaaa ccgaactccg cgctgcgtaa 420

agtatgccgt gttcgtctgt ctaacggttt cgaagtgact tcctacatcg gtggtgaagg 480

tcacaacctg caggagcact ccgtgatcct gatccgtggc ggtcgtgtta aagacctccc 540

gggtgttcgt taccacaccg tacgtggtgc gcttgactgc tccggcgtta aagaccgtaa 600
gcaggctcgt tccaagtatg gcgtgaagcg tcctaaggct taactagcac gcgccatgtc 660
agccgttaag tgttcctgtg tcactcaaaa ttgctttgag aggctctaag ggcttctcag 720
tgcgttacat ccctggcttg ttgtccacaa ccgttaaacc ttaaaagctt taaaagcctt 780
atatattctt ttttttctta taaaacttaa aaccttagag gctatttaag ttgctgattt 840
atattaattt tattgttcaa acatgagagc ttagtacgtg aaacatgaga gcttagtacg 900
ttagccatga gagcttagta cgttagccat gagggtttag ttcgttaaac atgagagctt 960
agtacgttaa acatgagagc ttagtacgtg aaacatgaga gcttagtacg tactatcaac 1020
aggttgaact gctgatcttc agatcctcta cgccggacgc atcgtggccg gatccgattt 1080
attcaacaaa gccacgttgt gtctcaaaat ctctgatgtt acattgcaca agataaaaat 1140
atatcatcat gaacaataaa actgtctgct tacataaaca gtaatacaag gggtgttatg 1200
agccatattc aacgggaaac gtcttgctcg aggccgcgat taaattccaa catggatgct 1260
gatttatatg ggtataaatg ggctcgcgat aatgtcgggc aatcaggtgc gacaatctat 1320
cgattgtatg ggaagcccga tgcgccagag ttgtttctga aacatggcaa aggtagcgtt 1380
gccaatgatg ttacagatga gatggtcaga ctaaactggc tgacggaatt tatgcctctt 1440
ccgaccatca agcattttat ccgtactcct gatgatgcat ggttactcac cactgcgatc 1500
cccgggaaaa cagcattcca ggtattagaa gaatatcctg attcaggtga aaatattgtt 1560
gatgcgctgg cagtgttcct gcgccggttg cattcgattc ctgtttgtaa ttgtcctttt 1620
aacagcgatc gcgtatttcg tctcgctcag gcgcaatcac gaatgaataa cggtttggtt 1680
gatgcgagtg attttgatga cgagcgtaat ggctggcctg ttgaacaagt ctggaaagaa 1740
atgcataagc ttttgccatt ctcaccggat tcagtcgtca ctcatggtga tttctcactt 1800
gataacctta tttttgacga ggggaaatta ataggttgta ttgatgttgg acgagtcgga 1860
atcgcagacc gataccagga tcttgccatc ctatggaact gcctcggtga gttttctcct 1920
tcattacaga aacggctttt tcaaaaatat ggtattgata atcctgatat gaataaattg 1980
cagtttcatt tgatgctcga tgagtttttc taatcagaat tggttaattg gttgtaacac 2040
tggcttaatt aacatcatca ataatatacc ttattttgga ttgaagccaa tatgataatg 2100
agggggtgga gtttgtgacg tggcgcgggg cgtgggaacg gggcgggtga cgtagtagtg 2160
tggcggaagt gtgatgttgc aagtgtggcg gaacacatgt aagcgacgga tgtggcaaaa 2220
gtgacgtttt tggtgtgcgc cggtgtacac aggaagtgac aattttcgcg cggttttagg 2280
cggatgttgt agtaaatttg ggcgtaaccg agtaagattt ggccattttc gcgggaaaac 2340
tgaataagag gaagtgaaat ctgaataatt ttgtgttact catagcgcgt aatactggta 2400

ccccccagtg gcaagacgcg caggcaaaac gcaccacgac tggatccgaa ttcgaagacc 2460
acggtgtttc gtcctttcca caagatatat aaagccaaga aatcgaaata ctttcaagtt 2520
acggtaagca tatgatagtc cattttaaaa cataatttta aaactgcaaa ctacccaaga 2580
aattattact ttctacgtca cgtattttgt actaatatct ttgtgtttac agtcaaatta 2640
attctaatta tctttctaac agccttgtat cgtatatgca aatatgaagg aatcatggga 2700
aataggccct cttcctgccc gaccttggcg cgcgctcggc gcgcggtcac gctccgtcac 2760
gtggtgcgtt ttgcctgcgc gtcttgccac tggggggtac cccgaatcac tagatctgag 2820
ctcggcctgg tgatgatggc gggatcgttg tatatttctt gacacctttt cggcatcgcc 2880
ctaaaattcg gcgtcctcat attgtgtgag gacgttttat tacgtgttta cgaagcaaaa 2940
gctaaaacca ggagctattt aatggcaaca accggtacca tgattacgcc aagctacgta 3000
atacgactca ctagtggaag ttcctattct ctagaaagta taggaacttc agagcgcttt 3060
tgaagctggg gtgggcgaag aactccagca tgagatcccc agagcgcttt tgaagctgcg 3120
tttaaacgcg atatcccggg agctcccgat atcgcgttta aacgcagctt ggcgtaatca 3180
tggtcatagc tgtttcctgt gtgaaattgt tatccgctca caattccaca caacatacga 3240
gccggaagac tgaaatgtgt gggcgtggct taagggtggg aaagaatata taaggtgggg 3300
gtcttatgta gttttgtatc tgttttgcag cagccgccgc cgccatgagc accaactcgt 3360
ttgatggaag cattgtgagc tcatatttga caacgcgcat gcccccatgg gccggggtgc 3420
gtcagaatgt gatgggctcc agcattgatg gtcgccccgt cctgcccgca aactctacta 3480
ccttgaccta cgagaccgtg tctggaacgc cgttggagac tgcagcctcc gccgccgctt 3540
cagccgctgc agccaccgcc cgcgggattg tgactgactt tgctttcctg agcccgcttg 3600
caagcagtgc agcttcccgt tcatccgccc gcgatgacaa gttgacggct cttttggcac 3660
aattggattc tttgacccgg gaacttaatg tcgtttctca gcagctgttg gatctgcgcc 3720
agcaggtttc tgccctgaag gcttcctccc ctcccaatgc ggtttaaaac ataaataaaa 3780
aaccagactc tgtttggatt tggatcaagc aagtgtcttg ctgtctttat ttaggggttt 3840
tgcgcgcgcg gtaggcccgg gaccagcggt ctcggtcgtt gagggtcctg tgtatttttt 3900
ccaggacgtg gtaaaggtga ctctggatgt tcagatacat gggcataagc ccgtctctgg 3960
ggtggaggta gcaccactgc agagcttcat gctgcggggt ggtgttgtag atgatccagt 4020
cgtagcagga gcgctgggcg tggtgcctaa aaatgtcttt cagtagcaag ctgattgcca 4080
ggggcaggcc cttggtgtaa gtgtttacaa agcggttaag ctgggatggg tgcatacgtg 4140
gggatatgag atgcatcttg gactgtattt ttaggttggc tatgttccca gccatatccc 4200
tccggggatt catgttgtgc agaaccacca gcacagtgta tccggtgcac ttgggaaatt 4260
tgtcatgtag cttagaagga aatgcgtgga agaacttgga gacgcccttg tgacctccaa 4320

gattttccat gcattcgtcc ataatgatgg caatgggccc acgggcggcg gcctgggcga 4380

agatatttct gggatcacta acgtcatagt tgtgttccag gatgagatcg tcataggcca 4440

tttttacaaa gcgcgggcgg agggtgccag actgcggtat aatggttcca tccggcccag 4500

gggcgtagtt accctcacag atttgcattt cccacgcttt gagttcagat ggggggatca 4560

tgtctacctg cggggcgatg aagaaaacgg tttccggggt aggggagatc agctgggaag 4620

aaagcaggtt cctgagcagc tgcgacttac cgcagccggt gggcccgtaa atcacaccta 4680

ttaccgggtg caactggtag ttaagagagc tgcagctgcc gtcatccctg agcagggggg 4740

ccacttcgtt aagcatgtcc ctgactcgca tgttttccct gaccaaatcc gccagaaggc 4800

gctcgccgcc cagcgatagc agttcttgca aggaagcaaa gtttttcaac ggtttgagac 4860

cgtccgccgt aggcatgctt ttgagcgttt gaccaagcag ttccaggcgg tcccacagct 4920

cggtcacctg ctctacggca tctcgatcca gcatatctcc tcgtttcgcg ggttggggcg 4980

gctttcgctg tacggcagta gtcggtgctc gtccagacgg gccagggtca tgtctttcca 5040

cgggcgcagg gtcctcgtca gcgtagtctg ggtcacggtg aaggggtgcg ctccgggctg 5100

cgcgctggcc agggtgcgct tgaggctggt cctgctggtg ctgaagcgct gccggtcttc 5160

gccctgcgcg tcggccaggt agcatttgac catggtgtca tagtccagcc cctccgcggc 5220

gtggcccttg gcgcgcagct tgcccttgga ggaggcgccg cacgaggggc agtgcagact 5280

tttgagggcg tagagcttgg gcgcgagaaa taccgattcc ggggagtagg catccgcgcc 5340

gcaggccccg cagacggtct cgcattccac gagccaggtg agctctggcc gttcggggtc 5400

aaaaaccagg tttcccccat gctttttgat gcgtttctta cctctggttt ccatgagccg 5460

gtgtccacgc tcggtgacga aaaggctgtc cgtgtccccg tatacagact tgagaggcct 5520

gtcctcgagc ggtgttccgc ggtcctcctc gtatagaaac tcggaccact ctgagacaaa 5580

ggctcgcgtc caggccagca cgaaggaggc taagtgggag gggtagcggt cgttgtccac 5640

tagggggtcc actcgctcca gggtgtgaag acacatgtcg ccctcttcgg catcaaggaa 5700

ggtgattggt ttgtaggtgt aggccacgtg accgggtgtt cctgaagggg ggctataaaa 5760

gggggtgggg gcgcgttcgt cctcactctc ttccgcatcg ctgtctgcga gggccagctg 5820

ttggggtgag tactccctct gaaaagcggg catgacttct gcgctaagat tgtcagtttc 5880

caaaaacgag gaggatttga tattcacctg gcccgcggtg atgcctttga gggtggccgc 5940

atccatctgg tcagaaaaga caatcttttt gttgtcaagc ttggtggcaa acgacccgta 6000

gagggcgttg gacagcaact tggcgatgga gcgcagggtt tggtttttgt cgcgatcggc 6060

gcgctccttg gccgcgatgt ttagctgcac gtattcgcgc gcaacgcacc gccattcggg 6120

aaagacggtg gtgcgctcgt cgggcaccag gtgcacgcgc caaccgcggt tgtgcagggt 6180

gacaaggtca acgctggtgg ctacctctcc gcgtaggcgc tcgttggtcc agcagaggcg 6240
gccgcccttg cgcgagcaga atggcggtag ggggtctagc tgcgtctcgt ccggggggtc 6300
tgcgtccacg gtaaagaccc cgggcagcag gcgcgcgtcg aagtagtcta tcttgcatcc 6360
ttgcaagtct agcgcctgct gccatgcgcg ggcggcaagc gcgcgctcgt atgggttgag 6420
tgggggaccc catggcatgg ggtgggtgag cgcggaggcg tacatgccgc aaatgtcgta 6480
aacgtagagg ggctctctga gtattccaag atatgtaggg tagcatcttc caccgcggat 6540
gctggcgcgc acgtaatcgt atagttcgtg cgagggagcg aggaggtcgg gaccgaggtt 6600
gctacgggcg ggctgctctg ctcggaagac tatctgcctg aagatggcat gtgagttgga 6660
tgatatggtt ggacgctgga agacgttgaa gctggcgtct gtgagaccta ccgcgtcacg 6720
cacgaaggag gcgtaggagt cgcgcagctt gttgaccagc tcggcggtga cctgcacgtc 6780
tagggcgcag tagtccaggg tttccttgat gatgtcatac ttatcctgtc cctttttttt 6840
ccacagctcg cggttgagga caaactcttc gcggtctttc cagtactctt ggatcggaaa 6900
cccgtcggcc tccgaacggt aagagcctag catgtagaac tggttgacgg cctggtaggc 6960
gcagcatccc ttttctacgg gtagcgcgta tgcctgcgcg gccttccgga gcgaggtgtg 7020
ggtgagcgca aaggtgtccc tgaccatgac tttgaggtac tggtatttga agtcagtgtc 7080
gtcgcatccg ccctgctccc agagcaaaaa gtccgtgcgc tttttggaac gcggatttgg 7140
cagggcgaag gtgacatcgt tgaagagtat ctttcccgcg cgaggcataa agttgcgtgt 7200
gatgcggaag ggtcccggca cctcggaacg gttgttaatt acctgggcgg cgagcacgat 7260
ctcgtcaaag ccgttgatgt tgtggcccac aatgtaaagt tccaagaagc gcgggatgcc 7320
cttgatggaa ggcaattttt taagttcctc gtaggtgagc tcttcagggg agctgagccc 7380
gtgctctgaa agggcccagt ctgcaagatg agggttggaa gcgacgaatg agctccacag 7440
gtcacgggcc attagcattt gcaggtggtc gcgaaaggtc ctaaactggc gacctatggc 7500
cattttttct ggggtgatgc agtagaaggt aagcgggtct tgttcccagc ggtcccatcc 7560
aaggttcgcg gctaggtctc gcgcggcagt cactagaggc tcatctccgc cgaacttcat 7620
gaccagcatg aagggcacga gctgcttccc aaaggccccc atccaagtat aggtctctac 7680
atcgtaggtg acaaagagac gctcggtgcg aggatgcgag ccgatcggga agaactggat 7740
ctcccgccac caattggagg agtggctatt gatgtggtga aagtagaagt ccctgcgacg 7800
ggccgaacac tcgtgctggc ttttgtaaaa acgtgcgcag tactggcagc ggtgcacggg 7860
ctgtacatcc tgcacgaggt tgacctgacg accgcgcaca aggaagcaga gtgggaattt 7920
gagcccctcg cctggcgggt ttggctggtg gtcttctact tcggctgctt gtccttgacc 7980
gtctggctgc tcgaggggag ttacggtgga tcggaccacc acgccgcgcg agcccaaagt 8040
ccagatgtcc gcgcgcggcg gtcggagctt gatgacaaca tcgcgcagat gggagctgtc 8100

catggtctgg agctcccgcg gcgtcaggtc aggcgggagc tcctgcaggt ttacctcgca 8160

tagacgggtc agggcgcggg ctagatccag gtgataccta atttccaggg gctggttggt 8220

ggcggcgtcg atggcttgca agaggccgca tccccgcggc gcgactacgg taccgcgcgg 8280

cgggcggtgg gccgcggggg tgtccttgga tgatgcatct aaaagcggtg acgcgggcga 8340

gcccccggag gtaggggggg ctccggaccc gccgggagag ggggcagggg cacgtcggcg 8400

ccgcgcgcgg gcaggagctg gtgctgcgcg cgtaggttgc tggcgaacgc gacgacgcgg 8460

cggttgatct cctgaatctg gcgcctctgc gtgaagacga cgggcccggt gagcttgagc 8520

ctgaaagaga gttcgacaga atcaatttcg gtgtcgttga cggcggcctg gcgcaaaatc 8580

tcctgcacgt ctcctgagtt gtcttgatag gcgatctcgg ccatgaactg ctcgatctct 8640

tcctcctgga gatctccgcg tccggctcgc tccacggtgg cggcgaggtc gttggaaatg 8700

cgggccatga gctgcgagaa ggcgttgagg cctccctcgt tccagacgcg gctgtagacc 8760

acgccccctt cggcatcgcg ggcgcgcatg accacctgcg cgagattgag ctccacgtgc 8820

cgggcgaaga cggcgtagtt tcgcaggcgc tgaaagaggt agttgagggt ggtggcggtg 8880

tgttctgcca cgaagaagta cataacccag cgtcgcaacg tggattcgtt gatatccccc 8940

aaggcctcaa ggcgctccat ggcctcgtag aagtccacgg cgaagttgaa aaactgggag 9000

ttgcgcgccg acacggttaa ctcctcctcc agaagacgga tgagctcggc gacagtgtcg 9060

cgcacctcgc gctcaaaggc tacaggggcc tcttcttctt cttcaatctc ctcttccata 9120

agggcctccc cttcttcttc ttctggcggc ggtgggggag gggggacacg gcggcgacga 9180

cggcgcaccg ggaggcggtc gacaaagcgc tcgatcatct ccccgcggcg acggcgcatg 9240

gtctcggtga cggcgcggcc gttctcgcgg gggcgcagtt ggaagacgcc gcccgtcatg 9300

tcccggttat gggttggcgg ggggctgcca tgcggcaggg atacggcgct aacgatgcat 9360

ctcaacaatt gttgtgtagg tactccgccg ccgagggacc tgagcgagtc cgcatcgacc 9420

ggatcggaaa acctctcgag aaaggcgtct aaccagtcac agtcgcaagg taggctgagc 9480

accgtggcgg gcggcagcgg gcggcggtcg gggttgtttc tggcggaggt gctgctgatg 9540

atgtaattaa agtaggcggt cttgagacgg cggatggtcg acagaagcac catgtccttg 9600

ggtccggcct gctgaatgcg caggcggtcg gccatgcccc aggcttcgtt ttgacatcgg 9660

cgcaggtctt tgtagtagtc ttgcatgagc ctttctaccg gcacttcttc ttctccttcc 9720

tcttgtcctg catctcttgc atctatcgct gcggcggcgg cggagtttgg ccgtaggtgg 9780

cgccctcttc ctcccatgcg tgtgaccccg aagcccctca tcggctgaag cagggctagg 9840

tcggcgacaa cgcgctcggc taatatggcc tgctgcacct gcgtgagggt agactggaag 9900

tcatccatgt ccacaaagcg gtggtatgcg cccgtgttga tggtgtaagt gcagttggcc 9960

ataacggacc agttaacggt ctggtgaccc ggctgcgaga gctcggtgta cctgagacgc 10020

gagtaagccc tcgagtcaaa tacgtagtcg ttgcaagtcc gcaccaggta ctggtatccc 10080

accaaaaagt gcggcggcgg ctggcggtag aggggccagc gtagggtggc cggggctccg 10140

ggggcgagat cttccaacat aaggcgatga tatccgtaga tgtacctgga catccaggtg 10200

atgccggcgg cggtggtgga ggcgcgcgga aagtcgcgga cgcggttcca gatgttgcgc 10260

agcggcaaaa agtgctccat ggtcgggacg ctctggccgg tcaggcgcgc gcaatcgttg 10320

acgctctaga ccgtgcaaaa ggagagcctg taagcgggca ctcttccgtg gtctggtgga 10380

taaattcgca agggtatcat ggcggacgac cggggttcga gccccgtatc cggccgtccg 10440

ccgtgatcca tgcggttacc gcccgcgtgt cgaacccagg tgtgcgacgt cagacaacgg 10500

gggagtgctc cttttggctt ccttccaggc gcggcggctg ctgcgctagc ttttttggcc 10560

actggccgcg cgcagcgtaa gcggttaggc tggaaagcga aagcattaag tggctcgctc 10620

cctgtagccg gagggttatt ttccaagggt tgagtcgcgg gacccccggt tcgagtctcg 10680

gaccggccgg actgcggcga acgggggttt gcctccccgt catgcaagac cccgcttgca 10740

aattcctccg gaaacaggga cgagcccctt ttttgctttt cccagatgca tccggtgctg 10800

cggcagatgc gcccccctcc tcagcagcgg caagagcaag agcagcggca gacatgcagg 10860

gcaccctccc ctcctcctac cgcgtcagga ggggcgacat ccgcggttga cgcggcagca 10920

gatggtgatt acgaaccccc gcggcgccgg gcccggcact acctggactt ggaggagggc 10980

gagggcctgg cgcggctagg agcgccctct cctgagcggt acccaagggt gcagctgaag 11040

cgtgatacgc gtgaggcgta cgtgccgcgg cagaacctgt ttcgcgaccg cgagggagag 11100

gagcccgagg agatgcggga tcgaaagttc cacgcagggc gcgagctgcg gcatggcctg 11160

aatcgcgagc ggttgctgcg cgaggaggac tttgagcccg acgcgcgaac cgggattagt 11220

cccgcgcgcg cacacgtggc ggccgccgac ctggtaaccg catacgagca gacggtgaac 11280

caggagatta actttcaaaa aagctttaac aaccacgtgc gtacgcttgt ggcgcgcgag 11340

gaggtggcta taggactgat gcatctgtgg gactttgtaa gcgcgctgga gcaaaaccca 11400

aatagcaagc cgctcatggc gcagctgttc cttatagtgc agcacagcag ggacaacgag 11460

gcattcaggg atgcgctgct aaacatagta gagcccgagg gccgctggct gctcgatttg 11520

ataaacatcc tgcagagcat agtggtgcag gagcgcagct tgagcctggc tgacaaggtg 11580

gccgccatca actattccat gcttagcctg ggcaagtttt acgcccgcaa gatataccat 11640

accccttacg ttcccataga caaggaggta aagatcgagg ggttctacat gcgcatggcg 11700

ctgaaggtgc ttaccttgag cgacgacctg ggcgtttatc gcaacgagcg catccacaag 11760

gccgtgagcg tgagccggcg gcgcgagctc agcgaccgcg agctgatgca cagcctgcaa 11820

agggccctgg ctggcacggg cagcggcgat agagaggccg agtcctactt tgacgcgggc 11880

gctgacctgc gctgggcccc aagccgacgc gccctggagg cagctggggc cggacctggg 11940
ctggcggtgg cacccgcgcg cgctggcaac gtcggcggcg tggaggaata tgacgaggac 12000
gatgagtacg agccagagga cggcgagtac taagcggtga tgtttctgat cagatgatgc 12060
aagacgcaac ggacccggcg gtgcgggcgg cgctgcagag ccagccgtcc ggccttaact 12120
ccacggacga ctggcgccag gtcatggacc gcatcatgtc gctgactgcg cgcaatcctg 12180
acgcgttccg gcagcagccg caggccaacc ggctctccgc aattctggaa gcggtggtcc 12240
cggcgcgcgc aaaccccacg cacgagaagg tgctggcgat cgtaaacgcg ctggccgaaa 12300
acagggccat ccggcccgac gaggccggcc tggtctacga cgcgctgctt cagcgcgtgg 12360
ctcgttacaa cagcggcaac gtgcagacca acctggaccg gctggtgggg gatgtgcgcg 12420
aggccgtggc gcagcgtgag cgcgcgcagc agcagggcaa cctgggctcc atggttgcac 12480
taaacgcctt cctgagtaca cagcccgcca acgtgccgcg gggacaggag gactacacca 12540
actttgtgag cgcactgcgg ctaatggtga ctgagacacc gcaaagtgag gtgtaccagt 12600
ctgggccaga ctattttttc cagaccagta gacaaggcct gcagaccgta aacctgagcc 12660
aggctttcaa aaacttgcag gggctgtggg gggtgcgggc tcccacaggc gaccgcgcga 12720
ccgtgtctag cttgctgacg cccaactcgc gcctgttgct gctgctaata gcgcccttca 12780
cggacagtgg cagcgtgtcc cgggacacat acctaggtca cttgctgaca ctgtaccgcg 12840
aggccatagg tcaggcgcat gtggacgagc atactttcca ggagattaca agtgtcagcc 12900
gcgcgctggg gcaggaggac acgggcagcc tggaggcaac cctaaactac ctgctgacca 12960
accggcggca gaagatcccc tcgttgcaca gtttaaacag cgaggaggag cgcattttgc 13020
gctacgtgca gcagagcgtg agccttaacc tgatgcgcga cggggtaacg cccagcgtgg 13080
cgctggacat gaccgcgcgc aacatggaac cgggcatgta tgcctcaaac cggccgttta 13140
tcaaccgcct aatggactac ttgcatcgcg cggccgccgt gaaccccgag tatttcacca 13200
atgccatctt gaacccgcac tggctaccgc cccctggttt ctacaccggg ggattcgagg 13260
tgcccgaggg taacgatgga ttcctctggg acgacataga cgacagcgtg ttttccccgc 13320
aaccgcagac cctgctagag ttgcaacagc gcgagcaggc agaggcggcg ctgcgaaagg 13380
aaagcttccg caggccaagc agcttgtccg atctaggcgc tgcggccccg cggtcagatg 13440
ctagtagccc atttccaagc ttgatagggt ctcttaccag cactcgcacc acccgcccgc 13500
gcctgctggg cgaggaggag tacctaaaca actcgctgct gcagccgcag cgcgaaaaaa 13560
acctgcctcc ggcatttccc aacaacggga tagagagcct agtggacaag atgagtagat 13620
ggaagacgta cgcgcaggag cacagggacg tgccaggccc gcgcccgccc acccgtcgtc 13680
aaaggcacga ccgtcagcgg ggtctggtgt gggaggacga tgactcggca gacgacagca 13740

gcgtcctgga tttgggaggg agtggcaacc cgtttgcgca ccttcgcccc aggctgggga 13800
gaatgtttta aaaaaaaaaa agcatgatgc aaaataaaaa actcaccaag gccatggcac 13860
cgagcgttgg ttttcttgta ttccccttag tatgcggcgc gcggcgatgt atgaggaagg 13920
tcctcctccc tcctacgaga gtgtggtgag cgcggcgcca gtggcggcgg cgctgggttc 13980
tcccttcgat gctcccctgg acccgccgtt tgtgcctccg cggtacctgc ggcctaccgg 14040
ggggagaaac agcatccgtt actctgagtt ggcaccccta ttcgacacca cccgtgtgta 14100
cctggtggac aacaagtcaa cggatgtggc atccctgaac taccagaacg accacagcaa 14160
ctttctgacc acggtcattc aaaacaatga ctacagcccg ggggaggcaa gcacacagac 14220
catcaatctt gacgaccggt cgcactgggg cggcgacctg aaaaccatcc tgcataccaa 14280
catgccaaat gtgaacgagt tcatgtttac caataagttt aaggcgcggg tgatggtgtc 14340
gcgcttgcct actaaggaca atcaggtgga gctgaaatac gagtgggtgg agttcacgct 14400
gcccgagggc aactactccg agaccatgac catagacctt atgaacaacg cgatcgtgga 14460
gcactacttg aaagtgggca gacagaacgg ggttctggaa agcgacatcg gggtaaagtt 14520
tgacacccgc aacttcagac tggggtttga ccccgtcact ggtcttgtca tgcctggggt 14580
atatacaaac gaagccttcc atccagacat cattttgctg ccaggatgcg gggtggactt 14640
cacccacagc cgcctgagca acttgttggg catccgcaag cggcaaccct tccaggaggg 14700
ctttaggatc acctacgatg atctggaggg tggtaacatt cccgcactgt tggatgtgga 14760
cgcctaccag gcgagcttga aagatgacac cgaacagggc gggggtggcg caggcggcag 14820
caacagcagt ggcagcggcg cggaagagaa ctccaacgcg gcagccgcgg caatgcagcc 14880
ggtggaggac atgaacgatc atgccattcg cggcgacacc tttgccacac gggctgagga 14940
gaagcgcgct gaggccgaag cagcggccga agctgccgcc cccgctgcgc aacccgaggt 15000
cgagaagcct cagaagaaac cggtgatcaa acccctgaca gaggacagca agaaacgcag 15060
ttacaaccta ataagcaatg acagcacctt cacccagtac cgcagctggt accttgcata 15120
caactacggc gaccctcaga ccggaatccg ctcatggacc ctgctttgca ctcctgacgt 15180
aacctgcggc tcggagcagg tctactggtc gttgccagac atgatgcaag accccgtgac 15240
cttccgctcc acgcgccaga tcagcaactt tccggtggtg ggcgccgagc tgttgcccgt 15300
gcactccaag agcttctaca acgaccaggc cgtctactcc caactcatcc gccagtttac 15360
ctctctgacc cacgtgttca atcgctttcc cgagaaccag attttggcgc gcccgccagc 15420
ccccaccatc accaccgtca gtgaaaacgt tcctgctctc acagatcacg ggacgctacc 15480
gctgcgcaac agcatcggag gagtccagcg agtgaccatt actgacgcca gacgccgcac 15540
ctgcccctac gtttacaagg ccctgggcat agtctcgccg cgcgtcctat cgagccgcac 15600
tttttgagca agcatgtcca tccttatatc gcccagcaat aacacaggct ggggcctgcg 15660

cttcccaagc aagatgtttg gcggggccaa gaagcgctcc gaccaacacc cagtgcgcgt 15720
gcgcgggcac taccgcgcgc cctggggcgc gcacaaacgc ggccgcactg ggcgcaccac 15780
cgtcgatgac gccatcgacg cggtggtgga ggaggcgcgc aactacacgc ccacgccgcc 15840
accagtgtcc acagtggacg cggccattca gaccgtggtg cgcggagccc ggcgctatgc 15900
taaaatgaag agacggcgga ggcgcgtagc acgtcgccac cgccgccgac ccggcactgc 15960
cgcccaacgc gcggcggcgg ccctgcttaa ccgcgcacgt cgcaccggcc gacgggcggc 16020
catgcgggcc gctcgaaggc tggccgcggg tattgtcact gtgcccccca ggtccaggcg 16080
acgagcggcc gccgcagcag ccgcggccat tagtgctatg actcagggtc gcaggggcaa 16140
cgtgtattgg gtgcgcgact cggttagcgg cctgcgcgtg cccgtgcgca cccgcccccc 16200
gcgcaactag attgcaagaa aaaactactt agactcgtac tgttgtatgt atccagcggc 16260
ggcggcgcgc aacgaagcta tgtccaagcg caaaatcaaa gaagagatgc tccaggtcat 16320
cgcgccggag atctatggcc ccccgaagaa ggaagagcag gattacaagc cccgaaagct 16380
aaagcgggtc aaaaagaaaa agaaagatga tgatgatgaa cttgacgacg aggtggaact 16440
gctgcacgct accgcgccca ggcgacgggt acagtggaaa ggtcgacgcg taaaacgtgt 16500
tttgcgaccc ggcaccaccg tagtctttac gcccggtgag cgctccaccc gcacctacaa 16560
gcgcgtgtat gatgaggtgt acggcgacga ggacctgctt gagcaggcca acgagcgcct 16620
cggggagttt gcctacggaa agcggcataa ggacatgctg gcgttgccgc tggacgaggg 16680
caacccaaca cctagcctaa agcccgtaac actgcagcag gtgctgcccg cgcttgcacc 16740
gtccgaagaa aagcgcggcc taaagcgcga gtctggtgac ttggcaccca ccgtgcagct 16800
gatggtaccc aagcgccagc gactggaaga tgtcttggaa aaaatgaccg tggaacctgg 16860
gctggagccc gaggtccgcg tgcggccaat caagcaggtg gcgccgggac tgggcgtgca 16920
gaccgtggac gttcagatac ccactaccag tagcaccagt attgccaccg ccacagaggg 16980
catggagaca caaacgtccc cggttgcctc agcggtggcg gatgccgcgg tgcaggcggt 17040
cgctgcggcc gcgtccaaga cctctacgga ggtgcaaacg gacccgtgga tgtttcgcgt 17100
ttcagccccc cggcgcccgc gcggttcgag gaagtacggc gccgccagcg cgctactgcc 17160
cgaatatgcc ctacatcctt ccattgcgcc tacccccggc tatcgtggct acacctaccg 17220
ccccagaaga cgagcaacta cccgacgccg aaccaccact ggaacccgcc gccgccgtcg 17280
ccgtcgccag cccgtgctgg ccccgatttc cgtgcgcagg gtggctcgcg aaggaggcag 17340
gaccctggtg ctgccaacag cgcgctacca ccccagcatc gtttaaaagc cggtctttgt 17400
ggttcttgca gatatggccc tcacctgccg cctccgtttc ccggtgccgg gattccgagg 17460
aagaatgcac cgtaggaggg gcatggccgg ccacggcctg acgggcggca tgcgtcgtgc 17520

gcaccaccgg cggcggcgcg cgtcgcaccg tcgcatgcgc ggcggtatcc tgcccctcct 17580
tattccactg atcgccgcgg cgattggcgc cgtgcccgga attgcatccg tggccttgca 17640
ggcgcagaga cactgattaa aaacaagttg catgtggaaa aatcaaaata aaaagtctgg 17700
actctcacgc tcgcttggtc ctgtaactat tttgtagaat ggaagacatc aactttgcgt 17760
ctctggcccc gcgacacggc tcgcgcccgt tcatgggaaa ctggcaagat atcggcacca 17820
gcaatatgag cggtggcgcc ttcagctggg gctcgctgtg gagcggcatt aaaaatttcg 17880
gttccaccgt taagaactat ggcagcaagg cctggaacag cagcacaggc cagatgctga 17940
gggataagtt gaaagagcaa aatttccaac aaaaggtggt agatggcctg gcctctggca 18000
ttagcggggt ggtggacctg gccaaccagg cagtgcaaaa taagattaac agtaagcttg 18060
atccccgccc tcccgtagag gagcctccac cggccgtgga gacagtgtct ccagaggggc 18120
gtggcgaaaa gcgtccgcgc cccgacaggg aagaaactct ggtgacgcaa atagacgagc 18180
ctccctcgta cgaggaggca ctaaagcaag gcctgcccac cacccgtccc atcgcgccca 18240
tggctaccgg agtgctgggc cagcacacac ccgtaacgct ggacctgcct ccccccgccg 18300
acacccagca gaaacctgtg ctgccaggcc cgaccgccgt tgttgtaacc cgtcctagcc 18360
gcgcgtccct gcgccgcgcc gccagcggtc cgcgatcgtt gcggcccgta gccagtggca 18420
actggcaaag cacactgaac agcatcgtgg gtctgggggt gcaatccctg aagcgccgac 18480
gatgcttctg aatagctaac gtgtcgtatg tgtgtcatgt atgcgtccat gtcgccgcca 18540
gaggagctgc tgagccgccg cgcgcccgct ttccaagatg gctacccctt cgatgatgcc 18600
gcagtggtct tacatgcaca tctcgggcca ggacgcctcg gagtacctga gccccgggct 18660
ggtgcagttt gcccgcgcca ccgagacgta cttcagcctg aataacaagt ttagaaaccc 18720
cacggtggcg cctacgcacg acgtgaccac agaccggtcc cagcgtttga cgctgcggtt 18780
catccctgtg gaccgtgagg atactgcgta ctcgtacaag gcgcggttca ccctagctgt 18840
gggtgataac cgtgtgctgg acatggcttc cacgtacttt gacatccgcg gcgtgctgga 18900
caggggccct acttttaagc cctactctgg cactgcctac aacgccctgg ctcccaaggg 18960
tgccccaaat ccttgcgaat gggatgaagc tgctactgct cttgaaataa acctagaaga 19020
agaggacgat gacaacgaag acgaagtaga cgagcaagct gagcagcaaa aaactcacgt 19080
atttgggcag gcgccttatt ctggtataaa tattacaaag gagggtattc aaataggtgt 19140
cgaaggtcaa acacctaaat atgccgataa aacatttcaa cctgaacctc aaataggaga 19200
atctcagtgg tacgaaactg aaattaatca tgcagctggg agagtcctta aaaagactac 19260
cccaatgaaa ccatgttacg gttcatatgc aaaacccaca aatgaaaatg gagggcaagg 19320
cattcttgta aagcaacaaa atggaaagct agaaagtcaa gtggaaatgc aatttttctc 19380
aactactgag gcgaccgcag gcaatggtga taacttgact cctaaagtgg tattgtacag 19440

tgaagatgta gatatagaaa ccccagacac tcatatttct tacatgccca ctattaagga 19500

aggtaactca cgagaactaa tgggccaaca atctatgccc aacaggccta attacattgc 19560

ttttagggac aattttattg gtctaatgta ttacaacagc acgggtaata tgggtgttct 19620

ggcgggccaa gcatcgcagt tgaatgctgt tgtagatttg caagacagaa acacagagct 19680

ttcataccag cttttgcttg attccattgg tgatagaacc aggtactttt ctatgtggaa 19740

tcaggctgtt gacagctatg atccagatgt tagaattatt gaaaatcatg gaactgaaga 19800

tgaacttcca aattactgct ttccactggg aggtgtgatt aatacagaga ctcttaccaa 19860

ggtaaaacct aaaacaggtc aggaaaatgg atgggaaaaa gatgctacag aattttcaga 19920

taaaaatgaa ataagagttg gaaataattt tgccatggaa atcaatctaa atgccaacct 19980

gtggagaaat ttcctgtact ccaacatagc gctgtatttg cccgacaagc taaagtacag 20040

tccttccaac gtaaaaattt ctgataaccc aaacacctac gactacatga acaagcgagt 20100

ggtggctccc gggttagtgg actgctacat taaccttgga gcacgctggt cccttgacta 20160

tatggacaac gtcaacccat ttaaccacca ccgcaatgct ggcctgcgct accgctcaat 20220

gttgctgggc aatggtcgct atgtgccctt ccacatccag gtgcctcaga agttctttgc 20280

cattaaaaac ctccttctcc tgccgggctc atacacctac gagtggaact tcaggaagga 20340

tgttaacatg gttctgcaga gctccctagg aaatgaccta agggttgacg gagccagcat 20400

taagtttgat agcatttgcc tttacgccac cttcttcccc atggcccaca acaccgcctc 20460

cacgcttgag gccatgctta gaaacgacac caacgaccag tcctttaacg actatctctc 20520

cgccgccaac atgctctacc ctatacccgc caacgctacc aacgtgccca tatccatccc 20580

ctcccgcaac tgggcggctt tccgcggctg ggccttcacg cgccttaaga ctaaggaaac 20640

cccatcactg ggctcgggct acgaccctta ttacacctac tctggctcta taccctacct 20700

agatggaacc ttttacctca accacacctt taagaaggtg gccattacct ttgactcttc 20760

tgtcagctgg cctggcaatg accgcctgct tacccccaac gagtttgaaa ttaagcgctc 20820

agttgacggg gagggttaca acgttgccca gtgtaacatg accaaagact ggttcctggt 20880

acaaatgcta gctaactaca acattggcta ccagggcttc tatatcccag agagctacaa 20940

ggaccgcatg tactccttct ttagaaactt ccagcccatg agccgtcagg tggtggatga 21000

tactaaatac aaggactacc aacaggtggg catcctacac caacacaaca actctggatt 21060

tgttggctac cttgccccca ccatgcgcga aggacaggcc taccctgcta acttccccta 21120

tccgcttata ggcaagaccg cagttgacag cattacccag aaaaagtttc tttgcgatcg 21180

caccctttgg cgcatcccat tctccagtaa ctttatgtcc atgggcgcac tcacagacct 21240

gggccaaaac cttctctacg ccaactccgc ccacgcgcta gacatgactt ttgaggtgga 21300

tcccatggac gagcccaccc ttctttatgt tttgtttgaa gtctttgacg tggtccgtgt 21360

gcaccggccg caccgcggcg tcatcgaaac cgtgtacctg cgcacgccct tctcggccgg 21420

caacgccaca acataaagaa gcaagcaaca tcaacaacag ctgccgccat gggctccagt 21480

gagcaggaac tgaaagccat tgtcaaagat cttggttgtg ggccatattt tttgggcacc 21540

tatgacaagc gctttccagg ctttgtttct ccacacaagc tcgcctgcgc catagtcaat 21600

acggccggtc gcgagactgg gggcgtacac tggatggcct ttgcctggaa cccgcactca 21660

aaaacatgct acctctttga gccctttggc ttttctgacc agcgactcaa gcaggtttac 21720

cagtttgagt acgagtcact cctgcgccgt agcgccattg cttcttcccc cgaccgctgt 21780

ataacgctgg aaaagtccac ccaaagcgta caggggccca actcggccgc ctgtggacta 21840

ttctgctgca tgtttctcca cgcctttgcc aactggcccc aaactcccat ggatcacaac 21900

cccaccatga accttattac cggggtaccc aactccatgc tcaacagtcc ccaggtacag 21960

cccaccctgc gtcgcaacca ggaacagctc tacagcttcc tggagcgcca ctcgccctac 22020

ttccgcagcc acagtgcgca gattaggagc gccacttctt tttgtcactt gaaaaacatg 22080

taaaaataat gtactagaga cactttcaat aaaggcaaat gcttttattt gtacactctc 22140

gggtgattat ttacccccac ccttgccgtc tgcgccgttt aaaaatcaaa ggggttctgc 22200

cgcgcatcgc tatgcgccac tggcagggac acgttgcgat actggtgttt agtgctccac 22260

ttaaactcag gcacaaccat ccgcggcagc tcggtgaagt tttcactcca caggctgcgc 22320

accatcacca acgcgtttag caggtcgggc gccgatatct tgaagtcgca gttggggcct 22380

ccgccctgcg cgcgcgagtt gcgatacaca gggttgcagc actggaacac tatcagcgcc 22440

gggtggtgca cgctggccag cacgctcttg tcggagatca gatccgcgtc caggtcctcc 22500

gcgttgctca gggcgaacgg agtcaacttt ggtagctgcc ttcccaaaaa gggcgcgtgc 22560

ccaggctttg agttgcactc gcaccgtagt ggcatcaaaa ggtgaccgtg cccggtctgg 22620

gcgttaggat acagcgcctg cataaaagcc ttgatctgct taaaagccac ctgagccttt 22680

gcgccttcag agaagaacat gccgcaagac ttgccggaaa actgattggc cggacaggcc 22740

gcgtcgtgca cgcagcacct tgcgtcggtg ttggagatct gcaccacatt tcggccccac 22800

cggttcttca cgatcttggc cttgctagac tgctccttca gcgcgcgctg cccgttttcg 22860

ctcgtcacat ccatttcaat cacgtgctcc ttatttatca taatgcttcc gtgtagacac 22920

ttaagctcgc cttcgatctc agcgcagcgg tgcagccaca acgcgcagcc cgtgggctcg 22980

tgatgcttgt aggtcacctc tgcaaacgac tgcaggtacg cctgcaggaa tcgccccatc 23040

atcgtcacaa aggtcttgtt gctggtgaag gtcagctgca acccgcggtg ctcctcgttc 23100

agccaggtct tgcatacggc cgccagagct tccacttggt caggcagtag tttgaagttc 23160

gcctttagat cgttatccac gtggtacttg tccatcagcg cgcgcgcagc ctccatgccc 23220

ttctcccacg cagacacgat cggcacactc agcgggttca tcaccgtaat ttcactttcc 23280

gcttcgctgg gctcttcctc ttcctcttgc gtccgcatac cacgcgccac tgggtcgtct 23340

tcattcagcc gccgcactgt gcgcttacct cctttgccat gcttgattag caccggtggg 23400

ttgctgaaac ccaccatttg tagcgccaca tcttctcttt cttcctcgct gtccacgatt 23460

acctctggtg atggcgggcg ctcgggcttg ggagaagggc gcttcttttt cttcttgggc 23520

gcaatggcca aatccgccgc cgaggtcgat ggccgcgggc tgggtgtgcg cggcaccagc 23580

gcgtcttgtg atgagtcttc ctcgtcctcg gactcgatac gccgcctcat ccgctttttt 23640

gggggcgccc ggggaggcgg cggcgacggg gacggggacg acacgtcctc catggttggg 23700

ggacgtcgcg ccgcaccgcg tccgcgctcg ggggtggttt cgcgctgctc ctcttcccga 23760

ctggccattt ccttctccta taggcagaaa aagatcatgg agtcagtcga gaagaaggac 23820

agcctaaccg ccccctctga gttcgccacc accgcctcca ccgatgccgc caacgcgcct 23880

accaccttcc ccgtcgaggc acccccgctt gaggaggagg aagtgattat cgagcaggac 23940

ccaggttttg taagcgaaga cgacgaggac cgctcagtac caacagagga taaaaagcaa 24000

gaccaggaca acgcagaggc aaacgaggaa caagtcgggc ggggggacga aaggcatggc 24060

gactacctag atgtgggaga cgacgtgctg ttgaagcatc tgcagcgcca gtgcgccatt 24120

atctgcgacg cgttgcaaga gcgcagcgat gtgcccctcg ccatagcgga tgtcagcctt 24180

gcctacgaac gccacctatt ctcaccgcgc gtacccccca aacgccaaga aaacggcaca 24240

tgcgagccca acccgcgcct caacttctac cccgtatttg ccgtgccaga ggtgcttgcc 24300

acctatcaca tctttttcca aaactgcaag atacccctat cctgccgtgc caaccgcagc 24360

cgagcggaca agcagctggc cttgcggcag ggcgctgtca tacctgatat cgcctcgctc 24420

aacgaagtgc caaaaatctt tgagggtctt ggacgcgacg agaagcgcgc ggcaaacgct 24480

ctgcaacagg aaaacagcga aaatgaaagt cactctggag tgttggtgga actcgagggt 24540

gacaacgcgc gcctagccgt actaaaacgc agcatcgagg tcacccactt tgcctacccg 24600

gcacttaacc taccccccaa ggtcatgagc acagtcatga gtgagctgat cgtgcgccgt 24660

gcgcagcccc tggagaggga tgcaaatttg caagaacaaa cagaggaggg cctacccgca 24720

gttggcgacg agcagctagc gcgctggctt caaacgcgcg agcctgccga cttggaggag 24780

cgacgcaaac taatgatggc cgcagtgctc gttaccgtgg agcttgagtg catgcagcgg 24840

ttctttgctg acccggagat gcagcgcaag ctagaggaaa cattgcacta cacctttcga 24900

cagggctacg tacgccaggc ctgcaagatc tccaacgtgg agctctgcaa cctggtctcc 24960

taccttggaa ttttgcacga aaaccgcctt gggcaaaacg tgcttcattc cacgctcaag 25020

ggcgaggcgc gccgcgacta cgtccgcgac tgcgtttact tatttctatg ctacacctgg 25080

cagacggcca tgggcgtttg gcagcagtgc ttggaggagt gcaacctcaa ggagctgcag 25140

aaactgctaa agcaaaactt gaaggaccta tggacggcct tcaacgagcg ctccgtggcc 25200

gcgcacctgg cggacatcat tttccccgaa cgcctgctta aaaccctgca acagggtctg 25260

ccagacttca ccagtcaaag catgttgcag aactttagga actttatcct agagcgctca 25320

ggaatcttgc ccgccacctg ctgtgcactt cctagcgact ttgtgcccat taagtaccgc 25380

gaatgccctc cgccgctttg gggccactgc taccttctgc agctagccaa ctaccttgcc 25440

taccactctg acataatgga agacgtgagc ggtgacggtc tactggagtg tcactgtcgc 25500

tgcaacctat gcaccccgca ccgctccctg gtttgcaatt cgcagctgct taacgaaagt 25560

caaattatcg gtacctttga gctgcagggt ccctcgcctg acgaaaagtc cgcggctccg 25620

gggttgaaac tcactccggg gctgtggacg tcggcttacc ttcgcaaatt tgtacctgag 25680

gactaccacg cccacgagat taggttctac gaagaccaat cccgcccgcc aaatgcggag 25740

cttaccgcct gcgtcattac ccagggccac attcttggcc aattgcaagc catcaacaaa 25800

gcccgccaag agtttctgct acgaaaggga cggggggttt acttggaccc ccagtccggc 25860

gaggagctca acccaatccc cccgccgccg cagccctatc agcagcagcc gcgggccctt 25920

gcttcccagg atggcaccca aaaagaagct gcagctgccg ccgccaccca cggacgagga 25980

ggaatactgg gacagtcagg cagaggaggt tttggacgag gaggaggagg acatgatgga 26040

agactgggag agcctagacg aggaagcttc cgaggtcgaa gaggtgtcag acgaaacacc 26100

gtcaccctcg gtcgcattcc cctcgccggc gccccagaaa tcggcaaccg gttccagcat 26160

ggctacaacc tccgctcctc aggcgccgcc ggcactgccc gttcgccgac ccaaccgtag 26220

atgggacacc actggaacca gggccggtaa gtccaagcag ccgccgccgt tagcccaaga 26280

gcaacaacag cgccaaggct accgctcatg gcgcgggcac aagaacgcca tagttgcttg 26340

cttgcaagac tgtgggggca acatctcctt cgcccgccgc tttcttctct accatcacgg 26400

cgtggccttc ccccgtaaca tcctgcatta ctaccgtcat ctctacagcc catactgcac 26460

cggcggcagc ggcagcggca gcaacagcag cggccacaca gaagcaaagg cgaccggata 26520

gcaagactct gacaaagccc aagaaatcca cagcggcggc agcagcagga ggaggagcgc 26580

tgcgtctggc gcccaacgaa cccgtatcga cccgcgagct tagaaacagg atttttccca 26640

ctctgtatgc tatatttcaa cagagcaggg gccaagaaca agagctgaaa ataaaaaaca 26700

ggtctctgcg atccctcacc cgcagctgcc tgtatcacaa aagcgaagat cagcttcggc 26760

gcacgctgga agacgcggag gctctcttca gtaaatactg cgcgctgact cttaaggact 26820

agtttcgcgc cctttctcaa atttaagcgc gaaaactacg tcatctccag cggccacacc 26880

cggcgccagc acctgtcgtc agcgccatta tgagcaagga aattcccacg ccctacatgt 26940

ggagttacca gccacaaatg ggacttgcgg ctggagctgc ccaagactac tcaacccgaa 27000

taaactacat gagcgcggga ccccacatga tatcccgggt caacggaatc cgcgcccacc 27060

gaaaccgaat tctcttggaa caggcggcta ttaccaccac acctcgtaat aaccttaatc 27120

cccgtagttg gcccgctgcc ctggtgtacc aggaaagtcc cgctcccacc actgtggtac 27180

ttcccagaga cgcccaggcc gaagttcaga tgactaactc aggggcgcag cttgcgggcg 27240

gctttcgtca cagggtgcgg tcgcccgggc agggtataac tcacctgaca atcagagggc 27300

gaggtattca gctcaacgac gagtcggtga gctcctcgct tggtctccgt ccggacggga 27360

catttcagat cggcggcgcc ggccgtcctt cattcacgcc tcgtcaggca atcctaactc 27420

tgcagacctc gtcctctgag ccgcgctctg gaggcattgg aactctgcaa tttattgagg 27480

agtttgtgcc atcggtctac tttaacccct tctcgggacc tcccggccac tatccggatc 27540

aatttattcc taactttgac gcggtaaagg actcggcgga cggctacgac tgaatgttaa 27600

gtggagaggc agagcaactg cgcctgaaac acctggtcca ctgtcgccgc cacaagtgct 27660

ttgcccgcga ctccggtgag ttttgctact ttgaattgcc cgaggatcat atcgagggcc 27720

cggcgcacgg cgtccggctt accgcccagg gagagcttgc ccgtagcctg attcgggagt 27780

ttacccagcg ccccctgcta gttgagcggg acaggggacc ctgtgttctc actgtgattt 27840

gcaactgtcc taaccttgga ttacatcaag atctttgttg ccatctctgt gctgagtata 27900

ataaatacag aaattaaaat atactggggc tcctatcgcc atcctgtaaa cgccaccgtc 27960

ttcacccgcc caagcaaacc aaggcgaacc ttacctggta cttttaacat ctctccctct 28020

gtgatttaca acagtttcaa cccagacgga gtgagtctac gagagaacct ctccgagctc 28080

agctactcca tcagaaaaaa caccaccctc cttacctgcc gggaacgtac gagtgcgtca 28140

ccggccgctg caccacacct accgcctgac cgtaaaccag actttttccg gacagacctc 28200

aataactctg tttaccagaa caggaggtga gcttagaaaa cccttagggt attaggccaa 28260

aggcgcagct actgtggggt ttatgaacaa ttcaagcaac tctacgggct attctaattc 28320

aggtttctct agaaatggac ggaattatta cagagcagcg cctgctagaa agacgcaggg 28380

cagcggccga gcaacagcgc atgaatcaag agctccaaga catggttaac ttgcaccagt 28440

gcaaaagggg tatcttttgt ctggtaaagc aggccaaagt cacctacgac agtaatacca 28500

ccggacaccg ccttagctac aagttgccaa ccaagcgtca gaaattggtg gtcatggtgg 28560

gagaaaagcc cattaccata actcagcact cggtagaaac cgaaggctgc attcactcac 28620

cttgtcaagg acctgaggat ctctgcaccc ttattaagac cctgtgcggt ctcaaagatc 28680

ttattccctt taactaataa aaaaaaataa taaagcatca cttacttaaa atcagttagc 28740

aaatttctgt ccagtttatt cagcagcacc tccttgccct cctcccagct ctggtattgc 28800

agcttcctcc tggctgcaaa ctttctccac aatctaaatg gaatgtcagt ttcctcctgt 28860

tcctgtccat ccgcacccac tatcttcatg ttgttgcaga tgaagcgcgc aagaccgtct 28920

gaagatacct tcaaccccgt gtatccatat gacacggaaa ccggtcctcc aactgtgcct 28980

tttcttactc ctccctttgt atcccccaat gggtttcaag agagtccccc tggggtactc 29040

tctttgcgcc tatccgaacc tctagttacc tccaatggca tgcttgcgct caaaatgggc 29100

aacggcctct ctctggacga ggccggcaac cttacctccc aaaatgtaac cactgtgagc 29160

ccacctctca aaaaaaccaa gtcaaacata aacctggaaa tatctgcacc cctcacagtt 29220

acctcagaag ccctaactgt ggctgccgcc gcacctctaa tggtcgcggg caacacactc 29280

accatgcaat cacaggcccc gctaaccgtg cacgactcca aacttagcat tgccacccaa 29340

ggacccctca cagtgtcaga aggaaagcta gccctgcaaa catcaggccc cctcaccacc 29400

accgatagca gtacccttac tatcactgcc tcaccccctc taactactgc cactggtagc 29460

ttgggcattg acttgaaaga gcccatttat acacaaaatg gaaaactagg actaaagtac 29520

ggggctcctt tgcatgtaac agacgaccta aacactttga ccgtagcaac tggtccaggt 29580

gtgactatta ataatacttc cttgcaaact aaagttactg gagccttggg ttttgattca 29640

caaggcaata tgcaacttaa tgtagcagga ggactaagga ttgattctca aaacagacgc 29700

cttatacttg atgttagtta tccgtttgat gctcaaaacc aactaaatct aagactagga 29760

cagggccctc tttttataaa ctcagcccac aacttggata ttaactacaa caaaggcctt 29820

tacttgttta cagcttcaaa caattccaaa aagcttgagg ttaacctaag cactgccaag 29880

gggttgatgt ttgacgctac agccatagcc attaatgcag gagatgggct tgaatttggt 29940

tcacctaatg caccaaacac aaatcccctc aaaacaaaaa ttggccatgg cctagaattt 30000

gattcaaaca aggctatggt tcctaaacta ggaactggcc ttagttttga cagcacaggt 30060

gccattacag taggaaacaa aaataatgat aagctaactt tgtggaccac accagctcca 30120

tctcctaact gtagactaaa tgcagagaaa gatgctaaac tcactttggt cttaacaaaa 30180

tgtggcagtc aaatacttgc tacagtttca gttttggctg ttaaaggcag tttggctcca 30240

atatctggaa cagttcaaag tgctcatctt attataagat ttgacgaaaa tggagtgcta 30300

ctaaacaatt ccttcctgga cccagaatat tggaacttta gaaatggaga tcttactgaa 30360

ggcacagcct atacaaacgc tgttggattt atgcctaacc tatcagctta tccaaaatct 30420

cacggtaaaa ctgccaaaag taacattgtc agtcaagttt acttaaacgg agacaaaact 30480

aaacctgtaa cactaaccat tacactaaac ggtacacagg aaacaggaga cacaactcca 30540

agtgcatact ctatgtcatt ttcatgggac tggtctggcc acaactacat taatgaaata 30600

tttgccacat cctcttacac tttttcatac attgcccaag aataaagaat cgtttgtgtt 30660

atgtttcaac gtgtttattt ttcaattgca gaaaatttcg aatcattttt cattcagtag 30720

tatagcccca ccaccacata gcttatacag atcaccgtac cttaatcaaa ctcacagaac 30780

cctagtattc aacctgccac ctccctccca acacacagag tacacagtcc tttctccccg 30840
gctggcctta aaaagcatca tatcatgggt aacagacata ttcttaggtg ttatattcca 30900
cacggtttcc tgtcgagcca aacgctcatc agtgatatta ataaactccc cgggcagctc 30960
acttaagttc atgtcgctgt ccagctgctg agccacaggc tgctgtccaa cttgcggttg 31020
cttaacgggc ggcgaaggag aagtccacgc ctacatgggg gtagagtcat aatcgtgcat 31080
caggataggg cggtggtgct gcagcagcgc gcgaataaac tgctgccgcc gccgctccgt 31140
cctgcaggaa tacaacatgg cagtggtctc ctcagcgatg attcgcaccg cccgcagcat 31200
aaggcgcctt gtcctccggg cacagcagcg caccctgatc tcacttaaat cagcacagta 31260
actgcagcac agcaccacaa tattgttcaa aatcccacag tgcaaggcgc tgtatccaaa 31320
gctcatggcg gggaccacag aacccacgtg gccatcatac cacaagcgca ggtagattaa 31380
gtggcgaccc ctcataaaca cgctggacat aaacattacc tcttttggca tgttgtaatt 31440
caccacctcc cggtaccata taaacctctg attaaacatg gcgccatcca ccaccatcct 31500
aaaccagctg gccaaaacct gcccgccggc tatacactgc agggaaccgg gactggaaca 31560
atgacagtgg agagcccagg actcgtaacc atggatcatc atgctcgtca tgatatcaat 31620
gttggcacaa cacaggcaca cgtgcataca cttcctcagg attacaagct cctcccgcgt 31680
tagaaccata tcccagggaa caacccattc ctgaatcagc gtaaatccca cactgcaggg 31740
aagacctcgc acgtaactca cgttgtgcat tgtcaaagtg ttacattcgg gcagcagcgg 31800
atgatcctcc agtatggtag cgcgggtttc tgtctcaaaa ggaggtagac gatccctact 31860
gtacggagtg cgccgagaca accgagatcg tgttggtcgt agtgtcatgc caaatggaac 31920
gccggacgta gtcatatttc ctgaagcaaa accaggtgcg ggcgtgacaa acagatctgc 31980
gtctccggtc tcgccgctta gatcgctctg tgtagtagtt gtagtatatc cactctctca 32040
aagcatccag gcgccccctg gcttcgggtt ctatgtaaac tccttcatgc gccgctgccc 32100
tgataacatc caccaccgca gaataagcca cacccagcca acctacacat tcgttctgcg 32160
agtcacacac gggaggagcg ggaagagctg gaagaaccat gttttttttt ttattccaaa 32220
agattatcca aaacctcaaa atgaagatct attaagtgaa cgcgctcccc tccggtggcg 32280
tggtcaaact ctacagccaa agaacagata atggcatttg taagatgttg cacaatggct 32340
tccaaaaggc aaacggccct cacgtccaag tggacgtaaa ggctaaaccc ttcagggtga 32400
atctcctcta taaacattcc agcaccttca accatgccca aataattctc atctcgccac 32460
cttctcaata tatctctaag caaatcccga atattaagtc cggccattgt aaaaatctgc 32520
tccagagcgc cctccacctt cagcctcaag cagcgaatca tgattgcaaa aattcaggtt 32580
cctcacagac ctgtataaga ttcaaaagcg gaacattaac aaaaataccg cgatcccgta 32640

ggtcccttcg cagggccagc tgaacataat cgtgcaggtc tgcacggacc agcgcggcca 32700

cttccccgcc aggaaccttg acaaaagaac ccacactgat tatgacacgc atactcggag 32760

ctatgctaac cagcgtagcc ccgatgtaag ctttgttgca tgggcggcga tataaaatgc 32820

aaggtgctgc tcaaaaaatc aggcaaagcc tcgcgcaaaa aagaaagcac atcgtagtca 32880

tgctcatgca gataaaggca ggtaagctcc ggaaccacca cagaaaaaga caccattttt 32940

ctctcaaaca tgtctgcggg tttctgcata aacacaaaat aaaataacaa aaaaacattt 33000

aaacattaga agcctgtctt acaacaggaa aaacaaccct tataagcata agacggacta 33060

cggccatgcc ggcgtgaccg taaaaaaact ggtcaccgtg attaaaaagc accaccgaca 33120

gctcctcggt catgtccgga gtcataatgt aagactcggt aaacacatca ggttgattca 33180

catcggtcag tgctaaaaag cgaccgaaat agcccggggg aatacatacc cgcaggcgta 33240

gagacaacat tacagccccc ataggaggta taacaaaatt aataggagag aaaaacacat 33300

aaacacctga aaaaccctcc tgcctaggca aaatagcacc ctcccgctcc agaacaacat 33360

acagcgcttc cacagcggca gccataacag tcagccttac cagtaaaaaa gaaaacctat 33420

taaaaaaaca ccactcgaca cggcaccagc tcaatcagtc acagtgtaaa aaagggccaa 33480

gtgcagagcg agtatatata ggactaaaaa atgacgtaac ggttaaagtc cacaaaaaac 33540

acccagaaaa ccgcacgcga acctacgccc agaaacgaaa gccaaaaaac ccacaacttc 33600

ctcaaatcgt cacttccgtt ttcccacgtt acgtcacttc ccattttaag aaaactacaa 33660

ttcccaacac atacaagtta ctccgcccta aaacctacgt cacccgcccc gttcccacgc 33720

cccgcgccac gtcacaaact ccaccccctc attatcatat tggcttcaat ccaaaataag 33780

gtatattatt gatgatgtta attaagggcg gccgcacggg ccatcgatgg ggatccatcc 33840

gcggagaagc ttctcgacca attctcatgt ttgacagctt atcatcgaat ttctgccatt 33900

catccgctta ttatcactta ttcaggcgta gcaaccaggc gtttaagggc accaataact 33960

gccttaaaaa aattacgccc cgccctgcca ctcatcgcag tactgttgta attcattaag 34020

cattctgccg acatggaagc catcacagac ggcatgatga acctgaatcg ccagcggcat 34080

cagcaccttg tcgccttgcg tataatattt gcccatggtg aaaacggggg cgaagaagtt 34140

gtccatattg gccacgttta aatcaaaact ggtgaaactc acccagggat tggctgagac 34200

gaaaaacata ttctcaataa accctttagg gaaataggcc aggttttcac cgtaacacgc 34260

cacatcttgc gaatatatgt gtagaaactg ccggaaatcg tcgtggtatt cactccagag 34320

cgatgaaaac gtttcagttt gctcatggaa aacggtgtaa caagggtgaa cactatccca 34380

tatcaccagc tcaccgtctt tcattgccat acggaattcc ggatgagcat tcatcaggcg 34440

ggcaagaatg tgaataaagg ccggataaaa cttgtgctta tttttcttta cggtctttaa 34500

aaaggccgta atatccagct gaacggtctg gttataggta cattgagcaa ctgactgaaa 34560

tgcctcaaaa tgttctttac gatgccattg ggatatatca acggtggtat atccagtgat 34620
ttttttctcc attttagctt ccttagctcc tgaaaatctc gataactcaa aaaatacgcc 34680
cggtagtgat cttatttcat tatggtgaaa gttggaacct cttacgtgcc gatcaacgtc 34740
tcattttcgc caaaagttgg cccagggctt cccggtatca acagggacac caggatttat 34800
ttattctgcg aagtgatctt ccgtcacagg tatttattcg cgataagctc atggagcggc 34860
gtaaccgtcg cacaggaagg acagagaaag cgcggatctg ggaagtgacg gacagaacgg 34920
tcaggacctg gattggggag gcggttgccg ccgctgctgc tgacggtgtg acgttctctg 34980
ttccggtcac accacatacg ttccgccatt cctatgcgat gcacatgctg tatgccggta 35040
taccgctgaa agttctgcaa agcctgatgg gacataagtc catcagttca acggaagtct 35100
acacgaaggt ttttgcgctg gatgtggctg cccggcaccg ggtgcagttt gcgatgccgg 35160
agtctgatgc ggttgcgatg ctgaaacaat tatcctgaga ataaatgcct tggcctttat 35220
atggaaatgt ggaactgagt ggatatgctg tttttgtctg ttaaacagag aagctggctg 35280
ttatccactg agaagcgaac gaaacagtcg ggaaaatctc ccattatcgt agagatccgc 35340
attattaatc tcaggagcct gtgtagcgtt tataggaagt agtgttctgt catgatgcct 35400
gcaagcggta acgaaaacga tttgaatatg ccttcaggaa caatagaaat cttcgtgcgg 35460
tgttacgttg aagtggagcg gattatgtca gcaatggaca gaacaaccta atgaacacag 35520
aaccatgatg tggtctgtcc ttttacagcc agtaggctcg ccgcagtcga gcgacggcga 35580
agccctcgag tgagcgagga agcaccaggg aacagcactt atatattctg cttacacacg 35640
atgcctgaaa aaacttccct tggggttatc cacttatcca cggggatatt tttataatta 35700
ttttttttat agtttttaga tcttcttttt tagagcgcct tgtaggcctt tatccatgct 35760
ggttctagag aaggtgttgt gacaaattgc cctttcagtg tgacaaatca ccctcaaatg 35820
acagtcctgt ctgtgacaaa ttgcccttaa ccctgtgaca aattgccctc agaagaagct 35880
gttttttcac aaagttatcc ctgcttattg actctttttt atttagtgtg acaatctaaa 35940
aacttgtcac acttcacatg gatctgtcat ggcggaaaca gcggttatca atcacaagaa 36000
acgtaaaaat agcccgcgaa tcgtccagtc aaacgacctc actgaggcgg catatagtct 36060
ctcccgggat caaaaacgta tgctgtatct gttcgttgac cagatcagaa aatctgatgg 36120
caccctacag gaacatgacg gtatctgcga gatccatgtt gctaaatatg ctgaaatatt 36180
cggattgacc tctgcggaag ccagtaagga tatacggcag gcattgaaga gtttcgcggg 36240
gaaggaagtg gttttttatc gccctgaaga ggatgccggc gatgaaaaag gctatgaatc 36300
ttttccttgg tttatcaaac gtgcgcacag tccatccaga gggctttaca gtgtacatat 36360
caacccatat ctcattccct tctttatcgg gttacagaac cggtttacgc agtttcggct 36420

tagtgaaaca aaagaaatca ccaatccgta tgccatgcgt ttatacgaat ccctgtgtca 36480

gtatcgtaag ccggatggct caggcatcgt ctctctgaaa atcgactgga tcatagagcg 36540

ttaccagctg cctcaaagtt accagcgtat gcctgacttc cgccgccgct tcctgcaggt 36600

ctgtgttaat gagatcaaca gcagaactcc aatgcgcctc tcatacattg agaaaaagaa 36660

aggccgccag acgactcata tcgtattttc cttccgcgat atcacttcca tgacgacagg 36720

atagtctgag ggttatctgt cacagatttg agggtggttc gtcacatttg ttctgaccta 36780

ctgagggtaa tttgtcacag ttttgctgtt tccttcagcc tgcatggatt ttctcatact 36840

ttttgaactg taatttttaa ggaagccaaa tttgagggca gtttgtcaca gttgatttcc 36900

ttctctttcc cttcgtcatg tgacctgata tcgggggtta gttcgtcatc attgatgagg 36960

gttgattatc acagtttatt actctgaatt ggctatccgc gtgtgtacct ctacctggag 37020

tttttcccac ggtggatatt tcttcttgcg ctgagcgtaa gagctatctg acagaacagt 37080

tcttctttgc ttcctcgcca gttcgctcgc tatgctcggt tacacggctg cggcgagcgc 37140

tagtgataat aagtgactga ggtatgtgct cttcttatct ccttttgtag tgttgctctt 37200

attttaaaca actttgcggt tttttgatga ctttgcgatt ttgttgttgc tttgcagtaa 37260

attgcaagat ttaataaaaa aacgcaaagc aatgattaaa ggatgttcag aatgaaactc 37320

atggaaacac ttaaccagtg cataaacgct ggtcatgaaa tgacgaaggc tatcgccatt 37380

gcacagttta atgatgacag cccggaagcg aggaaaataa cccggcgctg gagaataggt 37440

gaagcagcgg atttagttgg ggtttcttct caggctatca gagatgccga gaaagcaggg 37500

cgactaccgc acccggatat ggaaattcga ggacgggttg agcaacgtgt tggttataca 37560

attgaacaaa ttaatcatat gcgtgatgtg tttggtacgc gattgcgacg tgctgaagac 37620

gtatttccac cggtgatcgg ggttgctgcc cataaaggtg gcgtttacaa aacctcagtt 37680

tctgttcatc ttgctcagga tctggctctg aaggggctac gtgttttgct cgtggaaggt 37740

aacgaccccc agggaacagc ctcaatgtat cacggatggg taccagatct tcatattcat 37800

gcagaagaca ctctcctgcc tttctatctt ggggaaaagg acgatgtcac ttatgcaata 37860

aagcccactt gctggccggg gcttgacatt attccttcct gtctggctct gcaccgtatt 37920

gaaactgagt taatgggcaa atttgatgaa ggtaaactgc ccaccgatcc acacctgatg 37980

ctccgactgg ccattgaaac tgttgctcat gactatgatg tcatagttat tgacagcgcg 38040

cctaacctgg gtatcggcac gattaatgtc gtatgtgctg ctgatgtgct gattgttccc 38100

acgcctgctg agttgtttga ctacacctcc gcactgcagt ttttcgatat gcttcgtgat 38160

ctgctcaaga acgttgatct taaagggttc gagcctgatg tacgtatttt gcttaccaaa 38220

tacagcaata gtaatggctc tcagtccccg tggatggagg agcaaattcg ggatgcctgg 38280

ggaagcatgg ttctaaaaaa tgttgtacgt gaaacggatg aagttggtaa aggtcagatc 38340

cggatgagaa ctgtttttga acaggccatt gatcaacgct cttcaactgg tgcctggaga 38400
aatgctcttt ctatttggga acctgtctgc aatgaaattt tcgatcgtct gattaaacca 38460
cgctgggaga ttagataatg aagcgtgcgc ctgttattcc aaaacatacg ctcaatactc 38520
aaccggttga agatacttcg ttatcgacac cagctgcccc gatggtggat tcgttaattg 38580
cgcgcgtagg agtaatggct cgcggtaatg ccattacttt gcctgtatgt ggtcgggatg 38640
tgaagtttac tcttgaagtg ctccggggtg atagtgttga gaagacctct cgggtatggt 38700
caggtaatga acgtgaccag gagctgctta ctgaggacgc actggatgat ctcatccctt 38760
cttttctact gactggtcaa cagacaccgg cgttcggtcg aagagtatct ggtgtcatag 38820
aaattgccga tgggagtcgc cgtcgtaaag ctgctgcact taccgaaagt gattatcgtg 38880
ttctggttgg cgagctggat gatgagcaga tggctgcatt atccagattg ggtaacgatt 38940
atcgcccaac aagtgcttat gaacgtggtc agcgttatgc aagccgattg cagaatgaat 39000
ttgctggaaa tatttctgcg ctggctgatg cggaaaatat ttcacgtaag attattaccc 39060
gctgtatcaa caccgccaaa ttgcctaaat cagttgttgc tctttttctt caccccggtg 39120
aactatctgc ccggtcaggt gatgcacttc aaaaagcctt tacagataaa gaggaattac 39180
ttaagcagca ggcatctaac cttcatgagc agaaaaaagc tggggtgata tttgaagctg 39240
aagaagttat cactctttta acttctgtgc ttaaaacgtc atctgcatca agaactagtt 39300
taagctcacg acatcagttt gctcctggag cgacagtatt gtataagggc gataaaatgg 39360
tgcttaacct ggacaggtct cgtgttccaa ctgagtgtat agagaaaatt gaggccattc 39420
ttaaggaact tgaaaagcca gcaccctgat gcgaccacgt tttagtctac gtttatctgt 39480
ctttacttaa tgtcctttgt tacaggccag aaagcataac tggcctgaat attctctctg 39540
ggcccactgt tccacttgta tcgtcggtct gataatcaga ctgggaccac ggtcccactc 39600
gtatcgtcgg tctgattatt agtctgggac cacggtccca ctcgtatcgt cggtctgatt 39660
attagtctgg gaccacggtc ccactcgtat cgtcggtctg ataatcagac tgggaccacg 39720
gtcccactcg tatcgtcggt ctgattatta gtctgggacc atggtcccac tcgtatcgtc 39780
ggtctgatta ttagtctggg accacggtcc cactcgtatc gtcggtctga ttattagtct 39840
ggaaccacgg tcccactcgt atcgtcggtc tgattattag tctgggacca cggtcccact 39900
cgtatcgtcg gtctgattat tagtctggga ccacgatccc actcgtgttg tcggtctgat 39960
tatcggtctg ggaccacggt cccacttgta ttgtcgatca gactatcagc gtgagactac 40020
gattccatca atgcctgtca agggcaagta ttgacatgtc gtcgtaacct gtagaacgga 40080
gtaacctcgg tgtgcggttg tatgcctgct gtggattgct gctgtgtcct gcttatccac 40140
aacattttgc gcacggttat gtggacaaaa tacctggtta cccaggccgt gccggcacgt 40200

taaccgggct gcatccgatg caagtgtgtc gctgtcgagt tttcggggga gtccagggtt 40260

ttcccagtca cgacgttgta aaacgacggc cagtgaattc gagctcggta cccggggatc 40320

tt 40322

<210> 12
<211> 43324
<212> DNA
<213> Artificial

<220>
<223> synthetic

<400> 12
   gaagttccta ttccgaagtt cctattctct agaaagtata ggaacttcct taagtgagtc 60
   gtattacgga ctggccgtcg ttttacaacg tcgtgactgg gaaaaccctg gcgttaccca 120
   acttaatcgc cttgcagcac atcccccttt cgccagctgg cgtaatagcg aagaggcccg 180
   caccgatcgc ccttcccaac agttgcgcag cctgaatggc gaatggcgct tcgcttggta 240
   agctagctaa ggcttaagga ggacaatcat ggcaacagtt aaccagctgg tacgcaaacc 300
   acgtgctcgc aaagttgcga aaagcaacgt gcctgcgctg gaagcatgcc cgcaaaaacg 360
   tggcgtatgt actcgtgtat atactaccac tcctaaaaaa ccgaactccg cgctgcgtaa 420
   agtatgccgt gttcgtctgt ctaacggttt cgaagtgact tcctacatcg gtggtgaagg 480
   tcacaacctg caggagcact ccgtgatcct gatccgtggc ggtcgtgtta aagacctccc 540
   gggtgttcgt taccacaccg tacgtggtgc gcttgactgc tccggcgtta aagaccgtaa 600
   gcaggctcgt tccaagtatg gcgtgaagcg tcctaaggct taactagcac gcgccatgtc 660
   agccgttaag tgttcctgtg tcactcaaaa ttgctttgag aggctctaag ggcttctcag 720
   tgcgttacat ccctggcttg ttgtccacaa ccgttaaacc ttaaaagctt taaaagcctt 780
   atatattctt ttttttctta taaaacttaa aaccttagag gctatttaag ttgctgattt 840
   atattaattt tattgttcaa acatgagagc ttagtacgtg aaacatgaga gcttagtacg 900
   ttagccatga gagcttagta cgttagccat gagggtttag ttcgttaaac atgagagctt 960
   agtacgttaa acatgagagc ttagtacgtg aaacatgaga gcttagtacg tactatcaac 1020
   aggttgaact gctgatcttc agatcctcta cgccggacgc atcgtggccg gatccgattt 1080
   attcaacaaa gccacgttgt gtctcaaaat ctctgatgtt acattgcaca agataaaaat 1140
   atatcatcat gaacaataaa actgtctgct tacataaaca gtaatacaag gggtgttatg 1200
   agccatattc aacgggaaac gtcttgctcg aggccgcgat taaattccaa catggatgct 1260
   gatttatatg ggtataaatg ggctcgcgat aatgtcgggc aatcaggtgc gacaatctat 1320
   cgattgtatg ggaagcccga tgcgccagag ttgtttctga aacatggcaa aggtagcgtt 1380
   gccaatgatg ttacagatga gatggtcaga ctaaactggc tgacggaatt tatgcctctt 1440
   ccgaccatca agcattttat ccgtactcct gatgatgcat ggttactcac cactgcgatc 1500
   cccgggaaaa cagcattcca ggtattagaa gaatatcctg attcaggtga aaatattgtt 1560
   gatgcgctgg cagtgttcct gcgccggttg cattcgattc ctgtttgtaa ttgtcctttt 1620
   aacagcgatc gcgtatttcg tctcgctcag gcgcaatcac gaatgaataa cggtttggtt 1680
   gatgcgagtg attttgatga cgagcgtaat ggctggcctg ttgaacaagt ctggaaagaa 1740
   atgcataagc ttttgccatt ctcaccggat tcagtcgtca ctcatggtga tttctcactt 1800
   gataacctta tttttgacga ggggaaatta ataggttgta ttgatgttgg acgagtcgga 1860
   atcgcagacc gataccagga tcttgccatc ctatggaact gcctcggtga gttttctcct 1920
   tcattacaga aacggctttt tcaaaaatat ggtattgata atcctgatat gaataaattg 1980
   cagtttcatt tgatgctcga tgagtttttc taatcagaat tggttaattg gttgtaacac 2040
   tggcttaatt aacatcatca ataatatacc ttattttgga ttgaagccaa tatgataatg 2100
   agggggtgga gtttgtgacg tggcgcgggg cgtgggaacg gggcgggtga cgtagtagtg 2160
   tggcggaagt gtgatgttgc aagtgtggcg gaacacatgt aagcgacgga tgtggcaaaa 2220
   gtgacgtttt tggtgtgcgc cggtgtacac aggaagtgac aattttcgcg cggttttagg 2280
   cggatgttgt agtaaatttg ggcgtaaccg agtaagattt ggccattttc gcgggaaaac 2340
   tgaataagag gaagtgaaat ctgaataatt ttgtgttact catagcgcgt aatactggta 2400
   ccccccagtg gcaagacgcg caggcaaaac gcaccacgac tggatccgaa ttcgaagacc 2460
   acggtgtttc gtcctttcca caagatatat aaagccaaga aatcgaaata ctttcaagtt 2520
   acggtaagca tatgatagtc cattttaaaa cataatttta aaactgcaaa ctacccaaga 2580
   aattattact ttctacgtca cgtattttgt actaatatct ttgtgtttac agtcaaatta 2640
   attctaatta tctttctaac agccttgtat cgtatatgca aatatgaagg aatcatggga 2700
   aataggccct cttcctgccc gaccttggcg cgcgctcggc gcgcggtcac gctccgtcac 2760
   gtggtgcgtt ttgcctgcgc gtcttgccac tggggggtac cccgaatcac tagatctgag 2820
   ctcggcctgg tgatgatggc gggatcgttg tatatttctt gacacctttt cggcatcgcc 2880
   ctaaaattcg gcgtcctcat attgtgtgag gacgttttat tacgtgttta cgaagcaaaa 2940
   gctaaaacca ggagctattt aatggcaaca accggtacca tgattacgcc aagctacgta 3000
   atacgactca ctagtggaag ttcctattct ctagaaagta taggaacttc cttaagtgag 3060
   tcgtattacg gactggccgt cgttttacaa cgtcgtgact gggaaaaccc tggcgttacc 3120
   caacttaatc gccttgcagc acatccccct ttcgccagct ggcgtaatag cgaagaggcc 3180
   cgcaccgatc gcccttccca acagttgcgc agcctgaatg gcgaatggcg cttcgcttgg 3240
   taagctagct aaggcttaag gaggacaatc atggcaacag ttaaccagct ggtacgcaaa 3300
   ccacgtgctc gcaaagttgc gaaaagcaac gtgcctgcgc tggaagcatg cccgcaaaaa 3360
   cgtggcgtat gtactcgtgt atatactacc actcctaaaa aaccgaactc cgcgctgcgt 3420
   aaagtatgcc gtgttcgtct gtctaacggt ttcgaagtga cttcctacat cggtggtgaa 3480
   ggtcacaacc tgcaggagca ctccgtgatc ctgatccgtg gcggtcgtgt taaagacctc 3540
   ccgggtgttc gttaccacac cgtacgtggt gcgcttgact gctccggcgt taaagaccgt 3600
   aagcaggctc gttccaagta tggcgtgaag cgtcctaagg cttaactagc acgcgccatg 3660
   tcagccgtta agtgttcctg tgtcactcaa aattgctttg agaggctcta agggcttctc 3720
   agtgcgttac atccctggct tgttgtccac aaccgttaaa ccttaaaagc tttaaaagcc 3780
   ttatatattc ttttttttct tataaaactt aaaaccttag aggctattta agttgctgat 3840
   ttatattaat tttattgttc aaacatgaga gcttagtacg tgaaacatga gagcttagta 3900
   cgttagccat gagagcttag tacgttagcc atgagggttt agttcgttaa acatgagagc 3960
   ttagtacgtt aaacatgaga gcttagtacg tgaaacatga gagcttagta cgtactatca 4020
   acaggttgaa ctgctgatct tcagatcctc tacgccggac gcatcgtggc cggatccgat 4080
   ttattcaaca aagccacgtt gtgtctcaaa atctctgatg ttacattgca caagataaaa 4140
   atatatcatc atgaacaata aaactgtctg cttacataaa cagtaataca aggggtgtta 4200
   tgagccatat tcaacgggaa acgtcttgct cgaggccgcg attaaattcc aacatggatg 4260
   ctgatttata tgggtataaa tgggctcgcg ataatgtcgg gcaatcaggt gcgacaatct 4320
   atcgattgta tgggaagccc gatgcgccag agttgtttct gaaacatggc aaaggtagcg 4380
   ttgccaatga tgttacagat gagatggtca gactaaactg gctgacggaa tttatgcctc 4440
   ttccgaccat caagcatttt atccgtactc ctgatgatgc atggttactc accactgcga 4500
   tccccgggaa aacagcattc caggtattag aagaatatcc tgattcaggt gaaaatattg 4560
   ttgatgcgct ggcagtgttc ctgcgccggt tgcattcgat tcctgtttgt aattgtcctt 4620
   ttaacagcga tcgcgtattt cgtctcgctc aggcgcaatc acgaatgaat aacggtttgg 4680
   ttgatgcgag tgattttgat gacgagcgta atggctggcc tgttgaacaa gtctggaaag 4740
   aaatgcataa gcttttgcca ttctcaccgg attcagtcgt cactcatggt gatttctcac 4800
   ttgataacct tatttttgac gaggggaaat taataggttg tattgatgtt ggacgagtcg 4860
   gaatcgcaga ccgataccag gatcttgcca tcctatggaa ctgcctcggt gagttttctc 4920
   cttcattaca gaaacggctt tttcaaaaat atggtattga taatcctgat atgaataaat 4980
   tgcagtttca tttgatgctc gatgagtttt tctaatcaga attggttaat tggttgtaac 5040
   actggcttaa ttaacatcat caataatata ccttattttg gattgaagcc aatatgataa 5100
   tgagggggtg gagtttgtga cgtggcgcgg ggcgtgggaa cggggcgggt gacgtagtag 5160
   tgtggcggaa gtgtgatgtt gcaagtgtgg cggaacacat gtaagcgacg gatgtggcaa 5220
   aagtgacgtt tttggtgtgc gccggtgtac acaggaagtg acaattttcg cgcggtttta 5280
   ggcggatgtt gtagtaaatt tgggcgtaac cgagtaagat ttggccattt tcgcgggaaa 5340
   actgaataag aggaagtgaa atctgaataa ttttgtgtta ctcatagcgc gtaatactgg 5400
   taccccccag tggcaagacg cgcaggcaaa acgcaccacg actggatccg aattcgaaga 5460
   ccacggtgtt tcgtcctttc cacaagatat ataaagccaa gaaatcgaaa tactttcaag 5520
   ttacggtaag catatgatag tccattttaa aacataattt taaaactgca aactacccaa 5580
   gaaattatta ctttctacgt cacgtatttt gtactaatat ctttgtgttt acagtcaaat 5640
   taattctaat tatctttcta acagccttgt atcgtatatg caaatatgaa ggaatcatgg 5700
   gaaataggcc ctcttcctgc ccgaccttgg cgcgcgctcg gcgcgcggtc acgctccgtc 5760
   acgtggtgcg ttttgcctgc gcgtcttgcc actggggggt accccgaatc actagatctg 5820
   agctcggcct ggtgatgatg gcgggatcgt tgtatatttc ttgacacctt ttcggcatcg 5880
   ccctaaaatt cggcgtcctc atattgtgtg aggacgtttt attacgtgtt tacgaagcaa 5940
   aagctaaaac caggagctat ttaatggcaa caaccggtac catgattacg ccaagctacg 6000
   taatacgact cactagtgga agttcctatt ctctagaaag tataggaact tcagagcgct 6060
   tttgaagctg gggtgggcga agaactccag catgagatcc ccagagcgct tttgaagctg 6120
   cgtttaaacg cgatatcccg ggagctcccg atatcgcgtt taaacgcagc ttggcgtaat 6180
   catggtcata gctgtttcct gtgtgaaatt gttatccgct cacaattcca cacaacatac 6240
   gagccggaag actgaaatgt gtgggcgtgg cttaagggtg ggaaagaata tataaggtgg 6300
   gggtcttatg tagttttgta tctgttttgc agcagccgcc gccgccatga gcaccaactc 6360
   gtttgatgga agcattgtga gctcatattt gacaacgcgc atgcccccat gggccggggt 6420
   gcgtcagaat gtgatgggct ccagcattga tggtcgcccc gtcctgcccg caaactctac 6480
   taccttgacc tacgagaccg tgtctggaac gccgttggag actgcagcct ccgccgccgc 6540
   ttcagccgct gcagccaccg cccgcgggat tgtgactgac tttgctttcc tgagcccgct 6600
   tgcaagcagt gcagcttccc gttcatccgc ccgcgatgac aagttgacgg ctcttttggc 6660
   acaattggat tctttgaccc gggaacttaa tgtcgtttct cagcagctgt tggatctgcg 6720
   ccagcaggtt tctgccctga aggcttcctc ccctcccaat gcggtttaaa acataaataa 6780
   aaaaccagac tctgtttgga tttggatcaa gcaagtgtct tgctgtcttt atttaggggt 6840
   tttgcgcgcg cggtaggccc gggaccagcg gtctcggtcg ttgagggtcc tgtgtatttt 6900
   ttccaggacg tggtaaaggt gactctggat gttcagatac atgggcataa gcccgtctct 6960
   ggggtggagg tagcaccact gcagagcttc atgctgcggg gtggtgttgt agatgatcca 7020
   gtcgtagcag gagcgctggg cgtggtgcct aaaaatgtct ttcagtagca agctgattgc 7080
   caggggcagg cccttggtgt aagtgtttac aaagcggtta agctgggatg ggtgcatacg 7140
   tggggatatg agatgcatct tggactgtat ttttaggttg gctatgttcc cagccatatc 7200
   cctccgggga ttcatgttgt gcagaaccac cagcacagtg tatccggtgc acttgggaaa 7260
   tttgtcatgt agcttagaag gaaatgcgtg gaagaacttg gagacgccct tgtgacctcc 7320
   aagattttcc atgcattcgt ccataatgat ggcaatgggc ccacgggcgg cggcctgggc 7380
   gaagatattt ctgggatcac taacgtcata gttgtgttcc aggatgagat cgtcataggc 7440
   catttttaca aagcgcgggc ggagggtgcc agactgcggt ataatggttc catccggccc 7500
   aggggcgtag ttaccctcac agatttgcat ttcccacgct ttgagttcag atggggggat 7560
   catgtctacc tgcggggcga tgaagaaaac ggtttccggg gtaggggaga tcagctggga 7620
   agaaagcagg ttcctgagca gctgcgactt accgcagccg gtgggcccgt aaatcacacc 7680
   tattaccggg tgcaactggt agttaagaga gctgcagctg ccgtcatccc tgagcagggg 7740
   ggccacttcg ttaagcatgt ccctgactcg catgttttcc ctgaccaaat ccgccagaag 7800
   gcgctcgccg cccagcgata gcagttcttg caaggaagca aagtttttca acggtttgag 7860
   accgtccgcc gtaggcatgc ttttgagcgt ttgaccaagc agttccaggc ggtcccacag 7920
   ctcggtcacc tgctctacgg catctcgatc cagcatatct cctcgtttcg cgggttgggg 7980
   cggctttcgc tgtacggcag tagtcggtgc tcgtccagac gggccagggt catgtctttc 8040
   cacgggcgca gggtcctcgt cagcgtagtc tgggtcacgg tgaaggggtg cgctccgggc 8100
   tgcgcgctgg ccagggtgcg cttgaggctg gtcctgctgg tgctgaagcg ctgccggtct 8160
   tcgccctgcg cgtcggccag gtagcatttg accatggtgt catagtccag cccctccgcg 8220
   gcgtggccct tggcgcgcag cttgcccttg gaggaggcgc cgcacgaggg gcagtgcaga 8280
   cttttgaggg cgtagagctt gggcgcgaga aataccgatt ccggggagta ggcatccgcg 8340
   ccgcaggccc cgcagacggt ctcgcattcc acgagccagg tgagctctgg ccgttcgggg 8400
   tcaaaaacca ggtttccccc atgctttttg atgcgtttct tacctctggt ttccatgagc 8460
   cggtgtccac gctcggtgac gaaaaggctg tccgtgtccc cgtatacaga cttgagaggc 8520
   ctgtcctcga gcggtgttcc gcggtcctcc tcgtatagaa actcggacca ctctgagaca 8580
   aaggctcgcg tccaggccag cacgaaggag gctaagtggg aggggtagcg gtcgttgtcc 8640
   actagggggt ccactcgctc cagggtgtga agacacatgt cgccctcttc ggcatcaagg 8700
   aaggtgattg gtttgtaggt gtaggccacg tgaccgggtg ttcctgaagg ggggctataa 8760
   aagggggtgg gggcgcgttc gtcctcactc tcttccgcat cgctgtctgc gagggccagc 8820
   tgttggggtg agtactccct ctgaaaagcg ggcatgactt ctgcgctaag attgtcagtt 8880
   tccaaaaacg aggaggattt gatattcacc tggcccgcgg tgatgccttt gagggtggcc 8940
   gcatccatct ggtcagaaaa gacaatcttt ttgttgtcaa gcttggtggc aaacgacccg 9000
   tagagggcgt tggacagcaa cttggcgatg gagcgcaggg tttggttttt gtcgcgatcg 9060
   gcgcgctcct tggccgcgat gtttagctgc acgtattcgc gcgcaacgca ccgccattcg 9120
   ggaaagacgg tggtgcgctc gtcgggcacc aggtgcacgc gccaaccgcg gttgtgcagg 9180
   gtgacaaggt caacgctggt ggctacctct ccgcgtaggc gctcgttggt ccagcagagg 9240
   cggccgccct tgcgcgagca gaatggcggt agggggtcta gctgcgtctc gtccgggggg 9300
   tctgcgtcca cggtaaagac cccgggcagc aggcgcgcgt cgaagtagtc tatcttgcat 9360
   ccttgcaagt ctagcgcctg ctgccatgcg cgggcggcaa gcgcgcgctc gtatgggttg 9420
   agtgggggac cccatggcat ggggtgggtg agcgcggagg cgtacatgcc gcaaatgtcg 9480
   taaacgtaga ggggctctct gagtattcca agatatgtag ggtagcatct tccaccgcgg 9540
   atgctggcgc gcacgtaatc gtatagttcg tgcgagggag cgaggaggtc gggaccgagg 9600
   ttgctacggg cgggctgctc tgctcggaag actatctgcc tgaagatggc atgtgagttg 9660
   gatgatatgg ttggacgctg gaagacgttg aagctggcgt ctgtgagacc taccgcgtca 9720
   cgcacgaagg aggcgtagga gtcgcgcagc ttgttgacca gctcggcggt gacctgcacg 9780
   tctagggcgc agtagtccag ggtttccttg atgatgtcat acttatcctg tccctttttt 9840
   ttccacagct cgcggttgag gacaaactct tcgcggtctt tccagtactc ttggatcgga 9900
   aacccgtcgg cctccgaacg gtaagagcct agcatgtaga actggttgac ggcctggtag 9960
   gcgcagcatc ccttttctac gggtagcgcg tatgcctgcg cggccttccg gagcgaggtg 10020
   tgggtgagcg caaaggtgtc cctgaccatg actttgaggt actggtattt gaagtcagtg 10080
   tcgtcgcatc cgccctgctc ccagagcaaa aagtccgtgc gctttttgga acgcggattt 10140
   ggcagggcga aggtgacatc gttgaagagt atctttcccg cgcgaggcat aaagttgcgt 10200
   gtgatgcgga agggtcccgg cacctcggaa cggttgttaa ttacctgggc ggcgagcacg 10260
   atctcgtcaa agccgttgat gttgtggccc acaatgtaaa gttccaagaa gcgcgggatg 10320
   cccttgatgg aaggcaattt tttaagttcc tcgtaggtga gctcttcagg ggagctgagc 10380
   ccgtgctctg aaagggccca gtctgcaaga tgagggttgg aagcgacgaa tgagctccac 10440
   aggtcacggg ccattagcat ttgcaggtgg tcgcgaaagg tcctaaactg gcgacctatg 10500
   gccatttttt ctggggtgat gcagtagaag gtaagcgggt cttgttccca gcggtcccat 10560
   ccaaggttcg cggctaggtc tcgcgcggca gtcactagag gctcatctcc gccgaacttc 10620
   atgaccagca tgaagggcac gagctgcttc ccaaaggccc ccatccaagt ataggtctct 10680
   acatcgtagg tgacaaagag acgctcggtg cgaggatgcg agccgatcgg gaagaactgg 10740
   atctcccgcc accaattgga ggagtggcta ttgatgtggt gaaagtagaa gtccctgcga 10800
   cgggccgaac actcgtgctg gcttttgtaa aaacgtgcgc agtactggca gcggtgcacg 10860
   ggctgtacat cctgcacgag gttgacctga cgaccgcgca caaggaagca gagtgggaat 10920
   ttgagcccct cgcctggcgg gtttggctgg tggtcttcta cttcggctgc ttgtccttga 10980
   ccgtctggct gctcgagggg agttacggtg gatcggacca ccacgccgcg cgagcccaaa 11040
   gtccagatgt ccgcgcgcgg cggtcggagc ttgatgacaa catcgcgcag atgggagctg 11100
   tccatggtct ggagctcccg cggcgtcagg tcaggcggga gctcctgcag gtttacctcg 11160
   catagacggg tcagggcgcg ggctagatcc aggtgatacc taatttccag gggctggttg 11220
   gtggcggcgt cgatggcttg caagaggccg catccccgcg gcgcgactac ggtaccgcgc 11280
   ggcgggcggt gggccgcggg ggtgtccttg gatgatgcat ctaaaagcgg tgacgcgggc 11340
   gagcccccgg aggtaggggg ggctccggac ccgccgggag agggggcagg ggcacgtcgg 11400
   cgccgcgcgc gggcaggagc tggtgctgcg cgcgtaggtt gctggcgaac gcgacgacgc 11460
   ggcggttgat ctcctgaatc tggcgcctct gcgtgaagac gacgggcccg gtgagcttga 11520
   gcctgaaaga gagttcgaca gaatcaattt cggtgtcgtt gacggcggcc tggcgcaaaa 11580
   tctcctgcac gtctcctgag ttgtcttgat aggcgatctc ggccatgaac tgctcgatct 11640
   cttcctcctg gagatctccg cgtccggctc gctccacggt ggcggcgagg tcgttggaaa 11700
   tgcgggccat gagctgcgag aaggcgttga ggcctccctc gttccagacg cggctgtaga 11760
   ccacgccccc ttcggcatcg cgggcgcgca tgaccacctg cgcgagattg agctccacgt 11820
   gccgggcgaa gacggcgtag tttcgcaggc gctgaaagag gtagttgagg gtggtggcgg 11880
   tgtgttctgc cacgaagaag tacataaccc agcgtcgcaa cgtggattcg ttgatatccc 11940
   ccaaggcctc aaggcgctcc atggcctcgt agaagtccac ggcgaagttg aaaaactggg 12000
   agttgcgcgc cgacacggtt aactcctcct ccagaagacg gatgagctcg gcgacagtgt 12060
   cgcgcacctc gcgctcaaag gctacagggg cctcttcttc ttcttcaatc tcctcttcca 12120
   taagggcctc cccttcttct tcttctggcg gcggtggggg agggggacac cggcggcgac 12180
   gacggcgcac cgggaggcgg tcgacaaagc gctcgatcat ctccccgcgg cgacggcgca 12240
   tggtctcggt gacggcgcgg ccgttctcgc gggggcgcag ttggaagacg ccgcccgtca 12300
   tgtcccggtt atgggttggc ggggggctgc catgcggcag ggatacggcg ctaacgatgc 12360
   atctcaacaa ttgttgtgta ggtactccgc cgccgaggga cctgagcgag tccgcatcga 12420
   ccggatcgga aaacctctcg agaaaggcgt ctaaccagtc acagtcgcaa ggtaggctga 12480
   gcaccgtggc gggcggcagc gggcggcggt cggggttgtt tctggcggag gtgctgctga 12540
   tgatgtaatt aaagtaggcg gtcttgagac ggcggatggt cgacagaagc accatgtcct 12600
   tgggtccggc ctgctgaatg cgcaggcggt cggccatgcc ccaggcttcg ttttgacatc 12660
   ggcgcaggtc tttgtagtag tcttgcatga gcctttctac cggcacttct tcttctcctt 12720
   cctcttgtcc tgcatctctt gcatctatcg ctgcggcggc ggcggagttt ggccgtaggt 12780
   ggcgccctct tcctcccatg cgtgtgaccc cgaagcccct catcggctga agcagggcta 12840
   ggtcggcgac aacgcgctcg gctaatatgg cctgctgcac ctgcgtgagg gtagactgga 12900
   agtcatccat gtccacaaag cggtggtatg cgcccgtgtt gatggtgtaa gtgcagttgg 12960
   ccataacgga ccagttaacg gtctggtgac ccggctgcga gagctcggtg tacctgagac 13020
   gcgagtaagc cctcgagtca aatacgtagt cgttgcaagt ccgcaccagg tactggtatc 13080
   ccaccaaaaa gtgcggcggc ggctggcggt agaggggcca gcgtagggtg gccggggctc 13140
   cgggggcgag atcttccaac ataaggcgat gatatccgta gatgtacctg gacatccagg 13200
   tgatgccggc ggcggtggtg gaggcgcgcg gaaagtcgcg gacgcggttc cagatgttgc 13260
   gcagcggcaa aaagtgctcc atggtcggga cgctctggcc ggtcaggcgc gcgcaatcgt 13320
   tgacgctcta gaccgtgcaa aaggagagcc tgtaagcggg cactcttccg tggtctggtg 13380
   gataaattcg caagggtatc atggcggacg accggggttc gagccccgta tccggccgtc 13440
   cgccgtgatc catgcggtta ccgcccgcgt gtcgaaccca ggtgtgcgac gtcagacaac 13500
   gggggagtgc tccttttggc ttccttccag gcgcggcggc tgctgcgcta gcttttttgg 13560
   ccactggccg cgcgcagcgt aagcggttag gctggaaagc gaaagcatta agtggctcgc 13620
   tccctgtagc cggagggtta ttttccaagg gttgagtcgc gggacccccg gttcgagtct 13680
   cggaccggcc ggactgcggc gaacgggggt ttgcctcccc gtcatgcaag accccgcttg 13740
   caaattcctc cggaaacagg gacgagcccc ttttttgctt ttcccagatg catccggtgc 13800
   tgcggcagat gcgcccccct cctcagcagc ggcaagagca agagcagcgg cagacatgca 13860
   gggcaccctc ccctcctcct accgcgtcag gaggggcgac atccgcggtt gacgcggcag 13920
   cagatggtga ttacgaaccc ccgcggcgcc gggcccggca ctacctggac ttggaggagg 13980
   gcgagggcct ggcgcggcta ggagcgccct ctcctgagcg gtacccaagg gtgcagctga 14040
   agcgtgatac gcgtgaggcg tacgtgccgc ggcagaacct gtttcgcgac cgcgagggag 14100
   aggagcccga ggagatgcgg gatcgaaagt tccacgcagg gcgcgagctg cggcatggcc 14160
   tgaatcgcga gcggttgctg cgcgaggagg actttgagcc cgacgcgcga accgggatta 14220
   gtcccgcgcg cgcacacgtg gcggccgccg acctggtaac cgcatacgag cagacggtga 14280
   accaggagat taactttcaa aaaagcttta acaaccacgt gcgtacgctt gtggcgcgcg 14340
   aggaggtggc tataggactg atgcatctgt gggactttgt aagcgcgctg gagcaaaacc 14400
   caaatagcaa gccgctcatg gcgcagctgt tccttatagt gcagcacagc agggacaacg 14460
   aggcattcag ggatgcgctg ctaaacatag tagagcccga gggccgctgg ctgctcgatt 14520
   tgataaacat cctgcagagc atagtggtgc aggagcgcag cttgagcctg gctgacaagg 14580
   tggccgccat caactattcc atgcttagcc tgggcaagtt ttacgcccgc aagatatacc 14640
   atacccctta cgttcccata gacaaggagg taaagatcga ggggttctac atgcgcatgg 14700
   cgctgaaggt gcttaccttg agcgacgacc tgggcgttta tcgcaacgag cgcatccaca 14760
   aggccgtgag cgtgagccgg cggcgcgagc tcagcgaccg cgagctgatg cacagcctgc 14820
   aaagggccct ggctggcacg ggcagcggcg atagagaggc cgagtcctac tttgacgcgg 14880
   gcgctgacct gcgctgggcc ccaagccgac gcgccctgga ggcagctggg gccggacctg 14940
   ggctggcggt ggcacccgcg cgcgctggca acgtcggcgg cgtggaggaa tatgacgagg 15000
   acgatgagta cgagccagag gacggcgagt actaagcggt gatgtttctg atcagatgat 15060
   gcaagacgca acggacccgg cggtgcgggc ggcgctgcag agccagccgt ccggccttaa 15120
   ctccacggac gactggcgcc aggtcatgga ccgcatcatg tcgctgactg cgcgcaatcc 15180
   tgacgcgttc cggcagcagc cgcaggccaa ccggctctcc gcaattctgg aagcggtggt 15240
   cccggcgcgc gcaaacccca cgcacgagaa ggtgctggcg atcgtaaacg cgctggccga 15300
   aaacaggccc atccggcccg acgaggccgg cctggtctac gacgcgctgc ttcagcgcgt 15360
   ggctcgttac aacagcggca acgtgcagac caacctggac cggctggtgg gggatgtgcg 15420
   cgaggccgtg gcgcagcgtg agcgcgcgca gcagcagggc aacctgggct ccatggttgc 15480
   actaaacgcc ttcctgagta cacagcccgc caacgtgccg cggggacagg aggactacac 15540
   caactttgtg agcgcactgc ggctaatggt gactgagaca ccgcaaagtg aggtgtacca 15600
   gtctgggcca gactattttt tccagaccag tagacaaggc ctgcagaccg taaacctgag 15660
   ccaggctttc aaaaacttgc aggggctgtg gggggtgcgg gctcccacag gcgaccgcgc 15720
   gaccgtgtct agcttgctga cgcccaactc gcgcctgttg ctgctgctaa tagcgccctt 15780
   cacggacagt ggcagcgtgt cccgggacac atacctaggt cacttgctga cactgtaccg 15840
   cgaggccata ggtcaggcgc atgtggacga gcatactttc caggagatta caagtgtcag 15900
   ccgcgcgctg gggcaggagg acacgggcag cctggaggca accctaaact acctgctgac 15960
   caaccggcgg cagaagatcc cctcgttgca cagtttaaac agcgaggagg agcgcatttt 16020
   gcgctacgtg cagcagagcg tgagccttaa cctgatgcgc gacggggtaa cgcccagcgt 16080
   ggcgctggac atgaccgcgc gcaacatgga accgggcatg tatgcctcaa accggccgtt 16140
   tatcaaccgc ctaatggact acttgcatcg cgcggccgcc gtgaaccccg agtatttcac 16200
   caatgccatc ttgaacccgc actggctacc gccccctggt ttctacaccg ggggattcga 16260
   ggtgcccgag ggtaacgatg gattcctctg ggacgacata gacgacagcg tgttttcccc 16320
   gcaaccgcag accctgctag agttgcaaca gcgcgagcag gcagaggcgg cgctgcgaaa 16380
   ggaaagcttc cgcaggccaa gcagcttgtc cgatctaggc gctgcggccc cgcggtcaga 16440
   tgctagtagc ccatttccaa gcttgatagg gtctcttacc agcactcgca ccacccgccc 16500
   gcgcctgctg ggcgaggagg agtacctaaa caactcgctg ctgcagccgc agcgcgaaaa 16560
   aaacctgcct ccggcatttc ccaacaacgg gatagagagc ctagtggaca agatgagtag 16620
   atggaagacg tacgcgcagg agcacaggga cgtgccaggc ccgcgcccgc ccacccgtcg 16680
   tcaaaggcac gaccgtcagc ggggtctggt gtgggaggac gatgactcgg cagacgacag 16740
   cagcgtcctg gatttgggag ggagtggcaa cccgtttgcg caccttcgcc ccaggctggg 16800
   gagaatgttt taaaaaaaaa aaagcatgat gcaaaataaa aaactcacca aggccatggc 16860
   accgagcgtt ggttttcttg tattcccctt agtatgcggc gcgcggcgat gtatgaggaa 16920
   ggtcctcctc cctcctacga gagtgtggtg agcgcggcgc cagtggcggc ggcgctgggt 16980
   tctcccttcg atgctcccct ggacccgccg tttgtgcctc cgcggtacct gcggcctacc 17040
   ggggggagaa acagcatccg ttactctgag ttggcacccc tattcgacac cacccgtgtg 17100
   tacctggtgg acaacaagtc aacggatgtg gcatccctga actaccagaa cgaccacagc 17160
   aactttctga ccacggtcat tcaaaacaat gactacagcc cgggggaggc aagcacacag 17220
   accatcaatc ttgacgaccg gtcgcactgg ggcggcgacc tgaaaaccat cctgcatacc 17280
   aacatgccaa atgtgaacga gttcatgttt accaataagt ttaaggcgcg ggtgatggtg 17340
   tcgcgcttgc ctactaagga caatcaggtg gagctgaaat acgagtgggt ggagttcacg 17400
   ctgcccgagg gcaactactc cgagaccatg accatagacc ttatgaacaa cgcgatcgtg 17460
   gagcactact tgaaagtggg cagacagaac ggggttctgg aaagcgacat cggggtaaag 17520
   tttgacaccc gcaacttcag actggggttt gaccccgtca ctggtcttgt catgcctggg 17580
   gtatatacaa acgaagcctt ccatccagac atcattttgc tgccaggatg cggggtggac 17640
   ttcacccaca gccgcctgag caacttgttg ggcatccgca agcggcaacc cttccaggag 17700
   ggctttagga tcacctacga tgatctggag ggtggtaaca ttcccgcact gttggatgtg 17760
   gacgcctacc aggcgagctt gaaagatgac accgaacagg gcgggggtgg cgcaggcggc 17820
   agcaacagca gtggcagcgg cgcggaagag aactccaacg cggcagccgc ggcaatgcag 17880
   ccggtggagg acatgaacga tcatgccatt cgcggcgaca cctttgccac acgggctgag 17940
   gagaagcgcg ctgaggccga agcagcggcc gaagctgccg cccccgctgc gcaacccgag 18000
   gtcgagaagc ctcagaagaa accggtgatc aaacccctga cagaggacag caagaaacgc 18060
   agttacaacc taataagcaa tgacagcacc ttcacccagt accgcagctg gtaccttgca 18120
   tacaactacg gcgaccctca gaccggaatc cgctcatgga ccctgctttg cactcctgac 18180
   gtaacctgcg gctcggagca ggtctactgg tcgttgccag acatgatgca agaccccgtg 18240
   accttccgct ccacgcgcca gatcagcaac tttccggtgg tgggcgccga gctgttgccc 18300
   gtgcactcca agagcttcta caacgaccag gccgtctact cccaactcat ccgccagttt 18360
   acctctctga cccacgtgtt caatcgcttt cccgagaacc agattttggc gcgcccgcca 18420
   gcccccacca tcaccaccgt cagtgaaaac gttcctgctc tcacagatca cgggacgcta 18480
   ccgctgcgca acagcatcgg aggagtccag cgagtgacca ttactgacgc cagacgccgc 18540
   acctgcccct acgtttacaa ggccctgggc atagtctcgc cgcgcgtcct atcgagccgc 18600
   actttttgag caagcatgtc catccttata tcgcccagca ataacacagg ctggggcctg 18660
   cgcttcccaa gcaagatgtt tggcggggcc aagaagcgct ccgaccaaca cccagtgcgc 18720
   gtgcgcgggc actaccgcgc gccctggggc gcgcacaaac gcggccgcac tgggcgcacc 18780
   accgtcgatg acgccatcga cgcggtggtg gaggaggcgc gcaactacac gcccacgccg 18840
   ccaccagtgt ccacagtgga cgcggccatt cagaccgtgg tgcgcggagc ccggcgctat 18900
   gctaaaatga agagacggcg gaggcgcgta gcacgtcgcc accgccgccg acccggcact 18960
   gccgcccaac gcgcggcggc ggccctgctt aaccgcgcac gtcgcaccgg ccgacgggcg 19020
   gccatgcggg ccgctcgaag gctggccgcg ggtattgtca ctgtgccccc caggtccagg 19080
   cgacgagcgg ccgccgcagc agccgcggcc attagtgcta tgactcaggg tcgcaggggc 19140
   aacgtgtatt gggtgcgcga ctcggttagc ggcctgcgcg tgcccgtgcg cacccgcccc 19200
   ccgcgcaact agattgcaag aaaaaactac ttagactcgt actgttgtat gtatccagcg 19260
   gcggcggcgc gcaacgaagc tatgtccaag cgcaaaatca aagaagagat gctccaggtc 19320
   atcgcgccgg agatctatgg ccccccgaag aaggaagagc aggattacaa gccccgaaag 19380
   ctaaagcggg tcaaaaagaa aaagaaagat gatgatgatg aacttgacga cgaggtggaa 19440
   ctgctgcacg ctaccgcgcc caggcgacgg gtacagtgga aaggtcgacg cgtaaaacgt 19500
   gttttgcgac ccggcaccac cgtagtcttt acgcccggtg agcgctccac ccgcacctac 19560
   aagcgcgtgt atgatgaggt gtacggcgac gaggacctgc ttgagcaggc caacgagcgc 19620
   ctcggggagt ttgcctacgg aaagcggcat aaggacatgc tggcgttgcc gctggacgag 19680
   ggcaacccaa cacctagcct aaagcccgta acactgcagc aggtgctgcc cgcgcttgca 19740
   ccgtccgaag aaaagcgcgg cctaaagcgc gagtctggtg acttggcacc caccgtgcag 19800
   ctgatggtac ccaagcgcca gcgactggaa gatgtcttgg aaaaaatgac cgtggaacct 19860
   gggctggagc ccgaggtccg cgtgcggcca atcaagcagg tggcgccggg actgggcgtg 19920
   cagaccgtgg acgttcagat acccactacc agtagcacca gtattgccac cgccacagag 19980
   ggcatggaga cacaaacgtc cccggttgcc tcagcggtgg cggatgccgc ggtgcaggcg 20040
   gtcgctgcgg ccgcgtccaa gacctctacg gaggtgcaaa cggacccgtg gatgtttcgc 20100
   gtttcagccc cccggcgccc gcgcggttcg aggaagtacg gcgccgccag cgcgctactg 20160
   cccgaatatg ccctacatcc ttccattgcg cctacccccg gctatcgtgg ctacacctac 20220
   cgccccagaa gacgagcaac tacccgacgc cgaaccacca ctggaacccg ccgccgccgt 20280
   cgccgtcgcc agcccgtgct ggccccgatt tccgtgcgca gggtggctcg cgaaggaggc 20340
   aggaccctgg tgctgccaac agcgcgctac caccccagca tcgtttaaaa gccggtcttt 20400
   gtggttcttg cagatatggc cctcacctgc cgcctccgtt tcccggtgcc gggattccga 20460
   ggaagaatgc accgtaggag gggcatggcc ggccacggcc tgacgggcgg catgcgtcgt 20520
   gcgcaccacc ggcggcggcg cgcgtcgcac cgtcgcatgc gcggcggtat cctgcccctc 20580
   cttattccac tgatcgccgc ggcgattggc gccgtgcccg gaattgcatc cgtggccttg 20640
   caggcgcaga gacactgatt aaaaacaagt tgcatgtgga aaaatcaaaa taaaaagtct 20700
   ggactctcac gctcgcttgg tcctgtaact attttgtaga atggaagaca tcaactttgc 20760
   gtctctggcc ccgcgacacg gctcgcgccc gttcatggga aactggcaag atatcggcac 20820
   cagcaatatg agcggtggcg ccttcagctg gggctcgctg tggagcggca ttaaaaattt 20880
   cggttccacc gttaagaact atggcagcaa ggcctggaac agcagcacag gccagatgct 20940
   gagggataag ttgaaagagc aaaatttcca acaaaaggtg gtagatggcc tggcctctgg 21000
   cattagcggg gtggtggacc tggccaacca ggcagtgcaa aataagatta acagtaagct 21060
   tgatccccgc cctcccgtag aggagcctcc accggccgtg gagacagtgt ctccagaggg 21120
   gcgtggcgaa aagcgtccgc gccccgacag ggaagaaact ctggtgacgc aaatagacga 21180
   gcctccctcg tacgaggagg cactaaagca aggcctgccc accacccgtc ccatcgcgcc 21240
   catggctacc ggagtgctgg gccagcacac acccgtaacg ctggacctgc ctccccccgc 21300
   cgacacccag cagaaacctg tgctgccagg cccgaccgcc gttgttgtaa cccgtcctag 21360
   ccgcgcgtcc ctgcgccgcg ccgccagcgg tccgcgatcg ttgcggcccg tagccagtgg 21420
   caactggcaa agcacactga acagcatcgt gggtctgggg gtgcaatccc tgaagcgccg 21480
   acgatgcttc tgaatagcta acgtgtcgta tgtgtgtcat gtatgcgtcc atgtcgccgc 21540
   cagaggagct gctgagccgc cgcgcgcccg ctttccaaga tggctacccc ttcgatgatg 21600
   ccgcagtggt cttacatgca catctcgggc caggacgcct cggagtacct gagccccggg 21660
   ctggtgcagt ttgcccgcgc caccgagacg tacttcagcc tgaataacaa gtttagaaac 21720
   cccacggtgg cgcctacgca cgacgtgacc acagaccggt cccagcgttt gacgctgcgg 21780
   ttcatccctg tggaccgtga ggatactgcg tactcgtaca aggcgcggtt caccctagct 21840
   gtgggtgata accgtgtgct ggacatggct tccacgtact ttgacatccg cggcgtgctg 21900
   gacaggggcc ctacttttaa gccctactct ggcactgcct acaacgccct ggctcccaag 21960
   ggtgccccaa atccttgcga atgggatgaa gctgctactg ctcttgaaat aaacctagaa 22020
   gaagaggacg atgacaacga agacgaagta gacgagcaag ctgagcagca aaaaactcac 22080
   gtatttgggc aggcgcctta ttctggtata aatattacaa aggagggtat tcaaataggt 22140
   gtcgaaggtc aaacacctaa atatgccgat aaaacatttc aacctgaacc tcaaatagga 22200
   gaatctcagt ggtacgaaac tgaaattaat catgcagctg ggagagtcct taaaaagact 22260
   accccaatga aaccatgtta cggttcatat gcaaaaccca caaatgaaaa tggagggcaa 22320
   ggcattcttg taaagcaaca aaatggaaag ctagaaagtc aagtggaaat gcaatttttc 22380
   tcaactactg aggcgaccgc aggcaatggt gataacttga ctcctaaagt ggtattgtac 22440
   agtgaagatg tagatataga aaccccagac actcatattt cttacatgcc cactattaag 22500
   gaaggtaact cacgagaact aatgggccaa caatctatgc ccaacaggcc taattacatt 22560
   gcttttaggg acaattttat tggtctaatg tattacaaca gcacgggtaa tatgggtgtt 22620
   ctggcgggcc aagcatcgca gttgaatgct gttgtagatt tgcaagacag aaacacagag 22680
   ctttcatacc agcttttgct tgattccatt ggtgatagaa ccaggtactt ttctatgtgg 22740
   aatcaggctg ttgacagcta tgatccagat gttagaatta ttgaaaatca tggaactgaa 22800
   gatgaacttc caaattactg ctttccactg ggaggtgtga ttaatacaga gactcttacc 22860
   aaggtaaaac ctaaaacagg tcaggaaaat ggatgggaaa aagatgctac agaattttca 22920
   gataaaaatg aaataagagt tggaaataat tttgccatgg aaatcaatct aaatgccaac 22980
   ctgtggagaa atttcctgta ctccaacata gcgctgtatt tgcccgacaa gctaaagtac 23040
   agtccttcca acgtaaaaat ttctgataac ccaaacacct acgactacat gaacaagcga 23100
   gtggtggctc ccgggttagt ggactgctac attaaccttg gagcacgctg gtcccttgac 23160
   tatatggaca acgtcaaccc atttaaccac caccgcaatg ctggcctgcg ctaccgctca 23220
   atgttgctgg gcaatggtcg ctatgtgccc ttccacatcc aggtgcctca gaagttcttt 23280
   gccattaaaa acctccttct cctgccgggc tcatacacct acgagtggaa cttcaggaag 23340
   gatgttaaca tggttctgca gagctcccta ggaaatgacc taagggttga cggagccagc 23400
   attaagtttg atagcatttg cctttacgcc accttcttcc ccatggccca caacaccgcc 23460
   tccacgcttg aggccatgct tagaaacgac accaacgacc agtcctttaa cgactatctc 23520
   tccgccgcca acatgctcta ccctataccc gccaacgcta ccaacgtgcc catatccatc 23580
   ccctcccgca actgggcggc tttccgcggc tgggccttca cgcgccttaa gactaaggaa 23640
   accccatcac tgggctcggg ctacgaccct tattacacct actctggctc tataccctac 23700
   ctagatggaa ccttttacct caaccacacc tttaagaagg tggccattac ctttgactct 23760
   tctgtcagct ggcctggcaa tgaccgcctg cttaccccca acgagtttga aattaagcgc 23820
   tcagttgacg gggagggtta caacgttgcc cagtgtaaca tgaccaaaga ctggttcctg 23880
   gtacaaatgc tagctaacta caacattggc taccagggct tctatatccc agagagctac 23940
   aaggaccgca tgtactcctt ctttagaaac ttccagccca tgagccgtca ggtggtggat 24000
   gatactaaat acaaggacta ccaacaggtg ggcatcctac accaacacaa caactctgga 24060
   tttgttggct accttgcccc caccatgcgc gaaggacagg cctaccctgc taacttcccc 24120
   tatccgctta taggcaagac cgcagttgac agcattaccc agaaaaagtt tctttgcgat 24180
   cgcacccttt ggcgcatccc attctccagt aactttatgt ccatgggcgc actcacagac 24240
   ctgggccaaa accttctcta cgccaactcc gcccacgcgc tagacatgac ttttgaggtg 24300
   gatcccatgg acgagcccac ccttctttat gttttgtttg aagtctttga cgtggtccgt 24360
   gtgcaccggc cgcaccgcgg cgtcatcgaa accgtgtacc tgcgcacgcc cttctcggcc 24420
   ggcaacgcca caacataaag aagcaagcaa catcaacaac agctgccgcc atgggctcca 24480
   gtgagcagga actgaaagcc attgtcaaag atcttggttg tgggccatat tttttgggca 24540
   cctatgacaa gcgctttcca ggctttgttt ctccacacaa gctcgcctgc gccatagtca 24600
   atacggccgg tcgcgagact gggggcgtac actggatggc ctttgcctgg aacccgcact 24660
   caaaaacatg ctacctcttt gagccctttg gcttttctga ccagcgactc aagcaggttt 24720
   accagtttga gtacgagtca ctcctgcgcc gtagcgccat tgcttcttcc cccgaccgct 24780
   gtataacgct ggaaaagtcc acccaaagcg tacaggggcc caactcggcc gcctgtggac 24840
   tattctgctg catgtttctc cacgcctttg ccaactggcc ccaaactccc atggatcaca 24900
   accccaccat gaaccttatt accggggtac ccaactccat gctcaacagt ccccaggtac 24960
   agcccaccct gcgtcgcaac caggaacagc tctacagctt cctggagcgc cactcgccct 25020
   acttccgcag ccacagtgcg cagattagga gcgccacttc tttttgtcac ttgaaaaaca 25080
   tgtaaaaata atgtactaga gacactttca ataaaggcaa atgcttttat ttgtacactc 25140
   tcgggtgatt atttaccccc acccttgccg tctgcgccgt ttaaaaatca aaggggttct 25200
   gccgcgcatc gctatgcgcc actggcaggg acacgttgcg atactggtgt ttagtgctcc 25260
   acttaaactc aggcacaacc atccgcggca gctcggtgaa gttttcactc cacaggctgc 25320
   gcaccatcac caacgcgttt agcaggtcgg gcgccgatat cttgaagtcg cagttggggc 25380
   ctccgccctg cgcgcgcgag ttgcgataca cagggttgca gcactggaac actatcagcg 25440
   ccgggtggtg cacgctggcc agcacgctct tgtcggagat cagatccgcg tccaggtcct 25500
   ccgcgttgct cagggcgaac ggagtcaact ttggtagctg ccttcccaaa aagggcgcgt 25560
   gcccaggctt tgagttgcac tcgcaccgta gtggcatcaa aaggtgaccg tgcccggtct 25620
   gggcgttagg atacagcgcc tgcataaaag ccttgatctg cttaaaagcc acctgagcct 25680
   ttgcgccttc agagaagaac atgccgcaag acttgccgga aaactgattg gccggacagg 25740
   ccgcgtcgtg cacgcagcac cttgcgtcgg tgttggagat ctgcaccaca tttcggcccc 25800
   accggttctt cacgatcttg gccttgctag actgctcctt cagcgcgcgc tgcccgtttt 25860
   cgctcgtcac atccatttca atcacgtgct ccttatttat cataatgctt ccgtgtagac 25920
   acttaagctc gccttcgatc tcagcgcagc ggtgcagcca caacgcgcag cccgtgggct 25980
   cgtgatgctt gtaggtcacc tctgcaaacg actgcaggta cgcctgcagg aatcgcccca 26040
   tcatcgtcac aaaggtcttg ttgctggtga aggtcagctg caacccgcgg tgctcctcgt 26100
   tcagccaggt cttgcatacg gccgccagag cttccacttg gtcaggcagt agtttgaagt 26160
   tcgcctttag atcgttatcc acgtggtact tgtccatcag cgcgcgcgca gcctccatgc 26220
   ccttctccca cgcagacacg atcggcacac tcagcgggtt catcaccgta atttcacttt 26280
   ccgcttcgct gggctcttcc tcttcctctt gcgtccgcat accacgcgcc actgggtcgt 26340
   cttcattcag ccgccgcact gtgcgcttac ctcctttgcc atgcttgatt agcaccggtg 26400
   ggttgctgaa acccaccatt tgtagcgcca catcttctct ttcttcctcg ctgtccacga 26460
   ttacctctgg tgatggcggg cgctcgggct tgggagaagg gcgcttcttt ttcttcttgg 26520
   gcgcaatggc caaatccgcc gccgaggtcg atggccgcgg gctgggtgtg cgcggcacca 26580
   gcgcgtcttg tgatgagtct tcctcgtcct cggactcgat acgccgcctc atccgctttt 26640
   ttgggggcgc ccggggaggc ggcggcgacg gggacgggga cgacacgtcc tccatggttg 26700
   ggggacgtcg cgccgcaccg cgtccgcgct cgggggtggt ttcgcgctgc tcctcttccc 26760
   gactggccat ttccttctcc tataggcaga aaaagatcat ggagtcagtc gagaagaagg 26820
   acagcctaac cgccccctct gagttcgcca ccaccgcctc caccgatgcc gccaacgcgc 26880
   ctaccacctt ccccgtcgag gcacccccgc ttgaggagga ggaagtgatt atcgagcagg 26940
   acccaggttt tgtaagcgaa gacgacgagg accgctcagt accaacagag gataaaaagc 27000
   aagaccagga caacgcagag gcaaacgagg aacaagtcgg gcggggggac gaaaggcatg 27060
   gcgactacct agatgtggga gacgacgtgc tgttgaagca tctgcagcgc cagtgcgcca 27120
   ttatctgcga cgcgttgcaa gagcgcagcg atgtgcccct cgccatagcg gatgtcagcc 27180
   ttgcctacga acgccaccta ttctcaccgc gcgtaccccc caaacgccaa gaaaacggca 27240
   catgcgagcc caacccgcgc ctcaacttct accccgtatt tgccgtgcca gaggtgcttg 27300
   ccacctatca catctttttc caaaactgca agatacccct atcctgccgt gccaaccgca 27360
   gccgagcgga caagcagctg gccttgcggc agggcgctgt catacctgat atcgcctcgc 27420
   tcaacgaagt gccaaaaatc tttgagggtc ttggacgcga cgagaagcgc gcggcaaacg 27480
   ctctgcaaca ggaaaacagc gaaaatgaaa gtcactctgg agtgttggtg gaactcgagg 27540
   gtgacaacgc gcgcctagcc gtactaaaac gcagcatcga ggtcacccac tttgcctacc 27600
   cggcacttaa cctacccccc aaggtcatga gcacagtcat gagtgagctg atcgtgcgcc 27660
   gtgcgcagcc cctggagagg gatgcaaatt tgcaagaaca aacagaggag ggcctacccg 27720
   cagttggcga cgagcagcta gcgcgctggc ttcaaacgcg cgagcctgcc gacttggagg 27780
   agcgacgcaa actaatgatg gccgcagtgc tcgttaccgt ggagcttgag tgcatgcagc 27840
   ggttctttgc tgacccggag atgcagcgca agctagagga aacattgcac tacacctttc 27900
   gacagggcta cgtacgccag gcctgcaaga tctccaacgt ggagctctgc aacctggtct 27960
   cctaccttgg aattttgcac gaaaaccgcc ttgggcaaaa cgtgcttcat tccacgctca 28020
   agggcgaggc gcgccgcgac tacgtccgcg actgcgttta cttatttcta tgctacacct 28080
   ggcagacggc catgggcgtt tggcagcagt gcttggagga gtgcaacctc aaggagctgc 28140
   agaaactgct aaagcaaaac ttgaaggacc tatggacggc cttcaacgag cgctccgtgg 28200
   ccgcgcacct ggcggacatc attttccccg aacgcctgct taaaaccctg caacagggtc 28260
   tgccagactt caccagtcaa agcatgttgc agaactttag gaactttatc ctagagcgct 28320
   caggaatctt gcccgccacc tgctgtgcac ttcctagcga ctttgtgccc attaagtacc 28380
   gcgaatgccc tccgccgctt tggggccact gctaccttct gcagctagcc aactaccttg 28440
   cctaccactc tgacataatg gaagacgtga gcggtgacgg tctactggag tgtcactgtc 28500
   gctgcaacct atgcaccccg caccgctccc tggtttgcaa ttcgcagctg cttaacgaaa 28560
   gtcaaattat cggtaccttt gagctgcagg gtccctcgcc tgacgaaaag tccgcggctc 28620
   cggggttgaa actcactccg gggctgtgga cgtcggctta ccttcgcaaa tttgtacctg 28680
   aggactacca cgcccacgag attaggttct acgaagacca atcccgcccg ccaaatgcgg 28740
   agcttaccgc ctgcgtcatt acccagggcc acattcttgg ccaattgcaa gccatcaaca 28800
   aagcccgcca agagtttctg ctacgaaagg gacggggggt ttacttggac ccccagtccg 28860
   gcgaggagct caacccaatc cccccgccgc cgcagcccta tcagcagcag ccgcgggccc 28920
   ttgcttccca ggatggcacc caaaaagaag ctgcagctgc cgccgccacc cacggacgag 28980
   gaggaatact gggacagtca ggcagaggag gttttggacg aggaggagga ggacatgatg 29040
   gaagactggg agagcctaga cgaggaagct tccgaggtcg aagaggtgtc agacgaaaca 29100
   ccgtcaccct cggtcgcatt cccctcgccg gcgccccaga aatcggcaac cggttccagc 29160
   atggctacaa cctccgctcc tcaggcgccg ccggcactgc ccgttcgccg acccaaccgt 29220
   agatgggaca ccactggaac cagggccggt aagtccaagc agccgccgcc gttagcccaa 29280
   gagcaacaac agcgccaagg ctaccgctca tggcgcgggc acaagaacgc catagttgct 29340
   tgcttgcaag actgtggggg caacatctcc ttcgcccgcc gctttcttct ctaccatcac 29400
   ggcgtggcct tcccccgtaa catcctgcat tactaccgtc atctctacag cccatactgc 29460
   accggcggca gcggcagcgg cagcaacagc agcggccaca cagaagcaaa ggcgaccgga 29520
   tagcaagact ctgacaaagc ccaagaaatc cacagcggcg gcagcagcag gaggaggagc 29580
   gctgcgtctg gcgcccaacg aacccgtatc gacccgcgag cttagaaaca ggatttttcc 29640
   cactctgtat gctatatttc aacagagcag gggccaagaa caagagctga aaataaaaaa 29700
   caggtctctg cgatccctca cccgcagctg cctgtatcac aaaagcgaag atcagcttcg 29760
   gcgcacgctg gaagacgcgg aggctctctt cagtaaatac tgcgcgctga ctcttaagga 29820
   ctagtttcgc gccctttctc aaatttaagc gcgaaaacta cgtcatctcc agcggccaca 29880
   cccggcgcca gcacctgtcg tcagcgccat tatgagcaag gaaattccca cgccctacat 29940
   gtggagttac cagccacaaa tgggacttgc ggctggagct gcccaagact actcaacccg 30000
   aataaactac atgagcgcgg gaccccacat gatatcccgg gtcaacggaa tccgcgccca 30060
   ccgaaaccga attctcttgg aacaggcggc tattaccacc acacctcgta ataaccttaa 30120
   tccccgtagt tggcccgctg ccctggtgta ccaggaaagt cccgctccca ccactgtggt 30180
   acttcccaga gacgcccagg ccgaagttca gatgactaac tcaggggcgc agcttgcggg 30240
   cggctttcgt cacagggtgc ggtcgcccgg gcagggtata actcacctga caatcagagg 30300
   gcgaggtatt cagctcaacg acgagtcggt gagctcctcg cttggtctcc gtccggacgg 30360
   gacatttcag atcggcggcg ccggccgtcc ttcattcacg cctcgtcagg caatcctaac 30420
   tctgcagacc tcgtcctctg agccgcgctc tggaggcatt ggaactctgc aatttattga 30480
   ggagtttgtg ccatcggtct actttaaccc cttctcggga cctcccggcc actatccgga 30540
   tcaatttatt cctaactttg acgcggtaaa ggactcggcg gacggctacg actgaatgtt 30600
   aagtggagag gcagagcaac tgcgcctgaa acacctggtc cactgtcgcc gccacaagtg 30660
   ctttgcccgc gactccggtg agttttgcta ctttgaattg cccgaggatc atatcgaggg 30720
   cccggcgcac ggcgtccggc ttaccgccca gggagagctt gcccgtagcc tgattcggga 30780
   gtttacccag cgccccctgc tagttgagcg ggacagggga ccctgtgttc tcactgtgat 30840
   ttgcaactgt cctaaccttg gattacatca agatctttgt tgccatctct gtgctgagta 30900
   taataaatac agaaattaaa atatactggg gctcctatcg ccatcctgta aacgccaccg 30960
   tcttcacccg cccaagcaaa ccaaggcgaa ccttacctgg tacttttaac atctctccct 31020
   ctgtgattta caacagtttc aacccagacg gagtgagtct acgagagaac ctctccgagc 31080
   tcagctactc catcagaaaa aacaccaccc tccttacctg ccgggaacgt acgagtgcgt 31140
   caccggccgc tgcaccacac ctaccgcctg accgtaaacc agactttttc cggacagacc 31200
   tcaataactc tgtttaccag aacaggaggt gagcttagaa aacccttagg gtattaggcc 31260
   aaaggcgcag ctactgtggg gtttatgaac aattcaagca actctacggg ctattctaat 31320
   tcaggtttct ctagaaatgg acggaattat tacagagcag cgcctgctag aaagacgcag 31380
   ggcagcggcc gagcaacagc gcatgaatca agagctccaa gacatggtta acttgcacca 31440
   gtgcaaaagg ggtatctttt gtctggtaaa gcaggccaaa gtcacctacg acagtaatac 31500
   caccggacac cgccttagct acaagttgcc aaccaagcgt cagaaattgg tggtcatggt 31560
   gggagaaaag cccattacca taactcagca ctcggtagaa accgaaggct gcattcactc 31620
   accttgtcaa ggacctgagg atctctgcac ccttattaag accctgtgcg gtctcaaaga 31680
   tcttattccc tttaactaat aaaaaaaaat aataaagcat cacttactta aaatcagtta 31740
   gcaaatttct gtccagttta ttcagcagca cctccttgcc ctcctcccag ctctggtatt 31800
   gcagcttcct cctggctgca aactttctcc acaatctaaa tggaatgtca gtttcctcct 31860
   gttcctgtcc atccgcaccc actatcttca tgttgttgca gatgaagcgc gcaagaccgt 31920
   ctgaagatac cttcaacccc gtgtatccat atgacacgga aaccggtcct ccaactgtgc 31980
   cttttcttac tcctcccttt gtatccccca atgggtttca agagagtccc cctggggtac 32040
   tctctttgcg cctatccgaa cctctagtta cctccaatgg catgcttgcg ctcaaaatgg 32100
   gcaacggcct ctctctggac gaggccggca accttacctc ccaaaatgta accactgtga 32160
   gcccacctct caaaaaaacc aagtcaaaca taaacctgga aatatctgca cccctcacag 32220
   ttacctcaga agccctaact gtggctgccg ccgcacctct aatggtcgcg ggcaacacac 32280
   tcaccatgca atcacaggcc ccgctaaccg tgcacgactc caaacttagc attgccaccc 32340
   aaggacccct cacagtgtca gaaggaaagc tagccctgca aacatcaggc cccctcacca 32400
   ccaccgatag cagtaccctt actatcactg cctcaccccc tctaactact gccactggta 32460
   gcttgggcat tgacttgaaa gagcccattt atacacaaaa tggaaaacta ggactaaagt 32520
   acggggctcc tttgcatgta acagacgacc taaacacttt gaccgtagca actggtccag 32580
   gtgtgactat taataatact tccttgcaaa ctaaagttac tggagccttg ggttttgatt 32640
   cacaaggcaa tatgcaactt aatgtagcag gaggactaag gattgattct caaaacagac 32700
   gccttatact tgatgttagt tatccgtttg atgctcaaaa ccaactaaat ctaagactag 32760
   gacagggccc tctttttata aactcagccc acaacttgga tattaactac aacaaaggcc 32820
   tttacttgtt tacagcttca aacaattcca aaaagcttga ggttaaccta agcactgcca 32880
   aggggttgat gtttgacgct acagccatag ccattaatgc aggagatggg cttgaatttg 32940
   gttcacctaa tgcaccaaac acaaatcccc tcaaaacaaa aattggccat ggcctagaat 33000
   ttgattcaaa caaggctatg gttcctaaac taggaactgg ccttagtttt gacagcacag 33060
   gtgccattac agtaggaaac aaaaataatg ataagctaac tttgtggacc acaccagctc 33120
   catctcctaa ctgtagacta aatgcagaga aagatgctaa actcactttg gtcttaacaa 33180
   aatgtggcag tcaaatactt gctacagttt cagttttggc tgttaaaggc agtttggctc 33240
   caatatctgg aacagttcaa agtgctcatc ttattataag atttgacgaa aatggagtgc 33300
   tactaaacaa ttccttcctg gacccagaat attggaactt tagaaatgga gatcttactg 33360
   aaggcacagc ctatacaaac gctgttggat ttatgcctaa cctatcagct tatccaaaat 33420
   ctcacggtaa aactgccaaa agtaacattg tcagtcaagt ttacttaaac ggagacaaaa 33480
   ctaaacctgt aacactaacc attacactaa acggtacaca ggaaacagga gacacaactc 33540
   caagtgcata ctctatgtca ttttcatggg actggtctgg ccacaactac attaatgaaa 33600
   tatttgccac atcctcttac actttttcat acattgccca agaataaaga atcgtttgtg 33660
   ttatgtttca acgtgtttat ttttcaattg cagaaaattt cgaatcattt ttcattcagt 33720
   agtatagccc caccaccaca tagcttatac agatcaccgt accttaatca aactcacaga 33780
   accctagtat tcaacctgcc acctccctcc caacacacag agtacacagt cctttctccc 33840
   cggctggcct taaaaagcat catatcatgg gtaacagaca tattcttagg tgttatattc 33900
   cacacggttt cctgtcgagc caaacgctca tcagtgatat taataaactc cccgggcagc 33960
   tcacttaagt tcatgtcgct gtccagctgc tgagccacag gctgctgtcc aacttgcggt 34020
   tgcttaacgg gcggcgaagg agaagtccac gcctacatgg gggtagagtc ataatcgtgc 34080
   atcaggatag ggcggtggtg ctgcagcagc gcgcgaataa actgctgccg ccgccgctcc 34140
   gtcctgcagg aatacaacat ggcagtggtc tcctcagcga tgattcgcac cgcccgcagc 34200
   ataaggcgcc ttgtcctccg ggcacagcag cgcaccctga tctcacttaa atcagcacag 34260
   taactgcagc acagcaccac aatattgttc aaaatcccac agtgcaaggc gctgtatcca 34320
   aagctcatgg cggggaccac agaacccacg tggccatcat accacaagcg caggtagatt 34380
   aagtggcgac ccctcataaa cacgctggac ataaacatta cctcttttgg catgttgtaa 34440
   ttcaccacct cccggtacca tataaacctc tgattaaaca tggcgccatc caccaccatc 34500
   ctaaaccagc tggccaaaac ctgcccgccg gctatacact gcagggaacc gggactggaa 34560
   caatgacagt ggagagccca ggactcgtaa ccatggatca tcatgctcgt catgatatca 34620
   atgttggcac aacacaggca cacgtgcata cacttcctca ggattacaag ctcctcccgc 34680
   gttagaacca tatcccaggg aacaacccat tcctgaatca gcgtaaatcc cacactgcag 34740
   ggaagacctc gcacgtaact cacgttgtgc attgtcaaag tgttacattc gggcagcagc 34800
   ggatgatcct ccagtatggt agcgcgggtt tctgtctcaa aaggaggtag acgatcccta 34860
   ctgtacggag tgcgccgaga caaccgagat cgtgttggtc gtagtgtcat gccaaatgga 34920
   acgccggacg tagtcatatt tcctgaagca aaaccaggtg cgggcgtgac aaacagatct 34980
   gcgtctccgg tctcgccgct tagatcgctc tgtgtagtag ttgtagtata tccactctct 35040
   caaagcatcc aggcgccccc tggcttcggg ttctatgtaa actccttcat gcgccgctgc 35100
   cctgataaca tccaccaccg cagaataagc cacacccagc caacctacac attcgttctg 35160
   cgagtcacac acgggaggag cgggaagagc tggaagaacc atgttttttt ttttattcca 35220
   aaagattatc caaaacctca aaatgaagat ctattaagtg aacgcgctcc cctccggtgg 35280
   cgtggtcaaa ctctacagcc aaagaacaga taatggcatt tgtaagatgt tgcacaatgg 35340
   cttccaaaag gcaaacggcc ctcacgtcca agtggacgta aaggctaaac ccttcagggt 35400
   gaatctcctc tataaacatt ccagcacctt caaccatgcc caaataattc tcatctcgcc 35460
   accttctcaa tatatctcta agcaaatccc gaatattaag tccggccatt gtaaaaatct 35520
   gctccagagc gccctccacc ttcagcctca agcagcgaat catgattgca aaaattcagg 35580
   ttcctcacag acctgtataa gattcaaaag cggaacatta acaaaaatac cgcgatcccg 35640
   taggtccctt cgcagggcca gctgaacata atcgtgcagg tctgcacgga ccagcgcggc 35700
   cacttccccg ccaggaacct tgacaaaaga acccacactg attatgacac gcatactcgg 35760
   agctatgcta accagcgtag ccccgatgta agctttgttg catgggcggc gatataaaat 35820
   gcaaggtgct gctcaaaaaa tcaggcaaag cctcgcgcaa aaaagaaagc acatcgtagt 35880
   catgctcatg cagataaagg caggtaagct ccggaaccac cacagaaaaa gacaccattt 35940
   ttctctcaaa catgtctgcg ggtttctgca taaacacaaa ataaaataac aaaaaaacat 36000
   ttaaacatta gaagcctgtc ttacaacagg aaaaacaacc cttataagca taagacggac 36060
   tacggccatg ccggcgtgac cgtaaaaaaa ctggtcaccg tgattaaaaa gcaccaccga 36120
   cagctcctcg gtcatgtccg gagtcataat gtaagactcg gtaaacacat caggttgatt 36180
   cacatcggtc agtgctaaaa agcgaccgaa atagcccggg ggaatacata cccgcaggcg 36240
   tagagacaac attacagccc ccataggagg tataacaaaa ttaataggag agaaaaacac 36300
   ataaacacct gaaaaaccct cctgcctagg caaaatagca ccctcccgct ccagaacaac 36360
   atacagcgct tccacagcgg cagccataac agtcagcctt accagtaaaa aagaaaacct 36420
   attaaaaaaa caccactcga cacggcacca gctcaatcag tcacagtgta aaaaagggcc 36480
   aagtgcagag cgagtatata taggactaaa aaatgacgta acggttaaag tccacaaaaa 36540
   acacccagaa aaccgcacgc gaacctacgc ccagaaacga aagccaaaaa acccacaact 36600
   tcctcaaatc gtcacttccg ttttcccacg ttacgtcact tcccatttta agaaaactac 36660
   aattcccaac acatacaagt tactccgccc taaaacctac gtcacccgcc ccgttcccac 36720
   gccccgcgcc acgtcacaaa ctccaccccc tcattatcat attggcttca atccaaaata 36780
   aggtatatta ttgatgatgt taattaaggg cggccgcacg ggccatcgat ggggatccat 36840
   ccgcggagaa gcttctcgac caattctcat gtttgacagc ttatcatcga atttctgcca 36900
   ttcatccgct tattatcact tattcaggcg tagcaaccag gcgtttaagg gcaccaataa 36960
   ctgccttaaa aaaattacgc cccgccctgc cactcatcgc agtactgttg taattcatta 37020
   agcattctgc cgacatggaa gccatcacag acggcatgat gaacctgaat cgccagcggc 37080
   atcagcacct tgtcgccttg cgtataatat ttgcccatgg tgaaaacggg ggcgaagaag 37140
   ttgtccatat tggccacgtt taaatcaaaa ctggtgaaac tcacccaggg attggctgag 37200
   acgaaaaaca tattctcaat aaacccttta gggaaatagg ccaggttttc accgtaacac 37260
   gccacatctt gcgaatatat gtgtagaaac tgccggaaat cgtcgtggta ttcactccag 37320
   agcgatgaaa acgtttcagt ttgctcatgg aaaacggtgt aacaagggtg aacactatcc 37380
   catatcacca gctcaccgtc tttcattgcc atacggaatt ccggatgagc attcatcagg 37440
   cgggcaagaa tgtgaataaa ggccggataa aacttgtgct tatttttctt tacggtcttt 37500
   aaaaaggccg taatatccag ctgaacggtc tggttatagg tacattgagc aactgactga 37560
   aatgcctcaa aatgttcttt acgatgccat tgggatatat caacggtggt atatccagtg 37620
   atttttttct ccattttagc ttccttagct cctgaaaatc tcgataactc aaaaaatacg 37680
   cccggtagtg atcttatttc attatggtga aagttggaac ctcttacgtg ccgatcaacg 37740
   tctcattttc gccaaaagtt ggcccagggc ttcccggtat caacagggac accaggattt 37800
   atttattctg cgaagtgatc ttccgtcaca ggtatttatt cgcgataagc tcatggagcg 37860
   gcgtaaccgt cgcacaggaa ggacagagaa agcgcggatc tgggaagtga cggacagaac 37920
   ggtcaggacc tggattgggg aggcggttgc cgccgctgct gctgacggtg tgacgttctc 37980
   tgttccggtc acaccacata cgttccgcca ttcctatgcg atgcacatgc tgtatgccgg 38040
   tataccgctg aaagttctgc aaagcctgat gggacataag tccatcagtt caacggaagt 38100
   ctacacgaag gtttttgcgc tggatgtggc tgcccggcac cgggtgcagt ttgcgatgcc 38160
   ggagtctgat gcggttgcga tgctgaaaca attatcctga gaataaatgc cttggccttt 38220
   atatggaaat gtggaactga gtggatatgc tgtttttgtc tgttaaacag agaagctggc 38280
   tgttatccac tgagaagcga acgaaacagt cgggaaaatc tcccattatc gtagagatcc 38340
   gcattattaa tctcaggagc ctgtgtagcg tttataggaa gtagtgttct gtcatgatgc 38400
   ctgcaagcgg taacgaaaac gatttgaata tgccttcagg aacaatagaa atcttcgtgc 38460
   ggtgttacgt tgaagtggag cggattatgt cagcaatgga cagaacaacc taatgaacac 38520
   agaaccatga tgtggtctgt ccttttacag ccagtaggct cgccgcagtc gagcgacggc 38580
   gaagccctcg agtgagcgag gaagcaccag ggaacagcac ttatatattc tgcttacaca 38640
   cgatgcctga aaaaacttcc cttggggtta tccacttatc cacggggata tttttataat 38700
   tatttttttt atagttttta gatcttcttt tttagagcgc cttgtaggcc tttatccatg 38760
   ctggttctag agaaggtgtt gtgacaaatt gccctttcag tgtgacaaat caccctcaaa 38820
   tgacagtcct gtctgtgaca aattgccctt aaccctgtga caaattgccc tcagaagaag 38880
   ctgttttttc acaaagttat ccctgcttat tgactctttt ttatttagtg tgacaatcta 38940
   aaaacttgtc acacttcaca tggatctgtc atggcggaaa cagcggttat caatcacaag 39000
   aaacgtaaaa atagcccgcg aatcgtccag tcaaacgacc tcactgaggc ggcatatagt 39060
   ctctcccggg atcaaaaacg tatgctgtat ctgttcgttg accagatcag aaaatctgat 39120
   ggcaccctac aggaacatga cggtatctgc gagatccatg ttgctaaata tgctgaaata 39180
   ttcggattga cctctgcgga agccagtaag gatatacggc aggcattgaa gagtttcgcg 39240
   gggaaggaag tggtttttta tcgccctgaa gaggatgccg gcgatgaaaa aggctatgaa 39300
   tcttttcctt ggtttatcaa acgtgcgcac agtccatcca gagggcttta cagtgtacat 39360
   atcaacccat atctcattcc cttctttatc gggttacaga accggtttac gcagtttcgg 39420
   cttagtgaaa caaaagaaat caccaatccg tatgccatgc gtttatacga atccctgtgt 39480
   cagtatcgta agccggatgg ctcaggcatc gtctctctga aaatcgactg gatcatagag 39540
   cgttaccagc tgcctcaaag ttaccagcgt atgcctgact tccgccgccg cttcctgcag 39600
   gtctgtgtta atgagatcaa cagcagaact ccaatgcgcc tctcatacat tgagaaaaag 39660
   aaaggccgcc agacgactca tatcgtattt tccttccgcg atatcacttc catgacgaca 39720
   ggatagtctg agggttatct gtcacagatt tgagggtggt tcgtcacatt tgttctgacc 39780
   tactgagggt aatttgtcac agttttgctg tttccttcag cctgcatgga ttttctcata 39840
   ctttttgaac tgtaattttt aaggaagcca aatttgaggg cagtttgtca cagttgattt 39900
   ccttctcttt cccttcgtca tgtgacctga tatcgggggt tagttcgtca tcattgatga 39960
   gggttgatta tcacagttta ttactctgaa ttggctatcc gcgtgtgtac ctctacctgg 40020
   agtttttccc acggtggata tttcttcttg cgctgagcgt aagagctatc tgacagaaca 40080
   gttcttcttt gcttcctcgc cagttcgctc gctatgctcg gttacacggc tgcggcgagc 40140
   gctagtgata ataagtgact gaggtatgtg ctcttcttat ctccttttgt agtgttgctc 40200
   ttattttaaa caactttgcg gttttttgat gactttgcga ttttgttgtt gctttgcagt 40260
   aaattgcaag atttaataaa aaaacgcaaa gcaatgatta aaggatgttc agaatgaaac 40320
   tcatggaaac acttaaccag tgcataaacg ctggtcatga aatgacgaag gctatcgcca 40380
   ttgcacagtt taatgatgac agcccggaag cgaggaaaat aacccggcgc tggagaatag 40440
   gtgaagcagc ggatttagtt ggggtttctt ctcaggctat cagagatgcc gagaaagcag 40500
   ggcgactacc gcacccggat atggaaattc gaggacgggt tgagcaacgt gttggttata 40560
   caattgaaca aattaatcat atgcgtgatg tgtttggtac gcgattgcga cgtgctgaag 40620
   acgtatttcc accggtgatc ggggttgctg cccataaagg tggcgtttac aaaacctcag 40680
   tttctgttca tcttgctcag gatctggctc tgaaggggct acgtgttttg ctcgtggaag 40740
   gtaacgaccc ccagggaaca gcctcaatgt atcacggatg ggtaccagat cttcatattc 40800
   atgcagaaga cactctcctg cctttctatc ttggggaaaa ggacgatgtc acttatgcaa 40860
   taaagcccac ttgctggccg gggcttgaca ttattccttc ctgtctggct ctgcaccgta 40920
   ttgaaactga gttaatgggc aaatttgatg aaggtaaact gcccaccgat ccacacctga 40980
   tgctccgact ggccattgaa actgttgctc atgactatga tgtcatagtt attgacagcg 41040
   cgcctaacct gggtatcggc acgattaatg tcgtatgtgc tgctgatgtg ctgattgttc 41100
   ccacgcctgc tgagttgttt gactacacct ccgcactgca gtttttcgat atgcttcgtg 41160
   atctgctcaa gaacgttgat cttaaagggt tcgagcctga tgtacgtatt ttgcttacca 41220
   aatacagcaa tagtaatggc tctcagtccc cgtggatgga ggagcaaatt cgggatgcct 41280
   ggggaagcat ggttctaaaa aatgttgtac gtgaaacgga tgaagttggt aaaggtcaga 41340
   tccggatgag aactgttttt gaacaggcca ttgatcaacg ctcttcaact ggtgcctgga 41400
   gaaatgctct ttctatttgg gaacctgtct gcaatgaaat tttcgatcgt ctgattaaac 41460
   cacgctggga gattagataa tgaagcgtgc gcctgttatt ccaaaacata cgctcaatac 41520
   tcaaccggtt gaagatactt cgttatcgac accagctgcc ccgatggtgg attcgttaat 41580
   tgcgcgcgta ggagtaatgg ctcgcggtaa tgccattact ttgcctgtat gtggtcggga 41640
   tgtgaagttt actcttgaag tgctccgggg tgatagtgtt gagaagacct ctcgggtatg 41700
   gtcaggtaat gaacgtgacc aggagctgct tactgaggac gcactggatg atctcatccc 41760
   ttcttttcta ctgactggtc aacagacacc ggcgttcggt cgaagagtat ctggtgtcat 41820
   agaaattgcc gatgggagtc gccgtcgtaa agctgctgca cttaccgaaa gtgattatcg 41880
   tgttctggtt ggcgagctgg atgatgagca gatggctgca ttatccagat tgggtaacga 41940
   ttatcgccca acaagtgctt atgaacgtgg tcagcgttat gcaagccgat tgcagaatga 42000
   atttgctgga aatatttctg cgctggctga tgcggaaaat atttcacgta agattattac 42060
   ccgctgtatc aacaccgcca aattgcctaa atcagttgtt gctctttttt ctcaccccgg 42120
   tgaactatct gcccggtcag gtgatgcact tcaaaaagcc tttacagata aagaggaatt 42180
   acttaagcag caggcatcta accttcatga gcagaaaaaa gctggggtga tatttgaagc 42240
   tgaagaagtt atcactcttt taacttctgt gcttaaaacg tcatctgcat caagaactag 42300
   tttaagctca cgacatcagt ttgctcctgg agcgacagta ttgtataagg gcgataaaat 42360
   ggtgcttaac ctggacaggt ctcgtgttcc aactgagtgt atagagaaaa ttgaggccat 42420
   tcttaaggaa cttgaaaagc cagcaccctg atgcgaccac gttttagtct acgtttatct 42480
   gtctttactt aatgtccttt gttacaggcc agaaagcata actggcctga atattctctc 42540
   tgggcccact gttccacttg tatcgtcggt ctgataatca gactgggacc acggtcccac 42600
   tcgtatcgtc ggtctgatta ttagtctggg accacggtcc cactcgtatc gtcggtctga 42660
   ttattagtct gggaccacgg tcccactcgt atcgtcggtc tgataatcag actgggacca 42720
   cggtcccact cgtatcgtcg gtctgattat tagtctggga ccatggtccc actcgtatcg 42780
   tcggtctgat tattagtctg ggaccacggt cccactcgta tcgtcggtct gattattagt 42840
   ctggaaccac ggtcccactc gtatcgtcgg tctgattatt agtctgggac cacggtccca 42900
   ctcgtatcgt cggtctgatt attagtctgg gaccacgatc ccactcgtgt tgtcggtctg 42960
   attatcggtc tgggaccacg gtcccacttg tattgtcgat cagactatca gcgtgagact 43020
   acgattccat caatgcctgt caagggcaag tattgacatg tcgtcgtaac ctgtagaacg 43080
   gagtaacctc ggtgtgcggt tgtatgcctg ctgtggattg ctgctgtgtc ctgcttatcc 43140
   acaacatttt gcgcacggtt atgtggacaa aatacctggt tacccaggcc gtgccggcac 43200
   gttaaccggg ctgcatccga tgcaagtgtg tcgctgtcga gttttcgggg gagtccaggg 43260
   ttttcccagt cacgacgttg taaaacgacg gccagtgaat tcgagctcgg tacccgggga 43320
   tctt 43324

<210> 13
<211> 36680
<212> DNA
<213> Artificial

<220>
<223> synthetic

<220>
<221> misc_feature
<222> (27952)..(27952)
<223> n is a, c, g, or t

<220>
<221> misc_feature
<222> (30222)..(30223)
<223> n is a, c, g, or t

<400> 13
catcatcaat aatatacctt attttggatt gaagccaata tgataatgag ggggtggagt 60

ttgtgacgtg gcgcggggcg tgggaacggg gcgggtgacg tagtagtgtg gcggaagtgt 120

gatgttgcaa gtgtggcgga acacatgtaa gcgacggatg tggcaaaagt gacgtttttg 180

gtgtgcgccg gtgtacacag gaagtgacaa ttttcgcgcg gttttaggcg gatgttgtag 240

taaatttggg cgtaaccgag taagatttgg ccattttcgc gggaaaactg aataagagga 300

agtgaaatct gaataatttt gtgttactca tagcgcgtaa actggtaccc ggggatcttg 360

aagttcctat tccgaagttc ctattctcta gaaagtatag gaacttcaag agcgcttttg 420

agcttgttta tcgaattcaa gcttgtcgac tcgaagatct gggcgtggtt aagggtggga 480

aagaatatat aaggtggggg tcttatgtag ttttgtatct gttttgcagc agccgccgcc 540

gccatgagca ccaactcgtt tgatggaagc attgtgagct catatttgac aacgcgcatg 600

cccccatggg ccggggtgcg tcagaatgtg atgggctcca gcattgatgg tcgccccgtc 660

ctgcccgcaa actctactac cttgacctac gagaccgtgt ctggaacgcc gttggagact 720

gcagcctccg ccgccgcttc agccgctgca gccaccgccc gcgggattgt gactgacttt 780

gctttcctga gcccgcttgc aagcagtgca gcttcccgtt catccgcccg cgatgacaag 840

ttgacggctc ttttggcaca attggattct ttgacccggg aacttaatgt cgtttctcag 900

cagctgttgg atctgcgcca gcaggtttct gccctgaagg cttcctcccc tcccaatgcg 960
gtttaaaaca taaataaaaa accagactct gtttggattt ggatcaagca agtgtcttgc 1020
tgtctttatt taggggtttt gcgcgcgcgg taggcccggg accagcggtc tcggtcgttg 1080
agggtcctgt gtattttttc caggacgtgg taaaggtgac tctggatgtt cagatacatg 1140
ggcataagcc cgtctctggg gtggaggtag caccactgca gagcttcatg ctgcggggtg 1200
gtgttgtaga tgatccagtc gtagcaggag cgctgggcgt ggtgcctaaa aatgtctttc 1260
agtagcaagc tgattgccag gggcaggccc ttggtgtaag tgtttacaaa gcggttaagc 1320
tgggatgggt gcatacgtgg ggatatgaga tgcatcttgg actgtatttt taggttggct 1380
atgttcccag ccatatccct ccggggattc atgttgtgca gaaccaccag cacagtgtat 1440
ccggtgcact tgggaaattt gtcatgtagc ttagaaggaa atgcgtggaa gaacttggag 1500
acgcccttgt gacctccaag attttccatg cattcgtcca taatgatggc aatgggccca 1560
cgggcggcgg cctgggcgaa gatatttctg ggatcactaa cgtcatagtt gtgttccagg 1620
atgagatcgt cataggccat ttttacaaag cgcgggcgga gggtgccaga ctgcggtata 1680
atggttccat ccggcccagg ggcgtagtta ccctcacaga tttgcatttc ccacgctttg 1740
agttcagatg gggggatcat gtctacctgc ggggcgatga agaaaacggt ttccggggta 1800
ggggagatca gctgggaaga aagcaggttc ctgagcagct gcgacttacc gcagccggtg 1860
ggcccgtaaa tcacacctat taccgggtgc aactggtagt taagagagct gcagctgccg 1920
tcatccctga gcaggggggc cacttcgtta agcatgtccc tgactcgcat gttttccctg 1980
accaaatccg ccagaaggcg ctcgccgccc agcgatagca gttcttgcaa ggaagcaaag 2040
tttttcaacg gtttgagacc gtccgccgta ggcatgcttt tgagcgtttg accaagcagt 2100
tccaggcggt cccacagctc ggtcacctgc tctacggcat ctcgatccag catatctcct 2160
cgtttcgcgg gttggggcgg ctttcgctgt acggcagtag tcggtgctcg tccagacggg 2220
ccagggtcat gtctttccac gggcgcaggg tcctcgtcag cgtagtctgg gtcacggtga 2280
aggggtgcgc tccgggctgc gcgctggcca gggtgcgctt gaggctggtc ctgctggtgc 2340
tgaagcgctg ccggtcttcg ccctgcgcgt cggccaggta gcatttgacc atggtgtcat 2400
agtccagccc ctccgcggcg tggcccttgg cgcgcagctt gcccttggag gaggcgccgc 2460
acgaggggca gtgcagactt ttgagggcgt agagcttggg cgcgagaaat accgattccg 2520
gggagtaggc atccgcgccg caggccccgc agacggtctc gcattccacg agccaggtga 2580
gctctggccg ttcggggtca aaaaccaggt ttcccccatg ctttttgatg cgtttcttac 2640
ctctggtttc catgagccgg tgtccacgct cggtgacgaa aaggctgtcc gtgtccccgt 2700
atacagactt gagaggcctg tcctcgagcg gtgttccgcg gtcctcctcg tatagaaact 2760

cggaccactc tgagacaaag gctcgcgtcc aggccagcac gaaggaggct aagtgggagg 2820

ggtagcggtc gttgtccact agggggtcca ctcgctccag ggtgtgaaga cacatgtcgc 2880

cctcttcggc atcaaggaag gtgattggtt tgtaggtgta ggccacgtga ccgggtgttc 2940

ctgaaggggg gctataaaag ggggtggggg cgcgttcgtc ctcactctct tccgcatcgc 3000

tgtctgcgag ggccagctgt tggggtgagt actccctctg aaaagcgggc atgacttctg 3060

cgctaagatt gtcagtttcc aaaaacgagg aggatttgat attcacctgg cccgcggtga 3120

tgcctttgag ggtggccgca tccatctggt cagaaaagac aatctttttg ttgtcaagct 3180

tggtggcaaa cgacccgtag agggcgttgg acagcaactt ggcgatggag cgcagggttt 3240

ggtttttgtc gcgatcggcg cgctccttgg ccgcgatgtt tagctgcacg tattcgcgcg 3300

caacgcaccg ccattcggga aagacggtgg tgcgctcgtc gggcaccagg tgcacgcgcc 3360

aaccgcggtt gtgcagggtg acaaggtcaa cgctggtggc tacctctccg cgtaggcgct 3420

cgttggtcca gcagaggcgg ccgcccttgc gcgagcagaa tggcggtagg gggtctagct 3480

gcgtctcgtc cggggggtct gcgtccacgg taaagacccc gggcagcagg cgcgcgtcga 3540

agtagtctat cttgcatcct tgcaagtcta gcgcctgctg ccatgcgcgg gcggcaagcg 3600

cgcgctcgta tgggttgagt gggggacccc atggcatggg gtgggtgagc gcggaggcgt 3660

acatgccgca aatgtcgtaa acgtagaggg gctctctgag tattccaaga tatgtagggt 3720

agcatcttcc accgcggatg ctggcgcgca cgtaatcgta tagttcgtgc gagggagcga 3780

ggaggtcggg accgaggttg ctacgggcgg gctgctctgc tcggaagact atctgcctga 3840

agatggcatg tgagttggat gatatggttg gacgctggaa gacgttgaag ctggcgtctg 3900

tgagacctac cgcgtcacgc acgaaggagg cgtaggagtc gcgcagcttg ttgaccagct 3960

cggcggtgac ctgcacgtct agggcgcagt agtccagggt ttccttgatg atgtcatact 4020

tatcctgtcc cttttttttc cacagctcgc ggttgaggac aaactcttcg cggtctttcc 4080

agtactcttg gatcggaaac ccgtcggcct ccgaacggta agagcctagc atgtagaact 4140

ggttgacggc ctggtaggcg cagcatccct tttctacggg tagcgcgtat gcctgcgcgg 4200

ccttccggag cgaggtgtgg gtgagcgcaa aggtgtccct gaccatgact ttgaggtact 4260

ggtatttgaa gtcagtgtcg tcgcatccgc cctgctccca gagcaaaaag tccgtgcgct 4320

ttttggaacg cggatttggc agggcgaagg tgacatcgtt gaagagtatc tttcccgcgc 4380

gaggcataaa gttgcgtgtg atgcggaagg gtcccggcac ctcggaacgg ttgttaatta 4440

cctgggcggc gagcacgatc tcgtcaaagc cgttgatgtt gtggcccaca atgtaaagtt 4500

ccaagaagcg cgggatgccc ttgatggaag gcaatttttt aagttcctcg taggtgagct 4560

cttcagggga gctgagcccg tgctctgaaa gggcccagtc tgcaagatga gggttggaag 4620

cgacgaatga gctccacagg tcacgggcca ttagcatttg caggtggtcg cgaaaggtcc 4680

taaactggcg acctatggcc attttttctg gggtgatgca gtagaaggta agcgggtctt 4740

gttcccagcg gtcccatcca aggttcgcgg ctaggtctcg cgcggcagtc actagaggct 4800

catctccgcc gaacttcatg accagcatga agggcacgag ctgcttccca aaggccccca 4860

tccaagtata ggtctctaca tcgtaggtga caaagagacg ctcggtgcga ggatgcgagc 4920

cgatcgggaa gaactggatc tcccgccacc aattggagga gtggctattg atgtggtgaa 4980

agtagaagtc cctgcgacgg gccgaacact cgtgctggct tttgtaaaaa cgtgcgcagt 5040

actggcagcg gtgcacgggc tgtacatcct gcacgaggtt gacctgacga ccgcgcacaa 5100

ggaagcagag tgggaatttg agcccctcgc ctggcgggtt tggctggtgg tcttctactt 5160

cggctgcttg tccttgaccg tctggctgct cgaggggagt tacggtggat cggaccacca 5220

cgccgcgcga gcccaaagtc cagatgtccg cgcgcggcgg tcggagcttg atgacaacat 5280

cgcgcagatg ggagctgtcc atggtctgga gctcccgcgg cgtcaggtca ggcgggagct 5340

cctgcaggtt tacctcgcat agacgggtca gggcgcgggc tagatccagg tgatacctaa 5400

tttccagggg ctggttggtg gcggcgtcga tggcttgcaa gaggccgcat ccccgcggcg 5460

cgactacggt accgcgcggc gggcggtggg ccgcgggggt gtccttggat gatgcatcta 5520

aaagcggtga cgcgggcgag cccccggagg tagggggggc tccggacccg ccgggagagg 5580

gggcaggggc acgtcggcgc cgcgcgcggg caggagctgg tgctgcgcgc gtaggttgct 5640

ggcgaacgcg acgacgcggc ggttgatctc ctgaatctgg cgcctctgcg tgaagacgac 5700

gggcccggtg agcttgagcc tgaaagagag ttcgacagaa tcaatttcgg tgtcgttgac 5760

ggcggcctgg cgcaaaatct cctgcacgtc tcctgagttg tcttgatagg cgatctcggc 5820

catgaactgc tcgatctctt cctcctggag atctccgcgt ccggctcgct ccacggtggc 5880

ggcgaggtcg ttggaaatgc gggccatgag ctgcgagaag gcgttgaggc ctccctcgtt 5940

ccagacgcgg ctgtagacca cgcccccttc ggcatcgcgg gcgcgcatga ccacctgcgc 6000

gagattgagc tccacgtgcc gggcgaagac ggcgtagttt cgcaggcgct gaaagaggta 6060

gttgagggtg gtggcggtgt gttctgccac gaagaagtac ataacccagc gtcgcaacgt 6120

ggattcgttg atatccccca aggcctcaag gcgctccatg gcctcgtaga agtccacggc 6180

gaagttgaaa aactgggagt tgcgcgccga cacggttaac tcctcctcca gaagacggat 6240

gagctcggcg acagtgtcgc gcacctcgcg ctcaaaggct acaggggcct cttcttcttc 6300

ttcaatctcc tcttccataa gggcctcccc ttcttcttct tctggcggcg gtgggggagg 6360

ggggacacgg cggcgacgac ggcgcaccgg gaggcggtcg acaaagcgct cgatcatctc 6420

cccgcggcga cggcgcatgg tctcggtgac ggcgcggccg ttctcgcggg ggcgcagttg 6480

gaagacgccg cccgtcatgt cccggttatg ggttggcggg gggctgccat gcggcaggga 6540

tacggcgcta acgatgcatc tcaacaattg ttgtgtaggt actccgccgc cgagggacct 6600

gagcgagtcc gcatcgaccg gatcggaaaa cctctcgaga aaggcgtcta accagtcaca 6660

gtcgcaaggt aggctgagca ccgtggcggg cggcagcggg cggcggtcgg ggttgtttct 6720

ggcggaggtg ctgctgatga tgtaattaaa gtaggcggtc ttgagacggc ggatggtcga 6780

cagaagcacc atgtccttgg gtccggcctg ctgaatgcgc aggcggtcgg ccatgcccca 6840

ggcttcgttt tgacatcggc gcaggtcttt gtagtagtct tgcatgagcc tttctaccgg 6900

cacttcttct tctccttcct cttgtcctgc atctcttgca tctatcgctg cggcggcggc 6960

ggagtttggc cgtaggtggc gccctcttcc tcccatgcgt gtgaccccga agcccctcat 7020

cggctgaagc agggctaggt cggcgacaac gcgctcggct aatatggcct gctgcacctg 7080

cgtgagggta gactggaagt catccatgtc cacaaagcgg tggtatgcgc ccgtgttgat 7140

ggtgtaagtg cagttggcca taacggacca gttaacggtc tggtgacccg gctgcgagag 7200

ctcggtgtac ctgagacgcg agtaagccct cgagtcaaat acgtagtcgt tgcaagtccg 7260

caccaggtac tggtatccca ccaaaaagtg cggcggcggc tggcggtaga ggggccagcg 7320

tagggtggcc ggggctccgg gggcgagatc ttccaacata aggcgatgat atccgtagat 7380

gtacctggac atccaggtga tgccggcggc ggtggtggag gcgcgcggaa agtcgcggac 7440

gcggttccag atgttgcgca gcggcaaaaa gtgctccatg gtcgggacgc tctggccggt 7500

caggcgcgcg caatcgttga cgctctaccg tgcaaaagga gagcctgtaa gcgggcactc 7560

ttccgtggtc tggtggataa attcgcaagg gtatcatggc ggacgaccgg ggttcgagcc 7620

ccgtatccgg ccgtccgccg tgatccatgc ggttaccgcc cgcgtgtcga acccaggtgt 7680

gcgacgtcag acaacggggg agtgctcctt ttggcttcct tccaggcgcg gcggctgctg 7740

cgctagcttt tttggccact ggccgcgcgc agcgtaagcg gttaggctgg aaagcgaaag 7800

cattaagtgg ctcgctccct gtagccggag ggttattttc caagggttga gtcgcgggac 7860

ccccggttcg agtctcggac cggccggact gcggcgaacg ggggtttgcc tccccgtcat 7920

gcaagacccc gcttgcaaat tcctccggaa acagggacga gccccttttt tgcttttccc 7980

agatgcatcc ggtgctgcgg cagatgcgcc cccctcctca gcagcggcaa gagcaagagc 8040

agcggcagac atgcagggca ccctcccctc ctcctaccgc gtcaggaggg gcgacatccg 8100

cggttgacgc ggcagcagat ggtgattacg aacccccgcg gcgccgggcc cggcactacc 8160

tggacttgga ggagggcgag ggcctggcgc ggctaggagc gccctctcct gagcggtacc 8220

caagggtgca gctgaagcgt gatacgcgtg aggcgtacgt gccgcggcag aacctgtttc 8280

gcgaccgcga gggagaggag cccgaggaga tgcgggatcg aaagttccac gcagggcgcg 8340

agctgcggca tggcctgaat cgcgagcggt tgctgcgcga ggaggacttt gagcccgacg 8400

cgcgaaccgg gattagtccc gcgcgcgcac acgtggcggc cgccgacctg gtaaccgcat 8460

acgagcagac ggtgaaccag gagattaact ttcaaaaaag ctttaacaac cacgtgcgta 8520

cgcttgtggc gcgcgaggag gtggctatag gactgatgca tctgtgggac tttgtaagcg 8580

cgctggagca aaacccaaat agcaagccgc tcatggcgca gctgttcctt atagtgcagc 8640

acagcaggga caacgaggca ttcagggatg cgctgctaaa catagtagag cccgagggcc 8700

gctggctgct cgatttgata aacatcctgc agagcatagt ggtgcaggag cgcagcttga 8760

gcctggctga caaggtggcc gccatcaact attccatgct tagcctgggc aagttttacg 8820

cccgcaagat ataccatacc ccttacgttc ccatagacaa ggaggtaaag atcgaggggt 8880

tctacatgcg catggcgctg aaggtgctta ccttgagcga cgacctgggc gtttatcgca 8940

acgagcgcat ccacaaggcc gtgagcgtga gccggcggcg cgagctcagc gaccgcgagc 9000

tgatgcacag cctgcaaagg gccctggctg gcacgggcag cggcgataga gaggccgagt 9060

cctactttga cgcgggcgct gacctgcgct gggccccaag ccgacgcgcc ctggaggcag 9120

ctggggccgg acctgggctg gcggtggcac ccgcgcgcgc tggcaacgtc ggcggcgtgg 9180

aggaatatga cgaggacgat gagtacgagc cagaggacgg cgagtactaa gcggtgatgt 9240

ttctgatcag atgatgcaag acgcaacgga cccggcggtg cgggcggcgc tgcagagcca 9300

gccgtccggc cttaactcca cggacgactg gcgccaggtc atggaccgca tcatgtcgct 9360

gactgcgcgc aatcctgacg cgttccggca gcagccgcag gccaaccggc tctccgcaat 9420

tctggaagcg gtggtcccgg cgcgcgcaaa ccccacgcac gagaaggtgc tggcgatcgt 9480

aaacgcgctg gccgaaaaca gggccatccg gcccgacgag gccggcctgg tctacgacgc 9540

gctgcttcag cgcgtggctc gttacaacag cggcaacgtg cagaccaacc tggaccggct 9600

ggtgggggat gtgcgcgagg ccgtggcgca gcgtgagcgc gcgcagcagc agggcaacct 9660

gggctccatg gttgcactaa acgccttcct gagtacacag cccgccaacg tgccgcgggg 9720

acaggaggac tacaccaact ttgtgagcgc actgcggcta atggtgactg agacaccgca 9780

aagtgaggtg taccagtctg ggccagacta tttttttcag accagtagac aaggcctgca 9840

gaccgtaaac ctgagccagg ctttcaaaaa cttgcagggg ctgtgggggg tgcgggctcc 9900

cacaggcgac cgcgcgaccg tgtctagctt gctgacgccc aactcgcgcc tgttgctgct 9960

gctaatagcg cccttcacgg acagtggcag cgtgtcccgg gacacatacc taggtcactt 10020

gctgacactg taccgcgagg ccataggtca ggcgcatgtg gacgagcata ctttccagga 10080

gattacaagt gtcagccgcg cgctggggca ggaggacacg ggcagcctgg aggcaaccct 10140

aaactacctg ctgaccaacc ggcggcagaa gatcccctcg ttgcacagtt taaacagcga 10200

ggaggagcgc attttgcgct acgtgcagca gagcgtgagc cttaacctga tgcgcgacgg 10260

ggtaacgccc agcgtggcgc tggacatgac cgcgcgcaac atggaaccgg gcatgtatgc 10320

ctcaaaccgg ccgtttatca accgcctaat ggactacttg catcgcgcgg ccgccgtgaa 10380

ccccgagtat ttcaccaatg ccatcttgaa cccgcactgg ctaccgcccc ctggtttcta 10440

caccggggga ttcgaggtgc ccgagggtaa cgatggattc ctctgggacg acatagacga 10500

cagcgtgttt tccccgcaac cgcagaccct gctagagttg caacagcgcg agcaggcaga 10560

ggcggcgctg cgaaaggaaa gcttccgcag gccaagcagc ttgtccgatc taggcgctgc 10620

ggccccgcgg tcagatgcta gtagcccatt tccaagcttg atagggtctc ttaccagcac 10680

tcgcaccacc cgcccgcgcc tgctgggcga ggaggagtac ctaaacaact cgctgctgca 10740

gccgcagcgc gaaaaaaacc tgcctccggc atttcccaac aacgggatag agagcctagt 10800

ggacaagatg agtagatgga agacgtacgc gcaggagcac agggacgtgc caggcccgcg 10860

cccgcccacc cgtcgtcaaa ggcacgaccg tcagcggggt ctggtgtggg aggacgatga 10920

ctcggcagac gacagcagcg tcctggattt gggagggagt ggcaacccgt ttgcgcacct 10980

tcgccccagg ctggggagaa tgttttaaaa aaaaaaaagc atgatgcaaa ataaaaaact 11040

caccaaggcc atggcaccga gcgttggttt tcttgtattc cccttagtat gcggcgcgcg 11100

gcgatgtatg aggaaggtcc tcctccctcc tacgagagtg tggtgagcgc ggcgccagtg 11160

gcggcggcgc tgggttctcc cttcgatgct cccctggacc cgccgtttgt gcctccgcgg 11220

tacctgcggc ctaccggggg gagaaacagc atccgttact ctgagttggc acccctattc 11280

gacaccaccc gtgtgtacct ggtggacaac aagtcaacgg atgtggcatc cctgaactac 11340

cagaacgacc acagcaactt tctgaccacg gtcattcaaa acaatgacta cagcccgggg 11400

gaggcaagca cacagaccat caatcttgac gaccggtcgc actggggcgg cgacctgaaa 11460

accatcctgc ataccaacat gccaaatgtg aacgagttca tgtttaccaa taagtttaag 11520

gcgcgggtga tggtgtcgcg cttgcctact aaggacaatc aggtggagct gaaatacgag 11580

tgggtggagt tcacgctgcc cgagggcaac tactccgaga ccatgaccat agaccttatg 11640

aacaacgcga tcgtggagca ctacttgaaa gtgggcagac agaacggggt tctggaaagc 11700

gacatcgggg taaagtttga cacccgcaac ttcagactgg ggtttgaccc cgtcactggt 11760

cttgtcatgc ctggggtata tacaaacgaa gccttccatc cagacatcat tttgctgcca 11820

ggatgcgggg tggacttcac ccacagccgc ctgagcaact tgttgggcat ccgcaagcgg 11880

caacccttcc aggagggctt taggatcacc tacgatgatc tggagggtgg taacattccc 11940

gcactgttgg atgtggacgc ctaccaggcg agcttgaaag atgacaccga acagggcggg 12000

ggtggcgcag gcggcagcaa cagcagtggc agcggcgcgg aagagaactc caacgcggca 12060

gccgcggcaa tgcagccggt ggaggacatg aacgatcatg ccattcgcgg cgacaccttt 12120

gccacacggg ctgaggagaa gcgcgctgag gccgaagcag cggccgaagc tgccgccccc 12180

gctgcgcaac ccgaggtcga gaagcctcag aagaaaccgg tgatcaaacc cctgacagag 12240

gacagcaaga aacgcagtta caacctaata agcaatgaca gcaccttcac ccagtaccgc 12300

agctggtacc ttgcatacaa ctacggcgac cctcagaccg gaatccgctc atggaccctg 12360

ctttgcactc ctgacgtaac ctgcggctcg gagcaggtct actggtcgtt gccagacatg 12420

atgcaagacc ccgtgacctt ccgctccacg cgccagatca gcaactttcc ggtggtgggc 12480

gccgagctgt tgcccgtgca ctccaagagc ttctacaacg accaggccgt ctactcccaa 12540

ctcatccgcc agtttacctc tctgacccac gtgttcaatc gctttcccga gaaccagatt 12600

ttggcgcgcc cgccagcccc caccatcacc accgtcagtg aaaacgttcc tgctctcaca 12660

gatcacggga cgctaccgct gcgcaacagc atcggaggag tccagcgagt gaccattact 12720

gacgccagac gccgcacctg cccctacgtt tacaaggccc tgggcatagt ctcgccgcgc 12780

gtcctatcga gccgcacttt ttgagcaagc atgtccatcc ttatatcgcc cagcaataac 12840

acaggctggg gcctgcgctt cccaagcaag atgtttggcg gggccaagaa gcgctccgac 12900

caacacccag tgcgcgtgcg cgggcactac cgcgcgccct ggggcgcgca caaacgcggc 12960

cgcactgggc gcaccaccgt cgatgacgcc atcgacgcgg tggtggagga ggcgcgcaac 13020

tacacgccca cgccgccacc agtgtccaca gtggacgcgg ccattcagac cgtggtgcgc 13080

ggagcccggc gctatgctaa aatgaagaga cggcggaggc gcgtagcacg tcgccaccgc 13140

cgccgacccg gcactgccgc ccaacgcgcg gcggcggccc tgcttaaccg cgcacgtcgc 13200

accggccgac gggcggccat gcgggccgct cgaaggctgg ccgcgggtat tgtcactgtg 13260

ccccccaggt ccaggcgacg agcggccgcc gcagcagccg cggccattag tgctatgact 13320

cagggtcgca ggggcaacgt gtattgggtg cgcgactcgg ttagcggcct gcgcgtgccc 13380

gtgcgcaccc gccccccgcg caactagatt gcaagaaaaa actacttaga ctcgtactgt 13440

tgtatgtatc cagcggcggc ggcgcgcaac gaagctatgt ccaagcgcaa aatcaaagaa 13500

gagatgctcc aggtcatcgc gccggagatc tatggccccc cgaagaagga agagcaggat 13560

tacaagcccc gaaagctaaa gcgggtcaaa aagaaaaaga aagatgatga tgatgaactt 13620

gacgacgagg tggaactgct gcacgctacc gcgcccaggc gacgggtaca gtggaaaggt 13680

cgacgcgtaa aacgtgtttt gcgacccggc accaccgtag tctttacgcc cggtgagcgc 13740

tccacccgca cctacaagcg cgtgtatgat gaggtgtacg gcgacgagga cctgcttgag 13800

caggccaacg agcgcctcgg ggagtttgcc tacggaaagc ggcataagga catgctggcg 13860

ttgccgctgg acgagggcaa cccaacacct agcctaaagc ccgtaacact gcagcaggtg 13920

ctgcccgcgc ttgcaccgtc cgaagaaaag cgcggcctaa agcgcgagtc tggtgacttg 13980

gcacccaccg tgcagctgat ggtacccaag cgccagcgac tggaagatgt cttggaaaaa 14040

atgaccgtgg aacctgggct ggagcccgag gtccgcgtgc ggccaatcaa gcaggtggcg 14100

ccgggactgg gcgtgcagac cgtggacgtt cagataccca ctaccagtag caccagtatt 14160

gccaccgcca cagagggcat ggagacacaa acgtccccgg ttgcctcagc ggtggcggat 14220

gccgcggtgc aggcggtcgc tgcggccgcg tccaagacct ctacggaggt gcaaacggac 14280

ccgtggatgt ttcgcgtttc agccccccgg cgcccgcgcg gttcgaggaa gtacggcgcc 14340

gccagcgcgc tactgcccga atatgcccta catccttcca ttgcgcctac ccccggctat 14400

cgtggctaca cctaccgccc cagaagacga gcaactaccc gacgccgaac caccactgga 14460

acccgccgcc gccgtcgccg tcgccagccc gtgctggccc cgatttccgt gcgcagggtg 14520

gctcgcgaag gaggcaggac cctggtgctg ccaacagcgc gctaccaccc cagcatcgtt 14580

taaaagccgg tctttgtggt tcttgcagat atggccctca cctgccgcct ccgtttcccg 14640

gtgccgggat tccgaggaag aatgcaccgt aggaggggca tggccggcca cggcctgacg 14700

ggcggcatgc gtcgtgcgca ccaccggcgg cggcgcgcgt cgcaccgtcg catgcgcggc 14760

ggtatcctgc ccctccttat tccactgatc gccgcggcga ttggcgccgt gcccggaatt 14820

gcatccgtgg ccttgcaggc gcagagacac tgattaaaaa caagttgcat gtggaaaaat 14880

caaaataaaa agtctggact ctcacgctcg cttggtcctg taactatttt gtagaatgga 14940

agacatcaac tttgcgtctc tggccccgcg acacggctcg cgcccgttca tgggaaactg 15000

gcaagatatc ggcaccagca atatgagcgg tggcgccttc agctggggct cgctgtggag 15060

cggcattaaa aatttcggtt ccaccgttaa gaactatggc agcaaggcct ggaacagcag 15120

cacaggccag atgctgaggg ataagttgaa agagcaaaat ttccaacaaa aggtggtaga 15180

tggcctggcc tctggcatta gcggggtggt ggacctggcc aaccaggcag tgcaaaataa 15240

gattaacagt aagcttgatc cccgccctcc cgtagaggag cctccaccgg ccgtggagac 15300

agtgtctcca gaggggcgtg gcgaaaagcg tccgcgcccc gacagggaag aaactctggt 15360

gacgcaaata gacgagcctc cctcgtacga ggaggcacta aagcaaggcc tgcccaccac 15420

ccgtcccatc gcgcccatgg ctaccggagt gctgggccag cacacacccg taacgctgga 15480

cctgcctccc cccgccgaca cccagcagaa acctgtgctg ccaggcccga ccgccgttgt 15540

tgtaacccgt cctagccgcg cgtccctgcg ccgcgccgcc agcggtccgc gatcgttgcg 15600

gcccgtagcc agtggcaact ggcaaagcac actgaacagc atcgtgggtc tgggggtgca 15660

atccctgaag cgccgacgat gcttctgaat agctaacgtg tcgtatgtgt gtcatgtatg 15720

cgtccatgtc gccgccagag gagctgctga gccgccgcgc gcccgctttc caagatggct 15780

accccttcga tgatgccgca gtggtcttac atgcacatct cgggccagga cgcctcggag 15840

tacctgagcc ccgggctggt gcagtttgcc cgcgccaccg agacgtactt cagcctgaat 15900

aacaagttta gaaccccacc ggtggcgcct acgcacgacg tgaccacaga ccggtcccag 15960

cgtttgacgc tgcggttcat ccctgtggac cgtgaggata ctgcgtactc gtacaaggcg 16020

cggttcaccc tagctgtggg tgataaccgt gtgctggaca tggcttccac gtactttgac 16080
atccgcggcg tgctggacag gggccctact tttaagccct actctggcac tgcctacaac 16140
gccctggctc ccaagggtgc cccaaatcct tgcgaatggg atgaagctgc tactgctctt 16200
gaaataaacc tagaagaaga ggacgatgac aacgaagacg aagtagacga gcaagctgag 16260
cagcaaaaaa ctcacgtatt tgggcaggcg ccttattctg gtataaatat tacaaaggag 16320
ggtattcaaa taggtgtcga aggtcaaaca cctaaatatg ccgataaaac atttcaacct 16380
gaacctcaaa taggagaatc tcagtggtac gaaactgaaa ttaatcatgc agctgggaga 16440
gtccttaaaa agactacccc aatgaaacca tgttacggtt catatgcaaa acccacaaat 16500
gaaaatggag ggcaaggcat tcttgtaaag caacaaaatg gaaagctaga aagtcaagtg 16560
gaaatgcaat ttttctcaac tactgaggcg accgcaggca atggtgataa cttgactcct 16620
aaagtggtat tgtacagtga agatgtagat atagaaaccc cagacactca tatttcttac 16680
atgcccacta ttaaggaagg taactcacga gaactaatgg gccaacaatc tatgcccaac 16740
aggcctaatt acattgcttt tagggacaat tttattggtc taatgtatta caacagcacg 16800
ggtaatatgg gtgttctggc gggccaagca tcgcagttga atgctgttgt agatttgcaa 16860
gacagaaaca cagagctttc ataccagctt ttgcttgatt ccattggtga tagaaccagg 16920
tacttttcta tgtggaatca ggctgttgac agctatgatc cagatgttag aattattgaa 16980
aatcatggaa ctgaagatga acttccaaat tactgctttc cactgggagg tgtgattaat 17040
acagagactc ttaccaaggt aaaacctaaa acaggtcagg aaaatggatg ggaaaaagat 17100
gctacagaat tttcagataa aaatgaaata agagttggaa ataattttgc catggaaatc 17160
aatctaaatg ccaacctgtg gagaaatttc ctgtactcca acatagcgct gtatttgccc 17220
gacaagctaa agtacagtcc ttccaacgta aaaatttctg ataacccaaa cacctacgac 17280
tacatgaaca agcgagtggt ggctcccggg ttagtggact gctacattaa ccttggagca 17340
cgctggtccc ttgactatat ggacaacgtc aacccattta accaccaccg caatgctggc 17400
ctgcgctacc gctcaatgtt gctgggcaat ggtcgctatg tgcccttcca catccaggtg 17460
cctcagaagt tctttgccat taaaaacctc cttctcctgc cgggctcata cacctacgag 17520
tggaacttca ggaaggatgt taacatggtt ctgcagagct ccctaggaaa tgacctaagg 17580
gttgacggag ccagcattaa gtttgatagc atttgccttt acgccacctt cttccccatg 17640
gcccacaaca ccgcctccac gcttgaggcc atgcttagaa acgacaccaa cgaccagtcc 17700
tttaacgact atctctccgc cgccaacatg ctctacccta tacccgccaa cgctaccaac 17760
gtgcccatat ccatcccctc ccgcaactgg gcggctttcc gcggctgggc cttcacgcgc 17820
cttaagacta aggaaacccc atcactgggc tcgggctacg acccttatta cacctactct 17880

ggctctatac cctacctaga tggaaccttt tacctcaacc acacctttaa gaaggtggcc 17940

attacctttg actcttctgt cagctggcct ggcaatgacc gcctgcttac ccccaacgag 18000

tttgaaatta agcgctcagt tgacggggag ggttacaacg ttgcccagtg taacatgacc 18060

aaagactggt tcctggtaca aatgctagct aactacaaca ttggctacca gggcttctat 18120

atcccagaga gctacaagga ccgcatgtac tccttcttta gaaacttcca gcccatgagc 18180

cgtcaggtgg tggatgatac taaatacaag gactaccaac aggtgggcat cctacaccaa 18240

cacaacaact ctggatttgt tggctacctt gcccccacca tgcgcgaagg acaggcctac 18300

cctgctaact tcccctatcc gcttataggc aagaccgcag ttgacagcat tacccagaaa 18360

aagtttcttt gcgatcgcac cctttggcgc atcccattct ccagtaactt tatgtccatg 18420

ggcgcactca cagacctggg ccaaaacctt ctctacgcca actccgccca cgcgctagac 18480

atgacttttg aggtggatcc catggacgag cccacccttc tttatgtttt gtttgaagtc 18540

tttgacgtgg tccgtgtgca ccggccgcac cgcggcgtca tcgaaaccgt gtacctgcgc 18600

acgcccttct cggccggcaa cgccacaaca taaagaagca agcaacatca acaacagctg 18660

ccgccatggg ctccagtgag caggaactga aagccattgt caaagatctt ggttgtgggc 18720

catatttttt gggcacctat gacaagcgct ttccaggctt tgtttctcca cacaagctcg 18780

cctgcgccat agtcaatacg gccggtcgcg agactggggg cgtacactgg atggcctttg 18840

cctggaaccc gcactcaaaa acatgctacc tctttgagcc ctttggcttt tctgaccagc 18900

gactcaagca ggtttaccag tttgagtacg agtcactcct gcgccgtagc gccattgctt 18960

cttcccccga ccgctgtata acgctggaaa agtccaccca aagcgtacag gggcccaact 19020

cggccgcctg tggactattc tgctgcatgt ttctccacgc ctttgccaac tggccccaaa 19080

ctcccatgga tcacaacccc accatgaacc ttattaccgg ggtacccaac tccatgctca 19140

acagtcccca ggtacagccc accctgcgtc gcaaccagga acagctctac agcttcctgg 19200

agcgccactc gccctacttc cgcagccaca gtgcgcagat taggagcgcc acttcttttt 19260

gtcacttgaa aaacatgtaa aaataatgta ctagagacac tttcaataaa ggcaaatgct 19320

tttatttgta cactctcggg tgattattta cccccaccct tgccgtctgc gccgtttaaa 19380

aatcaaaggg gttctgccgc gcatcgctat gcgccactgg cagggacacg ttgcgatact 19440

ggtgtttagt gctccactta aactcaggca caaccatccg cggcagctcg gtgaagtttt 19500

cactccacag gctgcgcacc atcaccaacg cgtttagcag gtcgggcgcc gatatcttga 19560

agtcgcagtt ggggcctccg ccctgcgcgc gcgagttgcg atacacaggg ttgcagcact 19620

ggaacactat cagcgccggg tggtgcacgc tggccagcac gctcttgtcg gagatcagat 19680

ccgcgtccag gtcctccgcg ttgctcaggg cgaacggagt caactttggt agctgccttc 19740

ccaaaaaggg cgcgtgccca ggctttgagt tgcactcgca ccgtagtggc atcaaaaggt 19800

gaccgtgccc ggtctgggcg ttaggataca gcgcctgcat aaaagccttg atctgcttaa 19860

aagccacctg agcctttgcg ccttcagaga agaacatgcc gcaagacttg ccggaaaact 19920

gattggccgg acaggccgcg tcgtgcacgc agcaccttgc gtcggtgttg gagatctgca 19980

ccacatttcg gccccaccgg ttcttcacga tcttggcctt gctagactgc tccttcagcg 20040

cgcgctgccc gttttcgctc gtcacatcca tttcaatcac gtgctcctta tttatcataa 20100

tgcttccgtg tagacactta agctcgcctt cgatctcagc gcagcggtgc agccacaacg 20160

cgcagcccgt gggctcgtga tgcttgtagg tcacctctgc aaacgactgc aggtacgcct 20220

gcaggaatcg ccccatcatc gtcacaaagg tcttgttgct ggtgaaggtc agctgcaacc 20280

cgcggtgctc ctcgttcagc caggtcttgc atacggccgc cagagcttcc acttggtcag 20340

gcagtagttt gaagttcgcc tttagatcgt tatccacgtg gtacttgtcc atcagcgcgc 20400

gcgcagcctc catgcccttc tcccacgcag acacgatcgg cacactcagc gggttcatca 20460

ccgtaatttc actttccgct tcgctgggct cttcctcttc ctcttgcgtc cgcataccac 20520

gcgccactgg gtcgtcttca ttcagccgcc gcactgtgcg cttacctcct ttgccatgct 20580

tgattagcac cggtgggttg ctgaaaccca ccatttgtag cgccacatct tctctttctt 20640

cctcgctgtc cacgattacc tctggtgatg gcgggcgctc gggcttggga gaagggcgct 20700

tctttttctt cttgggcgca atggccaaat ccgccgccga ggtcgatggc cgcgggctgg 20760

gtgtgcgcgg caccagcgcg tcttgtgatg agtcttcctc gtcctcggac tcgatacgcc 20820

gcctcatccg cttttttggg ggcgcccggg gaggcggcgg cgacggggac ggggacgaca 20880

cgtcctccat ggttggggga cgtcgcgccg caccgcgtcc gcgctcgggg gtggtttcgc 20940

gctgctcctc ttcccgactg gccatttcct tctcctatag gcagaaaaag atcatggagt 21000

cagtcgagaa gaaggacagc ctaaccgccc cctctgagtt cgccaccacc gcctccaccg 21060

atgccgccaa cgcgcctacc accttccccg tcgaggcacc cccgcttgag gaggaggaag 21120

tgattatcga gcaggaccca ggttttgtaa gcgaagacga cgaggaccgc tcagtaccaa 21180

cagaggataa aaagcaagac caggacaacg cagaggcaaa cgaggaacaa gtcgggcggg 21240

gggacgaaag gcatggcgac tacctagatg tgggagacga cgtgctgttg aagcatctgc 21300

agcgccagtg cgccattatc tgcgacgcgt tgcaagagcg cagcgatgtg cccctcgcca 21360

tagcggatgt cagccttgcc tacgaacgcc acctattctc accgcgcgta ccccccaaac 21420

gccaagaaaa cggcacatgc gagcccaacc cgcgcctcaa cttctacccc gtatttgccg 21480

tgccagaggt gcttgccacc tatcacatct ttttccaaaa ctgcaagata cccctatcct 21540

gccgtgccaa ccgcagccga gcggacaagc agctggcctt gcggcagggc gctgtcatac 21600

ctgatatcgc ctcgctcaac gaagtgccaa aaatctttga gggtcttgga cgcgacgaga 21660

agcgcgcggc aaacgctctg caacaggaaa acagcgaaaa tgaaagtcac tctggagtgt 21720

tggtggaact cgagggtgac aacgcgcgcc tagccgtact aaaacgcagc atcgaggtca 21780

cccactttgc ctacccggca cttaacctac cccccaaggt catgagcaca gtcatgagtg 21840

agctgatcgt gcgccgtgcg cagcccctgg agagggatgc aaatttgcaa gaacaaacag 21900

aggagggcct acccgcagtt ggcgacgagc agctagcgcg ctggcttcaa acgcgcgagc 21960

ctgccgactt ggaggagcga cgcaaactaa tgatggccgc agtgctcgtt accgtggagc 22020

ttgagtgcat gcagcggttc tttgctgacc cggagatgca gcgcaagcta gaggaaacat 22080

tgcactacac ctttcgacag ggctacgtac gccaggcctg caagatctcc aacgtggagc 22140

tctgcaacct ggtctcctac cttggaattt tgcacgaaaa ccgccttggg caaaacgtgc 22200

ttcattccac gctcaagggc gaggcgcgcc gcgactacgt ccgcgactgc gtttacttat 22260

ttctatgcta cacctggcag acggccatgg gcgtttggca gcagtgcttg gaggagtgca 22320

acctcaagga gctgcagaaa ctgctaaagc aaaacttgaa ggacctatgg acggccttca 22380

acgagcgctc cgtggccgcg cacctggcgg acatcatttt ccccgaacgc ctgcttaaaa 22440

ccctgcaaca gggtctgcca gacttcacca gtcaaagcat gttgcagaac tttaggaact 22500

ttatcctaga gcgctcagga atcttgcccg ccacctgctg tgcacttcct agcgactttg 22560

tgcccattaa gtaccgcgaa tgccctccgc cgctttgggg ccactgctac cttctgcagc 22620

tagccaacta ccttgcctac cactctgaca taatggaaga cgtgagcggt gacggtctac 22680

tggagtgtca ctgtcgctgc aacctatgca ccccgcaccg ctccctggtt tgcaattcgc 22740

agctgcttaa cgaaagtcaa attatcggta cctttgagct gcagggtccc tcgcctgacg 22800

aaaagtccgc ggctccgggg ttgaaactca ctccggggct gtggacgtcg gcttaccttc 22860

gcaaatttgt acctgaggac taccacgccc acgagattag gttctacgaa gaccaatccc 22920

gcccgccaaa tgcggagctt accgcctgcg tcattaccca gggccacatt cttggccaat 22980

tgcaagccat caacaaagcc cgccaagagt ttctgctacg aaagggacgg ggggtttact 23040

tggaccccca gtccggcgag gagctcaacc caatcccccc gccgccgcag ccctatcagc 23100

agcagccgcg ggcccttgct tcccaggatg gcacccaaaa agaagctgca gctgccgccg 23160

ccacccacgg acgaggagga atactgggac agtcaggcag aggaggtttt ggacgaggag 23220

gaggaggaca tgatggaaga ctgggagagc ctagacgagg aagcttccga ggtcgaagag 23280

gtgtcagacg aaacaccgtc accctcggtc gcattcccct cgccggcgcc ccagaaatcg 23340

gcaaccggtt ccagcatggc tacaacctcc gctcctcagg cgccgccggc actgcccgtt 23400

cgccgaccca accgtagatg ggacaccact ggaaccaggg ccggtaagtc caagcagccg 23460

ccgccgttag cccaagagca acaacagcgc caaggctacc gctcatggcg cgggcacaag 23520

aacgccatag ttgcttgctt gcaagactgt gggggcaaca tctccttcgc ccgccgcttt 23580

cttctctacc atcacggcgt ggccttcccc cgtaacatcc tgcattacta ccgtcatctc 23640

tacagcccat actgcaccgg cggcagcggc agcggcagca acagcagcgg ccacacagaa 23700

gcaaaggcga ccggatagca agactctgac aaagcccaag aaatccacag cggcggcagc 23760

agcaggagga ggagcgctgc gtctggcgcc caacgaaccc gtatcgaccc gcgagcttag 23820

aaacaggatt tttcccactc tgtatgctat atttcaacag agcaggggcc aagaacaaga 23880

gctgaaaata aaaaacaggt ctctgcgatc cctcacccgc agctgcctgt atcacaaaag 23940

cgaagatcag cttcggcgca cgctggaaga cgcggaggct ctcttcagta aatactgcgc 24000

gctgactctt aaggactagt ttcgcgccct ttctcaaatt taagcgcgaa aactacgtca 24060

tctccagcgg ccacacccgg cgccagcacc tgtcgtcagc gccattatga gcaaggaaat 24120

tcccacgccc tacatgtgga gttaccagcc acaaatggga cttgcggctg gagctgccca 24180

agactactca acccgaataa actacatgag cgcgggaccc cacatgatat cccgggtcaa 24240

cggaatccgc gcccaccgaa accgaattct cttggaacag gcggctatta ccaccacacc 24300

tcgtaataac cttaatcccc gtagttggcc cgctgccctg gtgtaccagg aaagtcccgc 24360

tcccaccact gtggtacttc ccagagacgc ccaggccgaa gttcagatga ctaactcagg 24420

ggcgcagctt gcgggcggct ttcgtcacag ggtgcggtcg cccgggcagg gtataactca 24480

cctgacaatc agagggcgag gtattcagct caacgacgag tcggtgagct cctcgcttgg 24540

tctccgtccg gacgggacat ttcagatcgg cggcgccggc cgtccttcat tcacgcctcg 24600

tcaggcaatc ctaactctgc agacctcgtc ctctgagccg cgctctggag gcattggaac 24660

tctgcaattt attgaggagt ttgtgccatc ggtctacttt aaccccttct cgggacctcc 24720

cggccactat ccggatcaat ttattcctaa ctttgacgcg gtaaaggact cggcggacgg 24780

ctacgactga atgttaagtg gagaggcaga gcaactgcgc ctgaaacacc tggtccactg 24840

tcgccgccac aagtgctttg cccgcgactc cggtgagttt tgctactttg aattgcccga 24900

ggatcatatc gagggcccgg cgcacggcgt ccggcttacc gcccagggag agcttgcccg 24960

tagcctgatt cgggagttta cccagcgccc cctgctagtt gagcgggaca ggggaccctg 25020

tgttctcact gtgatttgca actgtcctaa ccttggatta catcaagatc ctctagttat 25080

aactagagta cccggggatc ttattccctt taactaataa aaaaaaataa taaagcatca 25140

cttacttaaa atcagttagc aaatttctgt ccagtttatt cagcagcacc tccttgccct 25200

cctcccagct ctggtattgc agcttcctcc tggctgcaaa ctttctccac aatctaaatg 25260

gaatgtcagt ttcctcctgt tcctgtccat ccgcacccac tatcttcatg ttgttgcaga 25320

tgaagcgcgc aagaccgtct gaagatacct tcaaccccgt gtatccatat gacacggaaa 25380

ccggtcctcc aactgtgcct tttcttactc ctccctttgt atcccccaat gggtttcaag 25440

agagtccccc tggggtactc tctttgcgcc tatccgaacc tctagttacc tccaatggca 25500
tgcttgcgct caaaatgggc aacggcctct ctctggacga ggccggcaac cttacctccc 25560
aaaatgtaac cactgtgagc ccacctctca aaaaaaccaa gtcaaacata aacctggaaa 25620
tatctgcacc cctcacagtt acctcagaag ccctaactgt ggctgccgcc gcacctctaa 25680
tggtcgcggg caacacactc accatgcaat cacaggcccc gctaaccgtg cacgactcca 25740
aacttagcat tgccacccaa ggacccctca cagtgtcaga aggaaagcta gccctgcaaa 25800
catcaggccc cctcaccacc accgatagca gtacccttac tatcactgcc tcaccccctc 25860
taactactgc cactggtagc ttgggcattg acttgaaaga gcccatttat acacaaaatg 25920
gaaaactagg actaaagtac ggggctcctt tgcatgtaac agacgaccta aacactttga 25980
ccgtagcaac tggtccaggt gtgactatta ataatacttc cttgcaaact aaagttactg 26040
gagccttggg ttttgattca caaggcaata tgcaacttaa tgtagcagga ggactaagga 26100
ttgattctca aaacagacgc cttatacttg atgttagtta tccgtttgat gctcaaaacc 26160
aactaaatct aagactagga cagggccctc tttttataaa ctcagcccac aacttggata 26220
ttaactacaa caaaggcctt tacttgttta cagcttcaaa caattccaaa aagcttgagg 26280
ttaacctaag cactgccaag gggttgatgt ttgacgctac agccatagcc attaatgcag 26340
gagatgggct tgaatttggt tcacctaatg caccaaacac aaatcccctc aaaacaaaaa 26400
ttggccatgg cctagaattt gattcaaaca aggctatggt tcctaaacta ggaactggcc 26460
ttagttttga cagcacaggt gccattacag taggaaacaa aaataatgat aagctaactt 26520
tgtggaccac accagctcca tctcctaact gtagactaaa tgcagagaaa gatgctaaac 26580
tcactttggt cttaacaaaa tgtggcagtc aaatacttgc tacagtttca gttttggctg 26640
ttaaaggcag tttggctcca atatctggaa cagttcaaag tgctcatctt attataagat 26700
ttgacgaaaa tggagtgcta ctaaacaatt ccttcctgga cccagaatat tggaacttta 26760
gaaatggaga tcttactgaa ggcacagcct atacaaacgc tgttggattt atgcctaacc 26820
tatcagctta tccaaaatct cacggtaaaa ctgccaaaag taacattgtc agtcaagttt 26880
acttaaacgg agacaaaact aaacctgtaa cactaaccat tacactaaac ggtacacagg 26940
aaacaggaga cacaactcca agtgcatact ctatgtcatt ttcatgggac tggtctggcc 27000
acaactacat taatgaaata tttgccacat cctcttacac tttttcatac attgcccaag 27060
aataaagaat cgtttgtgtt atgtttcaac gtgtttattt ttcaattgca gaaaatttca 27120
agtcattttt cattcagtag tatagcccca ccaccacata gcttatacag atcaccgtac 27180
cttaatcaaa ctcacagaac cctagtattc aacctgccac ctccctccca acacacagag 27240
tacacagtcc tttctccccg gctggcctta aaaagcatca tatcatgggt aacagacata 27300
ttcttaggtg ttatattcca cacggtttcc tgtcgagcca aacgctcatc aagtgatatt 27360

aataaactcc ccgggcagct cacttaagtt catgtcgctg tccagctgct gagccacagg 27420

ctgctgtcca acttgcggtt gcttaacggg cggcgaagga gaagtccacg cctacatggg 27480

gggagagtca taatcgtgca tcaggatagg gcggtggtgc tgcagcagcg cgcgaataaa 27540

ctgctgccgc cgccgctccg tcctgcagga atacaacatg gcagtggtct cctcagcgat 27600

gattcgcacc gcccgcagca taaggcgctt gtcctccggg cacagcagcg caccctgatc 27660

tcacttaaat cagcacagta actgcagcac agcaccacaa tattgttcaa aatcccacag 27720

tgcaaggcgc tgtatccaaa gctcatggcg gggaccacag aacccacgtg gccatcatac 27780

cacaagcgca ggtagattaa gtggcgaccc ctcataaaca cgctggacat aaacattacc 27840

tcttttggca tgttgtaatt caccacctcc cggtaccata taaacctctg attaaacatg 27900

gcgccatcca ccaccatcct aaaccagctg gccaaaacct gccccgccgg gntatacact 27960

gcagggaacc gggacttgga caatgacaag tgggagagcc caggactcgt aaccatggat 28020

catcatgctc gtcatgatat caatgttggc acaacacagg cacacgtgca tacacttcct 28080

caggattaca agctcctccc gcgttagaac catatcccag ggaacaaccc attcctgaat 28140

cagcgtaaat cccacactgc agggaagacc tcgcacgtaa ctcacgttgt gcattgtcaa 28200

agtgttacat tcgggcagca gcggatgatc ctccagtatg gtagcgcggg tttctgtctc 28260

aaaaggaggt agacgatccc tactgtacgg agtgcgccga gacaaccgag atcgtgttgg 28320

tcgtagtgtc atgccaaatg gaacgccgga cgtagtcata tttcctgaag caaaaccagg 28380

tgcgggcgtg acaaacagat ctgcgtctcc ggtctcgccg cttagatcgc tctgtgtagt 28440

agttgtagta tatccactct ctcaaagcat ccaggcgccc cctggcttcg ggttctatgt 28500

aaactccttc atgcgccgct gccctgataa catccaccac cgcagaataa gccacaccca 28560

gccaacctac acattcgttc tgcgagtcac acacgggagg agcgggaaga gctggaagaa 28620

ccatgttttt ttttttattc caaaagatta tccaaaacct caaaatgaag atctattaag 28680

tgaacgcgct cccctccggt ggcgtggtca aactctacag ccaaagaaca gataatggca 28740

tttgtaagat gttgcacaat ggcttccaaa aggcaaacgg ccctcacgtc caagtggacg 28800

taaaggctaa acccttcagg gtgaatctcc tctataaaca ttccagcacc ttcaaccatg 28860

cccaaataat tctcatctcg ccaccttctc aatatatctc taagcaaatc ccgaatattt 28920

aagtccgggc cattgtaaaa aatttggctc cagagcgccc tccaccttca gcctcaagca 28980

gcgaatcatg attgcaaaaa ttcaggttcc tcacagacct gtataagatt caaaagcgga 29040

acattaacaa aaataccgcg atcccgtagg tcccttcgca gggccagctg aacataatcg 29100

tgcaggtctg cacggaccag cgcggccact tccccgccag gaaccatgac aaaagaaccc 29160

acactgatta tgacacgcat actcggagct atgctaacca gcgtagcccc gatgtaagct 29220

tgttgcatgg gcggcgatat aaaatgcaag gtgctgctca aaaaatcagg caaagcctcg 29280

cgcaaaaaag aaagcacatc gtagtcatgc tcatgcagat aaaggcaggt aagctccgga 29340

accaccacag aaaaagacac catttttctc tcaaacatgt ctgcgggttt ctgcataaac 29400

acaaaataaa ataacaaaaa aacatttaaa cattagaagc ctgtcttaca acaggaaaaa 29460

caacccttat aagcataaga cggactacgg ccatgccggc gtgaccgtaa aaaaactggt 29520

caccgtgatt aaaaagcacc accgacagct cctcggtcag tccggagtca taatgtaaga 29580

ctcggtaaac acatcaggtt gattcacatc ggtcagtgtt aaaaagcgac cgaaatagcc 29640

cgggggaata catacccgca ggcgtagaga caacattaca gcccccatag gaggtataac 29700

aaaattaata ggagagaaaa acacataaac acctgaaaaa ccctcctgcc taggcaaaat 29760

agcaccctcc cgctccagaa caacatacag cgcttcacag cggcagccta acagtcagcc 29820

ttaccagtaa aaaagaaaac ctattaaaaa aacaccactc gacacggcac cagctcaatc 29880

agtcacagtg taaaaaaggg ccaagtgcag agcgagtata tataggacta aaaaatgacg 29940

taacggttaa agtccacaaa aaacacccag aaaaccgcac gcgaacctac gcccagaaac 30000

gaaagccaaa aaacccacaa cttcctcaaa tcgtcacttc cgttttccca cgttacgtaa 30060

cttcccattt taagaaaact acaattccca acacatacaa gttactccgc cctaaaacct 30120

acgtcacccg ccccgttccc acgccccgcg ccacgtcaca aactccaccc cctcattatc 30180

atattggctt caatccaaaa taaggtatat tattgatgat gnnttaatta agggcggccg 30240

cacgggccat cgatggatcc gcggaagctt ctcgacccat tctcatgttt gacagcttat 30300

catcgaattt ctgccattca tccgcttatt atcacttatt caggcgtagc aaccaggcgt 30360

ttaagggcac caataactgc cttaaaaaaa ttacgccccg ccctgccact catcgcagta 30420

ctgttgtaat tcattaagca ttctgccgac atggaagcca tcacagacgg catgatgaac 30480

ctgaatcgcc agcggcatca gcaccttgtc gccttgcgta taatatttgc ccatggtgaa 30540

aacgggggcg aagaagttgt ccatattggc cacgtttaaa tcaaaactgg tgaaactcac 30600

ccagggattg gctgagacga aaaacatatt ctcaataaac cctttaggga aataggccag 30660

gttttcaccg taacacgcca catcttgcga atatatgtgt agaaactgcc ggaaatcgtc 30720

gtggtattca ctccagagcg atgaaaacgt ttcagtttgc tcatggaaaa cggtgtaaca 30780

agggtgaaca ctatcccata tcaccagctc accgtctttc attgccatac ggaattccgg 30840

atgagcattc atcaggcggg caagaatgtg aataaaggcc ggataaaact tgtgcttatt 30900

tttctttacg gtctttaaaa aggccgtaat atccagctga acggtctggt tataggtaca 30960

ttgagcaact gactgaaatg cctcaaaatg ttctttacga tgccattggg atatatcaac 31020

ggtggtatat ccagtgattt ttttctccat tttagcttcc ttagctcctg aaaatctcga 31080

taactcaaaa aatacgcccg gtagtgatct tatttcatta tggtgaaagt tggaacctct 31140

tacgtgccga tcaacgtctc attttcgcca aaagttggcc cagggcttcc cggtatcaac 31200

agggacacca ggatttattt attctgcgaa gtgatcttcc gtcacaggta tttattcgcg 31260

ataagctcat ggagcggcgt aaccgtcgca caggaaggac agagaaagcg cggatctggg 31320

aagtgacgga cagaacggtc aggacctgga ttggggaggc ggttgccgcc gctgctgctg 31380

acggtgtgac gttctctgtt ccggtcacac cacatacgtt ccgccattcc tatgcgatgc 31440

acatgctgta tgccggtata ccgctgaaag ttctgcaaag cctgatggga cataagtcca 31500

tcagttcaac ggaagtctac acgaaggttt ttgcgctgga tgtggctgcc cggcaccggg 31560

tgcagtttgc gatgccggag tctgatgcgg ttgcgatgct gaaacaatta tcctgagaat 31620

aaatgccttg gcctttatat ggaaatgtgg aactgagtgg atatgctgtt tttgtctgtt 31680

aaacagagaa gctggctgtt atccactgag aagcgaacga aacagtcggg aaaatctccc 31740

attatcgtag agatccgcat tattaatctc aggagcctgt gtagcgttta taggaagtag 31800

tgttctgtca tgatgcctgc aagcggtaac gaaaacgatt tgaatatgcc ttcaggaaca 31860

atagaaatct tcgtgcggtg ttacgttgaa gtggagcgga ttatgtcagc aatggacaga 31920

acaacctaat gaacacagaa ccatgatgtg gtctgtcctt ttacagccag taggctcgcc 31980

gcagtcgagc gacggcgaag ccctcgagtg agcgaggaag caccagggaa cagcacttat 32040

atattctgct tacacacgat gcctgaaaaa acttcccttg gggttatcca cttatccacg 32100

gggatatttt tataattatt ttttttatag tttttagatc ttctttttta gagcgccttg 32160

taggccttta tccatgctgg ttctagagaa ggtgttgtga caaattgccc tttcagtgtg 32220

acaaatcacc ctcaaatgac agtcctgtct gtgacaaatt gcccttaacc ctgtgacaaa 32280

ttgccctcag aagaagctgt tttttcacaa agttatccct gcttattgac tcttttttat 32340

ttagtgtgac aatctaaaaa cttgtcacac ttcacatgga tctgtcatgg cggaaacagc 32400

ggttatcaat cacaagaaac gtaaaaatag cccgcgaatc gtccagtcaa acgacctcac 32460

tgaggcggca tatagtctct cccgggatca aaaacgtatg ctgtatctgt tcgttgacca 32520

gatcagaaaa tctgatggca ccctacagga acatgacggt atctgcgaga tccatgttgc 32580

taaatatgct gaaatattcg gattgacctc tgcggaagcc agtaaggata tacggcaggc 32640

attgaagagt ttcgcgggga aggaagtggt tttttatcgc cctgaagagg atgccggcga 32700

tgaaaaaggc tatgaatctt ttccttggtt tatcaaacgt gcgcacagtc catccagagg 32760

gctttacagt gtacatatca acccatatct cattcccttc tttatcgggt tacagaaccg 32820

gtttacgcag tttcggctta gtgaaacaaa agaaatcacc aatccgtatg ccatgcgttt 32880

atacgaatcc ctgtgtcagt atcgtaagcc ggatggctca ggcatcgtct ctctgaaaat 32940

cgactggatc atagagcgtt accagctgcc tcaaagttac cagcgtatgc ctgacttccg 33000

ccgccgcttc ctgcaggtct gtgttaatga gatcaacagc agaactccaa tgcgcctctc 33060

atacattgag aaaaagaaag gccgccagac gactcatatc gtattttcct tccgcgatat 33120

cacttccatg acgacaggat agtctgaggg ttatctgtca cagatttgag ggtggttcgt 33180

cacatttgtt ctgacctact gagggtaatt tgtcacagtt ttgctgtttc cttcagcctg 33240

catggatttt ctcatacttt ttgaactgta atttttaagg aagccaaatt tgagggcagt 33300

ttgtcacagt tgatttcctt ctctttccct tcgtcatgtg acctgatatc gggggttagt 33360

tcgtcatcat tgatgagggt tgattatcac agtttattac tctgaattgg ctatccgcgt 33420

gtgtacctct acctggagtt tttcccacgg tggatatttc ttcttgcgct gagcgtaaga 33480

gctatctgac agaacagttc ttctttgctt cctcgccagt tcgctcgcta tgctcggtta 33540

cacggctgcg gcgagcgcta gtgataataa gtgactgagg tatgtgctct tcttatctcc 33600

ttttgtagtg ttgctcttat tttaaacaac tttgcggttt tttgatgact ttgcgatttt 33660

gttgttgctt tgcagtaaat tgcaagattt aataaaaaaa cgcaaagcaa tgattaaagg 33720

atgttcagaa tgaaactcat ggaaacactt aaccagtgca taaacgctgg tcatgaaatg 33780

acgaaggcta tcgccattgc acagtttaat gatgacagcc cggaagcgag gaaaataacc 33840

cggcgctgga gaataggtga agcagcggat ttagttgggg tttcttctca ggctatcaga 33900

gatgccgaga aagcagggcg actaccgcac ccggatatgg aaattcgagg acgggttgag 33960

caacgtgttg gttatacaat tgaacaaatt aatcatatgc gtgatgtgtt tggtacgcga 34020

ttgcgacgtg ctgaagacgt atttccaccg gtgatcgggg ttgctgccca taaaggtggc 34080

gtttacaaaa cctcagtttc tgttcatctt gctcaggatc tggctctgaa ggggctacgt 34140

gttttgctcg tggaaggtaa cgacccccag ggaacagcct caatgtatca cggatgggta 34200

ccagatcttc atattcatgc agaagacact ctcctgcctt tctatcttgg ggaaaaggac 34260

gatgtcactt atgcaataaa gcccacttgc tggccggggc ttgacattat tccttcctgt 34320

ctggctctgc accgtattga aactgagtta atgggcaaat ttgatgaagg taaactgccc 34380

accgatccac acctgatgct ccgactggcc attgaaactg ttgctcatga ctatgatgtc 34440

atagttattg acagcgcgcc taacctgggt atcggcacga ttaatgtcgt atgtgctgct 34500

gatgtgctga ttgttcccac gcctgctgag ttgtttgact acacctccgc actgcagttt 34560

ttcgatatgc ttcgtgatct gctcaagaac gttgatctta aagggttcga gcctgatgta 34620

cgtattttgc ttaccaaata cagcaatagt aatggctctc agtccccgtg gatggaggag 34680

caaattcggg atgcctgggg aagcatggtt ctaaaaaatg ttgtacgtga aacggatgaa 34740

gttggtaaag gtcagatccg gatgagaact gtttttgaac aggccattga tcaacgctct 34800

tcaactggtg cctggagaaa tgctctttct atttgggaac ctgtctgcaa tgaaattttc 34860

gatcgtctga ttaaaccacg ctgggagatt agataatgaa gcgtgcgcct gttattccaa 34920

aacatacgct caatactcaa ccggttgaag atacttcgtt atcgacacca gctgccccga 34980

tggtggattc gttaattgcg cgcgtaggag taatggctcg cggtaatgcc attactttgc 35040

ctgtatgtgg tcgggatgtg aagtttactc ttgaagtgct ccggggtgat agtgttgaga 35100

agacctctcg ggtatggtca ggtaatgaac gtgaccagga gctgcttact gaggacgcac 35160

tggatgatct catcccttct tttctactga ctggtcaaca gacaccggcg ttcggtcgaa 35220

gagtatctgg tgtcatagaa attgccgatg ggagtcgccg tcgtaaagct gctgcactta 35280

ccgaaagtga ttatcgtgtt ctggttggcg agctggatga tgagcagatg gctgcattat 35340

ccagattggg taacgattat cgcccaacaa gtgcttatga acgtggtcag cgttatgcaa 35400

gccgattgca gaatgaattt gctggaaata tttctgcgct ggctgatgcg gaaaatattt 35460

cacgtaagat tattacccgc tgtatcaaca ccgccaaatt gcctaaatca gttgttgctc 35520

ttttttctca ccccggtgaa ctatctgccc ggtcaggtga tgcacttcaa aaagccttta 35580

cagataaaga ggaattactt aagcagcagg catctaacct tcatgagcag aaaaaagctg 35640

gggtgatatt tgaagctgaa gaagttatca ctcttttaac ttctgtgctt aaaacgtcat 35700

ctgcatcaag aactagttta agctcacgac atcagtttgc tcctggagcg acagtattgt 35760

ataagggcga taaaatggtg cttaacctgg acaggtctcg tgttccaact gagtgtatag 35820

agaaaattga ggccattctt aaggaacttg aaaagccagc accctgatgc gaccacgttt 35880

tagtctacgt ttatctgtct ttacttaatg tcctttgtta caggccagaa agcataactg 35940

gcctgaatat tctctctggg cccactgttc cacttgtatc gtcggtctga taatcagact 36000

gggaccacgg tcccactcgt atcgtcggtc tgattattag tctgggacca cggtcccact 36060

cgtatcgtcg gtctgattat tagtctggga ccacggtccc actcgtatcg tcggtctgat 36120

aatcagactg ggaccacggt cccactcgta tcgtcggtct gattattagt ctgggaccat 36180

ggtcccactc gtatcgtcgg tctgattatt agtctgggac cacggtccca ctcgtatcgt 36240

cggtctgatt attagtctgg aaccacggtc ccactcgtat cgtcggtctg attattagtc 36300

tgggaccacg gtcccactcg tatcgtcggt ctgattatta gtctgggacc acgatcccac 36360

tcgtgttgtc ggtctgatta tcggtctggg accacggtcc cacttgtatt gtcgatcaga 36420

ctatcagcgt gagactacga ttccatcaat gcctgtcaag ggcaagtatt gacatgtcgt 36480

cgtaacctgt agaacggagt aacctcggtg tgcggttgta tgcctgctgt ggattgctgc 36540

tgtgtcctgc ttatccacaa cattttgcgc acggttatgt ggacaaaata cctggttacc 36600

caggccgtgc cggcacgtta accgggctgc atccgatgca agtgtgtcgc tgtcgagttt 36660

aaacatgcat ccttaattaa 36680